# EUROPEAN PATENT SPECIFICATION

(11) **EP 3 655 532 B1**
(45) Date of publication and mention of the grant of the patent: **18.08.2021**
(21) Application number: 18743749.6
(22) Date of filing: 17.07.2018
(51) Int. Cl.: C12N 15/10, C07K 14/725, C07K 14/705

(54) **A TWO-COMPONENT VECTOR LIBRARY SYSTEM FOR RAPID ASSEMBLY AND DIVERSIFICATION OF FULL-LENGTH T-CELL RECEPTOR OPEN READING FRAMES**
ZWEIKOMPONENTIGES VEKTORBIBLIOTHEKSSYSTEM ZUR SCHNELLEN ANORDNUNG UND DIVERSIFIZIERUNG VON OFFENEN LESERAHMEN EINES T-ZELLREZEPTORS MIT VOLLER LÄNGE
SYSTÈME DE BIBLIOTHÈQUE DE VECTEURS À DEUX COMPOSANTS POUR ASSEMBLAGE RAPIDE ET DIVERSIFICATION DE CADRES DE LECTURE OUVERTS DU RÉCEPTEUR DES LYMPHOCYTES T PLEINE LONGUEUR

(30) Priority: 18.07.2017 EP 17181798
(43) Date of publication of application: 27.05.2020
(73) Proprietor: Genovie AB, 151 36 Södertälje (SE)
(72) Inventor: JARVIS, Reagan Micheal, 151 36 Södertälje (SE); HILL, Ryan Edward, 151 36 Södertälje (SE); PASE, Luke Benjamin, 151 36 Södertälje (SE)
(74) Representative: Chas. Hude A/S
(86) International application number: PCT/EP2018/069333
(87) International publication number: WO 2019/016175

(56) References cited:
- WO-A1-2008/095927
- WO-A1-2011/154147
- WO-A1-2013/144257
- WO-A1-2015/136072
- WO-A1-2016/193299
- XI-ZHI J GUO ET AL: "Rapid cloning, expression, and functional characterization of paired alphabeta and gammadelta T-cell receptor chains from single-cell analysis", MOLECULAR THERAPY - METHODS & CLINICAL DEVELOP, vol. 3, 1 January 2016 (2016-01-01), page 15054, XP055386592, ISSN: 2329-0501, DOI: 10.1038/mtm.2015.54 cited in the application
- ARNOLD HAN ET AL: "Linking T-cell receptor sequence to functional phenotype at the single-cell level", NATURE BIOTECHNOLOGY (ADVANCE ONLINE PUBLICATION), vol. 32, no. 7, 22 June 2014 (2014-06-22), pages 684-692, XP055309747, ISSN: 1087-0156, DOI: 10.1038/nbt.2938

## Description

### Field of the invention

The heterodimeric T-cell receptor (TCR) is central to adaptive immunity. Unique TCRs are continually generated during T-cell genesis whereby an array of gene segments are recombined into a single contiguous TCR open reading frame (ORF). Due to the large degree of diversity generated in this process of recombination, it is challenging to capture sequence data from TCR pairs that are expressed in single cells. Moreover, this diversity also makes it a challenging to provide these TCR open reading frames (ORFs) within genetic constructs on a high-throughput basis for testing and manipulation of TCR function. The present invention in the first aspect provides a pre-assembled two-component vector library system consisting of *Variable-Constant entry* vectors (V-C entry) and *Joining donor* (J donor) vectors comprising portions of TCR gene segments. The two component system is designed in such a way that when a V-C entry vector selected from the V-C entry vector library is combined with a J donor vector selected from the J donor vector library, along with a synthetic DNA *oligonucleotide duplex encoding TCR complementarity determining region 3* (odeCDR3) in a restriction enzyme digestion / ligase cycle reaction, a single vector is created reconstituting the full-length TCR ORF. Such a vector library system enables PCR-independent methods for rapid and cost effective generation of TCR ORFs in selected vector contexts. In addition, this system permits novel workflows for generating synthetic TCR sequences for affinity and/or functional maturation workflows. This TCR ORF Reconstitution and Engineering System (TORES) is thus a strong tool for TCR functional analysis and engineering. In the second aspect, a TORES with modified V-C entry vectors is provided along with a third component bidirectional terminator donor vector (BiT donor) as to enable a second step to adjoin two TCR ORF chain pairs, in an antiparallel coding sense, into a single product vector. This bidirectional TORES² system enables alternative workflows to deliver paired TCR ORF constructs for TCR manipulation and characterisation.

### Background of the invention

Immune surveillance by T lymphocytes (T-cells) is a central function in the adaptive immunity of all jawed vertebrates. Immune surveillance by T-cells is achieved through a rich functional diversity across T-cell subtypes, which serve to eliminate pathogeninfected and neoplastic cells and orchestrate adaptive immune responses to invading pathogens, commensal microorganisms, commensal non-self factors such as molecular components of foodstuffs, and even maintain immune tolerance of self. In order to respond to various foreign and self factors, T-cells must be able to specifically detect molecular constituents of these foreign and self factors. Thus T-cells must be able to detect a large cross-section of the self and non-self molecules that an individual encounters, with sufficient specificity to mount efficient responses against pathogenic organisms and diseased self, while avoiding the mounting of such responses against health self. The highly complex nature of this task becomes clear when considering the practically unlimited diversity of both foreign and self molecules, and that pathogenic organisms are under evolutionary pressure to evade detection by T-cells.

### The T-cell Receptor (TCR)

T-cells are primarily defined by the expression of a T-cell receptor (TCR). The TCR is the component of the T-cell that is responsible for interacting with and sensing the targets of T-cell adaptive immunity. In general terms, the TCR is comprised of a heterodimeric protein complex presented on the cell surface. Each of the two TCR chains are composed of two extracellular domains, being the variable (V)-region and the constant (C)-region, both of the immunoglobulin superfamily (IgSF) domain, forming antiparallel β-sheets. These are anchored in the cell membrane by a type-I transmembrane domain, which adjoins a short cytoplasmic tail. The quality of the T-cells to adapt and detect diverse molecular constituents arises from variation in the TCR chains that is generated during T-cell genesis. This variation is generated by somatic recombination in a similar manner to antibody genesis in B-cells.

### TCR chain diversity

The T cell pool consists of several functionally and phenotypically heterogeneous subpopulations. However, T cells may be broadly classified as αβ or γδ according to the somatically rearranged TCR form they express at their surface. There exist two TCR chain pair forms; TCR alpha (TRA) and TCR beta (TRB) pairs; and TRC gamma (TRG) and TCR delta (TRD) pairs. T-cells expressing TRA:TRB pairs are referred to as αβ T-cells, while T-cells expressing TRG:TRD pairs are often referred to as γδ T-cells.

TCRs of both αβ and γδ forms are responsible for recognition of diverse ligands, or 'antigens', and each T-cell generates αβ or γδ receptor chains de novo during T-cell maturation. These de novo TCR chain pairs achieve diversity of recognition through generation of receptor sequence diversity in a process called somatic V(D)J recombination after which each T-cell expresses copies of a single distinctly rearranged TCR. At the TRA and TRG loci, a number of discrete variable (V) and functional (J) gene segments are available for recombination and juxtaposed to a constant (C) gene segments, thus referred to as VJ recombination. Recombination at the TRB and TRD loci additionally includes a diversity (D) gene segment, and is referred to as VDJ recombination.

Each recombined TCR possess potential for unique ligand specificity, determined by the structure of the ligand-binding site formed by the α and β chains in the case of αβ T-cells or γ and δ chains in the case of γδ T-cells. The structural diversity of TCRs is largely confined to three short hairpin loops on each chain, called complementaritydetermining regions (CDR). Three CDRs are contributed from each chain of the receptor chain pair, and collectively these six CDR loops sit at the membrane-distal end of the TCR extracellular domain to form the antigen-binding site.

Sequence diversity in each TCR chain is achieved in two modes. First, the random selection of gene segments for recombination provides basal sequence diversity. For example, TRB recombination occurs between 47 unique V, 2 unique D and 13 unique J germline gene segments. In general, the V gene segment contributes both the CDR1 and CDR2 loops, and are thus germline encoded. The second mode to generate sequence diversity occurs within the hypervariable CDR3 loops, which are generated by random deletion of template nucleotides and addition of non-template nucleotides, at the junctions between recombining V, (D) and J gene segments.

### TCR:CD3 Complex

Mature αβ and γδ TCR chain pairs are presented at the cell surface in a complex with a number of accessory CD3 subunits, denoted ε, γ, δ and ζ. These subunits associate with αβ or γδ TCRs as three dimers (εγ, εδ, ζζ). This TCR:CD3 complex forms the unit for initiation of cellular signalling responses upon engagement of a αβ or γδ TCR with cognate antigen. The CD3 accessories associated as a TCR:CD3 complex contribute signalling motifs called immunoreceptor tyrosine-based activation motifs (ITAMs). CD3e, CD3y and CD3δ each contribute a single ITAM while the CD3ζ homodimer contains 3 ITAMs. The three CD3 dimers (εγ, εδ, ζζ) that assemble with the TCR thus contribute 10 ITAMs. Upon TCR ligation with cognate antigen, phosphorylation of the tandem tyrosine residues creates paired docking sites for proteins that contain Src homology 2 (SH2) domains, such as the critical ζ-chain-associated protein of 70 kDa (ZAP-70). Recruitment of such proteins initiate the formation of TCR:CD3 signalling complexes that are ultimately responsible for T-cell activation and differentiation.

### αβ T-cells

αβ T-cells are generally more abundant in humans than their γδ T-cell counterparts. A majority of αβ T-cells interact with peptide antigens that are presented by HLA complexes on the cell surface. These peptide-HLA (pHLA)-recognising T-cells were the first to be described and are by far the best characterised. More rare forms of αβ T-cells have also been described. Mucosal-associated invariant T (MAIT) cells appear to have a relatively limited α and β chain diversity, and recognise bacterial metabolites rather than protein fragments. The invariant natural killer T-cells (iNK T-cells) and germlineencoded mycolyl-reactive T-cells (GEM T-cells) are restricted to recognition of glycolipids that are cross-presented by non-HLA molecules. iNK T-cells are largely considered to interact with CD1d-presented glycolipids, whereas GEM T-cells interact with CD1b-presented glycolipids. Further forms of T-cells are thought to interact with glycolipids in the context of CD1a and CD1c, however, such cells are yet to be characterised in significant detail.

### Conventional αβ T-cells

The key feature of most αβ T-cells is the recognition of peptide antigens in the context of HLA molecules. These are often referred to as 'conventional' αβ T-cells. Within an individual, self-HLA molecules present peptides from self and foreign proteins to T-cells, providing the essential basis for adaptive immunity against malignancies and foreign pathogens, adaptive tolerance towards commensal organisms, foodstuffs and self. The HLA locus that encodes HLA proteins is the most gene-dense and polymorphic region of the human genome, and there are in excess of 12,000 alleles described in humans. The high degree of polymorphism in the HLA locus ensures a diversity of peptide antigen presentation between individuals, which is important for immunity at the population level.

### HLA class I and II

There are two forms of classical HLA complexes: HLA class I (HLAI) and HLA class II (HLAII). There are three classical HLAI genes: *HLA-A, HLA-B, HLA-C.* These genes encode a membrane-spanning α-chain, which associates with an invariant β2-microglobulin (β2M) chain. The HLAI α-chain is composed of three domains with an immunoglobulin fold: α1, α2 and α3. The α3 domain is membrane-proximal and largely invariant, while the α1 and α2 domains together form the polymorphic membrane-distal antigen-binding cleft. There are six classical HLAII genes: *HLA-DPA1, HLA-DPB1, HLA-DQA1, HLA-DQB1, HLA-DRA,* and *HLA-DRB1.* These genes encode paired DP, DQ and DR heterodimeric HLA complexes comprising a α-chain and a β-chain. Each chain has two major structural domains with an immunoglobulin fold, where the α2 and β2 domain comprise membrane-proximal and largely invariant modules similar to that of HLAI α3 domain. The HLAII α2 and β2 domains together form the membrane-distal antigen-binding cleft and are regions of high polymorphism.

The antigen-binding cleft of HLAI and HLAII comprises two anti-parallel α-helices on a platform of eight anti-parallel β-sheets. In this cleft the peptide antigen is bound and presented in an extended conformation. The peptide-contacting residues in HLAI and HLAII are the location of most of the sequence polymorphism, which constitutes the molecular basis of the diverse peptide repertoires presented by different HLA alleles. The peptide makes extensive contacts with the antigen-binding cleft and as a result each HLA allele imposes distinct sequence constraints and preferences on the presented peptides. A given peptide will thus only bind a limited number of HLAs, and reciprocally each allele only accommodates a particular fraction of the peptide collection from a given protein. The set of HLAI and HLAII alleles that is present in each individual is called the HLA haplotype. The polymorphism of HLAI and HLAII genes and the codominant expression of inherited alleles drives very large diversity of HLA haplotype across the human population, which when coupled to the enormous sequence diversity of αβ TCRs, presents high obstacles to standardisation of analysis of these HLA-antigen-TCR interactions.

### αβ TCR engagement of HLAI and HLAII

αβ TCRs recognize peptides as part of a mixed pHLA binding interface formed by residues of both the HLA and the peptide antigen (altered self). HLAI complexes are presented on the surface of nearly all nucleated cells and are generally considered to present peptides derived from endogenous proteins. T-cells can thus interrogate the endogenous cellular proteome of an HLAI-presenting cell by sampling pHLAI complexes of an interacting cell. Engagement of HLAI requires the expression of the TCR co-receptor CD8 by the interacting T-cell, thus HLAI sampling is restricted to CD8⁺ αβ T-cells. In contrast, the surface presentation of HLAII complexes is largely restricted to professional APC, and are generally considered to present peptides derived from proteins exogenous to the presenting cell. An interacting T-cell can therefore interrogate the proteome of the extracellular microenvironment in which the presenting cell resides.

The engagement of HLAII requires the expression of the TCR co-receptor CD4 by the interacting T-cell, thus HLAII sampling is restricted to CD4⁺ αβ T-cells.

### Thymic selection of αβ TCRs

The role of αβ TCRs as described above is the detection of pHLA complexes, such that the TCR-presenting T-cell can raise responses germane to the role of that T-cell in establishing immunity. It should be considered that the αβ TCR repertoire generated within an individual must account for the immense and unforeseen diversity of all foreign antigens likely to be encountered in the context of a specific haplotype and prior to their actual occurrence. This outcome is achieved on a background where extremely diverse and numerous αβ TCRs are generated in a quasi-randomised manner with the potential to recognise unspecified pHLA complexes while only being specifically instructed to avoid strong interactions with self pHLA. This is carefully orchestrated during T-cell maturation in a process call thymic selection.

During the first step of T-cell maturation in the thymus, T-cells bearing αβ TCRs that are incapable of interacting with self-pHLA complexes with sufficient affinity, are deprived of a survival signal and eliminated. This step called positive selection assures that the surviving T-cells carry a TCR repertoire that is at least potentially capable of recognizing foreign or altered peptides presented in the right HLA context. Subsequently, αβ TCR that strongly interact with self-pHLA and thus have the potential to drive autoimmunity are actively removed through a process of negative selection. This combination of positive and negative selection results in only T-cells bearing αβ TCRs with low affinity for self-pHLA populating the periphery. This establishes an αβ T-cell repertoire that is self-restricted but not self-reactive. This highly individualised nature of T-cell genesis against HLA haplotype underscores the challenges in standardised analysis αβ TCR-antigen-HLA interactions. Moreover, it forms the basis of both graft rejection and graft versus host disease and the general principle that αβ TCRs identified in one individual may have completely different effect in a second individual, which has clear implications for TCR-based and T-cell based therapeutic and diagnostic strategies emerging in clinical practice.

### Unconventional αβ T-cells

The non-HLA-restricted, or 'unconventional', forms of αβ T-cells have very different molecular antigen targets. These unconventional αβ T-cells do not engage classical HLA complexes, but rather engage conserved HLA-like proteins such as the CD1 family or MR1. The CD1 family comprises four forms involved in antigen cross-presentation (CD1a,b,c and d). These cell surface complexes have an α-chain resembling HLAI, which forms heterodimers with β2-M. A small hydrophobic pocket presented at the membrane distal surface of the α-chain forms a binding site for pathogen-derived lipidbased antigens. Innate like NK T-cells (iNK T-cells) form the best-understood example of lipid/CD1 family recognition with GEM T-cells representing another prominent example. 'Type I' iNK T-cells are known to interact strongly with the lipid α-GalCer in the context of CD1d. These iNK T-cells display very limited TCR diversity with a fixed TCR α-chain (Vα10/Jα18) and a limited number of β-chains (with restricted vβ usage) and they have been likened to innate pathogen-associated molecular patterns (PAMPS) recognition receptors such as Toll-like and Nod-like receptors. In contrast, 'type II' NK T-cells present a more diverse TCR repertoire, and appear to have a more diverse mode of CD1d-lipid complex engagement. GEM T-cells recognize mycobacteria-derived glycolipids presented by CD1b, however, the molecular details of antigen presentation by CD1a, b and c as well as their T-cell recognition are only beginning to be understood.

MAIT cells largely express an invariant TCR α-chain (TRAV1-2 ligated to TRAJ33, TRAJ20, or TRAJ12), which is capable of pairing with an array of TCR β-chains. Instead of peptides or lipids MAIT TCRs can bind pathogen-derived folate- and riboflavinbased metabolites presented by the HLAI-like molecule, MR1. The limited but significant diversity in the TCRs observed on MAIT TCRs appear to enable the recognition of diverse but related metabolites in the context of the conserved MR1.

It is not well-understood how non-classical HLA-restricted αβ T-cell TCRs are selected in the thymus during maturation. However, it appears likely that the fundamental process of negative and positive selection outlined above still applies and some evidence suggests that this occurs in specialized niches within the thymus.

### yδ T-cells

In contrast to the detailed mechanistic understanding of αβ TCR genesis and pHLA engagement, relatively little is known about the antigen targets and context of their γδ T-cell counterparts. This is in part due to their relatively low abundance in the circulating T-cell compartment. However, it is broadly considered that γδ T-cells are not strictly HLA restricted and appear to recognize surface antigen more freely not unlike antibodies. Additionally, more recently it has become appreciated that γδ T-cells can dominate the resident T-cell compartment of epithelial tissues, the main interaction site of the immune system with foreign antigen. In addition, various mechanisms for γδ T-cell tumour immunuosurveillance and surveillance of other forms of dysregulated-self are beginning to emerge in the literature. The specific antigen targets of both innate-like and adaptive γδ T-cells remain poorly defined but the tissue distribution and fast recognition of PAMPs suggests a fundamental role for γδ T-cells both early in responses to foreign antigens as well as early in life when the adaptive immune system is still maturing.

The diverse functions of γδ T-cells appear to be based on different Vy Vδ gene segment usage and can be broadly understood in two main categories in which γδ T-cells with largely invariant TCRs mediate innate-like recognition of PAMPs very early during infection. Beyond PAMPs these type of γδ T-cells are furthermore believed to recognize self-molecules, including phosphoantigens that could provide very early signatures of cellular stress, infection and potentially neoplastic development. Recognition of PAMPs and such so-called danger associated molecular patterns (DAMPS) as well as the large numbers of tissue-restricted innate-like γδ T-cells strongly suggests that these cells are suited to respond rapidly to antigenic challenge without the need for prior activation, homing and clonal expansion.

A second form of γδ T-cells are considered to be more adaptive in nature, with a highly diverse γδ TCR repertoire and the ability to peripherally circulate and access lymphoid tissues directly. Such antigen-specific γδ T-cells to common human pathogens such as CMV have been described and they appear to form a memory response. However, it has also been observed that γδ T-cells show only relatively limited clonal proliferation after activation and little data is available on the extent of TCR diversity and specific responses of γδ T-cells in peripheral circulation, or in tissues. Furthermore, while it is generally considered that γδ TCRs do not interact with pHLA complexes, and thus do not engage with peptide antigens in this context only few antigen targets of γδ T-cells have been characterised and the underlying molecular framework is only poorly understood.

The low frequency of peripheral γδ T-cells and the difficulty to study tissue-resident T-cells in humans has limited our knowledge of how this important and diverse type of T-cells participate in adaptive immune responses. This emerging area of research would require more reliable technologies with which to capture and characterise rare γδ T-cells, isolate their TCR pairs, and to identify their cognate antigens.

### Antigens and Antigen-presenting cells

In the context of T-cells and TCRs, antigens may be defined as any molecule that may be engaged by a TCR and resulting in a signal being transduced within the T-cell. The most well characterised T-cell antigens are peptides presented in an HLAI and HLAII complex, and which are engaged by conventional αβ T-cells. However, in recent years it has become apparent that non-conventional αβ T-cells and γδ T-cells are able to engage a wide range of biomolecules as antigens, including lipids, lipopeptides, glycopeptides, glycolipds and a range of metabolites and catabolites. In addition, it has emerged that γδ T-cells may be able to engage fully folded proteins directly in an antibody-like fashion. Therefore, the view of T-cell antigens being largely restricted to HLApresented peptides has expanded over the past two decades to include almost any biomolecule. With this concept in mind, it is relevant to define what may be considered an antigen-presenting cell (APC).

As defined in the above sections, HLAI and HLAII have a disparate expression profiles across cell types. It is widely accepted that nearly all nucleated cells present HLAI complexes on the cell surface, and are thus competent to present peptide antigens for T-cell sampling. In contrast, HLAII has a restricted expression profile, and at least in steady state conditions is only expressed on the surface of cells that have a specialist role in antigen presentation, including dendritic cells (DC), macrophage and B-cells. These specialist cell types are often referred to as professional APC. For the purposes of this document, the term APC is used to describe any nucleated cell that is capable of presenting an antigen for sampling by αβ or γδ T-cells. Such antigens are not restricted to those presented as 'cargo' in specific antigen-presenting complexes such as HLA and HLA-like molecules, but may also include any cell-surface presented moiety that is able to engage a αβ or γδ TCR-bearing cell.

### Therapeutic use of TCRs

Adoptive transfer of primary T-cells was first trialled in a clinical setting in the early 1990s, starting with ex vivo expanded T-cells polarised towards viral antigens to confer viral immunity in immunocompromised patients. Similar approaches using primary T-cells expanded ex vivo against specific cancer antigens were soon after trialled in treatment of malignancies. One limitation in these early approaches that continues to be a challenge today is a lack of understanding of the nature and diversity of T-cells clashing with the need to finely-optimize their composition in the therapeutic product. At present, the use of ex vivo expanded primary T-cells has largely been abandoned by the pharmaceutical industry with the exception of a handful of initiatives using primary T-cells with specificity for viral antigens.

In recent years the ability to reliably introduce genetic material into primary human cells has seen a variety of experimental genetically modified T-cell therapeutics arise. Such therapeutic cell products aim to harness the power of T-cell responses and redirect T-cell specificity towards a disease-associated antigen target, for example, an antigen uniquely expressed by malignant cells. These have largely relied on the transfer of a chimeric antigen receptor (CAR) into recipient T-cells, rather than actual TCR chain pairs. A CAR represents a targeting moiety (most often a single-chain antibody element targeting a surface expressed protein of malignant cells) grafted to signal receptor elements such as the ζ-chain of the CD3 complex, to produce a synthetic chimeric receptor that mimics CD3-TCR function. These so-called CAR T-cell (CAR-T) products have met mixed success in clinical trials to date and despite their potential are not easy to translate beyond tumours with inherent unique molecular targets such as B-cell malignancies. Alternatively, the transfer of full-length TCR chain pair ORFs into T-cells is of emerging interest. Such TCR-engineered T-cell therapeutics are at present limited by challenging manufacturing processes, arid like the CAR-T products, a dearth of validated antigen targets and targeting constructs. To date this has been focused on the use of αβ TCRs for recognition of peptide antigens presented by HLAI on malignant cells and a fundamental challenge of this approach is the need for antigens that are specific to malignant cells.

It has been considered that since the TCR-pHLA interaction is of relatively low-affinity, native TCRs are likely to be suboptimal for TCR-engineered T-cell therapies. Several approaches have been devised to affinity-mature TCRs in vitro, in much the same manner as single-chain antibody affinity maturation. These TCR affinity maturation approaches generally also utilise a single-chain formats, wherein the V-region of one chain is fused to V-region of another chain to make a single polypeptide construct. Such single polypeptides may then be used in phage- or yeast- display systems adapted from antibody engineering workflows, and passed through rounds of selection based on target binding. Two inherent limitations exist in such a single-chain TCR approach in terms of yielding functional TCR chain pairs. Firstly, the selection is based on binding affinity to the target. However, it has been well documented that TCR affinity does not always correlate to the strength or competency of TCR signalling output. Secondly, the selection of single-chain constructs based on affinity does not always translate to equivalent affinities once they are reconstituted as full-length receptors.

In a therapeutic context, there exists an additional limitation in affinity-matured TCR pairs. That is, considering their sequences have been altered, the resulting constructs by definition have no longer been subject to thymic selection, wherein TCRs that react strongly to self-antigens are deleted from the repertoire. Therefore, these modified TCRs carry an inherent risk of being auto-reactive, which is very difficult to rule out in vitro using current methods. For the same reason, any selected or engineered TCR for therapeutic application needs to be individualised. If TCRs are artificially engineered or native TCRs applied across individuals, cross-reactivities have to be ruled out on the basis of the HLA haplotype and presented peptide repertoire of each specific individual in order to avoid potentially catastrophic autoimmunity. This is due to the fact that thymic selection is conducted on a background of all available HLA molecules specific only to that given individual. The likelihood of such cross-reactivity is unclear. However, the ability of our TCR repertoire to recognize pHLA complexes of other individuals of the same species as foreign is a fundamental property of adaptive immunity and underpins graft rejection and graft versus host disease. Recent clinical trials using a matured TCR chain pair against the cancer-specific melanoma associated antigen (MAGE) highlighted the potential problem of bypassing thymic selection. When autologous T-cells harbouring the matured TCRs were infused back to two cancer patients, these patients rapidly developed a fatal heart disease. Subsequent studies determined that the MAGE-specific matured TCRs were cross-reactive with an HLAI-presented peptide from the heart protein titin. This strongly suggests that cross-reactivity is a distinct possibility in therapeutic use of TCRs.

A distinct avenue of utilising TCRs for therapeutic purposes is in their use as affinity reagents in much the same manner as antibody therapeutic substances. Single-chain TCR molecules have been trialled for delivery of conjugated drug substances to specific HLA-antigen expressing cell populations. Such an approach is generally considered safer than CAR-T or TCR engineered T-cell therapeutics, as administration of the drug substance may simply be withdrawn. However, the potential for cross-reactivity and off target effects that are difficult to predict remains a potential limitation in this setting.

### TCR repertoire detection in clinical diagnostics

In a related aspect, there is an emerging interest in using the detection of the abundance of specific TCR sequences for clinical diagnostic purposes. With the rise of deep-sequencing methods in particular, it is possible to capture the full TCR diversity within an individual globally and for matched αβ pairs in specific contexts. This potentially represents a means to diagnose specific conditions and disease states simply by detecting the abundance of expanded T-cell clones, as proxy readout for established immune response against a disease-associated antigen in the patient. However, such global approaches are currently limited to very strong immune responses with established clinical time-points and suffer from the underlying difficulty in identifying the specific antigen target of any particular TCR identified via sequencing.

### Therapeutic and diagnostic use of T-cell antigens

The fundamental strength of harnessing adaptive immune responses translates into a central technical challenge in that the exquisite specificity of the TCR-antigen interaction requires detailed knowledge of the antigens specifically associated with each pathogen, cancer cell or autoimmune disease. Furthermore, each antigen may be presented by a specific antigen presenting complex, or allele thereof, such that antigen discovery has be performed for each relevant HLA gene and allele. For several infectious diseases like HIV, influenza and CMV that are associated with strong adaptive immune responses and generally display conserved epitope response hierarchies, the most important epitopes have been mapped in context of some common HLA. Similarly, the fields of cancer, allergy and autoimmunity have seen increased and systematic efforts to map the associated T-cell antigens. However, these are challenging procedures and the efforts to systematically describe T-cell antigens associated with different clinical contexts are hindered by the absence of efficient, robust, fast and scalable protocols.

Specifically, cancer cells represent a challenging and important aspect as most of the peptides presented on the surface of malignant cells are self antigens or very similar to self antigens. Therefore, thymic selection will have deleted TCRs that could strongly recognize these peptides, while at the same time the tumour has evolved to evade immune recognition. This means that potent immune responses against established tumours are relatively rare and targets difficult to predict or discover. However, these responses do exist and, importantly, are generally associated with better outcome. The target of such responses, tumour-associated-antigens (TAA), will in most cases have distinguishing characteristics from self arid be derived from proteins that are overexpressed during cancer development, otherwise absent from the cell type at this stage of development or specifically altered through genetic mutation or post-translational modifications such as phosphorylation.

When available, the knowledge of such epitopes makes it possible to interrogate the associated T-cell response for fundamental discovery, diagnostic purposes and for example as a test of vaccine efficacy. Importantly, they also provide highly specific targets for T-cell tolerization in allergy and autoimmunity and, crucially, point towards valuable targets for specific immunotherapy and against malignant cells. Malignancies represent a particularly valuable target as the promise of cellular immunotherapies and the progress in the T-cell manipulations are slowed by a lack of validated target TAAs that go beyond the few cases where specific markers for the type of cancer happen to be available.

In the light of the potential of cellular therapy and lack of validated targets the identification of promising TCR antigens remains one of the most pressing bottlenecks of TCR-based immunotherapy, particularly in the effort to treat cancer.

### Obtaining full-length TCR ORFs

Primarily due to the diversity of usage in V gene segments in naturally occurring TCRs, it is challenging to capture sequence data of paired TCR chains from pools of T-cells. In general, in order to reliably capture sequence with high reliability it is necessary to amplify TCR using reverse transcription and/or PCR methods, using a pool of primers that cover the full V region diversity of the target pool. This process generates ORF fragments from which sequence data may be obtained, however, make unsuitable starting material for readily cloning full-length TCR ORFs. Therefore, high-efficiency strategies for mining paired sequence data from pools of T-cells is generally incompatible with cloning of full-length TCR ORFs for downstream applications. Time consuming and expensive synthesis of TCR ORFs is thus required for any functional testing or application of the sequenced TCR pairs.

### Diversification of full-length TCR ORFs

The emerging therapeutic use of TCRs has seen a rise in interest to diversify TCR sequences within affinity and/or functional maturation workflows to derive synthetic TCR pairs of defined specificity and function. Generally, this has been achieved through linking TCR chain pairs into a single-chain construct for phage-display methodologies, and introduction of sequence diversity into these single chain constructs. There is currently a lack of technologies that can rapidly diversify single TCR ORFs in a systematic manner, and where these full-length TCR ORFs are applicable to immediate testing and/or selection in a context of surface expression on viable mammalian cells. Such a technology would be of great value in the maturation of TCR pairs, with highly defined specificity and signalling capacity, for therapeutic use.

### Technological aspects of TCR and T-cell antigen analyses

Overall, the development of TCR-based therapies is still in its early stages, and success has been limited. Diagnostic approaches, while of immense potential, have seldom been deployed into controlled clinical studies that aim to assess patient disease states or response to therapy. Underdeveloped techniques for the reliable capture of native TCR chain pairs, and the systematic analysis of TCR-antigen interactions at high-throughput and in a functional context of cell-cell communication, have been the main hurdles to the development of TCR-based therapies and diagnostics.

Deep sequencing approaches have led to an improved understanding of T-cell receptor diversity in heath and disease. However, these approaches have generally focused on short stretches spanning the CDR3 regions, mainly of the TCR β-chain. Most studies have ignored the contribution of the TCR α-chain, and few have sought to analyse paired αβ chains as well as the antigen specificity of TCRs determined to be of interest. Recent workflows using single cell encapsulation and genetic barcoding has enabled the pairing of native TCR αβ or γδ chain pairs and analysis of full-length sequences, however, such workflows remain experimental.

Isolated TCR chain pairs may be analysed in terms of antigen specificity in either biophysical or functional modes. Biophysical analysis requires the recombinant production of both the TCR as well as the analyte antigen in soluble form. In the case of HLA-restricted TCRs this would thus require the generation of all individual TCRs as well as the cognate pHLA complexes. This is technically highly challenging, slow and very lowthroughput. Furthermore, such analysis would only provide interaction affinities, which are not well-correlated with functional characteristics in predictable ways.

Until recently, the detailed functional analysis of isolated TCR sequences in a cellular context has been limited to laborious protocols of transfection of analyte TCR chain pairs into primary T-cells or immortal T-cell lines, and detection of cellular responses by traditional flow cytometric analysis of cell activation, or detection of secreted factors from the transfected cells upon antigen challenge. In a recent publication by Guo et al, rapid cloning, expression, and functional characterization of paired TCR chains from single-cells was reported (Molecular Therapy - Methods and clinical development (2016) 3:15054). In this study, analyte human αβ TCR pairs were expressed in a reporter cell line that lacked αβ TCR expression, and which contained a green fluorescent protein (GFP) reporter system linked to the Nur77 promoter that is activated upon TCR stimulation. This system remains inefficient due to the lack of standardised TCR integration into the reporter cell line genome, and does not provide a systematic manner for cell-bound antigen challenge by an APC element.

Similar to workflows for identification of TCRs against known T-cell antigens, the de novo discovery of novel T-cell antigens in health and disease remains highly challenging. Most approaches remain biophysical in nature, and aim to produce candidate antigens that may be tested in immunisation protocols, or through identifying cognate TCRs as addressed above. Little or no standardisation exists in the field of T-cell antigen discovery, and the field is largely restricted to academic study.

With the accumulating interest in TCRs and their cognate antigens in both therapeutic and diagnostic use, and the emergence of means to capture significant numbers of native TCR αβ and γδ chain pairs, there remains a lack of reliable high-throughput and standardised technologies for the systematic analysis of TCR-antigen interactions. Importantly, there is a lack of standardised systems for rapid reconstitution and/or systematic diversification of full-length TCR ORFs, such that these ORFs may be directly applied to functional analysis of TCR chain pairs in the native context of an analyte TCR being presented on the surface of a viable cell in a native context. Such capability is important for achieving high-throughput analyses of native TCR chain pairs, but also affinity and/or functional maturation of TCR chain pairs, for therapeutic and diagnostic uses.

There is a clear need for rapid and systematic methods for TCR chain reconstitution, and their systematic diversification, in high-throughput methods that will enable the use of TCR diagnostics on an informatics and reagent basis, and also personalised TCR-based immunotherapies.

### Detailed description of the invention

The present invention addresses the above-mentioned needs. The present invention provides in a first aspect, a two-component vector system comprising pre-assembled libraries consisting of vectors harbouring variable (V), joining (J) and constant (C) sequences for TCR chains. The first component of such a system comprises a V-C entry vector containing V and C sequences. The second component of the system comprises a J donor vector containing J sequence. The two-component vector system is pre-assembled into libraries of V-C entry vectors and J donor vectors with all desirable V-C sequence combinations and J sequences, respectively. The two-component vector system is designed in such a manner that a single V-C entry vector and a single J donor vector with desired sequences can be combined with a short DNA oligonucleotide duplex encoding CDR3 (odeCDR3) sequence to reconstitute a full-length TCR ORF in vitro, in a single-tube reaction in a restriction enzyme and ligase dependent and PCR independent manner. In addition, the modular two-component system is ideally suited to rapidly generate large libraries of synthetic or mutant full-length TCR ORFs for affinity or functional maturation workflows. In a second aspect, modified V-C entry vectors for a reciprocal pair of TCR chains (i.e. TRA/TRB or TRD/TRG) are compiled into a 5-component vector system to provide a system in which reconstituted TCR chain pairs may be adjoined within a single vector. This second system utilises both modified V-C entry vectors, the same J donor vectors as the first system, and also a Bidirectional Terminator Donor Vector (BiT Donor) as to achieve adjoined TCR chain pairs encoded in antiparallel sense orientation in a final construct, with each TCR chain interposing a 'bidirectional terminator' element.

### TCR ORF Reconstitution and engineering System (TORES)

The present invention first provides a two-component vector system with unique characteristics suitable for the above-mentioned uses. This TCR ORF reconstitution and engineering system (TORES) is used in conjunction with a third component, an oligonucleotide duplex encoding CDR3 (odeCDR3), to de novo assemble full-length TCR ORFs within a defined vector context and/or generate formulaic sequence diversity within a given TCR ORF.

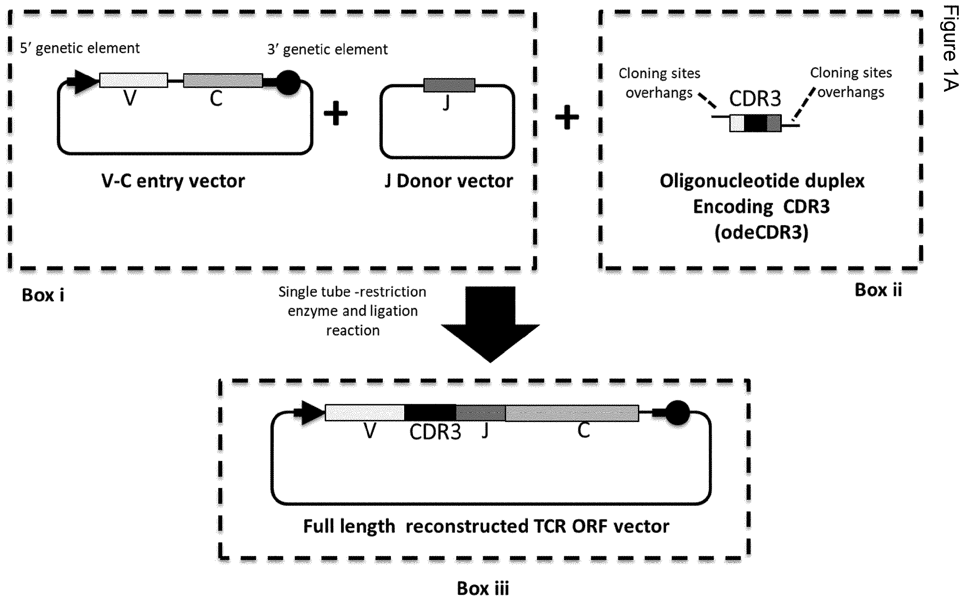

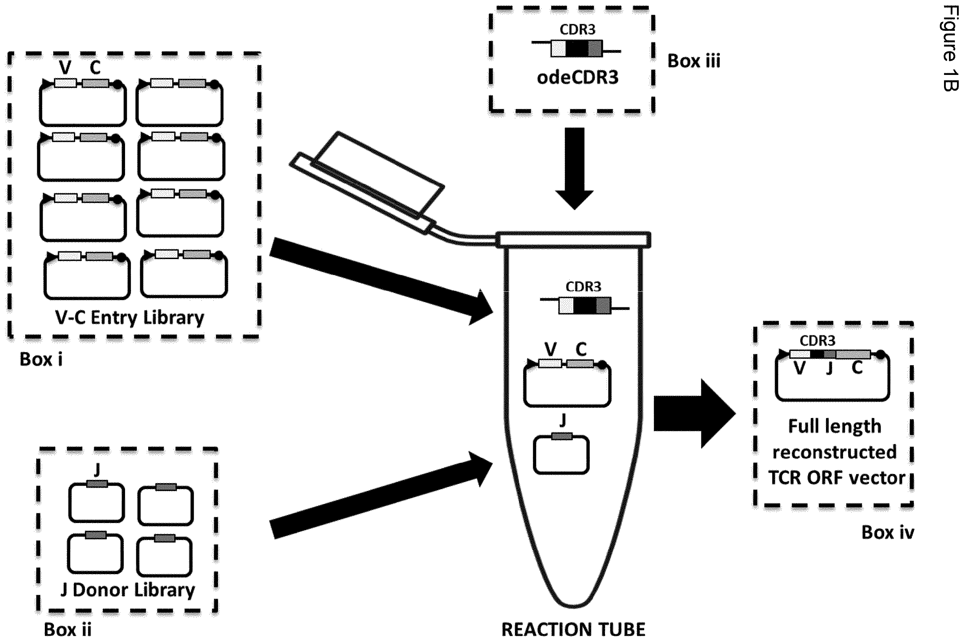

The present invention is summarised in Figure 1A. A selected V-C entry vector containing V and C TCR gene segments required for a target full-length TCR ORF is combined with a J donor vector that contains the required J TCR gene segment. The full-length TCR ORF is completed by the addition of an oligonucleotide duplex encoding CDR3 (odeCDR3), which accounts for unfixed non-germline sequence generated during V(D)J recombination and interposed by fixed germline encoded V and J sequence encoded by the V-C entry vector and J donor vector, respectively. The two-component vector system, and the third odeCDR3 component, is designed such that when combined into a restriction enzyme and ligase cycle reaction, the full V-CDR3-J-C TCR ORF is reconstituted. This is achieved via a Type IIS restriction enzyme(s) that are used to perform 'scarless' assembly of the genetic elements in a standardised manner. The two-component vector system is assembled into a library containing all required V, C and J gene segments for reconstitution of target full-length TCR ORFs (Figure 1B). For instance, a library can be constructed to contain all gene segments encoding native protein sequences of the human TRA repertoire as described in Examples 1 and 2, and the human TRB repertoire as described in Example 3.

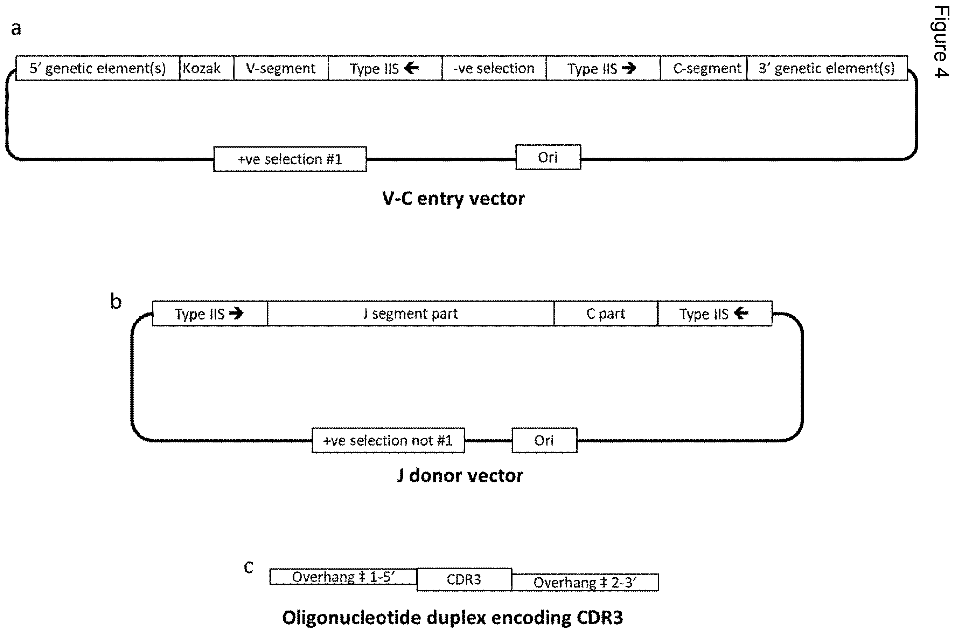

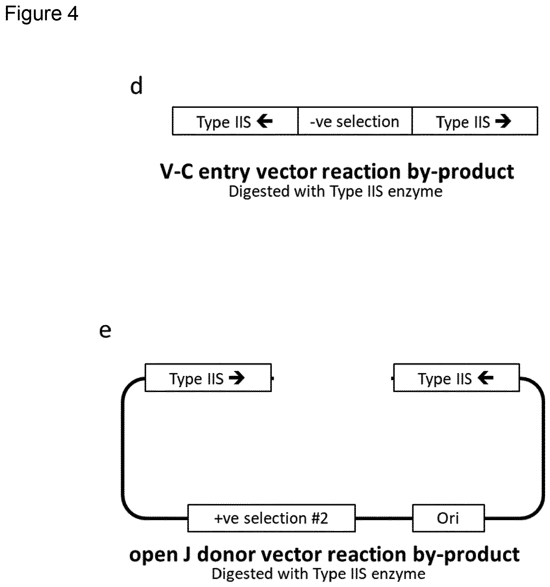

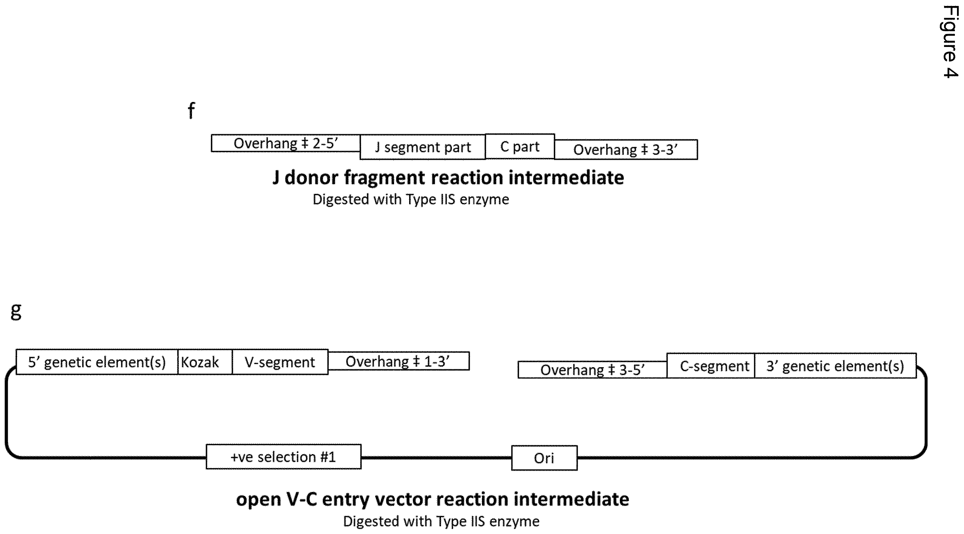

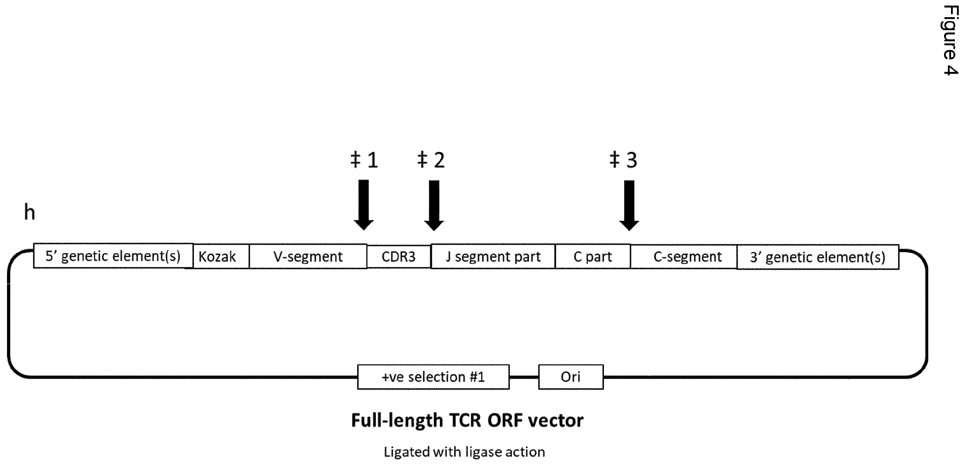

To reconstitute a full-length TCR ORF, from sequence information that is sufficient to define V, J and C gene segment usage, along with unfixed CDR3 sequence interposed by fixed V and J segments, the V-C entry vector and J donor vector that correspond to the V/C and J usage of the target TCR ORF are first selected. An odeCDR3 corresponding the unfixed CDR3 sequence that is needed to complete the full-length TCR ORF is also generated. These three components are combined with a Type IIS restriction enzyme and DNA ligase enzyme in a cycle reaction to generate the target full-length TCR ORF as described in Figure 4 and Example 7. The resulting reconstituted full-length TCR is contained within the V-C entry vector backbone, thus contains all vector features contained within this parent construct.

The action of the Type IIS restriction enzyme of the three combined components (Figure 4 a, b, c) within a restriction enzyme / ligase cycle reaction, results in two reaction by-products and two reaction intermediates. The V-C entry vector derived reaction by-product is the excised native selection marker and Type IIS binding sites (Figure 4d). The J donor vector backbone from which the J segment part has been excised represents a second reaction by-product (Figure 4e). The excised J segment part from the J donor vector represents a reaction intermediate, and contains both a J segment part, a small C part from the C segment, and single stranded overhangs required for ligation (Figure 4f). The second reaction intermediate is the parental V-C entry backbone containing the V and C segments, and single stranded overhangs required for ligation (Figure 4g). The final product of reaction represents a full-length TCR ORF reconstituted within the parental V-C entry vector backbone, comprised of ligation of the odeCDR3 (Figure 4c), the excised J segment part (Figure 4f) and the V-C entry backbone carrying the V and C gene segments (Figure 4g).

### The V-C entry vector and J donor vector components

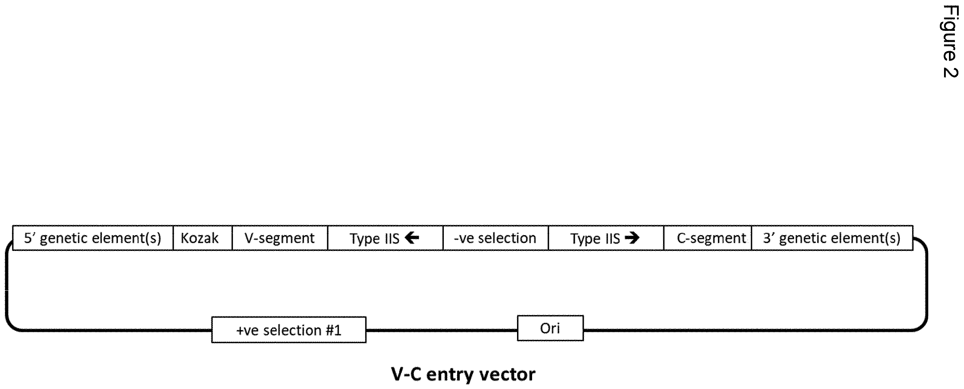

In the present context, a combined two-component system includes one or more V-C entry vector/s containing
a. origin of replication,
b. a first positive selection marker,
c. 5' genetic element, or elements,
d. Kozak Sequence,
e. TCR variable gene segment,
f. a first Type IIS sequence, for site specific recognition and cleavage by a Type IIS restriction enzyme,
g. a negative selection marker,
h. a second Type IIS sequence,
i. TCR constant gene segment, and
j. 3' genetic element, or elements
wherein, a) and b) are used for propagation and selection of both parental V-C entry vector and the reconstituted TCR-containing vector in a bacterial host; c) and j) are used to define the downstream application of the reconstituted full-length TCR ORF; d) ensures efficient initiation of translation in eukaryotic cells, which could alternatively represent a Shine-Dalgarno sequence for transitional regulation in prokaryotes and archaea; e) represents the variable (V) gene segment from the start codon to a motif at the 5' edge of the CDR3 region conserved across all V segments in a given two-component vector system; f) represents a Type IIS recognition sequence that directs a Type IIS restriction enzyme to cut in the 5' direction as to create a standardised single stranded overhang at the 3' end of the V gene segment; g) represents a negative selection marker to eliminate parental V-C entry vector during operation of the system to reconstitute a full-length TCR ORF; h) represents a Type IIS recognition sequence that directs a Type IIS restriction enzyme to cut in the 3' direction as to create a standardised single stranded overhang at the 5' end of the C gene segment; i) represents the constant (C) gene segment from a motif at the 5' end of the C gene segment conserved across all C segments in a given two-component vector system, and which defines the boundary with the J segment (see Figures 2 and 4).

A V-C entry vector that is used for genetic reconstitution of the full-length TCR ORF without the need for downstream biological application, for example, to be used as a template for molecular biology workflows, the minimal V-C entry vector would comprise elements a), b), e), f), h) and i), lacking regulatory elements.

A V-C entry vector can also contain one or more transcriptional units for the expression of additional ORFs suitable for downstream applications, for example, a mammalian antibiotic resistance gene or reporter construct.

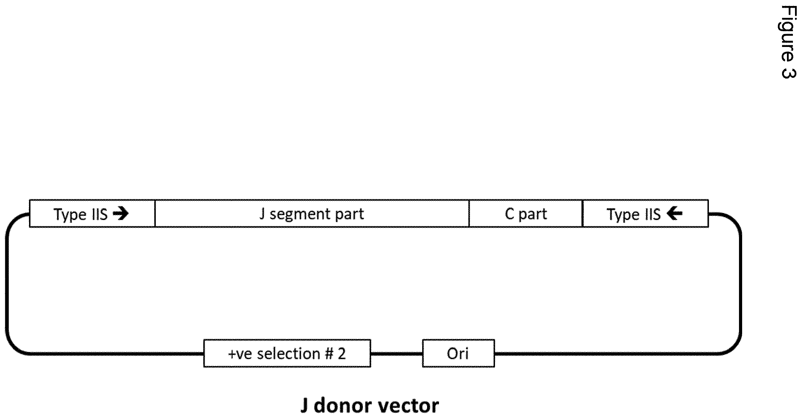

The combined two-component system includes one or more J-donor vector containing
a. origin of replication,
b. a second positive selection marker,
c. a third Type IIS sequence,
d. TCR Joining gene segment,
e. a C part, corresponding to a small 5' portion of a constant gene segment, and
f. a fourth Type IIS sequence.
wherein, a) and b) are used for propagation and selection of the J donor vector; c) represents a Type IIS recognition sequence that directs a Type IIS restriction enzyme to cut in the 3' direction as to create a standardised single stranded overhang at the 5' end of the J gene segment; d) represents the Joining (J) gene segment starting from a 5' from the motif defining the 3' edge of the CDR3 region conserved across all J segments in a given two-component vector system, to a 3' sequence that incorporates C part, representing a 5' portion of the C segment encoded by V-C entry vector(s) contained within the two-component system; represents a Type IIS recognition sequence that directs a Type IIS restriction enzyme to cut in the 5' direction as to create a standardised single stranded overhang at the 3' end of the J gene segment, and contained within the C part portion of the sequence (see figures 3 and 4).

A J-donor vector does not strictly need to carry a C part sequence, encoding a small 5' portion of the C gene segment. This C part is used to optimise and standardise overhangs for the reconstitution reaction during operation of a TORES. This is because of the higher sequence variation found at the 3' end of J gene segments, such that inclusion of a C part allows standardisation by generation of overhangs within the less diverse C gene segment. In the instance of constructing a TORES for a TCR loci from other organism that does not have 3' J segment diversity, or using synthetic J gene segments, this C-part may be omitted in favour of standardisation of overhangs within said J segments. This would reduce the complexity of the J donor library construction.

Each of first, second, third and fourth Type IIS sequences may be the same or different. Preferably, they are the same. This ensures that each of the restriction sites within the two-component vector system is compatible with the same Type IIS enzyme, and only a single enzyme is needed for the restriction enzyme / ligase cycle reaction during reconstitution of full-length TCR ORF using the system. Type IIS enzymes do not cut within their recognition sequence, and thus the single-stranded overhangs are generated extrinsic to the recognition sequence. Therefore, the nature of the overhang generated upon Type IIS restriction enzyme action is dependent on both the orientation of the recognition sequence, and indeed the adjacent sequence (see examples 1 to 4).

Alternatively, each of the Type IIS restriction sequences may be different from one another. However, with the addition of each unique recognition sequence, an additional Type IIS enzyme must be incorporated into the restriction enzyme / ligase cycle reaction. This would increase the complexity and cost of a reconstitution reaction for assembling a full-length TCR ORF.

The first and second positive selection markers within the V-C entry vector and J donor vector, respectively, are normally different. This is to ensure that the V-C entry vector, which provides the backbone of the final full-length TCR ORF product, can be selected for independently of the J donor vector, and thus eliminate transformants that carry undigested or re-circularised J donor vectors that would otherwise contribute background to the reconstitution reaction (see Figures 2 and 3 and Example 7).

The positive selection markers can be selected from
a. an antibiotic resistance gene,
b. an auxotroph complementing gene,
c. a reporter gene
wherein the choice, formatting and application of such positive selection markers are well known to those skilled in the art.

The 5' genetic element incorporated into a V-C entry vector comprises one or more elements selected from
a. gene cis/acting element,
b. heterospecific recognition site for recombinase enzymes,
c. a 5' homologous recombination arm for a genomic site of interest'
d. a mRNA splice acceptor site,
e. an internal ribosomal entry site, and
f. epigenetic insulator sequence
wherein, a) drives expression of the transcript encoded by the full-length TCR ORF product reconstituted within the V-C entry vector backbone; b) represents a sequence that directs site-directed recombination in the presence of recombinase enzymes to insert the full-length TCR ORF product reconstitute within the V-C entry vector backbone into a specific genetic context; c) represents a sequence that directs site-directed homologous recombination to insert the full-length TCR ORF product reconstituted within the V-C entry vector backbone into a specific genetic context; d) permits engineered domain-fusion approaches to manipulate the form of the protein expressed from the full-length TCR ORF reconstituted in the V-C entry vector backbone e) permits cap-independent initiation of translation of the mRNA expressed from the full-length TCR ORF reconstituted in the V-C entry vector backbone f) permits insulation of transcriptional activity otherwise affected by enhancer elements in a genomic context of where the full-length TCR ORF reconstituted in the V-C entry vector backbone may be inserted.

A cis/acting element may be used to drive transient expression of TCRs reconstituted into a V-C entry vector backbone provided in Examples 1 and 3, when said vector containing a reconstituted TCR ORF is transfected into mammalian cells.

A heterospecific recognition site for recombinase enzymes may be used to permit recombinase mediated cassette exchange of TCRs reconstituted into a V-C entry vector backbone provided in Example 4, when said vector containing a reconstituted TCR ORF is transfected into mammalian cells in the presence of appropriate recombinase enzyme.

A first Type IIS recognition sequence that is included in the V-C entry vector is oriented to cleave 5' of said recognition sequence and within the TCR variable gene segment (Figure 4a) to produce a single-stranded DNA overhang at the 3' end of the variable gene segment (Figure 4g) that is complementary to that at the 5' end of the synthesised odeCDR3 (Figure 4c) For details on how this first Type IIS recognition sequence is designed, see Examples 1, 3, 5 and 6.

A V-C entry vector contains a negative selection marker between the first Type IIS recognition sequence, and the second Type IIS recognition sequence (infra vide, Figure 2). This negative selection marker is selected from
a. a restriction enzyme recognition site not contained elsewhere in the first component or within the TCR joining gene segment,
b. a bacterial suicide gene, and
c. a reporter element.
wherein, the negative selection marker is used to eliminate host cells transformed with parental V-C entry vector, and thus reduce the background of a reconstitution reaction when using the first positive selection marker to select for transformants containing the target TCR ORF within the V-C entry vector backbone (see Example 7).

With the exception of the negative selection marker itself, all other sequences in the two-part system must be devoid of said sequence as to not confer undue negative selection on the basis of the inclusion of this sequence elsewhere in the system.

In the present context, a second Type IIS recognition sequence that is included in the V-C entry vector is orientated to cleave 3' of said recognition sequence and within the TCR constant gene segment (Figure 4a) to produce a single-stranded DNA overhang at the 5' end of the constant gene segment (Figure 4g) that is complementary to that at the 3' end of the J donor fragment reaction intermediate (Figure 4f). For details on how this second Type IIS recognition sequence is designed, see Examples 1, 2, 3 and 5.

The 3' genetic element incorporated into a V-C entry vector comprises one or more elements selected from
a. a terminator element,
b. heterospecific recognition site for recombinase enzymes,
c. a 3' homologous recombination arm for a genomic site of interest,
d. a mRNA splice donor site,
e. an internal ribosomal entry site, and
f. epigenetic insulator sequence.
wherein a) represents a sequence that directs transcriptional termination for effective mRNA production of the TCR ORF in situ and may encode a poly-A signal; b) represents a sequence that directs site-directed homologous recombination to insert the full-length TCR ORF product reconstituted within the V-C entry vector backbone into a specific genetic context; c) permits the fusion of a TCR ORF to a transcriptional unit after integration into a genomic locus encoding an downstream mRNA splice acceptor site to manipulate the strength of TCR expression levels or form of the protein expressed from the full-length TCR ORF reconstituted in the V-C entry vector backbone e) permits cap-independent initiation of translation of the mRNA expressed from the full-length TCR ORF reconstituted in the V-C entry vector backbone f) prevent inappropriate interaction between adjacent chromatin domains, thus insulating the full-length TCR ORF from adjacent transcriptional regulation or spread of heterochromatin in a genomic context of where the reconstituted TCR ORF in the V-C entry vector backbone may be inserted

A terminator element is used to ensure transcriptional termination during expression of TCRs reconstituted into a V-C entry vector backbone provided in Examples 1 and 3, when said vector containing a reconstituted TCR ORF is a transfected into mammalian cells.

A heterospecific recognition site for recombinase enzymes is used to permit recombinase mediated cassette exchange of TCRs reconstituted into V-C entry vector backbones provided in Examples 4, when said vector containing a reconstituted TCR ORF is transfected into mammalian cells in the presence of appropriate recombinase enzyme.

A J donor vector contains a J gene segment with a C-part sequence, representing a 5' fragment of the C gene segment, to the 3' of the J gene segment (Figure 3).

The C-part sequence is designed to standardise the single stranded overhangs generated by Type IIS enzyme action within the at the 3' end of the J donor vector-derived J fragment reaction intermediate (Figure 4f), and that at the 5' end of the C gene segment within the Type IIS digested open V-C entry vector reaction intermediate (Figure 4g).

A third Type IIS recognition sequence that is included in the J donor vector is oriented to cleave 3' of said recognition sequence and within the TCR joining gene segment (Figure 4b) to produce a single-stranded DNA overhang at the 5' end of the joining gene segment (Figure 4f) that is complementary to that at the 5' end of the synthesised odeCDR3 (Figure 4c) For details on how this third Type IIS recognition sequence is designed, see Examples 2, 3, 5 and 6.

A fourth Type IIS recognition sequence that is included in the J donor vector is orientated to cleave 5' of said recognition sequence and within the TCR C-part (Figure 4b) to produce a single-stranded DNA overhang at the 3' end of the C-part (Figure 4f) that is complementary to that at the 5' Type IIS digested open V-C entry vector reaction intermediate (Figure 4g). For details on how this third Type IIS recognition sequence is designed, see Examples 1, 2, 3, 5 and 6.

The two-part vector system, all encoded TCR gene segments and parts should not contain Type IIS recognition sequences that are used for operation, or assembly, of the V-C entry vector or J donor vector. Inclusion of such sequences would result in Type IIS restriction enzyme action within the encoded gene segments or parts, and result in disruption of the TCR reconstitution process. Similarly, the Type IIS recognition sequences should not be included in the vector backbones, or in any 5' and 3' genetic elements within these vectors, nor the cloning fragments used to assemble the two-part vector system, nor the odeCDR3 representing a third system component (*infra vide*)*.*

A two-component vector system of the TORES may be constructed for any collection of TCR chains. In examples 1 to 4 below, two-component vector systems are constructed for the human TRA and TRB loci, encoding the human TCR alpha and beta chains, respectively. The construction of such a TORES is equally applicable in the context of the TRD and TRG loci, encoding the TCR delta and gamma chain pair, respectively, or indeed for any TRA/TRB, TRD/TRG or variant TCR chain pair system found in jawed vertebrates.

### The third odeCDR3 component

To reconstitute a full-length TCR ORF using any given TORES, a small ORF fragment not encoded by the two-component V-C entry vector and J donor vector system is required as a third component. This third component takes the form of an oligonucleotide duplex encoding CDR3 (odeCDR3).

Such a third component, odeCDR3, comprises
a. a first single strand overhang sequence complimentary to first Type IIS restriction enzyme recognition and cleavage site orientated to cleave 5' of the recognition sequence and within the TCR variable gene segment of the V-C entry vector,
b. a double strand segment encoding a TCR CDR3 region and devoid of negative selection element, which negative selection element is as defined in item 10, and also devoid of any Type IIS restriction sequences of the first or second part, and
c. a second single strand overhang sequence complimentary to the third Type IIS restriction enzyme recognition and cleavage site orientated to cleave 3' of the recognition sequence and within the TCR joining gene segment of the J donor vector.

Alternatively, the odeCDR3 can be comprised of a dsDNA molecule and/or plasmid DNA encoding the CDR3 flanked by two Type IIS enzymes consistent with the first (V-C entry vector) or second (J donor vector) component, oriented such that when digested a product comprising of a, b and c described previously is generated, and two by-products encoding short dsDNA fragments flanked by the Type IIS sites. This alternative dsDNA odeCDR3 is compatible the restriction enzyme / ligase reaction, not necessarily requiring prior digestion or processing.

As an alternative to the use of V-C entry vector and J donor vector configuration in a TORES, J-C entry vector and V donor vector configuration may also be used by applying the same conceptual framework.

### Methods to use a TORES to reconstitute full-length TCR ORFs

A TORES can be used to reconstitute a full-length TCR ORF in a genetic vector context, from sequence information, as is presented for a human TRA/TRB chain pair in Example 7.

To operate a TORES to reconstitute a full-length TCR ORF from sequence information, given the resource of a two-component vector system for a given TCR chain, the method comprises
a. selecting a V-C entry vector,
b. selecting a J donor vector,
c. selecting an odeCDR3,
d. combining a, b and c to react with i) Type IIS restriction enzyme(s) to cleave all Type IIS restriction enzyme recognition and cleavage sites present in the V-C entry vector and in the J donor vector and ii) DNA ligase enzyme and iii) subjecting the combined mix to a thermocycling reaction,
e. transforming the reaction product obtained from step d to a selectable host organism competent for DNA vector propagation, and
f. performing a selection of host organism to obtain full length reconstituted TCR open reading frame in the V-C entry vector backbone.
wherein, a) and b) are selected on the basis of the selected vector encoding the V,J and C gene segments in the target full-length TCR ORF; c) is selected on the basis of completing the full-length TCR ORF sequence not encoded by the V-C entry or J donor vectors selected in a) and b), and bounded by the Variable and Joining segments encoded therein; d) combining the three selected components into a reaction mixture along with a restriction enzyme that will cut the first, second, third and fourth Type IIS restriction enzyme recognition sequences within the V-C entry and J donor vectors; e) generally represents transformation-competent bacteria; f) selection of host is on the basis of the first positive selection marker provided by the V-C entry vector backbone.

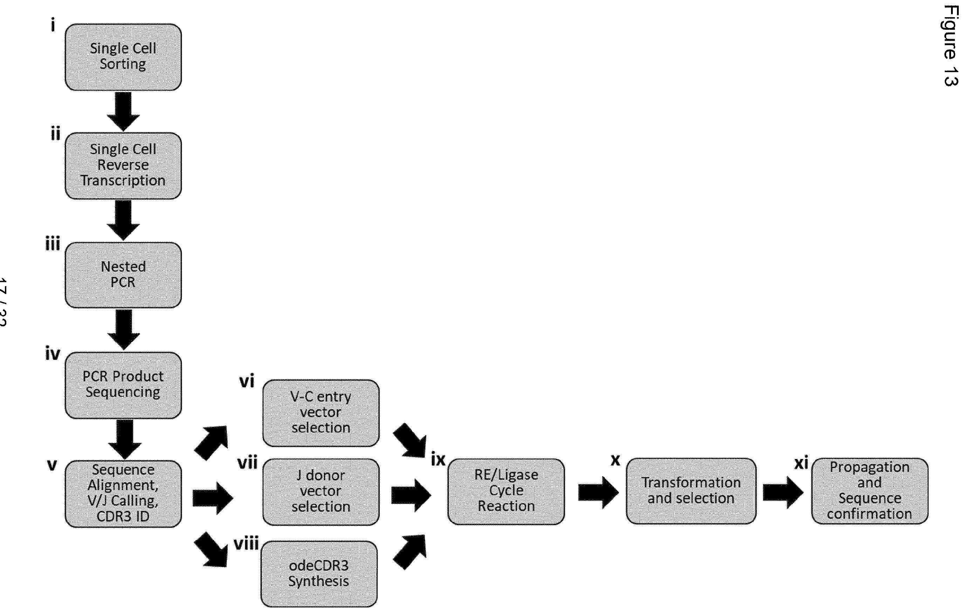

Generally, a workflow to select and define the genetic elements of a full-length TCR ORF for reconstitution entails de novo sequencing of TCR chains from target organism tissues. Example 8 below presents the *de* novo identification of a set of TRA/TRB chain pairs specific for a HCMV antigen in a HLA-B*07:02 restricted context. The workflow described in Figure 13, incorporates reverse transcription and PCR based amplification of TCR chain pairs from sorted single cells with subsequent Sanger sequencing. There exists a requirement for high-quality sequence information spanning V, CDR3, J and C segments of the TCR ORF, which dictates the specific sequencing approach(es) taken.

A method for selecting and reconstituting a TCR open reading frame thus comprises
a. Obtaining a TCR open reading frame sequence wherein said sequence information is sufficient to identify i) variable gene segment usage ii) constant gene segment usage iii) joining gene segment usage iv) a full CDR3 sequence spanning the variable gene segment border to the joining gene segment border, and
b. selecting a V-C entry vector corresponding to the variable and constant gene segments identified in step a. i) and a. ii), respectively, and
c. selecting a J donor vector corresponding to the joining gene segment identified in step a, iii), and
d. generating an odeCDR3 corresponding to CDR3 sequence identified in step a. iv), and
e. combining b, c and d to react with i) Type IIS restriction enzyme(s) to cleave all Type IIS restriction enzyme recognition and cleavage sites present in the V-C entry vector and in the J donor vector and ii) DNA ligase enzyme, iii) subjecting the combined mix to a thermocycling reaction, and
f. transforming the reaction product obtained from step e. to a selectable host organism competent for plasmid replication, and
g. performing a selection of host organism to obtain full length reconstituted TCR open reading frame in the V-C entry vector backbone.
wherein, a) is conducted by sequencing methods well known to one skilled in the art, able to obtain sufficient sequence length and quality to identify all four required genetic elements; b) and c) are selected from a TORES library containing required vectors; d) is synthesised de novo or selected from an odeCDR3 library; e) is conducted in a single reaction vessel.

In order to select the appropriate V-C entry vector, J donor vector and odeCDR3, target TCR sequences were aligned against a library of V, C and J gene segments for their corresponding TCR chains to determine the V, C and J segment usage of the target chain. This sequence alignment and analysis step must also permit the definition of the CDR3 coding sequence, and thus the definition of odeCDR3 sequence. Thus, overall such sequence analysis permits the selection of V-C entry vectors and J donor vectors for TCR chain reconstitution. The analysis also permits the synthesis of odeCDR3 for each chain reconstitution reaction. This process is well described in Example 8 and summarised as part of Figure 13.

It is desirable to conduct the Type IIS digestion and DNA ligase-dependent ligation (step e) in a single cycle reaction. This minimises processing steps and is made possible by the design of the system, with Type IIS restriction enzymes cutting outside their recognitions sequences, such that a number of unique overhangs may be generated with a single enzyme, thus maintaining efficient directional cloning of the J donor vector reaction intermediate and odeCDR3 into the V-C entry vector backbone.

Alternatively, the Type IIS restriction digest and DNA ligation may be performed in sequential procedures.

In Example 8, the application of the TORES is exemplified in the context of single-cell fluorescence-activated cell sorting (FACS) of antigen-specific CD8 T-cells from human peripheral blood for reverse transcription and PCR based amplification of TRA/TRB TCR chain pairs, followed by Sanger sequencing. This is a generally applicable workflow, wherein any tissue may be the source of T-cells from any jawed vertebrate, and cells may be sorted based on any phenotypic characteristic. Importantly, the singlesorted cells need not be stained for antigen specificity using HLA-multimer reagents.

The TCR sequencing approach used is not restricted to any particular method or technology, provided sufficient high-quality sequence information is obtained such that the above-defined genetic characteristics of the TCR ORF(s) can be defined based on said sequence information.

The use of FACS for partitioning single cells such that native TCR chain pairs may be sequenced and identified is a powerful method due to the accurate and rich phenotypic information that may be collected with multi-specificity antibody panels. However, other methods exist to partition cells, including; emulsion PCR; digital PCR approaches using microfluidic cell encapsulation, digital PCR using physical partitioning substrates.

It is generally desirable to obtain native TCR pairs from a source material, as both chains of a TCR pair contribute to HLA-antigen engagement and recognition. However, there are instances where recovery of just a single chain may be desirable, such as high-throughput screening of a single chain against a set specificity. In such a case, TCRs may be amplified and/or sequenced from non-partitioned cells.

### Methods to use a TORES to generate full-length TCR ORFs with diversified sequence

A TORES system is ideally suited to generate diversified full-length TCR ORFs in several systematic modes. Such systematic diversification may be applied to affinity and/or functional maturation workflows for TCR chains. Such diversification of target TCR chain sequences is well described in Examples 9 and 10.

Such TCR ORF sequence diversification methods follow the same general scheme as for a reconstitution reaction. Diversification can be conducted in multiple parallel reconstitution reactions, whereby a single variant TCR ORF is generated per reaction. However, in most scenarios it is desirable to generate a pool of variant TCR ORFs in a single reaction. Each of these approaches is achieved by providing multiple variants of one or more of each genetic component(s) (V-C entry vector, J donor vector, odeCDR3) to a reconstitution reaction.

As described in Example 9, a TCR ORF can be systematically diversified at the CDR3 region by adding a pool of odeCDR3 with defined positional sequence diversity.

A method for selecting and reconstituting a TCR open reading frame to achieve TCR ORF diversity in the CDR3 region, thus comprises
a. Obtaining a TCR open reading frame sequence wherein said sequence information is sufficient to identify i) variable gene segment usage ii) constant gene segment usage iii) joining gene segment usage iv) a full CDR3 sequence spanning the variable gene segment border to the joining gene segment border, and
b. selecting a V-C entry vector corresponding to the variable and constant gene segments identified in step a. i) and a. ii), respectively, and
c. selecting a J donor vector corresponding to the joining gene segment identified in step a, iii), and
d. generating two or more odeCDR3 corresponding to CDR3 sequence identified in step a. iv), with variant sequence composition, and
e. combining b, c and d to react with i) Type IIS restriction enzyme(s) to cleave all Type IIS restriction enzyme recognition and cleavage sites present in the V-C entry vector and in the J donor vector and ii) DNA ligase enzyme, iii) subjecting the combined mix to a thermocycling reaction, and
f. transforming the reaction product obtained from step e. to a selectable host organism competent for plasmid replication, and
g. performing a selection of host organism to obtain full length reconstituted TCR open reading frame in the V-C entry vector backbone,
wherein, a) is conducted by sequencing methods well known to one skilled in the art, able to obtain sufficient sequence length and quality to identify all four required genetic elements; b) and c) are selected from a TORES library containing required vectors; d) is synthesised de novo, or selected from an odeCDR3 library; e) is conducted in a single reaction vessel.

Such a method can be achieved by pooling all odeCDR3 variants to a single reaction to generate a pool of sequence-diversified but may be equally achieved by proving each odeCDR3 variant to a parallel reaction.

Variant odeCDR3 can be generated via a variety of methods well known to those skilled in the art. The selection of position and extent of odeCDR3 degeneracy/diversity can range from a single residue change at a single position, to completely degenerate sequence to the length of the odeCDR3.

As described in Example 10, a TCR ORF can be systematically diversified by maintaining the CDR3 region via provision of odeCDR3, but diversifying V, C and J segment usage by providing two or more of the V--C entry vector and/or J donor vector to the reconstitution reaction.

A method for selecting and reconstituting a TCR open reading frame with diversified V, C and/or J segment usage, thus comprises
a. Obtaining a TCR open reading frame sequence wherein said sequence information is sufficient to identify i) variable gene segment usage ii) constant gene segment usage iii) joining gene segment usage iv) a full CDR3 sequence spanning the variable gene segment border to the joining gene segment border, and
b. selecting two or more V-C entry vectors not corresponding to the variable and constant gene segments identified in step a. i) and a. ii), respectively, and
c. selecting two or more J donor vectors not corresponding to the joining gene segment identified in step a, iii), and
d. generating an odeCDR3 corresponding to CDR3 sequence identified in step a. iv), and
e. combining b, c and d to react with i) Type IIS restriction enzyme(s) to cleave all Type IIS restriction enzyme recognition and cleavage sites present in the V-C entry vector and in the J donor vector and ii) DNA ligase enzyme, iii) subjecting the combined mix to a thermocycling reaction, and
f. transforming the reaction product obtained from step e. to a selectable host organism competent for plasmid replication, and
g. performing a selection of host organism to obtain full length reconstituted TCR open reading frame in the V-C entry vector backbone.
wherein, a) is conducted by sequencing methods well known to one skilled in the art, able to obtain sufficient sequence length and quality to identify all four required genetic elements; b) and c) are selected from a TORES library containing required vectors; d) is synthesised de novo, or selected from an odeCDR3 library; e) is conducted in a single reaction vessel.

Such a method can be achieved by pooling all V-C entry vectors and/or J donor vevtor variants to a single reaction to generate a pool of sequence-diversified but may be equally achieved by proving each vector variant to a parallel reaction.

Each V-C entry and J donor vector from a given library could be selected to provide full coverage of V, C and J gene segments.

Any combination of CDR3 and V, C and J diversification describe above could be used to generate pools or libraries of diversified TCR ORFs.

This system can be used to generate entirely synthetic libraries of TCRs ORFs with full coverage of native V, C and J gene segment usage, and defined CDR3 characteristics.

### Features of a TORES with regard to Reconstitution/Diversification Methods

As mentioned above, it is desirable to conduct the assembly cycle reaction with a single Type IIS restriction enzyme. This economises the use of restriction enzyme and is made possible by the nature of Type IIS action, and the design of unique single stranded overhangs in the two-component vector system and odeCDR3.

Alternatively, up to four Type IIS restriction enzyme recognition sequences across the four Type IIS recognition sites of the V-C entry vector and J donor vector.

For efficient cloning of TCR ORF products, at least one step of negative selection is performed during the assembly of a full-length TCR ORF using the TORES, selected from
a. performing restriction enzyme digest of reaction product to eliminate parental V-C entry vector
b. performing a suicide gene selection to eliminate competent hosts transformed with parental V-C entry vector, and/or
c. performing selection of host cells transformed with parental V-C entry vector by way of reporter identification.
wherein, the negative selection is used to eliminate parental V-C entry vector that have remained undigested by the Type IIS enzyme(s), or have re-ligated to the parental form after digestion.

Elimination of parental V-C entry vector is critical, considering that the V-C entry vector backbone, and thus the positive selection marker carried in this backbone, is used for positive selection of the vector containing the full-length TCR ORF reaction product.

In the present context, negative selection is performed using a restriction enzyme site has been designed within the reaction by-product excised from the V-C entry vector (Figure 4d). This negative selection procedure is described in examples 7 and 8.

Any one, or a combination of the above-mentioned negative selection methods can be employed to eliminate parental V-C entry vector from the final cloned products. Such a negative selection procedure may be omitted if the cloning efficiency is deemed high enough for efficient recovery of cloned reaction products.

The selection of the cloned full-length TCR ORF containing vectors in transformed host organism is required to obtain the final cloned product. Such selections are well-known to those skilled in the art.

A host organism represents a transformation-competent bacterium, and the selection of transformants containing the full-length TCR ORF contained in a V-C entry vector backbone comprises antibiotic selection. This entails adding antibiotic to the culture system in which the transformed cells are placed, and resistance to this antibiotic is encoded by the gene represented as the first positive selection marker in the V-C entry vector backbone.

Alternatively, removal of auxotrophic factors of the culture system in which transformats are placed can be a form of positive selection, wherein auxotrophic complementation is conferred by a gene product encoded in the V-C entry vector backbone. A combination of the above-described positive selections may be employed.

### V-C entry vector and J donor vector libraries comprising a TORES

For the efficient operation of a TORES to perform reconstitution or diversification of selected TCR ORFs, the pre-construction of a V-C entry vector and J donor vector library is required. It is from this library, which is specific for each TCR chain form that selections are made to fulfil the V/J/C usage of the target TCR ORF sequence, when complemented with the odeCDR3 sequence.

V-C entry and J donor vector libraries may be constructed to contain all germline TCR variable, constant and joining gene segments of an organism having such TCRs. Such a library may also include all V-C combinations in the V-C entry vector, as for the TRB locus specific TORES presented in Example 3, wherein the library is replicated with both Constant gene segments against each Variable segment.

A library of V-C entry and J donor vectors may contain V/J/C gene segments, such that translated amino acid sequence of the encoded protein is unmodified in relation to the protein sequence encoded by the germline gene segments.

Such a library permits change in the underlying nucleic acid sequence as to generate a library otherwise devoid of unwanted Type IIS recognition sequences, or positive and negative selection elements. Changes in the underlying nucleic acid sequence can also be used for codon optimisation, for expression reconstituted TCR chains in cells from different host organisms.

Alternatively, a library of V-C entry and J donor vectors may contain V/J/C gene segments, such that translated amino acid sequence of the encoded protein is modified in relation to the protein sequence encoded by the germline gene segments.

Such a library may be used to construct TCRs with characteristics that are optimised for different diagnostic or therapeutic uses. Changes in framework residues or regions within the V/J/C gene segments could be used to increase expression or stability in various scenarios, such as expression of TCRs as soluble reagents. Similarly, alterations in framework regions that are not involved in direct HLA-antigen contacts may be used to alter the signalling capacity of reconstituted TCRs produced by the TORES.

Affinity tags or immunogenic sequences may also be encoded within framework regions as to aid in purification and/or detection of the reconstituted TCRs in downstream applications.

V-C entry and J donor vector libraries may be assembled into kit comprising a combination of
a. one or more V-C entry vectors encoding combinations of Variable and Constant gene segments, and
b. one or more J donor vectors encoding J gene segments, and optionally
c. one or more standardised odeCDR3 with single stranded overhangs matched to V-C entry vector and J donor vector single strand overhangs as positive control odeCDR3, and optionally
d. A pre-assembled full-length TCR ORF as a reference
wherein, a) and b) cover the required genetic diversity of gene segments from a target organism, with unmodified or modified amino acid sequence relevant for the intended application; c) is used as a positive control in reconstitution reactions d) is used as a positive control in downstream applications of full-length TCR ORFs reconstituted with the V-C entry vector and J donor vector libraries provided in said kit.

### Method to construct V-C entry vectors

The assembly of V-C entry and J donor vector libraries may be achieved by a variety of molecular biology methods well known to those skilled in the art, including direct DNA synthesis of the required vectors. However, a rapid and cost-effective combinatorial approach using small gene segment-containing fragments is desirable. Such a method permits rapid cycling of V-C entry and J donor vector forms that are important for TCR engineering workflows. Similarly, a rapid expansion of a given V-C entry vector and/or J donor vector library can be used to account for single nucleotide polymorphism and other allelic differences of TCR gene segments between individuals of a given population, which may have functional significance or impact the immunogenicity of a TCR sequence in a pseudo-allogeneic therapeutic context. Systematic methods for assembling V-C entry vector and J donor vector libraries are well described in Examples 1, 2 and 3.

A method to construct a V-C entry comprises combining three DNA components selected from
a. a Variable gene segment cloning fragment
b. a Constant gene segment cloning fragment
c. a V-C entry vector backbone
wherein, a) contains the Variable gene segment; b) contains the constant gene segment; c) represent the V-C entry vector backbone into which the Variable and Constant gene fragments are assembled.

In the present context, a Variable gene segment cloning fragment comprises
a. a 5' primer bind sequence for polymerase chain reaction dependent propagation of the fragment
b. a fifth Type IIS sequence orientated to cut in the 3' direction
c. a first overhang sequence that encodes a defined single stranded overhang upon Type IIs enzyme action on the fifth Type IIs sequence in b.
d. a Kozak sequence
e. a TCR variable gene segment
f. a first Type IIS sequence
g. a 5' sequence segment of a negative selection marker
h. a sixth Type IIS sequence orientated to cut in the 5' direction such that a single stranded overhang is generated within the 5' sequence segment of the negative selection marker in g.
i. a 3' primer bind sequence for polymerase chain reaction dependent propagation of the fragment
wherein, b) and h) encode Type IIS sequences used in the assemble of the V-C entry vector; f) encodes a Type IIS sequences used in the operation of a reconstitution reaction; g) represents a fragment of the negative selection marker sequence that is completed by a complementary fragment provided in the Constant gene segment cloning fragment.

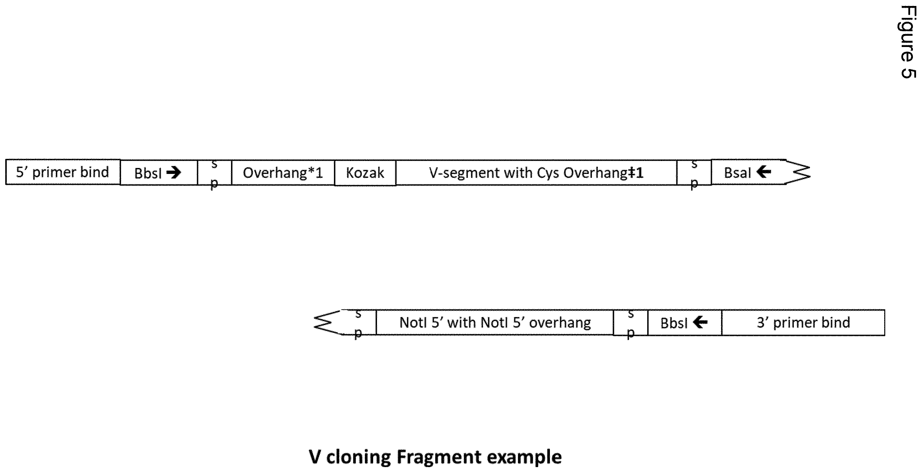

A schematic representation of the Variable gene segment cloning fragment is presented in Figure 5. Examples 1 and 3 describe the format and use of these cloning fragments to assemble V-C entry vectors for the human TRA and TRB loci, respectively.

These examples define Human TRA Variable gene segment cloning fragments as SEQ0001 to SEQ0046, and Human TRB Variable gene segment cloning fragments as SEQ0435 to SEQ0481.

To assemble a V-C entry vector, a Variable gene segment cloning fragment must be combined with a Constant gene segment cloning fragment.

A Constant gene segment cloning fragment comprises
a. a 5' primer bind sequence for polymerase chain reaction dependent propagation of the fragment
b. a seventh Type IIS sequence orientated to cut in the 3' direction such that a single stranded overhang is generated within the 3' sequence segment of the negative selection marker in c.
c. a 3' sequence segment of a negative selection marker
d. a second Type IIS sequence
e. a TCR constant gene segment
f. a second overhang sequence that encodes a defined single stranded overhang upon Type IIs enzyme action on the eighth Type IIs sequence in g.
g. an eighth Type IIS sequence orientated to cut in the 5' direction such that a single stranded overhang is generated in overhang sequence of f.
h. a 3' primer bind sequence for polymerase chain reaction dependent propagation of the fragment
wherein, b) and g) encode Type IIS sequences used in the assemble of the V-C entry vector; g) encodes a Type IIS sequences used in the operation of a reconstitution reaction; c) represents a fragment of the negative selection marker sequence that is completed by a complementary fragment provided in the Variable gene segment cloning fragment.

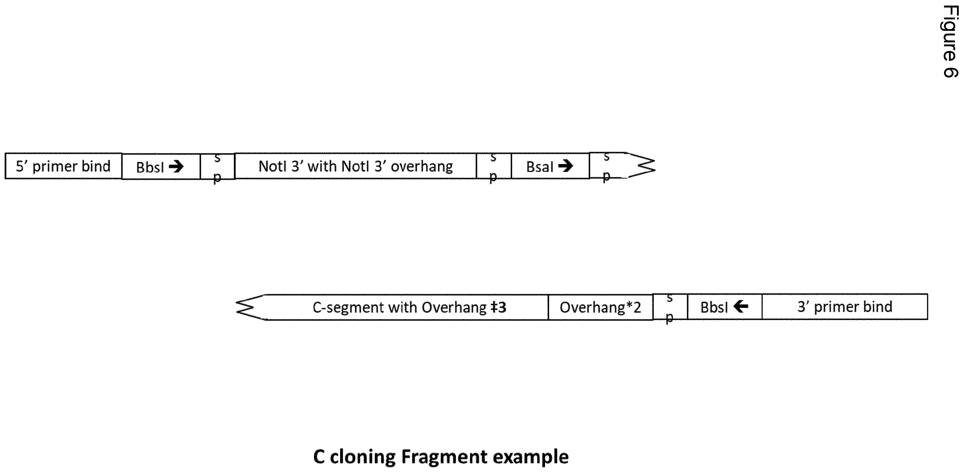

A schematic representation of the Variable gene segment cloning fragment is presented in Figure 6. Examples 1 and 3 describe the format and use of these cloning fragments to assemble V-C entry vectors for the human TRA and TRB loci, respectively.

These examples define Human TRB Constant gene segment cloning fragment as SEQ0047, and Human TRB Constant gene segment cloning fragments as SEQ0482 and SEQ0483.

The Variable and Constant gene segment cloning fragments are combined into a V-C entry vector backbone to assemble a V-C entry vector,

A V-C entry vector backbone comprises
a. an origin or replication
b. a first positive selection marker
c. a 5' genetic element
d. a first restriction enzyme recognition sequence permitting digestion of the backbone to create a single stranded overhang complimentary to the overhang created within the Variable gene segment cloning fragment by Type IIS action during the assemble reaction
e. a second restriction enzyme recognition sequence permitting digestion of the backbone to create the Constant gene segment cloning fragment by Type IIS action during the assemble reaction
f. a 3' genetic element.
wherein, c) and f) represent genetic elements used for application of reconstituted TCR ORFs in differing biological systems as mentioned above.

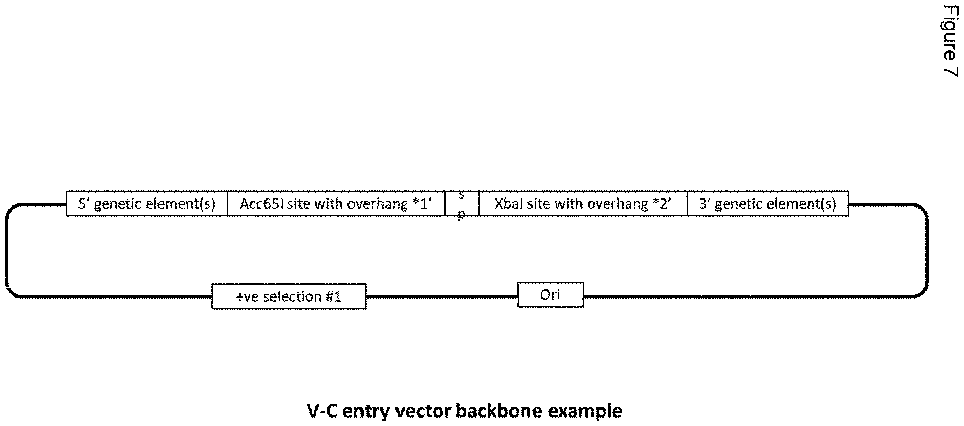

A schematic representation of the V-C entry vector backbone is presented in Figure 7. Examples 1, 3 and 4 describe the format and use of different V-C entry vector backbone forms to assemble V-C entry vectors for TRA and TRB loci, respectively.

A V-C entry vector backbone used for downstream transient expression of reconstituted TCR ORFs in mammalian cells is defined as SEQ0048, whereas a pair of V-C entry vector backbones used for recombinase mediated cassette exchange of reconstituted TCR ORFs are defined as SEQ0688 and SEQ0689,

Rapid cycling of Variable and Constant gene cloning fragments into different V-C entry vector backbones is a cost-effective approach for altering the characteristics of the vector context of reconstituted TCR ORFs from a given V/J/C combination. This permits rapid replication of re-tasking of native or synthetic TCR gene segment libraries into different biological applications.

Within the examples of Variable and Constant gene segment cloning fragments and V-C entry vector backbones cited herein, the Type IIS enzyme used for vector assembly is Bbsl, whereas the Type IIS enzyme used for TCR ORF reconstitution is Bsal. The V-C entry vector backbone contains restriction enzyme recognition sites for Acc65l and Xbal, to create compatible overhangs with the 5' overhang and 3' overhang generated in the Variable and Constant gene cloning fragments, respectively, by Bbsl action.

Any other combination of restriction enzymes could be used for assembly and reconstitution reactions, provided they satisfy the above-described criteria.

Preferably, the Type IIS sequences used for assembly of the V-C entry vector, designated the fifth, sixth, seventh and eighth Type IIS sequences above, are the same.

Alternatively, up to four different Type IIS recognition sequences could be used for this procedure.

The Type IIS sequences used for assembly of the V-C entry vector, designated the fifth, sixth, seventh and eighth Type IIS sequences above, must be different from those used for the reconstitution reaction; designated first, second third and fourth Type IIS sequences above.

The method for combining of the Variable and Constant gene segment cloning fragments with the V-C entry vector backbone to assemble a V-C entry vector is well described in examples 1 and 3.

A method for assembly of a V-C entry vector comprises
a. Digestion of the V-C entry vector backbone with the two restriction enzymes specific for the recognition sequences contained within the V-C entry vector backbone
**b.** Combining the digested V-C entry vector backbone with the V cloning fragment and the C cloning fragment, along with DNA ligase enzyme and one or more Type IIS restriction enzyme(s) recognising the fifth, sixth, seventh and eighth Type IIS sequences, and subjecting the combined mix to a thermocycling reaction, and
c. Transformation of the resulting reaction product into competent host organism and positive selection using said first positive selection marker to obtain complete V-C entry vector
wherein; a) creates single stranded overhangs complimentary with those generated in Variable and Constant cloning fragments by Type IIS enzyme action in b); b) represents the digestion of Variable and Constant fragments to generate overhangs that will ligate to the overhangs generated in a), and permit ligation of the complementary overhangs generated within the negative selection marker fragments to ligate the Variable and Constant fragments; c) represents selection and propagation of the V-C entry vector product.

### Generic features of V-C entry vectors and construction elements

The above descriptions use the human HLA TRA and TRB loci as templates for definition of a TORES system, as also outlined in Examples 1 to 4. However, a gene segment family from any TCR loci can be assembled into a TORES. In the above description, guidelines is given on where there is flexibility in the design of a TORES system, both for the human TRA/TRB loci, but also applicable to any other loci, from any organism. In the current section, generic features of a system are described using the TRA/TRB design presented in Examples 1 to 4 as a template.
To achieve a TORES for any given TCR loci, four sequence elements are required specific for a TCR chain encoded by said TCR loci:
X - a variable (V) gene segment fragment
Y - a constant (C) genes segment fragment
Y'- a constant (C) gene segment part
Z - a joining (J) gene segment fragment

According to the above description and examples below, these four forms of TCR sequence element can be assembled into various vector contexts to construct and deploy a TORES for any given V-J-C combination for any given TCR chain. For example, systems for the native human TRG and TRD locus, variant and/or synthetic TCR chain forms, or native TCR chain forms of an organism other than humans.

The V and C gene segment fragments are those assembled into a V-C entry vector via a V cloning fragment and C cloning fragment, respectively. This process is well described in examples 1 and 3.

A generic Variable gene segment cloning fragment, wherein the first Type IIS sequence encodes recognition for the enzyme Bsal, and the fifth and sixth Type IIS sequences encode recognition for the enzyme Bbsl, and the Variable gene segment cloning fragment is thus represented by the sequence SEQ0690. The encoded Variable gene segment fragment is denoted as XNₙ, wherein X is the designation for the Variable gene segment, N represents any nucleotide, and n represents the number of nucleotides in said sequence.

A generic Constant gene segment cloning fragment, wherein the first Type IIS sequence encodes recognition for the enzyme Bsal, and the seventh and eighth Type IIS sequences encode for recognition for the enzyme Bbsl, and the Constant gene segment cloning fragment is thus represented by the sequence SEQ0691. The encoded Constant gene segment fragment is denoted as YNₙ, wherein Y is the designation for the Constant gene segment, N represents any nucleotide, and n represents the number of nucleotides in said sequence.

A V-C entry vector backbone represented by sequence SEQ0048, is used to construct V-C entry vectors suitable for transient expression or reconstituted full-length TCR open reading frames in a mammalian host cell, wherein the first and second overhangs are generated by Acc65l and Xbal enzyme action of the backbone, respectively, and the 5' and 3' genetic elements are represented by constitutive promoter and polyadenylation signal, respectively.

A V-C entry vector backbone represented by sequence SEQ0688, is used to construct V-C entry vectors suitable for recombinase mediated cassette exchange with matched genetic targets with suitable heterospecific recombinase sequences, wherein the first and second overhangs are generated by Acc65l and Xbal enzyme action of the backbone, respectively, and the 5' and 3' genetic elements are represented by F14 and F15 heterospecific recombinase sequences directing flippase activity, respectively.

A V-C entry vector backbone represented by sequence SEQ0689, is used to construct V-C entry vectors suitable for recombinase mediated cassette exchange with genetic targets with matched heterospecific recombinase sequences, wherein the first and second overhangs are generated by Acc65l and Xbal enzyme action of the backbone, respectively, and the 5' and 3' genetic elements are represented by FRT and F3 heterospecific recombinase sequences directing flippase activity, respectively.

By combining the above-described generic V cloning fragment, C cloning fragment with a selected V-C entry vector backbone, Variant V-C entry vectors can be constructed for different downstream application of TCR ORFs reconstituted within these varying vector contexts.

A generic V-C entry vector constructed with the use of the V-C entry vector backbone having sequence SEQ0048, is represented by sequence SEQ0692. This resulting V-C entry vector is suitable for transient expression or reconstituted full-length TCR open reading frames in a mammalian host cell, wherein the first and second Type IIS sequences encode recognition for the enzyme Bsal, and the the Variable gene segment fragment is denoted XNₙ, and the Constant gene segment fragment is denoted YNₙ, wherein X and Y are designations for said sequences, N represents any nucleotide, and n represents the number of nucleotides in each sequence.

A generic V-C entry vector constructed with the use of the V-C entry vector backbone having sequence SEQ0688, is represented by sequence SEQ0693, containing F14 and F15 sequences suitable for recombinase mediated cassette exchange with genetic targets with matched heterospecific recombinase sequences, wherein the first and second Type IIS sequences encode recognition for the enzyme Bsal, and the the Variable gene segment fragment is denoted XNₙ, and the Constant gene segment fragment is denoted YNₙ, wherein X and Y are designations for said sequences, N represents any nucleotide, and n represents the number of nucleotides in each sequence.

A generic V-C entry vector constructed with the use of the V-C entry vector backbone having sequence SEQ0689, is represented by sequence SEQ0694, containing FRT and F3 sequences suitable for recombinase mediated cassette exchange with genetic targets with matched heterospecific recombinase sequences, wherein the first and second Type IIS sequences encode recognition for the enzyme Bsal, and the the Variable gene segment fragment is denoted XNₙ, and the Constant gene segment fragment is denoted YNₙ, wherein X and Y are designations for said sequences, N represents any nucleotide, and n represents the number of nucleotides in each sequence.

The use of pairs of V-C entry vectors with differing heterospecific recombinase sites may be used for each chain of a TCR chain pair, as presented in Example 4 for the human TRA and TRB chain pair. This means that in downstream application of TCR chains reconstituted in this paired TORES system, can be delivered into a genetic context with dual heterospecific recombinase receiver sites. For example, a cell line containing such dual heterospecific recombinase receiver sites for genomic integration of the TCR chain pair.

The above description of V-C entry vector, and the components from which they are assembled, is based on the use of Type IIS enzymes Bbsl and Bsal for construction and operation, respectively. Based on the guidance given above, one or more alternative Type IIS enzymes may be used for each of these tasks.

### Method to construct J donor vectors

As for the above-described combinatorial method for construction of V-C entry vectors, a combinatorial method may be used to construct J donor vectors. In the present method, a J donor vector is constructed in a two-step process involving the construction of an intermediate J receiving cassette vector in a first step, into which J segment parts are inserted in a second step to form the J donor vector. In this context, a J receiving cassette vector, as the J donor vector derived from it, contains a small fragment of a Constant gene segment, termed a C part. Thus a rapid combinatorial method or construction is desirable to iterate different J donor vector forms that require differential usage of Constant gene segments. This method is well described practically in Examples 2 and 3.

A method to construct J donor vector comprises combining four DNA components selected from
a. J receiving cassette fragment
b. J donor vector backbone
c. J receiving cassette vector
d. J segment part
wherein, a) contains the above-mentioned C-part and four distinct Type IIS cloning sites for vector assemble and reconstitution reaction operation; b) is the vector backbone into which one is inserted to create c); d) is the J gene segment part that is combined with c) to create a J donor vector.

A J receiving cassette fragment comprises
a. a first single stranded overhang at the 5' end complimentary to overhang sequence generated in the J donor vector backbone
b. a third Type IIS sequence orientated to cut in the 3' direction, joined with a sequence that forms a single stranded overhang when acted upon by the enzyme directed by a ninth Type IIS sequence mentioned in c.
c. a ninth Type IIs sequence orientated to cut in the 5' direction, and to create a single stranded overhang mentioned in b.
d. a negative selection marker
e. a tenth Type IIS sequence orientated to cut in the 3' direction and create a single stranded overhang at the 5' of the sequence described in f.
f. a C-part representing a 5' portion of the Constant gene fragment, with an overhang sequence at the 5' end generated by enzyme action directed by the tenth Type IIS sequence, and an overhang sequence at the 3' end generated by enzyme action directed by the fourth Type IIS sequence mentioned in g.
g. a fourth Type IIS sequence orientated to cut in the 5' direction such that a single stranded overhang is generated within the 5' sequence containing the C-part mentioned in g
h. a second single stranded overhang at the 3' end commentary to overhang sequence generated in the J donor vector backbone
wherein; a) and h) are used for directional cloning into the J donor vector backbone; b) and g) encode Type IIS sequences used in the operation of a reconstitution reaction; c) and e) encode Type IIS sequences used in the assemble of the J donor vector; d) represents a negative selection marker for elimination of parental J receiving cassette vector during assembly of a J donor vector; f) represents the fragment of the Constant gene segment carried by the J donor vector.

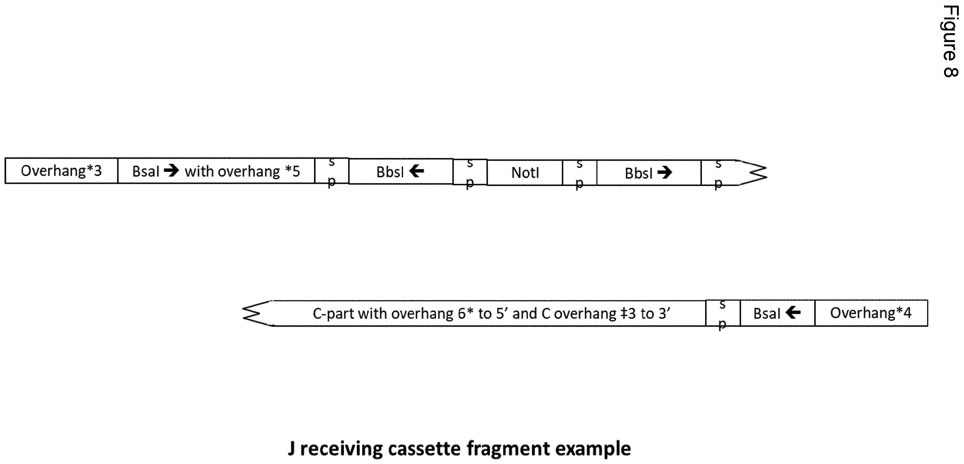

A schematic representation of the J receiving cassette fragment is presented in Figure 8. Examples 2 and 3 describe the format and use of J receiving cassette fragments to assemble J receiving cassette vectors for the human TRA and TRB loci, respectively.

In the present context, the J receiving cassette fragments are formed by annealing partially complimentary single stranded oligonucleotides resulting in a DNA duplex with single stranded overhangs at each termini.

A J receiving cassette fragment for the TRA locus are described as SEQ0098 and SEQ0099.

A J receiving cassette fragments for the TRB locus are described as SEQ0578 and SEQ0581, wherein two forms exist to account for the two Constant gene segments utilised at the human TRB locus.

In the provided examples, the J receiving cassette fragment, the Type IIS enzyme used for J donor vector assembly is Bbsl, whereas the Type IIS enzyme used for TCR ORF reconstitution is Bsal.

A J donor vector backbone comprises
a. an origin or replication
b. a second positive selection marker
c. a first restriction enzyme recognition sequence permitting digestion of the backbone to create a single stranded overhang complimentary to an overhang in the J receiving cassette fragment
d. a second restriction enzyme recognition sequence permitting digestion of the backbone to create a single stranded overhang complimentary to an overhang in the J receiving cassette fragment
wherein c) and d) permit the directional cloning of the J receiving cassette fragments through the use of unique complimentary overhang sequences.

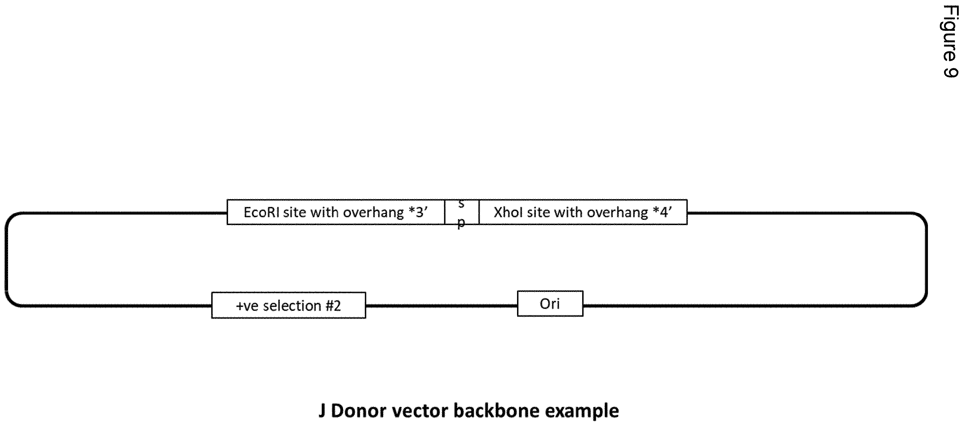

A J donor vector backbone is represented schematically in Figure 9 and described in detail in examples 2 and 3. A J donor vector backbone sequences is represented as SEQ0097.

The J donor vector backbone contains restriction enzyme recognition sites for EcoRI and Xhol, to create compatible overhangs with the 5' overhang and 3' overhangs provided in the J receiving cassette gene cloning fragments.

Any other combination of restriction enzymes could be used for assembly and reconstitution reactions, provided they satisfy the above-described criteria.

The method for generating a J receiving cassette vector by combining a J receiving cassette fragment and J donor vector backbone is well described in in examples 2 and 3.

A J receiving cassette vector is constructed by combining the TRAJ receiving cassette fragment and the TRAJ J donor vector backbone, wherein the method comprises
a. Digestion of the J donor vector backbone with the two restriction enzymes specific for the recognition sequences contained within the J donor vector backbone
b. Combining the digested J donor vector backbone with the J receiving cassette fragment with DNA ligase enzyme
c. Transformation of the resulting reaction product into competent host organism and positive selection using said the second positive selection marker to obtain complete J receiving cassette vector
wherein; a) creates single stranded overhangs complimentary with those contained within the J receiving cassette fragments; b) represents ligation of complimentary overhangs to form the J receiving cassette vector; c) represents selection and propagation of the J receiving cassette vector product.

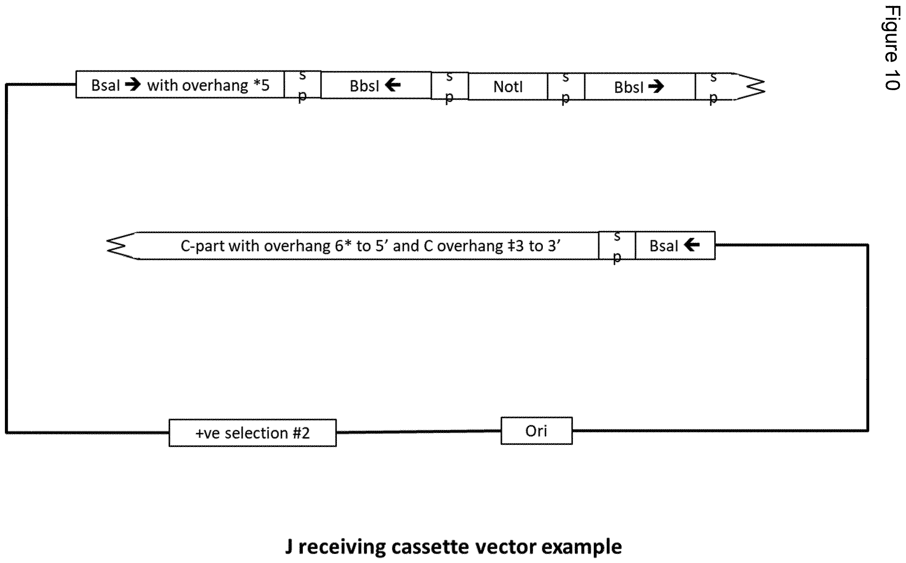

A schematic representation of a J receiving cassette vector is presented in Figure 10. Examples 2 and 3 describe the construction of J receiving cassette vectors for the human TRA and TRB loci, respectively.

A J receiving cassette vector for the TRA locus is described as SEQ0098, whereas those for the TRB locus are described as SEQ0582 and SEQ0583. The TRB locus utilises two Constant gene segments, thus the replica TRB J receiving cassette vectors contain C part sequences specific for each Constant gene segment.

A J receiving cassette vector is combined with a J segment part to create a J donor vector. The J segment part encodes the bulk of the J gene segment.

Said J segment part comprises
a. A first single stranded overhang sequence at the 5' end that is complementary to the overhang generated in the J receiving cassette vector, when acted on by the enzyme directed by the ninth Type IIS sequence
b. A Joining gene segment part
c. A second single stranded overhang sequence at the 3' end that is complementary to the overhang generated in the J receiving cassette vector, when acted on by the enzyme directed by the tenth Type IIS sequence
wherein, a) and c) direct directional cloning of the J segment part into the J receiving cassette vector that is digested by the Type IIS enzyme(s) provided in the assembly reaction; b) encodes the joining gene segment that is to be carried by the J donor vector product.

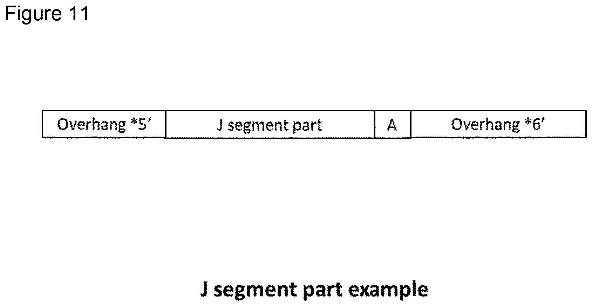

A schematic representation of a J segment part is presented in Figure 11. Examples 2 and 3 describe J segment parts used to construct J donor vectors for the human TRA and TRB loci, respectively.

Short J segment parts spanning the J gene segment usage of the human TRA locus are described as SEQ0099 to SEQ0210, wherein the J segment part is generated by annealing partially complimentary single stranded oligonucleotides resulting in a DNA duplex with single stranded overhangs at each termini.

A short J segment part can be used to generate J donor vectors with standardised J segment parts of minimal length, as is presented in Examples 2 and 3.

A long J segment part can be used to generate J donor vectors with extended coverage of the J gene segment to minimise the size of the odeCDR3 used in TCR reconstitution reactions. Shortening of the odeCDR3 element economises on synthesis cost for said element, and also minimises the mutational load within these oligonucleotide duplexes.

Long J segment parts spanning the J gene segment usage of the human TRA locus are described as SEQ0211 to SEQ0322, wherein the J segment part is generated by annealing partially complimentary single stranded oligonucleotides resulting in a DNA duplex with single stranded overhangs at each termini.

Short J segment parts spanning the J gene segment usage of the human TRB locus are described as SEQ0584 to SEQ0609, wherein the J segment part is generated by annealing partially complimentary single stranded oligonucleotides resulting in a DNA duplex with single stranded overhangs at each termini.

Long J segment parts spanning the J gene segment usage of the human TRB locus are described as SEQ0610 to SEQ0635, wherein the J segment part is generated by annealing partially complimentary single stranded oligonucleotides resulting in a DNA duplex with single stranded overhangs at each termini.

In the present context, the Type IIS sequences used for assembly of the J donor vector, designated the ninth and tenth Type IIS sequences above, are the same.

Alternatively, two different Type IIS recognition sequences could be used for this procedure.

The Type IIS sequences used for assembly of the J donor vector, designated the ninth, and tenth Type IIS sequences above, must be different from those used for the reconstitution reaction; designated first, second, third and fourth Type IIS sequences above.

The Type IIS sequences used for assembly of the J donor vector, designated the ninth, and tenth Type IIS sequences above, are the same as those used to assemble the V-C entry vector; designated fifth, sixth, seventh and eighth Type IIS sequences above.

These Type IIS sequences used to assemble the V-C entry and J donor vectors need not be the same or different, as they are treated independently.

A J segment part is combined with a J receiving cassette vector that contains a matched C part to generate a J donor vector.

A J donor vector is constructed by combining a J donor vector backbone with a J segment part, wherein the method comprises
a. Combining J receiving cassette vector with the J segment part, along with DNA ligase enzyme and one or more Type IIS restriction enzyme(s) recognising the ninth and tenth Type IIS sequences, and subjecting the combined mix to a thermocycling reaction, a
b. Transformation of the resulting reaction product into competent host organism and positive selection using said second positive selection marker to obtain complete J donor vector
wherein, the Type IIS enzyme action on the ninth and tenth Type IIS sequences in step a) creates single stranded overhangs within the J receiving cassette vector, through excision of the negative selection marker sequence.

A schematic representation of a resulting J donor vector is presented in Figure 3. Examples 2 and 3 describe the use of J segment parts and a J receiving cassette vectors to construct J donor vectors for the human TRA and TRB loci, respectively.

J donor vectors containing short J segments for the human TRA locus are described as SEQ0323 to SEQ0378, whereas J donor vectors containing long J segments for the human TRA locus are described as SEQ0379 to SEQ0434.

J donor vectors containing short J segments for the human TRB locus paired with C1 constant gene segment are described as SEQ0636 to SEQ0648, whereas J donor vectors containing long J segments for the human TRA locus are described as SEQ0662 to SEQ0674.

J donor vectors containing short J segments for the human TRB locus paired with C2 constant gene segment are described as SEQ0649 to SEQ0661, whereas J donor vectors containing long J segments for the human TRA locus are described as SEQ0675 to SEQ0687.

In the provided examples, the Type IIS enzyme used to assemble a J donor vector is Bbsl, whereas the Type IIS enzyme used within the reconstitution of a TCR is Bsal. The negative selection marker is a Notl restriction enzyme sequence.

The method to construct a J donor vector also entails a negative selection step to eliminate parental J receiving cassette vector prior to transformation and selection.

In the provided example, this negative selection entails a Notl digestion to eliminate parental J receiving cassette vector prior to transformation and selection.

### Generic features of J donor vectors and construction elements

As described above and in Example 5 below, several features of a V-C entry and J donor vector library may be generic, inasmuch that TCR gene segment elements are constructed into a generic context to achieve a TORES system.

To achieve a TORES for any given TCR loci, four sequence elements are required specific for a TCR chain encoded by said TCR loci:
X - a variable (V) gene segment fragment
Y - a constant (C) genes segment fragment
Y'- a constant (C) gene segment part
Z - a joining (J) gene segment fragment

According to the above description and examples below, these four forms of TCR sequence element can be assembled into various vector contexts to construct and deploy a TORES for any given V-J-C combination for any given TCR chain. For example, systems for the native human TRG and TRD locus, variant and/or synthetic TCR chain forms, or native TCR chain forms of an organism other than humans.

The C gene segment part and J gene segment fragment are those assembled into J donor vector via a J receiving cassette fragment and a J cloning fragment, respectively. This process is well described in Examples 2 and 3.

A generic J receiving cassette fragment represented by the sequences SEQ0695 and SEQ0696 wherein the third and fourth Type IIS sequences encode recognition for the enzyme Bsal, and the ninth and tenth Type IIS sequences encode recognition for the enzyme Bbsl, and the negative selection marker represents a Notl restriction enzyme recognition sequence. Within this sequence, the encoded C-part is denoted as Y'Nₙ, wherein Y' is the designation for the C-part, N represents any nucleotide, and n represents the number of nucleotides in said sequence.

The J receiving cassette fragments is generated by annealing the single stranded oligonucleotides represented by the pair of sequences with partially complementary sequence.

A J donor vector backbone to construct a J receiving cassette vector with the above generic J receiving cassette fragment is represented by SEQ0097, wherein the restriction enzymes EcoRI and Xhol are used to generate single stranded DNA overhangs required for insertion of the J receiving cassette fragment sequence.

A J receiving cassette vector constructed by the combination of the J receiving cassette fragment generated from the J receiving cassette fragments represented as sequences SEQ0695 and SEQ0696, and a J donor vector backbone represented by sequence SEQ0097, is thus represented by SEQ0697. Within this resulting J receiving cassette vector, the third and fourth Type IIS sequences encode recognition for the enzyme Bsal, and the ninth and tenth Type IIS sequences encode recognition for the enzyme Bbsl, and the negative selection marker represents a Notl restriction enzyme recognition sequence. As derived from the J receiving cassette fragment, the encoded C-part is denoted as Y'Nₙ, wherein Y' is the designation for the C-part, N represents any nucleotide, and n represents the number of nucleotides in said sequence.

The J receiving cassette vector has a J segment part inserted to obtain a J donor vector.

A generic J segment part used to insert into the generic J receiving cassette vector represented by SEQ0697, is thus represented by complementary sequences SEQ0698 and SEQ0699, wherein the J gene segment part is denoted as ZNₙ, wherein Z is the designation for the J gene segment, N represents any nucleotide, and n represents the number of nucleotides in said sequence.

A J segment part is generated by annealing the two single stranded oligonucleotides represented by SEQ0698 and SEQ0699.

A generic J donor vector assembled from the combination of the J receiving cassette vector represented by SEQ0697 and the J segment part represented by complementary sequences SEQ0698 and SEQ0699 is thus represented by sequence SEQ0700. This resulting J donor vector thus contains two annotated sequence inserts of ZNₙ and Y'Nₙ, wherein Z is the designation for the J gene segment, and Y' is the designation of the C segment part, N represents any nucleotide, and n represents the number of nucleotides in said sequences.

The above description of V-C entry vector, and the components from which they are assembled, is based on the use of Type IIS enzymes Bbsl and Bsal for construction and operation, respectively. Based on the guidance given above, one or more alternative Type IIS enzymes may be used for each of these tasks.

### Use of Full-length TCR ORFs in defined vector contexts for diagnostics and therapeutics

A key challenge in harnessing T-cell immunity for treatment of disease is the interindividual diversity of the HLA and TCR systems, along with the massive intraindividual content of the TCR repertoire. This means that medicaments and therapeutic strategies that rely on the assessment and/or provision of TCRs require robust assessment of TCR function in a bona fide biological context.

To achieve accurate assessment of native TCR chain pairs, a reliable and cost-effective high-throughput method for delivering captured TCR ORFs in defined vector contexts is needed. A TORES for any given chain pair is a means to deliver such TCR ORFs.

A defined vector context for TCR ORFs can be, for example, a transient expression vector, for instance, whereby the TCRs can be rapidly characterised on the surface of human cells as described in Example 8. In this example, TCRs could be sequenced and re-expressed to confirm expected specificity. This permits the use of validated TCR sequences for following TCR clonotype abundance by sequence or amplificationor probed- based assays for diagnostic procedures during immunotherapeutic interventions on a personalised basis. Similarly, rapid capture and validation of TCR chain pairs mean that said TCR pairs could be provided in personalised medicine - such as the delivery of soluble TCR constructs as a medicinal compound, or provision of the TCR in an effector cell as a cellular therapeutic.

In a similar approach, a TORES system is ideally suited for engineering TCR ORFs to change specificity and/or function, as outlined in Examples 9 and 10. This engineering can be used to enhance or reduce signalling strength and/or change or redirect the specificity of TCR chain pairs towards specific disease antigens. Such engineered TCR sequences could be provide in personalised immunotherapeutic strategies in place of native TCR chain pairs.

### The bidirectional TCR ORF Reconstitution and engineering System (TORES²)

The above-described TORES system treats each TCR chain within independent two-part vector library systems. Thus, the products of TORES operation are discrete vectors encoding reconstituted TCR ORFs. In a separate aspect, the present invention provides an alternative system to adjoin reciprocal TCR chain pairs into a single product vector using a two-step method. This is achieved in a five-component vector library system, wherein modified V-C entry vectors are combined with the J-donor vectors of the TORES system and odeCDR3 to achieve a reconstitution of a TCR chain pair in discrete reactions. The modified V-C entry vectors then permit the reconstitution TCR ORFs to be adjoined in a second step by addition of a fifth vector component, the Bidirectional Terminator donor vector (BiT donor), to achieve a vector encoding the two TCR OFR pairs in antiparallel coding sense.

This bidirectional TORES (TORES²) represents a combined five-component vector system as modifications to V-C entry vectors encoding elements of the reciprocal chain pairs are non-symmetrical. Meaning that the V-C entry vectors comprise distinct arrangements. That is, the reconstituted ORF of one chain is excised from the product vector of the first step and ligated in an antisense orientation into the reciprocal product vector of the first step. Therefore the pairing of the V-C entry vectors is critical, as one of the original V-C entry vectors of the TORES² system represents the final product backbone, and thus encodes the desired 3' and 5' genetic elements for downstream application of the reconstituted and adjoined TCR pair. For clarity, the description and examples presented below fix the TRA chain as the final product backbone (e.g. V-Ca entry vector) and the TRB as the chain integrated to this final product backbone (e.g. V-Cβ entry vector). The reciprocal arrangement is equally as valid, as is any other combination of reciprocal TCR chain pairs.

### V-C entry vector components of TORES²

As mentioned above, the five-component vector library system comprising TORES² differs from the TORES system in the provision of modified V-C entry vector contexts. These modifications incorporate distinct Type IIS sites (labelled as Type IIS #2) and negative selection elements (labelled as -ve selection #2) from those utilised for TCR ORF reconstitution in a first step, to direct the adjoining of those reconstituted ORFs into a single vector in a second step. In effect, the TORES² system incorporates the TORES system within a new V-C entry vector context. The description below treats the TRA chain as the backbone of the final product, and the TRB chain as that ligated into the reconstituted TRA chain backbone in an antisense orientation. The same framework applies for any pair of TCR chains, and there is no particular reasons as to why either of the TCR chains need to be in one of the described vector arrangements, or the other.

The V-Ca entry vector contains,
a. origin of replication,
b. a first positive selection marker,
c. 5' genetic element, or elements,
d. Kozak Sequence,
e. TCR alpha variable gene segment,
f. a first Type IIS sequence, for site specific recognition and cleavage by a Type IIS restriction enzyme (for Type IIS enzyme #1),
g. a first negative selection marker,
h. a second Type IIS sequence (for Type IIS enzyme #1),
i. TCR constant gene segment,
j. A third Type IIS sequence (for Type IIS restriction enzyme #2),
k. A second negative selection marker,
l. A fourth Type IIS sequence (for Type IIS restriction enzyme #2), and
m. 3' genetic element, or elements
wherein, a) and b) are used for propagation and selection of both parental V-C entry vector and the reconstituted TCR-containing vector in a bacterial host; c) and m) are used to define the downstream application of the reconstituted and adjoined full-length TCR ORFs, and may include elements as described above for the TORES; d) ensures efficient initiation of translation in eukaryotic cells, which could alternatively represent a Shine-Dalgarno sequence for transitional regulation in prokaryotes and archaea; e) represents the variable (V) alpha gene segment from the start codon to a motif at the 5' edge of the CDR3 region conserved across all V segments in a given two-component vector system; f) represents a Type IIS #1 recognition sequence that directs a Type IIS restriction enzyme to cut in the 5' direction as to create a standardised single stranded overhang at the 3' end of the V gene segment; g) represents a negative selection marker #1 to eliminate parental V-C entry vector during operation of the system to reconstitute a full-length TCR ORF; h) represents a Type IIS #1 recognition sequence that directs a Type IIS restriction enzyme to cut in the 3' direction as to create a standardised single stranded overhang at the 5' end of the C gene segment; i) represents the constant (C) gene segment from a motif at the 5' end of the C gene segment conserved across all C segments in a given two-component vector system, and which defines the boundary with the J segment (see Figures 2 and 4); j) represents a Type IIS #2 recognition sequence that directs a Type IIS restriction enzyme to cut in the 5' direction as to create a standardised single stranded overhang at the 3' end after the C gene segment, this Type IIS enzyme is different to the first and second Type IIS #1 enzymes; k) represents a negative selection marker #2 to eliminate parental TRA ORF vector during the operation of creating a bidirectional vector construct, it is distinct from the first negative selection marker; I) represents a Type IIS #2 recognition sequence that directs a Type IIS restriction enzyme to cut in the 3' direction as to create a standardised single stranded overhang before the 3' genetic element, this Type IIS enzyme is different to the first and second Type IIS #1 enzymes; m) represents the 3' genetic element, or elements.

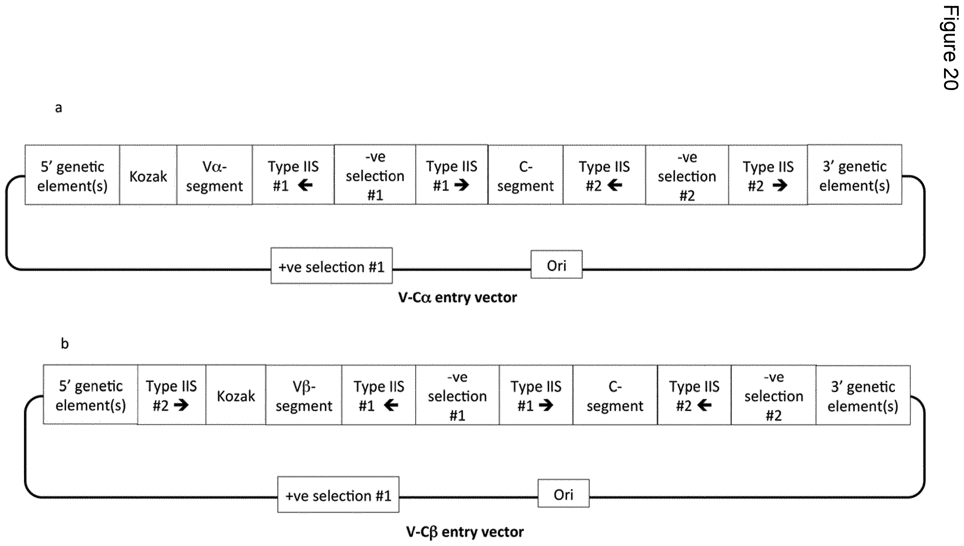

The arrangement of the above-described V-Ca entry vector is depicted in Figure 20 a.

Generally, the first and second Type IIS enzymes (designated as Type IIS #1) are the same but may also be different. Similarly, the third and fourth Type IIS enzymes (designated as Type IIS #2) are generally the same but may also be different. Critically, the Type IIS enzyme(s) used for the first and second Type IIS (Type IIS #1) sites must be different from the enzyme(s) used for the third and fourth Type IIS (Type IIS #2) sites.

A V-Ca entry vector backbone is represented by SEQ0756, as presented in Example 11.

Example 11 below presents a V-Ca entry vector of the TORES² for the human TRA locus (SEQ0756), wherein some of the V/C sequences have been modified relative to those incorporated into the TORES system as to be devoid of the Type IIS enzyme used in the third and fourth sites (SEQ0757 to SEQ0763)

The V-Cβ entry vector contains,
a. origin of replication,
b. a first positive selection marker,
c. 5' genetic element, or elements,
d. a first Type IIS sequence, for site specific recognition and cleavage by a Type IIS #2 restriction enzyme
e. Kozak Sequence,
f. TCR beta variable gene segment,
g. a second Type IIS sequence, for site specific recognition and cleavage by a Type IIS restriction enzyme (Type IIS #1),
h. a first negative selection marker,
i. a third Type IIS sequence (Type IIS #1),
j. TCR constant gene segment,
k. a fourth Type IIS sequence (Type IIS #2),
l. a second negative selection marker, and
m. 3' genetic element, or elements
wherein, a) and b) are used for propagation and selection of both parental V-C entry vector and the reconstituted TCR-containing vector in a bacterial host; c) and m) optionally define the downstream application of the reconstituted full-length TCR ORF as described above for the TORES system; d) represents a Type IIS #2 recognition sequence that directs a Type IIS restriction enzyme to cut in the 3' direction as to create a standardised single stranded overhang after the 5' genetic element and before the Kozak sequence, this Type IIS enzyme is different to the second and third Type IIS #1 enzymes; e) ensures efficient initiation of translation in eukaryotic cells, which could alternatively represent a Shine-Dalgarno sequence for transitional regulation in prokaryotes and archaea; f) represents the variable (V) gene segment from the start codon to a motif at the 5' edge of the CDR3 region conserved across all V segments in a given two-component vector system; g) represents a Type IIS #1 recognition sequence that directs a Type IIS restriction enzyme to cut in the 5' direction as to create a standardised single stranded overhang at the 3' end of the V gene segment; h) represents a negative selection marker to eliminate parental V-C entry vector during operation of the system to reconstitute a full-length TCR ORF; i) represents a Type IIS #1 recognition sequence that directs a Type IIS restriction enzyme to cut in the 3' direction as to create a standardised single stranded overhang at the 5' end of the C gene segment; j) represents the constant (C) gene segment from a motif at the 5' end of the C gene segment conserved across all C segments in a given two-component vector system, and which defines the boundary with the J segment (see Figures 2 and 4); k) represents a Type IIS #2 recognition sequence that directs a Type IIS restriction enzyme to cut in the 5' direction as to create a standardised single stranded overhang after the C segment but before the 3' genetic element, this Type IIS enzyme is different to the second and third Type IIS #1 enzymes; m) represents a second negative selection marker, which is different to the first -negative selection marker, I) represents 3' genetic element or elements.

The arrangement of the above-described V-Cβ entry vector is depicted in Figure 20b.

The 3' and 5' genetic elements are optional in the V-Cβ entry vector context, as the ORF reconstituted within this vector is later excised and ligated into the V-Ca entry vector backbone context. That is, the V-Ca entry vector backbone.

Generally, the second and third Type IIS enzymes (designated Type IIS #1) are the same but may also be different. Similarly, the first and fourth Type IIS enzymes (designated Type IIS #2) are generally the same but may also be different. Critically, the Type IIS enzyme(s) used for the second and third Type IIS sites (Type IIS #1) must be different from the enzyme(s) used for the first and fourth Type IIS sites (Type IIS #2).

Generally, the first and second Type IIS sites (Type IIS #1) of the V-Ca entry vector are the same as the second and third of the V-Cβ entry vector, but need not be as these reactions may be operated independently. The reconstitution of TRA chains and TRB chains in the described system could be conducted in a single reaction if the first and second Type IIS sites of the V-Ca entry vector were distinct from those of the second and third Type IIS sites of the V-Cβ entry vectors.

Generally, the third and fourth Type IIS sites (Type IIS #2) of the V-Ca entry vectors are the same as the first and fourth of the V-Cβ entry vectors as to require only the addition of a single Type IIS enzyme (Type IIS #2) to the second reaction adjoining reaction.

Generally, the second negative selection marker (-ve selection #2) of each V-Ca and V-Cβ entry vectors are the same as to facilitate efficient negative selection of parental vectors in the adjoining step of the TORES² operation, but may also be different.

A V-Cβ entry vector backbone is represented by SEQ0764, as presented in Example 11.

Example 11 below presents a V-Cβ entry vector of the TORES² for the human TRB locus (SEQ0764), wherein some of the V/C sequences have been modified relative to those incorporated into the TORES system as to be devoid of the Type IIS enzyme used in the first and fourth sites (SEQ0765 to SEQ0776)

### J donor and odeCDR3 components of the TORES²

The J donor vector and the oligonucleotide duplex encoding the CDR3 are the same as already described for the conventional TORES above. Both are used within the first step reaction(s) to reconstitute the reciprocal TCR chain pairs within the designated V-C entry vector backbone contexts in a procedure identical to that described above for the TORES.

### Bidirectional terminator donor vector (BiT donor) of the TORES²

The first four vector library components of the TORES² represent the V-Ca and V-Cβ entry vectors, along with the J donor vectors for each of the TRA and TRB chain in the TRA/TRB focused description. Again, it should be noted that any TCR chains may be incorporated into this system following the described design principles, and the specific TRA and TRB configuration is fixed for clarity.

The fifth component represents Bidirectional Terminator Donor vector (BiT donor), which provides a bidirectional terminator element that is interposed by the antiparallel sense TRA and TRB chains reconstituted in the first step reaction, and adjoined by this bidirectional terminator element in the second step reaction.

The bidirectional terminator is generally provided as a vector and is also surrounded by two Type IIS restriction enzymes (Type IIS #2). It is equally as relevant to provide the bidirectional terminator element as a linear dsDNA construct with these Type IIS sites, or with target single strand DNA overhangs without Type IIS sites.

The vector containing the bidirectional terminator contains,
a. origin of replication,
b. a second positive selection marker,
c. first Type IIS sequence (Type IIS #2),
d. bidirectional terminator, and
e. second Type IIS sequence (Type IIS #2)
wherein, a) and b) are used for propagation and selection of the vector carrying the bidirectional terminator in a bacterial host, c) represents a Type IIS recognition sequence that directs a Type IIS restriction enzyme (Type IIS #2) to cut in the 3' direction as to create a standardised single stranded overhang, d) represents the bidirectional terminator which is used to ensure transcriptional termination on both sense and antisense DNA strands during expression of TCRs reconstituted into the TRA backbone, when said vector containing a reconstituted TCR ORF is transfected into mammalian cells; e) represents a Type IIS recognition sequence (Type IIS #2) that directs a Type IIS restriction enzyme to cut in the 5' direction as to create a standardised single stranded overhang.

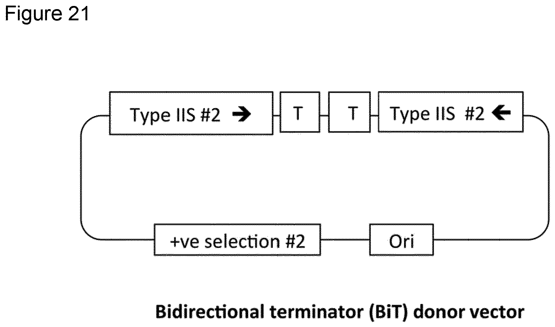

The arrangement of the above-described BiT donor vector is depicted in Figure 21.

Generally, the positive selection marker contained within the BiT donor is distinct from that of positive selection markers carried by the V-Ca and V-Cβ entry vectors, as to minimise carryover of parental BiT donor in the second step adjoining reaction.

Generally, the first and second Type IIS sites (Type IIS #2) in the BiT donor are the same as both the third and fourth Type IIS sites of the V-Ca entry vectors and the first and fourth of the V-Cβ entry vectors, as to require only the addition of a single Type IIS enzyme (Type IIS #2) to the second adjoining reaction.

The bidirectional terminator element sequence is represented by SEQ0777.

### Operation of TORES² to reconstitute and adjoin full length TCR ORF pairs

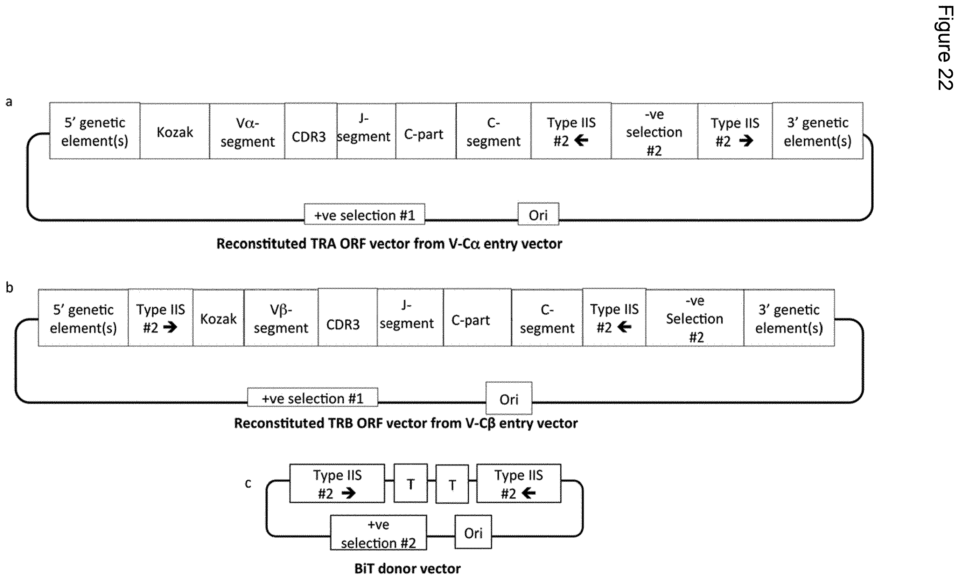

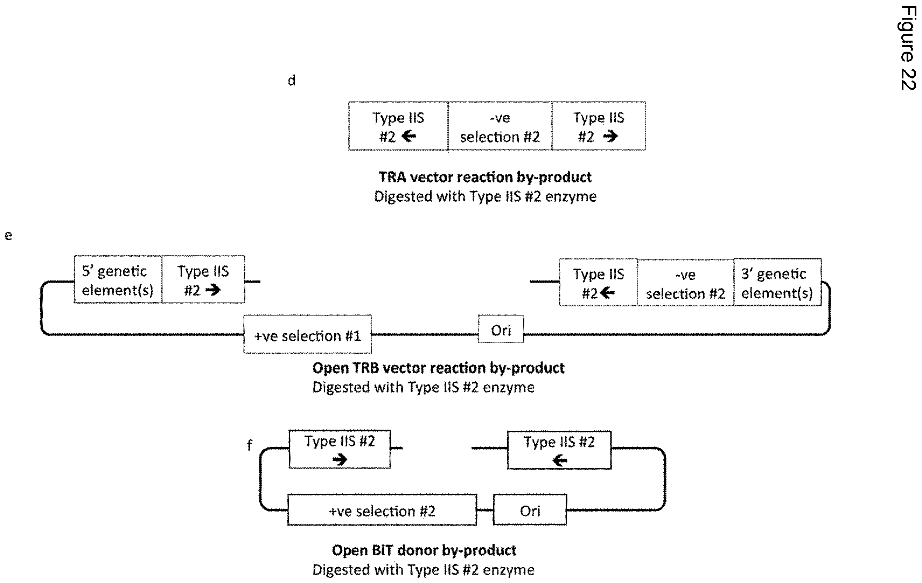

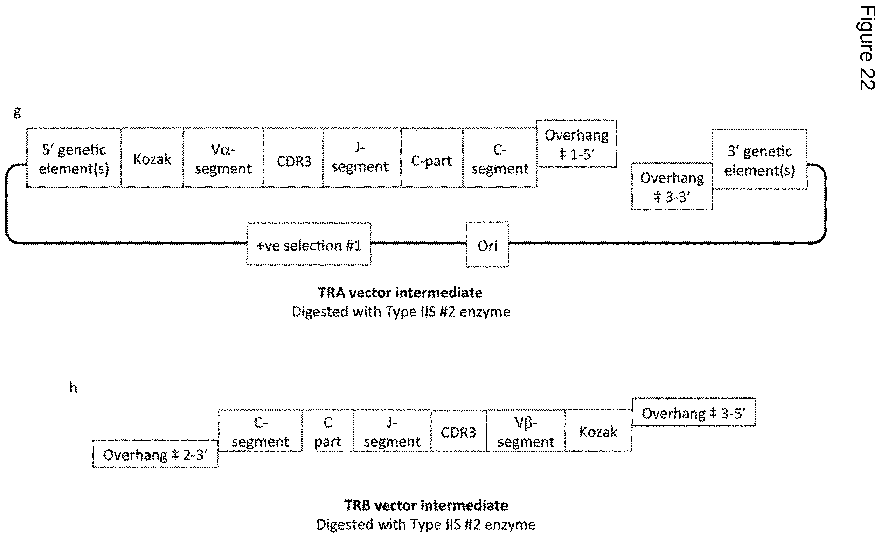

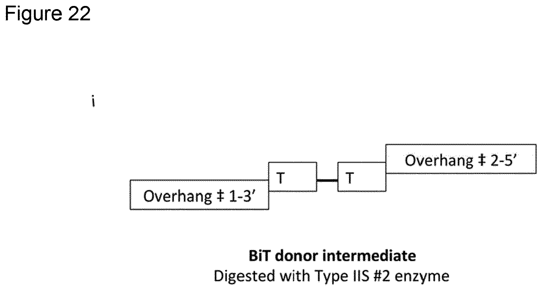

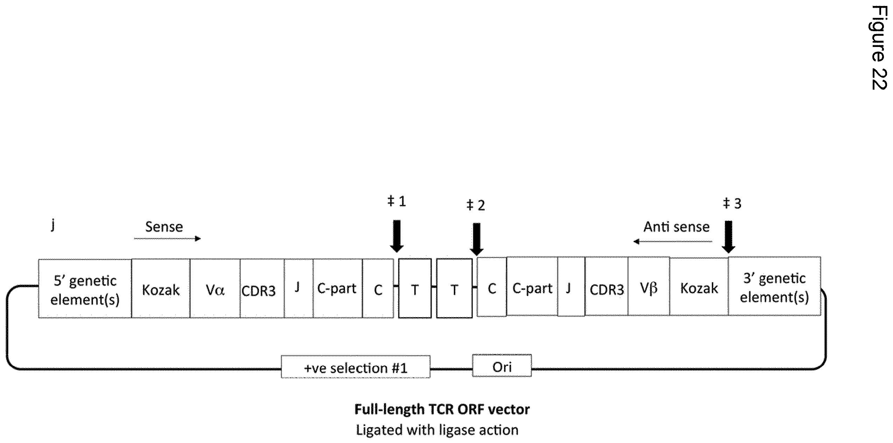

The operation of TORES² is a two-step process, incorporating a first TCR ORF reconstitution step, and second TCR ORF adjoining step. The method for reconstitution of TCR ORFs is identical to that described above for the TORES (see Figures 1 and 4). The products of the first step are therefore analogous to the products of TORES, comprising vectors encoding full-length TCR ORFs containing the V-CDR3-J-C element arrangement. However, the TORES² system provides these TCR ORFs in a distinct backbone context from the TORES system, wherein the TORES² backbone contains additional cloning sites to facilitate the second adjoining step reaction. This second step restriction enzyme and ligase cycle reaction entails the adjoining of the reconstituted TCR ORFs from the first step, in an antiparallel sense and interposing a bidirectional terminator element provided by the BiT donor. The overall process is depicted in Figure 22, which depicts the above-described TRA/TRB TORES² arrangement.

The reconstituted TRA encoding vector (Figure 22a), TRB encoding vector (Figure 22b) and the BiT donor (Figure 22c) are reacted in a single tube with Type IIS enzyme (Type IIS #2) and ligase and subjected to a restriction enzyme and ligase cycle reaction. The Type IIS restriction enzyme digests the three provided vectors into three reaction by-products, and three reaction intermediates with designed overhangs for directional ligation into the reaction product.

The three reaction by-products are represented by the excised -ve selection element #2 and Type IIS #2 sites from the TRA vector (figure 22d), the opened TRB encoding vector backbone from which the reconstituted TRB ORF has been excised (Figure 22e) and the opened BiT donor backbone from which the bidirectional terminator element has been excised (Figure 22f).

The first of the reaction intermediates is the open TRA vector, encoding the reconstituted TRA ORF in the V-Ca entry vector backbone context (Figure 22g). The third Type IIS #2 site in the original V-Ca entry vector as described above directs the enzyme to cut in the 5' direction to create a standardised single stranded overhang at the 3' end after the C gene segment (Figure 22 g, overhang ‡ 1-5'), while the fourth Type IIS #2 site directs enzyme cleavage in the 3' direction to create a standardised single stranded overhang before the 3' genetic element (Figure 22 e overhang ‡ 3-3').

The second reaction intermediate is the TRB ORF encoding fragment excised from the V-Cβ entry vector context. The first Type IIS #2 site in the V-Cβ entry vector described above directs the enzyme to cut in the 3' direction to create an overhang prior to the Kozak sequence (Figure 22h overhang ‡ 3-5'). The fourth Type IIS #2 site directs the enzyme to cut in 5' direction, creating an overhang after the C-segment (Figure 22h overhang ‡ 2-3'). It should be noted that Figure 22h depicts this intermediate in the antisense orientation in which it will be ligated into the product vector context.

The third reaction intermediate is the bidirectional terminator element excised from the BiT donor, where the first Type IIS #2 site directs the enzyme to cut in the 5' direction to yield a standardised single stranded overhang (Figure 22i overhang ‡ 1-3'). The second Type IIS #2 site directs the enzyme to cut in the 3' direction to create a standardised single stranded overhang (Figure 22i overhang ‡ 2-5').

Within the restriction enzyme and ligase cycle reaction, the single stranded overhangs drive ligase-mediated directional ligation into the product vector, adjoining both TRA and TRB chains within the original V-Ca entry vector backbone context (Figure 22j).

A TORES² may be constructed for any collection of TCR chains. For clarity, the TRA and TRB loci are used as a case study for description. The construction of such a TORES² is equally applicable in the context of the TRD and TRG loci, encoding the TCR delta and gamma chain pair, respectively, or indeed for any TRA/TRB, TRD/TRG or variant TCR chain pair system found in jawed vertebrates.

In the examples below, a TORES² system is provided for the human TRA/TRB loci, and applied to the reconstitution of a model human TCR ORF, and subsequently integrated into a matched engineered cell line harbouring RMCE sites matched with the 5' and 3' genetic elements of the originating V-Ca entry vector backbone context. This demonstrates that TORES² is readily used in combination with matched engineered cell lines optimised for integration and presentation of reconstituted TCR ORFs in a two-part device configuration similar to that described in application WO 2018/083318 A1.

In the following is given a table showing the sequences mentioned herein.

| SEQ ID | Name | Reference example | Description |
|---|---|---|---|
| 0001-0046 | TRA V cloning fragments | Example 1 | Full DNA sequences of the TRA V fragment |
| 0047 | TRA C constant cloning fragment | Example 1 | Full DNA sequence of the TRA C fraqment |
| 0048 | V-C entry vector backbone transient | Example 1 | DNA sequence of the vector backbone from the 5' genetic element encoding the CMV constitutive promoter to the 3' genetic element encoding the SV40pA polyadenylation siqnal |
| 0049-0094 | TRA V-C entry vector library sequence | Example 1 | DNA sequences of the cloned V-C fragments that make up the TRA V-C entry vector library |
| 0095-0096 | TRA J receiving cassette fragments | Example 2 | Full DNA sequence of the TRA J receiving cassette fragment oligonucleotides |
| 0097 | J donor backbone | Example 2 | J donor vector backbone is used to insert the TRA J receiving cassette fragment to create the TRA J receiving cassette vector |
| 0098 | TRA J receiving cassette vector | Example 2 | See above |
| 0099-0210 | TRA J Short segment part | Example 2 | Encodes all amino acids from the start of the CDR3-J border Phe codon |
| 0211-0322 | TRA J Long segment part | Example 2 | Encodes more amino acids N-terminal of the CDR3 border amino acids |
| 0323-0378 | TRA J Short donor vector | Example 2 | TRA short J donor library |
| 0379-0434 | TRA J Long donor vector | Example 2 | TRA long J donor library |
| 0435-0481 | TRB V cloning fragment | Example 3 | Full DNA sequences for the TRB V cloning fragments |
| 0482-0483 | TRB C constant cloning fragments | Example 3 | Full DNA sequences of the TRB C cloning fragments |
| 0484-0577 | TRB V-C entry vector library sequence | Example 3 | Sequences of the cloned V-C fragments that make up the TRA V-C entry vector library |
| 0578-0581 | TRB J receiving cassette fragments | Example 3 | TRB J receiving cassette fragments are constructed and inserted into a J donor vector backbone to create a TRB J receiving cassette vector |
| 0582-0583 | TRB J receiving cassette vectors | Example 3 | See above |
| 0584-0609 | TRB J Short segment part | Example 3 | DNA sequences of the short TRB J segment parts |
| 0610-0635 | TRB J Long segment part | Example 3 | DNA sequences of the long TRB J segment parts |
| 0636-0648 | TRB C1 J Short donor vector | Example 3 | TRB C1 short J donor library |
| 0649-0661 | TRB C2 J Short donor vector | Example 3 | TRB C2 short J donor library |
| 0662-0674 | TRB C1 J Long donor vector | Example 3 | TRB C1 long J donor library |
| 0675-0687 | TRB C2 J Long donor vector | Example 3 | TRB C2 long J donor library. |
| 0688 | V-C entry vector backbone F14-F15 | Example 4 | F14/F15 V-C entry vector backbone sequence used to construct TRA V-C entry library |
| 0689 | V-C entry vector backbone FRT-F3 | Example 4 | FRT/F3 V-C entry vector backbone sequence used to construct TRB V-C entry library |
| 0690 | Generic V cloning fragment XNₙ (left part) | Example 5 | Contain the required V gene segment fragment in the context of appropriate cloning sites for final V-C entry vector assembly (full sequence: 0690-XNₙ-0745) |
| 0691 | Generic C cloning fragment YNₙ (left part) | Example 5 | full sequence: 0691-YNₙ-0746 |
| 0692 | Generic Transient V-C Entry vector XNₙ-YNₙ (left part) | Example 5 | full sequence: 0692-XNₙ-0747-YNₙ-0748 |
| 0693 | Generic F14-F15 V-C Entry vector XNₙ-YNₙ (left part) | Example 5 | full sequence: 0693-XNⁿ-0749-YNₙ-0750 |
| 0694 | Generic FRT-F3 V-C Entry vector XNₙ-YNₙ (left part) | Example 5 | full sequence: 0694-XNₙ-0751-YNₙ-0752 |
| 0695-0696 | Generic J receiving cassette oligonucleotides Y'Nₙ (left part) | Example 5 | full sequence of 0695: 0695-Y'Nₙ-TGAGACCC; full sequence of 0696: 0696-Y'Nn-0753 |
| 0697 | Generic J receiving vector Y'Nₙ (left part) | Example 5 | full sequence: 0697-Y'Nₙ-0754 |
| 0698-0699 | Generic J segment oligonucleotides ZNₙ | Example 5 | full sequence of 0698: ctcgZNₙ; full sequence of 0699: Y'N(4)ZNₙ; for both 0698 and 0699, the dummy sequence "aaaaaaaaaa" was included in the sequence listing due to the 10 nucleotide minimum requirement of the ST.25 |
| 0700 | Generic J Donor vector ZNₙ-Y'Nₙ (left part) | Example 5 | full sequence: 0700-ZNₙ-Y'Nₙ-0755 |
| 0701-0702 | JG9 TRA and TRB full sequences copy | Example 6 | DNA sequences of the TRA and TRB chains |
| 0703-0706 | JG9 odeCDR3 sequences | Example 6 | odeCDR3 synthesised for the TRA and TRB chains |
| 0707-0718 | Sequence results from Example 8 | Example 8 | |
| 0719-0742 | odeCDR3 collection for reconstitution Example 8 | Example 8 | |
| 0743-0744 | degenerate TRA odeCDR3s Example 9 | Example 9 | odeCDR3 oligos |
| 0745 | Generic V cloning fragment XNₙ (right part) | Example 5 | Contain the required V gene segment fragment in the context of appropriate cloning sites for final V-C entry vector assembly (full sequence: 0690-XNₙ-0745) |
| 0746 | Generic C cloning fragment YNₙ (right part) | Example 5 | full sequence: 0691-YNₙ-0746 |
| 0747 | Generic Transient V-C Entry vector XNₙ-YNₙ (middle part) | Example 5 | full sequence: 0692-XNₙ-0747-YNₙ-0748 |
| 0748 | Generic Transient V-C Entry vector XNₙ-YNₙ (right part) | Example 5 | full sequence: 0692-XNₙ-0747-YNₙ-0748 |
| 0749 | Generic F14-F15 V-C Entry vector XNₙ-YNₙ (middle part) | Example 5 | full sequence: 0693-XNⁿ-0749-YNₙ-0750 |
| 0750 | Generic F14-F15 V-C Entry vector XNₙ-YNₙ (right part) | Example 5 | full sequence: 0693-XNⁿ-0749-YNₙ-0750 |
| 0751 | Generic FRT-F3 V-C Entry vector XNₙ-YNₙ (middle part) | Example 5 | full sequence: 0694-XNₙ-0751-YNₙ-0752 |
| 0752 | Generic FRT-F3 V-C Entry vector XNₙ-YNₙ (riqht part) | Example 5 | ull sequence: 0694-XNₙ-0751-YNₙ-0752 |
| 0753 | Generic J receiving cassette oligonucleotides Y'Nₙ (right part) | Example 5 | full sequence: 0696-Y'Nₙ-0753 |
| 0754 | Generic J receiving vector Y'Nₙ (riqht part) | Example 5 | full sequence: 0697-Y'Nₙ-0754 |
| 0755 | Generic J Donor vector ZNₙ-Y'Nₙ (right part) | Example 5 | full sequence: 0700-ZNₙ-Y'Nₙ-0755 |
| 0756 | V-Ca entry vector backbone FRT/F3 | Example 11 | V-Ca entry vector backbone for TORES2 |
| 0757-0763 | Modified TRA V-C sequences | Example 11 | Full DNA sequences of the TRA V fragment, modified for TORES2 |
| 0764 | V-Cβ entry vector backbone FRT/F3 | Example 11 | V-Cβ entry vector backbone for TORES2 |
| 0765-0776 | Modified TRB V-C sequences | Example 11 | Full DNA sequences of the TRB V fragment, modified for TORES2 |
| 0777 | Bidirectional terminator element | Example 11 | Bidirectional terminator element (Esp3I to Esp3I site) |
| 0778-0779 | Model TRA/TRB pair | Example 11 | known specificity for a HLA-A*02:01-restricted antigen. |

### List of abbreviations

- **APC**: Antigen-presenting cell
- **BiT**: Bidirectional Terminator donor
- **CAR-T**: CAR T-cell
- **CAR**: Chimeric antigen receptor
- **CDR**: Complementarity-determining regions
- **C segment**: Constant segment (also C region)
- **CMV**: Cytomegalovirus
- **DAMPS**: Danger associated molecular patterns
- **DC**: Dendritic cells
- **DNA**: Deoxyribonucleic acid
- **dsDNA**: Double stranded Deoxyribonucleic acid molecule
- **D segment**: Diversity segment (also D region)
- **eAPC**: Engineered antigen-presenting cell
- **FACS**: Fluorescence-activated cell sorting
- **FRT**: Flippase recognition target
- **GEM T-cells**: Germ line-encoded mycolyl-reactive T-cells
- **GFP**: Green fluorescent protein
- **HLAI**: HLA class I
- **HLAII**: HLA class II
- **HDR**: Homology directed recombination
- **HLA**: Human leukocyte antigen
- **IgSF**: Immunoglobulin superfamily
- **IRES**: Internal ribosome entry site
- **ITAM**: Immunoreceptor tyrosine-based activation motif
- **iNK T-cells**: Invariant natural killer T-cells
- **J segment**: Joining segment (also J region)
- **MACS**: Magnetic-activated cell sorting
- **MAGE**: Melanoma associated antigen
- **MAIT**: Mucosal-associated invariant T
- **odeCDR3**: Oligonucleotide duplex encoding CDR3
- **ORF**: Open reading frame
- **PAMPS**: Pathogen-associated molecular patterns
- **PCR**: Polymerase chain reaction
- **RMCE**: Recombinase mediated cassette exchange
- **RFP**: Red fluorescent protein
- **RT**: Reverse Transcription
- **DNA**: Ribonucleic acid
- **SH2**: Src homology 2
- **T-cells**: T lymphocytes
- **TCR**: T-cell Receptor
- **TRA**: TCR alpha
- **TRB**: TCR beta
- **TRD**: TCR delta
- **TRG**: TCR gamma
- **TAA**: Tumour-associated-antigens
- **TORES**: TCR ORF Reconstitution and Engineering System
- **TORES2**: Bidirectional TCR ORF Reconstitution and Engineering System
- **V segment**: Variable segment (also V region)
- **β2M**: β2-microglobulin
- **ZAP-70**: ζ-chain-associated protein of 70 kDa

### List of definitions

**A pair of complementary TCR chains:** two TCR chains wherein the translated proteins are capable of forming a TCRsp on the surface of a TCR presenting cell

**Affinity:** Kinetic or equilibrium parameter of an interaction between two or more molecules or proteins

**Allele:** Variant form of a given gene

**Amplicon:** a piece of DNA or RNA that is the source and/or product of artificial amplification using various methods including PCR.

**Analyte:** an entity that is of interest to be identified and/or measured and/or queried

**Antibody:** Affinity molecule that is expressed by specialized cells of the immune system called B-cells and that contains of two chains. B-cells express a very large and very diverse repertoire of antibodies that do generally not bind self proteins but can bind and neutralize pathogens or toxins that would threaten the host. Natural or artificially engineered antibodies are often used as affinity reagents.

**APC:** Antigen-presenting cell. A cell capable of presenting antigen on its cell surface, generally in the context of an HLA.

**C (-segment):** Constant segment. Also Constant region. One of the gene segments that is used to assemble the T-cell receptor. The c-region is a distinct segment that rather than driving diversity of the TCR, defines its general function in the immune system.

**C cloning fragment:** Constant Cloning fragment. Also referred to as a C gene segment cloning fragment. A construct carrying a portion of a C gene segment used to construct a V-C entry vector.

**C-part:** Constant part. A small portion of Constant gene segment sequence carried by a J receiving cassette fragment, J receiving cassette and J donor vector to standardise overhang sequences for operation of the TORES to reconstitute TCR ORFs.

**CDR:** complementarity-determining regions. Short sequences on the antigen-facing end of TCRs and antibodies that perform most of the target binding function. Each antibody and TCR contains six CDRs and they are generally the most variable part of the molecules allowing detection of a large number of diverse target molecules.

**Cis-acting element:** regions of non-coding DNA that regulate the transcription of nearby ORFs.

**D (-segment):** Diversity segment. Also D region. One of the gene segments that is used to assemble the T-cell receptor. Each individual has a large number of different variations of these regions making it possible for each individual to arm T-cells with a very large variety of different TCR.

**Diversification:** A process where a sequence is diversified.

**DNA:** Deoxyribonucleic acid. Chemical name of the molecule that forms genetic material encoding genes and proteins.

**Endogenous:** Substance that originated from within a cell

**Eukaryotic conditional regulatory element:** A DNA sequence that can influence the activity of a promoter, which may be induced or repressed under defined conditions

**Eukaryotic Promoter:** A DNA sequence that encodes an RNA polymerase binding site and response elements. The sequence of the promoter region controls the binding of the RNA polymerase and transcription factors, therefore promoters play a large role in determining where and when your gene of interest will be expressed.

**Eukaryotic terminator/Signal terminator:** A DNA sequence that are recognized by protein factors that are associated with the RNA polymerase II and which trigger the termination process of transcription. It also encodes the poly-A signal

**FACS/Flow Cytometry:** Fluorescence-activated cell sorting. Flow cytometry is a technique by which individual cells can be analyzed en masse for the expression of specific cell surface and intracellular markers. A variation of that technique, cell sorting, allows cells that carry a defined set of markers to be retrieved for further analysis.

**Flippase:** A recombinase (Flippase, Flp) derived from the 2 µm plasmid of baker's yeast *Saccharomyces cerevisiae.*

**Heterospecific recombinase sites:** A DNA sequence that is recognized by a recombinase enzyme to promote the crossover of two DNA molecules

**HLA I:** Human Leukocyte Antigen class I. A gene that is expressed in humans in all nucleated cells and exported to the cell surface where it presents as cargo short fragments, peptides, of internal proteins to T-cell receptors. As such it presents fragments of potential ongoing infections along with intrinsic proteins. The HLA I can additionally present as cargo peptides that are added to the culture medium, generated from proteins expressed form introduced genetic elements or generated from proteins that are taken up by the cell. HLA class I genes are polymorphic meaning that different individuals are likely to have variation in the same gene leading to a variation in presentation. Related to HLA class II.

**HLA II:** Human Leukocyte Antigen Class II. A gene that is expressed in humans in specific cells that are coordinating and helping the adaptive immune response for example dendritic cells. Related to HLA class I. HLA class II proteins are exported to the cell surface where they present as cargo short fragments, peptides, of external proteins to T-cell receptors. As such it presents fragments of potential ongoing infections along with intrinsic proteins. The HLA II can additionally present as cargo peptides that are added to the culture medium, generated from proteins expressed form introduced genetic elements or generated from proteins that are taken up by the cell. HLA class II genes are polymorphic meaning that different individuals are likely to have variation in the same gene leading to a variation in presentation.

**Homologous arms:** A stretch of DNA that has near identical sequence identity to a complement homologous arm and therefore promote the exchange of two DNA molecules by the cellular process, homology directed repair.

**Immune surveillance:** Process in which the immune system detects and becomes activated by infections, malignancies or other potentially pathogenic alterations.

**Insulator:** A DNA sequence that prevents a gene from being influenced by the activation or repression of nearby genes. Insulators also prevent the spread of heterochromatin from a silenced gene to an actively transcribed gene.

**Integration:** The physical ligation of a DNA sequence into a chromosome of a cell

**Internal ribosome entry site (IRES):** A DNA sequence that once transcribed encodes a RNA element that allows the initiation of translation in a cap-independent manner

**J (-segment):** Joining segment. Also J region. One of the gene segments that is used to assemble the T-cell receptor. Each individual has a large number of different variations of these regions making it possible for each individual to arm T-cells with a very large variety of different TCR.

**J-C entry vector:** The vector of the two-component vector system that carries the J and C TCR segments, and which receives sequences from the V donor vectors and odeCDR3 during reconstitution of a full-length TCR ORF.

**J donor backbone:** Joining donor backbone. The vector backbone into which a J receiving cassette fragment is inserted to create a J receiving cassette vector.

**J donor vector:** The vector of the two-component vector system that carries the J TCR segment, and donates this segment to the V-C entry vector during reconstitution of a full-length TCR ORF.

**J receiving cassette fragment:** Joining receiving cassette fragment. A cloning fragment that carries a C-part used to construct a J receiving cassette vector.

**J receiving cassette vector:** Joining receiving cassette vector. The vector, carrying a C-part, into which a J segment part is inserted to create a J donor vector.

**J segment part:** Joining segment part. A DNA construct carrying a portion of a J gene segment that is inserted into a J receiving cassette vector to generate a J donor vector.

K is a nucleotide code indicating Keto (K = G or T)

**Kozak Sequence:** Short sequence required for the efficient initiation of translation

**M** is a nucleotide code indicating aMino (M = A or C)

**Major HLA class I:** a Family of APX that comprise of the genes HLA-A, HLA-B and HLA-C

**Matched:** When two components encode genetic elements that direct and restrict the interaction between the complemented components

**Meganuclease recognition site:** A DNA sequence that is recognized by a endodeoxyribonuclease, commonly referred to as a meganuclease

**Mobile genetic element:** A DNA sequence that can permit the integration of DNA with the activity of transposase enzymes

**N** is a nucleotide code indicatin4g0 aNy nucleotide (N = A, T, C or G)

**Native:** an entity that is naturally occurring to the cell

**Negative Selection Marker:** A selectable marker that confers negative selection of a vector and/or of host organism carrying said marker-bearing vector

**odeCDR3:** oligonucleotide duplex encoding complementarity-determining regions. A synthetic construct carrying CDR3 genetic sequence with terminal overhangs, used in conjunction with the two-component vector system to reconstitute a full-length TCR ORF.

**Origin of replication:** a particular sequence in a vector, plasmid or genome at which replication is initiated.

**ORF:** Open reading frame. Stretch of genetic material that encodes a translation frame for synthesis of a protein (polypeptide) by the ribosome

**Overhang:** A single stranded sequence at the terminus of a double stranded nucleic acid molecule. Often referred to as sticky or cohesive ends.

**PCR:** Polymerase chain reaction in which a specific target DNA molecule is exponentially amplified

**Peptide:** short string of amino acids between 6 - 30 amino acids in length

**Phenotypic analysis:** Analysis of the observable characteristics of a cell.

**Plasmid:** A genetic construct can replicate independently of the chromosomes, typically a small circular DNA strand in the cytoplasm of a bacterium or a protozoan.

**Polymorphic:** Present in different forms in individuals of the same species through the presence of different alleles of the same gene.

**Polypeptide:** Protein consisting of a stretch of peptides, forming a three-dimensional structure.

**Positive Selection Marker:** A selectable marker that confers positive selection of a vector and/or host organism carrying said marker-bearing vector.

**Primer:** Short DNA sequence that allows specific recognition of a target DNA sequence for example during a PCR.

**Promoter:** Regulatory DNA element for the controlled initiation of gene expression.

**Recombinase:** Enzymes that mediate genetic recombination.

**Reconstitution:** In the present context, reconstitution describes the operation of the TORES and TORES² to assemble a TCR ORF. A 'reconstitution reaction' is the reaction mix and thermocycling reaction that this operation entails.

**Reporter Element:** A genetic element that mediates a reported signal in the organism or vector bearing said element. May be used as a positive or negative selection maker.

**Restriction Enzyme Cleavage Sequence:** The genetic sequence cleaved by a restriction enzyme, which can be intrinsic or intrinsic to the recognition sequence of said restriction enzyme.

**Restriction Enzyme Recognition Sequence:** The genetic sequence recognised and engaged by a restriction enzyme.

**Selectable marker:** A DNA sequence that confers a trait suitable for artificial selection methods.

**Slice acceptor site:** A DNA sequence at the 3' end of the intron AM, APX CM or affinity reagent for interaction with cells with TCRsp on the surface, or TCRsp based reagents.

**Slice donor site:** A DNA sequence at the 5' end of the intron.

**Suicide gene:** A gene that will mediate cell death within the host organism carrying said gene. May be used as a positive or negative selection marker.

**Synthetic:** an entity that is artificially generated.

**T-cell:** T lymphocyte. White blood cell that expresses a T-cell receptor on its surface. Selected by the immune system to not react with the own body but have the potential to recognize infections and malignancies as well as reject grafts from most members of the same species.

**TCR:** T-cell Receptor. Affinity molecule expressed by a subgroup of lymphocytes called T-lymphocytes. In humans the TCR recognizes cargo presented by APX CM or APX AM, including fragments from virus or bacterial infections or cancerous cells. Therefore, the TCR recognition is an integral part of the adaptive immune system. The TCR consists of two chains that are paired on the cell surface. The TCR expressed on the surface of each cells is assembled at random from a large pool of varied genes (the v,d,j and c segments) and thus each individual has a pool of T-cells expressing a very large and diverse repertoire of different TCRs.

**TRA:** TCR alpha encoding locus. One of the four different locus encoding genes that can form a VDJ recombined TCR chain. Translated TCR alpha chain proteins typically pair with translated TCR beta chain proteins to form alpha/beta TCRsp.

**TRB:** TCR beta encoding locus. One of the four different locus encoding genes that can form a VDJ recombined TCR chain. Translated TCR beta chain proteins typically pair with TCR alpha chain proteins to form alpha/beta TCRsp.

**TRD:** TCR delta encoding locus. One of the four different locus encoding genes that can form a VDJ recombined TCR chain. Translated TCR delta chain proteins typically pair with translated TCR gamma chain proteins to form gamma/delta TCRsp.

**TRG:** TCR gamma encoding locus. One of the four different locus encoding genes that can form a VDJ recombined TCR chain. Translated TCR gamma chain proteins typically pair with translate TCR delta chain proteins to form gamma/delta TCRsp.

**Type IIS Restriction Enzyme:** restriction enzymes that recognize asymmetric DNA sequences and cleave outside of their recognition sequence.

**V (-segment):** Variable region. Also V region. One of the gene segments that is used to assemble the T-cell receptor. Each individual has a large number of different variations of these regions making it possible for each individual to arm T-cells with a very large variety of different TCR.

**V-C entry vector:** The vector of the two-component vector system that carries the V and C TCR segments, and which receives sequences from the J donor vectors and odeCDR3 during reconstitution of a full-length TCR ORF.

**V cloning fragment:** Variable Cloning fragment. Also referred to as a V gene segment cloning fragment. A construct carrying a portion of a V gene segment used to construct a V-C entry vector.

**V donor vector:** The vector of the two-component vector system that carries the V TCR segment and donates this segment to the J-C entry vector during reconstitution of a full-length TCR ORF.

**Vector:** A vector is a genetic construct that carries genetic information. In the present context vector usually describes plasmid DNA vectors. A vector can represent any such construct that can be propagated and selected in a host organism.

**W** is a nucleotide code indicating Weak (W = A or T).

### Legends to figures

**Figure 1****. Two-component vector system for rapid full-length TCR ORF reconstitution.**
   A) Depicted are the core features of the two-component vector system (Box i), the required oligo duplex input (Box ii) and the resulting full-length TCR ORF vector (Box iii). The first component represents a V-C entry vector, incorporating a selected TCR V (variable) gene segment and a TCR C (constant) gene segment. This entry vector represents the backbone in which the final full-length TCR ORF will be carried. Thus, any desirable features of this vector backbone are tailored to the intended downstream application. Usually, this will be represented by at least a 5' and 3' genetic element. The required V gene segment and C gene segment of the target full-length TCR ORF is selected from a library of V-C entry vectors to construct a TCR ORF of any desired V/C combination (Box i). The second component represents a J donor vector, containing a selected J (joining) gene segment. This donor vector acts to donate the J gene segment, and thus the backbone of this vector is a reaction by-product. The desired J gene segment of the target TCR ORF is selected from a library J donor vectors. To complete the sequence required for the full-length TCR ORF, a short oligo duplex is synthesized corresponding largely to the CDR3 region of the mature TCR ORF (Box ii). This oligo duplex is synthesized with single strand overhangs compatible with unique overhangs generated by restriction enzyme digestion of V-C entry and J donor vectors. When combined into a single-tube reaction along with appropriate restriction and ligase enzymes, the full-length TCR ORF is reconstituted from the sequences provided by the V-C entry and J donor vectors selected from a pre-existing library, and the synthesized oligo duplex (Box iii).
   B) Function of the overall library feature of the TCR ORF reconstitution system is illustrated. A single V-C entry vector is selected from a library of V-C entry vectors with varying V-C combinations (Box i). This selection is based on the required V-C combination sequences for a selected TCR chain. A single J entry vector is selected from a library of J donor vectors with varying J gene segments encoded (Box ii). This selection is based on the required J combination sequences for the same selected TCR chain as that of the V-C entry vector. Finally, an *oligomeric duplex encoding CDR3* (odeCDR3) is selected as to complete the full-length ORF of the target TCR chain (Box iii). These three components (Box i, ii and iii) are combined into a single reaction along with appropriate restriction and ligase enzymes. The reaction cycle produces a reconstituted full-length TCR ORF in a single step in the V-C entry vector backbone context (Box iv).
**Figure 2****. Plasmid schematic of the genetic features of a generic V-C entry vector.** Depicted is a circularized plasmid schematic of a V-C entry vector with minimally required genetic elements depicted as labelled boxes. **Kozak,** refers to consensus sequence that plays a role in the efficient initiation of translation. **V-segment,** refers to a selected sequence encoding a proportion of a TCR variable germline ORF, or mutant/synthetic ORF. **Type IIS ←,** refers to a Type IIS restriction enzyme binding site orientated such the enzyme cleaves in the 5' direction. **Type IIS →,** refers to a Type IIS restriction enzyme binding site orientated such the enzyme cleaves in the 3' direction. **-ve selection,** refers to a negative selection element designed to be detrimental to a plasmid harbouring the sequence during the full-length TCR reconstruction reaction, or subsequent selection steps. **C-segment,** refers to a selected sequence encoding a proportion of a TCR constant germline ORF, or mutant/synthetic ORF. **+ve selection #1,** refers to a the first positive selection marker of the two-component system used to convey a selective advantage to the organism harbouring the vector, and which is different to the positive selection marker of the second vector component (see figure 3). **Ori,** refers to an origin of replication used for the propagation of plasmid within a compatible host. **5' genetic element,** refers to any desired genetic element that provides attributes required for downstream application of the reconstructed full-length TCR, and should be situated 5' of the reconstructed full-length TCR , for example, a cis-acting element. **3' genetic element, refers to a** to any desired genetic element that provides attributes required for downstream application of the reconstructed full-length TCR, and should be situated 3' of the full-length TCR ORF, for example, a transcriptional terminator element.
**Figure 3****. Plasmid schematic of the genetic features of a generic J donor vector.** Depicted is a circularized plasmid schematic of a J donor vector with minimally required genetic features depicted as labelled boxes. **J segment part,** refers to a DNA sequence encoding a proportion of a TCR joining germline ORF, or mutant/synthetic J gene segment. **C part,** refers to a small 5' portion of the TCR Constant gene segment. **Type IIS ←,** refers to a Type IIS restriction enzyme binding site orientated such the enzyme cleaves in the 5' direction. **Type IIS →,** refers to a Type IIS restriction enzyme binding site orientated such the enzyme cleaves in the 3' direction. **+ve selection #2,** refers to the first positive selection marker of the two-component system used to convey a selective advantage to the organism harbouring the vector, and which is different to the positive selection marker of the first vector component (see figure 2). Ori, refers to an origin of replication used for the propagation of plasmid within a compatible host.
**Figure 4****. Generic description of genetic input, by-products, intermediates and product of the two-component vector system to reconstruct a full-length TCR ORF.**
   Depicted are the two components of the vector system (a and b), the oligonucleotide duplex (c), when these three components are combined into a single reaction with Type IIS restriction enzyme and ligase, two reaction by-products (d and e), two reaction intermediates (f and g) and one reaction product (h) is generated. Input vectors and product of the two-component system are depicted as circularized plasmid schematics with genetic elements depicted as labelled boxes; open plasmid vectors that represent by-product or intermediate are non-circularized plasmid schematics with genetic elements depicted as labelled boxes; and linear DNA are depicted as series of labelled boxes describing genetic elements.
   a) A generic V-C entry plasmid as depicted in figure 2.
   b) A generic J donor plasmid as depicted in figure 3.
   c) A DNA oligo duplex containing CDR3 sequence flanked by two single stranded DNA overhangs, overhang **‡**1-5' and overhang **‡**1-3'. Overhang **‡**1-5' is compatible with the overhang **‡**1-3' in the open V-C entry vector intermediate (g). Overhang **‡**2-3' is compatible with the overhang **‡**2-5' in the donor fragment intermediate (f).
   d) Digestion of the V-C entry vector (a) by the Type IIS restriction enzyme results in a linear DNA V-C entry vector reaction by-product containing the -ve selection element and the **Type IIS ←** and **Type IIS →** elements.
   e) Digestion of the J donor vector (b) by the Type IIS restriction enzyme results in a non-circularised plasmid by-product containing all genetic elements of the parental plasmid except those carried in the excised J donor fragment intermediate (f).
   f) Digestion of the J donor vector (b) by the Type IIS restriction enzyme results in a linear DNA fragment containing the J segment part and C part flanked by single strand DNA overhangs, overhang **‡**2-5' and overhang ‡3-3'. Overhang **‡**2-5' is compatible with the overhang **‡**2-3' in CDR3 DNA oligonucleotide duplex (c). Overhang **‡**3-3' is compatible with the overhang **‡**3-5' in the open V-C entry vector intermediate (g).
   g) Digestion of the V-C entry vector (a) by the Type IIS restriction enzyme results in a non-circularized plasmid intermediate containing all genetic elements of the parental plasmid except those carried in the excised linear DNA V-C entry vector reaction byproduct (d). Digestion has additionally created two single stranded DNA overhangs, overhang **‡**1-3' and overhang **‡**3-5'. Overhang **‡**1-3' is compatible with the overhang **‡**1-5' in the CDR3 DNA oligonucleotide duplex (c). Overhang **‡**3-5' is compatible with the overhang **‡**3-3' in the J donor fragment intermediate (f).
   h) Ligation of all three compatible single stranded DNA overhangs results in the full-length TCR ORF vector as circularized plasmid. This plasmid contains all genetic elements of the parental V-C entry vector (a) with the exception of the excised V-C entry vector reaction by-product (d). In addition, the full-length TCR ORF vector incorporates the CDR3 sequence from the CDR3 DNA oligonucleotide duplex (c) and J segment part and C part from the J donor fragment reaction intermediate. Arrows indicate the approximate points of ligation between compatible single stranded DNA overhangs **‡**1, **‡**2 and **‡**3. Ligation point **‡**1 is comprised of the **‡**1-3' and **‡**1-5' elements donated by the V-C entry vector reaction intermediate (g) and CDR3 DNA oligonucleotide duplex (c), respectively. Ligation point **‡**2 is comprised **‡**2-3' and **‡**2-5' elements donated by the CDR3 DNA oligonucleotide duplex (c) and the J donor fragment reaction intermediate (f), respectively. Ligation point **‡**3 is comprised **‡**3-3' and **‡**3-5' elements donated by the J donor fragment reaction intermediate (f) and the V-C entry vector reaction intermediate (g), respectively.
**Figure 5** **Arrangement of V cloning fragments for the construction of V-C entry vectors**
   Depicted is a representation of the V cloning fragments used to assemble V-C entry vectors of a TORES for human TRA and TRB TCR chains as described in Examples 1 to 4.
   The V cloning fragment is flanked by unique primer bind sequences at 5' and 3' end to facilitate PCR-mediated amplification of the cloning fragments. Bbsl sites represent a specific Type IIS restriction enzyme binding sites used in the assembly of the V-C entry vector, where **→** indicates that the recognition site is orientated to cut in the 3' direction of the site, and **←** indicates that the site is orientated to cut in the 5' direction. The Bbsl **→** site cuts 5' of the encoded Kozak sequence to create overhang*1. The Bsal **←** site cuts to create the 5' Notl overhang within the Notl 5' fragment. Overhang*1 and the 5' Notl overhang ultimately ligate with overhang*1' of digested V-C entry vector backbone, and the 3' Notl overhang of the digested C cloning fragment, respectively, in assembly of the V-C entry vector. The NotI 5' fragment represents a 6 nucleotide 5' fragment of the Notl recognition sequence, wherein Notl acts as the negative selection marker to eliminate parental V-C entry vector in operation of the TORES. The complete Notl recognition site is reconstituted with the 3' Notl fragment, provided by the C cloning fragment. The V-segment represents the TCR V gene segment that is to be encoded by the final V-C entry vector and encodes from the ATG start codon of the give V segment to the last Cys codon of the V segment that defines the border of the CDR3 region. The Bsal **←** site is the Type IIS restriction enzyme recognition sequence used during operation of the TORES system to reconstitute a full-length TCR ORF. Action of the Bsal enzyme, wherein the site is orientated to cut in the 5' direction, results in the creation of overhang **‡**1 at the 3' end of the V segment that encompasses the three nucleotides of the last Cys codon of each V segment, and the third nucleotide of the codon preceding that Cys codon. This overhang is standardized among all V segments in a given TORES set. Ultimately, the overhang **‡**1 at the 3' of the V segment ligates with overhang **‡1** at the 5' odeCDR3 in operation of the TORES system to reconstitution of a full-length TCR ORF. All **sp** denote the addition of one or more nucleotides to create the correct spacing between the Type IIS recognition sequences and the target overhang sequences, or to space the Notl recognition and cut site for efficient action.
**Figure 6** **Arrangement of C cloning fragments for the construction of V-C entry vectors**
   Depicted is a representation of the C cloning fragments used to assemble V-C entry vectors of a TORES for human TRA and TRB TCR chains as described in Examples 1 to 4.
   The C cloning fragment is flanked by unique primer bind sequences at 5' and 3' end to facilitate PCR-mediated amplification of the cloning fragments. Bbsl sites represent a specific Type IIS restriction enzyme binding sites used in the assembly of the V-C entry vector, where **→** indicates that the recognition site is orientated to cut in the 3' direction of the site, and **←** indicates that the site is orientated to cut in the 5' direction. The Bbsl **→** site cuts to create the 3' Notl overhang within the Notl 3' fragment. The Bsal **←** site cuts 3' of the stop codon of the C segment to create **overhang*2** at the 3' end of the C segment. **Overhang*2** and the 3' Notl overhang ultimately ligate with **Overhang*2'** of the digested V-C entry vector backbone, and the 5' Notl overhang of the digested V cloning fragment, respectively, in assembly of the V-C entry vector. The Notl 3' fragment represents a 6 nucleotide 3' fragment of the Notl recognition sequence, wherein Notl acts as the negative selection marker to eliminate parental V-C entry vector in operation of the TORES. The complete Notl recognition site is reconstituted with the 5' Notl fragment, provided by the V cloning fragment.
   The C-segment represents the TCR C gene segment that is to be encoded by the final V-C entry vector and encodes from the cytosine residue 5' of the first Glu codon of the C gene segment to the stop codon. The Bsal **→** site is the Type IIS restriction enzyme recognition sequence used during operation of the TORES system to reconstitute a full-length TCR ORF. Action of the Bsal enzyme, wherein the site is orientated to cut in the 3' direction, results in the creation of overhang **‡3** at the 5' end of the C segment. This overhang is standardized among all C segments in a given TORES set. Ultimately, the overhang **‡3** at the 5' of the C segment ligates with overhang **‡3** at the 3' C part of the J donor vector in operation of the TORES system to reconstitution of a full-length TCR ORF. All **sp** denote the addition of one or more nucleotides to create the correct spacing between the Type IIS recognition sequences and the target overhang sequences, or to space the Notl recognition and cut site for efficient action.
**Figure 7** **Arrangement of V-C entry vector backbone for the construction of V-C entry vectors**
   Depicted is a representation of the V-C entry vector backbone used to assemble V-C entry vectors of a TORES for human TRA and TRB TCR chains as described in Examples 1 to 4.
   The circular plasmid DNA contains an origin of replication (Ori) and a positive selection marker #1. This selection marker is used for selection of transformed hosts when isolating clones of V-C entry vector backbone and V-C entry vectors during the assembly, and also for the selection of vectors containing full-length TCR ORFs during operation of the TORES. 5' and 3' genetic elements encode the target elements that flank the final TCR ORF after generation of full-length TCR ORF after its generation by TORES operation. A 5' genetic element might represent a mammalian promoter element to drive the expression of TCR transcripts, and a 3' genetic element might represent a transcriptional terminator sequence. The ACC65I site represents a restriction enzyme recognition sequence, wherein action of the Acc65I enzyme results in the creation of Overhang*1'. This Overhang*1' ligates with Overhang*1 in the digested V cloning fragment during assembly of the V-C entry vector. The Xbal site represents a restriction enzyme recognition sequence, wherein action of the Xbal enzyme results in the creation of Overhang*2'. This Overhang*2' ligates with Overhang*2 in the digested C cloning fragment during assembly of the V-C entry vector. Sp denotes the addition of nucleotides to space the Acc65I and Xbal recognition sites for efficient action of both enzymes.
**Figure 8** **Arrangement of the J receiving cassette fragment**
   Depicted is a representation of a J receiving cassette fragment used in the assembly of J donor vectors of a TORES for human TRA and TRB TCR chains as described in Examples 2 and 3. A J receiving cassette fragment is inserted into a J donor backbone to generate a J receiving cassette vector.
   A J receiving cassette fragment is generated by annealing two complimentary oligonucleotides to create a linear double stranded DNA construct with 4-nucleotide single stranded overhangs at the 5' and 3' ends that are used for insertion of the fragment to the J donor vector backbone. Overhang*3 at the 5' end of the J receiving cassette fragment ligates with Overhang*3' of the digested J donor vector backbone, whereas Overhang*4 at the 3' end ligates with Overhang*4' of the digested J donor vector backbone.
   The Bsal sites represent the Type IIS restriction recognition sites used in the operation of the TORES to assemble a full-length TCR ORF. Bsal **←** site is orientated to cut in the 5' direction, and acts upon the C part sequence to generate Overhang **‡3** at the 3' C part. Bsal **→** site ultimate acts on the J segment part of the J donor vector to create Overhang **‡2** at the 5'end of the J segment part. Bsal **→** element also contains Overhang*5, which is generated by action of the Bbsl on the Bbsl **←** site during assembly of the J donor vector.
   The Bbsl sites represent the Type IIS restriction recognition sites used to assemble the J donor vector. The Bbsl **←** site cuts the Bsal **→** element to generate Overhang*5, whereas the Bbsl **→** site cuts the 5' end of the C part to generate Overhang*6. Overhang*5 and Overhang*6 ultimately ligate with Overhang*5' and Overhang*6' of the J segment part, respectively.
   The C part represents a small portion of the target C gene segment to permit standardized generation of non-palindromic overhangs during operation of the TORES. This C part is ultimate carried at the 3' end of the J segment part, and forms part of the sequence that ligates with the C segment carried by the digested V-C entry vector in operation of the TORES to generate a full-length TCR ORF.
   The Notl site represents a negative selection marker used to eliminate the parental J receiving cassette vector during generation of the J donor vector.
   All sp denote the addition of one or more nucleotides to create the correct spacing between the Type IIS recognition sequences and the target overhang sequences, or to space the Notl recognition and cut site for efficient action.
**Figure 9** **Arrangement of the J donor backbone**
   Depicted is a representation of J a donor vector backbone used in the assembly of J donor vectors of a TORES for human TRA and TRB TCR chains as described in Examples 2 and 3. A J receiving cassette fragment is inserted into a J donor backbone to generate a J receiving cassette vector.
   The circular plasmid DNA contains an origin of replication (Ori) and a positive selection marker #2. This selection marker is used for selection of transformed hosts when isolating clones of J donor vector backbone and J donor vectors during the assembly. Importantly, this positive selection marker is distinct from positive selection marker #1 within the V-C entry vectors, such that parental J donor vectors are eliminated under positive selection on #1 during operation of the TORES to generate full-length TCR ORFs in the context of the V-C entry vector backbone.
   The EcoRI site represents a restriction enzyme recognition sequence, wherein action of the EcoRI enzyme results in the creation of Overhang*3'. This Overhang*3' ligates with Overhang*3 in the annealed J receiving cassette fragment during assembly of the J receiving cassette vector. The Xbol site represents a restriction enzyme recognition sequence, wherein action of the Xbol enzyme results in the creation of Overhang*4'. This Overhang*4' ligates with Overhang*4 in the annealed J receiving cassette fragment during assembly of the J receiving cassette vector. Sp denotes the addition of nucleotides to space the Acc65I and Xbal recognition sites for efficient action of both enzymes.
**Figure 10** **Arrangement of the J receiving cassette vector**
   Depicted is a representation of a J donor vector backbone used in the assembly of J donor vectors of a TORES for human TRA and TRB TCR chains as described in Examples 2 and 3. A J receiving cassette vector is created by insertion of a J receiving cassette fragment into a J donor backbone.
   The circular plasmid DNA contains an origin of replication (Ori) and a positive selection marker #2. This selection marker is used for selection of transformed hosts when isolating clones of J donor vector backbone and J donor vectors during the assembly. Importantly, this positive selection marker is distinct from positive selection marker #1 within the V-C entry vectors, such that parental J donor vectors are eliminated under positive selection on #1 during operation of the TORES to generate full-length TCR ORFs in the context of the V-C entry vector backbone.
   The Bsal sites represent the Type IIS restriction recognition sites used in the operation of the TORES to assemble a full-length TCR ORF. Bsal **←** site is orientated to cut in the 5' direction, and acts upon the C part sequence to generate Overhang **‡3** at the 3' C part. Bsal **→** site ultimate acts on the J segment part of the J donor vector to create Overhang **‡2** at the 5'end of the J segment part. Bsal **→** element also contains Overhang*5, which is generated by action of the Bbsl on the Bbsl ← site during assembly of the J donor vector.
   The Bbsl sites represent the Type IIS restriction recognition sites used to assemble the J donor vector. The Bbsl **←** site cuts the Bsal **→** element to generate Overhang*5, whereas the Bbsl **→** site cuts the 5' end of the C part to generate Overhang*6. Overhang*5 and Overhang*6 ultimately ligate with Overhang*5' and Overhang*6' of the J segment part, respectively.
   The C part represents a small portion of the target C gene segment to permit standardized generation of non-palindromic overhangs during operation of the TORES. This C part is ultimate carried at the 3' end of the J segment part, and forms part of the sequence that ligates with the C segment carried by the digested V-C entry vector in operation of the TORES to generate a full-length TCR ORF.
   The Notl site represents a negative selection marker used to eliminate the parental J receiving cassette vector during generation of the J donor vector.
   All **sp** denote the addition of one or more nucleotides to create the correct spacing between the Type IIS recognition sequences and the target overhang sequences, or to space the Notl recognition and cut site for efficient action.
**Figure 11** **Arrangement of a J segment part**
   Depicted is a representation of a J segment part that is used in the assembly of J donor vectors of a TORES for human TRA and TRB TCR chains as described in Examples 2 and 3. A J segment part is inserted into a J receiving cassette vector to create a J donor vector.
   Annealing complimentary single stranded oligonucleotides to form a linear double stranded DNA construct with single stranded overhangs at either terminus generates a J segment part. Overhang*5' at the 5' terminus anneals with Overhang*5 generated within the J receiving cassette vector digested with Bbsl. Overhang*6' at the 3' terminus anneals with Overhang*6 generated within the J receiving cassette vector digested with Bbsl.
   The J segment part represents the target J gene segment sequence. Depending on the style of the J donor vector being constructed (i.e. short or long) the 5' border of the J segment part is defined differently. For short J donor vectors, the 5' border of the J segment part is defined as the Phe-Ala/Gly or Trp-Gly motifs that are used to define the canonical border between the J and CDR3 portions of a full-length TCR ORF. For long J donor vectors, the 5' border of the J segment part is extended ten to twelve nucleotides 5' of the Phe-Ala/Gly or Trp-Gly motif. This extends the portion of the overall TCR ORF encoded by the J donor vector, and conversely shortens the length of the odeCDR3 required to construct a full-length TCR ORF in operation of the TORES. At the 3' end of the J segment part is encoded a single Adenine residue (A), which represent the first nucleotide of the C fragment. This adenine is excluded from the J receiving cassette vector.

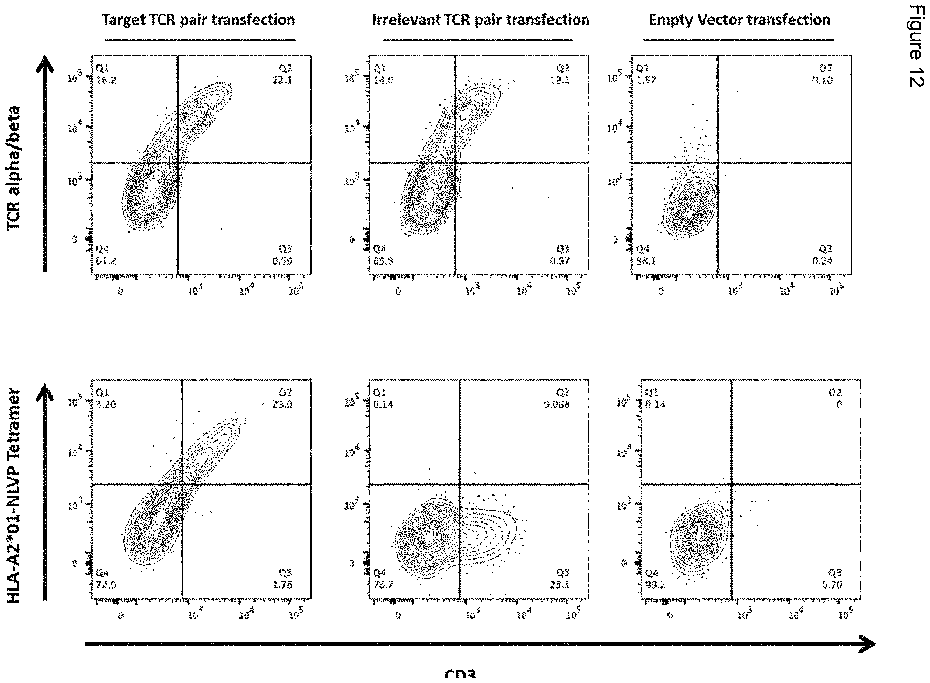

**Figure 12** **Validation of specificity of reconstituted model TCR TRA/TRB pair**
   A model TRA/TRB TCR chain pair with specificity for a HLA-A2*01- restricted HCMV pp65 derived antigen was reconstituted with the TORES system as described in example 7. The two TRA and TRB plasmid products were transiently transfected into a human cell line constitutively expressing CD3 components, but lacking endogenous expression of TCR TRA and TRB chains. 48 hours after the transfection, cells were with antibodies against CD3, TCRalpha/beta and specific HLA-A2*01- NLVPMVATV tetramer and analysed by flow cytometry. The model TCR pair presented on the cell surface as indicated by positive staining with CD3 and TCRalpha/beta antibodies (left, top panel). The model TCR pair also displayed positive staining for the HLA-A2*01-NLVPMVATV tetramer reagent (left, bottom panel), indicating expected specificity of binding. An irrelevant pair of TRA and TRB plasmid constructs was also transfected in parallel (centre panels), as was empty vector (rightmost panels). The irrelevant TCR was presented on the cell surface as indicated by positive staining for CD3 and TCRalpha/beta antibodies (centre, top panel), but displayed no binding for the HLA-A2*01 - NLVPMVATV tetramer reagent (centre, bottom panel). Empty vector transfected cells displayed no staining for either CD3 or TCRalpha/beta antibodies, or for the HLA-A2*01- NLVPMVATV tetramer reagent.
**Figure 13** **Workflow for the identification and reconstitution of a set of TCR TRA/TRB chain pairs from human peripheral blood**
   To demonstrate the use of the TORES system for the reconstitution of native TCR TRA/TRB chain pairs captured from a human specimen, a set of TCRs specific for the HLA-B*07:02 restricted HCMV pp65 antigen, TPRVTGGGAM, was generated via capture of sequence information from single-cell sorting of tetramer-positive cells from a human PBMC fraction. This process is described in Example 8.
   i) A PBMC fraction from a donor with HLA-B*07:02 allele is stained with a HLA-B*07:02-TPRV tetramer reagent. Single CD8⁺ T-cells showing positive staining for tetramer a deposited into single PCR tubes by way of FACS.
   ii) Single cell containing tubes were subjected to a reverse transcription reaction with primer sets anchored in all expressed variable and constant gene segments for both TRA and TRB chains.
   iii) Nested PCR reactions are performed on the reverse transcription product with sets of internal primers, still anchored in all expressed V and C gene segments, independently for the TRA and TRB chains.
   iv) The nested PCR product is subjected to Sanger sequencing
   v) PCR product sequences are aligned against a library of human germline TCR gene segments to determine the V and J (and C) gene segment usage for each amplified TCR chain. The CDR3 sequence from the 5' Cys codon to the 3' Phe-Ala/Gly or Trp-Gly motifs are determined.
   vi) A V-C entry vector is selected from the TORES library corresponding to the determined V and C gene segment usage of each TRA and TRB chain to be reconstituted.
   vii) A J donor vector is selected from the TORES library corresponding to the determined J gene segment usage of each TRA and TRB chain to be reconstituted.
   viii) An odeCDR3 is synthesized corresponding the CDR3 region determined from each TRA and TRB chain to be reconstituted.
   ix) An independent restriction enzyme (RE) / Ligase cycle reaction is assembled for each TRA and TRB TCR to be reconstituted, containing the selected V-C entry vector, J donor vector and synthesized odeCDR3.
   x) The RE/Ligase cycle reaction product is transformed into bacteria and placed under antibiotic selection for the first positive selection marker (i.e. V-C entry vector backbone).
   xi) Clones are propagated and confirmed by Sanger sequencing of the final full-length TCR ORF.

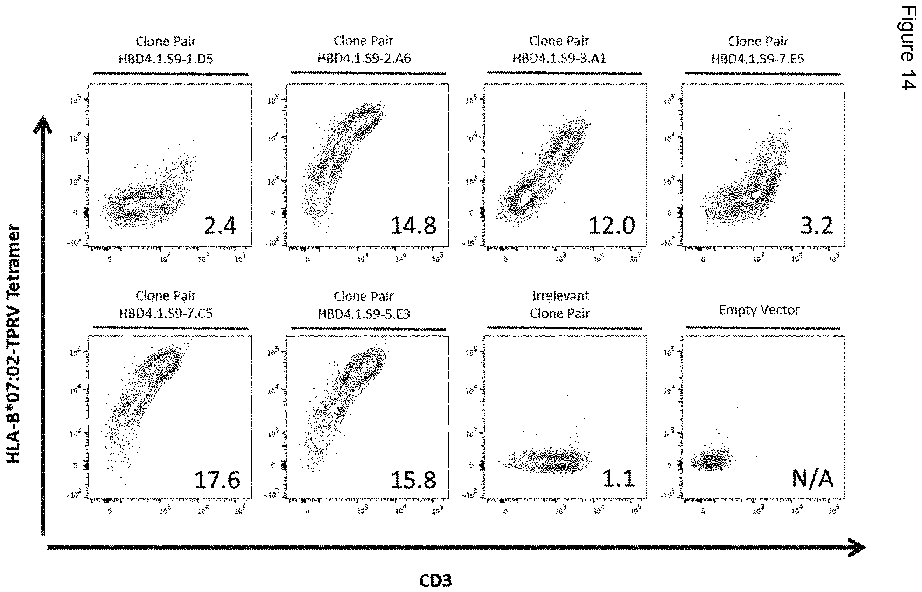

**Figure 14** **Validation of specificity for a set of TCR TRA/TRB chain pairs captured from human peripheral blood and reconstituted with TORES**
   A set of six TCR TRA/TRB chain pairs were captured from human peripheral blood and reconstituted via the workflow outlined in Figure 13 and described in Example 8. The six TCR pairs had expected specificity for the B*07:02-TPRV tetramer reagent used to isolate single CD8⁺ T-cells. Each of the six captured pairs, an irrelevant TCR chain pair, and an empty vector control, were transfected into human cells expressing CD3 components, but lacking endogenous expression of TCR TRA and TRB chains. 48 hours after the transfection, flow cytometric analysis was used to stain the cells with antibodies against CD3, TCR alpha and beta chains and the specificity of the reconstituted and expressed TCR was confirmed by specific HLA-B*07:02-TPRV tetramer reagent. The data are presented as contour plots. Each panel displays CD3 signal on the X-axis and HLA-B*07:02-TPRV tetramer on the Y-axis. Inset at the bottom right of each panel is the ratio of HLA-B*07:02-TPRV tetramer signal of the CD3 positive cells over the CD3 negative cells as an arbitrary unit of the degree of tetramer staining. All six TCR chain pairs presented display some degree of specific tetramer staining compared to the irrelevant TCR pair control.

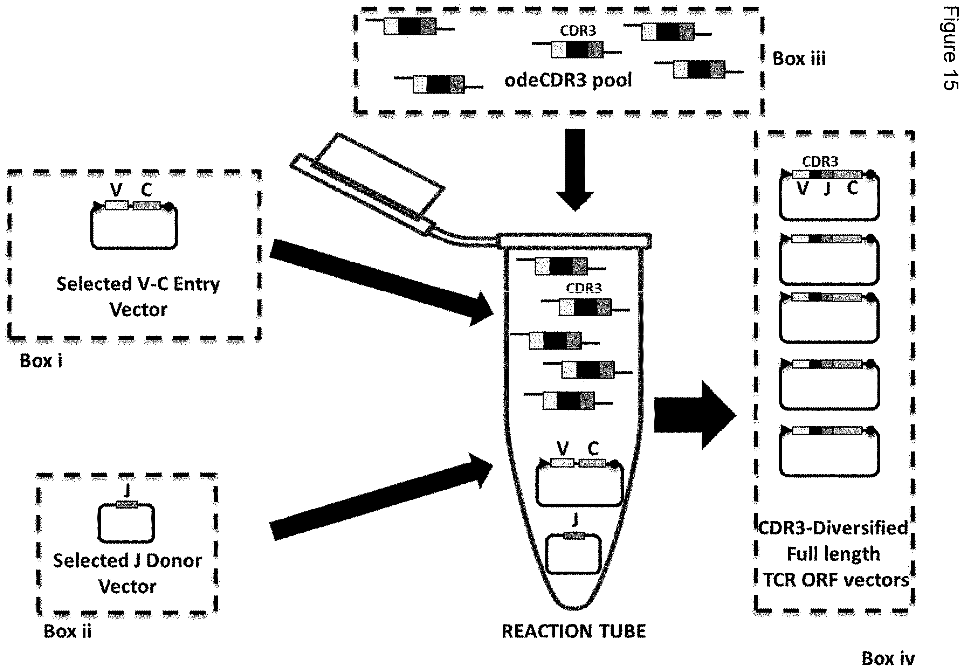

**Figure 15** **Operation of the TORES to generate CDR3-diversified TCR chains**
   Depicted is a schematic representation of the TORES when used to generate full-length TCR chains with diversified CDR3 inserts. A parental TCR is defined with V-J-C usage, and defined CDR3 region sequence. The corresponding single V-C entry vector (box i) and single J donor vector (box ii) are placed in the reaction tube. A pool of odeCDR3 with defined positional nucleotide degeneracy and/or point mutagenesis that changes the coded amino acid sequence is synthesized (Box iii). Such a CDR3 pool could include completely randomized CDR3 sequences within the bounds of the defined odeCDR3 framework, as to create a 'synthetic' full-length TCR ORFs CDR3 with germline V-J-C usage. These three components (Box i, ii and iii) are combined into a single reaction along with appropriate restriction and ligase enzymes. The reaction cycle produces a number of variant reconstituted full-length TCR ORFs, proportional to the number of variant odeCDR3 included, in a single step in the V-C entry vector backbone context (Box iv).

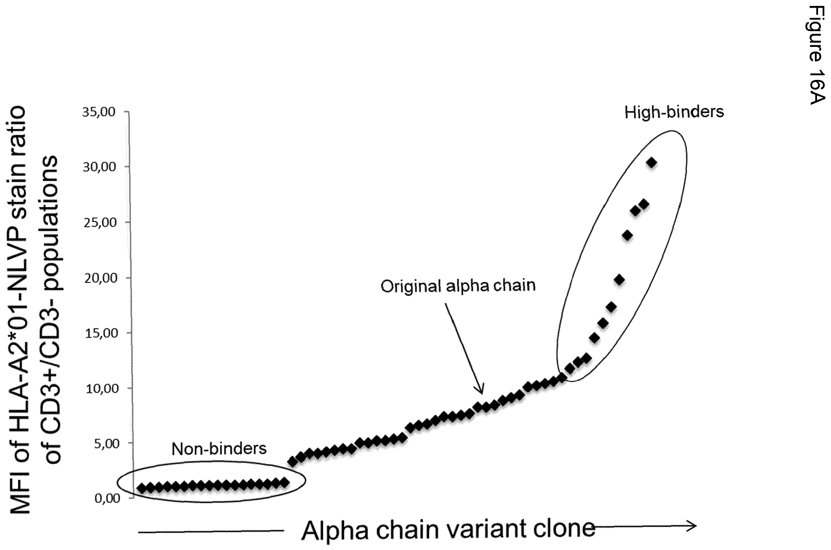

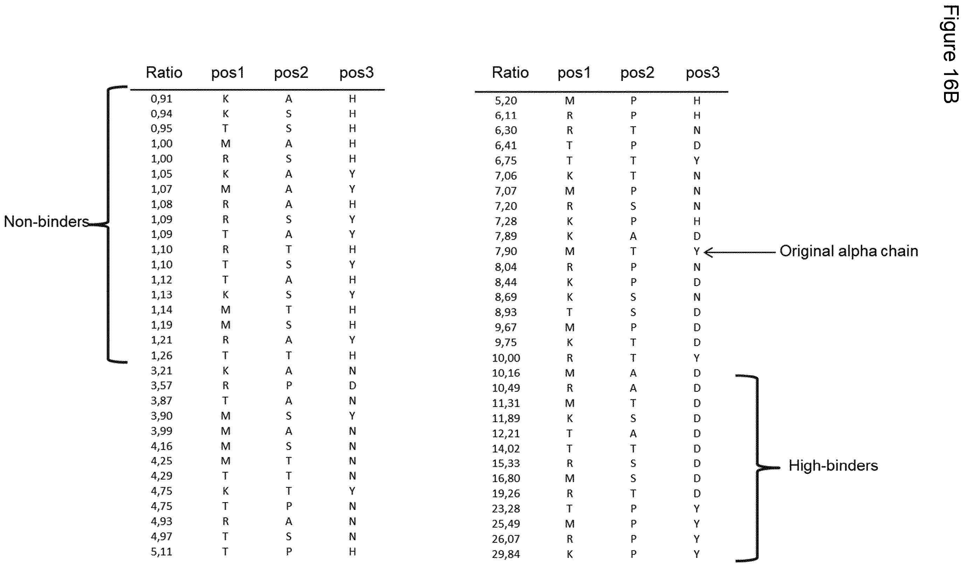

**Figure 16** **A Single round of TCR chain diversification using limited CDR3 degeneracy generates a TCR set with a large spectrum of target affinities**
   The TRA chain of a model TRA/TRB TCR chain pair with specificity for a HCMV pp65 derived antigen as described in example 7, was subject to a CDR3-diversification cycle according to the scheme presented in Figure 15 and as described in Example 9. The TRA chain odeCDR3 was synthesized with 4-fold nucleotide degeneracy at 3 separate positions, altering the codon usage at those three positions. The resulting reaction product contained 64 full-length TRA chain variants, including the parental sequence. Each of the 64 TRA chains was cloned and sequence confirmed.
   A) Each of the 64 TRA chain plasmids was transfected along with the parental TRB plasmid into a human cell line constitutively expressing CD3 components, but lacking endogenous expression of TCR TRA and TRB chains. 48 hours after transfection, the cells were stained with antibodies against CD3 and with a HLA-A2*01 - NLVPMVATV tetramer reagent and analysed by flow cytometry. The ratio of the mean fluorescence intensity (MFI) of HLA-A2*01 - NLVPMVATV tetramer signal for the CD3+ population over the CD3- population was plotted, as an indication of the binding strength of each TCR chain pair variant. The arrow indicates the data point corresponding to the parental TRA. Ovals indicate high-binder and non-binder variants at either end of the spectrum.
   B) Each TRA chain variant is presented in a table with the MFI ratio of HLA-A2*01 - NLVPMVATV tetramer signal for the CD3+ population over the CD3- population, and the amino acid present at each of the three diversified positions (Pos1, 2 and 3), in one letter code. The arrow indicates the data point corresponding to the parental TRA. Brackets indicate high-binder and non-binder variants at either end of the spectrum.

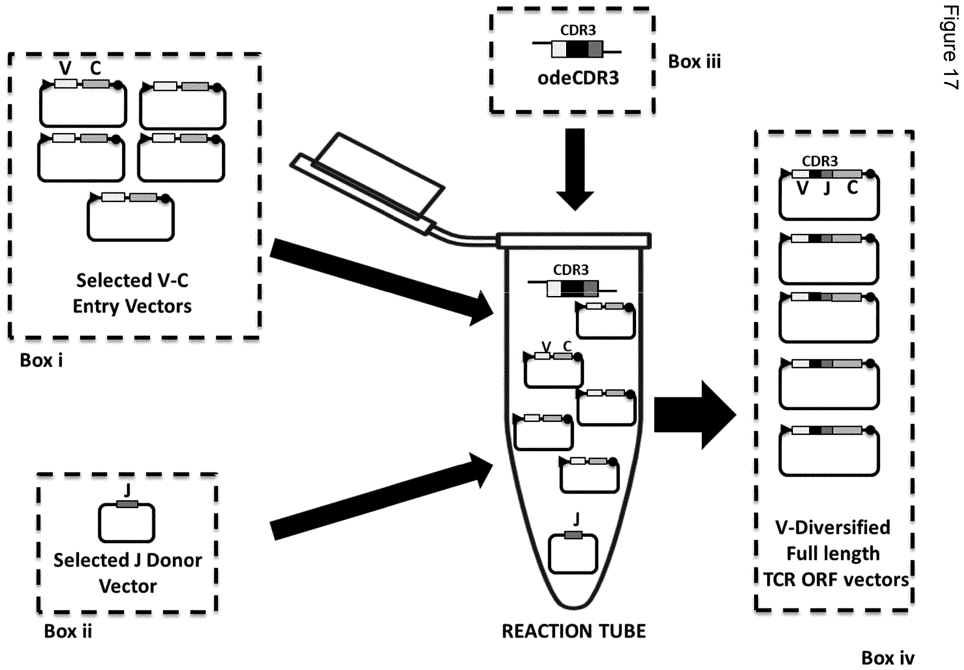

**Figure 17** **Operation of the TORES to generate V-segment diversified TCR chains**
   Depicted is a schematic representation of the TORES when used to generate full-length TCR chains with diversified V-segment usage. A parental TCR is defined with V-J-C usage, and defined CDR3 region sequence. The corresponding single J donor vector (box ii) is placed in the reaction tube, as is the single odeCDR3 synthesized to correspond with parental CDR3 region sequence (Box iii). A selection of V-C entry vectors is also added to the reaction tube, corresponding to the V- and C- segments desired in the product V-segment diversified full length TCR ORF product (Box i). These three components (Box i, ii and iii) are combined into a single reaction along with appropriate restriction and ligase enzymes. The reaction cycle produces a number of variant reconstituted full-length TCR ORFs, proportional to the number of variant V-C entry vectors included, in a single step in the V-C entry vector backbone context (Box iv).

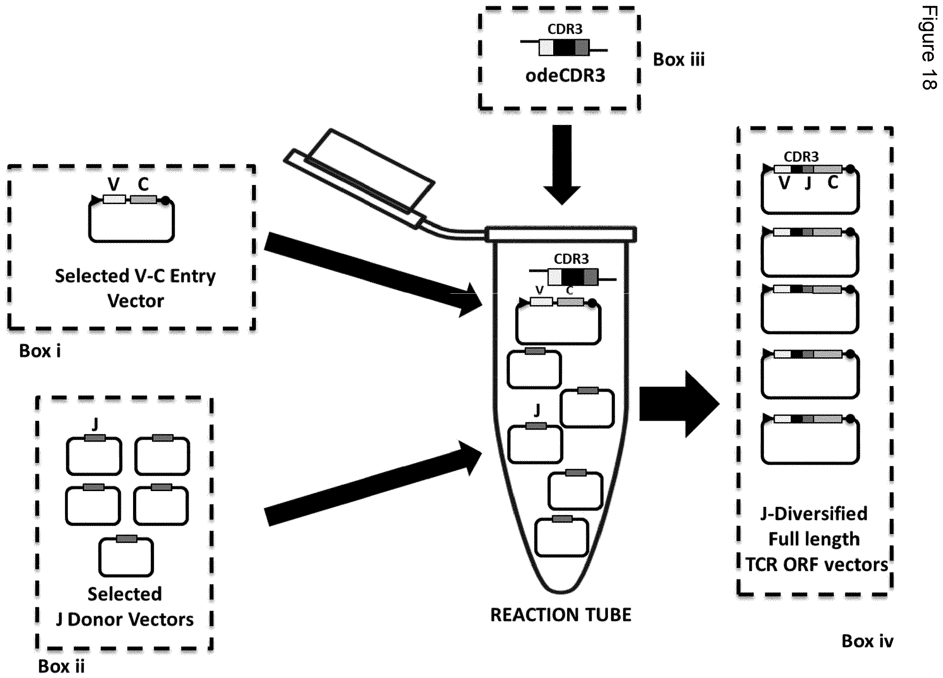

**Figure 18** **Operation of the TORES to generate J-segment diversified TCR chains**
   Depicted is a schematic representation of the TORES when used to generate full-length TCR chains with diversified J-segment usage. A parental TCR is defined with V-J-C usage, and defined CDR3 region sequence. The corresponding single V-C entry vector (box i) is placed in the reaction tube, as is the single odeCDR3 synthesized to correspond with parental CDR3 region sequence (Box iii). A selection of J donor is also added to the reaction tube, corresponding to the J segments desired in the product J-segment diversified full length TCR ORF product (Box ii). These three components (Box i, ii and iii) are combined into a single reaction along with appropriate restriction and ligase enzymes. The reaction cycle produces a number of variant reconstituted full-length TCR ORFs, proportional to the number of variant J donor vectors included, in a single step in the V-C entry vector backbone context (Box iv).

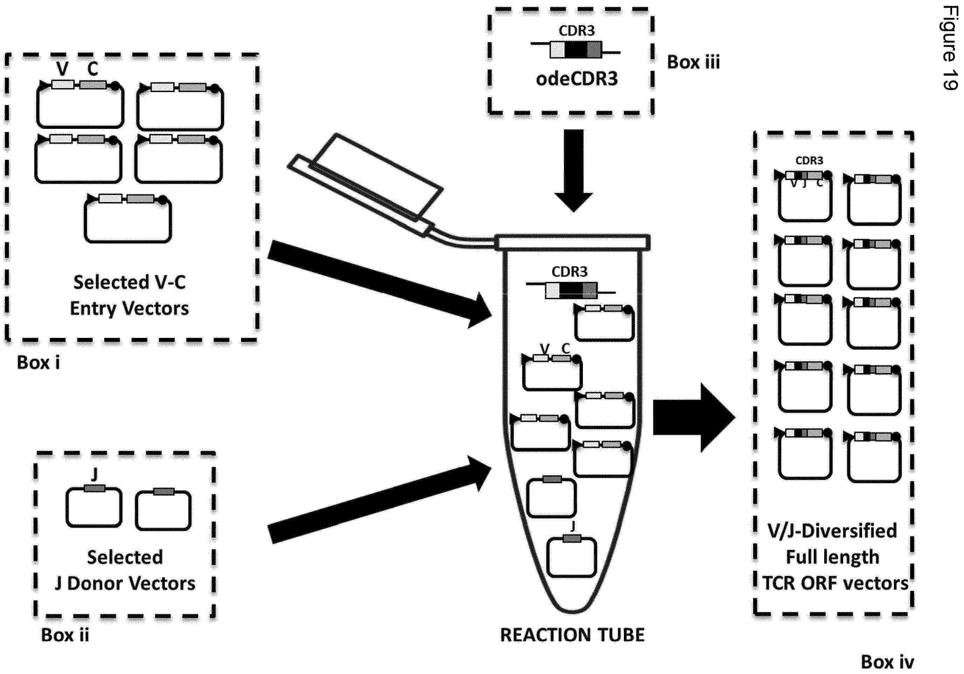

**Figure 19** **Operation of the TORES to generate V/J-segment diversified TCR chains**
   Depicted is a schematic representation of the TORES when used to generate full-length TCR chains with diversified V- and J- segment usage. A parental TCR is defined with V-J-C usage, and defined CDR3 region sequence. The corresponding single odeCDR3 synthesized to correspond with parental CDR3 region sequence (Box iii). A selection of V-C entry vectors and J donor vectors are added to the reaction tube, corresponding to the combination of V- (C-) and J- segments desired in the product V/J-segment diversified full length TCR ORF product (Box ii). These three components (Box i, ii and iii) are combined into a single reaction along with appropriate restriction and ligase enzymes. The reaction cycle produces a number of variant reconstituted full-length TCR ORFs, proportional to the number of V-C and J donor vector combinations possible from those included, in a single step in the V-C entry vector backbone context (Box iv).
**Figure 20****. Schematic of the genetic features of TORES² V-C□ and V-C□ entry vectors.**
   Depicted is a circularized plasmid schematic of a V-C□ (a) and V-C□ (b) entry vectors with minimally required genetic elements depicted as labelled boxes. **Kozak,** refers to consensus sequence that plays a role in the efficient initiation of translation. **V-segment,** refers to a selected sequence encoding a proportion of a TCR variable germline ORF, or mutant/synthetic ORF. **Type IIS #1←,** refers to a first Type IIS restriction enzyme binding site orientated such the enzyme cleaves in the 5' direction. **Type IIS #1 →**, refers to a first Type IIS restriction enzyme binding site orientated such the enzyme cleaves in the 3' direction. **-ve selection #1,** refers to a negative selection element designed to be detrimental to a plasmid harbouring the sequence during the full-length TCR reconstruction reaction, or subsequent selection steps. **C-segment,** refers to a selected sequence encoding a proportion of a TCR constant germline ORF, or mutant/synthetic ORF. **Type IIS #2 ←**, refers to a second Type IIS restriction enzyme binding site orientated such the enzyme cleaves in the 5' direction. The enzyme is different than the first Type IIS. **Type IIS #2 →**, refers to a second Type IIS restriction enzyme binding site orientated such the enzyme cleaves in the 3' direction. The enzyme is different than the first Type IIS. **-ve selection #2,** refers to a negative selection element designed to be detrimental to a plasmid harbouring the sequence during assembly of the full-length bidirectional TCR ORF vector. **-ve selection #2** is different to the first -ve selection. **+ve selection #1,** refers to the first positive selection marker of the two-component system used to convey a selective advantage to the organism harbouring the vector, and which is different to the positive selection marker a second (J donor) vector component (see figure 3). **Ori,** refers to an origin of replication used for the propagation of plasmid within a compatible host. **5' genetic element,** refers to any desired genetic element that provides attributes required for downstream application of the reconstituted full-length TCR, and should be situated 5' of the reconstituted full-length TCR, for example, an RMCE element. **3' genetic element, refers to** any desired genetic element that provides attributes required for downstream application of the reconstructed full-length TCR, and should be situated 3' of the full-length TCR ORF, for example, a second RMCE element.
**Figure 21****. Plasmid schematic of the genetic features of the bidirectional terminator donor (BiT donor) vector.**
   Depicted is a circularized plasmid schematic of a bidirectional terminator donor vector with minimally required genetic features depicted as labelled boxes. **The bidirectional terminator element (T-T),** refers to a DNA sequence encoding a bidirectional terminator. **Type IIS #2 →,** refers to a second Type IIS restriction enzyme binding site orientated such the enzyme cleaves in the 3' direction. **Type IIS #2 ←,** refers to a second Type IIS restriction enzyme binding site orientated such the enzyme cleaves in the 5' direction. **+ve selection #2,** refers to the first positive selection marker of the two-component system used to convey a selective advantage to the organism harbouring the vector, and which is different to the positive selection marker of the first vector component (see figure 20). Ori, refers to an origin of replication used for the propagation of plasmid within a compatible host.
**Figure 22****. Generic description of genetic input, by-products, intermediates and product of the bidirectional two-component vector system to reconstruct a paired TRA and TRB vector.**
   Depicted are the TRA (a), TRB (b) and the bidirectional terminator donor vector (c), when these three components are combined into a single reaction with second Type IIS restriction enzyme and ligase, three reaction by-products (d, e and f), three reaction intermediates (g, h and i) and one reaction product (j) are generated. Input vectors and product of the two-component system are depicted as circularized plasmid schematics with genetic elements depicted as labelled boxes; open plasmid vectors that represent by-product or intermediate are non-circularized plasmid schematics with genetic elements depicted as labelled boxes; and linear DNA are depicted as series of labelled boxes describing genetic elements.
   a) A reconstituted TRA ORF in V-C□ entry vector context as depicted in Figure 20a.
   b) A reconstituted TRA ORF in V-C□ entry vector context as depicted in Figure 20b
   c) A BiT donor vector as depicted in Figure 21
   d) Digestion of the TRA vector (a) with Type IIS #2 restriction enzyme results in a linear DNA TRA vector reaction by-product containing the -ve selection element #2 and the **Type IIS #2 ←** and **Type IIS #2 →** binding site.
   e) Digestion of the TRB vector (b) by the Type IIS #2 restriction enzyme results in a linear DNA TRB vector reaction by-product containing the 5' and 3' genetic elements, the **Type IIS #2 →** and **Type IIS #2 →** elements, the -ve selection element #2, the +ve selection element #1 and origin of replication element.
   f) Digestion of the bidirectional terminator donor vector (c) by the Type IIS #2 restriction enzyme results in a non-circularised plasmid by-product containing all vector elements of the parental plasmid except the excised bidirectional terminator fragment intermediate (i).
   g) Digestion of the TRA vector (a) by the Type IIS #2 restriction enzyme results in a non-circularized plasmid intermediate containing all genetic elements of the parental plasmid except those carried in the excised linear DNA TRA vector reaction by-product (d). Digestion has additionally created two single stranded DNA overhangs, overhang **‡**1-5' and overhang **‡**3-3'**.** Overhang **‡**1-5' is compatible with the overhang **‡**1-3' in the bidirectional terminator intermediate (i). Overhang **‡**3-3' is compatible with the overhang **‡**3-5' in the TRB fragment intermediate (h).
   h) Digestion of the TRB vector (b) by the Type IIS #2 restriction enzyme results in a linear DNA fragment containing the C segment, C part, J segment, CDR3, V□ segment and Kozak sequence flanked by single strand DNA overhangs, overhang ‡2-3' and overhang **‡**3-5'. Overhang ‡2-3' is compatible with the overhang ‡2-5' in the bidirectional terminator intermediate (i). Overhang ‡3-5' is compatible with the overhang ‡3-3' in the open TRA vector intermediate (g).
   i) Digestion of the bidirectional terminator donor vector (c) by the Type IIS #2 restriction enzyme results in a linear DNA fragment intermediate containing the bidirectional terminator. Digestion has additionally created two single stranded DNA overhangs, overhang **‡**1-3' and overhang **‡**2-5'. Overhang **‡**1-3' is compatible with the overhang **‡**1-5' in the open TRA vector intermediate (g). Overhang **‡**2-5' is compatible with the overhang **‡**2-3' in the TRB fragment intermediate (h).
   j) Ligation of all three compatible single stranded DNA overhangs results in the bidirectional paired TRA and TRB vector as circularized plasmid. This plasmid contains all genetic elements of the parental TRA vector (a) with the exception of the excised TRA vector reaction by-product (d). In addition, the full-length TCR ORF vector incorporates the TRB fragment intermediate (h) and the bidirectional terminator intermediate (i). Arrows indicate the approximate points of ligation between compatible single stranded DNA overhangs **‡**1, **‡**2 and **‡**3. Ligation point **‡**1 is comprised of the **‡**1-5' and **‡**1-3' elements donated by the TRA vector reaction intermediate (a) and the bidirectional terminator intermediate (i), respectively. Ligation point **‡**2 is comprised **‡**2-3' and **‡**2-5' elements donated by the TRB fragment intermediate (h) and the bidirectional terminator fragment (i), respectively. Ligation point **‡3** is comprised **‡**3-3' and **‡**3-5' elements donated by the TRA vector reaction intermediate (a) and the TRB fragment reaction intermediate (h), respectively.

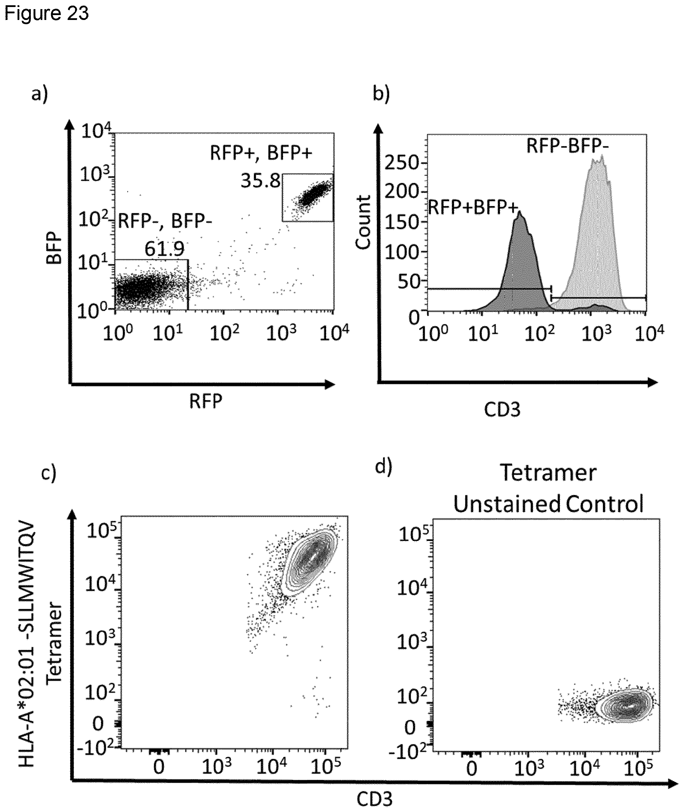

**Figure 23** **Validation of specificity of a model TCR TRA/TRB pair reconstituted using TORES²**
   A model TRA/TRB TCR chain pair with specificity for a HLA-A*02:01-restricted antigen was reconstituted with the TORES² system as described in example 11. The bidirectional TRA/TRB donor vector was used to stably integrate the TCR ORFs into a human cell line constitutively expressing CD3 components but lacking endogenous expression of TRA and TRB chains, and further encoding a genomic receiver site compatible with the RMCE sites contained within the product donor vector that have been contributed by the original V-Cα entry vector. The functionality of the reconstituted full length paired TRA and TRB donor vector generated by the TORES² was determined by surface staining of the CD3 complex, and expected specificity of the reconstituted and expressed TCR was confirmed by specific HLA-A*02:01- SLLMWITQV tetramer staining.
   a) The model TCR pair was integrated into the genome of the engineered TCR presenting cell line containing genomic receiver sites matched to the donor vector. This genomic receiver site contains promoter elements driving two separate fluorescent markers (red fluorescent protein, RFP, and blue fluorescent protein, BFP) in an antiparallel arrangement, interposed by a bidirectional terminator element similar to the final TRA/TRB donor vector product of the TRA/TRB TORES² described in Example 11. Upon transfection and outgrowth of the receiver cells, flow cytometric analysis was conducted to observe the persistence of these florescent reporters. The absence of the genomic receiver sites (RFP-BFP-) therefore indicated that the cells have successfully integrated the TRA/TRB donor vector. While the cells that did not integrate the TRA/TRB donor vector demonstrated presence of the genomic receiver sites (RFP+BFP+). Majority of the cells have successfully integrated the TRA/TRB donor vector sequences into genomic receiver site.
   b) Each of the RFP-BFP- and RFP+BFP+ populations of a) were gated and redisplayed in a second overlay histogram plot. The cells that integrated the bidirectional construct also showed positive staining with CD3 antibody, demonstrating that TCR was expressed on the cell surface (light histogram). The cells that did not integrate the bidirectional construct failed to stain with the CD3 antibody (dark histogram).
   c) and d) The RFP-BFP- cells as analysed in a) and b) were gated and redisplayed as contour plots. Parallel samples of the cells had been stained with HLA-A*02:01-SLLMWITQV tetramer reagent c), or not d), wherein tetramer/CD3 double positivity in c) indicates the expected specificity of the reconstituted and integrated TCR ORF pair.

### Materials and Methods

### DNA Sequencing

All sequencing referred to within the presented examples was conducted by the Sanger method, and conducted by *GATC Biotec AB,* Sweden.

### DNA Synthesis

All DNA synthesis referred to within the presented examples was conducted by *Integrated DNA technologies BVBA,* Belgium.

DNA Fragments >125 bp were synthesised as linear double stranded DNA molecules as a *'gBlock Gene Fragments'* product.

DNA Fragments 15-60 nt were synthesised as single stranded DNA molecules as a *'Custom Oligonucleotide Fragment'* product.

DNA Fragments 61-124 nt were synthesised as single stranded DNA molecules as a *'Ultramer DNA oligonucleotide Fragment'* product.

### Vector library assembly and cloning

The construction of vectors described in the examples comprises a variety of methods well known to those skilled in the art, and specific reaction compositions are outlined in detail in Examples 1 to 3. The following key materials were used in the described procedures:

| **Product** | **Supplier** | **Supplier Number** |
|---|---|---|
| Acc651 | New England BioLabs | R0599L |
| Bbsl HF | New England BioLabs | R3539L |
| DH5alpha competent cells | Thermo Fisher Scientific | 18265017 |
| DNA clean and concentrator kit | Zymo Research | D4030 |
| EcoR1 | New England BioLabs | R3101S |
| Notl | New England BioLabs | R3189L |
| QIAamp DNA Mini kit | Qiagen | 51306 |
| QIAquick Gel Extraction kit | Qiagen | 28704 |
| Qiagen Plasmid Plus Midi kit | Qiagen | 12945 |
| T4 ligase | New England BioLabs | M0202L |
| T4 ligase buffer 10x | New England BioLabs | B0202S |
| Xbal | New England BioLabs | R0145S |
| Xhol | New England BioLabs | R0146S |

### Oligonucleotide duplex encoding CDR3 (odeCDR3) assembly

odeCDR3 were routinely assembled by annealing partially complementary single stranded oligonucleotides. A detailed description of reaction compision and conditions is provide in Example 6. The following key materials were used in the described procedures:

| **Product** | **Supplier** | **Supplier Number** |
|---|---|---|
| T4 ligase buffer 10 x | New England BioLabs | B0202S |
| T4 PNK | New England BioLabs | M0201L |

### TCR reconstitution

Operation of a TORES to reconstitute full-length TCR ORFs is described in detail in Examples 7 to 9. The following key materials were used in the described procedures:

| **Product** | **Supplier** | **Supplier Number** |
|---|---|---|
| Bsal-HF | New England BioLabs | R3535L |
| CutSmart buffer 10 x | New England BioLabs | B7204S |
| DH5alpha competent cells | Thermo Fisher Scientific | 18265017 |
| NotI-HF | New England BioLabs | R3189L |
| QIAamp DNA Mini kit | Qiagen | 51306 |
| T4 Ligase | New England BioLabs | M0202L |
| T4 Ligase buffer 10 x | New England BioLabs | B0202S |

### Fluorescence activated cell sorting (FACS)

Single HLA-multimer positive CD8⁺ T-cells were sorted by FACS for amplification and sequencing of TCR chains. This was achieved through standard cell sorting methodologies using a *BDInflux* instrument. Briefly, cells were stained with HLA-multimer reagent on ice for 10 mins, then with CD3 and CD8 antibodies as markers of CD8⁺ T-cells. Cells with CD3+CD8+Multimer+ signal were sorted to PCR plate pre-loaded with 5 µL of nuclease-free water. Specimens were snap-frozen until subsequent processing. The following key materials were used in the described procedures:

| **Product** | **Supplier** | **Supplier Number** |
|---|---|---|
| CMV-B.07:02-TPRVT-pp65 (PE) | Immudex | WH2136 |
| Anti-CD8 (clone RPA-T8) (BV510) | BD | 563256 |
| Anti-CD3 (clone SK7) (APC-H7) | BD | 560176 |

### Sequencing of TCR alpha and beta chains from single T-cells

Singly FACS-sorted T-cells were subjected to a two-step amplification process that entails a V-region specific primer collection for each TRA and TRB, followed by paired nested PCR reactions that create TRA and TRB amplicons for sequence analysis as described in Example 8. This procedure is described previously (Han et. al. Nat Biotechnol. 2014 32(7): 684-692). The following materials were used in the described procedures:

| **Product** | **Supplier** | **Supplier Number** |
|---|---|---|
| 2x Reaction Mix | Thermo Scientific | 12574035 |
| 5X Phusion HF Buffer | Thermo Fisher Scientific | F-549S |
| dNTPs | Thermo Fisher Scientific | 10297018 |
| Nuclease free water | Qiagen | 129114 |
| Phusion Hot Start II DNA Polymerase | Thermo Fisher Scientific | F-549S |
| SuperScript® III One- Step RT-PCR System with Platinum® Taq High Fidelity DNA Polymerase | Thermo Scientific | 12574035 |

### Example 1

### Design and assembly of TRA V-C entry vector library for native human TRA repertoire

A TORES consists of a V-C entry vector library and J donor vector library for a given TCR chain. When combined with a target odeCDR3 sequence to be inserted into a selected V-J-C context, a full-length TCR ORF can be reconstituted. Through varying odeCDR3 sequence features and/or V/J/C selection, this reconstitution step may also represent a sequence diversification step in TCR ORF engineering workflows. In the present example, the design and assembly of a TRA V-C entry vector library that contains the native human TRA V-C sequence repertoire. This is an example of the generic V-C entry vector type depicted in Figure 2.

The DNA components required for a TRA V-C vector library are:
I. A TRA V cloning fragment for each functional TRA V gene segment encoded in the human genome
II. Single TRA C cloning fragment
III. A V-C entry vector backbone

In the present example, the TRA V and TRA C cloning fragments were synthesized and used to assemble into a target V-C entry vector backbone in a single restriction enzyme and ligase reaction. In the present example, the target V-C entry backbone was designed to permit transient expression of reconstituted TRA ORFs within mammalian cells.

In the present example, the TRA V-C entry vector library is constructed using Type IIS restriction enzyme Bbsl. The Type IIS restriction enzyme used in functioning of the complete TORES to reconstitute full-length TRA ORFs is Bsal.

### Design of synthetic TRA V cloning fragments

The arrangement of genetic elements of the TRA V cloning fragments in the present example is depicted in Figure 5.

Each end of the TRA V cloning fragment encodes a standardized 5' and 3' primer bind DNA sequence of 20 nucleotides for propagation of the overall fragment by PCR.

Proximal to the **5' primer bind** a Bbsl Type IIS restriction enzyme binding site is encoded, wherein the direction of the Bbsl binding site guides the Bbsl enzyme to cut the DNA 3' to its recognition sequence. Overhangs generated by Bbsl enzymatic activity are encoded by **Overhang *1.** This overhang is designed to permit directed ligase-dependent cloning with an arm of the V-C entry vector backbone.

A consensus **kozak** sequence is encoded 5' of the ATG start codon within the TRA V gene segment for efficient initiation of translation of the final reconstituted and expressed TRA mRNA. In the present example, each **TRA V segment** encodes all amino acids from the start methionine residue until its last cysteine (Cys) of the TRA V segment. This Cys residue is generally recognised as a border of the TRA variable gene segments, the deletion of which is rare in naturally occurring recombined and functional TRA chains. Where necessary, native human TRA V consensus sequences have been edited to remove recognition sequences for any restriction enzymes used within assembly or reconstitution operations with the TORES, and also any enzymes used in downstream applications.

To the 3' end of the **TRA V segment** a Bsal Type IIS restriction enzyme binding site is encoded, **Bsal ←.** The direction of the Bsal binding site guides the Bsal enzyme to cut the DNA 5' to its recognition sequence. The resulting overhang sequence is designed to encompass the last cysteine codon of the V segment element and the 3^{rd} nucleotide for amino acid codon preceding the cysteine. Thus the action of Bsal on the designed sequence creates a **TRA V Cys-overhang ‡1** at the 3' end of the **TRA V segment.** In the present example, this **Cys-overhang ‡**1 is standardized among all included TRA V segments to simplify and unify the cloning strategy. Where necessary the nucleotides encoding the TRA V genetic element were changed to encode this standardised overhang but not change the translated amino acid sequence. This **BsaI ←** site is utilized during the full length TRA reconstitution reaction.

In this present example, the V-C entry vector **negative selection marker** is a Notl restriction enzyme binding site. To construct a Notl binding site, two halves of the site are combined when the **TRA V cloning fragment** and **TRA C cloning fragment** are ligated together. The TRA V cloning fragment encodes the **Notl 5'** segment of six nucleotides.

To the 5' end of the 3' **primer bind** sequence encodes a second Bbsl restriction site, that directs Bbsl enzyme to cut the DNA 5' to its recognition sequence, **BbsI ←.** The action of Bbsl on the designed sequence thus creates an overhang of 4 nucleotides, **Notl 5' overhang,** which is designed to be complementary to the overhang generated on the TRA C DNA fragment and reconstitute a Notl binding site upon ligation.

Sp denote nucleotide additions to specific points of the TRA V cloning fragment to achieve the correct spacing of Type IIS restriction enzyme binding site and the cut site, when adjacent to such sites. **Sp** blocks flanking the Notl restriction enzyme binding site sequence have been used to space the Notl binding and cut site appropriately for efficient action. The selection of nucleotides considered the potential impact of DAM methylation of the Bsal binding site.

Full DNA sequences for the **TRA V cloning fragments** in the present example of native human TRA chains are provided as SEQ0001 to SEQ0046. These sequences includes the 5' primer bind and 3' primer bind sequences.

### Design of synthetic TRA C cloning fragment

The arrangement of genetic elements of the TRA C cloning fragments in the present example is depicted in Figure 6.

Each end of the TRA C cloning fragment encodes a standardized 5' and 3' **primer bind** DNA sequence of 20 nucleotides for propagation of the overall fragment by PCR.

Proximal to the **5' primer bind** sequence a Bbsl restriction enzyme recognition site is encoded, such that Bbsl enzyme will cut the DNA 3' to its recognition sequence, **BbsI** →.

The TRA C cloning fragment encodes the **NotI 3'** segment of six nucleotides, which completes a NotI recognition site that will make up the V-C entry vector **negative selection marker.** The adjacent **BbsI** → restriction site acts upon the **NotI 3'** element to create the **Notl 3' overhang** of four necleotides. This overhang is designed to be complementary to the Notl 5' overhang generated on the TRA V DNA fragment and reconstitute a full Notl binding site upon assembly of V-C entry vectors.

To the 3' end of the **Notl 3'** element, the TRA C cloning fragment encodes a Bsal restriction enzyme binding site, **Bsal →.** The direction of the Bsal binding site guides the Bsal enzyme to cut the DNA 5' to its recognition sequence. The resulting overhang sequence is designed to start from the first cytosine of the TRA C genetic fragment, **TRA C overhang ‡3.** This **Bsal →** site is utilized during the full length TRA reconstitution reaction. The **Bsal** → enzyme acts upon the **TRA C segment** encoded in the V-C entry vector to create the necessary **TRA C overhang ‡**3 during reconstitution reactions. A consensus TRA C sequence from the cytosine residue 5' of the first glutamine codon until the stop codon is included in the TRA C cloning fragment in the present example

To the 5' of the **3' primer bind** encodes a Bbsl restriction enzyme recognition sequence, **Bbsl** ←. The direction of the Bbsl binding site guides the Bbsl enzyme to cut the DNA 5' to its recognition sequence. Overhangs generated by Bbsl enzymatic activity are encoded by **Overhang *2.** The design of this overhang permits directed ligase-dependent cloning with an arm of the V-C□ entry vector backbone during assembly.

Sp denote nucleotide additions to specific points of the TRA C cloning fragment to achieve the correct spacing of Type IIS restriction enzyme binding site and the cut site, when adjacent to such sites. **Sp** blocks flanking the Notl restriction enzyme binding site sequence have been used to space the Notl binding and cut site appropriately for efficient action. The selection of nucleotides considered the potential impact of DAM methylation of the Bsal binding site

The full DNA sequence for the **TRA C cloning fragment** in the present example of native human TRA chains are presented as, SEQ0047. This sequence includes the 5' primer bind and 3' primer bind sequences.

### Design of V-C entry vector backbone for transient expression of reconstituted TRA ORF in mammalian cells

In the present example, the V-C entry vector backbone is derived from the pMA plasmid. It encodes a Col E1 origin of replication, **ori,** along with antibiotic resistance beta-lactamase gene, **positive selection #1.** Beta-lactamase confers resistance to the penicillin group of beta-lactam antibiotics such as ampicillin and carbenicillin.

The vector backbone, as depicted in Figure 7, encodes the required genetic elements that confer the appropriate functionality for downstream applications of the fully reconstituted TRA ORF. In this present example, the **5' genetic element** encodes the CMV constitutive mammalian promoter and the **3' genetic element** encodes the SV40pA polyadenylation signal to permit transient expression of the fully reconstituted TRA ORF in a mammalian cell.

In the present example, the vector backbone encodes **Acc65I** and **Xbal** restriction enzyme binding sites that generate **overhang *1'** and **overhang *2',** respectively. **Overhang *1'** is complementary to **overhang *1** within the TRA V cloning fragment (figure 5). **Overhang *2'** is complementary to **overhang *2** within the TRA C cloning fragment (figure 6). These complementary overhangs permit directed cloning of the TRA V and TRA C cloning fragments into the V-C entry vector backbone.

**Sp** feature denotes nucleotides added between the **Acc65I** and **Xbal** restriction enzyme recognition sites required for distancing the two sites for efficient action.

The sequence of the vector backbone from the **5' genetic element** encoding the CMV constitutive promoter, to the **3' genetic element** encoding the SV40pA polyadenylation signal is presented as SEQ0048.

### Method to assemble TRA V-C entry vector library

This method utilizes standard molecular biology techniques to assemble selected **TRA V cloning fragment** (Figure 5) and **TRA C cloning fragment** (Figure 6) into a given **V-C entry vector backbone** (Figure 7) to create a **TRA V-C entry vector** (Figure 2). In this present example, the method performs the restriction enzyme digestion and ligation reaction in a single reaction.

### RE digestion and ligation reaction

100 ng of linear vector backbone (linearised by ACC65I and Xbal digestion)
10 ng of TRA V genetic fragment
20 ng of TRA C genetic fragment
2 µl 10x NEB ligase buffer
0.5 µl of Bbsl
1 µl of T4 DNA ligase
Up to 20 µl of H₂O

### Reaction conditions

Step 1; 2 min at 37°C
Step 2; 3 min at 16°C
Repeat step 1 and 2, 20 times
5 min at 50°C
5 min at 80°C
Return to room temperature

Resulting product is transformed into competent E.coli cells that are selected for carbenicillin-resistant colonies. Plasmids isolated from selected colonies are sequenced to determine correctly assembled constructs. The procedure is repeated for each independent V segment cloning fragment. The resulting constructs make up the TRA V-C entry vector library for use in reconstitution of full-length TRA ORFs for later use in transient expression of said reconstituted TRA in mammalian cells. The sequence of the cloned V-C fragments that make up the TRA V-C entry vector library is presented as SEQ0049 to SEQ0094. The presented sequences include all the Kozac sequence preceding the start codon of the variable segment, to the stop codon of the C segment.

### Example 2

### Design and assembly of TRA J Donor vector library for native human TRA repertoire

A TORES consists of a V-C entry vector library and J donor vector library for a given TCR chain. When combined with a target odeCDR3 sequence to be inserted into a selected V-J-C context, a full-length TCR ORF can be reconstituted. Through varying odeCDR3 sequence features and/or V/J/C selection, this reconstitution step may also represent a sequence diversification step in TCR ORF engineering workflows
In the present example, the design and assembly of a TRA J Donor vector library that contains the native human TRA J sequence repertoire. This is an example of the generic J Donor vector type depicted in Figure 3.

In the present example, a **TRA J receiving cassette fragments** are constructed and inserted to a J donor vector backbone to create a **J receiving cassette vector.** Subsequently, a synthetic **TRA J segment parts** may be assembled into a **TRA J receiving cassette vector** to create the **J Donor vector** library. This flexible multistep assembly method allows rapid and cost effective engineering of J donor segment features, such as variations in J segment length.

The DNA components required for a **TRA J donor vector** library are:
I. TRA J receiving cassette fragment
II. J donor vector backbone
III. TRA J receiving cassette vector
IV. TRA J segment part

### Design of synthetic TRA J receiving cassette fragment

The annealing of two single stranded DNA oligonucleotides is used to generate the receiving site cassette fragment that by design contains 4-nucleotide single-strand overhangs at each end of the DNA fragment; **Overhang *3** and **Overhang *4.** The 4-nucleotide overhangs to permit directed ligase-dependent cloning into a J donor vector backbone to create the **TRA J receiving cassette vector,** depicted in Figure 8.

The pair of Type IIS restriction sites, **BsaI ←** and **Bsal →** are positioned at the 5' and 3' end of the receiving site cassette DNA fragment. The direction of the Bsal recognition site is to guide Bsal enzyme to cut the DNA towards the centre of the construct. These sites are used during TRA ORF reconstitution protocol by generating **overhang ‡2-5'** and **overhang ‡3-3'. Overhang ‡3** is a component of the TRA C part encoded in the receiving cassette fragment, while **overhang ‡2** is defined after the TRA J segment part is cloned *(infra vide).*

The Bbsl pair of Type IIS recognition sites **Bbsl ←** and **Bbsl →** are encoded near the middle of the cassette and used for assembly of the TRA J donor vector, in creating complementary overhangs included in synthesized **TRA J segment parts** (*infra vide*)*.* The 5' Bbsl site, **Bbsl ←**, cuts into the Bsal site to create **overhang *5** at the 3' end of this feature. The 3 Bbsl site, **Bbsl** cuts into the TRA C part element, to create **overhang *6** at the 5' end of this element. These overhangs are encoded within the Bsal and TRA C part features of this construct as to avoid addition of non-native nucleotides that would be incorporated into the final reconstituted TRA ORF.

The region between **Bbsl →** enzyme generated overhang and the **Bsal ←** enzyme generated overhang encodes a proportion of the TRA C region starting from the second nucleotide of the TRA C genetic fragment, **TRA C part.** The motivation for starting from the second nucleotide of the TRA C genetic fragment is because in the present example of a human TRA locus TORES, the resulting overhang is TATC and not a palindromic overhang, which would be the case if the beginning of the TRA C genetic fragment were including (resulting overhang ATAT). A palindromic overhang should be avoided, as it would permit two vector ends joining without the required TRA J segment part insert. The orientation of the **Bbsl →** site permits the in-frame ligase dependent cloning of all TRA J fragments 3' end to the 5' beginning of the TRA C region in the receiving site cassette. The orientation of the **Bsal ←** site permits the in-frame ligase-dependent cloning of the beginning of the TRA C region with the remaining TRA C fragments in the final step of the TRA full length ORF reconstitution protocol using a complete TORES.

Between the two Bbsl binding sites is an 8 nucleotide recognition sequence for the enzyme **Notl.** This restriction site is utilized as a **negative selection** marker to reduce the background of the parental plasmid colonies. This is achieved when Notl enzyme is added after the TRA J gene fragment insertion has been performed. Therefore plasmids correctly cloning a TRA J gene fragment would remain circular in the presence of Notl enzyme but parental plasmids that did not exchange its Notl site for a TRA J gene fragment will be linearized, in turn biasing the bacterial transformation to propagate a complete circular TRA J fragment-containing plasmid.

**Sp** denote nucleotide additions to specific points of the TRA J receiving cassette fragment to achieve the correct spacing of Type IIS restriction enzyme binding site and the cut site, when adjacent to such sites. **Sp** blocks flanking the Notl restriction enzyme binding site sequence have been used to space the Notl binding and cut site appropriately for efficient action. Additional nucleotides have been included to maintain correct reading frame within the final reconstituted full-length TRA. The selection of nucleotides considered the potential impact of DAM methylation of the Bsal binding site.

The full DNA sequence for the **TRA J receiving cassette fragment** oligonucleotides in the present example of native human TRA chains are presented as, SEQ0095 and SEQ0096. Both forward (F1) and reverse (R1) oligonucleotide sequences are listed.

### Design of the J donor vector backbone

The **J donor vector backbone** is used to insert the **TRA J receiving cassette fragment** to create the **TRA J receiving cassette vector.** The backbone is thus carried through to the J Donor vector library. In the final reaction to create TRA full-length ORFs, this backbone is a reaction byproduct (Figure 4e), and thus carries minimal features as depicted in Figure 9.

In the present example, the **J donor vector backbone** encodes a Col E1 origin of replication, ori. The antibiotic resistance is the aminoglycoside 3'-phosphotransferase gene, **positive selection selection #2.** Aminoglycoside 3'-phosphotransferase confers resistance to antibiotic substrates such as kanamycin, streptomycin, neomycin, and gentamicin. This alternate positive selection is used to ensure J donor vectors are not selected for after full-length TCR ORF reconstitution, which are selected on **positive selection #1.**

In the present example the vector **EcoRI** and **Xhol** restriction enzyme binding sites that generates complementary overhang, **overhang *3'** and **overhang *4',** respectively. **Overhang *3'** is complementary with **Overhang *3** contained within the **TRA J receiving cassette fragment. Overhang *4'** is complementary with **Overhang *4** contained within the **TRA J receiving cassette fragment.** These overhangs permits directed cloning of the TRA J receiving cassette fragment.

Sp block denotes nucleotides added between the **EcoRI** and **Xhol** restriction enzyme binding sites for distancing the two sites to ensure efficient action.

In the present example, the J donor backbone is presented as SEQ0097.

### Method to assemble the TRA J receiving cassette vector

This method utilizes standard molecular biology techniques to assemble the given **TRA J receiving cassette fragments** (Figure 8) into a given **J donor vector backbone** (Figure 9) to create a **TRA J receiving cassette vector** (Figure 10). The resulting **TRA J receiving cassette vector** is used to insert **TRA J segment parts** (Figure 11) to construct **TRA J Donor vectors** (Figure 3).

First, the two oligonucleotides to form the TRA J receiving cassette DNA fragment must be phosphorylated and annealed.

### Reaction mix

| | |
|---|---|
| Oligonucleotide (sense strand) (100 µM) | 1 µl |
| Oligonucleotide (anti-sense strand) (100 µM) | 1 µl |
| T4 ligase buffer 10x | 1 µl |
| T4 PNK | 1 µl |
| H₂O | 6 µl |

### Reaction conditions

Incubate for 37°C for 1 hour
Denature at 95°C for 5 min
Anneal sense and anti-sense oligonucleotides by slowly cooling the reaction down to 25°C at 3°C per min

Assembly ligation of TRA J receiving cassette fragments and J donor vector backbone.

### Reaction mix

| | |
|---|---|
| Linear vector backbone | 100 ng |
| Receiving site cassette DNA fragment (0.5 µM) | 2 µl |
| T4 ligase buffer 10x | 2 µl |
| T4 ligase | 0.5 µl |
| H₂O | up to 20 µl |

### Reaction conditions

Incubate for 1 hour at 25°C
Heat inactivate at 65°C for 10 min

Resulting product is transformed into competent E.coli cells and selected for Kanamycin resistant colonies. Resistant colonies are selected to determine correctly assembled constructs. The resulting plasmid is the **TRA J receiving cassette vector.** In the present example, the TRA J receiving cassette vector is presented as SEQ0098 and depicted in Figure 10.

### Design of synthetic TRA J segment parts

Having generated the **TRA J receiving cassette vector** synthetic TRA J segment parts must be generated to insert into this vector. Each TRA J sequence is inserted into an independent **TRA J receiving cassette vector** context to generate the **TRA J donor vector library as** part of the human TRA TORES.

The **TRA J donor vector library** comes in two different forms, comprised of a long or short J segment part. The **short TRA J segment part** encodes all amino acids from the start of the CDR3 border codon. However, considering that the majority of TRA J segments are trimmed back by less than 10 nucleotides during TCR rearrangement, a TRA J donor library containing a longer TRA J germline segment is designed, **long TRA J segment part.** The motivation for a longer TRA J gene fragment library is that a shorter oligonucleotide duplex encoding CDR3 (odeCDR3) would be required for the full length TRA reconstitution, than if the **short TRA J fragment** would be used. Since highly variable sequences are provided as short oligonucleotide duplexes, **odeCDR3,** a shorter CDR3 oligonucleotide synthesis is less likely to contain truncated or mutated oligonucleotide contaminants and therefore reduce the likelihood of oligonucleotide duplex with sequence errors being cloned during full length TRA reconstruction. Furthermore, shorter **odeCDR3** syntheses are cost-saving.

The **TRA J segment parts** are constructed by annealing two single-stranded DNA oligonucleotides designed to contain 4-nucleotide single-strand overhangs at each end of the DNA fragment. The resulting TRA J segment part is depicted in Figure 11.

The 5' overhang designated **Overhang *5'** is complementary to the **Overhang *5** generated within **J donor receiving cassette vector** by Bbsl action. The 3' overhang designated **Overhang *6'** is complementary to the **Overhang *6** generated within **J donor receiving cassette vector** by Bbsl action. This pair of complementary overhangs permits directional cloning of the **TRA J segment parts** into the **TRA J receiving cassette vector.**

The **Short TRA J segment part** encodes all amino acids from the start of the CDR3-J border Phe codon. The CDR3 is defined as the sequence flanked by the C-terminal-conserved Cys of the V region, and Phe of the J region which is part of the Phe-Gly/Ala conserved motif. This conserved Phe-Gly/Ala motif is utilized to standardize the 5' overhangs of the TRA J fragments to TTTG for downstream TRA reconstitution. The exceptions to this standardization in the present example are human TRAJ33 and TRAJ38 that border the CDR3 region with Trp and Gly. The 5' overhangs are TGGG for both TRAJ33 and TRAJ38 in the present example.

The **long TRA J segment part** is designed to encode more amino acids N-terminal of the CDR3 border amino acids. The start point of each long gene fragment is at the first nucleotide of an amino acid codon positioned 10-12 nt from the 5' end of the germline encoded TCR joining element. The 5' end of each **long TRA J segment part** remains identical to that of the **short TRA J segment part.**

To both **short and long TRA J segment parts** an adenine, represented as the A block in figure 11, is added to the 3' end of each TRA J segment part. This adenine represents the first nucleotide of the **TRA C fragment** that is excluded from the **TRA J receiving cassette.**

The sequences of the short TRA J segment parts of the present example of native human J segments are presented as SEQ0099 to SEQ0210 and the long TRA J segment parts SEQ0211 to SEQ0322. In both cases, both forward (F1) and reverse (R1) oligonucleotide sequences are listed.

### Method to assemble the Short or Long J-Donor vector library

This method utilizes standard molecular biology techniques to clone the **Short TRA J segment** or **Long TRA J segment part part** (Figure 11) into the **TRA J receiving cassette vector** (Figure 10) to create **TRA J donor vectors** (Figure 3) containing the **short** or **long TRA J segments.** In this present example, the method performs the restriction enzyme digestion and ligation reaction in a single reaction.

The DNA components required for a J donor vector library is as follows:
I. Short TRA J segment part or Long TRA J segment part
II. J donor receiving cassette vector

### Phosphorylation and Annealing two oligonucleotides to form the TRA J segment part DNA fragment

### Reaction mix

| | |
|---|---|
| Oligonucleotide (sense strand) (100 µM) | 1 µl |
| Oligonucleotide (anti-sense strand) (100 µM) | 1 µl |
| T4 ligase buffer 10x | 1 µl |
| T4 PNK | 1 µl |
| H₂O | 6 µl |

### Reaction conditions

Incubate for 37°C for 1 hour
Denature at 95°C for 5 min
Anneal sense and anti-sense oligonucleotides by slowly cooling the reaction down to 25°C at 3°C per min

### RE digestion and ligation reaction

| | |
|---|---|
| TRA J receiving cassette backbone | 100 ng |
| TRA J DNA fragment (0.5 µM) | 2 µl |
| 10x NEB T4 ligase buffer | 2 µl |
| Bbsl | 0.5 µl |
| T4 DNA ligase | 0.5 µl |
| H₂O | up to 20 µl |

### Reaction conditions

Step 1; 2 min at 37°C
   Step 2; 3 min at 16°C
   Repeat step 1 and 2, 20 times
   5 min at 50°C
   5 min at 80°C
   Return to room temperature
Add 0.5 µl of Notl enzyme and incubate for 30 min at 37°C to linearize parental vector.

Reaction product is transformed into competent E.coli cells and selected for Kanamycin resistance. Selected resistant colonies are sequenced to determine correctly assembled constructs. The resulting constructs make up the TRA J donor vector library, encoding either a long or a short TRA J gene fragment.

The sequence of the resulting libraries, excluding backbone sequence outside of the Bsal recognition sites, are presented as SEQ0323 to SEQ0378 for the **TRA short J donor library** and SEQ0379 to SEQ0434 for the **TRA long J donor library.**

### Example 3

### Design and assembly of TRB V-C entry vector and TRB J Donor vector libraries for native human TRB repertoire

In the above examples, the design and assembly of V-C entry vector and J donor vector libraries for the native human TRA repertoire was described in detail. The overall design and assembly of such vector libraries encoding sequences of the TRB repertoire is essentially the same. In the present example, the design and assembly of the **TRB V-C entry vector** and **TRB J Donor vector libraries** will be briefly outlined in order to construct a TORE or the native human TRB TCR locus.

### Design and assembly of TRB V-C entry vector library for native human TRB repertoire

In the present example, the design and assembly of a TRB V-C entry vector library that contains the native human TRB V-C sequence repertoire. This is an example of the generic V-C entry vector type depicted in Figure 2.

The DNA components required for a TRB V-C vector library are:
I. A TRB V cloning fragment for each functional TRB V gene segment encoded in the human genome
II. TRB C1 or TRB C2 cloning fragment
III. A V-C entry vector backbone

In contrast to the human TRA locus, the human TRB locus encodes two distinct constant segments, TRB C1 and C2. Thus, to capture both constant regions, two V-C entry vector sets are constructed to pair each of the V segments with each C1 and C2 segments.

In the present example, the TRB V and TRB C cloning fragments were synthesized and used to assemble into a target V-C entry vector backbone in a single restriction enzyme and ligase reaction. In the present example, the target V-C entry backbone was designed to permit transient expression of reconstituted TRB ORFs within mammalian cells.

In the present example, the TRB V-C entry vector library is constructed using Type IIS restriction enzyme Bbsl. The Type IIS restriction enzyme used in functioning of the library to reconstitute full-length TRB ORFs is Bsal.

### Design of synthetic TRB V cloning fragments

The arrangement of genetic elements of the TRB V cloning fragments is identical to those of the TRA V cloning fragments described in Example 1, as depicted in Figure 5.

Full DNA sequences for the **TRB V cloning fragments** in the present example of native human TRB chains are presented as SEQ0435 to SEQ481.

### Design of synthetic TRB C cloning fragment

The arrangement of genetic elements of the TRB C cloning fragments is identical to those of the TRA C cloning fragments described in Example 1, as depicted in Figure 6.

The TRB locus encodes two distinct C segments, and both are included in the design of the **TRB V-C entry vector** library.

The full DNA sequence for the **TRB C cloning fragments** in the present example of native human TRB chains are presented as SEQ0482 and SEQ0483.

### Method to assemble TRB V-C entry vector library

The method to assemble the given **TRB V and TRB C cloning fragments** into a given **V-C entry vector backbone** to create a **TRB V-C entry vector** is identical to that described in Example 1. The V-C entry vector backbone used, designed for transient expression of full-length TRA or TRB ORFs in mammalian cells is used in the present example, and in example 1 (SEQ0048).

The sequence of the cloned V-C fragments that make up the TRA V-C entry vector library is presented as SEQ0484 to SEQ0577.

### Design and assembly of TRB J Donor vector library for native human TRB repertoire

In the present example, the design and assembly of a TRB J Donor vector library that contains the native human TRB J sequence repertoire. This is an example of the generic J Donor vector type depicted in Figure 3.

In the present example, a **TRB J receiving cassette fragments** are constructed and inserted to a J donor vector backbone to create a **TRB J receiving cassette vector.** Subsequently, a synthetic **TRB J segment part** may be assembled into a **TRB J receiving cassette vector** to create the **TRB J Donor vector** library. This flexible multistep assembly method allows rapid and cost effective engineering of J donor segment features, such as variations in J segment length.

This procedure follows the same pattern as the TRA J donor vector assembly described in Example 2. However, it should be noted that since the J receiving cassette fragments contain parts of the C segment, the TRA J and TRB J receiving cassette fragments differ with regard to the C part sequence, that must correspond to the respective C gene segments. Moreover, in contrast to TRA J scenario that only requires a single J receiving cassette fragments, the TRB J requires two distinct J receiving cassette fragments to account for the use of alternate C1 and C2 segments.

The DNA components required for a **TRB J donor vector** library are:
I. TRB J C1 or TRB J C2 receiving cassette fragment
II. J donor vector backbone
III. TRB J C1 or TRB J C2 receiving cassette vector
IV. TRB J segment part

### Design of synthetic TRA J receiving cassette fragment

The annealing of two single stranded DNA oligonucleotides is used to generate the **receiving cassette fragments,** which contain 4-nucleotide single-strand overhangs at each end of the DNA fragment, depicted in Figure 8. The 4-nucleotide overhangs permit directed ligase-dependent cloning into a J donor vector backbone to create the **TRB J receiving cassette vector,**

The two receiving cassette fragments required for alternate use of C1 and C2 segments are presented as SEQ0578 and SEQ0581. For each fragment, the forward (F1) and reverse (R1) oligonucleotide sequences are provided.

### Method to assemble the TRB J receiving cassette vectors

The method for assembly of the **TRB J** receiving **cassette vectors** is identical to that of the method for assembly of **TRA J receiving cassette vectors** described in Example 2. The same **J donor vector backbone** (SEQ0097) is used to generate two **TRB J receiving cassette vectors,** each containing one C1 or C2 part corresponding to the alternate C segments for the TRB locus.

The resulting two **TRB J receiving cassette vector** is used to insert **TRB J segment parts** to construct **TRB J Donor vectors.**

The resulting **TRB J receiving cassette vectors** are presented as SEQ0582 and SEQ0583.

### Design of synthetic TRB J segment parts

The **TRB J segment parts** are constructed by annealing two single-stranded DNA oligonucleotides designed to contains 4-nucleotide single-strand overhangs at each end of the DNA fragment. The arrangement of this part and method of assembly are identical to that of the TRA J segment parts, and depicted in Figure 11.

In the case of the **Short TRB J segment part** encodes all amino acids from the start of the CDR3-J border Phe codon. The CDR3 is defined as the sequence flanked by the C-terminal-conserved Cys of the V region, and Phe of the J region, which is part of the Phe-Gly motif conserved across all human TRB J segments. This conserved Phe-Gly motif is utilized to standardize the 5' overhangs of the TRA J fragments to TTTG for downstream TRB reconstitution. Unlike the TRA J segments, there are no exceptions to this standardized overhang in the TRA J segment parts in the present example.

To both **short and long TRB J segment parts** an adenine, represented as the A block in figure 11, is added to the 3' end of each TRB J segment part. This adenine represents the first nucleotide of the **TRB C fragment** that is excluded from the **TRB J receiving cassettes.**

The sequences of the short TRB J segment parts of the present example of native human J segments are presented as SEQ0584 to SEQ0609, and the long TRB J segment parts SEQ0610 to SEQ0635. In both cases, both forward (F1) and reverse (R1) oligonucleotide sequences are listed.

### Method to assemble TRB Short or Long J Donor vector library

The procedure to assemble the TRB J donor libraries is identical to that of the TRA libraries described in Example 2. However, in the case of the TRB libraries, there are four libraries to generate, in contrast to the short and long libraries for the TRA locus segments.

In the case of TRB libraries, each short and long libraries can be constructed to carry each of the alternate C1 and C2 C segments, resulting in four subsets within the TRB J donor library.

The DNA components required for a J donor vector library is as follows:
I. Short TRB J segment part or Long TRB J segment part
II. TRB J C1 or TRB J C2 receiving cassette vector

Following the same procedure as described in example 2, the four resulting subsets within the TRB J donor library are generated. The sequence of the resulting libraries, excluding backbone sequence outside of the Bsal recognition sites is presented.
**TRB C1 short J donor library** presented as SEQ0636 to SEQ0648
**TRB C2 short J donor library** presented as SEQ0649 to SEQ0661
**TRB C1 long J donor library** presented as SEQ0662 to SEQ0674
**TRB C2 long J donor library** presented as SEQ0675 to SEQ0687

### Example 4

### Design of TRA and TRB V-C entry vectors for recombinase mediated cassette exchange application

The design of the TRA and TRB V-C entry vectors in examples 1 and 3, respectively, are such that the full-length TCRs reconstituted with the use of these TORES for each TCR chain may be directly applicable to transient expression in mammalian cells by transfection. The overall design of the TORES allows the V-C entry vector backbone to be exchanged for any backbone suitable for downstream applications, and the necessary V-C entry vector libraries to be rapidly assembled from V and C cloning fragments.

In the present example, a pair of *heterospecific FRT* V-C entry vector backbones are used to assemble TRA and TRB V-C entry vector libraries. Each TRA and TRB V-C entry vector libraries are constructed with vector backbones containing distinct *flippase* recognition target (FRT) sequences. One downstream application of such vectors, is the submission of final reconstituted TRA and TRB pairs to rapid genomic integration into mammalian cells harboring relevant FRT sites.

In the present example, TRA V-C entry vector of the design depicted in Figure 7, contains F14 and F15 FRT sequences as the 5' and 3' genetic elements, respectively. This F14/F15 V-C entry vector backbone sequence is presented as SEQ0688.

This backbone was used to construct TRA V-C entry library of the design outlined in Figure 2, with the use of TRA V cloning fragments (SEQ0001 to SEQ0046) and TRA C cloning fragment (SEQ0047) using methods outlined in Example 1. This construction yielded a TRA V-C entry library with sequences identical to that of the transient expression TRA V-C entry vector library (SEQ0049 to SEQ0094), but flanked by the FRT sequences in the context of the F14/F15 V-C entry vector backbone.

In the present example, TRB V-C entry vector of the design depicted in Figure 7, contains FRT and F3 FRT sequences as the 5' and 3' genetic elements, respectively. This FRT/F3 V-C entry vector backbone sequence is presented as SEQ0689.

This backbone was used to construct TRB V-C entry library of the design outlined in Figure 2, with the use of TRA V cloning fragments (SEQ0435 to SEQ0481) and TRB C cloning fragments (SEQ0482 and SEQ0483) using methods outlined in Example 3. This construction yielded a TRB V-C entry library with sequences identical to that of the transient expression TRB V-C entry vector library (SEQ0484 to SEQ0577), but flanked by the FRT sequences in the context of the FRT/F3 V-C entry vector backbone.
Overall, the TRA and TRB V-C entry vectors in the present example may be used interchangeably in with V-C entry vectors in above examples. In each case, the J donor vector remains unchanged, as does design and provision of odeCDR3 (see Example 6). In effect, different V-C entry vector backbones provide full-length TCR ORFs in desired vector contexts for downstream applications as a direct product of the TCR reconstitution reaction.

### Example 5

### Design of V-C entry vectors and J donor vectors suitable for provision of any V-J-C combinations

In the above examples of V-C entry vectors and J donor vectors, native human TRA and TRC TCR chains are used as a practical demonstration of the TORES. However, it is clear that any native or non-native TCR chain can be employed in the TORES format upon insertion of required TCR chain sequences into the specified vector formats.

To achieve a TORES for any given TCR chain, four sequence elements are required specific for said TCR chain:
X - a variable (V) gene segment fragment
Y - a constant (C) genes segment fragment
Y'- a constant (C) gene segment part
Z - a joining (J) gene segment fragment

According to the above examples, these four forms of sequence element can be assembled into various vector contexts to construct and deploy a TORES for any given V-J-C combination for any given TCR chain. For example, systems for the native human TRG and TRD locus, variant synthetic human TCR chain forms, or native TCR chain forms of an organism other than humans.

### V-C entry vector assembly and final construct

In order to achieve assemble of the V-C entry vector, two intermediates can be constructed that contain the relevant V and C gene segment fragments.

A V cloning fragment, according to Figure 5, is designed to contain required V gene segment fragment in the context of appropriate cloning sites for final V-C entry vector assembly. In the present example, as is also presented in examples 1, 3 and 4, the assembly of V-C entry vectors was achieved through use of Bbsl restriction enzyme sites create target overhangs at the 5' and 3' end as to match complementary overhangs in the V-C entry vector backbone and C cloning fragment, respectively. The exemplified strategy also incorporates a Notl restriction enzyme site as a negative selection marker.

The generic sequence of such a cloning approach is presented as SEQ-0690, wherein the V gene segment fragment is bounded by Bbsl sites at the 5' and 3' ends, and the V gene segment fragment is denoted as XNₙ, wherein X is the designation for the V gene segment, N represents any nucleotide, and n represents the number of nucleotides in said sequence. The V gene segment fragment is bounded by Kozac and spacer nucleotides at the 5' and 3' ends, respectively, and include the translation start codon.

The second intermediate that can constructed to assemble a V-C entry vector libraries for specific TCR chain sequences is the C cloning fragment as depicted in Figure 6. In the present example, as is also presented in examples 1, 3 and 4, the assembly of V-C entry vectors was achieved through use of Bbsl restriction enzyme sites create target overhangs at the 5' and 3' end as to match complementary overhangs in the V cloning fragment and backbone the V-C entry vector, respectively. The exemplified strategy also incorporates a Notl restriction enzyme site as a negative selection marker.

The generic sequence of such a cloning approach is presented as SEQ-0691, wherein the C gene segment fragment is bounded by Bbsl sites at the 5' and 3' ends, and the C gene segment fragment is denoted as YNₙ, wherein Y is the designation for the C gene segment, N represents any nucleotide, and n represents the number of nucleotides in said sequence. The C gene segment fragment is bounded by the cytosine 5' of the first Glu codon of the C gene segment, or equivalent conserved site, and spacer nucleotide(s) at the 5' and 3' ends, respectively, and would include the translation stop codon.

To assemble a V-C entry vector using the above defined V and C cloning fragments, these fragments are combined with a V-C entry vector backbone as depicted in Figure 7 and described in examples 1 and 3. In the present and above examples, this backbone is defined with the **Acc65I** and **Xbal** restriction enzyme binding sites that generate **overhang *1'** and **overhang *2',** respectively. **Overhang *1'** is complementary to **overhang *1** within the V cloning fragment (figure 5). **Overhang *2'** is complementary to **overhang *2** within the C cloning fragment (figure 6). These complementary overhangs permit directed cloning of the V and C cloning fragments into the V-C entry vector backbone.

**Sp** feature denotes nucleotides added between the **Acc65I** and **Xbal** restriction enzyme recognition sites required for distancing the two sites for efficient action.

As outlined in example 4, a V-C entry vector backbone (Figure 7), by way of providing the 5' and 3' genetic elements to the V-C entry vector (Figure 2), ultimately provides the vector context into which a full length TCR ORF is reconstituted (Figure 4h). Thus, a range of selections can be made for the V-C entry vector backbone / V-C entry vector to satisfy a range of downstream applications for the reconstituted TCR ORF.

In the present example, as in examples 1, 3 and 4 above, three different V-C entry vector backbones are provided.

The sequence of the V-C entry vector backbone with the **5' genetic element** encoding the CMV constitutive promoter, and the **3' genetic element** encoding the SV40pA polyadenylation signal is presented as SEQ0048. This vector context permits transient expression of reconstituted TCR ORFs in mammalian cells.

When this transient expression V-C entry vector backbone SEQ0048 is combined with the generic V cloning fragment (SEQ0690) and C cloning fragment (SEQ0691) in the present example, a generalized V-C entry vector can be defined by the sequence SEQ0692.

The sequence of the V-C entry vector backbone with the **5' genetic element** encoding the FTR site, F14, sequence, and the **3' genetic element** encoding the FRT site, F15, sequence is presented as SEQ0688. This vector context permits flippase mediate cassette exchange of reconstituted TCR ORFs into genetic contexts bounded by F14 and F15 heterospecific FRT sites. For example, such an approach can be employed for stable integration of TCR ORFs into mammalian cell lines harboring such heterospecific FRT receiver sites.

When this F14/F15 RCME V-C entry vector backbone SEQ0688 is combined with the generic V cloning fragment (SEQ0690) and C cloning fragment (SEQ0691) in the present example, a generalized V-C entry vector can be defined by the sequence SEQ0693.

The sequence of a second V-C entry vector backbone with the **5' genetic element** encoding the FRT stie, FRT, sequence, and the **3' genetic element** encoding the FRT site, F3, sequence is presented as SEQ0689. This vector context permits flippase mediate cassette exchange of reconstituted TCR ORFs into genetic contexts bounded by FRT and F3 heterospecific FRT sites. For example, such an approach can be employed for stable integration of TCR ORFs into mammalian cell lines harboring such heterospecific FRT receiver sites.

When this FRT/F3 RCME V-C entry vector backbone SEQ0689 is combined with the generic V cloning fragment (SEQ0691) and C cloning fragment (SEQ0692) in the present example, a generalized V-C entry vector can be defined by the sequence SEQ0694.

In the present example, one TCR chain can be reconstituted in one of the two RCME contexts, and a second chain in the other RCME context. For example, a TRA ORF can be reconstituted in the F14/F15 context, and a TRB ORF can be reconstituted in the FRT/F3 context. Thus, paired TRA/TRB constructs can be integrated into a mammalian cell line harboring F14/F15 and FRT/F3 sites by RCME for stable expression of the TRA/TRB pair in said cell line.

### J donor vector assembly and final construct

Described above are generic constructs that may be used to assemble various V and C TCR gene segment combinations into V-C entry vectors as a component of a TORES.

In order to complete a TORES, appropriate J donor vectors are required. As described above in example 2, a J donor vector may be assembled in a multistep process to achieve a J donor system with flexibility to design features of J segments. One important aspect to note is that the J donor vector contributes a C part corresponding to the C segment of a matched V-C entry vector library.

To assemble a J donor vector library, four different constructs are required;
I. J receiving cassette fragment
II. J donor vector backbone
III. J receiving cassette vector
IV. J segment part

In the present example, as in example 2, the J donor vector is assembled using Bbsl restriction sites, and the overall TORES operates with the use if Bsal restriction sites carried in both the resulting J donor vector and matched V-C entry vector.

The J receiving cassette fragment as depicted in Figure 8 importantly contains a C part corresponding to the C segment carried by the matched V-C donor vector library. The generic sequence of such a cloning approach is presented as SEQ0695 to SEQ0696, wherein the C part encoding a 5' portion on the C segment is bounded by a Bbsl recognition site to the 5' and a Bsal site to the 3'. The two presented sequences represent the sense and antisense oligonucleotides annealed to generate the J receiving cassette fragment, wherein the Y' sequences provided are complementary. The C part in the presented sequence is designated Y'Nn, wherein Y' is the designation for the C part matching the Y C segment described above, N represents any nucleotide, and n represents the number of nucleotides in said sequence. The Bbsl site is spaced and orientated to cut in the 3' direction, to create a cloning overhang to match a J segment part to be inserted. The Bsal site is spaced and cuts in the 5' direction to create the C overhang in the final assembly reaction for generating full-length TCR ORFs in operation of the TORES. A second Bsal site at the 5' terminus of the J receiving cassette fragment, cutting in the 3' direction, is also used for the final operation of the TORES. The internal Bbsl site cutting in the 5' direction cerates the second cloning overhang for assembly of the J-donor vector. In the present example, a spaced Notl restriction site is encoded between the Bbsl recognition sites to act as a negative selection marker to eliminate parental fragments from the J donor vector assembly process. The J receiving fragment is assembled by annealing two complimentary oligonucleotides designed to produce two 4-nucleodtide single-stranded overhangs, designated overhang*3 at the 5' terminus, and overhang*4 at the 3' terminus as described in Example 2.

To assemble the intermediate J receiving cassette vector with the above described J receiving cassette fragment, a J donor backbone must be created, as depicted in Figure 9. In the present example, the cloning features of this vector backbone are EcoRI and Xhol restriction sites spaced to allow efficient restriction enzyme action. Digestion of this backbone with EcoRI and Xhol generate overhang*3' and overhang*4' complementary with overhang*3 and overhang*4 in the assembled J receiving cassette fragment. This permits directional cloning of the J receiving cassette fragment into the J donor backbone to create the J receiving cassette vector as described in Example 2. An example of the J donor vector backbone with a positive selection marker different from that of the V-C entry vector described above is presented as SEQ0097.

The J receiving cassette vector obtained above is depicted in Figure 10, and essentially represents the J receiving cassette fragment in the context of the J donor vector backbone, and is presented as SEQ0697, wherein Y' is the designation for the C part matching the C segment that is designated Y and described above, N represents any nucleotide, and n represents the number of nucleotides in said sequence.

It is into the J receiving cassette vector that an array of J segments are inserted to construct a J donor vector library. The design of the J gene segment is depicted in figure 11, wherein a J gene segment is bounded by overhang*5' and overhang*6' to the 5' and 3' ends, respectively. The J segment part is spaced from the 3' overhang by a single adenine nucleotide in the present example, as to maintain enzyme recognition sequences and correct reading frame when inserting the J segment part into the J receiving cassette vector to create a J donor vector, as descried in Example 2. In the present example, a generic sequence for the J segment part is presented as SEQ0698 to SEQ0699 wherein the J gene segment part is denoted as ZNₙ, wherein Z is the designation for the J gene segment, N represents any nucleotide, and n represents the number of nucleotides in said sequence. The two sequences presented represent sense and antisense oligonucleotides annealed to assemble the J segment part, wherein the Z sequences are complementary.

The generic final obtained J donor vector in the present example is presented as SEQ0700, a representation of which is presented in Figure 3. This J donor vector in the present example represents an inserted J segment part and the C part from the J receiving cassette vector. The sequence is presented with two annotated sequence inserts of ZNₙ and Y'Nₙ, wherein Z is the designation for the J gene segment, and Y' is the designation of the C segment part, N represents any nucleotide, and n represents the number of nucleotides in said sequences.

Within all provided sequences, the selection of nucleotides considered the potential impact of DAM methylation of the Bsal binding sites. All additional recognition sequences for assembly and selection enzymes within the vectors were removed. Any such recognition sequences should also be removed from germline or synthetic TCR V, J and C elements inserted into the described fragment and vector contexts.

### Example 6

### Design and generation of oligonucleotide duplex encoding CDR3 (odeCDR3)

In the above example, various formats of TORES are described in the design and generation of matched V-C entry vector and J donor vecror libraries for various applications. The utilization of these V-C entry vector and J donor vector libraries for one-step reconstitution of full-length TCR open reading frames requires an oligonucleotide duplex encoding CDR3 (odeCDR3) construct to be provided in order to complete the target full-length TCR chain sequence (Figure 4c). Once V-C entry vector and J donor vector libraries are generated, these vectors represent stock items that may be drawn upon indefinitely to select desired V-J-C combinations of target full-length TCR chains sequences. In contrast, the odeCDR3 represents a short unique sequence specific to the target full-length TCR ORF.

The present example describes the design and generation of odeCDR3 for use in the native human TRA and TRB vector platforms.

### Design of the TRA odeCDR3

The annealing of two single stranded DNA oligonucleotides generates an **odeCDR3** that contains 4-nucleotide single-strand overhangs at each end of the DNA fragment, as depicted in Figure 4c. The 4-nucleotide overhangs are designed to permit directed ligase dependent cloning to the 3' end of the TRA V segment encoded in the entry vector, **(Overhang ‡1-5')** and the 5' end of the TRA J fragment during TRA reconstitution **(Overhang ‡2-3'). Overhang ‡1-5'** is standardised to **CTGC,** complementary to the standardized **Overhang ‡1-5** encoded in the V segment of the TRA V-C entry vector. In the case of **Overhang ‡2-3',** there are two sequence forms that this can take, which is determined by sequence divergence among J segments from the human TRA locus. For native human TRA J segments TRAJ33 and TRAJ38, the **Overhang ‡2-3'** is standardized to **TGGG,** complementary to the **Overhang ‡2-3** encoded in the J donor vector of these two J segments. For all other human TRA J segments **Overhang ‡2-3'** is standardized to **TTTG,** complementary to the **Overhang ‡2-3** encoded in the J donor vector of these J segments (see Example 2).

### Design of the TRB odeCDR3

As for the TRB odeCDR3, the annealing of two single stranded DNA oligonucleotides generates an **odeCDR3** that contains 4-nucleotide single-strand overhangs at each end of the DNA fragment, as depicted in Figure 4c. The 4-nucleotide overhangs are designed to permit directed ligase dependent cloning to the 3' end of the TRB V segment encoded in the entry vector, **Overhang ‡1-5',** and the 5' end of the TRB J fragment during TRB reconstitution, **Overhang ‡2-3'.** The **Overhang ‡1-5'** is standardised to **TTGC,** complementary to the standardized **Overhang ‡1-5** encoded in the V segment of the TRB V-C entry vectors. In contrast to the TRA odeCDR3 where two alternative **Overhang ‡2-3** forms are required, for the TRB odeCDR3 **Overhang ‡2-3** is standardized to **TTTG,** complementary to the **Overhang ‡2-3** encoded in the J donor vector of all TRB J segments (see Example 3).

### General odeCDR3 design

In general, an odeCDR3 design must be matched to the overhangs the 4-nucleotide overhangs are designed to permit directed ligase dependent cloning to the 3' end of the V segment encoded in the entry vector **(Overhang ‡1-5')** and the 5' end of the J fragment during reconstitution, **(Overhang ‡2-3').**

### Method to generate phosphorylated CDR3 DNA oligonucleotide duplex

### Phosphorylation and Annealing two oligonucleotides to form the odeCDR3

### Reaction mix

| | |
|---|---|
| Oligonucleotide (sense strand) (100 µM) | 1 µl |
| Oligonucleotide (anti-sense strand) (100 µM) | 1 µl |
| T4 ligase buffer 10x | 1 µl |
| T4 PNK | 1 µl |
| H₂O | 6 µl |

### Reaction conditions

Incubate for 37°C for 1 hour
Denature at 95°C for 5 min
Anneal sense and anti-sense oligonucleotides by slowly cooling the reaction down to 25°C at 3°C per min

### Example 7

### Reconstitution of TRA and TRB full-length ORFs from respective V-C entry and J donor vector libraries

### JG9a/b example.

This example describes the steps used for defining the vector library components and odeCDR3 required to reconstitute TRA and TRB full length TCR ORFs given sequence information of the target TCRs. The present example also demonstrates the assembly process of full-length a model TRA and TRB TCR chain pair, and confirms its specificity via transient expression in a human cell model, and staining of surface-presented TCR with specific HLA-multimer reagent.

### Selection of V-C entry vector, J donor vector and odeCDR3

The sequences of all possible germline fragments that are represented in the cloning library are aligned to a TRA or TRB sequences of interest. The genetic fragments with the highest identity to the TRA or TRB sequence determines which V, J and C genetic element will constitute the desired TRA or TRB clonotype sequences. For TRA, the appropriate V-C entry vector is selected based on the determination of the V usage of the desired TRA. For TRB, when sequence coverage is sufficient to determine the V and C usage, the appropriate V-C entry vector will be selected that corresponds to the V usage of the desired TRB clonotype, in addition to whether said clonotype uses TRBC1 or TRBC2.

In the case when both the short and long version of the specific TRAJ or TRB J genetic element align to the TRA and TRB sequence, respectively, the corresponding plasmids encoding the longer genetic elements will be used for the TRA reconstruction.

The odeCDR3 sequence required for the TRA to be synthesised is determined as the region between the 3' end of the TRA V aligned genetic fragment and the 5' end of the aligned TRAJ genetic fragment. The oligonucleotide sense strand requires the additional 5' 4-nucleotide overhang, **Overhang ‡1-5',** CTGC that is universal to the overhang generated on the TRA V entry vector when digested with Bsal, **Overhang ‡1-3'** The complementary oligonucleotide anti-senses strand requires the additional 5' 4-nucleotide overhang, **Overhang ‡2-3',** that is unique to the overhang specifically for the TRAJ vector added to the TRA reconstruction reaction, **Overhang ‡2-5'.**

The CDR3 sequence required for the TRB to be synthesised is determined as the region between the 3' end of the TRB V aligned genetic fragment and the 5' end of the aligned TRB J genetic fragment. The oligonucleotide sense strand requires the additional 5' 4-nucleotide overhang, **Overhang ‡1-5',** TTGC that is universal to the overhang generated on the TRB V entry vector when digested with Bsal, **Overhang ‡1-3'.** The complementary oligonucleotide anti-senses strand requires the additional 5' 4-nucleotide overhang, **Overhang ‡2-3',** that is unique to the overhang specifically for the TRB J vector added to the TCR reconstruction reaction **Overhang ‡2-5'.**

In the present example, a model TCR TRA/TRB pair is used with a known specificity for a HLA-A2*01- restricted antigen. The sequences of the TRA and TRB chains are presented as SEQ701 and SEQ702, respectively.

Based on this full-length sequence it was straightforward to select the appropriate V-C entry and J donor vectors from the TRA and TRB libraries. In the present example, V-C entry vector of the transient expression type were used, that is, with the backbone presented as SEQ0048.

In the present example the TRA V-C entry vector SEQ0088 (from list 0049 to 0094) and J donor vector SEQ0371 (from list 0323 to 0378) were selected.

In the present example the TRB V-C entry vector SEQ0563 (from list 0484 to 0577) and J donor vector SEQ0637 (from list 0636 to 0687) were selected.

The odeCDR3 synthesised for the TRA chain is presented in SEQ703 and SEQ704 as sense and antisense, respectively.

The odeCDR3 synthesised for the TRB chain is presented in SEQ705 and SEQ706 as sense and antisense, respectively.

### Method for full-length reconstitution

For each of the TRA and TRB components selected above, restriction enzyme / ligase cycle reactions were performed as described below.

### RE digestion and ligation reaction

| | |
|---|---|
| V-C entry vector | 100 ng |
| J donor vector | 60 ng |
| odeCDR3 oligonucleotide duplex (0.5 µM) | 2 µl |
| 10x T4 ligase buffer | 2 µl |
| Bsal | 0.5 µl |
| T4 DNA ligase | 0.5 µl |
| H₂O | up to 20 µl |

### Reaction conditions

Step 1; 2 min at 37°C
   Step 2; 3 min at 16°C
   Repeat step 1 and 2, 20 times
   5 min at 50°C
   5 min at 80°C
   Return to room temperature
Add 0.5 µl of Notl enzyme and incubate for 30 min at 37°C

The resulting reaction product was transformed into competent E.coli cells and plated on carbenicillin containing plates.

Screening and sequencing carbenicillin resistant colonies was conducted to determine correctly assembled constructs. Screening of colonies was performed by restriction enzyme diagnostic digest of isolated plasmid DNA, and the expected DNA fragment sizes were observed by DNA electrophoresis. The resulting constructs encode the full length TCR alpha and beta clone sequences.

### Validation of reconstituted TRA and TRB vectors.

To verify the specificity of the reconstituted TCR TRA/TRB pair above, both constructs were transiently transfected into a cell line that expresses all CD3 components, but lacks TRA and TRB expression. This analysis is presented in Figure 12, wherein the reconstituted TCR is transfected along side an irrelevant TCR, and an empty vector control. The empty vector shows no surface staining with antibodies for either TCRalpha/beta or CD3, nor do these control cells stain for HLA-A2*01- NLVPMVATV tetramer reagent (Figure 12, rightmost panels). TCR expression is required for surface presentation of the CD3 complex. The irrelevant TCR stains positively for TCRalpha/beta and CD3, but shows no staining for the HLA-A2*01- NLVPMVATV tetramer reagent (Figure 12, centre panels). This indicates that a TCR is expressed on the cell surface, but is not specific for the target antigen loaded in the HLA-A2*01 tetrameric reagent. The target TCR stains positively for TCRalpha/beta and CD3, and also shows positive staining for the HLA-A2*01- NLVPMVATV tetramer reagent (leftmost panels). This indicates that the reconstituted TCR both presents on the cell surface, and is also specific for the target antigen loaded in the HLA-A2*01 tetrameric reagent, as expected.

### Example 8

### Reconstitution of a set of antigen-specific TCR TRA/TRB chain pairs identified from human peripheral blood

One of the key requirements of upcoming strategies in personalised TCR-based diagnostics and therapeutics is the ability to rapidly capture and characterise antigen-specific TCR chain pairs from individuals. In the present example, a workflow is conducted wherein TCR TRA/TRB chain pairs are amplified and sequenced from single T-cells isolated from peripheral human blood, followed by reconstitution and validation of these pairs via transient expression in mammalian cells.

### Sequencing and reconstitution of TRA and TRB chain pairs

Figure 13 presents the overall workflow. Briefly, a fresh PBMC specimen was collected from a HLA-B*07:02 positive donor, and stained with an HLA-B*07:02-TPRV HLA-multimer reagent. This HLA-multimer is the HLA-B*07:02 allele loaded with a known immunodominant antigen from the HCMV pp65 gene, the full peptide sequence of which is; TPRVTGGGAM. Single HLA,-B*07:02-TPRV multimer-positive cells were FACS sorted into 0.2mL PCR tubes containing 5µL of water. Samples were snapfrozen and stored at -80°C until further processing (Figure 13 step i).

Single-cell isolates were subjected to a two-step amplification process that entails a V-region specific primer collection for each TRA and TRB (Figure 13 step ii), followed by paired nested PCR reactions that create TRA and TRB amplicons (Figure 13 step iii) for sequence analysis (Figure 13 step iv). This procedure is described previously (Han et. al. Nat Biotechnol. 2014 32(7): 684-692).

In the present example, 6 paired clones are presented as an example of downstream processing. The obtained sequences are presented as SEQ0707 to SEQ0718.

These sequences were then aligned against the a library of V, C and J gene segment for their corresponding TRA and TRB chains to determine the V, C and J segment usage of each amplified chain. This sequence analysis step also permits the definition of the CDR3 coding sequence, and thus the definition of odeCDR3 sequence (Figure 13 step v). This sequence analysis permits the selection of V-C entry vectors (Figure 13 step vi) and J donor vectors (Figure 13 step vii) for TCR chain reconstitution. The analysis also permits the synthesis of odeCDR3 for each chain reconstitution reaction (Figure 13 step viii). The selections and sequences are summarised in Table 1.

The table below presents as summary of sequence IDs for identified TCR TRA/TRB pairs in de novo sequencing and reconstitution example

| **Chain** | **SEQ ID** | **V Segment Usage** | **V-C Entry Vector SEQ** | **J Segment Usage** | **J Donor Vector SEQ** | **odCDR3 Forward SEQ** | **odCDR3 Reverse SEQ** |
|---|---|---|---|---|---|---|---|
| TRA | **SEQ0707** | TRAV26-1 | SEQ0082 | TRAJ49 | SEQ0370 | SEQ0719 | SEQ0720 |
| TRB | **SEQ0708** | TRBV27 | SEQ0574 | TRBJ2-7 | SEQ0661 | SEQ0721 | SEQ0722 |
| TRA | **SEQ0709** | TRAV14DV4 | SEQ0071 | TRAJ 12 | SEQ0334 | SEQ0723 | SEQ0724 |
| TRB | **SEQ0710** | TRBV7-9 | SEQ0554 | TRBJ1-1 | SEQ0636 | SEQ0725 | SEQ0726 |
| TRA | **SEQ0711** | TRAV27 | SEQ0084 | TRAJ9 | SEQ0331 | SEQ0727 | SEQ0728 |
| TRB | **SEQ0712** | TRBV27 | SEQ0574 | TRBJ1-2 | SEQ0637 | SEQ0729 | SEQ0730 |
| TRA | **SEQ0713** | TRAV14DV4 | SEQ0071 | TRAJ9 | SEQ0331 | SEQ0731 | SEQ0732 |
| TRB | **SEQ0714** | TRBV27 | SEQ0574 | TRBJ1-2 | SEQ0637 | SEQ0733 | SEQ0734 |
| TRA | **SEQ0715** | TRAV3 | SEQ0052 | TRAJ12 | SEQ0334 | SEQ0735 | SEQ0736 |
| TRB | **SEQ0716** | TRBV7-9 | SEQ0554 | TRBJ2-1 | SEQ0655 | SEQ0737 | SEQ0738 |
| TRA | **SEQ0717** | TRAV17 | SEQ0073 | TRAJ 12 | SEQ0334 | SEQ0739 | SEQ0740 |
| TRB | **SEQ0718** | TRBV7-9 | SEQ0554 | TRBJ2-5 | SEQ0659 | SEQ0741 | SEQ0742 |

Selection of these components allows the assembly of independent restriction enzyme / ligase cycle reactions for each TRA and TRB chain to perform reconstitution of the full-length TCR ORFs (Figure 13 step ix). Each reaction is performed as described in Example 7 above, which includes transformation and antibiotic selection and (Figure 13 step x), then propagation and sequencing confirmation of the resulting reconstituted TCR ORFs (Figure 13 step xi).

### Validation of TRA and TRB chain pairs

To verify the specificity of the set of reconstituted TCR TRA/TRB pairs above, paired constructs were transiently transfected into a cell line that expresses all CD3 components, but lacks TRA and TRB expression, as presented in example 7. This analysis is presented in Figure 14. Cells were transfected with each of the 6 TCR ORF construct pairs, in parallel with an irrelevant TRA/TRB clone pair, and an empty vector control. Cells were analysed by flow cytometry after staining with an antibody against CD3 and the HLA-B*07:02-TPRV tetramer reagent initially used to isolate the single T-cells from the PBMC specimen. The paired constructs in the present example all displayed binding to the target HLA-B*07:02-TPRV tetramer reagent, and quantitative differences are observed. The number inset into each box represents the MFI ratio of CD3 negative over CD3 positive cell events.

### Example 9

### Rapid TCR chain diversification via odeCDR3 degeneracy

The diversification and selection of TCR ORFs is desirable to engineer TCRs chain pairs with altered specificities, affinities and/or signalling capacity. The TORES system is suited to the rapid generation of collections of TCR chains that are systematically altered from the original target sequence. In the present example, an approach of diversifying a model TCR chain pair by including an odeCDR3 to a reconstitution reaction with a defined and limited nucleotide degeneracy at selected codon positions is presented. This approach was used to diversify the TRA chain of the model TCR TRA/TRB pair presented in Example 7. This single-reaction diversification is shown to produce a TCR set with a wide range of affinities to a specific HLA-multimer reagent when presented on the surface of mammalian cells with its natural TRB chain pair. This approach is ideally suited for rapid TCR-engineering using full-length TCR ORFs that may be presented and selected in a functional context of viable mammalian cells.

### Generation of diverse TRA chain collection

Figure 15 presents the overall strategy for generating a sequence-diversified collection of TCR chains in a single reaction by use of an odeCDR3 pool. A single C-V entry vector and J donor vector are selected to represent the target V,J and C gene segments in the final full-length TCR product (Figure 15, box i and box ii). An odeCDR3 pool is generated with selected diversity, such that there are a number of different CDR3 sequences represented in the odeCDR3 pool (Figure 15, Box iii). When all components are combined into a restriction enzyme / ligase cycle reaction, the resulting product are a collection of constructs containing full-length TCR chains of defined V,J C gene segment usage, and a defined diversity in the CDR3 region (Figure 15, Box iv). The number of diversified full-length TCR chains in the final product is directly proportional to the number of odeCDR3 variants in the initial odeCDR3 pool added to the reaction.

In the present example, a model TCR TRA/TRB pair is used with a known specificity for a Human cytomegalovirus (HCMV) antigen presented in HLA-A2*01 (The same pair as Example 7). This antigenic peptide is derived from the HCMV pp65 protein, and the full amino acid sequence of the peptide antigen that is presented in HLA-A2*01 is NLVPMVATV. The sequences of the TRA and TRB chains are presented as SEQ701 and SEQ702, respectively.

In the present example, the TRA chain was the target of sequence diversification, and this was achieved through synthesis of odeCDR3 sense and antisense oligos with nucleotide degeneracy at 3 distinct positions, each altering a separate codon to result in the possibility of 4 different amino acids at each of the three codons. The codons were selected for degeneracy were spaced across the CDR3 loop. The odeCDR3 oligos are presented as SEQ0743 and SEQ0744, wherein degenerate codons are denoted N.

The odeCDR3 oligos were annealed by the method outlined in Example 6, with the 4-fold amino acid degeneracy at 3 separate codon positions resulting in an odeCDR3 product pool with 64 unique sequences, including the original coding sequence (i.e.SEQ0701).

The odeCDR3 was used to assemble the full-length TRA ORFs by the method outlined in Example 7 to create 64 unique TRA ORFs with 4-fold amino acid degeneracy at 3 distinct codon positions. In the present example, the odeCDR3 was synthesised with degenerated nucleotide usage at the indicated positions, and thus reconstitution was performed in a single tube to generate all 64 chain variants. The approach is equally valid whereby a unique odeCDR3 is provided to discrete reactions in parallel. All of the expected clones were prepared and sequence confirmed from a single reaction. Each TRA chain was prepared as a separate plasmid stock for subsequent characterisation.

### Characterisation of diversified TRA chains with TRB chain pair

To characterise the specificity each of the 64 TCR TRA chains derived above were cotransfected with the TRB chain (SEQ0702) into a cell line that expresses all CD3 components, but lacks TRA and TRB expression. These cells were then stained with an antibody against CD3, and a HLA-A2*01- NLVPMVATV multimer reagent and analysed by flow cytometry. This analysis is presented in Figure 16.

Each transfectant population is expressed as the ratio of the mean fluorescence intensity (MFI) of HLA-A2*01- NLVPMVATV multimer signal of CD3 positive over the CD3 negative populations, and plotted as a scatter plot ranked on ascending ratio (Figure 16a). It is clear that the diversified TRA chains create a range of affinities to the HLA-A2*01- NLVPMVATV multimer. The original alpha chain is indicated with an arrow. The majority of diversified clones showed poorer binding than the original. A significant number of non-binders are also observed. Strikingly, a number of clones showed significant increase in HLA-A2*01- NLVPMVATV multimer staining (high-binders), including clones that improve the relative signal by over 3-fold. The clones are also presented as a table of ascending rank of MFI ratio, with the amino acids presented and diversified positions 1, 2 and 3 listed (figure 16b). It can be observed that a Pro at position 2 is highly stabilising of HLA-A2*01- NLVPMVATV multimer binding, and to a lesser degree an Asp at position 3. Conversely, a His at position 3 completely abolishes binding, except in the presence of a Pro at position 2. This positional bias of amino acid substitutions towards increased and decreased binding of the HLA-A2*01- NLVPMVATV multimer strongly suggests a bona fide alteration in binding affinity upon targeted amino acid degeneracy created in the TRA chain using the TORES.

Overall, this example demonstrates that minimal diversity in CDR3 loop introduced in a single step by odeCDR3 degeneracy within the TORES can create a collection of TCRs with diverse binding characteristics towards analyte HLA-antigen complex. This can be directly incorporated into TCR maturation workflows to generate synthetic TCRs with altered characteristics within various TCR engineering scenarios, while still maintaining the full-length native context of the TCR chains along with native V,J and C gene segment usage.

### Example 10

### Rapid TCR chain diversification via V and J segment shuffling

The diversification and selection of TCR ORFs is desirable to engineer TCRs chain pairs with altered specificities, affinities and/or signalling capacity. The TORES system is suited to the rapid generation of collections of TCR chains that are systematically altered from the original target sequence. In the present example, a TCR chain diversification approach is outlined, wherein a single odeCDR3 is provided to a full-length TCR chain reaction with multiple V-C entry vectors, J donor vectors or multiples of both V-C entry vectors and J donor vectors. Such an approach can be used to diversify a given TCR chain using native germline V, J and C segments, while maintaining the possibility of carrying through core CDR3 contacts with cognate HLA-antigen complex in de novo TCR chains. This approach is ideally suited for rapid TCR-engineering using full-length TCR ORFs that may be presented and selected in a functional context of viable mammalian cells.

An example of shuffling V gene segment usage is presented in Figure 17. This schematic example outlines an approach of maintaining a single odeCDR3 and J donor vector usage, with sequence matching the parental TCR chain, while providing a selection of V gene segments via provision of a number of V-C entry vectors to the reconstitution reaction. In the case of TCR chains that have the opportunity of multiple C gene segment usage (e.g. human TRB), this can also be factored into the diversification workflow. The product of a reaction containing single odeCRD3 and J donor vector, and a selection of V-C entry vectors, will be a number of full-length TCR chains containing parental CDR3/J sequence, and each of the provided V (and/or C) gene segments. Such an approach is equally valid to achieve in discrete reactions with a number of selected V-C entry vectors, rather than the pooled reaction depicted in Figure 17.

An example of shuffling J gene segment usage is presented in Figure 18. This schematic example outlines an approach of maintaining a single odeCDR3 and V-C entry vector usage, with sequence matching the parental TCR chain, while providing a selection of J gene segments via provision of a selection of J donor vectors to the reconstitution reaction. The product of a reaction containing single odeCRD3 and V-C entry vector, and a selection of J donor vectors, will be a number of full-length TCR chains containing parental CDR3/V-C sequence, and each of the provided J donor gene segments. Such an approach is equally valid to achieve in discrete reactions with a number of selected J donor vectors, rather than the pooled reaction depicted in Figure 18.

A final example of shuffling both V and J gene segment usage is presented in Figure 19. This schematic example outlines an approach of maintaining a single odeCDR3, with sequence matching the parental TCR chain, while providing a selection of both V(-C) and J gene segments via provision of a selection of both V-C and J donor vectors to the reconstitution reaction. The product of a reaction containing single odeCRD3, and a selection of both V-C entry and J donor vectors, will be a number of full-length TCR chains containing parental CDR3 sequence, and each combination of V(-C) and J donor gene segments provided to the reaction. Such an approach is equally valid to achieve in discrete reactions with a number of selected V-C entry and J donor vectors, rather than the pooled reaction depicted in Figure 19.

### Example 11

### Reconstitution of full length TCR ORFs from TRA and TRB using TORES²

This example describes a TORES² for the human TRA/TRB loci with RMCE sites as 5' and 3' genetic elements, and demonstrates the reconstitution of a model human TCR pair, and subsequent integration of the reconstituted ORFs into a engineered cell line harbouring RMCE sites matched with the 5' and 3' genetic elements of the originating V-Cα entry vector backbone context. The model TCR TRA/TRB pair has known specificity for a HLA-A*02:01-restricted antigen. The model TRA and TRB sequences are represented by SEQ0778 and SEQ0779, respectively. The antigen peptide has the amino acid sequence SLLMWITQV.

### V-C entry vectors, J donor vector, odeCDR3 and Bidirectional terminator for TORES²

The V-Cα, V-Cβ, J donor vector and the oligonucleotide duplex encoding the odeCDR3 are selected using the same selection criteria as outlined in example 7. As described above, the TORES² requires adaptation of the V-Cα and V-Cβ to incorporate distinct Type IIS sites and negative selection elements. The V-Cα and V-Cβ entry vector backbone sequences are represented by SEQ7056 and SEQ0764, respectively. Due to the addition of new restriction sites to these backbone sequences, some of the V-segment sequences require modification to underlying nucleotide sequences compared to those presented for the TORES system above, as to eliminate these new restriction sites from the TCR coding sequence. Modified TRA V-C sequences are represented by SEQ0757 to SEQ0763, and modified TRB V-C sequences by SEQ0765 to SEQ0776. All other TRA and TRB sequences used were as described for the TORES system above.

### Bidirectional terminator donor component

The Bidirectional Terminator Donor vector (BiT donor), provides the bidirectional terminator element introduced in the second step reaction, and which ultimately adjoins the antiparallel TRA and TRB chains reconstituted in the first step reaction. In the present example, the bidirectional terminator element is represented by SEQ0777, and is provided in a suitable vector backbone context.

### Reconstitution reactions

The first restriction enzyme / ligase cycle reaction reaction to reconstitute the target TRA and TRB ORFs is conducted as is described in Example 7. This results in reconstituted TRA and TRB, each within their respective V-C entry backbone contexts. In the second reaction, these two product vectors are combined with the BiT donor vector in a new enzyme ligase cycle reaction to generate the final product vector, encoding the reconstituted TRA and TRB ORFs in an antiparallel sense, and adjoined by the introduced bidirectional terminator element.

### RE digestion and ligation reaction for TORES²

| | |
|---|---|
| Reconstituted TRA ORF vector | 25 ng |
| Reconstituted TRB ORF vector | 25 ng |
| BiT donor vector | 25 ng |
| 10x T4 ligase buffer | 2 µl |
| Esp3I | 0.5 µl |
| T4 DNA ligase | 0.5 µl |
| H₂O | up to 20 µl |

### Reaction conditions

Step 1; 2 min at 37°C
   Step 2; 3 min at 16°C
   Repeat step 1 and 2, 20 times
   5 min at 50°C
   5 min at 80°C
   Return to room temperature
Add 0.5 µl of Sall and Mlul enzymes and incubate for 30 min at 37°C

The resulting reaction product was transformed into competent E.coli cells and plated on carbenicillin containing plates.

Screening and sequencing carbenicillin resistant colonies was conducted to determine correctly assembled constructs. Screening of colonies was performed by restriction enzyme diagnostic digest of isolated plasmid DNA, and the expected DNA fragment sizes were observed by DNA electrophoresis. The resulting constructs encode the full length TCR alpha and beta clone sequences.

### Validation of the reconstituted full length TCR ORF

To verify the functionality of the reconstituted bi-directional TRA/TRB donor vector generated by the TORES² system, the construct was delivered into a cell line that expresses all CD3 components but lacks TRA and TRB expression, and further encodes genomic receiver sites compatible with the RMCE sites contained within the product donor vector and which are contributed by the original V-Cα entry vector, and suitable antiparallel promoter elements at the 5' and 3' ends. The analysis is presented in Figure 23, wherein the reconstituted donor vector is transfected into cells. Cells that have lost the genomic receiver site markers (Figure 23 a, RFP- BFP-) are the cells that express the bidirectional TCR ORF, resulting in CD3 surface expression (Figure 23 b, light grey histogram). While the cells that did not lose the genomic receiver site markers are cells that failed to integrate the bidirectional TCR construct (Figure 23 a) RFP+BFP+) and do not stain for CD3 surface expression (Figure 23 b, dark grey histogram). TCR expression is required for surface presentation of the CD3 complex. All the RFP-BFP- cells that have lost the genomic receiver markers are positive for CD3 surface expression (Figure 23 b, light grey histogram) All the RFP+BFP+ cells that have not lost the genomic receiver markers are negative for CD3 surface expression (Figure 23 b) dark grey histogram). These CD3+ cells expressing the reconstituted TCR also show positive staining for the HLA-A*02:01-SLLMWITQV tetramer reagent (Figure 23 c) compared to the cells that have not been stained with the said tetramer (Figure 23 d). Together the results indicate that the reconstituted TCR both presents on the cell surface and is specific for the target antigen loaded in the HLA-A*02:01 - SLLMWITQV tetramer reagent.

## Claims

1. A combined system comprising two separate components, wherein a first component is a vector carrying variable and constant (V-C) T-cell receptor (TCR) gene segments, and a second component is a vector carrying joining (J) TCR gene segments,
wherein the first component is a V-C entry vector containing
a. origin of replication,
b. a first positive selection marker
c. 5' genetic element, or elements,
d. Kozak Sequence,
e. TCR variable gene segment,
f. a first Type IIS sequence, for site specific recognition and cleavage by a first Type IIS restriction enzyme, wherein the sequence is orientated such that the enzyme cleaves 5' of said recognition sequence and at the 3' end of the TCR variable gene segment,
g. a negative selection marker,
h. a second Type IIS sequence for site specific recognition and cleavage by a second Type IIS restriction enzyme, wherein the sequence is orientated such that the enzyme cleaves to direct cleavage 3' of said recognition sequence and at the 5' end of the TCR constant gene segment,.
i. TCR constant gene segment, and
j. 3' genetic element, or elements,
wherein the second component is a J donor vector containing
a. origin of replication,
b. a second positive selection marker,
c. a third Type IIS sequence for site specific recognition and cleavage by a third Type IIS restriction enzyme, wherein the sequence is orientated such that the enzyme cleaves 3' of said recognition sequence and at the 5' end of the TCR joining gene segment,
d. TCR Joining gene segment,
e. A C-part, corresponding to a small 5' portion of a constant gene segment, and
f. a fourth Type IIS sequence for site specific recognition and cleavage by fourth Type IIS restriction enzyme, wherein the sequence is orientated such that the enzyme cleaves 5' of said recognition sequence and at the 3' end TCR C-part portion of the construct, such that the single strand overhang sequence generated is complimentary with that generated by said second Type IIS sequence.

2. A combined system according to claim 1, wherein the 5' genetic element comprises one or more elements selected from
a. gene cis/acting element,
b. heterospecific recognition site for recombinase enzymes,
c. a 5' homologous recombination arm for a genomic site of interest,
d. a mRNA splice acceptor site,
e. an internal ribosomal entry site, and
f. an epigenetic insulator sequence.

3. A combined system according any of claims 1-2, wherein the negative selection marker is selected from
a. a restriction enzyme recognition site not contained elsewhere in the first component or within the TCR joining gene segment,
b. a bacterial suicide gene, and
c. a reporter element.

4. A combined system according to any of claims 1-3, wherein the 3' genetic element comprises one or more elements selected from
a. a terminator element,
b. heterospecific recognition site for recombinase enzymes,
c. a 3' homologous recombination arm for a genomic site of interest,
d. a mRNA splice donor site,
e. an internal ribosomal entry site, and
f. an epigenetic insulator sequence.

5. A combined system according to any of claims 1-4, wherein the components do not contain any Type IIS sequences further than those defined above, and only contain said negative selection marker.

6. A combined system according to any of the preceding claims further comprising a third component comprising an oligonucleotide duplex encoding CDR3 (odeCDR3), wherein the odeCDR3 has
a. a first single strand overhang sequence complimentary to that generated by cleavage of the first Type IIS restriction site at the 3' end of TCR variable gene segment,
b. a double strand segment encoding a TCR CDR3 region and devoid of negative selection marker, which negative selection marker is as defined in claim 5, and said double strand element is also devoid of any Type IIS restriction sequences of the first or second component, and
c. a second single strand overhang sequence complimentary to that generated by cleavage of the third Type IIS restriction enzyme site at the 5' end of TCR joining gene segment.

7. A combined system according to any of claims 1-5 further comprising a third component comprising an oligonucleotide duplex encoding CDR3 (odeCDR3), wherein the odeCDR3 is a dsDNA molecule or plasmid DNA encoding the CDR3 flanked by two Type IIS recognition and cleavage sites, such that cleavage by said enzymes results in single strand overhangs complimentary to those generated by the first Type IIS cleavage in the V-C entry vector at the 5' end, and with the third Type IIS cleavage of the J donor vector at the 3' end, to obtain a digested product of said odeCDR3 comprising a, b and c as described in claim 6.

8. A combined system according to any of claims 1-7, wherein the first to fourth Type IIS sequences are the same or different.

9. A two-part vector library comprising a library of a V-C entry vectors as defined in claim 1 and a library of J donor vectors as defined in claim 1, wherein the library of V-C entry vectors is
a collection of one or more vectors representing all germline TCR variable and constant gene segments of an organism having such TCRs, or
a collection of one or more vectors representing a collection of variant germline TCR variable and constant gene segments of an organism having such TCRs, such that translated amino acid sequence of the encoded protein is unmodified in relation to the protein sequence encoded by the germline gene segment, or a collection of one or more vectors representing a collection of variant germline TCR variable and constant gene segments of an organism having such TCRs, such that translated amino acid sequence of the encoded protein is modified in relation to the protein sequence encoded by the germline gene segment and wherein
the J donor vector library is a collection of one or more vectors representing germline TCR joining gene segments of an organism having such TCRs, or
a collection of one or more vectors representing variant germline TCR joining gene segments of an organism having such TCRs, such that translated amino acid sequence of the encoded protein is unmodified in relation to the protein sequence encoded by the germline gene segment, or
a collection of one or more vectors representing variant germline TCR joining gene segments of an organism having such TCRs, such that translated amino acid sequence of the encoded protein is modified in relation to the protein sequence encoded by the germline gene segment.

10. A method for in vitro reconstitution of a full length TCR open reading frame (ORF), said method comprising
a. selecting a V-C entry vector,
b. selecting a J donor vector,
c. selecting an odeCDR3,
d. combining a, b and c to react with i) Type IIS restriction enzyme(s) to cleave all Type IIS restriction enzyme recognition and cleavage sites present in the V-C entry vector and in the J donor vector and ii) DNA ligase enzyme and subjecting the combined mix to a thermocycling reaction,
e. transforming the reaction product obtained from step d. into a host organism competent for DNA vector propagation, and
f. propagating said host organism to obtain full length reconstituted TCR open reading frame in the resulting V-C entry vector backbone.

11. A V-C entry vector selected from
sequence SEQ0692,
sequence SEQ0693, and
sequence SEQ0694.

12. A J donor vector represented by the sequence SEQ0700.

13. A library of V-C entry vectors as defined in any of claims 1-8 for use in combination with a library of J donor vectors and an odeCDR3 to reconstitute a full-length TCR ORF, wherein the V-C entry vectors contain Variable and Constant gene segments to recapitulate the gene segment usage of the human TRA locus, represented by the sequences SEQ0049 to SEQ0094; and/or the V-C entry vectors contain Variable and Constant gene segments to recapitulate the gene segment usage of the human TRB locus, represented by the sequences SEQ0484 to SEQ0577.

14. A library of J donor vectors as defined in any of claims 1-8, containing Joining gene segments to recapitulate the gene segment usage of the human TRA locus, represented by sequences SEQ0323 to SEQ0378, wherein the vectors are used in conjunction with an odeCDR3 spanning the entire CDR3 region; and/or
containing Joining gene segments to recapitulate the gene segment usage of the human TRA locus represented by sequences SEQ0379 to SEQ0434,
wherein the vectors are used in conjunction with an odeCDR3 reduced in length by three to four codons.

15. A library of J donor vectors as defined in any of claims 1-8, containing Joining gene segments to recapitulate the gene segment usage of the human TRB locus, represented by sequences SEQ0636 to SEQ0661, wherein the vectors are used in conjunction with an odeCDR3 spanning the entire CDR3 region, and/or
containing Joining gene segments to recapitulate the gene segment usage of the human TRB locus, represented by sequences SEQ0662 to SEQ0687,
wherein the vectors are used in conjunction with an odeCDR3 reduced in length by three to four codons.

16. A library according to claim 14 or 15 for use in combination with a V-C entry vector and an odeCDR3 to reconstitute a full-length TCR ORF.

17. A combined system according to any of claims 1-8, wherein said first component is a V-C entry vector encoding a first TCR chain, the system further comprises a component comprising a Bidirectional Terminator (BiT) donor vector, and a component comprising a V-C entry vector encoding a second TCR chain complimentary to said first TCR chain.

18. A combined system according to claim 17, wherein said V-C entry vector of said first componentfurther comprises
a. a fifth Type IIS sequence for site specific recognition and cleavage by a fifth Type IIS restriction enzyme, wherein the sequence is oriented such that the enzyme cleaves 5' of said recognition sequence, and at the 3' end of the constant gene segment,
b. a negative selection marker located 3' of the fifth Type IIS sequence,
c. a sixth Type IIS sequence for site specific recognition and cleavage by a sixth Type IIS restriction enzyme located 3' of the negative selection marker of b., wherein the sequence is oriented such that the enzyme cleaves 3' of said recognition sequence, and to the 5' of the 3' genetic element(s).

19. A combined system according to claim 17 or 18, wherein said V-C entry vector encoding a second TCR chain complimentary to said first chain and further comprises
a. a seventh Type IIS sequence for site specific recognition and cleavage by a seventh Type IIS restriction enzyme, wherein the sequence is oriented such that the enzyme cleaves 3' of said recognition sequence and at the 5' of the Kozak sequence,
b. an eighth Type IIS sequence for site specific recognition and cleavage by an eighth Type IIS restriction enzyme, wherein the sequence is oriented such that the enzyme cleaves 5' of said recognition sequence, and at the 3' end of the constant gene segment,
c. a negative selection marker located at the 3' of the eighth Type IIS sequence.

20. A combined system according to any of claims 17-19, wherein said BiT donor vector contains an origin of replication,
a. a positive selection marker,
b. a ninth Type IIS sequence for site specific recognition and cleavage by a ninth Type IIS restriction enzyme, wherein the sequence is oriented such that the enzyme cleaves 3' of said recognition sequence and the 5' end of a bidirectional terminator element, d,
c. a bidirectional terminator element encoding two transcriptional terminators in antisense arrangement, and
d. a tenth Type IIS sequence for site specific recognition and cleavage by a tenth Type IIS restriction enzyme, wherein the sequence is oriented such that the enzyme cleaves 5' of said recognition sequence and the 3' of the bidirectional terminator element, d.

21. A combined system according to any of claims 17-20, wherein the first to fourth Type IIS sequences are the same or different.

22. A combined system according to any one of claims 17-20, wherein the fifth to tenth Type IIS sequences are the same or different, and are different from those of the first to fourth Type IIS sequences.

23. A combined system according to any one of claims 17-22 wherein the negative selection marker is selected from
a. a restriction enzyme recognition site not contained elsewhere in the first component or within the TCR joining gene segment,
b. a bacterial suicide gene, and
c. a reporter element,
and wherein the negative selection markers defined in b. of claim 18 and in c. of claim 19 are different from the negative selection marker defined in g. of claim 1, and the negative selection marker defined in g. of claim 1 is different from those in the V-C entry vector of the first component and the V-C entry vector encoding saida second TCR chain complimentary to said first TCR chain.

24. A library of V-C entry vectors as defined in any of claims 17-23, wherein the library comprises a first and second V-C entry vector library, each containing
a collection of one or more vectors representing all germline TCR variable and constant gene segments of an organism having such TCRs, or
a collection of one or more vectors representing a collection of variant germline TCR variable and constant gene segments of an organism having such TCRs, such that translated amino acid sequence of the encoded protein is unmodified in relation to the protein sequence encoded by the germline gene segment, or
a collection of one or more vectors representing a collection of variant germline TCR variable and constant gene segments of an organism having such TCRs, such that translated amino acid sequence of the encoded protein is modified in relation to the protein sequence encoded by the germline gene segment,
wherein the first and second V-C entry vector libraries encode reciprocal TCR chains.

25. A library of V-C entry vectors according to claim 24 for use to provide a pair of full length TCR open reading frames (ORFs), encoded in a antiparallel arrangement in a single product construct.

26. A method for in vitro reconstitution of a pair of full length TCR open reading frames (ORFs), encoded in an antiparallel arrangement in a single product construct, said method comprising
a. selecting a V-C entry vector for each TCR chain,
b. selecting a J donor vector for each TCR chain,
c. selecting an odeCDR3 for each TCR chain,
d. combining a, b and c for each TCR in an independent reaction to react with i) Type IIS restriction enzyme(s) to cleave the first to fourth Type IIS restriction enzyme recognition and cleavage sites present in the V-C entry vectors and in the J donor vectors and ii) DNA ligase enzyme and subjecting the combined mixes to thermocycling reactions to obtain a first and second reaction product,
e. combining both reaction products from step d. with a BiT donor vector as defined in claim 17 with i) Type IIS restriction enzyme(s) to cleave the fifth to tenth Type IIS restriction enzyme recognition and cleavage sites and ii) DNA ligase enzyme, and subjecting the combined reaction products to thermocycling reactions to obtain a third reaction product,
f. transforming the third reaction product from step e. into a host organism competent for DNA vector propagation, and
g. propagating said host organism to obtain full length reconstituted TCR open reading frame in the resulting V-C entry vector backbone.

27. A pair of V-C entry vectors represented by SEQ ID NO: 0756 and 0764.

28. A pair of V-C entry vectors according to claim 27, for use in recombinase mediated cassette exchange with genetic targets with matched heterospecific recombinase sequences.

29. A bidirectional terminator element, represented by the sequence SEQ0777.

## Patentansprüche

1. Ein kombiniertes System, umfassend zwei getrennte Komponenten, wobei eine erste Komponente ein Vektor ist, der variable und konstante (V-C) T-Zell-Rezeptor (TCR) Gensegmente trägt, und eine zweite Komponente ein Vektor ist, der verbindende (J) TCR-Gensegmente trägt,
wobei die erste Komponente ein V-C-Eintrittsvektor ist, enthaltend
a. einen Replikationsursprung,
b. einen ersten positiven Selektionsmarker,
c. 5'-genetisches Element, oder Elemente,
d. Kozak-Sequenz,
e. TCR variables Gensegment,
f. eine erste Typ IIS-Sequenz, zur ortsspezifischen Erkennung und Spaltung durch ein erstes Typ IIS Restriktionsenzym, wobei die Sequenz so orientiert ist orientiert ist, so dass das Enzym 5' jener Erkennungssequenz und am 3'-Ende des variablen TCR-Gensegments spaltet,
g. einen negativen Selektionsmarker,
h. eine zweite Typ-IIS-Sequenz zur ortsspezifischen Erkennung und Spaltung durch ein zweites Typ IIS Restriktionsenzym, wobei die Sequenz so orientiert ist, so dass das Enzym direkt 3' jener Erkennungssequenz und am 5'-Ende des konstanten TCR-Gensegments spaltet,
i. TCR-konstantes Gensegment, und
j. 3'-genetisches Element, oder Elemente,
wobei die zweite Komponente ein J-Donor-Vektor ist, enthaltend
a. Replikationsursprung,
b. einen zweiten positiven Selektionsmarker,
c. eine dritte Typ IIS-Sequenz zur ortsspezifischen Erkennung und Spaltung durch ein drittes Typ-IIS-Restriktionsenzym, wobei die Sequenz so orientiert ist, so dass das Enzym 3' jener Erkennungssequenz und am 5'-Ende des TCR verbindenden Gensegments spaltet,
d. TCR-verbindendes Gensegment,
e. einen C-Teil, der einem kleinen 5'-Abschnitt eines konstanten Gensegments entspricht, und
f. eine vierte Typ IIS-Sequenz zur ortsspezifischen Erkennung und Spaltung durch ein viertes Typ IIS Restriktionsenzym, wobei die Sequenz so orientiert ist, so dass das Enzym 5' jener Erkennungssequenz und am 3'-Ende des TCR C-Teils des Konstrukts spaltet, so dass die erzeugte Einzelstrang-Überhangsequenz komplementär zu derjenigen ist, die durch jene zweite Typ IIS-Sequenz erzeugt wird.

2. Ein kombiniertes System gemäß Anspruch 1, wobei das 5'-genetische Element eines oder mehrere Elemente umfasst, ausgewählt aus
a. Gen cis/wirkendes Element,
b. heterospezifischer Erkennungsstelle für Rekombinase-Enzyme,
c. einem 5'-homologen Rekombinationsarm für eine genomische Stelle von Interesse,
d. einer mRNA-Spleißakzeptorstelle,
e. einer internen ribosomalen Eintrittsstelle, und
f. einer epigenetischen Isolatorsequenz.

3. Ein kombiniertes System gemäß einem der Ansprüche 1 bis 2, wobei der negative Selektionsmarker ausgewählt wird aus
a. einer Restriktionsenzym-Erkennungsstelle, die nicht an anderer Stelle in der ersten Komponente oder innerhalb des TCR verbindenden Gensegments enthalten ist,
b. einem bakteriellen Suizidgen, und
c. einem Reporterelement.

4. Ein kombiniertes System gemäß einem der Ansprüche 1 bis 3, wobei das 3'-genetische Element ein oder mehrere Elemente umfasst, ausgewählt aus
a. einem Terminator-Element,
b. einer heterospezifischen Erkennungsstelle für Rekombinase-Enzyme,
c. einem 3'-homologen Rekombinationsarm für eine genomische Stelle von Interesse
d. einer mRNA-Spleißdonorstelle,
e. einer internen ribosomalen Eintrittsstelle, und
f. einer epigenetischen Isolatorsequenz.

5. Ein kombiniertes System gemäß einem der Ansprüche 1-4, wobei die Komponenten keine weiteren Typ-IIS-Sequenzen als die oben definierten enthalten und nur jenen negativen Selektionsmarker enthalten.

6. Ein kombiniertes System gemäß einem der vorhergehenden Ansprüche, das ferner eine dritte Komponente umfasst, die einen Oligonukleotid-Duplex umfasst, der CDR3 (odeCDR3) kodiert, wobei der odeCDR3 folgendes aufweist
a. eine erste Einzelstrang-Überhangsequenz, die komplementär zu derjenigen ist, die durch Spaltung der ersten Typ-IIS-Restriktionsstelle am 3'-Ende des variablen TCR Gensegments erzeugt wird,
b. ein Doppelstrangsegment, das eine TCR CDR3 Region kodiert und frei von negativem Selektionsmarker ist, wobei der negative Selektionsmarker wie in Anspruch 5 definiert ist, und jenes Doppelstrangelement auch frei von sämtlichen Typ-IIS Restriktionssequenzen der ersten oder zweiten Komponente ist, und
c. eine zweite Einzelstrang-Überhangsequenz, die komplementär zu derjenigen ist, die durch Spaltung der dritten Typ-IIS-Restriktionsenzymstelle am 5'-Ende des TCR verbindenden Gensegments erzeugt wird.

7. Ein kombiniertes System gemäß einem der Ansprüche 1-5, das ferner eine dritte Komponente umfasst, umfassend einen Oligonukleotid-Duplex, der für CDR3 (odeCDR3) kodiert, wobei das odeCDR3 ein dsDNA-Molekül oder Plasmid-DNA ist, das für CDR3 kodiert, das von zwei Typ-IIS-Erkennungs- und -Schnittstellen flankiert wird, so dass die Spaltung durch jene Enzyme zu Einzelstrangüberhängen führt, die komplementär zu denen sind, die durch die erste Typ IIS-Spaltung im V-C-Eintrittsvektor am 5'-Ende und mit der dritten Typ IIS-Spaltung des J-Donor-Vektors am 3'-Ende erzeugt werden, um ein verdautes Produkt jenes odeCDR3 zu erhalten, das a, b und c umfasst, wie in Anspruch 6 beschrieben.

8. Ein kombiniertes System gemäß einem der Ansprüche 1-7, wobei die erste bis vierte Typ IIS-Sequenz gleich oder verschieden sind.

9. Eine zweiteilige Vektorbibliothek, umfassend eine Bibliothek von V-C-Eintrittsvektoren wie in Anspruch 1 definiert und eine Bibliothek von J-Donor-Vektoren wie in Anspruch 1 definiert, wobei die Bibliothek von V-C-Eintrittsvektoren
eine Sammlung von einem oder mehreren Vektoren ist, die alle Keimbahn TCR-variablen und konstanten Gensegmente eines Organismus, der solche TCRs aufweist, darstellen, oder
eine Sammlung von einem oder mehreren Vektoren, die eine Sammlung von varianten Keimbahn TCR-variablen und konstanten Gensegmenten eines Organismus, der solche TCRs aufweist, darstellen, so dass die translatierte Aminosäuresequenz des kodierten Proteins in Bezug auf die durch das Keimbahn-Gensegment kodierte Proteinsequenz unmodifiziert ist, oder
eine Sammlung von einem oder mehreren Vektoren, die eine Sammlung von varianten Keimbahn TCR-variablen und konstanten Gensegmenten eines Organismus, der solche TCRs aufweist, darstellen, so dass die translatierte Aminosäuresequenz des kodierten Proteins in Bezug auf die durch das Keimbahn-Gensegment kodierte Proteinsequenz modifiziert ist und wobei
die J-Donor-Vektor-Bibliothek eine Sammlung von einem oder mehreren Vektoren ist, die Keimbahn TCR-verbindende Gensegmente eines Organismus, der solche TCRs aufweist, darstellt, oder
eine Sammlung von einem oder mehreren Vektoren, die variante Keimbahn TCR-verbindende Gensegmente eines Organismus, der solche TCRs aufweist, darstellen, so dass die translatierte Aminosäuresequenz des kodierten Proteins in Bezug auf die durch das Keimbahn-Gensegment kodierte Proteinsequenz unmodifiziert ist, oder
eine Sammlung von einem oder mehreren Vektoren, die variante Keimbahn-TCR verbindenden
Gensegmente eines Organismus mit solchen TCRs darstellen, so dass die translatierte Aminosäuresequenz des kodierten Proteins in Bezug auf die durch das Keimbahngensegment kodierte Proteinsequenz modifiziert ist.

10. Ein Verfahren zur in vitro Rekonstitution eines offenen Leserahmens (ORF) eines TCRs voller Länge, jenes Verfahren umfassend
a. Auswählen eines V-C-Eintrittsvektors,
b. Auswählen eines J-Donor-Vektors,
c. Auswählen eines odeCDR3,
d. Kombinieren von a, b und c, um mit i) Typ-IIS-Restriktionsenzym(en) zu reagieren, um alle Typ IIS-Restriktionsenzym-Erkennungs- und Spaltstellen zu spalten, die in dem V-C-Eintrittsvektor und in dem J-Donor-Vektor vorhanden sind, und ii) das DNA-Ligase-Enzym zu spalten und die kombinierte Mischung einer Thermocycling-Reaktion zu unterziehen,
e. Transformieren des in Schritt d. erhaltenen Reaktionsprodukts in einen Wirtsorganismus, der für die Vermehrung von DNA-Vektoren kompetent ist, und
f. Vermehren jenes Wirtsorganismus, um den rekonstituierten TCR offenen Leserahmen in voller Länge in dem resultierenden V-C-Eintrittsvektor Grundgerüst zu erhalten.

11. Ein V-C-Eintrittsvektor, ausgewählt aus
Sequenz SEQ0692,
Sequenz SEQ0693 und
Sequenz SEQ0694.

12. Ein J-Donor-Vektor, dargestellt durch die Sequenz SEQ0700.

13. Eine Bibliothek von V-C-Eintrittsvektoren, wie in einem der Ansprüche 1 bis 8 definiert, zur Verwendung in Kombination mit einer Bibliothek von J-Donor-Vektoren und einem odeCDR3, um einen TCR-ORF in voller Länge zu rekonstruieren, wobei die V-C-Eintrittsvektoren variable und konstante Gensegmente enthalten, um die Gensegmentverwendung des menschlichen TRA Lokus zu rekapitulieren, dargestellt durch die Sequenzen SEQ0049 bis SEQ0094; und/oder die V-C-Eintrittsvektoren variable und konstante Gensegmente enthalten, um die Gensegmentnutzung des menschlichen TRB-Lokus, dargestellt durch die Sequenzen SEQ0484 bis SEQ0577, zu rekapitulieren.

14. Eine Bibliothek von J-Donor-Vektoren, wie in einem der Ansprüche 1-8 definiert, enthaltend verbindende Gensegmente zur Rekapitulation der Gensegment-Nutzung des humanen TRA Lokus, dargestellt durch die Sequenzen SEQ0323 bis SEQ0378, wobei die Vektoren in Verbindung mit einem odeCDR3 verwendet werden, der die gesamte CDR3-Region überspannt; und/oder
die verbindenden Gensegmente enthalten, um die Gensegmentverwendung des menschlichen TRA-Lokus zu rekapitulieren, der durch die Sequenzen SEQ0379 bis SEQ0434 dargestellt wird,
wobei die Vektoren in Verbindung mit einem odeCDR3 verwendet werden, das in der Länge um drei bis vier Codons reduziert ist.

15. Eine Bibliothek von J-Donor-Vektoren, wie in einem der Ansprüche 1-8 definiert, enthaltend verbindende Gensegmente zur Rekapitulation der Gensegmentnutzung des humanen TRB Lokus, dargestellt durch die Sequenzen SEQ0636 bis SEQ0661, wobei die Vektoren in Verbindung mit einem odeCDR3 verwendet werden, der die gesamte CDR3-Region überspannt, und/oder
die verbindende Gensegmente enthalten, um die Gensegmentverwendung des menschlichen TRB-Lokus zu rekapitulieren, der durch die Sequenzen SEQ0662 bis SEQ0687, dargestellt wird,
wobei die Vektoren in Verbindung mit einem odeCDR3 verwendet werden, das in der Länge um drei bis vier Codons reduziert ist.

16. Eine Bibliothek gemäß Anspruch 14 oder 15 zur Verwendung in Kombination mit einem V-C-Eintrittsvektor und einem odeCDR3, um einen TCR-ORF in voller Länge zu rekonstituieren.

17. Ein kombiniertes System gemäß einem der Ansprüche 1-8, wobei jene erste Komponente ein V-C-Eintrittsvektor ist, der eine erste TCR-Kette kodiert, wobei das System ferner eine Komponente umfasst, die einen Bidirektionalen Terminator (BiT)-Donor-Vektor umfasst, und eine Komponente, die einen V-C-Eintrittsvektor umfasst, der eine zweite TCR-Kette kodiert, die komplementär zu jener ersten TCR-Kette ist.

18. Ein kombiniertes System gemäß Anspruch 17, wobei der V-C-Eintrittsvektor jener ersten Komponente ferner umfasst
a. eine fünfte Typ-IIS-Sequenz zur ortsspezifischen Erkennung und Spaltung durch ein fünftes Typ-IIS Restriktionsenzym, wobei die Sequenz so orientiert ist, so dass das Enzym 5' jener Erkennungssequenz spaltet, und am 3' Ende des konstanten Gensegments spaltet,
b. einem negativen Selektionsmarker, der sich 3' der fünften Typ IIS-Sequenz befindet,
c. eine sechste Typ IIS-Sequenz zur ortsspezifischen Erkennung und Spaltung durch ein Restriktionsenzym vom sechsten Typ IIS, das sich 3' des negativen Selektionsmarkers von b. befindet, wobei die Sequenz so orientiert ist, so dass das Enzym 3' jener Erkennungssequenz und zum 5' des/der 3'-genetischen Element(e) spaltet.

19. Ein kombiniertes System gemäß Anspruch 17 oder 18, wobei jener V-C-Eintrittsvektor, der eine zweite TCR-Kette kodiert, die komplementär zu jener ersten Kette ist, und weiterhin umfasst
a. eine siebte Typ IIS-Sequenz zur ortsspezifischen Erkennung und Spaltung durch ein siebtes Typ IIS-Restriktionsenzym, wobei die Sequenz so orientiert ist, so dass das Enzym 3' jener Erkennungssequenz und am 5' der Kozak-Sequenz spaltet,
b. eine achte Typ IIS-Sequenz zur ortsspezifischen Erkennung und Spaltung durch ein achtes Typ-IIS-Restriktionsenzym, wobei die Sequenz so orientiert ist, so dass das Enzym 5' jener Erkennungssequenz spaltet, und am 3'-Ende des konstanten Gensegments,
c. einen negativen Selektionsmarker, der sich am 3' der achten Typ-IIS-Sequenz befindet.

20. Ein kombiniertes System gemäß einem der Ansprüche 17-19, wobei jener BiT-Donor-Vektor einen Replikationsursprung enthält,
a. einen positiven Selektionsmarker,
b. eine neunte Typ IIS-Sequenz zur ortsspezifischen Erkennung und Spaltung durch ein neuntes Typ IIS-Restriktionsenzym, wobei die Sequenz so orientiert ist, so dass das Enzym 3' jener Erkennungssequenz und am 5'-Ende eines bidirektionalen Terminatorelements spaltet, d,
c. ein bidirektionales Terminatorelement, das zwei Transkriptionsterminatoren in Antisense-Anordnung kodiert, und
d. eine zehnte Typ IIS-Sequenz zur ortsspezifischen Erkennung und Spaltung durch ein zehntes Typ-IIS-Restriktionsenzym, wobei die Sequenz so orientiert ist, so dass das Enzym 5' jener Erkennungssequenz und 3' des bidirektionalen Terminatorelements d, spaltet

21. Ein kombiniertes System gemäß einem der Ansprüche 17-20, wobei die ersten bis vierten Typ IIS-Sequenzen gleich oder verschieden sind.

22. Ein kombiniertes System gemäß einem der Ansprüche 17-20, wobei die fünften bis zehnten Typ-IIS-Sequenzen gleich oder verschieden sind und sich von denen der ersten bis vierten Typ-IIS-Sequenzen unterscheiden.

23. Ein kombiniertes System gemäß einem der Ansprüche 17-22, wobei der negative Selektionsmarker ausgewählt wird aus
a. einer Restriktionsenzymerkennungsstelle, die nicht an anderer Stelle in der ersten Komponente oder innerhalb des TCR Verbindungsgensegments enthalten ist,
b. einem bakteriellen Suizidgen, und
c. einem Reporterelement,
und wobei die in b. von Anspruch 18 und in c. von Anspruch 19 definierten negativen Selektionsmarker sich von dem in g. von Anspruch 1 definierten negativen Selektionsmarker unterscheiden und der in g. von Anspruch 1 definierte negative Selektionsmarker sich von denen im V-C-Eintrittsvektor der ersten Komponente und dem V-C-Eintrittsvektor, der jene zweite TCR-Kette kodiert, die zu jener ersten TCR-Kette komplementär ist, unterscheidet.

24. Eine Bibliothek von V-C-Eintrittsvektoren, wie in einem der Ansprüche 17-23 definiert, wobei die Bibliothek eine erste und zweite V-C-Eintrittsvektor-Bibliothek umfasst, die jeweils eine Sammlung von einem oder mehreren Vektoren enthalten, die alle Keimbahn TCR-variablen und -konstanten Gensegmente eines Organismus, der solche TCRs aufweist, darstellen, oder
eine Sammlung von einem oder mehreren Vektoren, die eine Sammlung von varianten Keimbahn TCR-variablen und -konstanten Gensegmenten eines Organismus, der solche TCRs aufweist, darstellen, so dass die translatierte Aminosäuresequenz des kodierten Proteins in Bezug auf die durch das Keimbahn-Gensegment kodierte Proteinsequenz unmodifiziert ist oder
eine Sammlung von einem oder mehreren Vektoren, die eine Sammlung von varianten Keimbahn-TCR-variablen und- konstanten Gensegmenten eines Organismus, der solche TCRs aufweist, darstellen, so dass die translatierte Aminosäuresequenz des kodierten Proteins in Bezug auf die durch das Keimbahn-Gensegment kodierte Proteinsequenz modifiziert ist,
wobei die erste und zweite Bibliothek von V-C-Eintrittsvektoren reziproke TCR-Ketten kodieren.

25. Eine Bibliothek von V-C-Eintrittsvektoren gemäß Anspruch 24 zur Verwendung zur Bereitstellung eines Paares von TCR offenen Leserahmen (ORFs) in voller Länge, kodiert in einer antiparallelen Anordnung in einem einzigen Produktkonstrukt.

26. Ein Verfahren zur in vitro Rekonstitution eines Paares von TCR offenen Leserahmen (ORFs) in voller Länge, kodiert in einer antiparallelen Anordnung in einem einzelnen Produktkonstrukt, jenes Verfahren umfassend
a. Auswählen eines V-C-Eintrittsvektors für jede TCR-Kette,
b. Auswählen eines J-Donor-Vektors für jede TCR-Kette,
c. Auswählen eines odeCDR3 für jede TCR-Kette,
d. Kombinieren von a, b und c für jeden TCR in einer unabhängigen Reaktion, um mit i) Typ IIS-Restriktionsenzym(en) zur Spaltung der ersten bis vierten Typ IIS-Restriktionsenzym-Erkennungs- und Spaltungsstellen zu reagieren, die in den V-C-Eintrittsvektoren und in den J-Donor-Vektoren vorhanden sind, und ii) das DNA-Ligase-Enzym und aussetzen der kombinierten Reaktionsprodukte Thermocycling-Reaktionen, um ein erstes und zweites Reaktionsprodukt zu erhalten,
e. Kombinieren beider Reaktionsprodukte aus Schritt d. mit einem BiT-Donor-Vektor wie in Anspruch 17 definiert, mit i) Typ IIS-Restriktionsenzym(en) zur Spaltung des/der fünften bis zehnten Typ IIS-Restriktionsenzym Erkennungs- und Spaltstellen und ii) DNA-Ligase-Enzym zu spalten, und aussetzen der kombinierten Reaktionsprodukte Thermocycling-Reaktionen, um ein drittes Reaktionsprodukt zu erhalten,
f. Transformieren des dritten Reaktionsprodukts aus Schritt e. in einen Wirtsorganismus, der für die DNA-Vektorvermehrung kompetent ist, und
g. Vermehren jenes Wirtsorganismus, um rekonstituierten TCR offene Leserahmen in voller Länge in dem resultierenden V-C-Eintrittsvektor-Rückgrat zu erhalten.

27. Ein V-C-Eintrittsvektor-Paar, dargestellt durch SEQ ID NO: 0756 und 0764.

28. Ein V-C-Eintrittsvektor-Paar gemäß Anspruch 27 zur Verwendung beim rekombinasevermittelten Kassettenaustausch mit genetischen Targets mit angepassten heterospezifischen Rekombinase-Sequenzen.

29. Ein bidirektionales Terminatorelement, dargestellt durch die Sequenz SEQ0777.

## Revendications

1. Système combiné comprenant deux composants séparés, dans lequel un premier composant est un vecteur porteur de segments de gène de récepteur de lymphocyte T (TCR) variables et constants (V-C), et un deuxième composant est un vecteur porteur de segments de gène TCR de jonction (J),
dans lequel le premier composant est un vecteur d'entrée V-C contenant
a. une origine de réplication,
b. un premier marqueur de sélection positive
c. un élément génétique 5', ou des éléments,
d. une séquence de Kozak,
e. un segment de gène variable TCR,
f. une première séquence de type IIS, pour une reconnaissance spécifique de site et un clivage par une première enzyme de restriction de type IIS, dans lequel la séquence est orientée de sorte que l'enzyme clive en 5' de ladite séquence de reconnaissance et au niveau de l'extrémité 3' du segment de gène variable TCR,
g. un marqueur de sélection négative,
h. une deuxième séquence de type IIS, pour une reconnaissance spécifique de site et un clivage par une deuxième enzyme de restriction de type IIS, dans lequel la séquence est orientée de sorte que l'enzyme clive pour un clivage direct en 3' de ladite séquence de reconnaissance et au niveau de l'extrémité 5' du segment de gène constant TCR,
i. un segment de gène constant TCR, et
j. un élément génétique 3', ou des éléments,
dans lequel le deuxième composant est un vecteur donneur J contenant
a. une origine de réplication,
b. un deuxième marqueur de sélection positive,
c. une troisième séquence de type IIS, pour une reconnaissance spécifique de site et un clivage par une troisième enzyme de restriction de type IIS, dans lequel la séquence est orientée de sorte que l'enzyme clive en 3' de ladite séquence de reconnaissance et au niveau de l'extrémité 5' du segment de gène de jonction TCR,
d. un segment de gène de jonction TCR,
e. une partie C, correspondant à une petite portion 5' d'un segment de gène constant, et
f. une quatrième séquence de type IIS, pour une reconnaissance spécifique de site et un clivage par une quatrième enzyme de restriction de type IIS, dans lequel la séquence est orientée de sorte que l'enzyme clive en 5' de ladite séquence de reconnaissance et au niveau de l'extrémité 3' de la portion de partie C TCR de la construction, de sorte que la séquence d'extrémité simple brin générée soit complémentaire de celle générée par ladite deuxième séquence de type IIS.

2. Système combiné selon la revendication 1, dans lequel l'élément génétique 5' comprend au moins un élément choisi parmi
a. un élément agissant en cis de gène,
b. un site de reconnaissance hétérospécifique pour des enzymes recombinase,
c. un bras de recombinaison homologue 5' pour un site génomique d'intérêt,
d. un site accepteur d'épissage d'ARNm,
e. un site d'entrée ribosomique interne, et
f. une séquence isolante épigénétique.

3. Système combiné selon l'une des revendications 1 à 2, dans lequel le marqueur de sélection négative est choisi parmi
a. un site de reconnaissance d'enzyme de restriction non contenu ailleurs dans le premier composant ou au sein du segment de gène de jonction TCR,
b. un gène suicide bactérien, et
c. un élément rapporteur.

4. Système combiné selon l'une quelconque des revendications 1 à 3, dans lequel l'élément génétique 3' comprend au moins un élément choisi parmi
a. un élément terminateur,
b. un site de reconnaissance hétérospécifique pour des enzymes recombinase,
c. un bras de recombinaison homologue 3' pour un site génomique d'intérêt,
d. un site donneur d'épissage d'ARNm,
e. un site d'entrée ribosomique interne, et
f. une séquence isolante épigénétique.

5. Système combiné selon l'une quelconque des revendications 1 à 4, dans lequel les composants ne contiennent aucune séquence de type IIS autre que celles définies ci-dessus, et contiennent uniquement ledit marqueur de sélection négative.

6. Système combiné selon l'une quelconque des revendications précédentes comprenant en outre un troisième composant comprenant un duplex oligonucléotidique codant pour CDR3 (odeCDR3), dans lequel l'odeCDR3 a
a. une première séquence d'extrémité simple brin complémentaire de celle générée par clivage du premier site de restriction de type IIS au niveau de l'extrémité 3' du segment de gène variable TCR,
b. un segment double brin codant pour une région CDR3 TCR et dépourvu de marqueur de sélection négative, lequel marqueur de sélection négative est tel que défini dans la revendication 5, et ledit élément double brin est également dépourvu de toute séquence de restriction de type IIS du premier ou deuxième composant, et
c. une deuxième séquence d'extrémité simple brin complémentaire de celle générée par clivage du troisième site d'enzyme de restriction de type IIS au niveau de l'extrémité 5' du segment de gène de jonction TCR.

7. Système combiné selon l'une quelconque des revendications 1 à 5 comprenant en outre un troisième composant comprenant un comprenant un duplex oligonucléotidique codant pour CDR3 (odeCDR3), dans lequel l'odeCDR3 est une molécule d'ADN double brin ou d'ADN de plasmide codant pour la CDR3 flanquée de deux sites de reconnaissance et de clivage de type IIS, de sorte qu'un clivage par lesdites enzymes aboutisse à des extrémités simple brin complémentaires de celles générées par le premier clivage de type IIS dans le vecteur d'entrée V-C au niveau de l'extrémité 5', et avec le troisième clivage de type IIS du vecteur donneur J au niveau de l'extrémité 3', pour obtenir un produit digéré dudit odeCDR3 comprenant a, b et c tels que décrits dans la revendication 6.

8. Système combiné selon l'une quelconque des revendications 1 à 7, dans lequel les première à quatrième séquences de type IIS sont identiques ou différentes.

9. Banque de vecteurs en deux parties comprenant une banque de vecteurs d'entrée V-C tels que définis dans la revendication 1 et une banque de vecteurs donneurs J tels que définis dans la revendication 1, dans laquelle la banque de vecteurs d'entrée V-C est
une collection d'au moins un vecteur représentant l'ensemble des segments de gène variables et constants du TCR de lignée germinale d'un organisme ayant de tels TCRs, ou
une collection d'au moins un vecteur représentant une collection de segments de gène variables et constants du TCR d'une lignée germinale de variant d'un organisme ayant de tels TCR, de sorte que la séquence d'acides aminés traduite de la protéine codée soit inchangée par rapport avec la séquence de protéine codée par le segment de gène de lignée germinale, ou une collection d'au moins un vecteur représentant une collection de segments de gène variables et constants du TCR d'une lignée germinale de variant d'un organisme ayant de tels TCRs, de sorte que la séquence d'acides aminés traduite de la protéine codée soit changée par rapport avec la séquence de protéine codée par le segment de gène de lignée germinale et dans laquelle
la banque de vecteurs donneurs J est une collection d'au moins un vecteur représentant des segments de gène de jonction TCR de lignée germinal d'un organisme ayant des tels TCRs ou
une collection d'au moins un vecteur représentant des segments de gène de jonction TCR d'une lignée germinale de variant d'un organisme ayant des tels TCRs, de sorte que la séquence d'acides aminés traduite de la protéine codée soit inchangée par rapport avec la séquence de protéine codée par le segment de gène de lignée germinale, ou
une collection d'au moins un vecteur représentant des segments de gène de jonction TCR d'une lignée germinale de variant d'un organisme ayant des tels TCRs, de sorte que la séquence d'acides aminés traduite de la protéine codée soit changée par rapport avec la séquence de protéine codée par le segment de gène de lignée germinale.

10. Procédé de reconstitution in vitro d'une phase ouverte de lecture (ORF) de TCR de pleine longueur, ledit procédé comprenant
a. la sélection d'un vecteur d'entrée V-C,
b. la sélection d'un vecteur donneur J,
c. la sélection d'un odeCDR3,
d. la combinaison de a, b et c pour réagir avec i) au moins une enzyme de restriction de type IIS pour cliver l'ensemble des sites de reconnaissance et de clivage d'enzyme de restriction de type IIS présents dans le vecteur d'entrée V-C et dans le vecteur donneur J et ii) une enzyme ADN ligase et la soumission du mélange combiné à une réaction de thermocyclage,
e. la transformation du produit de réaction obtenu à partir de l'étape d. dans un organisme hôte compétent pour une propagation de vecteur d'ADN, et
f. la propagation dudit organisme hôte pour obtenir une phase ouverte de lecture de TCR reconstituée de pleine longueur dans le squelette de vecteur d'entrée V-C résultant.

11. Vecteur d'entrée V-C choisi parmi
la séquence SEQ0692,
la séquence SEQ0693, et
la séquence SEQ0694.

12. Vecteur donneur J représenté par la séquence SEQ0700.

13. Banque de vecteurs d'entrée V-C tels que définis dans l'une quelconque des revendications 1 à 8 pour une utilisation en combinaison avec une banque de vecteurs donneurs J et un odeCDR3 pour reconstituer une ORF TCR de pleine longueur, dans laquelle les vecteurs d'entrée V-C contiennent des segments de gène variables et constants pour récapituler l'usage de segment de gène du locus TRA humain, représentés par les séquences SEQ0049 à SEQ0094 ; et/ou les vecteurs d'entrée V-C contiennent des segments de gène variables et constants pour récapituler l'usage de segment de gène du locus TRB humain, représentés par les séquences SEQ0484 à SEQ0577.

14. Banque de vecteurs donneurs J tels que définis dans l'une quelconque des revendications 1 à 8, contenant des segments de gène de jonction pour récapituler l'usage de segment de gène du locus TRA humain, représentés par les séquences SEQ0323 à SEQ0378, dans laquelle les vecteurs sont utilisés conjointement avec un odeCDR3 couvrant la région CDR3 entière ; et/ou contenant des segments de gène de jonction pour récapituler l'usage de segment de gène du locus TRA humain représentés par les séquences SEQ0379 à SEQ0434, dans laquelle les vecteurs sont utilisés conjointement avec un odeCDR3 d'une longueur réduite de trois ou quatre codons.

15. Banque de vecteurs donneurs J tels que définis dans l'une quelconque des revendications 1 à 8, contenant des segments de gène de jonction pour récapituler l'usage de segment de gène du locus TRB humain, représentés par les séquences SEQ0636 à SEQ0661, dans laquelle les vecteurs sont utilisés conjointement avec un odeCDR3 couvrant la région CDR3 entière, et/ou contenant des segments de gène de jonction pour récapituler l'usage de segment de gène du locus TRB humain représentés par les séquences SEQ0662 à SEQ0687, dans laquelle les vecteurs sont utilisés conjointement avec un odeCDR3 d'une longueur réduite de trois ou quatre codons.

16. Banque selon la revendication 14 ou 15 pour une utilisation en combinaison avec un vecteur d'entrée V-C et un odeCDR3 pour reconstituer une ORF TCR de pleine longueur.

17. Système combiné selon l'une quelconque des revendications 1 à 8, dans lequel ledit premier composant est un vecteur d'entrée V-C codant pour une première chaîne TCR, le système comprend en outre un composant comprenant un vecteur donneur terminateur bidirectionnel (BiT), et un composant comprenant un vecteur d'entrée V-C codant pour une deuxième chaîne TCR complémentaire de ladite première chaîne TCR.

18. Système combiné selon la revendication 17, dans lequel ledit vecteur d'entrée V-C dudit premier composant comprend en outre
a. une cinquième séquence de type IIS pour une reconnaissance spécifique de site et un clivage par une cinquième enzyme de restriction de type IIS, dans lequel la séquence est orientée de sorte que l'enzyme clive en 5' de ladite séquence de reconnaissance et au niveau de l'extrémité 3' du segment de gène constant,
b. un marqueur de sélection négative situé en 3' de la cinquième séquence de type IIS,
c. une sixième séquence de type IIS pour une reconnaissance spécifique de site et un clivage par une sixième enzyme de restriction de type IIS située en 3' dudit marqueur de sélection négative de b., dans lequel la séquence est orientée de sorte que l'enzyme clive en 3' de ladite séquence de reconnaissance, et en 5' du ou des élément(s) génétique(s) 3'.

19. Système combiné selon la revendication 17 ou 18, dans lequel ledit vecteur d'entrée V-C code pour une deuxième chaîne TCR complémentaire de ladite première chaîne et comprend en outre
a. une septième séquence de type IIS pour une reconnaissance spécifique de site et un clivage par une septième enzyme de restriction de type IIS, dans lequel la séquence est orientée de sorte que l'enzyme clive en 3' de ladite séquence de reconnaissance et en 5' de la séquence de Kozak,
b. une huitième séquence de type IIS pour une reconnaissance spécifique de site et un clivage par une huitième enzyme de restriction de type IIS, dans lequel la séquence est orientée de sorte que l'enzyme clive en 5' de ladite séquence de reconnaissance et au niveau de l'extrémité 3' du segment de gène constant,
c. un marqueur de sélection négative situé en 3' de la huitième séquence de type IIS.

20. Système combiné selon l'une quelconque des revendications 17 à 19, dans lequel ledit vecteur donneur BiT contient
une origine de réplication,
a. un marqueur de sélection positive,
b. une neuvième séquence de type IIS pour une reconnaissance spécifique de site et un clivage par une neuvième enzyme de restriction de type IIS, dans lequel la séquence est orientée de sorte que l'enzyme clive en 3' de ladite séquence de reconnaissance et l'extrémité 5' d'un élément terminateur bidirectionnel, d,
c. un élément terminateur bidirectionnel codant pour deux terminateurs transcriptionnels en arrangement antisens, et
d. une dixième séquence de type IIS pour une reconnaissance spécifique de site et un clivage par une dixième enzyme de restriction de type IIS, dans lequel la séquence est orientée de sorte que l'enzyme clive en 5' de ladite séquence de reconnaissance et en 3' de l'élément terminateur bidirectionnel, d,

21. Système combiné selon l'une quelconque des revendications 17 à 20, dans lequel les première à quatrième séquences de type IIS sont identiques ou différentes.

22. Système combiné selon l'une quelconque des revendications 17 à 20, dans lequel les cinquième à dixième séquences de type IIS sont identiques ou différentes, et sont différentes de celles des première à quatrième séquence de type IIS.

23. Système combiné selon l'une quelconque des revendications 17 à 22, dans lequel le marqueur de sélection négative est choisi parmi
a. un site de reconnaissance d'enzyme de restriction non contenu ailleurs dans le premier composant ou au sein du segment de gène de jonction TCR,
b. un gène suicide bactérien, et
c. un élément rapporteur,
et dans lequel les marqueurs de sélection négative définis en b. de la revendication 18 et en c. de la revendication 19 sont différents du marqueur de sélection négative défini en g. de la revendication 1, et le marqueur de sélection négative défini en g. de la revendication 1 est différent de ceux dans le vecteur d'entrée V-C du premier composant et du vecteur d'entrée V-C codant pour ladite deuxième chaîne TCR complémentaire de ladite première chaîne TCR.

24. Banque de vecteurs d'entrée V-C tels que définis dans l'une quelconque des revendications 17 à 23, dans laquelle la banque comprend une première et deuxième banque de vecteurs d'entrée V-C, chacune contenant une collection d'au moins un vecteur représentant l'ensemble des segments de gène variables et constants TCR de lignée germinale d'un organisme ayant de tels TCR, ou
une collection d'au moins un vecteur représentant une collection des segments de gène variables et constants TCR d'une lignée germinale de variant d'un organisme ayant des tels TCR, de sorte que la séquence d'acides aminés traduite de la protéine codée soit inchangée par rapport avec la séquence de protéine codée par le segment de gène de lignée germinale, ou
une collection d'au moins un vecteur représentant une collection de segments de gène variables et constants TCR d'une lignée germinale de variant d'un organisme ayant des tels TCR, de sorte que la séquence d'acides aminés traduite de la protéine codée soit modifiée par rapport avec la séquence de protéine codée par le segment de gène de lignée germinale,
dans laquelle les première et deuxième banques de vecteurs d'entrée V-C codent pour des chaînes TCR réciproques.

25. Banque de vecteurs d'entrée V-C selon la revendication 24 pour une utilisation pour fournir une paire de phases ouvertes de lecture (ORFs) de TCR de pleine longueur, codées en un arrangement antiparallèle dans une construction de produit unique.

26. Procédé de reconstitution in vitro d'une paire de phases ouvertes de lecture (ORFs) de TCR de pleine longueur, codées en un arrangement antiparallèle dans une construction de produit unique, ledit procédé comprenant
a. la sélection d'un vecteur d'entrée V-C pour chaque chaîne TCR,
b. la sélection d'un vecteur donneur J pour chaque chaîne TCR,
c. la sélection d'un odeCDR3 pour chaque chaîne TCR,
d. la combinaison de a, b et c pour chaque TCR dans une réaction indépendante pour réagir avec i) au moins une enzyme de restriction de type IIS pour cliver l' les premier à quatrième sites de reconnaissance et de clivage d'enzyme de restriction de type IIS présents dans les vecteurs d'entrée V-C et dans les vecteurs donneur J et ii) une enzyme ADN ligase et la soumission des mélanges combinés à des réactions de thermocyclage pour obtenir un premier et deuxième produit de réaction,
e. la combinaison des deux produits de réaction issus de l'étape d. à un vecteur donneur BiT tel que défini dans la revendication 17 avec i) une ou des enzyme(s) de restriction de type IIS pour cliver les cinquième à dixième sites de reconnaissance et de clivage d'enzyme de restriction de type IIS et ii) une enzyme ADN ligase, et la soumission des produits de réaction combinés à des réactions de thermocyclage pour obtenir un troisième produit de réaction,
f. la transformation du troisième produit de réaction issu de l'étape e. dans un organisme hôte compétent pour une propagation de vecteur d'ADN, et
g. la propagation dudit organisme hôte pour obtenir une phase ouverte de lecture de TCR reconstituée de pleine longueur dans le squelette de vecteur d'entrée V-C résultant.

27. Paire de vecteurs d'entrée V-C représentés par SEQ ID N° : 0756 et 0764.

28. Paire de vecteurs d'entrée V-C selon la revendication 27, pour une utilisation dans un échange de cassette médié par recombinase avec des cibles génétiques ayant des séquences de recombinase hétérospécifiques appareillées.

29. Élément terminateur bidirectionnel, représenté par la séquence SEQ0777.


### SEQUENCE LISTING

<110> Genovie AB
<120> ANO5
<130> ANO5
<160> 779
<170> BiSSAP 1.3
<210> 1
   <211> 396
   <212> DNA
   <213> Homo sapiens
<220>
   <223> TRAV1-1_BB_1
<400> 1
   gtcagatact ccatgagcac gaagacttgt acgccaccat gtggggagct ttccttctct       60
   atgtttccat gaagatggga ggcactgcag gacaaagcct tgagcagccc tctgaagtga      120
   cagctgtgga aggagccatt gtccagataa actgcacgta ccagacatct gggttttatg      180
   ggctgtcctg gtaccagcaa catgatggcg gagcacccac atttctttct tacaatgctc      240
   tggatggttt ggaggagaca ggtcgttttt cttcattcct tagtcgctct gatagttatg      300
   gttacctcct tctacaggag ctccagatga aagactctgc ctcttacttc tgcagagacc      360
   ttgcggccgt gtcttcgact agtagctcac ctacga                                396
<210> 2
   <211> 396
   <212> DNA
   <213> Homo sapiens
<220>
   <223> TRAV1-2_BB_1
<400> 2
   gtcagatact ccatgagcac gaagacttgt acgccaccat gtggggagtt ttccttcttt       60
   atgtttccat gaagatggga ggcactacag gacaaaacat tgaccagccc actgagatga      120
   cagctacgga aggtgccatt gtccagatca actgcacgta ccagacatct gggttcaacg      180
   ggctgttctg gtaccagcaa catgctggcg aagcacccac atttctgtct tacaatgttc      240
   tggatggttt ggaggagaaa ggtcgttttt cttcattcct tagtcggtct aaagggtaca      300
   gttacctcct tttgaaggag ctccagatga aagactctgc ctcttacctc tgcagagacc      360
   ttgcggccgt gtcttcgact agtagctcac ctacga                                396
<210> 3
   <211> 408
   <212> DNA
   <213> Homo sapiens
<220>
   <223> TRAV2_BB_1
<400> 3
   gtcagatact ccatgagcac gaagacttgt acgccaccat ggctttgcag agcactctgg      60
   gggcggtgtg gctagggctt ctcctcaact ctctctggaa ggttgcagaa agcaaggacc     120
   aagtgtttca gccttccaca gtggcatctt cagagggagc tgtggtggaa atcttctgta     180
   atcactctgt gtccaatgct tacaacttct tctggtacct tcacttcccg ggatgtgcac     240
   caagactcct tgttaaaggc tcaaagcctt ctcagcaggg acgatacaac atgacctatg     300
   aacggttctc ttcatcgctg ctcatcctcc aggtgcggga ggcagatgct gctgtttact     360
   actgcagaga ccttgcggcc gtgtcttcga ctagtagctc acctacga                  408
<210> 4
   <211> 411
   <212> DNA
   <213> Homo sapiens
<220>
   <223> TRAV3_BB_1
<400> 4
   gtcagatact ccatgagcac gaagacttgt acgccaccat ggcctctgca cccatctcga      60
   tgcttgcgat gctcttcaca ttgagtgggc tgagagctca gtcagtggct cagccggaag     120
   atcaggtcaa cgttgctgaa gggaatcctc tgactgtgaa atgcacctat tcagtctctg     180
   gaaaccctta tctttttggg tatgttcaat accccaaccg aggcctccag ttccttctga     240
   aatacatcac aggggataac ctggttaaag gcagctatgg ctttgaagct gaatttaaca     300
   agagccaaac ctccttccac ctgaagaaac catctgccct tgtgagcgac tccgctttgt     360
   acttctgcag agaccttgcg gccgtgtctt cgactagtag ctcacctacg a              411
<210> 5
   <211> 396
   <212> DNA
   <213> Homo sapiens
<220>
   <223> TRAV4_BB_1
<400> 5
   gtcagatact ccatgagcac gaagacttgt acgccaccat gaggcaagtg gcgagagtga      60
   tcgtgttcct gaccctgagt actttgagcc ttgctaagac cacccagccc atctccatgg     120
   actcatatga aggacaagaa gtgaacataa cctgtagcca caacaacatt gctacaaatg     180
   attatatcac gtggtaccaa cagtttccca gccaaggacc acgatttatt attcaaggat     240
   acaagacaaa agttacaaac gaagtggcct ccctgtttat ccctgccgac agaaagtcca     300
   gcactctgag cctgccccgg gtttccctga gcgacactgc tgtgtactac tgcagagacc     360
   ttgcggccgt gtcttcgact agtagctcac ctacga                               396
<210> 6
   <211> 411
   <212> DNA
   <213> Homo sapiens
<220>
   <223> TRAV5_BB_1
<400> 6
   gtcagatact ccatgagcac gaagacttgt acgccaccat gaaaacattt gctggatttt      60
   cgttcctgtt tttgtggctg cagctggact gtatgagtag aggagaggat gtggagcaga     120
   gtcttttcct gagtgtccga gagggagaca gctccgttat aaactgcact tacacagaca     180
   gctcctccac ctacttatac tggtataagc aagaacctgg agcaggtcta cagttgctga     240
   cgtatatttt ttcaaatatg gacatgaaac aagaccaaag actcactgtt ctattgaata     300
   aaaaggataa acatctgtct ctgcgcattg cagacaccca gactggggac tcagctatct     360
   acttctgcag agaccttgcg gccgtgtctt cgactagtag ctcacctacg a              411
<210> 7
   <211> 411
   <212> DNA
   <213> Homo sapiens
<220>
   <223> TRAV6_BB_1
<400> 7
   gtcagatact ccatgagcac gaagacttgt acgccaccat ggagtcattc ctgggaggtg      60
   ttttgctgat tttgtggctt caagtggact gggtgaagag ccaaaagata gaacagaatt     120
   ccgaggccct gaacattcag gagggtaaaa cggccaccct gacctgcaac tatacaaact     180
   attccccagc atacttacag tggtaccgac aagatccagg aagaggccct gttttcttgc     240
   tactcatacg tgaaaatgag aaagaaaaaa ggaaagaaag actgaaggtc acctttgata     300
   ccacccttaa acagagtttg tttcatatca cagcctccca gcctgcagac tcagctacct     360
   acctctgcag agaccttgcg gccgtgtctt cgactagtag ctcacctacg a              411
<210> 8
   <211> 408
   <212> DNA
   <213> Homo sapiens
<220>
   <223> TRAV7_BB_1
<400> 8
   gtcagatact ccatgagcac gaagacttgt acgccaccat ggagaagatg cgtagacctg      60
   tcctaattat attttgtcta tgtcttggct gggcaaatgg agaaaaccag gtggagcaca     120
   gccctcattt tctgggaccc cagcagggag acgttgcctc catgagctgc acgtactctg     180
   tcagtcgttt taacaatttg cagtggtaca ggcaaaatac agggatgggt cccaaacacc     240
   tattatccat gtattcagct ggatatgaga agcagaaagg aaggctaaat gctacattac     300
   tgaagaatgg aagcagcttg tacattacag ccgtgcagcc tgaagattca gccacctatt     360
   tctgcagaga ccttgcggcc gtgtcttcga ctagtagctc acctacga                  408
<210> 9
   <211> 411
   <212> DNA
   <213> Homo sapiens
<220>
   <223> TRAV8-1_BB_1
<400> 9
   gtcagatact ccatgagcac gaagacttgt acgccaccat gctcctgttg ctcataccag 60
   tgctggggat gatttttgcc ctgagagatg ccagagccca gtctgtgagc cagcataacc 120
   accacgtaat tctctctgaa gcagcctcac tggagttggg atgcaactat tcctatggtg 180
   gaactgttaa tctcttctgg tatgtccagt accctggtca acaccttcag cttctcctca 240
   agtacttttc aggggatcca ctggttaaag gcatcaaggg ctttgaggct gaatttataa 300
   agagtaaatt ctcctttaat ctgaggaaac cctctgtgca gtggagtgac acagctgagt 360
   acttctgcag agaccttgcg gccgtgtctt cgactagtag ctcacctacg a 411
<210> 10
   <211> 411
   <212> DNA
   <213> Homo sapiens
<220>
   <223> TRAV8-2_BB_1
<400> 10
   gtcagatact ccatgagcac gaagacttgt acgccaccat gctcctgctg ctcgtcccag 60
   tgctcgaggt gatttttact ctgggaggaa ccagagccca gtcggtgacc cagcttgaca 120
   gccacgtctc tgtctctgaa ggaaccccgg tgctgctgag gtgcaactac tcatcttctt 180
   attcaccatc tctcttctgg tatgtgcaac accccaacaa aggactccag cttctcctga 240
   agtacacatc agcggccacc ctggttaaag gcatcaacgg ttttgaggct gaatttaaga 300
   agagtgaaac ctccttccac ctgacgaaac cctcagccca tatgagcgac gcggctgagt 360
   acttctgcag agaccttgcg gccgtgtctt cgactagtag ctcacctacg a 411
<210> 11
   <211> 411
   <212> DNA
   <213> Homo sapiens
<220>
   <223> TRAV8-3_BB_1
<400> 11
   gtcagatact ccatgagcac gaagacttgt acgccaccat gctcctggag cttatcccac 60
   tgctggggat acattttgtc ctgagaactg ccagagccca gtcagtgacc cagcctgaca 120
   tccacatcac tgtctctgaa ggagcctcac tggagttgag atgtaactat tcctatgggg 180
   caacacctta tctcttctgg tatgtccagt cccccggcca aggcctccag ctgctcctga 240
   agtacttttc aggagacact ctggttcaag gcattaaagg ctttgaggct gaatttaaga 300
   ggagtcaatc ttccttcaat ctgaggaaac cctctgtgca ttggagtgat gctgctgagt 360
   acttctgcag agaccttgcg gccgtgtctt cgactagtag ctcacctacg a 411
<210> 12
   <211> 411
   <212> DNA
   <213> Homo sapiens
<220>
   <223> TRAV8-4_BB_1
<400> 12
   gtcagatact ccatgagcac gaagacttgt acgccaccat gctcctgctg ctcgtcccag 60
   tgctcgaggt gatttttacc ctgggaggaa ccagagccca gtcggtgacc cagcttggca 120
   gccacgtctc tgtctctgaa ggagccctgg ttctgctgag gtgcaactac tcatcgtctg 180
   ttccaccata tctcttctgg tatgtgcaat accccaacca aggactccag cttctcctga 240
   agtacacatc agcggccacc ctggttaaag gcatcaacgg ttttgaggct gaatttaaga 300
   agagtgaaac ctccttccac ctgacgaaac cctcagccca tatgagcgac gcggctgagt 360
   acttctgcag agaccttgcg gccgtgtctt cgactagtag ctcacctacg a 411
<210> 13
   <211> 411
   <212> DNA
   <213> Homo sapiens
<220>
   <223> TRAV8-6_BB_1
<400> 13
   gtcagatact ccatgagcac gaagacttgt acgccaccat gctcctgctg ctcgtcccag 60
   cgttccaggt gatttttacc ctgggaggaa ccagagccca gtctgtgacc cagcttgaca 120
   gccaagtccc tgtctttgaa gaagcccctg tggagctgag gtgcaactac tcatcgtctg 180
   tttcagtgta tctcttctgg tatgtgcaat accccaacca aggactccag cttctcctga 240
   agtatttatc aggatccacc ctggttaaag gcatcaacgg ttttgaggct gaatttaaca 300
   agagtcaaac ttccttccac ttgaggaaac cctcagtcca tataagcgac acggctgagt 360
   acttctgcag agaccttgcg gccgtgtctt cgactagtag ctcacctacg a 411
<210> 14
   <211> 408
   <212> DNA
   <213> Homo sapiens
<220>
   <223> TRAV8-7_BB_1
<400> 14
   gtcagatact ccatgagcac gaagacttgt acgccaccat gctcttagtg gtcattctgc 60
   tgcttggaat gttcttcaca ctgagaacca gaacccagtc ggtgacccag cttgatggcc 120
   acatcactgt ctctgaagaa gcccctctgg aactgaagtg caactattcc tatagtggag 180
   ttccttctct cttctggtat gtccaatact ctagccaaag cctccagctt ctcctcaaag 240
   acctaacaga ggccacccag gttaaaggca tcagaggttt tgaggctgaa tttaagaaga 300
   gcgaaacctc cttctacctg aggaaaccat caacccatgt gagtgatgct gctgagtact 360
   tctgcagaga ccttgcggcc gtgtcttcga ctagtagctc acctacga 408
<210> 15
   <211> 408
   <212> DNA
   <213> Homo sapiens
<220>
   <223> TRAV9-1_BB_1
<400> 15
   gtcagatact ccatgagcac gaagacttgt acgccaccat gaattcttct ccaggaccag 60
   cgattgcact attcttaatg tttgggggaa tcaatggaga ttcagtggtc cagacagaag 120
   gccaagtgct cccctctgaa ggggattccc tgattgtgaa ctgctcctat gaaaccacac 180
   agtacccttc cctttttggg tatgtccaat atcctggaga aggtccacag ctccacctga 240
   aagccatgaa ggccaatgac aagggaagga acaaaggttt tgaagccatg taccgtaaag 300
   aaaccacttc tttccacttg gagaaagact cagttcaaga gtcagactcc gctgtgtact 360
   tctgcagaga ccttgcggcc gtgtcttcga ctagtagctc acctacga 408
<210> 16
   <211> 408
   <212> DNA
   <213> Homo sapiens
<220>
   <223> TRAV9-2_BB_1
<400> 16
   gtcagatact ccatgagcac gaagacttgt acgccaccat gaactattct ccaggcttag      60
   tatctctgat actcttactg cttggaagaa cccgtggaaa ttcagtgacc cagatggaag     120
   ggccagtgac tctctcagaa gaggccttcc tgactataaa ctgcacgtac acagccacag     180
   gatacccttc ccttttctgg tatgtccaat atcctggaga aggtctacag ctcctcctga     240
   aagccacgaa ggctgatgac aagggaagca acaaaggttt tgaagccaca taccgtaaag     300
   aaaccacttc tttccacttg gagaaaggct cagttcaagt gtcagactca gcggtgtact     360
   tctgcagaga ccttgcggcc gtgtcttcga ctagtagctc acctacga                  408
<210> 17
   <211> 414
   <212> DNA
   <213> Homo sapiens
<220>
   <223> TRAV10_BB_1
<400> 17
   gtcagatact ccatgagcac gaagacttgt acgccaccat gaaaaagcat ctgacgacct      60
   tcttggtgat tttgtggctt tatttttata gggggaatgg caaaaaccaa gtggagcaga     120
   gtcctcagtc cctgatcatc ctggagggaa agaactgcac tcttcaatgc aattatacag     180
   tgagcccctt cagcaactta aggtggtata agcaagatac tgggagaggt cctgtttccc     240
   tgacaatcat gactttcagt gagaacacaa agtcgaacgg aagatataca gcaactctgg     300
   atgcagacac aaagcaaagc tctctgcaca tcacagcctc ccagctcagc gattcagcct     360
   cctacatctg cagagacctt gcggccgtgt cttcgactag tagctcacct acga            414
<210> 18
   <211> 408
   <212> DNA
   <213> Homo sapiens
<220>
   <223> TRAV12-1_BB_1
<400> 18
   gtcagatact ccatgagcac gaagacttgt acgccaccat gatatccttg agagttttac 60
   tggtgatcct gtggcttcag ttaagctggg tttggagcca acggaaggag gtggagcagg 120
   atcctggacc cttcaatgtt ccagagggag ccactgtcgc tttcaactgt acttacagca 180
   acagtgcttc tcagtctttc ttctggtaca gacaggattg caggaaagaa cctaagttgc 240
   tgatgtccgt atactccagt ggtaatgaag atggaaggtt tacagcacag ctcaatagag 300
   ccagccagta tatttccctg ctcatcagag actccaagct cagtgattca gccacctacc 360
   tctgcagaga ccttgcggcc gtgtcttcga ctagtagctc acctacga 408
<210> 19
   <211> 414
   <212> DNA
   <213> Homo sapiens
<220>
   <223> TRAV12-2_BB_1
<400> 19
   gtcagatact ccatgagcac gaagacttgt acgccaccat gatgaaatcc ttgagagttt 60
   tactagtgat cctgtggctt cagttgagct gggtttggag ccaacagaag gaggtggagc 120
   agaattctgg acccctcagt gttccagagg gagccattgc ctctctcaac tgcacttaca 180
   gtgaccgagg ttcccagtcc ttcttctggt acagacaata ttctgggaaa agccctgagt 240
   tgataatgtt catatactcc aatggtgaca aagaagatgg aaggtttaca gcacagctca 300
   ataaagccag ccagtatgtt tctctgctca tcagagactc ccagcccagt gattcagcca 360
   cctacctctg cagagacctt gcggccgtgt cttcgactag tagctcacct acga 414
<210> 20
   <211> 414
   <212> DNA
   <213> Homo sapiens
<220>
   <223> TRAV12-3_BB_1
<400> 20
   gtcagatact ccatgagcac gaagacttgt acgccaccat gatgaaatcc ttgagagttt      60
   tactggtgat cctgtggctt cagttaagct gggtttggag ccaacagaag gaggtggagc     120
   aggatcctgg accactcagt gttccagagg gagccattgt ttctctcaac tgcacttaca     180
   gcaacagtgc ttttcaatac ttcatgtggt acagacagta ttccagaaaa ggccctgagt     240
   tgctgatgta cacatactcc agtggtaaca aagaagatgg aaggtttaca gcacaggtcg     300
   ataaatccag caagtatatc tccttgttca tcagagactc acagcccagt gattcagcca     360
   cctacctctg cagagacctt gcggccgtgt cttcgactag tagctcacct acga           414
<210> 21
   <211> 408
   <212> DNA
   <213> Homo sapiens
<220>
   <223> TRAV13-1_BB_1
<400> 21
   gtcagatact ccatgagcac gaagacttgt acgccaccat gacatccatt cgagctgtat      60
   ttatattcct gtggctgcag ctggacttgg tgaatggaga gaatgtggag cagcatcctt     120
   caaccctgag tgtccaggag ggagacagcg ctgttatcaa gtgtacttat tcagacagtg     180
   cctcaaacta cttcccttgg tataagcaag aacttggaaa aggacctcag cttattatag     240
   acattcgttc aaatgtgggc gaaaagaaag accaacgaat tgctgttaca ttgaacaaga     300
   cagccaaaca tttctccctg cacatcacag aaacccaacc tgaggactcg gctgtctact     360
   tctgcagaga ccttgcggcc gtgtcttcga ctagtagctc acctacga                  408
<210> 22
   <211> 411
   <212> DNA
   <213> Homo sapiens
<220>
   <223> TRAV13-2_BB_1
<400> 22
   gtcagatact ccatgagcac gaagacttgt acgccaccat ggcaggcatt cgagctttat      60
   ttatgtactt gtggctgcag ctggactggg tgagcagagg agagagtgtg gggctgcatc     120
   ttcctaccct gagtgtccag gagggtgaca actctattat caactgtgct tattcaaaca     180
   gcgcctcaga ctacttcatt tggtacaagc aagaatctgg aaaaggtcct caattcatta     240
   tagacattcg ttcaaatatg gacaaaaggc aaggccaaag agtcaccgtt ttattgaata     300
   agacagtgaa acatctctct ctgcaaattg cagctactca acctggagac tcagctgtct     360
   acttctgcag agaccttgcg gccgtgtctt cgactagtag ctcacctacg a              411
<210> 23
   <211> 417
   <212> DNA
   <213> Homo sapiens
<220>
   <223> TRAV14DV4_BB_1
<400> 23
   gtcagatact ccatgagcac gaagacttgt acgccaccat gtcactttct agcctgctga      60
   aggtggtcac agcttcactg tggctaggac ctggcattgc ccagaagata actcaaaccc     120
   aaccaggaat gttcgtgcag gaaaaggagg ctgtgactct ggactgcaca tatgacacca     180
   gtgatcaaag ttatggtcta ttctggtaca agcagcccag cagtggggaa atgatttttc     240
   ttatttatca ggggtcttat gacgagcaaa atgcaacaga aggtcgctac tcattgaatt     300
   tccagaaggc aagaaaatcc gccaaccttg tcatctccgc ttcacaactg ggggactcag      360
   caatgtattt ctgcagagac cttgcggccg tgtcttcgac tagtagctca cctacga        417
<210> 24
   <211> 399
   <212> DNA
   <213> Homo sapiens
<220>
   <223> TRAV16_BB_1
<400> 24
   gtcagatact ccatgagcac gaagacttgt acgccaccat gaagcccacc ctcatctcag      60
   tgcttgtgat aatatttata ctcagaggaa caagagccca gagagtgact cagcccgaga     120
   agctcctctc tgtctttaaa ggggccccag tggagctgaa gtgcaactat tcctattctg     180
   ggagtcctga actcttctgg tatgtccagt actccagaca acgcctccag ttactcttga     240
   gacacatctc tagagagagc atcaaaggct tcactgctga ccttaacaaa ggcgagacat     300
   ctttccacct gaagaaacca tttgctcaag aggaagattc agccatgtat tactgcagag     360
   accttgcggc cgtgtcttcg actagtagct cacctacga                            399
<210> 25
   <211> 408
   <212> DNA
   <213> Homo sapiens
<220>
   <223> TRAV17_BB_1
<400> 25
   gtcagatact ccatgagcac gaagacttgt acgccaccat ggaaactctc ctgggagtgt      60
   ctttggtgat tctatggctt caactggcta gggtgaacag tcaacaggga gaagaggatc     120
   ctcaggcctt gagcatccag gagggtgaaa atgccaccat gaactgcagt tacaaaacta     180
   gtataaacaa tttacagtgg tatagacaaa attcaggtag aggccttgtc cacctaattt     240
   taatacgttc aaatgaaaga gagaaacaca gtggaagatt aagagtcacg cttgacactt     300
   ccaagaaaag cagttccttg ttgatcacgg cttcccgggc agcagacact gcttcttact     360
   tctgcagaga ccttgcggcc gtgtcttcga ctagtagctc acctacga                  408
<210> 26
   <211> 405
   <212> DNA
   <213> Homo sapiens
<220>
   <223> TRAV18_BB_1
<400> 26
   gtcagatact ccatgagcac gaagacttgt acgccaccat gctgtctgct tcctgctcag      60
   gacttgtgat cttgttgata ttcagaagga ccagtggaga ctcggttacc cagacagaag     120
   gcccagttac cctccctgag agggcagctc tgacattaaa ctgcacttat cagtccagct     180
   attcaacttt tctattctgg tatgtccagt atctaaacaa agagcctgag ctcctcctga     240
   aaagttcaga aaaccaggag acggacagca gaggttttca ggccagtcct atcaagagtg     300
   acagttcctt ccacctggag aagccctcgg tgcagctgtc ggactctgcc gtgtactact 360
   gcagagacct tgcggccgtg tcttcgacta gtagctcacc tacga 405
<210> 27
   <211> 417
   <212> DNA
   <213> Homo sapiens
<220>
   <223> TRAV19_BB_1
<400> 27
   gtcagatact ccatgagcac gaagacttgt acgccaccat gctgactgcc agcctgttga 60
   gggcagtcat agcctccatc tgtgttgtat ccagcatggc tcagaaggta actcaagcgc 120
   agactgaaat ttctgtggtg gagaaggagg atgtgacctt ggactgtgtg tatgaaaccc 180
   gtgatactac ttattactta ttctggtaca agcaaccacc aagtggagaa ttggttttcc 240
   ttattcgtcg gaactctttt gatgagcaaa atgaaataag tggtcggtat tcttggaact 300
   tccagaaatc caccagttcc ttcaacttca ccatcacagc ctcacaagtc gtggactcag 360
   cagtatactt ctgcagagac cttgcggccg tgtcttcgac tagtagctca cctacga 417
<210> 28
   <211> 408
   <212> DNA
   <213> Homo sapiens
<220>
   <223> TRAV20_BB_1
<400> 28
   gtcagatact ccatgagcac gaagacttgt acgccaccat ggagaaaatg ttggagtgtg 60
   cattcatagt cttgtggctt cagcttggct ggttgagtgg agaggaccag gtgacgcaga 120
   gtcccgaggc cctgagactc caggagggag agagtagcag tcttaactgc agttacacag 180
   tcagcggttt aagagggctg ttctggtata ggcaagatcc tgggaaaggc cctgaattcc 240
   tcttcaccct gtattcagct ggggaagaaa aggagaaaga aaggctaaaa gccacattaa 300
   caaagaagga aagctttctg cacatcacag cccctaaacc tgaggactca gccacttatc 360
   tctgcagaga ccttgcggcc gtgtcttcga ctagtagctc acctacga 408
<210> 29
   <211> 408
   <212> DNA
   <213> Homo sapiens
<220>
   <223> TRAV21_BB_1
<400> 29
   gtcagatact ccatgagcac gaagacttgt acgccaccat ggagaccctc ttgggcctgc      60
   ttatcctttg gctgcagctg caatgggtga gcagcaaaca ggaggtgacg cagattcctg     120
   cagctctgag tgtcccagaa ggagaaaact tggttctcaa ctgcagtttc actgatagcg     180
   ctatttacaa cctccagtgg tttaggcagg accctgggaa aggactcaca tctctgttgc     240
   ttattcagtc aagtcagaga gagcaaacaa gtggacgcct taatgcctcg ctggataaat     300
   catcaggacg tagtacttta tacattgcag cttctcagcc tggtgactca gccacctacc     360
   tctgcagaga ccttgcggcc gtgtcttcga ctagtagctc acctacga                 408
<210> 30
   <211> 402
   <212> DNA
   <213> Homo sapiens
<220>
   <223> TRAV22_BB_1
<400> 30
   gtcagatact ccatgagcac gaagacttgt acgccaccat gaagaggata ttgggagctc      60
   tgctggggct cttgagtgcc caggtttgct gtgtgagagg aatacaagtg gagcagagtc     120
   ctccagacct gattctccag gagggagcca attccacgct gcggtgcaat ttttctgact      180
   ctgtgaacaa tttgcagtgg tttcatcaaa acccttgggg acagctcatc aacctgtttt     240
   acattccctc agggacaaaa cagaatggaa gattaagcgc cacgactgtc gctacggaac     300
   gctacagctt attgtacatt tcctcttccc agaccacaga ctcaggcgtt tatttctgca     360
   gagaccttgc ggccgtgtct tcgactagta gctcacctac ga                        402
<210> 31
   <211> 435
   <212> DNA
   <213> Homo sapiens
<220>
   <223> TRAV23DV6_BB_1
<400> 31
   gtcagatact ccatgagcac gaagacttgt acgccaccat ggacaagatc ttaggagcat 60
   catttttagt tctgtggctt caactatgct gggtgagtgg ccaacagaag gagaaaagtg 120
   accagcagca ggtgaaacaa agtcctcaat ctttgatagt ccagaaagga gggatttcaa 180
   ttataaactg tgcttatgag aacactgcgt ttgactactt tccatggtac caacaattcc 240
   ctgggaaagg ccctgcatta ttgatagcca tacgtccaga tgtgagtgaa aagaaagaag 300
   gaagattcac aatctccttc aataaaagtg ccaagcagtt ctcattgcat atcatggatt 360
   cccagcctgg agactcagcc acctacttct gcagagacct tgcggccgtg tcttcgacta 420
   gtagctcacc tacga 435
<210> 32
   <211> 417
   <212> DNA
   <213> Homo sapiens
<220>
   <223> TRAV24_BB_1
<400> 32
   gtcagatact ccatgagcac gaagacttgt acgccaccat ggagaagaat cctttggcag 60
   ccccattact aatcctctgg tttcatcttg actgcgtgag cagcatactg aacgtggaac 120
   aaagtcctca gtcactgcat gttcaggagg gagacagcac caatttcacc tgcagcttcc 180
   cttccagcaa tttttatgcc ttacactggt acagatggga aactgcaaaa agccccgagg 240
   ccttgtttgt aatgacttta aatggggatg aaaagaagaa aggacgaata agtgccactc 300
   ttaataccaa ggagggttac agctatttgt acatcaaagg atcccagcct gaagattcag 360
   ccacatacct ctgcagagac cttgcggccg tgtcttcgac tagtagctca cctacga 417
<210> 33
   <211> 402
   <212> DNA
   <213> Homo sapiens
<220>
   <223> TRAV25_BB_1
<400> 33
   gtcagatact ccatgagcac gaagacttgt acgccaccat gctactcatc acatcaatgt      60
   tggtcttatg gatgcaattg tcacaggtga atggacaaca ggtaatgcaa attcctcagt     120
   accagcatgt acaagaagga gaggacttca ccacgtactg caattcctca actactttaa     180
   gcaatataca gtggtataag caaaggcctg gtggacatcc cgtattcttg atacagttag     240
   tgaagagtgg agaagtgaag aagcagaaaa gactgacatt tcagtttgga gaagcaaaaa     300
   agaacagctc cctgcacatc acagccaccc agactacaga tgtaggaacc tacttctgca     360
   gagaccttgc ggccgtgtct tcgactagta gctcacctac ga                        402
<210> 34
   <211> 396
   <212> DNA
   <213> Homo sapiens
<220>
   <223> TRAV26-1_BB_1
<400> 34
   gtcagatact ccatgagcac gaagacttgt acgccaccat gaggctggtg gcaagagtaa      60
   ctgtgtttct gacctttgga actataattg atgctaagac cacccagccc ccctccatgg     120
   attgcgctga aggaagagct gcaaacctgc cttgtaatca ctctaccatc agtggaaatg     180
   agtatgtgta ttggtatcga cagattcact cccaggggcc acagtatatc attcatggtc     240
   taaaaaacaa tgaaaccaat gaaatggcct ctctgatcat cacagaagat agaaagtcca     300
   gcaccttgat cctgccccac gctacgctga gagacactgc tgtgtactac tgcagagacc     360
   ttgcggccgt gtcttcgact agtagctcac ctacga                               396
<210> 35
   <211> 396
   <212> DNA
   <213> Homo sapiens
<220>
   <223> TRAV26-2_BB_1
<400> 35
   gtcagatact ccatgagcac gaagacttgt acgccaccat gaagttggtg acaagcatta 60
   ctgtactcct atctttgggt attatgggtg atgctaagac cacacagcca aattcaatgg 120
   agagtaacga agaagagcct gttcacttgc cttgtaacca ctccacaatc agtggaactg 180
   attacataca ttggtatcga cagcttccct cccagggtcc agagtacgtg attcatggtc 240
   ttacaagcaa tgtgaacaac agaatggcct ctctggcaat cgctgaggac agaaagtcca 300
   gtaccttgat cctgcaccgt gctaccttga gagatgctgc tgtgtactac tgcagagacc 360
   ttgcggccgt gtcttcgact agtagctcac ctacga 396
<210> 36
   <211> 402
   <212> DNA
   <213> Homo sapiens
<220>
   <223> TRAV27_BB_1
<400> 36
   gtcagatact ccatgagcac gaagacttgt acgccaccat ggtcctgaaa ttctccgtgt 60
   ccattctttg gattcagttg gcatgggtga gcacccagct gctggagcag agccctcagt 120
   ttctaagcat ccaagaggga gaaaatctca ctgtgtactg caactcctca agtgtttttt 180
   ccagcttaca atggtacaga caggagcctg gggaaggtcc tgtcctcctg gtgacagtag 240
   ttacgggtgg agaagtgaag aagctgaaga gactaacctt tcagtttggt gatgcaagaa 300
   aggacagttc tctccacatc actgcagccc agcctggtga tacaggcctc tacctctgca 360
   gagaccttgc ggccgtgtct tcgactagta gctcacctac ga 402
<210> 37
   <211> 429
   <212> DNA
   <213> Homo sapiens
<220>
   <223> TRAV29DV5_BB_1
<400> 37
   gtcagatact ccatgagcac gaagacttgt acgccaccat ggccatgctc ctgggggcat 60
   cagtgctgat tctgtggctt cagccagact gggtaaacag tcaacagaag aatgatgacc 120
   agcaagttaa gcaaaattca ccatccctga gcgtccagga aggaagaatt tctattctga 180
   actgtgacta tactaacagc atgtttgatt atttcctatg gtacaaaaaa taccctgctg 240
   aaggtcctac attcctgata tctataagtt ccattaagga taaaaatgaa gatggaagat 300
   tcactgtttt cttaaacaaa agtgccaagc acctctctct gcacattgtg ccctcccagc 360
   ctggagactc tgcagtgtac ttctgcagag accttgcggc cgtgtcttcg actagtagct 420
   cacctacga 429
<210> 38
   <211> 408
   <212> DNA
   <213> Homo sapiens
<220>
   <223> TRAV30_BB_1
<400> 38
   gtcagatact ccatgagcac gaagacttgt acgccaccat ggagactctc ctgaaagtgc 60
   tttcaggcac cttgttgtgg cagttgacct gggtgagaag ccaacaacca gtgcagagtc 120
   ctcaagccgt gatcctccga gaaggggaag atgctgtcat caactgcagt tcctccaagg 180
   ctttatattc tgtacactgg tacaggcaga agcatggtga agcacccgtt ttcctgatga 240
   tattactgaa gggtggagaa cagaagggtc atgaaaaaat atctgcttca tttaatgaaa 300
   aaaagcagca aagctccctg taccttacgg cctcccagct cagttactca ggaacctact 360
   tctgcagaga ccttgcggcc gtgtcttcga ctagtagctc acctacga 408
<210> 39
   <211> 408
   <212> DNA
   <213> Homo sapiens
<220>
   <223> TRAV34_BB_1
<400> 39
   gtcagatact ccatgagcac gaagacttgt acgccaccat ggagactgtt ctgcaagtac 60
   tcctagggat attggggttc caagcagcct gggtcagtag ccaagaactg gagcagagtc 120
   ctcagtcctt gatcgtccaa gagggaaaga atctcaccat aaactgcacg tcatcaaaga 180
   cgttatatgg cttatactgg tataagcaaa agtatggtga aggtcttatc ttcttgatga 240
   tgctacagaa aggtggggaa gagaaaagtc atgaaaagat aactgccaag ttggatgaga 300
   aaaagcagca aagttccctg catatcacag cctcccagcc cagccatgca ggcatctacc 360
   tctgcagaga ccttgcggcc gtgtcttcga ctagtagctc acctacga 408
<210> 40
   <211> 402
   <212> DNA
   <213> Homo sapiens
<220>
   <223> TRAV35_BB_1
<400> 40
   gtcagatact ccatgagcac gaagacttgt acgccaccat gctccttgaa catttattaa 60
   taatcttgtg gatgcagctg acatgggtca gtggtcaaca gctgaatcag agtcctcaat 120
   ctatgtttat ccaggaagga gaagatgtct ccatgaactg cacttcttca agcatattta 180
   acacctggct atggtacaag caggaacctg gggaaggtcc tgtcctcttg atagccttat 240
   ataaggctgg tgaattgacc tcaaatggaa ggctgactgc tcagtttggt ataaccagaa 300
   aggacagctt cctgaatatc tcagcatcca tacctagtga tgtaggcatc tacttctgca 360
   gagaccttgc ggccgtgtct tcgactagta gctcacctac ga 402
<210> 41
   <211> 411
   <212> DNA
   <213> Homo sapiens
<220>
   <223> TRAV36DV7_BB_1
<400> 41
   gtcagatact ccatgagcac gaagacttgt acgccaccat gatgaagtgt ccacaggctt 60
   tactagctat cttttggctt ctactgagct gggtgagcag tgaagataag gtggtacaaa 120
   gccctctatc tctggttgtc cacgagggag acaccgtaac tctcaattgc agttatgaag 180
   tgactaactt tcgaagccta ctatggtaca agcaggaaaa gaaagctccc acatttctat 240
   ttatgctaac ttcaagtgga attgaaaaga agtcaggtag actaagtagc atattagata 300
   agaaagaact ttccagcatc ctgaacatca cagccaccca gaccggagac tcggccatct 360
   acctctgcag agaccttgcg gccgtgtctt cgactagtag ctcacctacg a 411
<210> 42
   <211> 417
   <212> DNA
   <213> Homo sapiens
<220>
   <223> TRAV38-1_BB_1
<400> 42
   gtcagatact ccatgagcac gaagacttgt acgccaccat gacacgagtt agcttgctgt      60
   gggcagtcgt ggtcagtacc tgtcttgaat ccggcatggc ccagacagtc actcagtctc     120
   aaccagagat gtctgtgcag gaggcagaga ctgtgaccct gagttgcaca tatgacacca     180
   gtgagaataa ttattatttg ttctggtaca agcagcctcc cagcaggcag atgattctcg     240
   ttattcgcca agaagcttat aagcaacaga atgcaacgga gaatcgtttc tctgtgaact     300
   tccagaaagc agccaaatcc ttcagtctca agatctcaga ctcacagctg ggggacactg      360
   cgatgtattt ctgcagagac cttgcggccg tgtcttcgac tagtagctca cctacga        417
<210> 43
   <211> 417
   <212> DNA
   <213> Homo sapiens
<220>
   <223> TRAV38-2DV8_BB_1
<400> 43
   gtcagatact ccatgagcac gaagacttgt acgccaccat ggcatgccct ggcttcctgt      60
   gggcacttgt gatctccacc tgtcttgaat ttagcatggc tcagacagtc actcagtctc     120
   aaccagagat gtctgtgcag gaggcagaga cggtgaccct gagctgcaca tatgacacca     180
   gtgagagtga ttattattta ttctggtaca agcagcctcc cagcaggcag atgattctcg     240
   ttattcgcca agaagcttat aagcaacaga atgcaacaga gaatcgtttc tctgtgaact     300
   tccagaaagc agccaaatcc ttcagtctca agatctcaga ctcacagctg ggggatgccg      360
   cgatgtattt ctgcagagac cttgcggccg tgtcttcgac tagtagctca cctacga        417
<210> 44
   <211> 402
   <212> DNA
   <213> Homo sapiens
<220>
   <223> TRAV39_BB_1
<400> 44
   gtcagatact ccatgagcac gaagacttgt acgccaccat gaagaagcta ctagcaatga 60
   ttctgtggct tcaactagac cggttaagtg gagagctgaa agtggaacaa aaccctctgt 120
   tcctgagcat gcaggaggga aaaaactata ccatctactg caattattca accacttcag 180
   acagactgta ttggtacagg caggatcctg ggaaaagtct ggaatctctg tttgtgttgc 240
   tatcaaatgg agcagtgaag caggagggac gattaatggc ctcacttgat accaaagccc 300
   gtctcagcac cctccacatc acagctgccg tgcatgacct ctctgccacc tacttctgca 360
   gagaccttgc ggccgtgtct tcgactagta gctcacctac ga 402
<210> 45
   <211> 387
   <212> DNA
   <213> Homo sapiens
<220>
   <223> TRAV40_BB_1
<400> 45
   gtcagatact ccatgagcac gaagacttgt acgccaccat gaactcctct ctggactttc 60
   taattctgat cttaatgttt ggaggaacca gcagcaattc agtcaagcag acgggccaaa 120
   taaccgtctc ggagggagca tctgtgacta tgaactgcac atacacatcc acggggtacc 180
   ctaccctttt ctggtatgtg gaatacccca gcaaacctct gcagcttctt cagagagaga 240
   caatggaaaa cagcaaaaac ttcggaggcg gaaatattaa agacaaaaac tcccccattg 300
   tgaaatattc agtccaggta tcagactcag ccgtgtacta ctgcagagac cttgcggccg 360
   tgtcttcgac tagtagctca cctacga 387
<210> 46
   <211> 408
   <212> DNA
   <213> Homo sapiens
<220>
   <223> TRAV41_BB_1
<400> 46
   gtcagatact ccatgagcac gaagacttgt acgccaccat ggtgaagatc cggcaatttt      60
   tgttggctat tttgtggctt cagctaagct gtgtaagtgc cgccaaaaat gaagtggagc     120
   agagtcctca gaacctgact gcccaggaag gagaatttat cacaatcaac tgcagttact     180
   cggtaggaat aagtgcctta cactggctgc aacagcatcc aggaggaggc attgtttcct     240
   tgtttatgct gagctcaggg aagaagaagc atggaagatt aattgccaca ataaacatac     300
   aggaaaagca cagctccctg cacatcacag cctcccatcc cagagactct gccgtctaca     360
   tctgcagaga ccttgcggcc gtgtcttcga ctagtagctc acctacga                  408
<210> 47
   <211> 497
   <212> DNA
   <213> Homo sapiens
<220>
   <223> TRAC1 cloning fragment
<400> 47
   gtgcactcct atgactaacg gaagactagg ccgcataggt ctcaccagaa ccctgaccct      60
   gccgtgtacc agctgagaga ctctaaatcc agtgacaagt ctgtctgcct attcaccgat     120
   tttgattctc aaacaaatgt gtcacaaagt aaggattctg atgtgtatat cacagacaaa     180
   actgtgctag acatgaggtc tatggacttc aagagcaaca gtgctgtggc ctggagcaac     240
   aaatctgact ttgcatgtgc aaacgccttc aacaacagca ttattccaga ggacaccttc     300
   ttccccagcc cagaaagttc ctgtgatgtc aagctggtcg agaaaagctt tgaaacagat     360
   acgaacctaa actttcaaaa cctgtcagtg attgggttcc gaatcctcct cctgaaagtg     420
   gccgggttta atctgctcat gacgctgcgg ctgtggtcca gctgactagg tgtcttccct     480
   atgctgaatc gatggtc                                                    497
<210> 48
   <211> 3210
   <212> DNA
   <213> Artificial Sequence
<220>
   <223> V-C entry backbone transient
<400> 48
   tctagacctg atcataatca agccatatca catctgtaga ggtttacttg ctttaaaaaa      60
   cctccacacc tccccctgaa cctgaaacat aaaatgaatg caattgttgt tgttaacttg    120
   tttattgcag cttataatgg ttacaaataa agcaatagca tcacaaattt cacaaataaa     180
   gcattttttt cactgcattc tagttgtggt ttgtccaaac tcatcaatgt atcttatcat     240
   gtctggatct gcggatccaa tctcgagctg ggcctcatgg gccttccgct cactgcccgc     300
   tttccagtcg ggaaacctgt cgtgccagct gcattaacat ggtcatagct gtttccttgc     360
   gtattgggcg ctctccgctt cctcgctcac tgactcgctg cgctcggtcg ttcgggtaaa     420
   gcctggggtg cctaatgagc aaaaggccag caaaaggcca ggaaccgtaa aaaggccgcg     480
   ttgctggcgt ttttccatag gctccgcccc cctgacgagc atcacaaaaa tcgacgctca     540
   agtcagaggt ggcgaaaccc gacaggacta taaagatacc aggcgtttcc ccctggaagc     600
   tccctcgtgc gctctcctgt tccgaccctg ccgcttaccg gatacctgtc cgcctttctc     660
   ccttcgggaa gcgtggcgct ttctcatagc tcacgctgta ggtatctcag ttcggtgtag     720
   gtcgttcgct ccaagctggg ctgtgtgcac gaaccccccg ttcagcccga ccgctgcgcc     780
   ttatccggta actatcgtct tgagtccaac ccggtaagac acgacttatc gccactggca     840
   gcagccactg gtaacaggat tagcagagcg aggtatgtag gcggtgctac agagttcttg     900
   aagtggtggc ctaactacgg ctacactaga agaacagtat ttggtatctg cgctctgctg     960
   aagccagtta ccttcggaaa aagagttggt agctcttgat ccggcaaaca aaccaccgct    1020
   ggtagcggtg gtttttttgt ttgcaagcag cagattacgc gcagaaaaaa aggatctcaa    1080
   gaagatcctt tgatcttttc tacggggtct gacgctcagt ggaacgaaaa ctcacgttaa    1140
   gggattttgg tcatgagatt atcaaaaagg atcttcacct agatcctttt aaattaaaaa    1200
   tgaagtttta aatcaatcta aagtatatat gagtaaactt ggtctgacag ttaccaatgc 1260
   ttaatcagtg aggcacctat ctcagcgatc tgtctatttc gttcatccat agttgcctga 1320
   ctccccgtcg tgtagataac tacgatacgg gagggcttac catctggccc cagtgctgca 1380
   atgataccgc gagaaccacg ctcaccggct ccagatttat cagcaataaa ccagccagcc 1440
   ggaagggccg agcgcagaag tggtcctgca actttatccg cctccatcca gtctattaat 1500
   tgttgccggg aagctagagt aagtagttcg ccagttaata gtttgcgcaa cgttgttgcc 1560
   attgctacag gcatcgtggt gtcacgctcg tcgtttggta tggcttcatt cagctccggt 1620
   tcccaacgat caaggcgagt tacatgatcc cccatgttgt gcaaaaaagc ggttagctcc 1680
   ttcggtcctc cgatcgttgt cagaagtaag ttggccgcag tgttatcact catggttatg 1740
   gcagcactgc ataattctct tactgtcatg ccatccgtaa gatgcttttc tgtgactggt 1800
   gagtactcaa ccaagtcatt ctgagaatag tgtatgcggc gaccgagttg ctcttgcccg 1860
   gcgtcaatac gggataatac cgcgccacat agcagaactt taaaagtgct catcattgga 1920
   aaacgttctt cggggcgaaa actctcaagg atcttaccgc tgttgagatc cagttcgatg 1980
   taacccactc gtgcacccaa ctgatcttca gcatctttta ctttcaccag cgtttctggg 2040
   tgagcaaaaa caggaaggca aaatgccgca aaaaagggaa taagggcgac acggaaatgt 2100
   tgaatactca tactcttcct ttttcaatat tattgaagca tttatcaggg ttattgtctc 2160
   atgagcggat acatatttga atgtatttag aaaaataaac aaataggggt tccgcgcaca 2220
   tttccccgaa aagtgccacc taaattgtaa gcgttaatat tttgttaaaa ttcgcgttaa 2280
   atttttgtta aatcagctca ttttttaacc aataggccga aatcggcaaa atcccttata 2340
   aatcaaaaga atagaccgag atagggttga gtggccgcta cagggcgctc ccattcgcca 2400
   ttcaggctgc gcaactgttg ggaagggcgt ttcggtgcgg gcctcttcgc tattacgcca 2460
   gctggcgaaa gggggatgtg ctgcaaggcg attaagttgg gtaacgccag ggttttccca 2520
   gtcacgacgt tgtaaaacga cggccagtga gcgcgacgta atacgactca ctatagggcg 2580
   aattggcgga aggccgtcaa ggccgcatga attcgctacc ggtatagtaa tcaattacgg 2640
   ggtcattagt tcatagccca tatatggagt tccgcgttac ataacttacg gtaaatggcc 2700
   cgcctggctg accgcccaac gacccccgcc cattgacgtc aataatgacg tatgttccca 2760
   tagtaacgcc aatagggact ttccattgac gtcaatgggt ggagtattta cggtaaactg 2820
   cccacttggc agtacatcaa gtgtatcata tgccaagtac gccccctatt gacgtcaatg 2880
   acggtaaatg gcccgcctgg cattatgccc agtacatgac cttatgggac tttcctactt 2940
   ggcagtacat ctacgtatta gtcatcgcta ttaccatggt gatgcggttt tggcagtaca 3000
   tcaatgggcg tggatagcgg tttgactcac ggggatttcc aagtctccac cccattgacg 3060
   tcaatgggag tttgttttgg caccaaaatc aacgggactt tccaaaatgt cgtaacaact 3120
   ccgccccatt gacgcaaatg ggcggtaggc gtgtacggtg ggaggtctat ataagcagag 3180
   ctggtttagt gaaccgtcag atcaggtacc 3210
<210> 49
   <211> 769
   <212> DNA
   <213> Homo sapiens
<220>
   <223> V-C entry TRAV1-1_TRAC
<400> 49
   gccaccatgt ggggagcttt ccttctctat gtttccatga agatgggagg cactgcagga 60
   caaagccttg agcagccctc tgaagtgaca gctgtggaag gagccattgt ccagataaac 120
   tgcacgtacc agacatctgg gttttatggg ctgtcctggt accagcaaca tgatggcgga 180
   gcacccacat ttctttctta caatgctctg gatggtttgg aggagacagg tcgtttttct 240
   tcattcctta gtcgctctga tagttatggt tacctccttc tacaggagct ccagatgaaa 300
   gactctgcct cttacttctg cagagacctt gcggccgcat aggtctcacc agaaccctga 360
   ccctgccgtg taccagctga gagactctaa atccagtgac aagtctgtct gcctattcac 420
   cgattttgat tctcaaacaa atgtgtcaca aagtaaggat tctgatgtgt atatcacaga 480
   caaaactgtg ctagacatga ggtctatgga cttcaagagc aacagtgctg tggcctggag 540
   caacaaatct gactttgcat gtgcaaacgc cttcaacaac agcattattc cagaggacac 600
   cttcttcccc agcccagaaa gttcctgtga tgtcaagctg gtcgagaaaa gctttgaaac 660
   agatacgaac ctaaactttc aaaacctgtc agtgattggg ttccgaatcc tcctcctgaa 720
   agtggccggg tttaatctgc tcatgacgct gcggctgtgg tccagctga 769
<210> 50
   <211> 769
   <212> DNA
   <213> Homo sapiens
<220>
   <223> V-C entry TRAV1-2_TRAC
<400> 50
   gccaccatgt ggggagtttt ccttctttat gtttccatga agatgggagg cactacagga      60
   caaaacattg accagcccac tgagatgaca gctacggaag gtgccattgt ccagatcaac     120
   tgcacgtacc agacatctgg gttcaacggg ctgttctggt accagcaaca tgctggcgaa     180
   gcacccacat ttctgtctta caatgttctg gatggtttgg aggagaaagg tcgtttttct     240
   tcattcctta gtcggtctaa agggtacagt tacctccttt tgaaggagct ccagatgaaa     300
   gactctgcct cttacctctg cagagacctt gcggccgcat aggtctcacc agaaccctga     360
   ccctgccgtg taccagctga gagactctaa atccagtgac aagtctgtct gcctattcac     420
   cgattttgat tctcaaacaa atgtgtcaca aagtaaggat tctgatgtgt atatcacaga     480
   caaaactgtg ctagacatga ggtctatgga cttcaagagc aacagtgctg tggcctggag     540
   caacaaatct gactttgcat gtgcaaacgc cttcaacaac agcattattc cagaggacac     600
   cttcttcccc agcccagaaa gttcctgtga tgtcaagctg gtcgagaaaa gctttgaaac     660
   agatacgaac ctaaactttc aaaacctgtc agtgattggg ttccgaatcc tcctcctgaa     720
   agtggccggg tttaatctgc tcatgacgct gcggctgtgg tccagctga                 769
<210> 51
   <211> 781
   <212> DNA
   <213> Homo sapiens
<220>
   <223> V-C entry TRAV2_TRAC
<400> 51
   gccaccatgg ctttgcagag cactctgggg gcggtgtggc tagggcttct cctcaactct 60
   ctctggaagg ttgcagaaag caaggaccaa gtgtttcagc cttccacagt ggcatcttca 120
   gagggagctg tggtggaaat cttctgtaat cactctgtgt ccaatgctta caacttcttc 180
   tggtaccttc acttcccggg atgtgcacca agactccttg ttaaaggctc aaagccttct 240
   cagcagggac gatacaacat gacctatgaa cggttctctt catcgctgct catcctccag 300
   gtgcgggagg cagatgctgc tgtttactac tgcagagacc ttgcggccgc ataggtctca 360
   ccagaaccct gaccctgccg tgtaccagct gagagactct aaatccagtg acaagtctgt 420
   ctgcctattc accgattttg attctcaaac aaatgtgtca caaagtaagg attctgatgt 480
   gtatatcaca gacaaaactg tgctagacat gaggtctatg gacttcaaga gcaacagtgc 540
   tgtggcctgg agcaacaaat ctgactttgc atgtgcaaac gccttcaaca acagcattat 600
   tccagaggac accttcttcc ccagcccaga aagttcctgt gatgtcaagc tggtcgagaa 660
   aagctttgaa acagatacga acctaaactt tcaaaacctg tcagtgattg ggttccgaat 720
   cctcctcctg aaagtggccg ggtttaatct gctcatgacg ctgcggctgt ggtccagctg 780
   a 781
<210> 52
   <211> 784
   <212> DNA
   <213> Homo sapiens
<220>
   <223> V-C entry TRAV3_TRAC
<400> 52
   gccaccatgg cctctgcacc catctcgatg cttgcgatgc tcttcacatt gagtgggctg 60
   agagctcagt cagtggctca gccggaagat caggtcaacg ttgctgaagg gaatcctctg 120
   actgtgaaat gcacctattc agtctctgga aacccttatc ttttttggta tgttcaatac 180
   cccaaccgag gcctccagtt ccttctgaaa tacatcacag gggataacct ggttaaaggc 240
   agctatggct ttgaagctga atttaacaag agccaaacct ccttccacct gaagaaacca 300
   tctgcccttg tgagcgactc cgctttgtac ttctgcagag accttgcggc cgcataggtc 360
   tcaccagaac cctgaccctg ccgtgtacca gctgagagac tctaaatcca gtgacaagtc 420
   tgtctgccta ttcaccgatt ttgattctca aacaaatgtg tcacaaagta aggattctga 480
   tgtgtatatc acagacaaaa ctgtgctaga catgaggtct atggacttca agagcaacag 540
   tgctgtggcc tggagcaaca aatctgactt tgcatgtgca aacgccttca acaacagcat 600
   tattccagag gacaccttct tccccagccc agaaagttcc tgtgatgtca agctggtcga 660
   gaaaagcttt gaaacagata cgaacctaaa ctttcaaaac ctgtcagtga ttgggttccg 720
   aatcctcctc ctgaaagtgg ccgggtttaa tctgctcatg acgctgcggc tgtggtccag 780
   ctga 784
<210> 53
   <211> 769
   <212> DNA
   <213> Homo sapiens
<220>
   <223> V-C entry TRAV4_TRAC
<400> 53
   gccaccatga ggcaagtggc gagagtgatc gtgttcctga ccctgagtac tttgagcctt 60
   gctaagacca cccagcccat ctccatggac tcatatgaag gacaagaagt gaacataacc 120
   tgtagccaca acaacattgc tacaaatgat tatatcacgt ggtaccaaca gtttcccagc 180
   caaggaccac gatttattat tcaaggatac aagacaaaag ttacaaacga agtggcctcc 240
   ctgtttatcc ctgccgacag aaagtccagc actctgagcc tgccccgggt ttccctgagc 300
   gacactgctg tgtactactg cagagacctt gcggccgcat aggtctcacc agaaccctga 360
   ccctgccgtg taccagctga gagactctaa atccagtgac aagtctgtct gcctattcac 420
   cgattttgat tctcaaacaa atgtgtcaca aagtaaggat tctgatgtgt atatcacaga 480
   caaaactgtg ctagacatga ggtctatgga cttcaagagc aacagtgctg tggcctggag 540
   caacaaatct gactttgcat gtgcaaacgc cttcaacaac agcattattc cagaggacac 600
   cttcttcccc agcccagaaa gttcctgtga tgtcaagctg gtcgagaaaa gctttgaaac 660
   agatacgaac ctaaactttc aaaacctgtc agtgattggg ttccgaatcc tcctcctgaa 720
   agtggccggg tttaatctgc tcatgacgct gcggctgtgg tccagctga 769
<210> 54
   <211> 784
   <212> DNA
   <213> Homo sapiens
<220>
   <223> V-C entry TRAV5_TRAC
<400> 54
   gccaccatga aaacatttgc tggattttcg ttcctgtttt tgtggctgca gctggactgt      60
   atgagtagag gagaggatgt ggagcagagt cttttcctga gtgtccgaga gggagacagc     120
   tccgttataa actgcactta cacagacagc tcctccacct acttatactg gtataagcaa     180
   gaacctggag caggtctaca gttgctgacg tatatttttt caaatatgga catgaaacaa     240
   gaccaaagac tcactgttct attgaataaa aaggataaac atctgtctct gcgcattgca     300
   gacacccaga ctggggactc agctatctac ttctgcagag accttgcggc cgcataggtc     360
   tcaccagaac cctgaccctg ccgtgtacca gctgagagac tctaaatcca gtgacaagtc     420
   tgtctgccta ttcaccgatt ttgattctca aacaaatgtg tcacaaagta aggattctga     480
   tgtgtatatc acagacaaaa ctgtgctaga catgaggtct atggacttca agagcaacag     540
   tgctgtggcc tggagcaaca aatctgactt tgcatgtgca aacgccttca acaacagcat     600
   tattccagag gacaccttct tccccagccc agaaagttcc tgtgatgtca agctggtcga     660
   gaaaagcttt gaaacagata cgaacctaaa ctttcaaaac ctgtcagtga ttgggttccg     720
   aatcctcctc ctgaaagtgg ccgggtttaa tctgctcatg acgctgcggc tgtggtccag     780
   ctga                                                                  784
<210> 55
   <211> 784
   <212> DNA
   <213> Homo sapiens
<220>
   <223> V-C entry TRAV6_TRAC
<400> 55
   gccaccatgg agtcattcct gggaggtgtt ttgctgattt tgtggcttca agtggactgg      60
   gtgaagagcc aaaagataga acagaattcc gaggccctga acattcagga gggtaaaacg     120
   gccaccctga cctgcaacta tacaaactat tccccagcat acttacagtg gtaccgacaa     180
   gatccaggaa gaggccctgt tttcttgcta ctcatacgtg aaaatgagaa agaaaaaagg     240
   aaagaaagac tgaaggtcac ctttgatacc acccttaaac agagtttgtt tcatatcaca     300
   gcctcccagc ctgcagactc agctacctac ctctgcagag accttgcggc cgcataggtc     360
   tcaccagaac cctgaccctg ccgtgtacca gctgagagac tctaaatcca gtgacaagtc     420
   tgtctgccta ttcaccgatt ttgattctca aacaaatgtg tcacaaagta aggattctga     480
   tgtgtatatc acagacaaaa ctgtgctaga catgaggtct atggacttca agagcaacag 540
   tgctgtggcc tggagcaaca aatctgactt tgcatgtgca aacgccttca acaacagcat 600
   tattccagag gacaccttct tccccagccc agaaagttcc tgtgatgtca agctggtcga 660
   gaaaagcttt gaaacagata cgaacctaaa ctttcaaaac ctgtcagtga ttgggttccg 720
   aatcctcctc ctgaaagtgg ccgggtttaa tctgctcatg acgctgcggc tgtggtccag 780
   ctga 784
<210> 56
   <211> 781
   <212> DNA
   <213> Homo sapiens
<220>
   <223> V-C entry TRAV7_TRAC
<400> 56
   gccaccatgg agaagatgcg tagacctgtc ctaattatat tttgtctatg tcttggctgg 60
   gcaaatggag aaaaccaggt ggagcacagc cctcattttc tgggacccca gcagggagac 120
   gttgcctcca tgagctgcac gtactctgtc agtcgtttta acaatttgca gtggtacagg 180
   caaaatacag ggatgggtcc caaacaccta ttatccatgt attcagctgg atatgagaag 240
   cagaaaggaa ggctaaatgc tacattactg aagaatggaa gcagcttgta cattacagcc 300
   gtgcagcctg aagattcagc cacctatttc tgcagagacc ttgcggccgc ataggtctca 360
   ccagaaccct gaccctgccg tgtaccagct gagagactct aaatccagtg acaagtctgt 420
   ctgcctattc accgattttg attctcaaac aaatgtgtca caaagtaagg attctgatgt 480
   gtatatcaca gacaaaactg tgctagacat gaggtctatg gacttcaaga gcaacagtgc 540
   tgtggcctgg agcaacaaat ctgactttgc atgtgcaaac gccttcaaca acagcattat 600
   tccagaggac accttcttcc ccagcccaga aagttcctgt gatgtcaagc tggtcgagaa 660
   aagctttgaa acagatacga acctaaactt tcaaaacctg tcagtgattg ggttccgaat 720
   cctcctcctg aaagtggccg ggtttaatct gctcatgacg ctgcggctgt ggtccagctg 780
   a 781
<210> 57
   <211> 784
   <212> DNA
   <213> Homo sapiens
<220>
   <223> V-C entry TRAV8-1_TRAC
<400> 57
   gccaccatgc tcctgttgct cataccagtg ctggggatga tttttgccct gagagatgcc      60
   agagcccagt ctgtgagcca gcataaccac cacgtaattc tctctgaagc agcctcactg     120
   gagttgggat gcaactattc ctatggtgga actgttaatc tcttctggta tgtccagtac     180
   cctggtcaac accttcagct tctcctcaag tacttttcag gggatccact ggttaaaggc    240
   atcaagggct ttgaggctga atttataaag agtaaattct cctttaatct gaggaaaccc     300
   tctgtgcagt ggagtgacac agctgagtac ttctgcagag accttgcggc cgcataggtc     360
   tcaccagaac cctgaccctg ccgtgtacca gctgagagac tctaaatcca gtgacaagtc     420
   tgtctgccta ttcaccgatt ttgattctca aacaaatgtg tcacaaagta aggattctga     480
   tgtgtatatc acagacaaaa ctgtgctaga catgaggtct atggacttca agagcaacag     540
   tgctgtggcc tggagcaaca aatctgactt tgcatgtgca aacgccttca acaacagcat     600
   tattccagag gacaccttct tccccagccc agaaagttcc tgtgatgtca agctggtcga     660
   gaaaagcttt gaaacagata cgaacctaaa ctttcaaaac ctgtcagtga ttgggttccg     720
   aatcctcctc ctgaaagtgg ccgggtttaa tctgctcatg acgctgcggc tgtggtccag     780
   ctga                                                                  784
<210> 58
   <211> 784
   <212> DNA
   <213> Homo sapiens
<220>
   <223> V-C entry TRAV8-2_TRAC
<400> 58
   gccaccatgc tcctgctgct cgtcccagtg ctcgaggtga tttttactct gggaggaacc      60
   agagcccagt cggtgaccca gcttgacagc cacgtctctg tctctgaagg aaccccggtg     120
   ctgctgaggt gcaactactc atcttcttat tcaccatctc tcttctggta tgtgcaacac     180
   cccaacaaag gactccagct tctcctgaag tacacatcag cggccaccct ggttaaaggc     240
   atcaacggtt ttgaggctga atttaagaag agtgaaacct ccttccacct gacgaaaccc     300
   tcagcccata tgagcgacgc ggctgagtac ttctgcagag accttgcggc cgcataggtc     360
   tcaccagaac cctgaccctg ccgtgtacca gctgagagac tctaaatcca gtgacaagtc     420
   tgtctgccta ttcaccgatt ttgattctca aacaaatgtg tcacaaagta aggattctga     480
   tgtgtatatc acagacaaaa ctgtgctaga catgaggtct atggacttca agagcaacag     540
   tgctgtggcc tggagcaaca aatctgactt tgcatgtgca aacgccttca acaacagcat     600
   tattccagag gacaccttct tccccagccc agaaagttcc tgtgatgtca agctggtcga     660
   gaaaagcttt gaaacagata cgaacctaaa ctttcaaaac ctgtcagtga ttgggttccg     720
   aatcctcctc ctgaaagtgg ccgggtttaa tctgctcatg acgctgcggc tgtggtccag     780
   ctga                                                                  784
<210> 59
   <211> 784
   <212> DNA
   <213> Homo sapiens
<220>
   <223> V-C entry TRAV8-3_TRAC
<400> 59
   gccaccatgc tcctggagct tatcccactg ctggggatac attttgtcct gagaactgcc 60
   agagcccagt cagtgaccca gcctgacatc cacatcactg tctctgaagg agcctcactg 120
   gagttgagat gtaactattc ctatggggca acaccttatc tcttctggta tgtccagtcc 180
   cccggccaag gcctccagct gctcctgaag tacttttcag gagacactct ggttcaaggc 240
   attaaaggct ttgaggctga atttaagagg agtcaatctt ccttcaatct gaggaaaccc 300
   tctgtgcatt ggagtgatgc tgctgagtac ttctgcagag accttgcggc cgcataggtc 360
   tcaccagaac cctgaccctg ccgtgtacca gctgagagac tctaaatcca gtgacaagtc 420
   tgtctgccta ttcaccgatt ttgattctca aacaaatgtg tcacaaagta aggattctga 480
   tgtgtatatc acagacaaaa ctgtgctaga catgaggtct atggacttca agagcaacag 540
   tgctgtggcc tggagcaaca aatctgactt tgcatgtgca aacgccttca acaacagcat 600
   tattccagag gacaccttct tccccagccc agaaagttcc tgtgatgtca agctggtcga 660
   gaaaagcttt gaaacagata cgaacctaaa ctttcaaaac ctgtcagtga ttgggttccg 720
   aatcctcctc ctgaaagtgg ccgggtttaa tctgctcatg acgctgcggc tgtggtccag 780
   ctga 784
<210> 60
   <211> 784
   <212> DNA
   <213> Homo sapiens
<220>
   <223> V-C entry TRAV8-4_TRAC
<400> 60
   gccaccatgc tcctgctgct cgtcccagtg ctcgaggtga tttttaccct gggaggaacc 60
   agagcccagt cggtgaccca gcttggcagc cacgtctctg tctctgaagg agccctggtt 120
   ctgctgaggt gcaactactc atcgtctgtt ccaccatatc tcttctggta tgtgcaatac 180
   cccaaccaag gactccagct tctcctgaag tacacatcag cggccaccct ggttaaaggc 240
   atcaacggtt ttgaggctga atttaagaag agtgaaacct ccttccacct gacgaaaccc 300
   tcagcccata tgagcgacgc ggctgagtac ttctgcagag accttgcggc cgcataggtc 360
   tcaccagaac cctgaccctg ccgtgtacca gctgagagac tctaaatcca gtgacaagtc 420
   tgtctgccta ttcaccgatt ttgattctca aacaaatgtg tcacaaagta aggattctga 480
   tgtgtatatc acagacaaaa ctgtgctaga catgaggtct atggacttca agagcaacag 540
   tgctgtggcc tggagcaaca aatctgactt tgcatgtgca aacgccttca acaacagcat 600
   tattccagag gacaccttct tccccagccc agaaagttcc tgtgatgtca agctggtcga 660
   gaaaagcttt gaaacagata cgaacctaaa ctttcaaaac ctgtcagtga ttgggttccg 720
   aatcctcctc ctgaaagtgg ccgggtttaa tctgctcatg acgctgcggc tgtggtccag 780
   ctga 784
<210> 61
   <211> 784
   <212> DNA
   <213> Homo sapiens
<220>
   <223> V-C entry TRAV8-6_TRAC
<400> 61
   gccaccatgc tcctgctgct cgtcccagcg ttccaggtga tttttaccct gggaggaacc 60
   agagcccagt ctgtgaccca gcttgacagc caagtccctg tctttgaaga agcccctgtg 120
   gagctgaggt gcaactactc atcgtctgtt tcagtgtatc tcttctggta tgtgcaatac 180
   cccaaccaag gactccagct tctcctgaag tatttatcag gatccaccct ggttaaaggc 240
   atcaacggtt ttgaggctga atttaacaag agtcaaactt ccttccactt gaggaaaccc 300
   tcagtccata taagcgacac ggctgagtac ttctgcagag accttgcggc cgcataggtc 360
   tcaccagaac cctgaccctg ccgtgtacca gctgagagac tctaaatcca gtgacaagtc 420
   tgtctgccta ttcaccgatt ttgattctca aacaaatgtg tcacaaagta aggattctga 480
   tgtgtatatc acagacaaaa ctgtgctaga catgaggtct atggacttca agagcaacag 540
   tgctgtggcc tggagcaaca aatctgactt tgcatgtgca aacgccttca acaacagcat 600
   tattccagag gacaccttct tccccagccc agaaagttcc tgtgatgtca agctggtcga 660
   gaaaagcttt gaaacagata cgaacctaaa ctttcaaaac ctgtcagtga ttgggttccg 720
   aatcctcctc ctgaaagtgg ccgggtttaa tctgctcatg acgctgcggc tgtggtccag 780
   ctga 784
<210> 62
   <211> 781
   <212> DNA
   <213> Homo sapiens
<220>
   <223> V-C entry TRAV8-7_TRAC
<400> 62
   gccaccatgc tcttagtggt cattctgctg cttggaatgt tcttcacact gagaaccaga      60
   acccagtcgg tgacccagct tgatggccac atcactgtct ctgaagaagc ccctctggaa     120
   ctgaagtgca actattccta tagtggagtt ccttctctct tctggtatgt ccaatactct     180
   agccaaagcc tccagcttct cctcaaagac ctaacagagg ccacccaggt taaaggcatc     240
   agaggttttg aggctgaatt taagaagagc gaaacctcct tctacctgag gaaaccatca     300
   acccatgtga gtgatgctgc tgagtacttc tgcagagacc ttgcggccgc ataggtctca     360
   ccagaaccct gaccctgccg tgtaccagct gagagactct aaatccagtg acaagtctgt     420
   ctgcctattc accgattttg attctcaaac aaatgtgtca caaagtaagg attctgatgt     480
   gtatatcaca gacaaaactg tgctagacat gaggtctatg gacttcaaga gcaacagtgc     540
   tgtggcctgg agcaacaaat ctgactttgc atgtgcaaac gccttcaaca acagcattat     600
   tccagaggac accttcttcc ccagcccaga aagttcctgt gatgtcaagc tggtcgagaa     660
   aagctttgaa acagatacga acctaaactt tcaaaacctg tcagtgattg ggttccgaat     720
   cctcctcctg aaagtggccg ggtttaatct gctcatgacg ctgcggctgt ggtccagctg     780
   a                                                                     781
<210> 63
   <211> 781
   <212> DNA
   <213> Homo sapiens
<220>
   <223> V-C entry TRAV9-1_TRAC
<400> 63
   gccaccatga attcttctcc aggaccagcg attgcactat tcttaatgtt tgggggaatc      60
   aatggagatt cagtggtcca gacagaaggc caagtgctcc cctctgaagg ggattccctg     120
   attgtgaact gctcctatga aaccacacag tacccttccc ttttttggta tgtccaatat     180
   cctggagaag gtccacagct ccacctgaaa gccatgaagg ccaatgacaa gggaaggaac     240
   aaaggttttg aagccatgta ccgtaaagaa accacttctt tccacttgga gaaagactca     300
   gttcaagagt cagactccgc tgtgtacttc tgcagagacc ttgcggccgc ataggtctca     360
   ccagaaccct gaccctgccg tgtaccagct gagagactct aaatccagtg acaagtctgt     420
   ctgcctattc accgattttg attctcaaac aaatgtgtca caaagtaagg attctgatgt     480
   gtatatcaca gacaaaactg tgctagacat gaggtctatg gacttcaaga gcaacagtgc     540
   tgtggcctgg agcaacaaat ctgactttgc atgtgcaaac gccttcaaca acagcattat     600
   tccagaggac accttcttcc ccagcccaga aagttcctgt gatgtcaagc tggtcgagaa     660
   aagctttgaa acagatacga acctaaactt tcaaaacctg tcagtgattg ggttccgaat     720
   cctcctcctg aaagtggccg ggtttaatct gctcatgacg ctgcggctgt ggtccagctg     780
   a                                                                     781
<210> 64
   <211> 781
   <212> DNA
   <213> Homo sapiens
<220>
   <223> V-C entry TRAV9-2_TRAC
<400> 64
   gccaccatga actattctcc aggcttagta tctctgatac tcttactgct tggaagaacc 60
   cgtggaaatt cagtgaccca gatggaaggg ccagtgactc tctcagaaga ggccttcctg     120
   actataaact gcacgtacac agccacagga tacccttccc ttttctggta tgtccaatat     180
   cctggagaag gtctacagct cctcctgaaa gccacgaagg ctgatgacaa gggaagcaac 240
   aaaggttttg aagccacata ccgtaaagaa accacttctt tccacttgga gaaaggctca 300
   gttcaagtgt cagactcagc ggtgtacttc tgcagagacc ttgcggccgc ataggtctca 360
   ccagaaccct gaccctgccg tgtaccagct gagagactct aaatccagtg acaagtctgt 420
   ctgcctattc accgattttg attctcaaac aaatgtgtca caaagtaagg attctgatgt 480
   gtatatcaca gacaaaactg tgctagacat gaggtctatg gacttcaaga gcaacagtgc 540
   tgtggcctgg agcaacaaat ctgactttgc atgtgcaaac gccttcaaca acagcattat 600
   tccagaggac accttcttcc ccagcccaga aagttcctgt gatgtcaagc tggtcgagaa 660
   aagctttgaa acagatacga acctaaactt tcaaaacctg tcagtgattg ggttccgaat 720
   cctcctcctg aaagtggccg ggtttaatct gctcatgacg ctgcggctgt ggtccagctg 780
   a 781
<210> 65
   <211> 787
   <212> DNA
   <213> Homo sapiens
<220>
   <223> V-C entry TRAV10-TRAC
<400> 65
   gccaccatga aaaagcatct gacgaccttc ttggtgattt tgtggcttta tttttatagg 60
   gggaatggca aaaaccaagt ggagcagagt cctcagtccc tgatcatcct ggagggaaag 120
   aactgcactc ttcaatgcaa ttatacagtg agccccttca gcaacttaag gtggtataag 180
   caagatactg ggagaggtcc tgtttccctg acaatcatga ctttcagtga gaacacaaag 240
   tcgaacggaa gatatacagc aactctggat gcagacacaa agcaaagctc tctgcacatc 300
   acagcctccc agctcagcga ttcagcctcc tacatctgca gagaccttgc ggccgcatag 360
   gtctcaccag aaccctgacc ctgccgtgta ccagctgaga gactctaaat ccagtgacaa 420
   gtctgtctgc ctattcaccg attttgattc tcaaacaaat gtgtcacaaa gtaaggattc      480
   tgatgtgtat atcacagaca aaactgtgct agacatgagg tctatggact tcaagagcaa      540
   cagtgctgtg gcctggagca acaaatctga ctttgcatgt gcaaacgcct tcaacaacag      600
   cattattcca gaggacacct tcttccccag cccagaaagt tcctgtgatg tcaagctggt      660
   cgagaaaagc tttgaaacag atacgaacct aaactttcaa aacctgtcag tgattgggtt      720
   ccgaatcctc ctcctgaaag tggccgggtt taatctgctc atgacgctgc ggctgtggtc      780
   cagctga                                                                787
<210> 66
   <211> 781
   <212> DNA
   <213> Homo sapiens
<220>
   <223> V-C entry TRAV12-1_TRAC
<400> 66
   gccaccatga tatccttgag agttttactg gtgatcctgt ggcttcagtt aagctgggtt       60
   tggagccaac ggaaggaggt ggagcaggat cctggaccct tcaatgttcc agagggagcc      120
   actgtcgctt tcaactgtac ttacagcaac agtgcttctc agtctttctt ctggtacaga      180
   caggattgca ggaaagaacc taagttgctg atgtccgtat actccagtgg taatgaagat      240
   ggaaggttta cagcacagct caatagagcc agccagtata tttccctgct catcagagac      300
   tccaagctca gtgattcagc cacctacctc tgcagagacc ttgcggccgc ataggtctca      360
   ccagaaccct gaccctgccg tgtaccagct gagagactct aaatccagtg acaagtctgt      420
   ctgcctattc accgattttg attctcaaac aaatgtgtca caaagtaagg attctgatgt      480
   gtatatcaca gacaaaactg tgctagacat gaggtctatg gacttcaaga gcaacagtgc      540
   tgtggcctgg agcaacaaat ctgactttgc atgtgcaaac gccttcaaca acagcattat      600
   tccagaggac accttcttcc ccagcccaga aagttcctgt gatgtcaagc tggtcgagaa      660
   aagctttgaa acagatacga acctaaactt tcaaaacctg tcagtgattg ggttccgaat      720
   cctcctcctg aaagtggccg ggtttaatct gctcatgacg ctgcggctgt ggtccagctg      780
   a                                                                      781
<210> 67
   <211> 787
   <212> DNA
   <213> Homo sapiens
<220>
   <223> V-C entry TRAV12-2_TRAC
<400> 67
   gccaccatga tgaaatcctt gagagtttta ctagtgatcc tgtggcttca gttgagctgg 60
   gtttggagcc aacagaagga ggtggagcag aattctggac ccctcagtgt tccagaggga 120
   gccattgcct ctctcaactg cacttacagt gaccgaggtt cccagtcctt cttctggtac 180
   agacaatatt ctgggaaaag ccctgagttg ataatgttca tatactccaa tggtgacaaa 240
   gaagatggaa ggtttacagc acagctcaat aaagccagcc agtatgtttc tctgctcatc 300
   agagactccc agcccagtga ttcagccacc tacctctgca gagaccttgc ggccgcatag 360
   gtctcaccag aaccctgacc ctgccgtgta ccagctgaga gactctaaat ccagtgacaa 420
   gtctgtctgc ctattcaccg attttgattc tcaaacaaat gtgtcacaaa gtaaggattc 480
   tgatgtgtat atcacagaca aaactgtgct agacatgagg tctatggact tcaagagcaa 540
   cagtgctgtg gcctggagca acaaatctga ctttgcatgt gcaaacgcct tcaacaacag 600
   cattattcca gaggacacct tcttccccag cccagaaagt tcctgtgatg tcaagctggt 660
   cgagaaaagc tttgaaacag atacgaacct aaactttcaa aacctgtcag tgattgggtt 720
   ccgaatcctc ctcctgaaag tggccgggtt taatctgctc atgacgctgc ggctgtggtc 780
   cagctga 787
<210> 68
   <211> 787
   <212> DNA
   <213> Homo sapiens
<220>
   <223> V-C entry TRAV12-3_TRAC
<400> 68
   gccaccatga tgaaatcctt gagagtttta ctggtgatcc tgtggcttca gttaagctgg 60
   gtttggagcc aacagaagga ggtggagcag gatcctggac cactcagtgt tccagaggga 120
   gccattgttt ctctcaactg cacttacagc aacagtgctt ttcaatactt catgtggtac 180
   agacagtatt ccagaaaagg ccctgagttg ctgatgtaca catactccag tggtaacaaa 240
   gaagatggaa ggtttacagc acaggtcgat aaatccagca agtatatctc cttgttcatc 300
   agagactcac agcccagtga ttcagccacc tacctctgca gagaccttgc ggccgcatag 360
   gtctcaccag aaccctgacc ctgccgtgta ccagctgaga gactctaaat ccagtgacaa 420
   gtctgtctgc ctattcaccg attttgattc tcaaacaaat gtgtcacaaa gtaaggattc 480
   tgatgtgtat atcacagaca aaactgtgct agacatgagg tctatggact tcaagagcaa 540
   cagtgctgtg gcctggagca acaaatctga ctttgcatgt gcaaacgcct tcaacaacag 600
   cattattcca gaggacacct tcttccccag cccagaaagt tcctgtgatg tcaagctggt 660
   cgagaaaagc tttgaaacag atacgaacct aaactttcaa aacctgtcag tgattgggtt 720
   ccgaatcctc ctcctgaaag tggccgggtt taatctgctc atgacgctgc ggctgtggtc 780
   cagctga 787
<210> 69
   <211> 781
   <212> DNA
   <213> Homo sapiens
<220>
   <223> V-C entry TRAV13-1_TRAC
<400> 69
   gccaccatga catccattcg agctgtattt atattcctgt ggctgcagct ggacttggtg      60
   aatggagaga atgtggagca gcatccttca accctgagtg tccaggaggg agacagcgct     120
   gttatcaagt gtacttattc agacagtgcc tcaaactact tcccttggta taagcaagaa     180
   cttggaaaag gacctcagct tattatagac attcgttcaa atgtgggcga aaagaaagac     240
   caacgaattg ctgttacatt gaacaagaca gccaaacatt tctccctgca catcacagaa     300
   acccaacctg aggactcggc tgtctacttc tgcagagacc ttgcggccgc ataggtctca     360
   ccagaaccct gaccctgccg tgtaccagct gagagactct aaatccagtg acaagtctgt     420
   ctgcctattc accgattttg attctcaaac aaatgtgtca caaagtaagg attctgatgt     480
   gtatatcaca gacaaaactg tgctagacat gaggtctatg gacttcaaga gcaacagtgc     540
   tgtggcctgg agcaacaaat ctgactttgc atgtgcaaac gccttcaaca acagcattat     600
   tccagaggac accttcttcc ccagcccaga aagttcctgt gatgtcaagc tggtcgagaa     660
   aagctttgaa acagatacga acctaaactt tcaaaacctg tcagtgattg ggttccgaat     720
   cctcctcctg aaagtggccg ggtttaatct gctcatgacg ctgcggctgt ggtccagctg     780
   a                                                                     781
<210> 70
   <211> 784
   <212> DNA
   <213> Homo sapiens
<220>
   <223> V-C entry TRAV13-2_TRAC
<400> 70
   gccaccatgg caggcattcg agctttattt atgtacttgt ggctgcagct ggactgggtg      60
   agcagaggag agagtgtggg gctgcatctt cctaccctga gtgtccagga gggtgacaac     120
   tctattatca actgtgctta ttcaaacagc gcctcagact acttcatttg gtacaagcaa     180
   gaatctggaa aaggtcctca attcattata gacattcgtt caaatatgga caaaaggcaa     240
   ggccaaagag tcaccgtttt attgaataag acagtgaaac atctctctct gcaaattgca     300
   gctactcaac ctggagactc agctgtctac ttctgcagag accttgcggc cgcataggtc     360
   tcaccagaac cctgaccctg ccgtgtacca gctgagagac tctaaatcca gtgacaagtc 420
   tgtctgccta ttcaccgatt ttgattctca aacaaatgtg tcacaaagta aggattctga 480
   tgtgtatatc acagacaaaa ctgtgctaga catgaggtct atggacttca agagcaacag 540
   tgctgtggcc tggagcaaca aatctgactt tgcatgtgca aacgccttca acaacagcat 600
   tattccagag gacaccttct tccccagccc agaaagttcc tgtgatgtca agctggtcga 660
   gaaaagcttt gaaacagata cgaacctaaa ctttcaaaac ctgtcagtga ttgggttccg 720
   aatcctcctc ctgaaagtgg ccgggtttaa tctgctcatg acgctgcggc tgtggtccag 780
   ctga 784
<210> 71
   <211> 790
   <212> DNA
   <213> Homo sapiens
<220>
   <223> V-C entry TRAV14DV4_TRAC
<400> 71
   gccaccatgt cactttctag cctgctgaag gtggtcacag cttcactgtg gctaggacct 60
   ggcattgccc agaagataac tcaaacccaa ccaggaatgt tcgtgcagga aaaggaggct 120
   gtgactctgg actgcacata tgacaccagt gatcaaagtt atggtctatt ctggtacaag 180
   cagcccagca gtggggaaat gatttttctt atttatcagg ggtcttatga cgagcaaaat 240
   gcaacagaag gtcgctactc attgaatttc cagaaggcaa gaaaatccgc caaccttgtc 300
   atctccgctt cacaactggg ggactcagca atgtatttct gcagagacct tgcggccgca 360
   taggtctcac cagaaccctg accctgccgt gtaccagctg agagactcta aatccagtga 420
   caagtctgtc tgcctattca ccgattttga ttctcaaaca aatgtgtcac aaagtaagga 480
   ttctgatgtg tatatcacag acaaaactgt gctagacatg aggtctatgg acttcaagag 540
   caacagtgct gtggcctgga gcaacaaatc tgactttgca tgtgcaaacg ccttcaacaa 600
   cagcattatt ccagaggaca ccttcttccc cagcccagaa agttcctgtg atgtcaagct 660
   ggtcgagaaa agctttgaaa cagatacgaa cctaaacttt caaaacctgt cagtgattgg 720
   gttccgaatc ctcctcctga aagtggccgg gtttaatctg ctcatgacgc tgcggctgtg 780
   gtccagctga 790
<210> 72
   <211> 772
   <212> DNA
   <213> Homo sapiens
<220>
   <223> V-C entry TRAV16_TRAC
<400> 72
   gccaccatga agcccaccct catctcagtg cttgtgataa tatttatact cagaggaaca      60
   agagcccaga gagtgactca gcccgagaag ctcctctctg tctttaaagg ggccccagtg     120
   gagctgaagt gcaactattc ctattctggg agtcctgaac tcttctggta tgtccagtac     180
   tccagacaac gcctccagtt actcttgaga cacatctcta gagagagcat caaaggcttc     240
   actgctgacc ttaacaaagg cgagacatct ttccacctga agaaaccatt tgctcaagag     300
   gaagattcag ccatgtatta ctgcagagac cttgcggccg cataggtctc accagaaccc     360
   tgaccctgcc gtgtaccagc tgagagactc taaatccagt gacaagtctg tctgcctatt     420
   caccgatttt gattctcaaa caaatgtgtc acaaagtaag gattctgatg tgtatatcac     480
   agacaaaact gtgctagaca tgaggtctat ggacttcaag agcaacagtg ctgtggcctg     540
   gagcaacaaa tctgactttg catgtgcaaa cgccttcaac aacagcatta ttccagagga     600
   caccttcttc cccagcccag aaagttcctg tgatgtcaag ctggtcgaga aaagctttga     660
   aacagatacg aacctaaact ttcaaaacct gtcagtgatt gggttccgaa tcctcctcct     720
   gaaagtggcc gggtttaatc tgctcatgac gctgcggctg tggtccagct ga             772
<210> 73
   <211> 781
   <212> DNA
   <213> Homo sapiens
<220>
   <223> V-C entry TRAV17_TRAC
<400> 73
   gccaccatgg aaactctcct gggagtgtct ttggtgattc tatggcttca actggctagg 60
   gtgaacagtc aacagggaga agaggatcct caggccttga gcatccagga gggtgaaaat 120
   gccaccatga actgcagtta caaaactagt ataaacaatt tacagtggta tagacaaaat 180
   tcaggtagag gccttgtcca cctaatttta atacgttcaa atgaaagaga gaaacacagt 240
   ggaagattaa gagtcacgct tgacacttcc aagaaaagca gttccttgtt gatcacggct 300
   tcccgggcag cagacactgc ttcttacttc tgcagagacc ttgcggccgc ataggtctca 360
   ccagaaccct gaccctgccg tgtaccagct gagagactct aaatccagtg acaagtctgt 420
   ctgcctattc accgattttg attctcaaac aaatgtgtca caaagtaagg attctgatgt 480
   gtatatcaca gacaaaactg tgctagacat gaggtctatg gacttcaaga gcaacagtgc 540
   tgtggcctgg agcaacaaat ctgactttgc atgtgcaaac gccttcaaca acagcattat 600
   tccagaggac accttcttcc ccagcccaga aagttcctgt gatgtcaagc tggtcgagaa 660
   aagctttgaa acagatacga acctaaactt tcaaaacctg tcagtgattg ggttccgaat 720
   cctcctcctg aaagtggccg ggtttaatct gctcatgacg ctgcggctgt ggtccagctg 780
   a 781
<210> 74
   <211> 778
   <212> DNA
   <213> Homo sapiens
<220>
   <223> V-C entry TRAV18_TRAC
<400> 74
   gccaccatgc tgtctgcttc ctgctcagga cttgtgatct tgttgatatt cagaaggacc      60
   agtggagact cggttaccca gacagaaggc ccagttaccc tccctgagag ggcagctctg     120
   acattaaact gcacttatca gtccagctat tcaacttttc tattctggta tgtccagtat     180
   ctaaacaaag agcctgagct cctcctgaaa agttcagaaa accaggagac ggacagcaga     240
   ggttttcagg ccagtcctat caagagtgac agttccttcc acctggagaa gccctcggtg     300
   cagctgtcgg actctgccgt gtactactgc agagaccttg cggccgcata ggtctcacca     360
   gaaccctgac cctgccgtgt accagctgag agactctaaa tccagtgaca agtctgtctg     420
   cctattcacc gattttgatt ctcaaacaaa tgtgtcacaa agtaaggatt ctgatgtgta     480
   tatcacagac aaaactgtgc tagacatgag gtctatggac ttcaagagca acagtgctgt     540
   ggcctggagc aacaaatctg actttgcatg tgcaaacgcc ttcaacaaca gcattattcc     600
   agaggacacc ttcttcccca gcccagaaag ttcctgtgat gtcaagctgg tcgagaaaag     660
   ctttgaaaca gatacgaacc taaactttca aaacctgtca gtgattgggt tccgaatcct     720
   cctcctgaaa gtggccgggt ttaatctgct catgacgctg cggctgtggt ccagctga       778
<210> 75
   <211> 790
   <212> DNA
   <213> Homo sapiens
<220>
   <223> V-C entry TRAV19_TRAC
<400> 75
   gccaccatgc tgactgccag cctgttgagg gcagtcatag cctccatctg tgttgtatcc      60
   agcatggctc agaaggtaac tcaagcgcag actgaaattt ctgtggtgga gaaggaggat     120
   gtgaccttgg actgtgtgta tgaaacccgt gatactactt attacttatt ctggtacaag     180
   caaccaccaa gtggagaatt ggttttcctt attcgtcgga actcttttga tgagcaaaat     240
   gaaataagtg gtcggtattc ttggaacttc cagaaatcca ccagttcctt caacttcacc     300
   atcacagcct cacaagtcgt ggactcagca gtatacttct gcagagacct tgcggccgca     360
   taggtctcac cagaaccctg accctgccgt gtaccagctg agagactcta aatccagtga     420
   caagtctgtc tgcctattca ccgattttga ttctcaaaca aatgtgtcac aaagtaagga     480
   ttctgatgtg tatatcacag acaaaactgt gctagacatg aggtctatgg acttcaagag 540
   caacagtgct gtggcctgga gcaacaaatc tgactttgca tgtgcaaacg ccttcaacaa 600
   cagcattatt ccagaggaca ccttcttccc cagcccagaa agttcctgtg atgtcaagct 660
   ggtcgagaaa agctttgaaa cagatacgaa cctaaacttt caaaacctgt cagtgattgg 720
   gttccgaatc ctcctcctga aagtggccgg gtttaatctg ctcatgacgc tgcggctgtg 780
   gtccagctga 790
<210> 76
   <211> 781
   <212> DNA
   <213> Homo sapiens
<220>
   <223> V-C entry TRAV20_TRAC
<400> 76
   gccaccatgg agaaaatgtt ggagtgtgca ttcatagtct tgtggcttca gcttggctgg 60
   ttgagtggag aggaccaggt gacgcagagt cccgaggccc tgagactcca ggagggagag 120
   agtagcagtc ttaactgcag ttacacagtc agcggtttaa gagggctgtt ctggtatagg 180
   caagatcctg ggaaaggccc tgaattcctc ttcaccctgt attcagctgg ggaagaaaag 240
   gagaaagaaa ggctaaaagc cacattaaca aagaaggaaa gctttctgca catcacagcc 300
   cctaaacctg aggactcagc cacttatctc tgcagagacc ttgcggccgc ataggtctca 360
   ccagaaccct gaccctgccg tgtaccagct gagagactct aaatccagtg acaagtctgt 420
   ctgcctattc accgattttg attctcaaac aaatgtgtca caaagtaagg attctgatgt 480
   gtatatcaca gacaaaactg tgctagacat gaggtctatg gacttcaaga gcaacagtgc 540
   tgtggcctgg agcaacaaat ctgactttgc atgtgcaaac gccttcaaca acagcattat 600
   tccagaggac accttcttcc ccagcccaga aagttcctgt gatgtcaagc tggtcgagaa 660
   aagctttgaa acagatacga acctaaactt tcaaaacctg tcagtgattg ggttccgaat 720
   cctcctcctg aaagtggccg ggtttaatct gctcatgacg ctgcggctgt ggtccagctg 780
   a 781
<210> 77
   <211> 781
   <212> DNA
   <213> Homo sapiens
<220>
   <223> V-C entry TRAV21_TRAC
<400> 77
   gccaccatgg agaccctctt gggcctgctt atcctttggc tgcagctgca atgggtgagc      60
   agcaaacagg aggtgacgca gattcctgca gctctgagtg tcccagaagg agaaaacttg     120
   gttctcaact gcagtttcac tgatagcgct atttacaacc tccagtggtt taggcaggac     180
   cctgggaaag gactcacatc tctgttgctt attcagtcaa gtcagagaga gcaaacaagt     240
   ggacgcctta atgcctcgct ggataaatca tcaggacgta gtactttata cattgcagct     300
   tctcagcctg gtgactcagc cacctacctc tgcagagacc ttgcggccgc ataggtctca     360
   ccagaaccct gaccctgccg tgtaccagct gagagactct aaatccagtg acaagtctgt     420
   ctgcctattc accgattttg attctcaaac aaatgtgtca caaagtaagg attctgatgt     480
   gtatatcaca gacaaaactg tgctagacat gaggtctatg gacttcaaga gcaacagtgc     540
   tgtggcctgg agcaacaaat ctgactttgc atgtgcaaac gccttcaaca acagcattat     600
   tccagaggac accttcttcc ccagcccaga aagttcctgt gatgtcaagc tggtcgagaa     660
   aagctttgaa acagatacga acctaaactt tcaaaacctg tcagtgattg ggttccgaat     720
   cctcctcctg aaagtggccg ggtttaatct gctcatgacg ctgcggctgt ggtccagctg     780
   a                                                                     781
<210> 78
   <211> 775
   <212> DNA
   <213> Homo sapiens
<220>
   <223> V-C entry TRAV22_TRAC
<400> 78
   gccaccatga agaggatatt gggagctctg ctggggctct tgagtgccca ggtttgctgt      60
   gtgagaggaa tacaagtgga gcagagtcct ccagacctga ttctccagga gggagccaat     120
   tccacgctgc ggtgcaattt ttctgactct gtgaacaatt tgcagtggtt tcatcaaaac     180
   ccttggggac agctcatcaa cctgttttac attccctcag ggacaaaaca gaatggaaga     240
   ttaagcgcca cgactgtcgc tacggaacgc tacagcttat tgtacatttc ctcttcccag     300
   accacagact caggcgttta tttctgcaga gaccttgcgg ccgcataggt ctcaccagaa     360
   ccctgaccct gccgtgtacc agctgagaga ctctaaatcc agtgacaagt ctgtctgcct     420
   attcaccgat tttgattctc aaacaaatgt gtcacaaagt aaggattctg atgtgtatat     480
   cacagacaaa actgtgctag acatgaggtc tatggacttc aagagcaaca gtgctgtggc     540
   ctggagcaac aaatctgact ttgcatgtgc aaacgccttc aacaacagca ttattccaga     600
   ggacaccttc ttccccagcc cagaaagttc ctgtgatgtc aagctggtcg agaaaagctt     660
   tgaaacagat acgaacctaa actttcaaaa cctgtcagtg attgggttcc gaatcctcct     720
   cctgaaagtg gccgggttta atctgctcat gacgctgcgg ctgtggtcca gctga          775
<210> 79
   <211> 808
   <212> DNA
   <213> Homo sapiens
<220>
   <223> V-C entry TRAV23DV6_TRAC
<400> 79
   gccaccatgg acaagatctt aggagcatca tttttagttc tgtggcttca actatgctgg 60
   gtgagtggcc aacagaagga gaaaagtgac cagcagcagg tgaaacaaag tcctcaatct 120
   ttgatagtcc agaaaggagg gatttcaatt ataaactgtg cttatgagaa cactgcgttt 180
   gactactttc catggtacca acaattccct gggaaaggcc ctgcattatt gatagccata 240
   cgtccagatg tgagtgaaaa gaaagaagga agattcacaa tctccttcaa taaaagtgcc 300
   aagcagttct cattgcatat catggattcc cagcctggag actcagccac ctacttctgc 360
   agagaccttg cggccgcata ggtctcacca gaaccctgac cctgccgtgt accagctgag 420
   agactctaaa tccagtgaca agtctgtctg cctattcacc gattttgatt ctcaaacaaa 480
   tgtgtcacaa agtaaggatt ctgatgtgta tatcacagac aaaactgtgc tagacatgag 540
   gtctatggac ttcaagagca acagtgctgt ggcctggagc aacaaatctg actttgcatg 600
   tgcaaacgcc ttcaacaaca gcattattcc agaggacacc ttcttcccca gcccagaaag 660
   ttcctgtgat gtcaagctgg tcgagaaaag ctttgaaaca gatacgaacc taaactttca 720
   aaacctgtca gtgattgggt tccgaatcct cctcctgaaa gtggccgggt ttaatctgct 780
   catgacgctg cggctgtggt ccagctga 808
<210> 80
   <211> 790
   <212> DNA
   <213> Homo sapiens
<220>
   <223> V-C entry TRAV24_TRAC
<400> 80
   gccaccatgg agaagaatcc tttggcagcc ccattactaa tcctctggtt tcatcttgac 60
   tgcgtgagca gcatactgaa cgtggaacaa agtcctcagt cactgcatgt tcaggaggga 120
   gacagcacca atttcacctg cagcttccct tccagcaatt tttatgcctt acactggtac 180
   agatgggaaa ctgcaaaaag ccccgaggcc ttgtttgtaa tgactttaaa tggggatgaa 240
   aagaagaaag gacgaataag tgccactctt aataccaagg agggttacag ctatttgtac 300
   atcaaaggat cccagcctga agattcagcc acatacctct gcagagacct tgcggccgca 360
   taggtctcac cagaaccctg accctgccgt gtaccagctg agagactcta aatccagtga 420
   caagtctgtc tgcctattca ccgattttga ttctcaaaca aatgtgtcac aaagtaagga 480
   ttctgatgtg tatatcacag acaaaactgt gctagacatg aggtctatgg acttcaagag      540
   caacagtgct gtggcctgga gcaacaaatc tgactttgca tgtgcaaacg ccttcaacaa      600
   cagcattatt ccagaggaca ccttcttccc cagcccagaa agttcctgtg atgtcaagct      660
   ggtcgagaaa agctttgaaa cagatacgaa cctaaacttt caaaacctgt cagtgattgg      720
   gttccgaatc ctcctcctga aagtggccgg gtttaatctg ctcatgacgc tgcggctgtg      780
   gtccagctga                                                             790
<210> 81
   <211> 775
   <212> DNA
   <213> Homo sapiens
<220>
   <223> V-C entry TRAV25_TRAC
<400> 81
   gccaccatgc tactcatcac atcaatgttg gtcttatgga tgcaattgtc acaggtgaat       60
   ggacaacagg taatgcaaat tcctcagtac cagcatgtac aagaaggaga ggacttcacc      120
   acgtactgca attcctcaac tactttaagc aatatacagt ggtataagca aaggcctggt      180
   ggacatcccg tattcttgat acagttagtg aagagtggag aagtgaagaa gcagaaaaga      240
   ctgacatttc agtttggaga agcaaaaaag aacagctccc tgcacatcac agccacccag      300
   actacagatg taggaaccta cttctgcaga gaccttgcgg ccgcataggt ctcaccagaa      360
   ccctgaccct gccgtgtacc agctgagaga ctctaaatcc agtgacaagt ctgtctgcct      420
   attcaccgat tttgattctc aaacaaatgt gtcacaaagt aaggattctg atgtgtatat      480
   cacagacaaa actgtgctag acatgaggtc tatggacttc aagagcaaca gtgctgtggc      540
   ctggagcaac aaatctgact ttgcatgtgc aaacgccttc aacaacagca ttattccaga      600
   ggacaccttc ttccccagcc cagaaagttc ctgtgatgtc aagctggtcg agaaaagctt      660
   tgaaacagat acgaacctaa actttcaaaa cctgtcagtg attgggttcc gaatcctcct      720
   cctgaaagtg gccgggttta atctgctcat gacgctgcgg ctgtggtcca gctga           775
<210> 82
   <211> 769
   <212> DNA
   <213> Homo sapiens
<220>
   <223> V-C entry TRAV26-1_TRAC
<400> 82
   gccaccatga ggctggtggc aagagtaact gtgtttctga cctttggaac tataattgat      60
   gctaagacca cccagccccc ctccatggat tgcgctgaag gaagagctgc aaacctgcct     120
   tgtaatcact ctaccatcag tggaaatgag tatgtgtatt ggtatcgaca gattcactcc     180
   caggggccac agtatatcat tcatggtcta aaaaacaatg aaaccaatga aatggcctct     240
   ctgatcatca cagaagatag aaagtccagc accttgatcc tgccccacgc tacgctgaga     300
   gacactgctg tgtactactg cagagacctt gcggccgcat aggtctcacc agaaccctga     360
   ccctgccgtg taccagctga gagactctaa atccagtgac aagtctgtct gcctattcac     420
   cgattttgat tctcaaacaa atgtgtcaca aagtaaggat tctgatgtgt atatcacaga     480
   caaaactgtg ctagacatga ggtctatgga cttcaagagc aacagtgctg tggcctggag     540
   caacaaatct gactttgcat gtgcaaacgc cttcaacaac agcattattc cagaggacac     600
   cttcttcccc agcccagaaa gttcctgtga tgtcaagctg gtcgagaaaa gctttgaaac     660
   agatacgaac ctaaactttc aaaacctgtc agtgattggg ttccgaatcc tcctcctgaa     720
   agtggccggg tttaatctgc tcatgacgct gcggctgtgg tccagctga                 769
<210> 83
   <211> 769
   <212> DNA
   <213> Homo sapiens
<220>
   <223> V-C entry TRAV26-2_TRAC
<400> 83
   gccaccatga agttggtgac aagcattact gtactcctat ctttgggtat tatgggtgat      60
   gctaagacca cacagccaaa ttcaatggag agtaacgaag aagagcctgt tcacttgcct     120
   tgtaaccact ccacaatcag tggaactgat tacatacatt ggtatcgaca gcttccctcc     180
   cagggtccag agtacgtgat tcatggtctt acaagcaatg tgaacaacag aatggcctct     240
   ctggcaatcg ctgaggacag aaagtccagt accttgatcc tgcaccgtgc taccttgaga     300
   gatgctgctg tgtactactg cagagacctt gcggccgcat aggtctcacc agaaccctga     360
   ccctgccgtg taccagctga gagactctaa atccagtgac aagtctgtct gcctattcac     420
   cgattttgat tctcaaacaa atgtgtcaca aagtaaggat tctgatgtgt atatcacaga     480
   caaaactgtg ctagacatga ggtctatgga cttcaagagc aacagtgctg tggcctggag     540
   caacaaatct gactttgcat gtgcaaacgc cttcaacaac agcattattc cagaggacac     600
   cttcttcccc agcccagaaa gttcctgtga tgtcaagctg gtcgagaaaa gctttgaaac     660
   agatacgaac ctaaactttc aaaacctgtc agtgattggg ttccgaatcc tcctcctgaa     720
   agtggccggg tttaatctgc tcatgacgct gcggctgtgg tccagctga                 769
<210> 84
   <211> 775
   <212> DNA
   <213> Homo sapiens
<220>
   <223> V-C entry TRAV27_TRAC
<400> 84
   gccaccatgg tcctgaaatt ctccgtgtcc attctttgga ttcagttggc atgggtgagc      60
   acccagctgc tggagcagag ccctcagttt ctaagcatcc aagagggaga aaatctcact     120
   gtgtactgca actcctcaag tgttttttcc agcttacaat ggtacagaca ggagcctggg     180
   gaaggtcctg tcctcctggt gacagtagtt acgggtggag aagtgaagaa gctgaagaga     240
   ctaacctttc agtttggtga tgcaagaaag gacagttctc tccacatcac tgcagcccag     300
   cctggtgata caggcctcta cctctgcaga gaccttgcgg ccgcataggt ctcaccagaa     360
   ccctgaccct gccgtgtacc agctgagaga ctctaaatcc agtgacaagt ctgtctgcct     420
   attcaccgat tttgattctc aaacaaatgt gtcacaaagt aaggattctg atgtgtatat     480
   cacagacaaa actgtgctag acatgaggtc tatggacttc aagagcaaca gtgctgtggc     540
   ctggagcaac aaatctgact ttgcatgtgc aaacgccttc aacaacagca ttattccaga     600
   ggacaccttc ttccccagcc cagaaagttc ctgtgatgtc aagctggtcg agaaaagctt     660
   tgaaacagat acgaacctaa actttcaaaa cctgtcagtg attgggttcc gaatcctcct     720
   cctgaaagtg gccgggttta atctgctcat gacgctgcgg ctgtggtcca gctga          775
<210> 85
   <211> 802
   <212> DNA
   <213> Homo sapiens
<220>
   <223> V-C entry TRAV29DV5_TRAC
<400> 85
   gccaccatgg ccatgctcct gggggcatca gtgctgattc tgtggcttca gccagactgg      60
   gtaaacagtc aacagaagaa tgatgaccag caagttaagc aaaattcacc atccctgagc     120
   gtccaggaag gaagaatttc tattctgaac tgtgactata ctaacagcat gtttgattat     180
   ttcctatggt acaaaaaata ccctgctgaa ggtcctacat tcctgatatc tataagttcc     240
   attaaggata aaaatgaaga tggaagattc actgttttct taaacaaaag tgccaagcac     300
   ctctctctgc acattgtgcc ctcccagcct ggagactctg cagtgtactt ctgcagagac     360
   cttgcggccg cataggtctc accagaaccc tgaccctgcc gtgtaccagc tgagagactc     420
   taaatccagt gacaagtctg tctgcctatt caccgatttt gattctcaaa caaatgtgtc     480
   acaaagtaag gattctgatg tgtatatcac agacaaaact gtgctagaca tgaggtctat     540
   ggacttcaag agcaacagtg ctgtggcctg gagcaacaaa tctgactttg catgtgcaaa     600
   cgccttcaac aacagcatta ttccagagga caccttcttc cccagcccag aaagttcctg     660
   tgatgtcaag ctggtcgaga aaagctttga aacagatacg aacctaaact ttcaaaacct     720
   gtcagtgatt gggttccgaa tcctcctcct gaaagtggcc gggtttaatc tgctcatgac 780
   gctgcggctg tggtccagct ga 802
<210> 86
   <211> 781
   <212> DNA
   <213> Homo sapiens
<220>
   <223> V-C entry TRAV30_TRAC
<400> 86
   gccaccatgg agactctcct gaaagtgctt tcaggcacct tgttgtggca gttgacctgg 60
   gtgagaagcc aacaaccagt gcagagtcct caagccgtga tcctccgaga aggggaagat 120
   gctgtcatca actgcagttc ctccaaggct ttatattctg tacactggta caggcagaag 180
   catggtgaag cacccgtttt cctgatgata ttactgaagg gtggagaaca gaagggtcat 240
   gaaaaaatat ctgcttcatt taatgaaaaa aagcagcaaa gctccctgta ccttacggcc 300
   tcccagctca gttactcagg aacctacttc tgcagagacc ttgcggccgc ataggtctca 360
   ccagaaccct gaccctgccg tgtaccagct gagagactct aaatccagtg acaagtctgt 420
   ctgcctattc accgattttg attctcaaac aaatgtgtca caaagtaagg attctgatgt 480
   gtatatcaca gacaaaactg tgctagacat gaggtctatg gacttcaaga gcaacagtgc 540
   tgtggcctgg agcaacaaat ctgactttgc atgtgcaaac gccttcaaca acagcattat 600
   tccagaggac accttcttcc ccagcccaga aagttcctgt gatgtcaagc tggtcgagaa 660
   aagctttgaa acagatacga acctaaactt tcaaaacctg tcagtgattg ggttccgaat 720
   cctcctcctg aaagtggccg ggtttaatct gctcatgacg ctgcggctgt ggtccagctg 780
   a 781
<210> 87
   <211> 781
   <212> DNA
   <213> Homo sapiens
<220>
   <223> V-C entry TRAV34_TRAC
<400> 87
   gccaccatgg agactgttct gcaagtactc ctagggatat tggggttcca agcagcctgg 60
   gtcagtagcc aagaactgga gcagagtcct cagtccttga tcgtccaaga gggaaagaat 120
   ctcaccataa actgcacgtc atcaaagacg ttatatggct tatactggta taagcaaaag 180
   tatggtgaag gtcttatctt cttgatgatg ctacagaaag gtggggaaga gaaaagtcat 240
   gaaaagataa ctgccaagtt ggatgagaaa aagcagcaaa gttccctgca tatcacagcc 300
   tcccagccca gccatgcagg catctacctc tgcagagacc ttgcggccgc ataggtctca 360
   ccagaaccct gaccctgccg tgtaccagct gagagactct aaatccagtg acaagtctgt 420
   ctgcctattc accgattttg attctcaaac aaatgtgtca caaagtaagg attctgatgt 480
   gtatatcaca gacaaaactg tgctagacat gaggtctatg gacttcaaga gcaacagtgc 540
   tgtggcctgg agcaacaaat ctgactttgc atgtgcaaac gccttcaaca acagcattat 600
   tccagaggac accttcttcc ccagcccaga aagttcctgt gatgtcaagc tggtcgagaa 660
   aagctttgaa acagatacga acctaaactt tcaaaacctg tcagtgattg ggttccgaat 720
   cctcctcctg aaagtggccg ggtttaatct gctcatgacg ctgcggctgt ggtccagctg 780
   a 781
<210> 88
   <211> 775
   <212> DNA
   <213> Homo sapiens
<220>
   <223> V-C entry TRAV35_TRAC
<400> 88
   gccaccatgc tccttgaaca tttattaata atcttgtgga tgcagctgac atgggtcagt 60
   ggtcaacagc tgaatcagag tcctcaatct atgtttatcc aggaaggaga agatgtctcc 120
   atgaactgca cttcttcaag catatttaac acctggctat ggtacaagca ggaacctggg 180
   gaaggtcctg tcctcttgat agccttatat aaggctggtg aattgacctc aaatggaagg 240
   ctgactgctc agtttggtat aaccagaaag gacagcttcc tgaatatctc agcatccata 300
   cctagtgatg taggcatcta cttctgcaga gaccttgcgg ccgcataggt ctcaccagaa 360
   ccctgaccct gccgtgtacc agctgagaga ctctaaatcc agtgacaagt ctgtctgcct 420
   attcaccgat tttgattctc aaacaaatgt gtcacaaagt aaggattctg atgtgtatat 480
   cacagacaaa actgtgctag acatgaggtc tatggacttc aagagcaaca gtgctgtggc 540
   ctggagcaac aaatctgact ttgcatgtgc aaacgccttc aacaacagca ttattccaga 600
   ggacaccttc ttccccagcc cagaaagttc ctgtgatgtc aagctggtcg agaaaagctt 660
   tgaaacagat acgaacctaa actttcaaaa cctgtcagtg attgggttcc gaatcctcct 720
   cctgaaagtg gccgggttta atctgctcat gacgctgcgg ctgtggtcca gctga 775
<210> 89
   <211> 784
   <212> DNA
   <213> Homo sapiens
<220>
   <223> V-C entry TRAV36DV7_TRAC
<400> 89
   gccaccatga tgaagtgtcc acaggcttta ctagctatct tttggcttct actgagctgg       60
   gtgagcagtg aagataaggt ggtacaaagc cctctatctc tggttgtcca cgagggagac      120
   accgtaactc tcaattgcag ttatgaagtg actaactttc gaagcctact atggtacaag      180
   caggaaaaga aagctcccac atttctattt atgctaactt caagtggaat tgaaaagaag      240
   tcaggtagac taagtagcat attagataag aaagaacttt ccagcatcct gaacatcaca      300
   gccacccaga ccggagactc ggccatctac ctctgcagag accttgcggc cgcataggtc      360
   tcaccagaac cctgaccctg ccgtgtacca gctgagagac tctaaatcca gtgacaagtc      420
   tgtctgccta ttcaccgatt ttgattctca aacaaatgtg tcacaaagta aggattctga      480
   tgtgtatatc acagacaaaa ctgtgctaga catgaggtct atggacttca agagcaacag      540
   tgctgtggcc tggagcaaca aatctgactt tgcatgtgca aacgccttca acaacagcat      600
   tattccagag gacaccttct tccccagccc agaaagttcc tgtgatgtca agctggtcga      660
   gaaaagcttt gaaacagata cgaacctaaa ctttcaaaac ctgtcagtga ttgggttccg      720
   aatcctcctc ctgaaagtgg ccgggtttaa tctgctcatg acgctgcggc tgtggtccag      780
   ctga                                                                   784
<210> 90
   <211> 790
   <212> DNA
   <213> Homo sapiens
<220>
   <223> V-C entry TRAV38-1_TRAC
<400> 90
   gccaccatga cacgagttag cttgctgtgg gcagtcgtgg tcagtacctg tcttgaatcc       60
   ggcatggccc agacagtcac tcagtctcaa ccagagatgt ctgtgcagga ggcagagact      120
   gtgaccctga gttgcacata tgacaccagt gagaataatt attatttgtt ctggtacaag      180
   cagcctccca gcaggcagat gattctcgtt attcgccaag aagcttataa gcaacagaat      240
   gcaacggaga atcgtttctc tgtgaacttc cagaaagcag ccaaatcctt cagtctcaag      300
   atctcagact cacagctggg ggacactgcg atgtatttct gcagagacct tgcggccgca      360
   taggtctcac cagaaccctg accctgccgt gtaccagctg agagactcta aatccagtga      420
   caagtctgtc tgcctattca ccgattttga ttctcaaaca aatgtgtcac aaagtaagga      480
   ttctgatgtg tatatcacag acaaaactgt gctagacatg aggtctatgg acttcaagag      540
   caacagtgct gtggcctgga gcaacaaatc tgactttgca tgtgcaaacg ccttcaacaa      600
   cagcattatt ccagaggaca ccttcttccc cagcccagaa agttcctgtg atgtcaagct      660
   ggtcgagaaa agctttgaaa cagatacgaa cctaaacttt caaaacctgt cagtgattgg      720
   gttccgaatc ctcctcctga aagtggccgg gtttaatctg ctcatgacgc tgcggctgtg      780
   gtccagctga                                                             790
<210> 91
   <211> 790
   <212> DNA
   <213> Homo sapiens
<220>
   <223> V-C entry TRAV38-2DV8_TRAC
<400> 91
   gccaccatgg catgccctgg cttcctgtgg gcacttgtga tctccacctg tcttgaattt      60
   agcatggctc agacagtcac tcagtctcaa ccagagatgt ctgtgcagga ggcagagacg     120
   gtgaccctga gctgcacata tgacaccagt gagagtgatt attatttatt ctggtacaag     180
   cagcctccca gcaggcagat gattctcgtt attcgccaag aagcttataa gcaacagaat     240
   gcaacagaga atcgtttctc tgtgaacttc cagaaagcag ccaaatcctt cagtctcaag     300
   atctcagact cacagctggg ggatgccgcg atgtatttct gcagagacct tgcggccgca     360
   taggtctcac cagaaccctg accctgccgt gtaccagctg agagactcta aatccagtga     420
   caagtctgtc tgcctattca ccgattttga ttctcaaaca aatgtgtcac aaagtaagga     480
   ttctgatgtg tatatcacag acaaaactgt gctagacatg aggtctatgg acttcaagag     540
   caacagtgct gtggcctgga gcaacaaatc tgactttgca tgtgcaaacg ccttcaacaa     600
   cagcattatt ccagaggaca ccttcttccc cagcccagaa agttcctgtg atgtcaagct     660
   ggtcgagaaa agctttgaaa cagatacgaa cctaaacttt caaaacctgt cagtgattgg     720
   gttccgaatc ctcctcctga aagtggccgg gtttaatctg ctcatgacgc tgcggctgtg     780
   gtccagctga                                                            790
<210> 92
   <211> 775
   <212> DNA
   <213> Homo sapiens
<220>
   <223> V-C entry TRAV39_TRAC
<400> 92
   gccaccatga agaagctact agcaatgatt ctgtggcttc aactagaccg gttaagtgga      60
   gagctgaaag tggaacaaaa ccctctgttc ctgagcatgc aggagggaaa aaactatacc     120
   atctactgca attattcaac cacttcagac agactgtatt ggtacaggca ggatcctggg     180
   aaaagtctgg aatctctgtt tgtgttgcta tcaaatggag cagtgaagca ggagggacga     240
   ttaatggcct cacttgatac caaagcccgt ctcagcaccc tccacatcac agctgccgtg     300
   catgacctct ctgccaccta cttctgcaga gaccttgcgg ccgcataggt ctcaccagaa     360
   ccctgaccct gccgtgtacc agctgagaga ctctaaatcc agtgacaagt ctgtctgcct 420
   attcaccgat tttgattctc aaacaaatgt gtcacaaagt aaggattctg atgtgtatat 480
   cacagacaaa actgtgctag acatgaggtc tatggacttc aagagcaaca gtgctgtggc 540
   ctggagcaac aaatctgact ttgcatgtgc aaacgccttc aacaacagca ttattccaga 600
   ggacaccttc ttccccagcc cagaaagttc ctgtgatgtc aagctggtcg agaaaagctt 660
   tgaaacagat acgaacctaa actttcaaaa cctgtcagtg attgggttcc gaatcctcct 720
   cctgaaagtg gccgggttta atctgctcat gacgctgcgg ctgtggtcca gctga 775
<210> 93
   <211> 760
   <212> DNA
   <213> Homo sapiens
<220>
   <223> V-C entry TRAV40_TRAC
<400> 93
   gccaccatga actcctctct ggactttcta attctgatct taatgtttgg aggaaccagc 60
   agcaattcag tcaagcagac gggccaaata accgtctcgg agggagcatc tgtgactatg 120
   aactgcacat acacatccac ggggtaccct acccttttct ggtatgtgga ataccccagc 180
   aaacctctgc agcttcttca gagagagaca atggaaaaca gcaaaaactt cggaggcgga 240
   aatattaaag acaaaaactc ccccattgtg aaatattcag tccaggtatc agactcagcc 300
   gtgtactact gcagagacct tgcggccgca taggtctcac cagaaccctg accctgccgt 360
   gtaccagctg agagactcta aatccagtga caagtctgtc tgcctattca ccgattttga 420
   ttctcaaaca aatgtgtcac aaagtaagga ttctgatgtg tatatcacag acaaaactgt 480
   gctagacatg aggtctatgg acttcaagag caacagtgct gtggcctgga gcaacaaatc 540
   tgactttgca tgtgcaaacg ccttcaacaa cagcattatt ccagaggaca ccttcttccc 600
   cagcccagaa agttcctgtg atgtcaagct ggtcgagaaa agctttgaaa cagatacgaa 660
   cctaaacttt caaaacctgt cagtgattgg gttccgaatc ctcctcctga aagtggccgg 720
   gtttaatctg ctcatgacgc tgcggctgtg gtccagctga 760
<210> 94
   <211> 781
   <212> DNA
   <213> Homo sapiens
<220>
   <223> V-C entry TRAV41_TRAC
<400> 94
   gccaccatgg tgaagatccg gcaatttttg ttggctattt tgtggcttca gctaagctgt      60
   gtaagtgccg ccaaaaatga agtggagcag agtcctcaga acctgactgc ccaggaagga     120
   gaatttatca caatcaactg cagttactcg gtaggaataa gtgccttaca ctggctgcaa     180
   cagcatccag gaggaggcat tgtttccttg tttatgctga gctcagggaa gaagaagcat     240
   ggaagattaa ttgccacaat aaacatacag gaaaagcaca gctccctgca catcacagcc     300
   tcccatccca gagactctgc cgtctacatc tgcagagacc ttgcggccgc ataggtctca     360
   ccagaaccct gaccctgccg tgtaccagct gagagactct aaatccagtg acaagtctgt     420
   ctgcctattc accgattttg attctcaaac aaatgtgtca caaagtaagg attctgatgt     480
   gtatatcaca gacaaaactg tgctagacat gaggtctatg gacttcaaga gcaacagtgc     540
   tgtggcctgg agcaacaaat ctgactttgc atgtgcaaac gccttcaaca acagcattat     600
   tccagaggac accttcttcc ccagcccaga aagttcctgt gatgtcaagc tggtcgagaa     660
   aagctttgaa acagatacga acctaaactt tcaaaacctg tcagtgattg ggttccgaat     720
   cctcctcctg aaagtggccg ggtttaatct gctcatgacg ctgcggctgt ggtccagctg     780
   a                                                                     781
<210> 95
   <211> 53
   <212> DNA
   <213> Artificial Sequence
<220>
   <223> TRAJ_receiving_F1
<400> 95
   aattcggtct cgaagtcttc tgcggccgct gaagacacta tccagtgaga ccc 53
<210> 96
   <211> 53
   <212> DNA
   <213> Artificial Sequence
<220>
   <223> TRAJ_receiving_R1
<400> 96
   tcgagggtct cactggatag tgtcttcagc ggccgcagaa gacttcgaga ccg 53
<210> 97
   <211> 2308
   <212> DNA
   <213> Artificial Sequence
<220>
   <223> J donor backbone
<400> 97
   ctcgagctgg gcctcatggg ccttccgctc actgcccgct ttccagtcgg gaaacctgtc      60
   gtgccagctg cattaacatg gtcatagctg tttccttgcg tattgggcgc tctccgcttc     120
   ctcgctcact gactcgctgc gctcggtcgt tcgggtaaag cctggggtgc ctaatgagca 180
   aaaggccagc aaaaggccag gaaccgtaaa aaggccgcgt tgctggcgtt tttccatagg 240
   ctccgccccc ctgacgagca tcacaaaaat cgacgctcaa gtcagaggtg gcgaaacccg 300
   acaggactat aaagatacca ggcgtttccc cctggaagct ccctcgtgcg ctctcctgtt 360
   ccgaccctgc cgcttaccgg atacctgtcc gcctttctcc cttcgggaag cgtggcgctt 420
   tctcatagct cacgctgtag gtatctcagt tcggtgtagg tcgttcgctc caagctgggc 480
   tgtgtgcacg aaccccccgt tcagcccgac cgctgcgcct tatccggtaa ctatcgtctt 540
   gagtccaacc cggtaagaca cgacttatcg ccactggcag cagccactgg taacaggatt 600
   agcagagcga ggtatgtagg cggtgctaca gagttcttga agtggtggcc taactacggc 660
   tacactagaa gaacagtatt tggtatctgc gctctgctga agccagttac cttcggaaaa 720
   agagttggta gctcttgatc cggcaaacaa accaccgctg gtagcggtgg tttttttgtt 780
   tgcaagcagc agattacgcg cagaaaaaaa ggatctcaag aagatccttt gatcttttct 840
   acggggtctg acgctcagtg gaacgaaaac tcacgttaag ggattttggt catgagatta 900
   tcaaaaagga tcttcaccta gatcctttta aattaaaaat gaagttttaa atcaatctaa 960
   agtatatatg agtaaacttg gtctgacagt tagaaaaatt cgtccagcat cagatgaaat 1020
   tgcagtttgt tcatgtccgg gttatcaata ccatatttct ggaacagacg tttctgcagg 1080
   ctcgggctaa attcacccag acaattccac agaattgcca gatcctgata acgatctgca 1140
   ataccaacac gaccaacatc aatgcagcca atcagtttac cctcatcaaa aatcaggtta 1200
   tccaggctaa aatcaccatg ggtaacaacg ctatccggac taaacggcag cagtttatgc 1260
   atttctttcc aaacctgttc aacaggccaa ccattacgtt catcatcaaa atcgcttgca 1320
   tcaaccagac cattattcat acggctctgt gcctgtgcca gacgaaaaac acgatcgcta 1380
   ttaaacggac aattacaaac cggaatgcta tgcagacgac gcagaaaaac tgccagtgca 1440
   tcaacaatat tttcgcctga atccggatat tcttccagaa cctgaaatgc ggttttaccc 1500
   ggaattgcgg tggtcagcag ccatgcatca tccggtgtac gaataaaatg tttaatggtc 1560
   ggcagcggca taaattcggt cagccaattc agacgaacca tttcatcggt cacatcattt 1620
   gcaacgctac ctttaccatg tttcagaaac agttccggtg catccggttt accatacaga 1680
   cgataaatgg ttgcaccgct ctgaccaaca ttatcacgtg cccatttata gccatacaga 1740
   tctgcatcca tattgctatt cagacgcgga cggctacagc tggtttcacg ctgaatatgg 1800
   ctcatactct tcctttttca atattattga agcatttatc agggttattg tctcatgagc 1860
   ggatacatat ttgaatgtat ttagaaaaat aaacaaatag gggttccgcg cacatttccc 1920
   cgaaaagtgc cacctaaatt gtaagcgtta atattttgtt aaaattcgcg ttaaattttt 1980
   gttaaatcag ctcatttttt aaccaatagg ccgaaatcgg caaaatccct tataaatcaa    2040
   aagaatagac cgagataggg ttgagtggcc gctacagggc gctcccattc gccattcagg    2100
   ctgcgcaact gttgggaagg gcgtttcggt gcgggcctct tcgctattac gccagctggc    2160
   gaaaggggga tgtgctgcaa ggcgattaag ttgggtaacg ccagggtttt cccagtcacg    2220
   acgttgtaaa acgacggcca gtgagcgcga cgtaatacga ctcactatag ggcgaattgg    2280
   cggaaggccg tcaaggccgc atgaattc                                      2308
<210> 98
   <211> 2355
   <212> DNA
   <213> Artificial Sequence
<220>
   <223> TRA J receiving cassette vector
<400> 98
   cgctattacg ccagctggcg aaagggggat gtgctgcaag gcgattaagt tgggtaacgc 60
   cagggttttc ccagtcacga cgttgtaaaa cgacggccag tgagcgcgac gtaatacgac 120
   tcactatagg gcgaattggc ggaaggccgt caaggccgca tgaattcggt ctcgaagtct 180
   tctgcggccg ctgaagacac tatccagtga gaccctcgag ctgggcctca tgggccttcc 240
   gctcactgcc cgctttccag tcgggaaacc tgtcgtgcca gctgcattaa catggtcata 300
   gctgtttcct tgcgtattgg gcgctctccg cttcctcgct cactgactcg ctgcgctcgg 360
   tcgttcgggt aaagcctggg gtgcctaatg agcaaaaggc cagcaaaagg ccaggaaccg 420
   taaaaaggcc gcgttgctgg cgtttttcca taggctccgc ccccctgacg agcatcacaa 480
   aaatcgacgc tcaagtcaga ggtggcgaaa cccgacagga ctataaagat accaggcgtt 540
   tccccctgga agctccctcg tgcgctctcc tgttccgacc ctgccgctta ccggatacct 600
   gtccgccttt ctcccttcgg gaagcgtggc gctttctcat agctcacgct gtaggtatct 660
   cagttcggtg taggtcgttc gctccaagct gggctgtgtg cacgaacccc ccgttcagcc 720
   cgaccgctgc gccttatccg gtaactatcg tcttgagtcc aacccggtaa gacacgactt 780
   atcgccactg gcagcagcca ctggtaacag gattagcaga gcgaggtatg taggcggtgc 840
   tacagagttc ttgaagtggt ggcctaacta cggctacact agaagaacag tatttggtat 900
   ctgcgctctg ctgaagccag ttaccttcgg aaaaagagtt ggtagctctt gatccggcaa 960
   acaaaccacc gctggtagcg gtggtttttt tgtttgcaag cagcagatta cgcgcagaaa 1020
   aaaaggatct caagaagatc ctttgatctt ttctacgggg tctgacgctc agtggaacga 1080
   aaactcacgt taagggattt tggtcatgag attatcaaaa aggatcttca cctagatcct 1140
   tttaaattaa aaatgaagtt ttaaatcaat ctaaagtata tatgagtaaa cttggtctga 1200
   cagttagaaa aattcgtcca gcatcagatg aaattgcagt ttgttcatgt ccgggttatc 1260
   aataccatat ttctggaaca gacgtttctg caggctcggg ctaaattcac ccagacaatt 1320
   ccacagaatt gccagatcct gataacgatc tgcaatacca acacgaccaa catcaatgca 1380
   gccaatcagt ttaccctcat caaaaatcag gttatccagg ctaaaatcac catgggtaac 1440
   aacgctatcc ggactaaacg gcagcagttt atgcatttct ttccaaacct gttcaacagg 1500
   ccaaccatta cgttcatcat caaaatcgct tgcatcaacc agaccattat tcatacggct 1560
   ctgtgcctgt gccagacgaa aaacacgatc gctattaaac ggacaattac aaaccggaat 1620
   gctatgcaga cgacgcagaa aaactgccag tgcatcaaca atattttcgc ctgaatccgg 1680
   atattcttcc agaacctgaa atgcggtttt acccggaatt gcggtggtca gcagccatgc 1740
   atcatccggt gtacgaataa aatgtttaat ggtcggcagc ggcataaatt cggtcagcca 1800
   attcagacga accatttcat cggtcacatc atttgcaacg ctacctttac catgtttcag 1860
   aaacagttcc ggtgcatccg gtttaccata cagacgataa atggttgcac cgctctgacc 1920
   aacattatca cgtgcccatt tatagccata cagatctgca tccatattgc tattcagacg 1980
   cggacggcta cagctggttt cacgctgaat atggctcata ctcttccttt ttcaatatta 2040
   ttgaagcatt tatcagggtt attgtctcat gagcggatac atatttgaat gtatttagaa 2100
   aaataaacaa ataggggttc cgcgcacatt tccccgaaaa gtgccaccta aattgtaagc 2160
   gttaatattt tgttaaaatt cgcgttaaat ttttgttaaa tcagctcatt ttttaaccaa 2220
   taggccgaaa tcggcaaaat cccttataaa tcaaaagaat agaccgagat agggttgagt 2280
   ggccgctaca gggcgctccc attcgccatt caggctgcgc aactgttggg aagggcgttt 2340
   cggtgcgggc ctctt 2355
<210> 99
   <211> 39
   <212> DNA
   <213> Homo sapiens
<220>
   <223> TRAJ1*01_BB-S_F1
<400> 99
   ctcgtttggc aaaggaacca gagtttccac ttctcccca 39
<210> 100
   <211> 39
   <212> DNA
   <213> Homo sapiens
<220>
   <223> TRAJ2*01_BB-S_F1
<400> 100
   ctcgtttggg aaagggaccc atgtattcat tatatctga 39
<210> 101
   <211> 39
   <212> DNA
   <213> Homo sapiens
<220>
   <223> TRAJ3*01_BB-S_F1
<400> 101
   ctcgtttgga tcagggacca gactcagcat ccggccaaa 39
<210> 102
   <211> 39
   <212> DNA
   <213> Homo sapiens
<220>
   <223> TRAJ4*01_BB-S_F1
<400> 102
   ctcgtttgga gcagggacca ggctggctgt acacccata 39
<210> 103
   <211> 39
   <212> DNA
   <213> Homo sapiens
<220>
   <223> TRAJ5*01_BB-S_F1
<400> 103
   ctcgtttggg agtggaacaa gactccaagt gcaaccaaa 39
<210> 104
   <211> 39
   <212> DNA
   <213> Homo sapiens
<220>
   <223> TRAJ6*01_BB-S_F1
<400> 104
   ctcgtttgga agaggaacca gccttattgt tcatccgta 39
<210> 105
   <211> 39
   <212> DNA
   <213> Homo sapiens
<220>
   <223> TRAJ7*01_BB-S_F1
<400> 105
   ctcgtttggg aaggggaacc aagtggtggt cataccaaa 39
<210> 106
   <211> 39
   <212> DNA
   <213> Homo sapiens
<220>
   <223> TRAJ8*01_BB-S_F1
<400> 106
   ctcgtttgga actggcaccc gacttctggt cagtccaaa 39
<210> 107
   <211> 39
   <212> DNA
   <213> Homo sapiens
<220>
   <223> TRAJ9*01_BB-S_F1
<400> 107
   ctcgtttgga gcaggaacaa gactatttgt taaagcaaa 39
<210> 108
   <211> 39
   <212> DNA
   <213> Homo sapiens
<220>
   <223> TRAJ10*01_BB-S_F1
<400> 108
   ctcgtttggg acaggcactc agctaaaagt ggaactcaa 39
<210> 109
   <211> 39
   <212> DNA
   <213> Homo sapiens
<220>
   <223> TRAJ11*01_BB-S_F1
<400> 109
   ctcgtttggg aaggggacta tgcttctagt ctctccaga 39
<210> 110
   <211> 39
   <212> DNA
   <213> Homo sapiens
<220>
   <223> TRAJ12*01_BB-S_F1
<400> 110
   ctcgtttggg agtgggacca gactgctggt caggcctga 39
<210> 111
   <211> 39
   <212> DNA
   <213> Homo sapiens
<220>
   <223> TRAJ13*01_BB-S_F1
<400> 111
   ctcgtttgga attggaacaa agctccaagt catcccaaa 39
<210> 112
   <211> 39
   <212> DNA
   <213> Homo sapiens
<220>
   <223> TRAJ14*01_BB-S_F1
<400> 112
   ctcgtttggg agtgggacaa gattatcagt aaaacctga 39
<210> 113
   <211> 39
   <212> DNA
   <213> Homo sapiens
<220>
   <223> TRAJ15*01_BB-S_F1
<400> 113
   ctcgtttggg aagggaaccc acctatcagt gagttccaa 39
<210> 114
   <211> 39
   <212> DNA
   <213> Homo sapiens
<220>
   <223> TRAJ16*01_BB-S_F1
<400> 114
   ctcgtttgca aggggaacca tgttaaaggt ggatcttaa 39
<210> 115
   <211> 39
   <212> DNA
   <213> Homo sapiens
<220>
   <223> TRAJ17*01_BB-S_F1
<400> 115
   ctcgtttgga ggaggaacca gggtgctagt taaaccaaa 39
<210> 116
   <211> 39
   <212> DNA
   <213> Homo sapiens
<220>
   <223> TRAJ18*01_BB-S_F1
<400> 116
   ctcgtttgga agaggaactc agttgactgt ctggcctga 39
<210> 117
   <211> 39
   <212> DNA
   <213> Homo sapiens
<220>
   <223> TRAJ19*01_BB-S_F1
<400> 117
   ctcgtttgga aagggatcca aacataatgt cactccaaa 39
<210> 118
   <211> 39
   <212> DNA
   <213> Homo sapiens
<220>
   <223> TRAJ20*01_BB-S_F1
<400> 118
   ctcgtttgga gccggaacca cagtaactgt aagagcaaa 39
<210> 119
   <211> 39
   <212> DNA
   <213> Homo sapiens
<220>
   <223> TRAJ21*01_BB-S_F1
<400> 119
   ctcgtttgga tctgggacca aactcaatgt aaaaccaaa 39
<210> 120
   <211> 39
   <212> DNA
   <213> Homo sapiens
<220>
   <223> TRAJ22*01_BB-S_F1
<400> 120
   ctcgtttgga tctgggacac aattgactgt tttacctga 39
<210> 121
   <211> 39
   <212> DNA
   <213> Homo sapiens
<220>
   <223> TRAJ23*01_BB-S_F1
<400> 121
   ctcgtttgga cagggaacgg agttatctgt gaaacccaa 39
<210> 122
   <211> 39
   <212> DNA
   <213> Homo sapiens
<220>
   <223> TRAJ24*01_BB-S_F1
<400> 122
   ctcgtttgga gcagggaccc aggttgtggt caccccaga 39
<210> 123
   <211> 39
   <212> DNA
   <213> Homo sapiens
<220>
   <223> TRAJ25*01_BB-S_F1
<400> 123
   ctcgtttggg aaggggacaa ggctgcttgt caagccaaa 39
<210> 124
   <211> 39
   <212> DNA
   <213> Homo sapiens
<220>
   <223> TRAJ26*01_BB-S_F1
<400> 124
   ctcgtttggt cccggaacca gattgtccgt gctgcccta 39
<210> 125
   <211> 39
   <212> DNA
   <213> Homo sapiens
<220>
   <223> TRAJ27*01_BB-S_F1
<400> 125
   ctcgtttggg gatgggacta cgctcactgt gaagccaaa 39
<210> 126
   <211> 39
   <212> DNA
   <213> Homo sapiens
<220>
   <223> TRAJ28*01_BB-S_F1
<400> 126
   ctcgtttggg aaggggacca aactctcggt cataccaaa 39
<210> 127
   <211> 39
   <212> DNA
   <213> Homo sapiens
<220>
   <223> TRAJ29*01_BB-S_F1
<400> 127
   ctcgtttgga aagggcacaa gactttctgt gattgcaaa 39
<210> 128
   <211> 39
   <212> DNA
   <213> Homo sapiens
<220>
   <223> TRAJ30*01_BB-S_F1
<400> 128
   ctcgtttgga aaagggacac gacttcatat tctccccaa 39
<210> 129
   <211> 39
   <212> DNA
   <213> Homo sapiens
<220>
   <223> TRAJ31*01_BB-S_F1
<400> 129
   ctcgtttgga gatggaactc agctggtggt gaagcccaa 39
<210> 130
   <211> 39
   <212> DNA
   <213> Homo sapiens
<220>
   <223> TRAJ32*01_BB-S_F1
<400> 130
   ctcgtttgga actggcactc tgcttgctgt ccagccaaa 39
<210> 131
   <211> 39
   <212> DNA
   <213> Homo sapiens
<220>
   <223> TRAJ33*01_BB-S_F1
<400> 131
   ctcgtggggc gctgggacca agctaattat aaagccaga 39
<210> 132
   <211> 38
   <212> DNA
   <213> Homo sapiens
<220>
   <223> TRAJ34*01_BB-S_F1
<400> 132
   ctcgtttggg actgggacca gattacaagt ctttccaa 38
<210> 133
   <211> 39
   <212> DNA
   <213> Homo sapiens
<220>
   <223> TRAJ36*01_BB-S_F1
<400> 133
   ctcgtttggg actggaacga gactcaccgt tattcccta 39
<210> 134
   <211> 39
   <212> DNA
   <213> Homo sapiens
<220>
   <223> TRAJ37*01_BB-S_F1
<400> 134
   ctcgtttggg caagggacaa ctttacaagt aaaaccaga 39
<210> 135
   <211> 39
   <212> DNA
   <213> Homo sapiens
<220>
   <223> TRAJ38*01_BB-S_F1
<400> 135
   ctcgtgggga ttgggaacaa gcctggcagt aaatccgaa 39
<210> 136
   <211> 39
   <212> DNA
   <213> Homo sapiens
<220>
   <223> TRAJ39*01_BB-S_F1
<400> 136
   ctcgtttgga ggaggaacaa ggttaatggt caaacccca 39
<210> 137
   <211> 39
   <212> DNA
   <213> Homo sapiens
<220>
   <223> TRAJ40*01_BB-S_F1
<400> 137
   ctcgtttgga acaggcacca ggctgaaggt tttagcaaa 39
<210> 138
   <211> 39
   <212> DNA
   <213> Homo sapiens
<220>
   <223> TRAJ41*01_BB-S_F1
<400> 138
   ctcgtttggc aaaggcacct cgctgttggt cacacccca 39
<210> 139
   <211> 39
   <212> DNA
   <213> Homo sapiens
<220>
   <223> TRAJ42*01_BB-S_F1
<400> 139
   ctcgtttgga aaaggcacta aactctctgt taaaccaaa 39
<210> 140
   <211> 39
   <212> DNA
   <213> Homo sapiens
<220>
   <223> TRAJ43*01_BB-S_F1
<400> 140
   ctcgtttgga gcagggacca gactgacagt aaaaccaaa 39
<210> 141
   <211> 39
   <212> DNA
   <213> Homo sapiens
<220>
   <223> TRAJ44*01_BB-S_F1
<400> 141
   ctcgtttggg actggaacaa gacttcaggt cacgctcga 39
<210> 142
   <211> 39
   <212> DNA
   <213> Homo sapiens
<220>
   <223> TRAJ45*01_BB-S_F1
<400> 142
   ctcgtttggc aaagggactc atctaatcat ccagcccta 39
<210> 143
   <211> 39
   <212> DNA
   <213> Homo sapiens
<220>
   <223> TRAJ46*01_BB-S_F1
<400> 143
   ctcgtttggg accgggactc gtttagcagt taggcccaa 39
<210> 144
   <211> 39
   <212> DNA
   <213> Homo sapiens
<220>
   <223> TRAJ47*01_BB-S_F1
<400> 144
   ctcgtttggc gcaggaacca ttctgagagt caagtccta 39
<210> 145
   <211> 39
   <212> DNA
   <213> Homo sapiens
<220>
   <223> TRAJ48*01_BB-S_F1
<400> 145
   ctcgtttggg actggaacaa gactcaccat catacccaa 39
<210> 146
   <211> 39
   <212> DNA
   <213> Homo sapiens
<220>
   <223> TRAJ49*01_BB-S_F1
<400> 146
   ctcgtttggg acagggacaa gtttgacggt cattccaaa 39
<210> 147
   <211> 39
   <212> DNA
   <213> Homo sapiens
<220>
   <223> TRAJ50*01_BB-S_F1
<400> 147
   ctcgtttggg ccagggacaa gcttatcagt cattccaaa 39
<210> 148
   <211> 39
   <212> DNA
   <213> Homo sapiens
<220>
   <223> TRAJ52*01_BB-S_F1
<400> 148
   ctcgtttgga caagggacca tcttgactgt ccatccaaa 39
<210> 149
   <211> 39
   <212> DNA
   <213> Homo sapiens
<220>
   <223> TRAJ53*01_BB-S_F1
<400> 149
   ctcgtttgga aaaggaactc tcttaaccgt gaatccaaa 39
<210> 150
   <211> 39
   <212> DNA
   <213> Homo sapiens
<220>
   <223> TRAJ54*01_BB-S_F1
<400> 150
   ctcgtttggc caaggaacca ggctgactat caacccaaa 39
<210> 151
   <211> 39
   <212> DNA
   <213> Homo sapiens
<220>
   <223> TRAJ56*01_BB-S_F1
<400> 151
   ctcgtttgga aaaggaataa ctctgagtgt tagaccaga 39
<210> 152
   <211> 39
   <212> DNA
   <213> Homo sapiens
<220>
   <223> TRAJ57*01_BB-S_F1
<400> 152
   ctcgtttgga aagggaacga aactgacagt aaacccata 39
<210> 153
   <211> 33
   <212> DNA
   <213> Homo sapiens
<220>
   <223> TRAJ59*01_BB-S_F1
<400> 153
   ctcgtttgga atggggacgc aagtgagagt gaa 33
<210> 154
   <211> 39
   <212> DNA
   <213> Homo sapiens
<220>
   <223> TRAJ61*01_BB-S_F1
<400> 154
   ctcgtttgga gccaacacta gaggaatcat gaaactcaa 39
<210> 155
   <211> 39
   <212> DNA
   <213> Homo sapiens
<220>
   <223> TRAJ1*01_BB-S_R1
<400> 155
   gatatgggga gaagtggaaa ctctggttcc tttgccaaa 39
<210> 156
   <211> 39
   <212> DNA
   <213> Homo sapiens
<220>
   <223> TRAJ2*01_BB-S_R1
<400> 156
   gatatcagat ataatgaata catgggtccc tttcccaaa 39
<210> 157
   <211> 39
   <212> DNA
   <213> Homo sapiens
<220>
   <223> TRAJ3*01_BB-S_R1
<400> 157
   gatatttggc cggatgctga gtctggtccc tgatccaaa 39
<210> 158
   <211> 39
   <212> DNA
   <213> Homo sapiens
<220>
   <223> TRAJ4*01_BB-S_R1
<400> 158
   gatatatggg tgtacagcca gcctggtccc tgctccaaa 39
<210> 159
   <211> 39
   <212> DNA
   <213> Homo sapiens
<220>
   <223> TRAJ5*01_BB-S_R1
<400> 159
   gatatttggt tgcacttgga gtcttgttcc actcccaaa 39
<210> 160
   <211> 39
   <212> DNA
   <213> Homo sapiens
<220>
   <223> TRAJ6*01_BB-S_R1
<400> 160
   gatatacgga tgaacaataa ggctggttcc tcttccaaa 39
<210> 161
   <211> 39
   <212> DNA
   <213> Homo sapiens
<220>
   <223> TRAJ7*01_BB-S_R1
<400> 161
   gatatttggt atgaccacca cttggttccc cttcccaaa 39
<210> 162
   <211> 39
   <212> DNA
   <213> Homo sapiens
<220>
   <223> TRAJ8*01_BB-S_R1
<400> 162
   gatatttgga ctgaccagaa gtcgggtgcc agttccaaa 39
<210> 163
   <211> 39
   <212> DNA
   <213> Homo sapiens
<220>
   <223> TRAJ9*01_BB-S_R1
<400> 163
   gatatttgct ttaacaaata gtcttgttcc tgctccaaa 39
<210> 164
   <211> 39
   <212> DNA
   <213> Homo sapiens
<220>
   <223> TRAJ10*01_BB-S_R1
<400> 164
   gatattgagt tccactttta gctgagtgcc tgtcccaaa 39
<210> 165
   <211> 39
   <212> DNA
   <213> Homo sapiens
<220>
   <223> TRAJ11*01_BB-S_R1
<400> 165
   gatatctgga gagactagaa gcatagtccc cttcccaaa 39
<210> 166
   <211> 39
   <212> DNA
   <213> Homo sapiens
<220>
   <223> TRAJ12*01_BB-S_R1
<400> 166
   gatatcaggc ctgaccagca gtctggtccc actcccaaa 39
<210> 167
   <211> 39
   <212> DNA
   <213> Homo sapiens
<220>
   <223> TRAJ13*01_BB-S_R1
<400> 167
   gatatttggg atgacttgga gctttgttcc aattccaaa 39
<210> 168
   <211> 39
   <212> DNA
   <213> Homo sapiens
<220>
   <223> TRAJ14*01_BB-S_R1
<400> 168
   gatatcaggt tttactgata atcttgtccc actcccaaa 39
<210> 169
   <211> 39
   <212> DNA
   <213> Homo sapiens
<220>
   <223> TRAJ15*01_BB-S_R1
<400> 169
   gatattggaa ctcactgata ggtgggttcc cttcccaaa 39
<210> 170
   <211> 39
   <212> DNA
   <213> Homo sapiens
<220>
   <223> TRAJ16*01_BB-S_R1
<400> 170
   gatattaaga tccaccttta acatggttcc ccttgcaaa 39
<210> 171
   <211> 39
   <212> DNA
   <213> Homo sapiens
<220>
   <223> TRAJ17*01_BB-S_R1
<400> 171
   gatatttggt ttaactagca ccctggttcc tcctccaaa 39
<210> 172
   <211> 39
   <212> DNA
   <213> Homo sapiens
<220>
   <223> TRAJ18*01_BB-S_R1
<400> 172
   gatatcaggc cagacagtca actgagttcc tcttccaaa 39
<210> 173
   <211> 39
   <212> DNA
   <213> Homo sapiens
<220>
   <223> TRAJ19*01_BB-S_R1
<400> 173
   gatatttgga gtgacattat gtttggatcc ctttccaaa 39
<210> 174
   <211> 39
   <212> DNA
   <213> Homo sapiens
<220>
   <223> TRAJ20*01_BB-S_R1
<400> 174
   gatatttgct cttacagtta ctgtggttcc ggctccaaa 39
<210> 175
   <211> 39
   <212> DNA
   <213> Homo sapiens
<220>
   <223> TRAJ21*01_BB-S_R1
<400> 175
   gatatttggt tttacattga gtttggtccc agatccaaa 39
<210> 176
   <211> 39
   <212> DNA
   <213> Homo sapiens
<220>
   <223> TRAJ22*01_BB-S_R1
<400> 176
   gatatcaggt aaaacagtca attgtgtccc agatccaaa 39
<210> 177
   <211> 39
   <212> DNA
   <213> Homo sapiens
<220>
   <223> TRAJ23*01_BB-S_R1
<400> 177
   gatattgggt ttcacagata actccgttcc ctgtccaaa 39
<210> 178
   <211> 39
   <212> DNA
   <213> Homo sapiens
<220>
   <223> TRAJ24*01_BB-S_R1
<400> 178
   gatatctggg gtgaccacaa cctgggtccc tgctccaaa 39
<210> 179
   <211> 39
   <212> DNA
   <213> Homo sapiens
<220>
   <223> TRAJ25*01_BB-S_R1
<400> 179
   gatatttggc ttgacaagca gccttgtccc cttcccaaa 39
<210> 180
   <211> 39
   <212> DNA
   <213> Homo sapiens
<220>
   <223> TRAJ26*01_BB-S_R1
<400> 180
   gatatagggc agcacggaca atctggttcc gggaccaaa 39
<210> 181
   <211> 39
   <212> DNA
   <213> Homo sapiens
<220>
   <223> TRAJ27*01_BB-S_R1
<400> 181
   gatatttggc ttcacagtga gcgtagtccc atccccaaa 39
<210> 182
   <211> 39
   <212> DNA
   <213> Homo sapiens
<220>
   <223> TRAJ28*01_BB-S_R1
<400> 182
   gatatttggt atgaccgaga gtttggtccc cttcccaaa 39
<210> 183
   <211> 39
   <212> DNA
   <213> Homo sapiens
<220>
   <223> TRAJ29*01_BB-S_R1
<400> 183
   gatatttgca atcacagaaa gtcttgtgcc ctttccaaa 39
<210> 184
   <211> 39
   <212> DNA
   <213> Homo sapiens
<220>
   <223> TRAJ30*01_BB-S_R1
<400> 184
   gatattgggg agaatatgaa gtcgtgtccc ttttccaaa 39
<210> 185
   <211> 39
   <212> DNA
   <213> Homo sapiens
<220>
   <223> TRAJ31*01_BB-S_R1
<400> 185
   gatattgggc ttcaccacca gctgagttcc atctccaaa 39
<210> 186
   <211> 39
   <212> DNA
   <213> Homo sapiens
<220>
   <223> TRAJ32*01_BB-S_R1
<400> 186
   gatatttggc tggacagcaa gcagagtgcc agttccaaa 39
<210> 187
   <211> 39
   <212> DNA
   <213> Homo sapiens
<220>
   <223> TRAJ33*01_BB-S_R1
<400> 187
   gatatctggc tttataatta gcttggtccc agcgcccca 39
<210> 188
   <211> 39
   <212> DNA
   <213> Homo sapiens
<220>
   <223> TRAJ34*01_BB-S_R1
<400> 188
   gatatttgga aagacttgta atctggtccc agtcccaaa 39
<210> 189
   <211> 39
   <212> DNA
   <213> Homo sapiens
<220>
   <223> TRAJ36*01_BB-S_R1
<400> 189
   gatataggga ataacggtga gtctcgttcc agtcccaaa 39
<210> 190
   <211> 39
   <212> DNA
   <213> Homo sapiens
<220>
   <223> TRAJ37*01_BB-S_R1
<400> 190
   gatatctggt tttacttgta aagttgtccc ttgcccaaa 39
<210> 191
   <211> 39
   <212> DNA
   <213> Homo sapiens
<220>
   <223> TRAJ38*01_BB-S_R1
<400> 191
   gatattcgga tttactgcca ggcttgttcc caatcccca 39
<210> 192
   <211> 39
   <212> DNA
   <213> Homo sapiens
<220>
   <223> TRAJ39*01_BB-S_R1
<400> 192
   gatatggggt ttgaccatta accttgttcc tcctccaaa 39
<210> 193
   <211> 39
   <212> DNA
   <213> Homo sapiens
<220>
   <223> TRAJ40*01_BB-S_R1
<400> 193
   gatatttgct aaaaccttca gcctggtgcc tgttccaaa 39
<210> 194
   <211> 39
   <212> DNA
   <213> Homo sapiens
<220>
   <223> TRAJ41*01_BB-S_R1
<400> 194
   gatatggggt gtgaccaaca gcgaggtgcc tttgccaaa 39
<210> 195
   <211> 39
   <212> DNA
   <213> Homo sapiens
<220>
   <223> TRAJ42*01_BB-S_R1
<400> 195
   gatatttggt ttaacagaga gtttagtgcc ttttccaaa 39
<210> 196
   <211> 39
   <212> DNA
   <213> Homo sapiens
<220>
   <223> TRAJ43*01_BB-S_R1
<400> 196
   gatatttggt tttactgtca gtctggtccc tgctccaaa 39
<210> 197
   <211> 39
   <212> DNA
   <213> Homo sapiens
<220>
   <223> TRAJ44*01_BB-S_R1
<400> 197
   gatatcgagc gtgacctgaa gtcttgttcc agtcccaaa 39
<210> 198
   <211> 39
   <212> DNA
   <213> Homo sapiens
<220>
   <223> TRAJ45*01_BB-S_R1
<400> 198
   gatatagggc tggatgatta gatgagtccc tttgccaaa 39
<210> 199
   <211> 39
   <212> DNA
   <213> Homo sapiens
<220>
   <223> TRAJ46*01_BB-S_R1
<400> 199
   gatattgggc ctaactgcta aacgagtccc ggtcccaaa 39
<210> 200
   <211> 39
   <212> DNA
   <213> Homo sapiens
<220>
   <223> TRAJ47*01_BB-S_R1
<400> 200
   gatataggac ttgactctca gaatggttcc tgcgccaaa 39
<210> 201
   <211> 39
   <212> DNA
   <213> Homo sapiens
<220>
   <223> TRAJ48*01_BB-S_R1
<400> 201
   gatattgggt atgatggtga gtcttgttcc agtcccaaa 39
<210> 202
   <211> 39
   <212> DNA
   <213> Homo sapiens
<220>
   <223> TRAJ49*01_BB-S_R1
<400> 202
   gatatttgga atgaccgtca aacttgtccc tgtcccaaa 39
<210> 203
   <211> 39
   <212> DNA
   <213> Homo sapiens
<220>
   <223> TRAJ50*01_BB-S_R1
<400> 203
   gatatttgga atgactgata agcttgtccc tggcccaaa 39
<210> 204
   <211> 39
   <212> DNA
   <213> Homo sapiens
<220>
   <223> TRAJ52*01_BB-S_R1
<400> 204
   gatatttgga tggacagtca agatggtccc ttgtccaaa 39
<210> 205
   <211> 39
   <212> DNA
   <213> Homo sapiens
<220>
   <223> TRAJ53*01_BB-S_R1
<400> 205
   gatatttgga ttcacggtta agagagttcc ttttccaaa 39
<210> 206
   <211> 39
   <212> DNA
   <213> Homo sapiens
<220>
   <223> TRAJ54*01_BB-S_R1
<400> 206
   gatatttggg ttgatagtca gcctggttcc ttggccaaa 39
<210> 207
   <211> 39
   <212> DNA
   <213> Homo sapiens
<220>
   <223> TRAJ56*01_BB-S_R1
<400> 207
   gatatctggt ctaacactca gagttattcc ttttccaaa 39
<210> 208
   <211> 39
   <212> DNA
   <213> Homo sapiens
<220>
   <223> TRAJ57*01_BB-S_R1
<400> 208
   gatatatggg tttactgtca gtttcgttcc ctttccaaa 39
<210> 209
   <211> 33
   <212> DNA
   <213> Homo sapiens
<220>
   <223> TRAJ59*01_BB-S_R1
<400> 209
   gatattcact ctcacttgcg tccccattcc aaa 33
<210> 210
   <211> 39
   <212> DNA
   <213> Homo sapiens
<220>
   <223> TRAJ61*01_BB-S_R1
<400> 210
   gatattgagt ttcatgattc ctctagtgtt ggctccaaa 39
<210> 211
   <211> 57
   <212> DNA
   <213> Homo sapiens
<220>
   <223> TRAJ1*01_BB-L_F1
<400> 211
   ctcgattacc tcccagttgc aatttggcaa aggaaccaga gtttccactt ctcccca 57
<210> 212
   <211> 60
   <212> DNA
   <213> Homo sapiens
<220>
   <223> TRAJ2*01_BB-L_F1
<400> 212
   ctcgggaaca attgataaac tcacatttgg gaaagggacc catgtattca ttatatctga 60
<210> 213
   <211> 57
   <212> DNA
   <213> Homo sapiens
<220>
   <223> TRAJ3*01_BB-L_F1
<400> 213
   ctcgagtgct tccaagataa tctttggatc agggaccaga ctcagcatcc ggccaaa 57
<210> 214
   <211> 57
   <212> DNA
   <213> Homo sapiens
<220>
   <223> TRAJ4*01_BB-L_F1
<400> 214
   ctcgggctac aataagctga tctttggagc agggaccagg ctggctgtac acccata 57
<210> 215
   <211> 54
   <212> DNA
   <213> Homo sapiens
<220>
   <223> TRAJ5*01_BB-L_F1
<400> 215
   ctcgaggaga gcacttactt ttgggagtgg aacaagactc caagtgcaac caaa 54
<210> 216
   <211> 57
   <212> DNA
   <213> Homo sapiens
<220>
   <223> TRAJ6*01_BB-L_F1
<400> 216
   ctcgggaagc tacataccta catttggaag aggaaccagc cttattgttc atccgta 57
<210> 217
   <211> 54
   <212> DNA
   <213> Homo sapiens
<220>
   <223> TRAJ7*01_BB-L_F1
<400> 217
   ctcgaacaac agactcgctt ttgggaaggg gaaccaagtg gtggtcatac caaa 54
<210> 218
   <211> 54
   <212> DNA
   <213> Homo sapiens
<220>
   <223> TRAJ8*01_BB-L_F1
<400> 218
   ctcgtttcag aaacttgtat ttggaactgg cacccgactt ctggtcagtc caaa 54
<210> 219
   <211> 54
   <212> DNA
   <213> Homo sapiens
<220>
   <223> TRAJ9*01_BB-L_F1
<400> 219
   ctcgggcttc aaaactatct ttggagcagg aacaagacta tttgttaaag caaa 54
<210> 220
   <211> 57
   <212> DNA
   <213> Homo sapiens
<220>
   <223> TRAJ10*01_BB-L_F1
<400> 220
   ctcgggagga aacaaactca cctttgggac aggcactcag ctaaaagtgg aactcaa 57
<210> 221
   <211> 54
   <212> DNA
   <213> Homo sapiens
<220>
   <223> TRAJ11*01_BB-L_F1
<400> 221
   ctcgtacagc accctcacct ttgggaaggg gactatgctt ctagtctctc caga 54
<210> 222
   <211> 54
   <212> DNA
   <213> Homo sapiens
<220>
   <223> TRAJ12*01_BB-L_F1
<400> 222
   ctcgagttat aaattgatct ttgggagtgg gaccagactg ctggtcaggc ctga 54
<210> 223
   <211> 57
   <212> DNA
   <213> Homo sapiens
<220>
   <223> TRAJ13*01_BB-L_F1
<400> 223
   ctcgggttac cagaaagtta cctttggaat tggaacaaag ctccaagtca tcccaaa 57
<210> 224
   <211> 45
   <212> DNA
   <213> Homo sapiens
<220>
   <223> TRAJ14*01_BB-L_F1
<400> 224
   ctcgttcatc tttgggagtg ggacaagatt atcagtaaaa cctga 45
<210> 225
   <211> 54
   <212> DNA
   <213> Homo sapiens
<220>
   <223> TRAJ15*01_BB-L_F1
<400> 225
   ctcgggaact gctctgatct ttgggaaggg aacccaccta tcagtgagtt ccaa 54
<210> 226
   <211> 54
   <212> DNA
   <213> Homo sapiens
<220>
   <223> TRAJ16*01_BB-L_F1
<400> 226
   ctcgggacag aagctgctct ttgcaagggg aaccatgtta aaggtggatc ttaa 54
<210> 227
   <211> 57
   <212> DNA
   <213> Homo sapiens
<220>
   <223> TRAJ17*01_BB-L_F1
<400> 227
   ctcggcaggc aacaagctaa cttttggagg aggaaccagg gtgctagtta aaccaaa 57
<210> 228
   <211> 60
   <212> DNA
   <213> Homo sapiens
<220>
   <223> TRAJ18*01_BB-L_F1
<400> 228
   ctcgtcaacc ctggggaggc tatactttgg aagaggaact cagttgactg tctggcctga 60
<210> 229
   <211> 54
   <212> DNA
   <213> Homo sapiens
<220>
   <223> TRAJ19*01_BB-L_F1
<400> 229
   ctcgttctac aatttcacct ttggaaaggg atccaaacat aatgtcactc caaa 54
<210> 230
   <211> 51
   <212> DNA
   <213> Homo sapiens
<220>
   <223> TRAJ20*01_BB-L_F1
<400> 230
   ctcgtacaag ctcagctttg gagccggaac cacagtaact gtaagagcaa a 51
<210> 231
   <211> 48
   <212> DNA
   <213> Homo sapiens
<220>
   <223> TRAJ21*01_BB-L_F1
<400> 231
   ctcgaaattt tactttggat ctgggaccaa actcaatgta aaaccaaa 48
<210> 232
   <211> 57
   <212> DNA
   <213> Homo sapiens
<220>
   <223> TRAJ22*01_BB-L_F1
<400> 232
   ctcgtctgca aggcaactga cctttggatc tgggacacaa ttgactgttt tacctga 57
<210> 233
   <211> 57
   <212> DNA
   <213> Homo sapiens
<220>
   <223> TRAJ23*01_BB-L_F1
<400> 233
   ctcgcaggga ggaaagctta tctttggaca gggaacggag ttatctgtga aacccaa 57
<210> 234
   <211> 57
   <212> DNA
   <213> Homo sapiens
<220>
   <223> TRAJ24*01_BB-L_F1
<400> 234
   ctcgagttgg ggtaaattgc agtttggagc agggacccag gttgtggtca ccccaga 57
<210> 235
   <211> 54
   <212> DNA
   <213> Homo sapiens
<220>
   <223> TRAJ25*01_BB-L_F1
<400> 235
   ctcgggcttc tcctttatct ttgggaaggg gacaaggctg cttgtcaagc caaa 54
<210> 236
   <211> 54
   <212> DNA
   <213> Homo sapiens
<220>
   <223> TRAJ26*01_BB-L_F1
<400> 236
   ctcgggtcag aattttgtct ttggtcccgg aaccagattg tccgtgctgc ccta 54
<210> 237
   <211> 54
   <212> DNA
   <213> Homo sapiens
<220>
   <223> TRAJ27*01_BB-L_F1
<400> 237
   ctcggcaggc aaatcaacct ttggggatgg gactacgctc actgtgaagc caaa 54
<210> 238
   <211> 60
   <212> DNA
   <213> Homo sapiens
<220>
   <223> TRAJ28*01_BB-L_F1
<400> 238
   ctcggctggg agttaccaac tcacttttgg gaaggggacc aaactctcgg tcataccaaa 60
<210> 239
   <211> 54
   <212> DNA
   <213> Homo sapiens
<220>
   <223> TRAJ29*01_BB-L_F1
<400> 239
   ctcgaacaca cctcttgtct ttggaaaggg cacaagactt tctgtgattg caaa 54
<210> 240
   <211> 51
   <212> DNA
   <213> Homo sapiens
<220>
   <223> TRAJ30*01_BB-L_F1
<400> 240
   ctcggacaag atcatctttg gaaaagggac acgacttcat attctcccca a 51
<210> 241
   <211> 51
   <212> DNA
   <213> Homo sapiens
<220>
   <223> TRAJ31*01_BB-L_F1
<400> 241
   ctcggccaga ctcatgtttg gagatggaac tcagctggtg gtgaagccca a 51
<210> 242
   <211> 60
   <212> DNA
   <213> Homo sapiens
<220>
   <223> TRAJ32*01_BB-L_F1
<400> 242
   ctcgggtgct acaaacaagc tcatctttgg aactggcact ctgcttgctg tccagccaaa 60
<210> 243
   <211> 51
   <212> DNA
   <213> Homo sapiens
<220>
   <223> TRAJ33*01_BB-L_F1
<400> 243
   ctcgtatcag ttaatctggg gcgctgggac caagctaatt ataaagccag a 51
<210> 244
   <211> 51
   <212> DNA
   <213> Homo sapiens
<220>
   <223> TRAJ34*01_BB-L_F1
<400> 244
   ctcggacaag ctcatctttg ggactgggac cagattacaa gtctttccaa a 51
<210> 245
   <211> 54
   <212> DNA
   <213> Homo sapiens
<220>
   <223> TRAJ36*01_BB-L_F1
<400> 245
   ctcggcaaac aacctcttct ttgggactgg aacgagactc accgttattc ccta 54
<210> 246
   <211> 57
   <212> DNA
   <213> Homo sapiens
<220>
   <223> TRAJ37*01_BB-L_F1
<400> 246
   ctcgaacaca ggcaaactaa tctttgggca agggacaact ttacaagtaa aaccaga 57
<210> 247
   <211> 57
   <212> DNA
   <213> Homo sapiens
<220>
   <223> TRAJ38*01_BB-L_F1
<400> 247
   ctcgaacaac cgtaagctga tttggggatt gggaacaagc ctggcagtaa atccgaa 57
<210> 248
   <211> 57
   <212> DNA
   <213> Homo sapiens
<220>
   <223> TRAJ39*01_BB-L_F1
<400> 248
   ctcggcaggc aacatgctca cctttggagg aggaacaagg ttaatggtca aacccca 57
<210> 249
   <211> 54
   <212> DNA
   <213> Homo sapiens
<220>
   <223> TRAJ40*01_BB-L_F1
<400> 249
   ctcgacctac aaatacatct ttggaacagg caccaggctg aaggttttag caaa 54
<210> 250
   <211> 57
   <212> DNA
   <213> Homo sapiens
<220>
   <223> TRAJ41*01_BB-L_F1
<400> 250
   ctcgtccggg tatgcactca actttggcaa aggcacctcg ctgttggtca cacccca 57
<210> 251
   <211> 60
   <212> DNA
   <213> Homo sapiens
<220>
   <223> TRAJ42*01_BB-L_F1
<400> 251
   ctcgggaagc caaggaaatc tcatctttgg aaaaggcact aaactctctg ttaaaccaaa 60
<210> 252
   <211> 48
   <212> DNA
   <213> Homo sapiens
<220>
   <223> TRAJ43*01_BB-L_F1
<400> 252
   ctcggacatg cgctttggag cagggaccag actgacagta aaaccaaa 48
<210> 253
   <211> 57
   <212> DNA
   <213> Homo sapiens
<220>
   <223> TRAJ44*01_BB-L_F1
<400> 253
   ctcgactgcc agtaaactca cctttgggac tggaacaaga cttcaggtca cgctcga 57
<210> 254
   <211> 60
   <212> DNA
   <213> Homo sapiens
<220>
   <223> TRAJ45*01_BB-L_F1
<400> 254
   ctcgggaggt gctgacggac tcacctttgg caaagggact catctaatca tccagcccta 60
<210> 255
   <211> 57
   <212> DNA
   <213> Homo sapiens
<220>
   <223> TRAJ46*01_BB-L_F1
<400> 255
   ctcgagcgga gacaagctga cttttgggac cgggactcgt ttagcagtta ggcccaa 57
<210> 256
   <211> 51
   <212> DNA
   <213> Homo sapiens
<220>
   <223> TRAJ47*01 BB-L F1
<400> 256
   ctcgaacaaa ctggtctttg gcgcaggaac cattctgaga gtcaagtcct a 51
<210> 257
   <211> 57
   <212> DNA
   <213> Homo sapiens
<220>
   <223> TRAJ48*01_BB-L_F1
<400> 257
   ctcgggaaat gagaaattaa cctttgggac tggaacaaga ctcaccatca tacccaa 57
<210> 258
   <211> 51
   <212> DNA
   <213> Homo sapiens
<220>
   <223> TRAJ49*01_BB-L_F1
<400> 258
   ctcgaaccag ttctattttg ggacagggac aagtttgacg gtcattccaa a 51
<210> 259
   <211> 54
   <212> DNA
   <213> Homo sapiens
<220>
   <223> TRAJ50*01_BB-L_F1
<400> 259
   ctcgtacgac aaggtgatat ttgggccagg gacaagctta tcagtcattc caaa 54
<210> 260
   <211> 63
   <212> DNA
   <213> Homo sapiens
<220>
   <223> TRAJ52*01_BB-L_F1
<400> 260
   ctcgggtact agctatggaa agctgacatt tggacaaggg accatcttga ctgtccatcc 60
   aaa 63
<210> 261
   <211> 60
   <212> DNA
   <213> Homo sapiens
<220>
   <223> TRAJ53*01_BB-L_F1
<400> 261
   ctcgggtagc aactataaac tgacatttgg aaaaggaact ctcttaaccg tgaatccaaa 60
<210> 262
   <211> 54
   <212> DNA
   <213> Homo sapiens
<220>
   <223> TRAJ54*01_BB-L_F1
<400> 262
   ctcggcccag aagctggtat ttggccaagg aaccaggctg actatcaacc caaa 54
<210> 263
   <211> 57
   <212> DNA
   <213> Homo sapiens
<220>
   <223> TRAJ56*01_BB-L_F1
<400> 263
   ctcggccaat agtaagctga catttggaaa aggaataact ctgagtgtta gaccaga 57
<210> 264
   <211> 57
   <212> DNA
   <213> Homo sapiens
<220>
   <223> TRAJ57*01_BB-L_F1
<400> 264
   ctcgggatct gaaaagctgg tctttggaaa gggaacgaaa ctgacagtaa acccata 57
<210> 265
   <211> 48
   <212> DNA
   <213> Homo sapiens
<220>
   <223> TRAJ59*01_BB-L_F1
<400> 265
   ctcgaacagg aaatttacat ttggaatggg gacgcaagtg agagtgaa 48
<210> 266
   <211> 54
   <212> DNA
   <213> Homo sapiens
<220>
   <223> TRAJ61*01_BB-L_F1
<400> 266
   ctcgaatagg aaactgacat ttggagccaa cactagagga atcatgaaac tcaa 54
<210> 267
   <211> 57
   <212> DNA
   <213> Homo sapiens
<220>
   <223> TRAJ1*01_BB-L_R1
<400> 267
   gatatgggga gaagtggaaa ctctggttcc tttgccaaat tgcaactggg aggtaat 57
<210> 268
   <211> 60
   <212> DNA
   <213> Homo sapiens
<220>
   <223> TRAJ2*01_BB-L_R1
<400> 268
   gatatcagat ataatgaata catgggtccc tttcccaaat gtgagtttat caattgttcc 60
<210> 269
   <211> 57
   <212> DNA
   <213> Homo sapiens
<220>
   <223> TRAJ3*01_BB-L_R1
<400> 269
   gatatttggc cggatgctga gtctggtccc tgatccaaag attatcttgg aagcact 57
<210> 270
   <211> 57
   <212> DNA
   <213> Homo sapiens
<220>
   <223> TRAJ4*01_BB-L_R1
<400> 270
   gatatatggg tgtacagcca gcctggtccc tgctccaaaa atcagcttat tgtagcc 57
<210> 271
   <211> 54
   <212> DNA
   <213> Homo sapiens
<220>
   <223> TRAJ5*01_BB-L_R1
<400> 271
   gatatttggt tgcacttgga gtcttgttcc actcccaaaa gtaagtgctc tcct 54
<210> 272
   <211> 57
   <212> DNA
   <213> Homo sapiens
<220>
   <223> TRAJ6*01_BB-L_R1
<400> 272
   gatatacgga tgaacaataa ggctggttcc tcttccaaat gtaggtatgt agcttcc 57
<210> 273
   <211> 54
   <212> DNA
   <213> Homo sapiens
<220>
   <223> TRAJ7*01_BB-L_R1
<400> 273
   gatatttggt atgaccacca cttggttccc cttcccaaaa gcgagtctgt tgtt 54
<210> 274
   <211> 54
   <212> DNA
   <213> Homo sapiens
<220>
   <223> TRAJ8*01_BB-L_R1
<400> 274
   gatatttgga ctgaccagaa gtcgggtgcc agttccaaat acaagtttct gaaa 54
<210> 275
   <211> 54
   <212> DNA
   <213> Homo sapiens
<220>
   <223> TRAJ9*01_BB-L_R1
<400> 275
   gatatttgct ttaacaaata gtcttgttcc tgctccaaag atagttttga agcc 54
<210> 276
   <211> 57
   <212> DNA
   <213> Homo sapiens
<220>
   <223> TRAJ10*01_BB-L_R1
<400> 276
   gatattgagt tccactttta gctgagtgcc tgtcccaaag gtgagtttgt ttcctcc 57
<210> 277
   <211> 54
   <212> DNA
   <213> Homo sapiens
<220>
   <223> TRAJ11*01-BB-L_R1
<400> 277
   gatatctgga gagactagaa gcatagtccc cttcccaaag gtgagggtgc tgta 54
<210> 278
   <211> 54
   <212> DNA
   <213> Homo sapiens
<220>
   <223> TRAJ12*01_BB-L_R1
<400> 278
   gatatcaggc ctgaccagca gtctggtccc actcccaaag atcaatttat aact 54
<210> 279
   <211> 57
   <212> DNA
   <213> Homo sapiens
<220>
   <223> TRAJ13*01_BB-L_R1
<400> 279
   gatatttggg atgacttgga gctttgttcc aattccaaag gtaactttct ggtaacc 57
<210> 280
   <211> 45
   <212> DNA
   <213> Homo sapiens
<220>
   <223> TRAJ14*01_BB-L_R1
<400> 280
   gatatcaggt tttactgata atcttgtccc actcccaaag atgaa 45
<210> 281
   <211> 54
   <212> DNA
   <213> Homo sapiens
<220>
   <223> TRAJ15*01_BB-L_R1
<400> 281
   gatattggaa ctcactgata ggtgggttcc cttcccaaag atcagagcag ttcc 54
<210> 282
   <211> 54
   <212> DNA
   <213> Homo sapiens
<220>
   <223> TRAJ16*01_BB-L_R1
<400> 282
   gatattaaga tccaccttta acatggttcc ccttgcaaag agcagcttct gtcc 54
<210> 283
   <211> 57
   <212> DNA
   <213> Homo sapiens
<220>
   <223> TRAJ17*01_BB-L_R1
<400> 283
   gatatttggt ttaactagca ccctggttcc tcctccaaaa gttagcttgt tgcctgc 57
<210> 284
   <211> 60
   <212> DNA
   <213> Homo sapiens
<220>
   <223> TRAJ18*01_BB-L_R1
<400> 284
   gatatcaggc cagacagtca actgagttcc tcttccaaag tatagcctcc ccagggttga 60
<210> 285
   <211> 54
   <212> DNA
   <213> Homo sapiens
<220>
   <223> TRAJ19*01_BB-L_R1
<400> 285
   gatatttgga gtgacattat gtttggatcc ctttccaaag gtgaaattgt agaa 54
<210> 286
   <211> 51
   <212> DNA
   <213> Homo sapiens
<220>
   <223> TRAJ20*01_BB-L_R1
<400> 286
   gatatttgct cttacagtta ctgtggttcc ggctccaaag ctgagcttgt a 51
<210> 287
   <211> 48
   <212> DNA
   <213> Homo sapiens
<220>
   <223> TRAJ21*01_BB-L_R1
<400> 287
   gatatttggt tttacattga gtttggtccc agatccaaag taaaattt 48
<210> 288
   <211> 57
   <212> DNA
   <213> Homo sapiens
<220>
   <223> TRAJ22*01_BB-L_R1
<400> 288
   gatatcaggt aaaacagtca attgtgtccc agatccaaag gtcagttgcc ttgcaga 57
<210> 289
   <211> 57
   <212> DNA
   <213> Homo sapiens
<220>
   <223> TRAJ23*01_BB-L_R1
<400> 289
   gatattgggt ttcacagata actccgttcc ctgtccaaag ataagctttc ctccctg 57
<210> 290
   <211> 57
   <212> DNA
   <213> Homo sapiens
<220>
   <223> TRAJ24*01_BB-L_R1
<400> 290
   gatatctggg gtgaccacaa cctgggtccc tgctccaaac tgcaatttac cccaact 57
<210> 291
   <211> 54
   <212> DNA
   <213> Homo sapiens
<220>
   <223> TRAJ25*01_BB-L_R1
<400> 291
   gatatttggc ttgacaagca gccttgtccc cttcccaaag ataaaggaga agcc 54
<210> 292
   <211> 54
   <212> DNA
   <213> Homo sapiens
<220>
   <223> TRAJ26*01_BB-L_R1
<400> 292
   gatatagggc agcacggaca atctggttcc gggaccaaag acaaaattct gacc 54
<210> 293
   <211> 54
   <212> DNA
   <213> Homo sapiens
<220>
   <223> TRAJ27*01_BB-L_R1
<400> 293
   gatatttggc ttcacagtga gcgtagtccc atccccaaag gttgatttgc ctgc 54
<210> 294
   <211> 60
   <212> DNA
   <213> Homo sapiens
<220>
   <223> TRAJ28*01_BB-L_R1
<400> 294
   gatatttggt atgaccgaga gtttggtccc cttcccaaaa gtgagttggt aactcccagc 60
<210> 295
   <211> 54
   <212> DNA
   <213> Homo sapiens
<220>
   <223> TRAJ29*01_BB-L_R1
<400> 295
   gatatttgca atcacagaaa gtcttgtgcc ctttccaaag acaagaggtg tgtt 54
<210> 296
   <211> 51
   <212> DNA
   <213> Homo sapiens
<220>
   <223> TRAJ30*01_BB-L_R1
<400> 296
   gatattgggg agaatatgaa gtcgtgtccc ttttccaaag atgatcttgt c 51
<210> 297
   <211> 51
   <212> DNA
   <213> Homo sapiens
<220>
   <223> TRAJ31*01_BB-L_R1
<400> 297
   gatattgggc ttcaccacca gctgagttcc atctccaaac atgagtctgg c 51
<210> 298
   <211> 60
   <212> DNA
   <213> Homo sapiens
<220>
   <223> TRAJ32*01_BB-L_R1
<400> 298
   gatatttggc tggacagcaa gcagagtgcc agttccaaag atgagcttgt ttgtagcacc 60
<210> 299
   <211> 51
   <212> DNA
   <213> Homo sapiens
<220>
   <223> TRAJ33*01_BB-L_R1
<400> 299
   gatatctggc tttataatta gcttggtccc agcgccccag attaactgat a 51
<210> 300
   <211> 51
   <212> DNA
   <213> Homo sapiens
<220>
   <223> TRAJ34*01_BB-L_R1
<400> 300
   gatatttgga aagacttgta atctggtccc agtcccaaag atgagcttgt c 51
<210> 301
   <211> 54
   <212> DNA
   <213> Homo sapiens
<220>
   <223> TRAJ36*01_BB-L_R1
<400> 301
   gatataggga ataacggtga gtctcgttcc agtcccaaag aagaggttgt ttgc 54
<210> 302
   <211> 57
   <212> DNA
   <213> Homo sapiens
<220>
   <223> TRAJ37*01_BB-L_R1
<400> 302
   gatatctggt tttacttgta aagttgtccc ttgcccaaag attagtttgc ctgtgtt 57
<210> 303
   <211> 57
   <212> DNA
   <213> Homo sapiens
<220>
   <223> TRAJ38*01_BB-L_R1
<400> 303
   gatattcgga tttactgcca ggcttgttcc caatccccaa atcagcttac ggttgtt 57
<210> 304
   <211> 57
   <212> DNA
   <213> Homo sapiens
<220>
   <223> TRAJ39*01_BB-L_R1
<400> 304
   gatatggggt ttgaccatta accttgttcc tcctccaaag gtgagcatgt tgcctgc 57
<210> 305
   <211> 54
   <212> DNA
   <213> Homo sapiens
<220>
   <223> TRAJ40*01_BB-L_R1
<400> 305
   gatatttgct aaaaccttca gcctggtgcc tgttccaaag atgtatttgt aggt 54
<210> 306
   <211> 57
   <212> DNA
   <213> Homo sapiens
<220>
   <223> TRAJ41*01_BB-L_R1
<400> 306
   gatatggggt gtgaccaaca gcgaggtgcc tttgccaaag ttgagtgcat acccgga 57
<210> 307
   <211> 60
   <212> DNA
   <213> Homo sapiens
<220>
   <223> TRAJ42*01_BB-L_R1
<400> 307
   gatatttggt ttaacagaga gtttagtgcc ttttccaaag atgagatttc cttggcttcc 60
<210> 308
   <211> 48
   <212> DNA
   <213> Homo sapiens
<220>
   <223> TRAJ43*01_BB-L_R1
<400> 308
   gatatttggt tttactgtca gtctggtccc tgctccaaag cgcatgtc 48
<210> 309
   <211> 57
   <212> DNA
   <213> Homo sapiens
<220>
   <223> TRAJ44*01_BB-L_R1
<400> 309
   gatatcgagc gtgacctgaa gtcttgttcc agtcccaaag gtgagtttac tggcagt 57
<210> 310
   <211> 60
   <212> DNA
   <213> Homo sapiens
<220>
   <223> TRAJ45*01_BB-L_R1
<400> 310
   gatatagggc tggatgatta gatgagtccc tttgccaaag gtgagtccgt cagcacctcc 60
<210> 311
   <211> 57
   <212> DNA
   <213> Homo sapiens
<220>
   <223> TRAJ46*01_BB-L_R1
<400> 311
   gatattgggc ctaactgcta aacgagtccc ggtcccaaaa gtcagcttgt ctccgct 57
<210> 312
   <211> 51
   <212> DNA
   <213> Homo sapiens
<220>
   <223> TRAJ47*01_BB-L_R1
<400> 312
   gatataggac ttgactctca gaatggttcc tgcgccaaag accagtttgt t 51
<210> 313
   <211> 57
   <212> DNA
   <213> Homo sapiens
<220>
   <223> TRAJ48*01_BB-L_R1
<400> 313
   gatattgggt atgatggtga gtcttgttcc agtcccaaag gttaatttct catttcc 57
<210> 314
   <211> 51
   <212> DNA
   <213> Homo sapiens
<220>
   <223> TRAJ49*01_BB-L_R1
<400> 314
   gatatttgga atgaccgtca aacttgtccc tgtcccaaaa tagaactggt t 51
<210> 315
   <211> 54
   <212> DNA
   <213> Homo sapiens
<220>
   <223> TRAJ50*01_BB-L_R1
<400> 315
   gatatttgga atgactgata agcttgtccc tggcccaaat atcaccttgt cgta 54
<210> 316
   <211> 63
   <212> DNA
   <213> Homo sapiens
<220>
   <223> TRAJ52*01_BB-L_R1
<400> 316
   gatatttgga tggacagtca agatggtccc ttgtccaaat gtcagctttc catagctagt 60
   acc 63
<210> 317
   <211> 60
   <212> DNA
   <213> Homo sapiens
<220>
   <223> TRAJ53*01_BB-L_R1
<400> 317
   gatatttgga ttcacggtta agagagttcc ttttccaaat gtcagtttat agttgctacc 60
<210> 318
   <211> 51
   <212> DNA
   <213> Homo sapiens
<220>
   <223> TRAJ54*01_BB-L_R1
<400> 318
   gatatttggg ttgatagtca gcctggttcc ttggccaaat accagcttct g 51
<210> 319
   <211> 57
   <212> DNA
   <213> Homo sapiens
<220>
   <223> TRAJ56*01_BB-L_R1
<400> 319
   gatatctggt ctaacactca gagttattcc ttttccaaat gtcagcttac tattggc 57
<210> 320
   <211> 57
   <212> DNA
   <213> Homo sapiens
<220>
   <223> TRAJ57*01_BB-L_R1
<400> 320
   gatatatggg tttactgtca gtttcgttcc ctttccaaag accagctttt cagatcc 57
<210> 321
   <211> 48
   <212> DNA
   <213> Homo sapiens
<220>
   <223> TRAJ59*01_BB-L_R1
<400> 321
   gatattcact ctcacttgcg tccccattcc aaatgtaaat ttcctgtt 48
<210> 322
   <211> 54
   <212> DNA
   <213> Homo sapiens
<220>
   <223> TRAJ61*01_BB-L_R1
<400> 322
   gatattgagt ttcatgattc ctctagtgtt ggctccaaat gtcagtttcc tatt 54
<210> 323
   <211> 56
   <212> DNA
   <213> Homo sapiens
<220>
   <223> J Donor_Short_TRAJ1
<400> 323
   ggtctcgttt ggcaaaggaa ccagagtttc cacttctccc catatccagt gagacc 56
<210> 324
   <211> 56
   <212> DNA
   <213> Homo sapiens
<220>
   <223> J Donor_Short_TRAJ2
<400> 324
   ggtctcgttt gggaaaggga cccatgtatt cattatatct gatatccagt gagacc 56
<210> 325
   <211> 56
   <212> DNA
   <213> Homo sapiens
<220>
   <223> J Donor_Short_TRAJ3
<400> 325
   ggtctcgttt ggatcaggga ccagactcag catccggcca aatatccagt gagacc 56
<210> 326
   <211> 56
   <212> DNA
   <213> Homo sapiens
<220>
   <223> J Donor_Short_TRAJ4
<400> 326
   ggtctcgttt ggagcaggga ccaggctggc tgtacaccca tatatccagt gagacc 56
<210> 327
   <211> 56
   <212> DNA
   <213> Homo sapiens
<220>
   <223> J Donor_Short_TRAJ5
<400> 327
   ggtctcgttt gggagtggaa caagactcca agtgcaacca aatatccagt gagacc 56
<210> 328
   <211> 56
   <212> DNA
   <213> Homo sapiens
<220>
   <223> J Donor_Short_TRAJ6
<400> 328
   ggtctcgttt ggaagaggaa ccagccttat tgttcatccg tatatccagt gagacc 56
<210> 329
   <211> 56
   <212> DNA
   <213> Homo sapiens
<220>
   <223> J Donor_Short_TRAJ7
<400> 329
   ggtctcgttt gggaagggga accaagtggt ggtcatacca aatatccagt gagacc 56
<210> 330
   <211> 56
   <212> DNA
   <213> Homo sapiens
<220>
   <223> J Donor_Short_TRAJ8
<400> 330
   ggtctcgttt ggaactggca cccgacttct ggtcagtcca aatatccagt gagacc 56
<210> 331
   <211> 56
   <212> DNA
   <213> Homo sapiens
<220>
   <223> J Donor_Short_TRAJ9
<400> 331
   ggtctcgttt ggagcaggaa caagactatt tgttaaagca aatatccagt gagacc 56
<210> 332
   <211> 56
   <212> DNA
   <213> Homo sapiens
<220>
   <223> J Donor_Short_TRAJ10
<400> 332
   ggtctcgttt gggacaggca ctcagctaaa agtggaactc aatatccagt gagacc 56
<210> 333
   <211> 56
   <212> DNA
   <213> Homo sapiens
<220>
   <223> J Donor_Short_TRAJ11
<400> 333
   ggtctcgttt gggaagggga ctatgcttct agtctctcca gatatccagt gagacc 56
<210> 334
   <211> 56
   <212> DNA
   <213> Homo sapiens
<220>
   <223> J Donor_Short_TRAJ12
<400> 334
   ggtctcgttt gggagtggga ccagactgct ggtcaggcct gatatccagt gagacc 56
<210> 335
   <211> 56
   <212> DNA
   <213> Homo sapiens
<220>
   <223> J Donor_Short_TRAJ13
<400> 335
   ggtctcgttt ggaattggaa caaagctcca agtcatccca aatatccagt gagacc 56
<210> 336
   <211> 56
   <212> DNA
   <213> Homo sapiens
<220>
   <223> J Donor_Short_TRAJ14
<400> 336
   ggtctcgttt gggagtggga caagattatc agtaaaacct gatatccagt gagacc 56
<210> 337
   <211> 56
   <212> DNA
   <213> Homo sapiens
<220>
   <223> J Donor_Short_TRAJ15
<400> 337
   ggtctcgttt gggaagggaa cccacctatc agtgagttcc aatatccagt gagacc 56
<210> 338
   <211> 56
   <212> DNA
   <213> Homo sapiens
<220>
   <223> J Donor_Short_TRAJ16
<400> 338
   ggtctcgttt gcaaggggaa ccatgttaaa ggtggatctt aatatccagt gagacc 56
<210> 339
   <211> 56
   <212> DNA
   <213> Homo sapiens
<220>
   <223> J Donor_Short_TRAJ17
<400> 339
   ggtctcgttt ggaggaggaa ccagggtgct agttaaacca aatatccagt gagacc 56
<210> 340
   <211> 56
   <212> DNA
   <213> Homo sapiens
<220>
   <223> J Donor_Short_TRAJ18
<400> 340
   ggtctcgttt ggaagaggaa ctcagttgac tgtctggcct gatatccagt gagacc 56
<210> 341
   <211> 56
   <212> DNA
   <213> Homo sapiens
<220>
   <223> J Donor_Short_TRAJ19
<400> 341
   ggtctcgttt ggaaagggat ccaaacataa tgtcactcca aatatccagt gagacc 56
<210> 342
   <211> 56
   <212> DNA
   <213> Homo sapiens
<220>
   <223> J Donor_Short_TRAJ20
<400> 342
   ggtctcgttt ggagccggaa ccacagtaac tgtaagagca aatatccagt gagacc 56
<210> 343
   <211> 56
   <212> DNA
   <213> Homo sapiens
<220>
   <223> J Donor_Short_TRAJ21
<400> 343
   ggtctcgttt ggatctggga ccaaactcaa tgtaaaacca aatatccagt gagacc 56
<210> 344
   <211> 56
   <212> DNA
   <213> Homo sapiens
<220>
   <223> J Donor_Short_TRAJ22
<400> 344
   ggtctcgttt ggatctggga cacaattgac tgttttacct gatatccagt gagacc 56
<210> 345
   <211> 56
   <212> DNA
   <213> Homo sapiens
<220>
   <223> J Donor_Short_TRAJ23
<400> 345
   ggtctcgttt ggacagggaa cggagttatc tgtgaaaccc aatatccagt gagacc 56
<210> 346
   <211> 56
   <212> DNA
   <213> Homo sapiens
<220>
   <223> J Donor_Short_TRAJ24
<400> 346
   ggtctcgttt ggagcaggga cccaggttgt ggtcacccca gatatccagt gagacc 56
<210> 347
   <211> 56
   <212> DNA
   <213> Homo sapiens
<220>
   <223> J Donor_Short_TRAJ25
<400> 347
   ggtctcgttt gggaagggga caaggctgct tgtcaagcca aatatccagt gagacc 56
<210> 348
   <211> 56
   <212> DNA
   <213> Homo sapiens
<220>
   <223> J Donor_Short_TRAJ26
<400> 348
   ggtctcgttt ggtcccggaa ccagattgtc cgtgctgccc tatatccagt gagacc 56
<210> 349
   <211> 56
   <212> DNA
   <213> Homo sapiens
<220>
   <223> J Donor_Short_TRAJ27
<400> 349
   ggtctcgttt ggggatggga ctacgctcac tgtgaagcca aatatccagt gagacc 56
<210> 350
   <211> 56
   <212> DNA
   <213> Homo sapiens
<220>
   <223> J Donor_Short_TRAJ28
<400> 350
   ggtctcgttt gggaagggga ccaaactctc ggtcatacca aatatccagt gagacc 56
<210> 351
   <211> 56
   <212> DNA
   <213> Homo sapiens
<220>
   <223> J Donor_Short_TRAJ29
<400> 351
   ggtctcgttt ggaaagggca caagactttc tgtgattgca aatatccagt gagacc 56
<210> 352
   <211> 56
   <212> DNA
   <213> Homo sapiens
<220>
   <223> J Donor_Short_TRAJ30
<400> 352
   ggtctcgttt ggaaaaggga cacgacttca tattctcccc aatatccagt gagacc 56
<210> 353
   <211> 56
   <212> DNA
   <213> Homo sapiens
<220>
   <223> J Donor_Short_TRAJ31
<400> 353
   ggtctcgttt ggagatggaa ctcagctggt ggtgaagccc aatatccagt gagacc 56
<210> 354
   <211> 56
   <212> DNA
   <213> Homo sapiens
<220>
   <223> J Donor_Short_TRAJ32
<400> 354
   ggtctcgttt ggaactggca ctctgcttgc tgtccagcca aatatccagt gagacc 56
<210> 355
   <211> 56
   <212> DNA
   <213> Homo sapiens
<220>
   <223> J Donor_Short_TRAJ33
<400> 355
   ggtctcgtgg ggcgctggga ccaagctaat tataaagcca gatatccagt gagacc 56
<210> 356
   <211> 56
   <212> DNA
   <213> Homo sapiens
<220>
   <223> J Donor_Short_TRAJ34
<400> 356
   ggtctcgttt gggactggga ccagattaca agtctttcca aatatccagt gagacc 56
<210> 357
   <211> 56
   <212> DNA
   <213> Homo sapiens
<220>
   <223> J Donor_Short_TRAJ36
<400> 357
   ggtctcgttt gggactggaa cgagactcac cgttattccc tatatccagt gagacc 56
<210> 358
   <211> 56
   <212> DNA
   <213> Homo sapiens
<220>
   <223> J Donor_Short_TRAJ37
<400> 358
   ggtctcgttt gggcaaggga caactttaca agtaaaacca gatatccagt gagacc 56
<210> 359
   <211> 56
   <212> DNA
   <213> Homo sapiens
<220>
   <223> J Donor_Short_TRAJ38
<400> 359
   ggtctcgtgg ggattgggaa caagcctggc agtaaatccg aatatccagt gagacc 56
<210> 360
   <211> 56
   <212> DNA
   <213> Homo sapiens
<220>
   <223> J Donor_Short_TRAJ39
<400> 360
   ggtctcgttt ggaggaggaa caaggttaat ggtcaaaccc catatccagt gagacc 56
<210> 361
   <211> 56
   <212> DNA
   <213> Homo sapiens
<220>
   <223> J Donor_Short_TRAJ40
<400> 361
   ggtctcgttt ggaacaggca ccaggctgaa ggttttagca aatatccagt gagacc 56
<210> 362
   <211> 56
   <212> DNA
   <213> Homo sapiens
<220>
   <223> J Donor_Short_TRAJ41
<400> 362
   ggtctcgttt ggcaaaggca cctcgctgtt ggtcacaccc catatccagt gagacc 56
<210> 363
   <211> 56
   <212> DNA
   <213> Homo sapiens
<220>
   <223> J Donor_Short_TRAJ42
<400> 363
   ggtctcgttt ggaaaaggca ctaaactctc tgttaaacca aatatccagt gagacc 56
<210> 364
   <211> 56
   <212> DNA
   <213> Homo sapiens
<220>
   <223> J Donor_Short_TRAJ43
<400> 364
   ggtctcgttt ggagcaggga ccagactgac agtaaaacca aatatccagt gagacc 56
<210> 365
   <211> 56
   <212> DNA
   <213> Homo sapiens
<220>
   <223> J Donor_Short_TRAJ44
<400> 365
   ggtctcgttt gggactggaa caagacttca ggtcacgctc gatatccagt gagacc 56
<210> 366
   <211> 56
   <212> DNA
   <213> Homo sapiens
<220>
   <223> J Donor_Short_TRAJ45
<400> 366
   ggtctcgttt ggcaaaggga ctcatctaat catccagccc tatatccagt gagacc 56
<210> 367
   <211> 56
   <212> DNA
   <213> Homo sapiens
<220>
   <223> J Donor_Short_TRAJ46
<400> 367
   ggtctcgttt gggaccggga ctcgtttagc agttaggccc aatatccagt gagacc 56
<210> 368
   <211> 56
   <212> DNA
   <213> Homo sapiens
<220>
   <223> J Donor_Short_TRAJ47
<400> 368
   ggtctcgttt ggcgcaggaa ccattctgag agtcaagtcc tatatccagt gagacc 56
<210> 369
   <211> 56
   <212> DNA
   <213> Homo sapiens
<220>
   <223> J Donor_Short_TRAJ48
<400> 369
   ggtctcgttt gggactggaa caagactcac catcataccc aatatccagt gagacc 56
<210> 370
   <211> 56
   <212> DNA
   <213> Homo sapiens
<220>
   <223> J Donor_Short_TRAJ49
<400> 370
   ggtctcgttt gggacaggga caagtttgac ggtcattcca aatatccagt gagacc 56
<210> 371
   <211> 56
   <212> DNA
   <213> Homo sapiens
<220>
   <223> J Donor_Short_TRAJ50
<400> 371
   ggtctcgttt gggccaggga caagcttatc agtcattcca aatatccagt gagacc 56
<210> 372
   <211> 56
   <212> DNA
   <213> Homo sapiens
<220>
   <223> J Donor_Short_TRAJ52
<400> 372
   ggtctcgttt ggacaaggga ccatcttgac tgtccatcca aatatccagt gagacc 56
<210> 373
   <211> 56
   <212> DNA
   <213> Homo sapiens
<220>
   <223> J Donor_Short_TRAJ53
<400> 373
   ggtctcgttt ggaaaaggaa ctctcttaac cgtgaatcca aatatccagt gagacc 56
<210> 374
   <211> 56
   <212> DNA
   <213> Homo sapiens
<220>
   <223> J Donor_Short_TRAJ54
<400> 374
   ggtctcgttt ggccaaggaa ccaggctgac tatcaaccca aatatccagt gagacc 56
<210> 375
   <211> 56
   <212> DNA
   <213> Homo sapiens
<220>
   <223> J Donor_Short_TRAJ56
<400> 375
   ggtctcgttt ggaaaaggaa taactctgag tgttagacca gatatccagt gagacc 56
<210> 376
   <211> 56
   <212> DNA
   <213> Homo sapiens
<220>
   <223> J Donor_Short_TRAJ57
<400> 376
   ggtctcgttt ggaaagggaa cgaaactgac agtaaaccca tatatccagt gagacc 56
<210> 377
   <211> 50
   <212> DNA
   <213> Homo sapiens
<220>
   <223> J Donor_Short_TRAJ59
<400> 377
   ggtctcgttt ggaatgggga cgcaagtgag agtgaatatc cagtgagacc 50
<210> 378
   <211> 56
   <212> DNA
   <213> Homo sapiens
<220>
   <223> J Donor_Short_TRAJ61
<400> 378
   ggtctcgttt ggagccaaca ctagaggaat catgaaactc aatatccagt gagacc 56
<210> 379
   <211> 74
   <212> DNA
   <213> Homo sapiens
<220>
   <223> J Donor_Long_TRAJ1
<400> 379
   ggtctcgatt acctcccagt tgcaatttgg caaaggaacc agagtttcca cttctcccca 60
   tatccagtga gacc 74
<210> 380
   <211> 77
   <212> DNA
   <213> Homo sapiens
<220>
   <223> J Donor_Long_TRAJ2
<400> 380
   ggtctcggga acaattgata aactcacatt tgggaaaggg acccatgtat tcattatatc      60
   tgatatccag tgagacc                                                     77
<210> 381
   <211> 74
   <212> DNA
   <213> Homo sapiens
<220>
   <223> J Donor_Long_TRAJ3
<400> 381
   ggtctcgagt gcttccaaga taatctttgg atcagggacc agactcagca tccggccaaa      60
   tatccagtga gacc                                                        74
<210> 382
   <211> 74
   <212> DNA
   <213> Homo sapiens
<220>
   <223> J Donor_Long_TRAJ4
<400> 382
   ggtctcgggc tacaataagc tgatctttgg agcagggacc aggctggctg tacacccata      60
   tatccagtga gacc                                                        74
<210> 383
   <211> 71
   <212> DNA
   <213> Homo sapiens
<220>
   <223> J Donor_Long_TRAJ5
<400> 383
   ggtctcgagg agagcactta cttttgggag tggaacaaga ctccaagtgc aaccaaatat      60
   ccagtgagac c                                                           71
<210> 384
   <211> 74
   <212> DNA
   <213> Homo sapiens
<220>
   <223> J Donor_Long_TRAJ6
<400> 384
   ggtctcggga agctacatac ctacatttgg aagaggaacc agccttattg ttcatccgta 60
   tatccagtga gacc 74
<210> 385
   <211> 71
   <212> DNA
   <213> Homo sapiens
<220>
   <223> J Donor_Long_TRAJ7
<400> 385
   ggtctcgaac aacagactcg cttttgggaa ggggaaccaa gtggtggtca taccaaatat 60
   ccagtgagac c 71
<210> 386
   <211> 71
   <212> DNA
   <213> Homo sapiens
<220>
   <223> J Donor_Long_TRAJ8
<400> 386
   ggtctcgttt cagaaacttg tatttggaac tggcacccga cttctggtca gtccaaatat 60
   ccagtgagac c 71
<210> 387
   <211> 71
   <212> DNA
   <213> Homo sapiens
<220>
   <223> J Donor_Long_TRAJ9
<400> 387
   ggtctcgggc ttcaaaacta tctttggagc aggaacaaga ctatttgtta aagcaaatat 60
   ccagtgagac c 71
<210> 388
   <211> 74
   <212> DNA
   <213> Homo sapiens
<220>
   <223> J Donor_Long_TRAJ10
<400> 388
   ggtctcggga ggaaacaaac tcacctttgg gacaggcact cagctaaaag tggaactcaa 60
   tatccagtga gacc 74
<210> 389
   <211> 71
   <212> DNA
   <213> Homo sapiens
<220>
   <223> J Donor_Long_TRAJ11
<400> 389
   ggtctcgtac agcaccctca cctttgggaa ggggactatg cttctagtct ctccagatat 60
   ccagtgagac c 71
<210> 390
   <211> 71
   <212> DNA
   <213> Homo sapiens
<220>
   <223> J Donor_Long_TRAJ12
<400> 390
   ggtctcgagt tataaattga tctttgggag tgggaccaga ctgctggtca ggcctgatat 60
   ccagtgagac c 71
<210> 391
   <211> 74
   <212> DNA
   <213> Homo sapiens
<220>
   <223> J Donor_Long_TRAJ13
<400> 391
   ggtctcgggt taccagaaag ttacctttgg aattggaaca aagctccaag tcatcccaaa 60
   tatccagtga gacc 74
<210> 392
   <211> 62
   <212> DNA
   <213> Homo sapiens
<220>
   <223> J Donor_Long_TRAJ14
<400> 392
   ggtctcgttc atctttggga gtgggacaag attatcagta aaacctgata tccagtgaga 60
   cc 62
<210> 393
   <211> 71
   <212> DNA
   <213> Homo sapiens
<220>
   <223> J Donor_Long_TRAJ15
<400> 393
   ggtctcggga actgctctga tctttgggaa gggaacccac ctatcagtga gttccaatat 60
   ccagtgagac c 71
<210> 394
   <211> 71
   <212> DNA
   <213> Homo sapiens
<220>
   <223> J Donor_Long_TRAJ16
<400> 394
   ggtctcggga cagaagctgc tctttgcaag gggaaccatg ttaaaggtgg atcttaatat 60
   ccagtgagac c 71
<210> 395
   <211> 74
   <212> DNA
   <213> Homo sapiens
<220>
   <223> J Donor_Long_TRAJ17
<400> 395
   ggtctcggca ggcaacaagc taacttttgg aggaggaacc agggtgctag ttaaaccaaa 60
   tatccagtga gacc 74
<210> 396
   <211> 77
   <212> DNA
   <213> Homo sapiens
<220>
   <223> J Donor_Long_TRAJ18
<400> 396
   ggtctcgtca accctgggga ggctatactt tggaagagga actcagttga ctgtctggcc      60
   tgatatccag tgagacc                                                     77
<210> 397
   <211> 71
   <212> DNA
   <213> Homo sapiens
<220>
   <223> J Donor_Long_TRAJ19
<400> 397
   ggtctcgttc tacaatttca cctttggaaa gggatccaaa cataatgtca ctccaaatat      60
   ccagtgagac c                                                           71
<210> 398
   <211> 68
   <212> DNA
   <213> Homo sapiens
<220>
   <223> J Donor_Long_TRAJ20
<400> 398
   ggtctcgtac aagctcagct ttggagccgg aaccacagta actgtaagag caaatatcca      60
   gtgagacc                                                               68
<210> 399
   <211> 65
   <212> DNA
   <213> Homo sapiens
<220>
   <223> J Donor_Long_TRAJ21
<400> 399
   ggtctcgaaa ttttactttg gatctgggac caaactcaat gtaaaaccaa atatccagtg 60
   agacc 65
<210> 400
   <211> 74
   <212> DNA
   <213> Homo sapiens
<220>
   <223> J Donor_Long_TRAJ22
<400> 400
   ggtctcgtct gcaaggcaac tgacctttgg atctgggaca caattgactg ttttacctga 60
   tatccagtga gacc 74
<210> 401
   <211> 74
   <212> DNA
   <213> Homo sapiens
<220>
   <223> J Donor_Long_TRAJ23
<400> 401
   ggtctcgcag ggaggaaagc ttatctttgg acagggaacg gagttatctg tgaaacccaa 60
   tatccagtga gacc 74
<210> 402
   <211> 74
   <212> DNA
   <213> Homo sapiens
<220>
   <223> J Donor_Long_TRAJ24
<400> 402
   ggtctcgagt tggggtaaat tgcagtttgg agcagggacc caggttgtgg tcaccccaga 60
   tatccagtga gacc 74
<210> 403
   <211> 71
   <212> DNA
   <213> Homo sapiens
<220>
   <223> J Donor_Long_TRAJ25
<400> 403
   ggtctcgggc ttctccttta tctttgggaa ggggacaagg ctgcttgtca agccaaatat 60
   ccagtgagac c 71
<210> **404**
   <211> 71
   <212> DNA
   <213> Homo sapiens
<220>
   <223> J Donor_Long_TRAJ26
<400> 404
   ggtctcgggt cagaattttg tctttggtcc cggaaccaga ttgtccgtgc tgccctatat 60
   ccagtgagac c 71
<210> 405
   <211> 71
   <212> DNA
   <213> Homo sapiens
<220>
   <223> J Donor_Long_TRAJ27
<400> 405
   ggtctcggca ggcaaatcaa cctttgggga tgggactacg ctcactgtga agccaaatat 60
   ccagtgagac c 71
<210> 406
   <211> 77
   <212> DNA
   <213> Homo sapiens
<220>
   <223> J Donor_Long_TRAJ28
<400> 406
   ggtctcggct gggagttacc aactcacttt tgggaagggg accaaactct cggtcatacc 60
   aaatatccag tgagacc 77
<210> 407
   <211> 71
   <212> DNA
   <213> Homo sapiens
<220>
   <223> J Donor_Long_TRAJ29
<400> 407
   ggtctcgaac acacctcttg tctttggaaa gggcacaaga ctttctgtga ttgcaaatat      60
   ccagtgagac c                                                           71
<210> 408
   <211> 68
   <212> DNA
   <213> Homo sapiens
<220>
   <223> J Donor_Long_TRAJ30
<400> 408
   ggtctcggac aagatcatct ttggaaaagg gacacgactt catattctcc ccaatatcca      60
   gtgagacc                                                               68
<210> 409
   <211> 68
   <212> DNA
   <213> Homo sapiens
<220>
   <223> J Donor_Long_TRAJ31
<400> 409
   ggtctcggcc agactcatgt ttggagatgg aactcagctg gtggtgaagc ccaatatcca      60
   gtgagacc                                                               68
<210> 410
   <211> 77
   <212> DNA
   <213> Homo sapiens
<220>
   <223> J Donor_Long_TRAJ32
<400> 410
   ggtctcgggt gctacaaaca agctcatctt tggaactggc actctgcttg ctgtccagcc      60
   aaatatccag tgagacc                                                     77
<210> 411
   <211> 68
   <212> DNA
   <213> Homo sapiens
<220>
   <223> J Donor_Long_TRAJ33
<400> 411
   ggtctcgtat cagttaatct ggggcgctgg gaccaagcta attataaagc cagatatcca 60
   gtgagacc 68
<210> 412
   <211> 68
   <212> DNA
   <213> Homo sapiens
<220>
   <223> J Donor_Long_TRAJ34
<400> 412
   ggtctcggac aagctcatct ttgggactgg gaccagatta caagtctttc caaatatcca 60
   gtgagacc 68
<210> 413
   <211> 71
   <212> DNA
   <213> Homo sapiens
<220>
   <223> J Donor_Long_TRAJ36
<400> 413
   ggtctcggca aacaacctct tctttgggac tggaacgaga ctcaccgtta ttccctatat 60
   ccagtgagac c 71
<210> 414
   <211> 74
   <212> DNA
   <213> Homo sapiens
<220>
   <223> J Donor_Long_TRAJ37
<400> 414
   ggtctcgaac acaggcaaac taatctttgg gcaagggaca actttacaag taaaaccaga 60
   tatccagtga gacc 74
<210> 415
   <211> 74
   <212> DNA
   <213> Homo sapiens
<220>
   <223> J Donor_Long_TRAJ38
<400> 415
   ggtctcgaac aaccgtaagc tgatttgggg attgggaaca agcctggcag taaatccgaa 60
   tatccagtga gacc 74
<210> 416
   <211> 74
   <212> DNA
   <213> Homo sapiens
<220>
   <223> J Donor_Long_TRAJ39
<400> 416
   ggtctcggca ggcaacatgc tcacctttgg aggaggaaca aggttaatgg tcaaacccca 60
   tatccagtga gacc 74
<210> 417
   <211> 71
   <212> DNA
   <213> Homo sapiens
<220>
   <223> J Donor_Long_TRAJ40
<400> 417
   ggtctcgacc tacaaataca tctttggaac aggcaccagg ctgaaggttt tagcaaatat 60
   ccagtgagac c 71
<210> 418
   <211> 74
   <212> DNA
   <213> Homo sapiens
<220>
   <223> J Donor_Long_TRAJ41
<400> 418
   ggtctcgtcc gggtatgcac tcaactttgg caaaggcacc tcgctgttgg tcacacccca 60
   tatccagtga gacc 74
<210> 419
   <211> 77
   <212> DNA
   <213> Homo sapiens
<220>
   <223> J Donor_Long_TRAJ42
<400> 419
   ggtctcggga agccaaggaa atctcatctt tggaaaaggc actaaactct ctgttaaacc 60
   aaatatccag tgagacc 77
<210> 420
   <211> 65
   <212> DNA
   <213> Homo sapiens
<220>
   <223> J Donor_Long_TRAJ43
<400> 420
   ggtctcggac atgcgctttg gagcagggac cagactgaca gtaaaaccaa atatccagtg 60
   agacc 65
<210> 421
   <211> 74
   <212> DNA
   <213> Homo sapiens
<220>
   <223> J Donor_Long_TRAJ44
<400> 421
   ggtctcgact gccagtaaac tcacctttgg gactggaaca agacttcagg tcacgctcga 60
   tatccagtga gacc 74
<210> 422
   <211> 77
   <212> DNA
   <213> Homo sapiens
<220>
   <223> J Donor_Long_TRAJ45
<400> 422
   ggtctcggga ggtgctgacg gactcacctt tggcaaaggg actcatctaa tcatccagcc 60
   ctatatccag tgagacc 77
<210> 423
   <211> 74
   <212> DNA
   <213> Homo sapiens
<220>
   <223> J Donor_Long_TRAJ46
<400> 423
   ggtctcgagc ggagacaagc tgacttttgg gaccgggact cgtttagcag ttaggcccaa 60
   tatccagtga gacc 74
<210> 424
   <211> 68
   <212> DNA
   <213> Homo sapiens
<220>
   <223> J Donor_Long_TRAJ47
<400> 424
   ggtctcgaac aaactggtct ttggcgcagg aaccattctg agagtcaagt cctatatcca 60
   gtgagacc 68
<210> 425
   <211> 74
   <212> DNA
   <213> Homo sapiens
<220>
   <223> J Donor_Long_TRAJ48
<400> 425
   ggtctcggga aatgagaaat taacctttgg gactggaaca agactcacca tcatacccaa 60
   tatccagtga gacc 74
<210> 426
   <211> 68
   <212> DNA
   <213> Homo sapiens
<220>
   <223> J Donor_Long_TRAJ49
<400> 426
   ggtctcgaac cagttctatt ttgggacagg gacaagtttg acggtcattc caaatatcca 60
   gtgagacc 68
<210> 427
   <211> 71
   <212> DNA
   <213> Homo sapiens
<220>
   <223> J Donor_Long_TRAJ50
<400> 427
   ggtctcgtac gacaaggtga tatttgggcc agggacaagc ttatcagtca ttccaaatat 60
   ccagtgagac c 71
<210> 428
   <211> 80
   <212> DNA
   <213> Homo sapiens
<220>
   <223> J Donor_Long_TRAJ52
<400> 428
   ggtctcgggt actagctatg gaaagctgac atttggacaa gggaccatct tgactgtcca 60
   tccaaatatc cagtgagacc 80
<210> 429
   <211> 77
   <212> DNA
   <213> Homo sapiens
<220>
   <223> J Donor_Long_TRAJ53
<400> 429
   ggtctcgggt agcaactata aactgacatt tggaaaagga actctcttaa ccgtgaatcc 60
   aaatatccag tgagacc 77
<210> 430
   <211> 71
   <212> DNA
   <213> Homo sapiens
<220>
   <223> J Donor_Long_TRAJ54
<400> 430
   ggtctcggcc cagaagctgg tatttggcca aggaaccagg ctgactatca acccaaatat 60
   ccagtgagac c 71
<210> 431
   <211> 74
   <212> DNA
   <213> Homo sapiens
<220>
   <223> J Donor_Long_TRAJ56
<400> 431
   ggtctcggcc aatagtaagc tgacatttgg aaaaggaata actctgagtg ttagaccaga 60
   tatccagtga gacc 74
<210> 432
   <211> 74
   <212> DNA
   <213> Homo sapiens
<220>
   <223> J Donor_Long_TRAJ57
<400> 432
   ggtctcggga tctgaaaagc tggtctttgg aaagggaacg aaactgacag taaacccata 60
   tatccagtga gacc 74
<210> 433
   <211> 65
   <212> DNA
   <213> Homo sapiens
<220>
   <223> J Donor_Long_TRAJ59
<400> 433
   ggtctcgaac aggaaattta catttggaat ggggacgcaa gtgagagtga atatccagtg 60
   agacc 65
<210> 434
   <211> 71
   <212> DNA
   <213> Homo sapiens
<220>
   <223> J Donor_Long_TRAJ61
<400> 434
   ggtctcgaat aggaaactga catttggagc caacactaga ggaatcatga aactcaatat 60
   ccagtgagac c 71
<210> 435
   <211> 414
   <212> DNA
   <213> Homo sapiens
<220>
   <223> TRBV2_BB_1
<400> 435
   gttcgaagct ggcatgacac gaagacttgt acgccaccat ggatacctgg ctcgtatgct      60
   gggcaatttt tagtctcttg aaagcaggac tcacagaacc tgaagtcacc cagactccca     120
   gccatcaggt cacacagatg ggacaggaag tgatcttgcg ctgtgtcccc atctctaatc     180
   acttatactt ctattggtac agacaaatct tggggcagaa agtcgagttt ctggtttcct     240
   tttataataa tgaaatctca gagaagtctg aaatattcga tgatcaattc tcagttgaaa     300
   ggcctgatgg atcaaatttc actctgaaga tccggtccac aaagctggag gactcagcca     360
   tgtacttttg cagagacctt gcggccgtgt cttccgacgc tgacagtgta gata          414
<210> 436
   <211> 411
   <212> DNA
   <213> Homo sapiens
<220>
   <223> TRBV3-1_BB_1
<400> 436
   gttcgaagct ggcatgacac gaagacttgt acgccaccat gggctgcagg ctcctctgct      60
   gtgtggtctt ttgcctcctc caagcaggtc ccttggacac agctgtttcc cagactccaa     120
   aatacctggt cacacagatg ggaaacgaca agtccattaa atgtgaacaa aatctgggcc     180
   atgatactat gtattggtat aaacaggact ctaagaaatt tctgaagata atgtttagct     240
   acaataataa ggagctcatt ataaatgaaa cagttccaaa tcgcttctca cctaaatctc     300
   cagacaaagc tcacttaaat cttcacatca attccctgga gcttggtgac tctgctgtgt     360
   atttttgcag agaccttgcg gccgtgtctt ccgacgctga cagtgtagat a              411
<210> 437
   <211> 411
   <212> DNA
   <213> Homo sapiens
<220>
   <223> TRBV4-1_BB_1
<400> 437
   gttcgaagct ggcatgacac gaagacttgt acgccaccat gggctgcagg ctgctctgct      60
   gtgcggttct ctgtctcctg ggagcagttc ccatagacac tgaagttacc cagacaccaa     120
   aacacctggt catgggaatg acaaataaga agtctttgaa atgtgaacaa catatggggc     180
   acagggctat gtattggtac aagcagaaag ctaagaagcc accggagctc atgtttgtct     240
   acagctatga gaaactctct ataaatgaaa gtgtgccaag tcgcttctca cctgaatgcc     300
   ccaacagctc tctcttaaac cttcacctac acgccctgca gccagaagat tcagccctgt     360
   atctttgcag agaccttgcg gccgtgtctt ccgacgctga cagtgtagat a              411
<210> 438
   <211> 411
   <212> DNA
   <213> Homo sapiens
<220>
   <223> TRBV4-2_BB_1
<400> 438
   gttcgaagct ggcatgacac gaagacttgt acgccaccat gggctgcagg ctgctctgct      60
   gtgcggttct ctgtctcctg ggagcggtcc ccatggaaac gggagttacg cagacaccaa     120
   gacacctggt catgggaatg acaaataaga agtctttgaa atgtgaacaa catctggggc     180
   ataacgctat gtattggtac aagcaaagtg ctaagaagcc actggagctc atgtttgtct     240
   acaactttaa agaacagact gaaaacaaca gtgtgccaag tcgcttctca cctgaatgcc     300
   ccaacagctc tcacttattc cttcacctac acaccctgca gccagaagat tcggccctgt     360
   atctttgcag agaccttgcg gccgtgtctt ccgacgctga cagtgtagat a              411
<210> 439
   <211> 411
   <212> DNA
   <213> Homo sapiens
<220>
   <223> TRBV4-3_BB_1
<400> 439
   gttcgaagct ggcatgacac gaagacttgt acgccaccat gggctgcagg ctgctctgct 60
   gtgcggttct ctgtctcctg ggagcggtcc ccatggaaac gggagttacg cagacaccaa 120
   gacacctggt catgggaatg acaaataaga agtctttgaa atgtgaacaa catctgggtc 180
   ataacgctat gtattggtac aagcaaagtg ctaagaagcc actggagctc atgtttgtct 240
   acagtcttga agaacgggtt gaaaacaaca gtgtgccaag tcgcttctca cctgaatgcc 300
   ccaacagctc tcacttattc cttcacctac acaccctgca gccagaagat tcggccctgt 360
   atctttgcag agaccttgcg gccgtgtctt ccgacgctga cagtgtagat a 411
<210> 440
   <211> 411
   <212> DNA
   <213> Homo sapiens
<220>
   <223> TRBV5-1_BB_1
<400> 440
   gttcgaagct ggcatgacac gaagacttgt acgccaccat gggctccagg ctgctctgtt 60
   gggtgctgct ttgtctcctg ggagcaggcc cagtaaaggc tggagtcact caaactccaa 120
   gatatctgat caaaacgaga ggacagcaag tgacactgag ctgctcccct atctctgggc 180
   ataggagtgt atcctggtac caacagaccc caggacaggg ccttcagttc ctctttgaat 240
   acttcagtga gacacagaga aacaaaggaa acttccctgg tcgattctca gggcgccagt 300
   tctctaactc tcgctctgag atgaatgtga gcaccttgga gctgggggac tcggcccttt 360
   atctttgcag agaccttgcg gccgtgtctt ccgacgctga cagtgtagat a 411
<210> 441
   <211> 411
   <212> DNA
   <213> Homo sapiens
<220>
   <223> TRBV5-4_BB_1
<400> 441
   gttcgaagct ggcatgacac gaagacttgt acgccaccat gggccctggg ctcctctgct 60
   gggtgctgct ttgtctcctg ggagcaggct cagtggagac tggagtcacc caaagtccca 120
   cacacctgat caaaacgaga ggacagcaag tgactctgag atgctcttct cagtctgggc 180
   acaacactgt gtcctggtac caacaggccc tgggtcaggg gccccagttt atctttcagt 240
   attataggga ggaagagaat ggcagaggaa acttccctcc tagattctca ggactccagt 300
   tccctaatta tagctctgag ctgaatgtga acgccttgga gctggacgac tcggccctgt 360
   atctttgcag agaccttgcg gccgtgtctt ccgacgctga cagtgtagat a 411
<210> 442
   <211> 411
   <212> DNA
   <213> Homo sapiens
<220>
   <223> TRBV5-5_BB_1
<400> 442
   gttcgaagct ggcatgacac gaagacttgt acgccaccat gggccctggg ctcctctgct 60
   gggtgctgct ttgtctcctg ggagcaggcc cagtggacgc tggagtcacc caaagtccca 120
   cacacctgat caaaacgaga ggacagcaag tgactctgag atgctctcct atctctgggc 180
   acaagagtgt gtcctggtac caacaggtcc tgggtcaggg gccccagttt atctttcagt 240
   attatgagaa agaagagaga ggaagaggaa acttccctga tcgattctca gctcgccagt 300
   tccctaacta tagctctgag ctgaatgtga acgccttgtt gctgggggac tcggccctgt 360
   atctttgcag agaccttgcg gccgtgtctt ccgacgctga cagtgtagat a 411
<210> 443
   <211> 411
   <212> DNA
   <213> Homo sapiens
<220>
   <223> TRBV5-6_BB_1
<400> 443
   gttcgaagct ggcatgacac gaagacttgt acgccaccat gggccccggg ctcctctgct 60
   gggcactgct ttgtctcctg ggagcaggct tagtggacgc tggagtcacc caaagtccca 120
   cacacctgat caaaacgaga ggacagcaag tgactctgag atgctctcct aagtctgggc 180
   atgacactgt gtcctggtac caacaggccc tgggtcaggg gccccagttt atctttcagt 240
   attatgagga ggaagagaga cagagaggca acttccctga tcgattctca ggtcaccagt 300
   tccctaacta tagctctgag ctgaatgtga acgccttgtt gctgggggac tcggccctct 360
   atctttgcag agaccttgcg gccgtgtctt ccgacgctga cagtgtagat a 411
<210> 444
   <211> 411
   <212> DNA
   <213> Homo sapiens
<220>
   <223> TRBV5-7_BB_1
<400> 444
   gttcgaagct ggcatgacac gaagacttgt acgccaccat gggccccggg ctcctctgct      60
   gggtgctgct ttgtccccta ggagaaggcc cagtggacgc tggagtcacc caaagtccca     120
   cacacctgat caaaacgaga ggacagcacg tgactctgag atgctctcct atctctgggc     180
   acaccagtgt gtcctcgtac caacaggccc tgggtcaggg gccccagttt atctttcagt     240
   attatgagaa agaagagaga ggaagaggaa acttccctga tcaattctca ggtcaccagt     300
   tccctaacta tagctctgag ctgaatgtga acgccttgtt gctaggggac tcggccctct     360
   atctttgcag agaccttgcg gccgtgtctt ccgacgctga cagtgtagat a              411
<210> 445
   <211> 411
   <212> DNA
   <213> Homo sapiens
<220>
   <223> TRBV5-8_BB_1
<400> 445
   gttcgaagct ggcatgacac gaagacttgt acgccaccat gggacccagg ctcctcttct      60
   gggcactgct ttgtctcctc ggaacaggcc cagtggaggc tggagtcaca caaagtccca     120
   cacacctgat caaaacgaga ggacagcaag cgactctgag atgctctcct atctctgggc     180
   acaccagtgt gtactggtac caacaggccc tgggtctggg cctccagttc ctcctttggt     240
   atgacgaggg tgaagagaga aacagaggaa acttccctcc tagattttca ggtcgccagt     300
   tccctaatta tagctctgag ctgaatgtga acgccttgga gctggaggac tcggccctgt     360
   atctttgcag agaccttgcg gccgtgtctt ccgacgctga cagtgtagat a              411
<210> 446
   <211> 411
   <212> DNA
   <213> Homo sapiens
<220>
   <223> TRBV6-1_BB_1
<400> 446
   gttcgaagct ggcatgacac gaagacttgt acgccaccat gagcatcggg ctcctgtgct      60
   gtgtggcctt ttctctcctg tgggcaagtc cagtgaatgc tggtgtcact cagaccccaa     120
   aattccaggt cctgaaaaca ggacagagca tgacactgca gtgtgcccag gatatgaacc     180
   ataactccat gtactggtat cgacaagacc caggcatggg actgaggctg atttattact     240
   cagcttctga gggtaccact gacaaaggag aagtccccaa tggctacaat gtctccagat     300
   taaacaaacg ggagttctcg ctcaggctgg agtcggctgc tccctcccag acatctgtgt     360
   acttttgcag agaccttgcg gccgtgtctt ccgacgctga cagtgtagat a              411
<210> 447
   <211> 411
   <212> DNA
   <213> Homo sapiens
<220>
   <223> TRBV6-3_BB_1
<400> 447
   gttcgaagct ggcatgacac gaagacttgt acgccaccat gagcctcggg ctcctgtgct      60
   gtggggtctt ttctctcctg tgggcaggtc cagtgaatgc tggtgtcact cagaccccaa     120
   aattccgggt cctgaaaaca ggacagagca tgacactgct gtgtgcccag gatatgaacc     180
   atgaatacat gtactggtat cgacaagacc caggcatggg gctgaggctg attcattact     240
   cagttggtga gggtacaact gccaaaggag aggtccctga tggctacaat gtctccagat     300
   taaaaaaaca gaatttcctg ctggggttgg agtcggctgc tccctcccaa acatctgtgt     360
   acttttgcag agaccttgcg gccgtgtctt ccgacgctga cagtgtagat a              411
<210> 448
   <211> 411
   <212> DNA
   <213> Homo sapiens
<220>
   <223> TRBV6-4_BB_1
<400> 448
   gttcgaagct ggcatgacac gaagacttgt acgccaccat gagaatcagg ctcctgtgct 60
   gtgtggcctt ttctctcctg tgggcaggtc cagtgattgc tgggatcacc caggcaccaa 120
   catctcagat cctggcagca ggacggcgca tgacactgag atgtacccag gatatgagac 180
   ataatgccat gtactggtat agacaagatc taggactggg gctaaggctc atccattatt 240
   caaatactgc aggtaccact ggcaaaggag aagtccctga tggttatagt gtctccagag 300
   caaacacaga tgatttcccc ctcacgttgg cgtctgctgt accctctcag acatctgtgt 360
   acttttgcag agaccttgcg gccgtgtctt ccgacgctga cagtgtagat a 411
<210> 449
   <211> 411
   <212> DNA
   <213> Homo sapiens
<220>
   <223> TRBV6-5_BB_1
<400> 449
   gttcgaagct ggcatgacac gaagacttgt acgccaccat gagcatcggc ctcctgtgct 60
   gtgcagcctt gtctctcctg tgggcaggtc cagtgaatgc tggtgtcact cagaccccaa 120
   aattccaggt cctgaaaaca ggacagagca tgacactgca gtgtgcccag gatatgaacc 180
   atgaatacat gtcctggtat cgacaagacc caggcatggg gctgaggctg attcattact 240
   cagttggtgc tggtatcact gaccaaggag aagtccccaa tggctacaat gtctccagat 300
   caaccacaga ggatttcccg ctcaggctgc tgtcggctgc tccctcccag acatctgtgt 360
   acttttgcag agaccttgcg gccgtgtctt ccgacgctga cagtgtagat a 411
<210> 450
   <211> 411
   <212> DNA
   <213> Homo sapiens
<220>
   <223> TRBV6-6_BB_1
<400> 450
   gttcgaagct ggcatgacac gaagacttgt acgccaccat gagcatcagc ctcctgtgct 60
   gtgcagcctt tcctctcctg tgggcaggtc cagtgaatgc tggtgtcact cagaccccaa 120
   aattccgcat cctgaagata ggacagagca tgacactgca gtgtacccag gatatgaacc 180
   ataactacat gtactggtat cgacaagacc caggcatggg gctgaagctg atttattatt 240
   cagttggtgc tggtatcact gataaaggag aagtcccgaa tggctacaac gtctccagat 300
   caaccacaga ggatttcccg ctcaggctgg agttggctgc tccctcccag acatctgtgt 360
   acttttgcag agaccttgcg gccgtgtctt ccgacgctga cagtgtagat a 411
<210> 451
   <211> 408
   <212> DNA
   <213> Homo sapiens
<220>
   <223> TRBV6-8_BB_1
<400> 451
   gttcgaagct ggcatgacac gaagacttgt acgccaccat gagcctcggg ctcctgtgct 60
   gtgcggcctt ttctctcctg tgggcaggtc ccgtgaatgc tggtgtcact cagaccccaa 120
   aattccacat cctgaaaaca ggacagagca tgacactgca gtgtgcccag gatatgaacc 180
   atggatacat gtcctggtat cgacaagacc caggcatggg gctgagactg atttactact 240
   cagctgctgc tggtactact gacaaagaag tccccaatgg ctacaatgtc tctagattaa 300
   acacagagga tttcccactc aggctggtgt cggctgctcc ctcccagaca tctgtgtacc 360
   tttgcagaga ccttgcggcc gtgtcttccg acgctgacag tgtagata 408
<210> 452
   <211> 411
   <212> DNA
   <213> Homo sapiens
<220>
   <223> TRBV6-9_BB_1
<400> 452
   gttcgaagct ggcatgacac gaagacttgt acgccaccat gagcatcggg ctcctgtgct      60
   gtgtggcctt ttctctcctg tgggcaggtc cagtgaatgc tggtgtcact cagaccccaa     120
   aattccacat cctgaaaaca ggacagagca tgacactgca gtgtgcccag gatatgaacc     180
   atggatactt gtcctggtat cgacaagacc caggcatggg gctgaggcgc attcattact     240
   cagttgctgc tggtatcact gacaaaggag aagtccccga tggctacaat gtatccagat     300
   caaacacaga ggatttcccg ctcaggctgg agtcagctgc tccctcccag acatctgtat     360
   acttttgcag agaccttgcg gccgtgtctt ccgacgctga cagtgtagat a              411
<210> 453
   <211> 414
   <212> DNA
   <213> Homo sapiens
<220>
   <223> TRBV7-2_BB_1
<400> 453
   gttcgaagct ggcatgacac gaagacttgt acgccaccat gggcaccagg ctcctcttct      60
   gggtggcctt ctgtctcctg ggggcagatc acacaggagc tggagtctcc cagtccccca     120
   gtaacaaggt cacagagaag ggaaaggatg tagagctcag gtgtgatcca atttcaggtc     180
   atactgccct ttactggtac cgacagagcc tggggcaggg cctggagttt ttaatttact     240
   tccaaggcaa cagtgcacca gacaaatcag ggctgcccag tgatcgcttc tctgcagaga     300
   ggactggggg atccgtctcc actctgacga tccagcgcac acagcaggag gactcggccg     360
   tgtatctttg cagagacctt gcggccgtgt cttccgacgc tgacagtgta gata           414
<210> 454
   <211> 414
   <212> DNA
   <213> Homo sapiens
<220>
   <223> TRBV7-3_BB_1
<400> 454
   gttcgaagct ggcatgacac gaagacttgt acgccaccat gggcaccagg ctcctctgct      60
   gggcagccct gtgcctcctg ggggcagatc acacaggtgc tggagtctcc cagaccccca     120
   gtaacaaggt cacagagaag ggaaaatatg tagagctcag gtgtgatcca atttcaggtc     180
   atactgccct ttactggtac cgacaaagcc tggggcaggg cccagagttt ctaatttact     240
   tccaaggcac gggtgcggca gatgactcag ggctgcccaa cgatcggttc tttgcagtca     300
   ggcctgaggg atccgtctct actctgaaga tccagcgcac agagcggggg gactcagccg 360
   tgtatctttg cagagacctt gcggccgtgt cttccgacgc tgacagtgta gata 414
<210> 455
   <211> 414
   <212> DNA
   <213> Homo sapiens
<220>
   <223> TRBV7-6_BB_1
<400> 455
   gttcgaagct ggcatgacac gaagacttgt acgccaccat gggcaccagt ctcctatgct 60
   gggtggtcct gggtttccta gggacagatc acacaggtgc tggagtctcc cagtctccca 120
   ggtacaaagt cacaaagagg ggacaggatg tagctctcag gtgtgatcca atttcgggtc 180
   atgtatccct ttattggtac cgacaggccc tggggcaggg cccagagttt ctgacttact 240
   tcaattatga agcccaacaa gacaaatcag ggctgcccaa tgatcggttc tctgcagaga 300
   ggcctgaggg atccatctcc actctgacga tccagcgcac agagcagcgg gactcggcca 360
   tgtatcgttg cagagacctt gcggccgtgt cttccgacgc tgacagtgta gata 414
<210> 456
   <211> **414**
   <212> DNA
   <213> Homo sapiens
<220>
   <223> TRBV7-7_BB_1
<400> 456
   gttcgaagct ggcatgacac gaagacttgt acgccaccat gggtaccagt ctcctatgct 60
   gggtggtcct gggtttccta gggacagatc acacaggtgc tggagtctcc cagtctccca 120
   ggtacaaagt cacaaagagg ggacaggatg taactctcag gtgtgatcca atttcgagtc 180
   atgcaaccct ttattggtat caacaggccc tggggcaggg cccagagttt ctgacttact 240
   tcaattatga agctcaacca gacaaatcag ggctgcccag tgatcggttc tctgcagaga 300
   ggcctgaggg atccatctcc actctgacga ttcagcgcac agagcagcgg gactcagcca 360
   tgtatcgttg cagagacctt gcggccgtgt cttccgacgc tgacagtgta gata 414
<210> 457
   <211> 414
   <212> DNA
   <213> Homo sapiens
<220>
   <223> TRBV7-8_BB_1
<400> 457
   gttcgaagct ggcatgacac gaagacttgt acgccaccat gggcaccagg ctcctctgct      60
   gggtggtcct gggtttccta gggacagatc acacaggtgc tggagtctcc cagtccccta     120
   ggtacaaagt cgcaaagaga ggacaggatg tagctctcag gtgtgatcca atttcgggtc     180
   atgtatccct tttttggtac caacaggccc tggggcaggg gccagagttt ctgacttatt     240
   tccagaatga agctcaacta gacaaatcgg ggctgcccag tgatcgcttc tttgcagaaa     300
   ggcctgaggg atccgtctcc actctgaaga tccagcgcac acagcaggag gactccgccg     360
   tgtatctttg cagagacctt gcggccgtgt cttccgacgc tgacagtgta gata           414
<210> 458
   <211> 414
   <212> DNA
   <213> Homo sapiens
<220>
   <223> TRBV7-9_BB_1
<400> 458
   gttcgaagct ggcatgacac gaagacttgt acgccaccat gggcaccagc ctcctctgct      60
   ggatggccct gtgtctcctg ggggcagatc acgcagatac tggagtctcc cagaacccca     120
   gacacaagat cacaaagagg ggacagaatg taactttcag gtgtgatcca atttctgaac     180
   acaaccgcct ttattggtac cgacagaccc tggggcaggg cccagagttt ctgacttact     240
   tccagaatga agctcaacta gaaaaatcaa ggctgctcag tgatcggttc tctgcagaga     300
   ggcctaaggg atctttctcc accttggaga tccagcgcac agagcagggg gactcggcca     360
   tgtatctttg cagagacctt gcggccgtgt cttccgacgc tgacagtgta gata           414
<210> 459
   <211> 411
   <212> DNA
   <213> Homo sapiens
<220>
   <223> TRBV9_BB_1
<400> 459
   gttcgaagct ggcatgacac gaagacttgt acgccaccat gggcttcagg ctcctctgct 60
   gtgtggcctt ttgtctcctg ggagcaggcc cagtggattc tggagtcaca caaaccccaa 120
   agcacctgat cacagcaact ggacagcgag tgacgctgag atgctcccct aggtctggtg 180
   acctctctgt gtactggtac caacagagcc tggaccaggg cctccagttc ctcattcagt 240
   attataatgg agaagagaga gcaaaaggaa acattcttga acgattctcc gcacaacagt 300
   tccctgactt gcactctgaa ctaaacctga gctctctgga gctgggggac tcagctttgt 360
   acttttgcag agaccttgcg gccgtgtctt ccgacgctga cagtgtagat a 411
<210> 460
   <211> 411
   <212> DNA
   <213> Homo sapiens
<220>
   <223> TRBV10-1_BB_1
<400> 460
   gttcgaagct ggcatgacac gaagacttgt acgccaccat gggcacgagg ctcttcttct 60
   atgtggccct ttgtctgctg tgggcaggac acagggatgc tgaaatcacc cagagcccaa 120
   gacacaagat cacagagaca ggaaggcagg tgaccttggc gtgtcaccag acttggaacc 180
   acaacaatat gttctggtat cgacaagacc tgggacatgg gctgaggctg atccattact 240
   catatggtgt tcaagacact aacaaaggag aagtctcaga tggctacagt gtctctagat 300
   caaacacaga ggacctcccc ctcactctgg agtctgctgc ctcctcccag acatctgtat 360
   acttttgcag agaccttgcg gccgtgtctt ccgacgctga cagtgtagat a 411
<210> 461
   <211> 411
   <212> DNA
   <213> Homo sapiens
<220>
   <223> TRBV10-2_BB_1
<400> 461
   gttcgaagct ggcatgacac gaagacttgt acgccaccat gggcaccagg ctcttcttct      60
   atgtggccct ttgtctgctg tgggcaggac acagggatgc tggaatcacc cagagcccaa     120
   gatacaagat cacagagaca ggaaggcagg tgaccttgat gtgtcaccag acttggagcc     180
   acagctatat gttctggtat cgacaagacc tgggacatgg gctgaggctg atctattact     240
   cagcagctgc tgatattaca gataaaggag aagtccccga tggctatgtt gtctccagat     300
   ccaagacaga gaatttcccc ctcactctgg agtcagctac ccgctcccag acatctgtgt     360
   acttttgcag agaccttgcg gccgtgtctt ccgacgctga cagtgtagat a              411
<210> 462
   <211> 411
   <212> DNA
   <213> Homo sapiens
<220>
   <223> TRBV10-3_BB_1
<400> 462
   gttcgaagct ggcatgacac gaagacttgt acgccaccat gggcacaagg ttgttcttct      60
   atgtggccct ttgtctcctg tggacaggac acatggatgc tggaatcacc cagagcccaa     120
   gacacaaggt cacagagaca ggaacaccag tgactctgag atgtcaccag actgagaacc     180
   accgctatat gtactggtat cgacaagacc cggggcatgg gctgaggctg atccattact     240
   catatggtgt taaagatact gacaaaggag aagtctcaga tggctatagt gtctctagat     300
   caaagacaga ggatttcctc ctcactctgg agtccgctac cagctcccag acatctgtgt     360
   acttttgcag agaccttgcg gccgtgtctt ccgacgctga cagtgtagat a              411
<210> 463
   <211> 414
   <212> DNA
   <213> Homo sapiens
<220>
   <223> TRBV11-1_BB_1
<400> 463
   gttcgaagct ggcatgacac gaagacttgt acgccaccat gagcaccagg cttctctgct 60
   ggatggccct ctgtctcctg ggggcagaac tctcagaagc tgaagttgcc cagtccccca 120
   gatataagat tacagagaaa agccaggctg tggctttttg gtgtgatcct atttctggcc 180
   atgctaccct ttactggtac cggcagatcc tgggacaggg cccggagctt ctggttcaat 240
   ttcaggatga gagtgtagta gatgattcac agttgcctaa ggatcgattt tctgcagaga 300
   ggctcaaagg agtagactcc actctcaaga tccagcctgc agagcttggg gactcggcca 360
   tgtatctttg cagagacctt gcggccgtgt cttccgacgc tgacagtgta gata 414
<210> 464
   <211> 414
   <212> DNA
   <213> Homo sapiens
<220>
   <223> TRBV11-2_BB_1
<400> 464
   gttcgaagct ggcatgacac gaagacttgt acgccaccat gggcaccagg ctcctctgct 60
   gggcggccct ctgtctcctg ggagcagaac tcacagaagc tggagttgcc cagtctccca 120
   gatataagat tatagagaaa aggcagagtg tggctttttg gtgcaatcct atatctggcc 180
   atgctaccct ttactggtac cagcagatcc tgggacaggg cccaaagctt ctgattcagt 240
   ttcagaataa cggtgtagtg gatgattcac agttgcctaa ggatcgattt tctgcagaga 300
   ggctcaaagg agtagactcc actctcaaga tccagcctgc aaagcttgag gactcggccg 360
   tgtatctttg cagagacctt gcggccgtgt cttccgacgc tgacagtgta gata 414
<210> 465
   <211> 414
   <212> DNA
   <213> Homo sapiens
<220>
   <223> TRBV11-3_BB_1
<400> 465
   gttcgaagct ggcatgacac gaagacttgt acgccaccat gggtaccagg ctcctctgct 60
   gggtggcctt ctgtctcctg gtggaagaac tcatagaagc tggagtggtt cagtctccca 120
   gatataagat tatagagaaa aaacagcctg tggctttttg gtgcaatcct atttctggcc 180
   acaataccct ttactggtac ctgcagaact tgggacaggg cccggagctt ctgattcgat 240
   atgagaatga ggaagcagta gacgattcac agttgcctaa ggatcgattt tctgcagaga 300
   ggctcaaagg agtagactcc actctcaaga tccagcctgc agagcttggg gactcggccg 360
   tgtatctttg cagagacctt gcggccgtgt cttccgacgc tgacagtgta gata 414
<210> 466
   <211> 414
   <212> DNA
   <213> Homo sapiens
<220>
   <223> TRBV12-3_BB_1
<400> 466
   gttcgaagct ggcatgacac gaagacttgt acgccaccat ggactcctgg accttctgct 60
   gtgtgtccct ttgcatcctg gtagcgaagc atacagatgc tggagttatc cagtcacccc 120
   gccatgaggt gacagagatg ggacaagaag tgactctgag atgtaaacca atttcaggcc 180
   acaactccct tttctggtac agacagacca tgatgcgggg actggagttg ctcatttact 240
   ttaacaacaa cgttccgata gatgattcag ggatgcccga ggatcgattc tcagctaaga 300
   tgcctaatgc atcattctcc actctgaaga tccagccctc agaacccagg gactcagctg 360
   tgtacttttg cagagacctt gcggccgtgt cttccgacgc tgacagtgta gata 414
<210> 467
   <211> **414**
   <212> DNA
   <213> Homo sapiens
<220>
   <223> TRBV12-4_BB_1
<400> 467
   gttcgaagct ggcatgacac gaagacttgt acgccaccat ggactcctgg accctctgct 60
   gtgtgtccct ttgcatcctg gtagcaaagc acacagatgc tggagttatc cagtcacccc 120
   ggcacgaggt gacagagatg ggacaagaag tgactctgag atgtaaacca atttcaggac 180
   acgactacct tttctggtac agacagacca tgatgcgggg actggagttg ctcatttact 240
   ttaacaacaa cgttccgata gatgattcag ggatgcccga ggatcgattc tcagctaaga 300
   tgcctaatgc atcattctcc actctgaaga tccagccctc agaacccagg gactcagctg 360
   tgtacttttg cagagacctt gcggccgtgt cttccgacgc tgacagtgta gata 414
<210> 468
   <211> 414
   <212> DNA
   <213> Homo sapiens
<220>
   <223> TRBV12-5_BB_1
<400> 468
   gttcgaagct ggcatgacac gaagacttgt acgccaccat ggccaccagg ctcctctgct 60
   gtgtggttct ttgtctcctg ggagaagagc ttatagatgc tagagtcacc cagacaccaa 120
   ggcacaaggt gacagagatg ggacaagaag taacaatgag atgtcagcca attttaggcc 180
   acaatactgt tttctggtac agacagacca tgatgcaagg actggagttg ctggcttact 240
   tccgcaaccg ggctcctcta gatgattcgg ggatgccgaa ggatcgattc tcagcagaga 300
   tgcctgatgc aactttagcc actctgaaga tccagccctc agaacccagg gactcagctg 360
   tgtacttttg cagagacctt gcggccgtgt cttccgacgc tgacagtgta gata 414
<210> 469
   <211> 441
   <212> DNA
   <213> Homo sapiens
<220>
   <223> TRBV13_BB_1
<400> 469
   gttcgaagct ggcatgacac gaagacttgt acgccaccat gcttagtcct gacctgcctg 60
   actctgcctg gaacaccagg ctcctctgcc atgtcatgct ttgtctcctg ggagcagttt 120
   cagtggctgc tggagtcatc cagtccccaa gacatctgat caaagaaaag agggaaacag 180
   ccactctgaa atgctatcct atccctagac acgacactgt ctactggtac cagcagggtc 240
   caggtcagga cccccagttc ctcatttcgt tttatgaaaa gatgcagagc gataaaggaa 300
   gcatccctga tcgattctca gctcaacagt tcagtgacta tcattctgaa ctgaacatga 360
   gctccttgga gctgggggac tcagccctgt acttttgcag agaccttgcg gccgtgtctt 420
   ccgacgctga cagtgtagat a 441
<210> 470
   <211> 414
   <212> DNA
   <213> Homo sapiens
<220>
   <223> TRBV14_BB_1
<400> 470
   gttcgaagct ggcatgacac gaagacttgt acgccaccat ggtttccagg cttctcagtt 60
   tagtgtccct ttgtctcctg ggagcaaagc acatagaagc tggagttact cagttcccca 120
   gccacagcgt aatagagaag ggccagactg tgactctgag atgtgaccca atttctggac 180
   atgataatct ttattggtat cgacgtgtta tgggaaaaga aataaaattt ctgttacatt 240
   ttgtgaaaga gtctaaacag gatgagtccg gtatgcccaa caatcgattc ttagctgaaa 300
   ggactggagg gacgtattct actctgaagg tgcagcctgc agaactggag gattctggag 360
   tttacttttg cagagacctt gcggccgtgt cttccgacgc tgacagtgta gata 414
<210> 471
   <211> 411
   <212> DNA
   <213> Homo sapiens
<220>
   <223> TRBV15_BB_1
<400> 471
   gttcgaagct ggcatgacac gaagacttgt acgccaccat gggtcctggg cttctccact      60
   ggatggccct ttgtctcctt ggaacaggtc atggggatgc catggtcatc cagaacccaa     120
   gataccaggt tacccagttt ggaaagccag tgaccctgag ttgttctcag actttgaacc     180
   ataacgtcat gtactggtac cagcagaagt caagtcaggc cccaaagctg ctgttccact     240
   actatgacaa agattttaac aatgaagcag acacccctga taacttccaa tccaggaggc     300
   cgaacacttc tttctgcttt cttgacatcc gctcaccagg cctgggggac acagccatgt     360
   acctttgcag agaccttgcg gccgtgtctt ccgacgctga cagtgtagat a              411
<210> 472
   <211> 414
   <212> DNA
   <213> Homo sapiens
<220>
   <223> TRBV16_BB_1
<400> 472
   gttcgaagct ggcatgacac gaagacttgt acgccaccat gagcccaata ttcacctgca      60
   tcacaatcct ttgtctgctg gctgcaggtt ctcctggtga agaagtcgcc cagactccaa     120
   aacatcttgt cagaggggaa ggacagaaag caaaattata ttgtgcccca ataaaaggac     180
   acagttatgt tttttggtac caacaggtcc tgaaaaacga gttcaagttc ttgatttcct     240
   tccagaatga aaatgtcttt gatgaaacag gtatgcccaa ggaaagattt tcagctaagt     300
   gcctcccaaa ttcaccctgt agccttgaga tccaggctac gaagcttgag gattcagcag     360
   tgtacttttg cagagacctt gcggccgtgt cttccgacgc tgacagtgta gata           414
<210> 473
   <211> 414
   <212> DNA
   <213> Homo sapiens
<220>
   <223> TRBV18_BB_1
<400> 473
   gttcgaagct ggcatgacac gaagacttgt acgccaccat ggacaccaga gtactctgct 60
   gtgcggtcat ctgccttctg ggggcaggac tctcaaatgc cggcgtcatg cagaacccaa 120
   gacacctggt caggaggagg ggacaggagg caagactgag atgcagccca atgaaaggac 180
   acagtcatgt ttactggtat cggcagctcc cagaggaagg tctgaaattc atggtttatc 240
   tccagaaaga aaatatcata gatgagtcag gaatgccaaa ggaacgattt tctgctgaat 300
   ttcccaaaga gggccccagc atcctgagga tccagcaggt agtgcgagga gattcggcag 360
   cttacttttg cagagacctt gcggccgtgt cttccgacgc tgacagtgta gata 414
<210> 474
   <211> 411
   <212> DNA
   <213> Homo sapiens
<220>
   <223> TRBV19_BB_1
<400> 474
   gttcgaagct ggcatgacac gaagacttgt acgccaccat gagcaaccag gtgctctgct 60
   gtgtggtcct ttgtttcctg ggagcaaaca ccgtggatgg tggaatcact cagtccccaa 120
   agtacctgtt cagaaaggaa ggacagaatg tgaccctgag ttgtgaacag aatttgaacc 180
   acgatgccat gtactggtac cgacaggacc cagggcaagg gctgagattg atctactact 240
   cacagatagt aaatgacttt cagaaaggag atatagctga agggtacagc gtctctcggg 300
   agaagaagga atcctttcct ctcactgtga catcggccca aaagaacccg acagctttct 360
   atctttgcag agaccttgcg gccgtgtctt ccgacgctga cagtgtagat a 411
<210> 475
   <211> 405
   <212> DNA
   <213> Homo sapiens
<220>
   <223> TRBV20-1_BB_1
<400> 475
   gttcgaagct ggcatgacac gaagacttgt acgccaccat gctgctgctt ctgctgcttc 60
   tggggccagg ctccgggctt ggtgctgtcg tctctcaaca tccgagctgg gttatctgta 120
   agagtggaac ctctgtgaag atcgagtgcc gttccctgga ctttcaggcc acaactatgt 180
   tttggtatcg tcagttcccg aaacagagtc tcatgctgat ggcaacttcc aatgagggct 240
   ccaaggccac atacgagcaa ggcgtcgaga aggacaagtt tctcatcaac catgcaagcc 300
   tgaccttgtc cactctgaca gtgaccagtg cccatcctga agatagcagc ttctacattt 360
   gcagagacct tgcggccgtg tcttccgacg ctgacagtgt agata 405
<210> 476
   <211> 411
   <212> DNA
   <213> Homo sapiens
<220>
   <223> TRBV24-1_BB_1
<400> 476
   gttcgaagct ggcatgacac gaagacttgt acgccaccat ggcctccctg ctcttcttct 60
   gtggggcctt ttatctcctg ggaacagggt ccatggatgc tgatgttacc cagaccccaa 120
   ggaataggat cacaaagaca ggaaagagga ttatgctgga atgttctcag actaagggtc 180
   atgatagaat gtactggtat cgacaagacc caggactggg cctacggttg atctattact 240
   cctttgatgt caaagatata aacaaaggag agatctctga tggatacagt gtctctcgac 300
   aggcacaggc taaattctcc ctgtccctag agtctgccat ccccaaccag acagctcttt 360
   acttttgcag agaccttgcg gccgtgtctt ccgacgctga cagtgtagat a 411
<210> 477
   <211> 411
   <212> DNA
   <213> Homo sapiens
<220>
   <223> TRBV25-1_BB_1
<400> 477
   gttcgaagct ggcatgacac gaagacttgt acgccaccat gactatcagg ctcctctgct 60
   acatgggctt ttattttctg ggggcaggcc tcatggaagc tgacatctac cagaccccaa 120
   gataccttgt tatagggaca ggaaagaaga tcactctgga atgttctcaa accatgggcc 180
   atgacaaaat gtactggtat caacaagatc caggaatgga actacacctc atccactatt 240
   cctatggagt taattccaca gagaagggag atctttcctc tgagtcaaca gtctccagaa 300
   taaggacgga gcattttccc ctgaccctgg agtctgccag gccctcacat acctctcagt 360
   acctttgcag agaccttgcg gccgtgtctt ccgacgctga cagtgtagat a 411
<210> 478
   <211> 411
   <212> DNA
   <213> Homo sapiens
<220>
   <223> TRBV27_BB_1
<400> 478
   gttcgaagct ggcatgacac gaagacttgt acgccaccat gggcccccag ctccttggct 60
   atgtggtcct ttgccttcta ggagcaggcc ccctggaagc ccaagtgacc cagaacccaa 120
   gatacctcat cacagtgact ggaaagaagt taacagtgac ttgttctcag aatatgaacc 180
   atgagtatat gtcctggtat cgacaagacc cagggctggg cttaaggcag atctactatt 240
   caatgaatgt tgaggtgact gataagggag atgttcctga agggtacaaa gtctctcgaa 300
   aagagaagag gaatttcccc ctgatcctgg agtcgcccag ccccaaccag acctctctgt 360
   acttttgcag agaccttgcg gccgtgtctt ccgacgctga cagtgtagat a 411
<210> 479
   <211> 411
   <212> DNA
   <213> Homo sapiens
<220>
   <223> TRBV28_BB_1
<400> 479
   gttcgaagct ggcatgacac gaagacttgt acgccaccat gggaatcagg ctcctctgtc 60
   gtgtggcctt ttgtttcctg gctgtaggcc tcgtagatgt gaaagtaacc cagagctcga 120
   gatatctagt caaaaggacg ggagagaaag tttttctgga atgtgtccag gatatggacc 180
   atgaaaatat gttctggtat cgacaagacc caggtctggg gctacggctg atctatttct 240
   catatgatgt taaaatgaaa gaaaaaggag atattcctga ggggtacagt gtctctagag 300
   agaagaagga gcgcttctcc ctgattctgg agtccgccag caccaaccag acatctatgt 360
   acctttgcag agaccttgcg gccgtgtctt ccgacgctga cagtgtagat a 411
<210> 480
   <211> 405
   <212> DNA
   <213> Homo sapiens
<220>
   <223> TRBV29-1_BB_1
<400> 480
   gttcgaagct ggcatgacac gaagacttgt acgccaccat gctgagtcta ctgctccttc 60
   tcctgggact aggctctgtg ttcagtgctg tcatctctca aaagccaagc agggatatct 120
   gtcaacgtgg aacctccctg acgatccagt gtcaagtcga tagccaagtc accatgatgt 180
   tctggtaccg tcagcaacct ggacagagcc tgacactgat cgcaactgca aatcagggct 240
   ctgaggccac atatgagagt ggatttgtca ttgacaagtt tcccatcagc cgcccaaacc 300
   taacattctc aactctgact gtgagcaaca tgagccctga agatagcagc atatatcttt 360
   gcagagacct tgcggccgtg tcttccgacg ctgacagtgt agata 405
<210> 481
   <211> 405
   <212> DNA
   <213> Homo sapiens
<220>
   <223> TRBV30_BB_1
<400> 481
   gttcgaagct ggcatgacac gaagacttgt acgccaccat gctctgctct ctccttgccc 60
   ttctcctggg cactttcttt ggggtcagat ctcagactat tcatcaatgg ccagcgaccc 120
   tggtgcagcc tgtgggcagc ccgctctctc tggagtgcac tgtggaggga acatcaaacc 180
   ccaacctata ctggtaccga caggctgcag gcaggggcct ccagctgctc ttctactccg 240
   ttggtattgg ccagatcagc tctgaggtgc cccagaatct ctcagcctcc agaccccagg 300
   accggcagtt catcctgagt tctaagaagc tccttctcag tgactctggc ttctatcttt 360
   gcagagacct tgcggccgtg tcttccgacg ctgacagtgt agata 405
<210> 482
   <211> 595
   <212> DNA
   <213> Homo sapiens
<220>
   <223> TRBC1 cloning fragment
<400> 482
   cagcactcgt atgatcagag gaagactagg ccgcataggt ctcagtgttc ccacccgagg      60
   tcgctgtgtt tgagccatca gaagcagaga tctcccacac ccaaaaggcc acactggtgt     120
   gcctggccac aggcttcttc cccgaccacg tggagctgag ctggtgggtg aatgggaagg     180
   aggtgcacag tggggtcagc acagacccgc agcccctcaa ggagcagccc gccctcaatg     240
   actccagata ctgcctgagc agccgcctga gggtgtcggc caccttctgg cagaaccccc     300
   gcaaccactt ccgctgtcaa gtccagttct acgggctctc ggagaatgac gagtggaccc     360
   aggatagggc caaacccgtc acccagatcg tcagcgccga ggcctggggt agagcagact     420
   gtggctttac ctcggtgtcc taccagcaag gggtcctgtc tgccaccatc ctctatgaga     480
   tcctgctagg gaaggccacc ctgtatgctg tgctggtcag cgcccttgtg ttgatggcca     540
   tggtcaagag aaaggatttc tgactaggtg tcttcgctac actgatccga tggtc          595
<210> 483
   <211> 601
   <212> DNA
   <213> Homo sapiens
<220>
   <223> TRBC2 cloning fragment
<400> 483
   cagcactcgt atgatcagag gaagactagg ccgcataggt ctcagtgttc ccacccgagg      60
   tcgctgtgtt tgagccatca gaagcagaga tctcccacac ccaaaaggcc acactggtat     120
   gcctggccac aggcttctac cccgaccacg tggagctgag ctggtgggtg aatgggaagg     180
   aggtgcacag tggggtcagc acagacccgc agcccctcaa ggagcagccc gccctcaatg     240
   actccagata ctgcctgagc agccgcctga gggtgtcggc caccttctgg cagaaccccc     300
   gcaaccactt ccgctgtcaa gtccagttct acgggctctc ggagaatgac gagtggaccc     360
   aggatagggc caaacccgtc acccagatcg tcagcgccga ggcctggggt agagcagact     420
   gtggcttcac ctccgagtct taccagcaag gggtcctgtc tgccaccatc ctctatgaga     480
   tcttgctagg gaaggccacc ttgtatgccg tgctggtcag tgccctcgtg ctgatggcca     540
   tggtcaagag aaaggattcc agaggctagc taggtgtctt cgctacactg atccgatggt     600
   c                                                                     601
<210> 484
   <211> 891
   <212> DNA
   <213> Homo sapiens
<220>
   <223> V-C entry TRBV2_TRBC2
<400> 484
   gccaccatgg atacctggct cgtatgctgg gcaattttta gtctcttgaa agcaggactc      60
   acagaacctg aagtcaccca gactcccagc catcaggtca cacagatggg acaggaagtg     120
   atcttgcgct gtgtccccat ctctaatcac ttatacttct attggtacag acaaatcttg     180
   gggcagaaag tcgagtttct ggtttccttt tataataatg aaatctcaga gaagtctgaa     240
   atattcgatg atcaattctc agttgaaagg cctgatggat caaatttcac tctgaagatc     300
   cggtccacaa agctggagga ctcagccatg tacttttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g              891
<210> 485
   <211> 888
   <212> DNA
   <213> Homo sapiens
<220>
   <223> V-C entry TRBV3-1_TRBC2
<400> 485
   gccaccatgg gctgcaggct cctctgctgt gtggtctttt gcctcctcca agcaggtccc      60
   ttggacacag ctgtttccca gactccaaaa tacctggtca cacagatggg aaacgacaag     120
   tccattaaat gtgaacaaaa tctgggccat gatactatgt attggtataa acaggactct     180
   aagaaatttc tgaagataat gtttagctac aataataagg agctcattat aaatgaaaca     240
   gttccaaatc gcttctcacc taaatctcca gacaaagctc acttaaatct tcacatcaat     300
   tccctggagc ttggtgactc tgctgtgtat ttttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 486
   <211> 888
   <212> DNA
   <213> Homo sapiens
<220>
   <223> V-C entry TRBV4-1_TRBC2
<400> 486
   gccaccatgg gctgcaggct gctctgctgt gcggttctct gtctcctggg agcagttccc      60
   atagacactg aagttaccca gacaccaaaa cacctggtca tgggaatgac aaataagaag     120
   tctttgaaat gtgaacaaca tatggggcac agggctatgt attggtacaa gcagaaagct     180
   aagaagccac cggagctcat gtttgtctac agctatgaga aactctctat aaatgaaagt     240
   gtgccaagtc gcttctcacc tgaatgcccc aacagctctc tcttaaacct tcacctacac     300
   gccctgcagc cagaagattc agccctgtat ctttgcagag accttgcggc cgcataggtc     360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 487
   <211> 888
   <212> DNA
   <213> Homo sapiens
<220>
   <223> V-C entry TRBV4-2_TRBC2
<400> 487
   gccaccatgg gctgcaggct gctctgctgt gcggttctct gtctcctggg agcggtcccc 60
   atggaaacgg gagttacgca gacaccaaga cacctggtca tgggaatgac aaataagaag 120
   tctttgaaat gtgaacaaca tctggggcat aacgctatgt attggtacaa gcaaagtgct 180
   aagaagccac tggagctcat gtttgtctac aactttaaag aacagactga aaacaacagt 240
   gtgccaagtc gcttctcacc tgaatgcccc aacagctctc acttattcct tcacctacac 300
   accctgcagc cagaagattc ggccctgtat ctttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 488
   <211> 888
   <212> DNA
   <213> Homo sapiens
<220>
   <223> V-C entry TRBV4-3_TRBC2
<400> 488
   gccaccatgg gctgcaggct gctctgctgt gcggttctct gtctcctggg agcggtcccc      60
   atggaaacgg gagttacgca gacaccaaga cacctggtca tgggaatgac aaataagaag     120
   tctttgaaat gtgaacaaca tctgggtcat aacgctatgt attggtacaa gcaaagtgct     180
   aagaagccac tggagctcat gtttgtctac agtcttgaag aacgggttga aaacaacagt     240
   gtgccaagtc gcttctcacc tgaatgcccc aacagctctc acttattcct tcacctacac     300
   accctgcagc cagaagattc ggccctgtat ctttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 489
   <211> 888
   <212> DNA
   <213> Homo sapiens
<220>
   <223> V-C entry TRBV5-1_TRBC2
<400> 489
   gccaccatgg gctccaggct gctctgttgg gtgctgcttt gtctcctggg agcaggccca      60
   gtaaaggctg gagtcactca aactccaaga tatctgatca aaacgagagg acagcaagtg     120
   acactgagct gctcccctat ctctgggcat aggagtgtat cctggtacca acagacccca     180
   ggacagggcc ttcagttcct ctttgaatac ttcagtgaga cacagagaaa caaaggaaac     240
   ttccctggtc gattctcagg gcgccagttc tctaactctc gctctgagat gaatgtgagc     300
   accttggagc tgggggactc ggccctttat ctttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 490
   <211> 888
   <212> DNA
   <213> Homo sapiens
<220>
   <223> V-C entry TRBV5-4_TRBC2
<400> 490
   gccaccatgg gccctgggct cctctgctgg gtgctgcttt gtctcctggg agcaggctca      60
   gtggagactg gagtcaccca aagtcccaca cacctgatca aaacgagagg acagcaagtg     120
   actctgagat gctcttctca gtctgggcac aacactgtgt cctggtacca acaggccctg     180
   ggtcaggggc cccagtttat ctttcagtat tatagggagg aagagaatgg cagaggaaac     240
   ttccctccta gattctcagg actccagttc cctaattata gctctgagct gaatgtgaac     300
   gccttggagc tggacgactc ggccctgtat ctttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 491
   <211> 888
   <212> DNA
   <213> Homo sapiens
<220>
   <223> V-C entry TRBV5-5_TRBC2
<400> 491
   gccaccatgg gccctgggct cctctgctgg gtgctgcttt gtctcctggg agcaggccca      60
   gtggacgctg gagtcaccca aagtcccaca cacctgatca aaacgagagg acagcaagtg 120
   actctgagat gctctcctat ctctgggcac aagagtgtgt cctggtacca acaggtcctg 180
   ggtcaggggc cccagtttat ctttcagtat tatgagaaag aagagagagg aagaggaaac 240
   ttccctgatc gattctcagc tcgccagttc cctaactata gctctgagct gaatgtgaac 300
   gccttgttgc tgggggactc ggccctgtat ctttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 492
   <211> 888
   <212> DNA
   <213> Homo sapiens
<220>
   <223> V-C entry TRBV5-6_TRBC2
<400> 492
   gccaccatgg gccccgggct cctctgctgg gcactgcttt gtctcctggg agcaggctta 60
   gtggacgctg gagtcaccca aagtcccaca cacctgatca aaacgagagg acagcaagtg 120
   actctgagat gctctcctaa gtctgggcat gacactgtgt cctggtacca acaggccctg 180
   ggtcaggggc cccagtttat ctttcagtat tatgaggagg aagagagaca gagaggcaac 240
   ttccctgatc gattctcagg tcaccagttc cctaactata gctctgagct gaatgtgaac 300
   gccttgttgc tgggggactc ggccctctat ctttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 493
   <211> 888
   <212> DNA
   <213> Homo sapiens
<220>
   <223> V-C entry TRBV5-7_TRBC2
<400> 493
   gccaccatgg gccccgggct cctctgctgg gtgctgcttt gtcccctagg agaaggccca      60
   gtggacgctg gagtcaccca aagtcccaca cacctgatca aaacgagagg acagcacgtg     120
   actctgagat gctctcctat ctctgggcac accagtgtgt cctcgtacca acaggccctg     180
   ggtcaggggc cccagtttat ctttcagtat tatgagaaag aagagagagg aagaggaaac     240
   ttccctgatc aattctcagg tcaccagttc cctaactata gctctgagct gaatgtgaac     300
   gccttgttgc taggggactc ggccctctat ctttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 494
   <211> 888
   <212> DNA
   <213> Homo sapiens
<220>
   <223> V-C entry TRBV5-8_TRBC2
<400> 494
   gccaccatgg gacccaggct cctcttctgg gcactgcttt gtctcctcgg aacaggccca      60
   gtggaggctg gagtcacaca aagtcccaca cacctgatca aaacgagagg acagcaagcg     120
   actctgagat gctctcctat ctctgggcac accagtgtgt actggtacca acaggccctg     180
   ggtctgggcc tccagttcct cctttggtat gacgagggtg aagagagaaa cagaggaaac     240
   ttccctccta gattttcagg tcgccagttc cctaattata gctctgagct gaatgtgaac 300
   gccttggagc tggaggactc ggccctgtat ctttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 495
   <211> 888
   <212> DNA
   <213> Homo sapiens
<220>
   <223> V-C entry TRBV6-1_TRBC2
<400> 495
   gccaccatga gcatcgggct cctgtgctgt gtggcctttt ctctcctgtg ggcaagtcca      60
   gtgaatgctg gtgtcactca gaccccaaaa ttccaggtcc tgaaaacagg acagagcatg     120
   acactgcagt gtgcccagga tatgaaccat aactccatgt actggtatcg acaagaccca     180
   ggcatgggac tgaggctgat ttattactca gcttctgagg gtaccactga caaaggagaa     240
   gtccccaatg gctacaatgt ctccagatta aacaaacggg agttctcgct caggctggag     300
   tcggctgctc cctcccagac atctgtgtac ttttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 496
   <211> 888
   <212> DNA
   <213> Homo sapiens
<220>
   <223> V-C entry TRBV6-3_TRBC2
<400> 496
   gccaccatga gcctcgggct cctgtgctgt ggggtctttt ctctcctgtg ggcaggtcca 60
   gtgaatgctg gtgtcactca gaccccaaaa ttccgggtcc tgaaaacagg acagagcatg 120
   acactgctgt gtgcccagga tatgaaccat gaatacatgt actggtatcg acaagaccca 180
   ggcatggggc tgaggctgat tcattactca gttggtgagg gtacaactgc caaaggagag 240
   gtccctgatg gctacaatgt ctccagatta aaaaaacaga atttcctgct ggggttggag 300
   tcggctgctc cctcccaaac atctgtgtac ttttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 497
   <211> 888
   <212> DNA
   <213> Homo sapiens
<220>
   <223> V-C entry TRBV6-4_TRBC2
<400> 497
   gccaccatga gaatcaggct cctgtgctgt gtggcctttt ctctcctgtg ggcaggtcca 60
   gtgattgctg ggatcaccca ggcaccaaca tctcagatcc tggcagcagg acggcgcatg 120
   acactgagat gtacccagga tatgagacat aatgccatgt actggtatag acaagatcta 180
   ggactggggc taaggctcat ccattattca aatactgcag gtaccactgg caaaggagaa 240
   gtccctgatg gttatagtgt ctccagagca aacacagatg atttccccct cacgttggcg 300
   tctgctgtac cctctcagac atctgtgtac ttttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 498
   <211> 888
   <212> DNA
   <213> Homo sapiens
<220>
   <223> V-C entry TRBV6-5_TRBC2
<400> 498
   gccaccatga gcatcggcct cctgtgctgt gcagccttgt ctctcctgtg ggcaggtcca 60
   gtgaatgctg gtgtcactca gaccccaaaa ttccaggtcc tgaaaacagg acagagcatg 120
   acactgcagt gtgcccagga tatgaaccat gaatacatgt cctggtatcg acaagaccca 180
   ggcatggggc tgaggctgat tcattactca gttggtgctg gtatcactga ccaaggagaa 240
   gtccccaatg gctacaatgt ctccagatca accacagagg atttcccgct caggctgctg 300
   tcggctgctc cctcccagac atctgtgtac ttttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 499
   <211> 888
   <212> DNA
   <213> Homo sapiens
<220>
   <223> V-C entry TRBV6-6_TRBC2
<400> 499
   gccaccatga gcatcagcct cctgtgctgt gcagcctttc ctctcctgtg ggcaggtcca      60
   gtgaatgctg gtgtcactca gaccccaaaa ttccgcatcc tgaagatagg acagagcatg     120
   acactgcagt gtacccagga tatgaaccat aactacatgt actggtatcg acaagaccca     180
   ggcatggggc tgaagctgat ttattattca gttggtgctg gtatcactga taaaggagaa     240
   gtcccgaatg gctacaacgt ctccagatca accacagagg atttcccgct caggctggag     300
   ttggctgctc cctcccagac atctgtgtac ttttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 500
   <211> 885
   <212> DNA
   <213> Homo sapiens
<220>
   <223> V-C entry TRBV6-8_TRBC2
<400> 500
   gccaccatga gcctcgggct cctgtgctgt gcggcctttt ctctcctgtg ggcaggtccc      60
   gtgaatgctg gtgtcactca gaccccaaaa ttccacatcc tgaaaacagg acagagcatg     120
   acactgcagt gtgcccagga tatgaaccat ggatacatgt cctggtatcg acaagaccca     180
   ggcatggggc tgagactgat ttactactca gctgctgctg gtactactga caaagaagtc     240
   cccaatggct acaatgtctc tagattaaac acagaggatt tcccactcag gctggtgtcg     300
   gctgctccct cccagacatc tgtgtacctt tgcagagacc ttgcggccgc ataggtctca     360
   gtgttcccac ccgaggtcgc tgtgtttgag ccatcagaag cagagatctc ccacacccaa     420
   aaggccacac tggtatgcct ggccacaggc ttctaccccg accacgtgga gctgagctgg     480
   tgggtgaatg ggaaggaggt gcacagtggg gtcagcacag acccgcagcc cctcaaggag     540
   cagcccgccc tcaatgactc cagatactgc ctgagcagcc gcctgagggt gtcggccacc     600
   ttctggcaga acccccgcaa ccacttccgc tgtcaagtcc agttctacgg gctctcggag     660
   aatgacgagt ggacccagga tagggccaaa cccgtcaccc agatcgtcag cgccgaggcc     720
   tggggtagag cagactgtgg cttcacctcc gagtcttacc agcaaggggt cctgtctgcc     780
   accatcctct atgagatctt gctagggaag gccaccttgt atgccgtgct ggtcagtgcc     840
   ctcgtgctga tggccatggt caagagaaag gattccagag gctag                     885
<210> 501
   <211> 888
   <212> DNA
   <213> Homo sapiens
<220>
   <223> V-C entry TRBV6-9_TRBC2
<400> 501
   gccaccatga gcatcgggct cctgtgctgt gtggcctttt ctctcctgtg ggcaggtcca      60
   gtgaatgctg gtgtcactca gaccccaaaa ttccacatcc tgaaaacagg acagagcatg     120
   acactgcagt gtgcccagga tatgaaccat ggatacttgt cctggtatcg acaagaccca     180
   ggcatggggc tgaggcgcat tcattactca gttgctgctg gtatcactga caaaggagaa     240
   gtccccgatg gctacaatgt atccagatca aacacagagg atttcccgct caggctggag     300
   tcagctgctc cctcccagac atctgtatac ttttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 502
   <211> 891
   <212> DNA
   <213> Homo sapiens
<220>
   <223> V-C entry TRBV7-2_TRBC2
<400> 502
   gccaccatgg gcaccaggct cctcttctgg gtggccttct gtctcctggg ggcagatcac      60
   acaggagctg gagtctccca gtcccccagt aacaaggtca cagagaaggg aaaggatgta 120
   gagctcaggt gtgatccaat ttcaggtcat actgcccttt actggtaccg acagagcctg 180
   gggcagggcc tggagttttt aatttacttc caaggcaaca gtgcaccaga caaatcaggg 240
   ctgcccagtg atcgcttctc tgcagagagg actgggggat ccgtctccac tctgacgatc 300
   cagcgcacac agcaggagga ctcggccgtg tatctttgca gagaccttgc ggccgcatag 360
   gtctcagtgt tcccacccga ggtcgctgtg tttgagccat cagaagcaga gatctcccac 420
   acccaaaagg ccacactggt atgcctggcc acaggcttct accccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttc acctccgagt cttaccagca aggggtcctg 780
   tctgccacca tcctctatga gatcttgcta gggaaggcca ccttgtatgc cgtgctggtc 840
   agtgccctcg tgctgatggc catggtcaag agaaaggatt ccagaggcta g 891
<210> 503
   <211> 891
   <212> DNA
   <213> Homo sapiens
<220>
   <223> V-C entry TRBV7-3_TRBC2
<400> 503
   gccaccatgg gcaccaggct cctctgctgg gcagccctgt gcctcctggg ggcagatcac      60
   acaggtgctg gagtctccca gacccccagt aacaaggtca cagagaaggg aaaatatgta     120
   gagctcaggt gtgatccaat ttcaggtcat actgcccttt actggtaccg acaaagcctg     180
   gggcagggcc cagagtttct aatttacttc caaggcacgg gtgcggcaga tgactcaggg     240
   ctgcccaacg atcggttctt tgcagtcagg cctgagggat ccgtctctac tctgaagatc     300
   cagcgcacag agcgggggga ctcagccgtg tatctttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g              891
<210> 504
   <211> 891
   <212> DNA
   <213> Homo sapiens
<220>
   <223> V-C entry TRBV7-6_TRBC2
<400> 504
   gccaccatgg gcaccagtct cctatgctgg gtggtcctgg gtttcctagg gacagatcac        60
   acaggtgctg gagtctccca gtctcccagg tacaaagtca caaagagggg acaggatgta       120
   gctctcaggt gtgatccaat ttcgggtcat gtatcccttt attggtaccg acaggccctg       180
   gggcagggcc cagagtttct gacttacttc aattatgaag cccaacaaga caaatcaggg       240
   ctgcccaatg atcggttctc tgcagagagg cctgagggat ccatctccac tctgacgatc       300
   cagcgcacag agcagcggga ctcggccatg tatcgttgca gagaccttgc ggccgcatag       360
   gtctcagtgt tcccacccga ggtcgctgtg tttgagccat cagaagcaga gatctcccac       420
   acccaaaagg ccacactggt atgcctggcc acaggcttct accccgacca cgtggagctg       480
   agctggtggg tgaatgggaa ggaggtgcac agtggggtca gcacagaccc gcagcccctc       540
   aaggagcagc ccgccctcaa tgactccaga tactgcctga gcagccgcct gagggtgtcg       600
   gccaccttct ggcagaaccc ccgcaaccac ttccgctgtc aagtccagtt ctacgggctc       660
   tcggagaatg acgagtggac ccaggatagg gccaaacccg tcacccagat cgtcagcgcc       720
   gaggcctggg gtagagcaga ctgtggcttc acctccgagt cttaccagca aggggtcctg       780
   tctgccacca tcctctatga gatcttgcta gggaaggcca ccttgtatgc cgtgctggtc       840
   agtgccctcg tgctgatggc catggtcaag agaaaggatt ccagaggcta g                891
<210> 505
   <211> 891
   <212> DNA
   <213> Homo sapiens
<220>
   <223> V-C entry TRBV7-7_TRBC2
<400> 505
   gccaccatgg gtaccagtct cctatgctgg gtggtcctgg gtttcctagg gacagatcac        60
   acaggtgctg gagtctccca gtctcccagg tacaaagtca caaagagggg acaggatgta       120
   actctcaggt gtgatccaat ttcgagtcat gcaacccttt attggtatca acaggccctg       180
   gggcagggcc cagagtttct gacttacttc aattatgaag ctcaaccaga caaatcaggg       240
   ctgcccagtg atcggttctc tgcagagagg cctgagggat ccatctccac tctgacgatt 300
   cagcgcacag agcagcggga ctcagccatg tatcgttgca gagaccttgc ggccgcatag 360
   gtctcagtgt tcccacccga ggtcgctgtg tttgagccat cagaagcaga gatctcccac 420
   acccaaaagg ccacactggt atgcctggcc acaggcttct accccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttc acctccgagt cttaccagca aggggtcctg 780
   tctgccacca tcctctatga gatcttgcta gggaaggcca ccttgtatgc cgtgctggtc 840
   agtgccctcg tgctgatggc catggtcaag agaaaggatt ccagaggcta g 891
<210> 506
   <211> 891
   <212> DNA
   <213> Homo sapiens
<220>
   <223> V-C entry TRBV7-8_TRBC2
<400> 506
   gccaccatgg gcaccaggct cctctgctgg gtggtcctgg gtttcctagg gacagatcac      60
   acaggtgctg gagtctccca gtcccctagg tacaaagtcg caaagagagg acaggatgta     120
   gctctcaggt gtgatccaat ttcgggtcat gtatcccttt tttggtacca acaggccctg     180
   gggcaggggc cagagtttct gacttatttc cagaatgaag ctcaactaga caaatcgggg     240
   ctgcccagtg atcgcttctt tgcagaaagg cctgagggat ccgtctccac tctgaagatc     300
   cagcgcacac agcaggagga ctccgccgtg tatctttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g              891
<210> 507
   <211> 891
   <212> DNA
   <213> Homo sapiens
<220>
   <223> V-C entry TRBV7-9_TRBC2
<400> 507
   gccaccatgg gcaccagcct cctctgctgg atggccctgt gtctcctggg ggcagatcac     60
   gcagatactg gagtctccca gaaccccaga cacaagatca caaagagggg acagaatgta    120
   actttcaggt gtgatccaat ttctgaacac aaccgccttt attggtaccg acagaccctg    180
   gggcagggcc cagagtttct gacttacttc cagaatgaag ctcaactaga aaaatcaagg    240
   ctgctcagtg atcggttctc tgcagagagg cctaagggat ctttctccac cttggagatc    300
   cagcgcacag agcaggggga ctcggccatg tatctttgca gagaccttgc ggccgcatag    360
   gtctcagtgt tcccacccga ggtcgctgtg tttgagccat cagaagcaga gatctcccac    420
   acccaaaagg ccacactggt atgcctggcc acaggcttct accccgacca cgtggagctg    480
   agctggtggg tgaatgggaa ggaggtgcac agtggggtca gcacagaccc gcagcccctc    540
   aaggagcagc ccgccctcaa tgactccaga tactgcctga gcagccgcct gagggtgtcg    600
   gccaccttct ggcagaaccc ccgcaaccac ttccgctgtc aagtccagtt ctacgggctc    660
   tcggagaatg acgagtggac ccaggatagg gccaaacccg tcacccagat cgtcagcgcc    720
   gaggcctggg gtagagcaga ctgtggcttc acctccgagt cttaccagca aggggtcctg    780
   tctgccacca tcctctatga gatcttgcta gggaaggcca ccttgtatgc cgtgctggtc    840
   agtgccctcg tgctgatggc catggtcaag agaaaggatt ccagaggcta g             891
<210> 508
   <211> 888
   <212> DNA
   <213> Homo sapiens
<220>
   <223> V-C entry TRBV9_TRBC2
<400> 508
   gccaccatgg gcttcaggct cctctgctgt gtggcctttt gtctcctggg agcaggccca     60
   gtggattctg gagtcacaca aaccccaaag cacctgatca cagcaactgg acagcgagtg    120
   acgctgagat gctcccctag gtctggtgac ctctctgtgt actggtacca acagagcctg    180
   gaccagggcc tccagttcct cattcagtat tataatggag aagagagagc aaaaggaaac    240
   attcttgaac gattctccgc acaacagttc cctgacttgc actctgaact aaacctgagc    300
   tctctggagc tgggggactc agctttgtac ttttgcagag accttgcggc cgcataggtc    360
   tcagtgttcc cacccgaggt cgctgtgttt gagccatcag aagcagagat ctcccacacc    420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 509
   <211> 888
   <212> DNA
   <213> Homo sapiens
<220>
   <223> V-C entry TRBV10-1_TRBC2
<400> 509
   gccaccatgg gcacgaggct cttcttctat gtggcccttt gtctgctgtg ggcaggacac 60
   agggatgctg aaatcaccca gagcccaaga cacaagatca cagagacagg aaggcaggtg 120
   accttggcgt gtcaccagac ttggaaccac aacaatatgt tctggtatcg acaagacctg 180
   ggacatgggc tgaggctgat ccattactca tatggtgttc aagacactaa caaaggagaa 240
   gtctcagatg gctacagtgt ctctagatca aacacagagg acctccccct cactctggag 300
   tctgctgcct cctcccagac atctgtatac ttttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 510
   <211> 888
   <212> DNA
   <213> Homo sapiens
<220>
   <223> V-C entry TRBV10-2_TRBC2
<400> 510
   gccaccatgg gcaccaggct cttcttctat gtggcccttt gtctgctgtg ggcaggacac      60
   agggatgctg gaatcaccca gagcccaaga tacaagatca cagagacagg aaggcaggtg     120
   accttgatgt gtcaccagac ttggagccac agctatatgt tctggtatcg acaagacctg     180
   ggacatgggc tgaggctgat ctattactca gcagctgctg atattacaga taaaggagaa     240
   gtccccgatg gctatgttgt ctccagatcc aagacagaga atttccccct cactctggag      300
   tcagctaccc gctcccagac atctgtgtac ttttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                 888
<210> 511
   <211> 888
   <212> DNA
   <213> Homo sapiens
<220>
   <223> V-C entry TRBV10-3_TRBC2
<400> 511
   gccaccatgg gcacaaggtt gttcttctat gtggcccttt gtctcctgtg gacaggacac 60
   atggatgctg gaatcaccca gagcccaaga cacaaggtca cagagacagg aacaccagtg 120
   actctgagat gtcaccagac tgagaaccac cgctatatgt actggtatcg acaagacccg 180
   gggcatgggc tgaggctgat ccattactca tatggtgtta aagatactga caaaggagaa 240
   gtctcagatg gctatagtgt ctctagatca aagacagagg atttcctcct cactctggag 300
   tccgctacca gctcccagac atctgtgtac ttttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 512
   <211> 891
   <212> DNA
   <213> Homo sapiens
<220>
   <223> V-C entry TRBV11-1_TRBC2
<400> 512
   gccaccatga gcaccaggct tctctgctgg atggccctct gtctcctggg ggcagaactc 60
   tcagaagctg aagttgccca gtcccccaga tataagatta cagagaaaag ccaggctgtg 120
   gctttttggt gtgatcctat ttctggccat gctacccttt actggtaccg gcagatcctg 180
   ggacagggcc cggagcttct ggttcaattt caggatgaga gtgtagtaga tgattcacag 240
   ttgcctaagg atcgattttc tgcagagagg ctcaaaggag tagactccac tctcaagatc 300
   cagcctgcag agcttgggga ctcggccatg tatctttgca gagaccttgc ggccgcatag 360
   gtctcagtgt tcccacccga ggtcgctgtg tttgagccat cagaagcaga gatctcccac 420
   acccaaaagg ccacactggt atgcctggcc acaggcttct accccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttc acctccgagt cttaccagca aggggtcctg 780
   tctgccacca tcctctatga gatcttgcta gggaaggcca ccttgtatgc cgtgctggtc 840
   agtgccctcg tgctgatggc catggtcaag agaaaggatt ccagaggcta g 891
<210> 513
   <211> 891
   <212> DNA
   <213> Homo sapiens
<220>
   <223> V-C entry TRBV11-2_TRBC2
<400> 513
   gccaccatgg gcaccaggct cctctgctgg gcggccctct gtctcctggg agcagaactc 60
   acagaagctg gagttgccca gtctcccaga tataagatta tagagaaaag gcagagtgtg 120
   gctttttggt gcaatcctat atctggccat gctacccttt actggtacca gcagatcctg     180
   ggacagggcc caaagcttct gattcagttt cagaataacg gtgtagtgga tgattcacag     240
   ttgcctaagg atcgattttc tgcagagagg ctcaaaggag tagactccac tctcaagatc     300
   cagcctgcaa agcttgagga ctcggccgtg tatctttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g              891
<210> 514
   <211> 891
   <212> DNA
   <213> Homo sapiens
<220>
   <223> V-C entry TRBV11-3_TRBC2
<400> 514
   gccaccatgg gtaccaggct cctctgctgg gtggccttct gtctcctggt ggaagaactc      60
   atagaagctg gagtggttca gtctcccaga tataagatta tagagaaaaa acagcctgtg     120
   gctttttggt gcaatcctat ttctggccac aatacccttt actggtacct gcagaacttg     180
   ggacagggcc cggagcttct gattcgatat gagaatgagg aagcagtaga cgattcacag     240
   ttgcctaagg atcgattttc tgcagagagg ctcaaaggag tagactccac tctcaagatc     300
   cagcctgcag agcttgggga ctcggccgtg tatctttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g              891
<210> 515
   <211> 891
   <212> DNA
   <213> Homo sapiens
<220>
   <223> V-C entry TRBV12-3_TRBC2
<400> 515
   gccaccatgg actcctggac cttctgctgt gtgtcccttt gcatcctggt agcgaagcat      60
   acagatgctg gagttatcca gtcaccccgc catgaggtga cagagatggg acaagaagtg     120
   actctgagat gtaaaccaat ttcaggccac aactcccttt tctggtacag acagaccatg     180
   atgcggggac tggagttgct catttacttt aacaacaacg ttccgataga tgattcaggg     240
   atgcccgagg atcgattctc agctaagatg cctaatgcat cattctccac tctgaagatc     300
   cagccctcag aacccaggga ctcagctgtg tacttttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g               891
<210> 516
   <211> 891
   <212> DNA
   <213> Homo sapiens
<220>
   <223> V-C entry TRBV12-4_TRBC2
<400> 516
   gccaccatgg actcctggac cctctgctgt gtgtcccttt gcatcctggt agcaaagcac      60
   acagatgctg gagttatcca gtcaccccgg cacgaggtga cagagatggg acaagaagtg     120
   actctgagat gtaaaccaat ttcaggacac gactaccttt tctggtacag acagaccatg     180
   atgcggggac tggagttgct catttacttt aacaacaacg ttccgataga tgattcaggg     240
   atgcccgagg atcgattctc agctaagatg cctaatgcat cattctccac tctgaagatc     300
   cagccctcag aacccaggga ctcagctgtg tacttttgca gagaccttgc ggccgcatag      360
   gtctcagtgt tcccacccga ggtcgctgtg tttgagccat cagaagcaga gatctcccac      420
   acccaaaagg ccacactggt atgcctggcc acaggcttct accccgacca cgtggagctg      480
   agctggtggg tgaatgggaa ggaggtgcac agtggggtca gcacagaccc gcagcccctc      540
   aaggagcagc ccgccctcaa tgactccaga tactgcctga gcagccgcct gagggtgtcg      600
   gccaccttct ggcagaaccc ccgcaaccac ttccgctgtc aagtccagtt ctacgggctc      660
   tcggagaatg acgagtggac ccaggatagg gccaaacccg tcacccagat cgtcagcgcc      720
   gaggcctggg gtagagcaga ctgtggcttc acctccgagt cttaccagca aggggtcctg      780
   tctgccacca tcctctatga gatcttgcta gggaaggcca ccttgtatgc cgtgctggtc      840
   agtgccctcg tgctgatggc catggtcaag agaaaggatt ccagaggcta g              891
<210> 517
   <211> 891
   <212> DNA
   <213> Homo sapiens
<220>
   <223> V-C entry TRBV12-5_TRBC2
<400> 517
   gccaccatgg ccaccaggct cctctgctgt gtggttcttt gtctcctggg agaagagctt       60
   atagatgcta gagtcaccca gacaccaagg cacaaggtga cagagatggg acaagaagta      120
   acaatgagat gtcagccaat tttaggccac aatactgttt tctggtacag acagaccatg      180
   atgcaaggac tggagttgct ggcttacttc cgcaaccggg ctcctctaga tgattcgggg      240
   atgccgaagg atcgattctc agcagagatg cctgatgcaa ctttagccac tctgaagatc      300
   cagccctcag aacccaggga ctcagctgtg tacttttgca gagaccttgc ggccgcatag      360
   gtctcagtgt tcccacccga ggtcgctgtg tttgagccat cagaagcaga gatctcccac      420
   acccaaaagg ccacactggt atgcctggcc acaggcttct accccgacca cgtggagctg      480
   agctggtggg tgaatgggaa ggaggtgcac agtggggtca gcacagaccc gcagcccctc      540
   aaggagcagc ccgccctcaa tgactccaga tactgcctga gcagccgcct gagggtgtcg      600
   gccaccttct ggcagaaccc ccgcaaccac ttccgctgtc aagtccagtt ctacgggctc      660
   tcggagaatg acgagtggac ccaggatagg gccaaacccg tcacccagat cgtcagcgcc      720
   gaggcctggg gtagagcaga ctgtggcttc acctccgagt cttaccagca aggggtcctg      780
   tctgccacca tcctctatga gatcttgcta gggaaggcca ccttgtatgc cgtgctggtc      840
   agtgccctcg tgctgatggc catggtcaag agaaaggatt ccagaggcta g              891
<210> 518
   <211> 918
   <212> DNA
   <213> Homo sapiens
<220>
   <223> V-C entry TRBV13_TRBC2
<400> 518
   gccaccatgc ttagtcctga cctgcctgac tctgcctgga acaccaggct cctctgccat 60
   gtcatgcttt gtctcctggg agcagtttca gtggctgctg gagtcatcca gtccccaaga 120
   catctgatca aagaaaagag ggaaacagcc actctgaaat gctatcctat ccctagacac 180
   gacactgtct actggtacca gcagggtcca ggtcaggacc cccagttcct catttcgttt 240
   tatgaaaaga tgcagagcga taaaggaagc atccctgatc gattctcagc tcaacagttc 300
   agtgactatc attctgaact gaacatgagc tccttggagc tgggggactc agccctgtac 360
   ttttgcagag accttgcggc cgcataggtc tcagtgttcc cacccgaggt cgctgtgttt 420
   gagccatcag aagcagagat ctcccacacc caaaaggcca cactggtatg cctggccaca 480
   ggcttctacc ccgaccacgt ggagctgagc tggtgggtga atgggaagga ggtgcacagt 540
   ggggtcagca cagacccgca gcccctcaag gagcagcccg ccctcaatga ctccagatac 600
   tgcctgagca gccgcctgag ggtgtcggcc accttctggc agaacccccg caaccacttc 660
   cgctgtcaag tccagttcta cgggctctcg gagaatgacg agtggaccca ggatagggcc 720
   aaacccgtca cccagatcgt cagcgccgag gcctggggta gagcagactg tggcttcacc 780
   tccgagtctt accagcaagg ggtcctgtct gccaccatcc tctatgagat cttgctaggg 840
   aaggccacct tgtatgccgt gctggtcagt gccctcgtgc tgatggccat ggtcaagaga 900
   aaggattcca gaggctag 918
<210> 519
   <211> 891
   <212> DNA
   <213> Homo sapiens
<220>
   <223> V-C entry TRBV14_TRBC2
<400> 519
   gccaccatgg tttccaggct tctcagttta gtgtcccttt gtctcctggg agcaaagcac      60
   atagaagctg gagttactca gttccccagc cacagcgtaa tagagaaggg ccagactgtg     120
   actctgagat gtgacccaat ttctggacat gataatcttt attggtatcg acgtgttatg     180
   ggaaaagaaa taaaatttct gttacatttt gtgaaagagt ctaaacagga tgagtccggt     240
   atgcccaaca atcgattctt agctgaaagg actggaggga cgtattctac tctgaaggtg     300
   cagcctgcag aactggagga ttctggagtt tacttttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g              891
<210> 520
   <211> 888
   <212> DNA
   <213> Homo sapiens
<220>
   <223> V-C entry TRBV15_TRBC2
<400> 520
   gccaccatgg gtcctgggct tctccactgg atggcccttt gtctccttgg aacaggtcat      60
   ggggatgcca tggtcatcca gaacccaaga taccaggtta cccagtttgg aaagccagtg     120
   accctgagtt gttctcagac tttgaaccat aacgtcatgt actggtacca gcagaagtca     180
   agtcaggccc caaagctgct gttccactac tatgacaaag attttaacaa tgaagcagac     240
   acccctgata acttccaatc caggaggccg aacacttctt tctgctttct tgacatccgc     300
   tcaccaggcc tgggggacac agccatgtac ctttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 521
   <211> 891
   <212> DNA
   <213> Homo sapiens
<220>
   <223> V-C entry TRBV16_TRBC2
<400> 521
   gccaccatga gcccaatatt cacctgcatc acaatccttt gtctgctggc tgcaggttct 60
   cctggtgaag aagtcgccca gactccaaaa catcttgtca gaggggaagg acagaaagca 120
   aaattatatt gtgccccaat aaaaggacac agttatgttt tttggtacca acaggtcctg 180
   aaaaacgagt tcaagttctt gatttccttc cagaatgaaa atgtctttga tgaaacaggt 240
   atgcccaagg aaagattttc agctaagtgc ctcccaaatt caccctgtag ccttgagatc 300
   caggctacga agcttgagga ttcagcagtg tacttttgca gagaccttgc ggccgcatag 360
   gtctcagtgt tcccacccga ggtcgctgtg tttgagccat cagaagcaga gatctcccac 420
   acccaaaagg ccacactggt atgcctggcc acaggcttct accccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttc acctccgagt cttaccagca aggggtcctg 780
   tctgccacca tcctctatga gatcttgcta gggaaggcca ccttgtatgc cgtgctggtc 840
   agtgccctcg tgctgatggc catggtcaag agaaaggatt ccagaggcta g 891
<210> 522
   <211> 891
   <212> DNA
   <213> Homo sapiens
<220>
   <223> V-C entry TRBV18_TRBC2
<400> 522
   gccaccatgg acaccagagt actctgctgt gcggtcatct gccttctggg ggcaggactc 60
   tcaaatgccg gcgtcatgca gaacccaaga cacctggtca ggaggagggg acaggaggca 120
   agactgagat gcagcccaat gaaaggacac agtcatgttt actggtatcg gcagctccca 180
   gaggaaggtc tgaaattcat ggtttatctc cagaaagaaa atatcataga tgagtcagga 240
   atgccaaagg aacgattttc tgctgaattt cccaaagagg gccccagcat cctgaggatc 300
   cagcaggtag tgcgaggaga ttcggcagct tacttttgca gagaccttgc ggccgcatag 360
   gtctcagtgt tcccacccga ggtcgctgtg tttgagccat cagaagcaga gatctcccac 420
   acccaaaagg ccacactggt atgcctggcc acaggcttct accccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttc acctccgagt cttaccagca aggggtcctg 780
   tctgccacca tcctctatga gatcttgcta gggaaggcca ccttgtatgc cgtgctggtc 840
   agtgccctcg tgctgatggc catggtcaag agaaaggatt ccagaggcta g 891
<210> 523
   <211> 888
   <212> DNA
   <213> Homo sapiens
<220>
   <223> V-C entry TRBV19_TRBC2
<400> 523
   gccaccatga gcaaccaggt gctctgctgt gtggtccttt gtttcctggg agcaaacacc 60
   gtggatggtg gaatcactca gtccccaaag tacctgttca gaaaggaagg acagaatgtg 120
   accctgagtt gtgaacagaa tttgaaccac gatgccatgt actggtaccg acaggaccca 180
   gggcaagggc tgagattgat ctactactca cagatagtaa atgactttca gaaaggagat 240
   atagctgaag ggtacagcgt ctctcgggag aagaaggaat cctttcctct cactgtgaca 300
   tcggcccaaa agaacccgac agctttctat ctttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 524
   <211> 882
   <212> DNA
   <213> Homo sapiens
<220>
   <223> V-C entry TRBV20-1_TRBC2
<400> 524
   gccaccatgc tgctgcttct gctgcttctg gggccaggct ccgggcttgg tgctgtcgtc      60
   tctcaacatc cgagctgggt tatctgtaag agtggaacct ctgtgaagat cgagtgccgt     120
   tccctggact ttcaggccac aactatgttt tggtatcgtc agttcccgaa acagagtctc     180
   atgctgatgg caacttccaa tgagggctcc aaggccacat acgagcaagg cgtcgagaag     240
   gacaagtttc tcatcaacca tgcaagcctg accttgtcca ctctgacagt gaccagtgcc     300
   catcctgaag atagcagctt ctacatttgc agagaccttg cggccgcata ggtctcagtg     360
   ttcccacccg aggtcgctgt gtttgagcca tcagaagcag agatctccca cacccaaaag     420
   gccacactgg tatgcctggc cacaggcttc taccccgacc acgtggagct gagctggtgg     480
   gtgaatggga aggaggtgca cagtggggtc agcacagacc cgcagcccct caaggagcag     540
   cccgccctca atgactccag atactgcctg agcagccgcc tgagggtgtc ggccaccttc     600
   tggcagaacc cccgcaacca cttccgctgt caagtccagt tctacgggct ctcggagaat     660
   gacgagtgga cccaggatag ggccaaaccc gtcacccaga tcgtcagcgc cgaggcctgg     720
   ggtagagcag actgtggctt cacctccgag tcttaccagc aaggggtcct gtctgccacc     780
   atcctctatg agatcttgct agggaaggcc accttgtatg ccgtgctggt cagtgccctc     840
   gtgctgatgg ccatggtcaa gagaaaggat tccagaggct ag                        882
<210> 525
   <211> 888
   <212> DNA
   <213> Homo sapiens
<220>
   <223> V-C entry TRBV24-1_TRBC2
<400> 525
   gccaccatgg cctccctgct cttcttctgt ggggcctttt atctcctggg aacagggtcc 60
   atggatgctg atgttaccca gaccccaagg aataggatca caaagacagg aaagaggatt 120
   atgctggaat gttctcagac taagggtcat gatagaatgt actggtatcg acaagaccca 180
   ggactgggcc tacggttgat ctattactcc tttgatgtca aagatataaa caaaggagag 240
   atctctgatg gatacagtgt ctctcgacag gcacaggcta aattctccct gtccctagag 300
   tctgccatcc ccaaccagac agctctttac ttttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 526
   <211> 888
   <212> DNA
   <213> Homo sapiens
<220>
   <223> V-C entry TRBV25-1_TRBC2
<400> 526
   gccaccatga ctatcaggct cctctgctac atgggctttt attttctggg ggcaggcctc 60
   atggaagctg acatctacca gaccccaaga taccttgtta tagggacagg aaagaagatc 120
   actctggaat gttctcaaac catgggccat gacaaaatgt actggtatca acaagatcca 180
   ggaatggaac tacacctcat ccactattcc tatggagtta attccacaga gaagggagat 240
   ctttcctctg agtcaacagt ctccagaata aggacggagc attttcccct gaccctggag 300
   tctgccaggc cctcacatac ctctcagtac ctttgcagag accttgcggc cgcataggtc 360
   tcagtgttcc cacccgaggt cgctgtgttt gagccatcag aagcagagat ctcccacacc 420
   caaaaggcca cactggtatg cctggccaca ggcttctacc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggcttcacc tccgagtctt accagcaagg ggtcctgtct 780
   gccaccatcc tctatgagat cttgctaggg aaggccacct tgtatgccgt gctggtcagt 840
   gccctcgtgc tgatggccat ggtcaagaga aaggattcca gaggctag 888
<210> 527
   <211> 888
   <212> DNA
   <213> Homo sapiens
<220>
   <223> V-C entry TRBV27_TRBC2
<400> 527
   gccaccatgg gcccccagct ccttggctat gtggtccttt gccttctagg agcaggcccc 60
   ctggaagccc aagtgaccca gaacccaaga tacctcatca cagtgactgg aaagaagtta 120
   acagtgactt gttctcagaa tatgaaccat gagtatatgt cctggtatcg acaagaccca 180
   gggctgggct taaggcagat ctactattca atgaatgttg aggtgactga taagggagat 240
   gttcctgaag ggtacaaagt ctctcgaaaa gagaagagga atttccccct gatcctggag     300
   tcgcccagcc ccaaccagac ctctctgtac ttttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 528
   <211> 888
   <212> DNA
   <213> Homo sapiens
<220>
   <223> V-C entry TRBV28_TRBC2
<400> 528
   gccaccatgg gaatcaggct cctctgtcgt gtggcctttt gtttcctggc tgtaggcctc      60
   gtagatgtga aagtaaccca gagctcgaga tatctagtca aaaggacggg agagaaagtt     120
   tttctggaat gtgtccagga tatggaccat gaaaatatgt tctggtatcg acaagaccca     180
   ggtctggggc tacggctgat ctatttctca tatgatgtta aaatgaaaga aaaaggagat     240
   attcctgagg ggtacagtgt ctctagagag aagaaggagc gcttctccct gattctggag     300
   tccgccagca ccaaccagac atctatgtac ctttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 529
   <211> 882
   <212> DNA
   <213> Homo sapiens
<220>
   <223> V-C entry TRBV29-1_TRBC2
<400> 529
   gccaccatgc tgagtctact gctccttctc ctgggactag gctctgtgtt cagtgctgtc      60
   atctctcaaa agccaagcag ggatatctgt caacgtggaa cctccctgac gatccagtgt     120
   caagtcgata gccaagtcac catgatgttc tggtaccgtc agcaacctgg acagagcctg     180
   acactgatcg caactgcaaa tcagggctct gaggccacat atgagagtgg atttgtcatt     240
   gacaagtttc ccatcagccg cccaaaccta acattctcaa ctctgactgt gagcaacatg     300
   agccctgaag atagcagcat atatctttgc agagaccttg cggccgcata ggtctcagtg     360
   ttcccacccg aggtcgctgt gtttgagcca tcagaagcag agatctccca cacccaaaag     420
   gccacactgg tatgcctggc cacaggcttc taccccgacc acgtggagct gagctggtgg     480
   gtgaatggga aggaggtgca cagtggggtc agcacagacc cgcagcccct caaggagcag     540
   cccgccctca atgactccag atactgcctg agcagccgcc tgagggtgtc ggccaccttc     600
   tggcagaacc cccgcaacca cttccgctgt caagtccagt tctacgggct ctcggagaat     660
   gacgagtgga cccaggatag ggccaaaccc gtcacccaga tcgtcagcgc cgaggcctgg     720
   ggtagagcag actgtggctt cacctccgag tcttaccagc aaggggtcct gtctgccacc     780
   atcctctatg agatcttgct agggaaggcc accttgtatg ccgtgctggt cagtgccctc     840
   gtgctgatgg ccatggtcaa gagaaaggat tccagaggct ag                        882
<210> 530
   <211> 882
   <212> DNA
   <213> Homo sapiens
<220>
   <223> V-C entry TRBV30_TRBC2
<400> 530
   gccaccatgc tctgctctct ccttgccctt ctcctgggca ctttctttgg ggtcagatct      60
   cagactattc atcaatggcc agcgaccctg gtgcagcctg tgggcagccc gctctctctg     120
   gagtgcactg tggagggaac atcaaacccc aacctatact ggtaccgaca ggctgcaggc     180
   aggggcctcc agctgctctt ctactccgtt ggtattggcc agatcagctc tgaggtgccc     240
   cagaatctct cagcctccag accccaggac cggcagttca tcctgagttc taagaagctc     300
   cttctcagtg actctggctt ctatctttgc agagaccttg cggccgcata ggtctcagtg     360
   ttcccacccg aggtcgctgt gtttgagcca tcagaagcag agatctccca cacccaaaag     420
   gccacactgg tatgcctggc cacaggcttc taccccgacc acgtggagct gagctggtgg 480
   gtgaatggga aggaggtgca cagtggggtc agcacagacc cgcagcccct caaggagcag 540
   cccgccctca atgactccag atactgcctg agcagccgcc tgagggtgtc ggccaccttc 600
   tggcagaacc cccgcaacca cttccgctgt caagtccagt tctacgggct ctcggagaat 660
   gacgagtgga cccaggatag ggccaaaccc gtcacccaga tcgtcagcgc cgaggcctgg 720
   ggtagagcag actgtggctt cacctccgag tcttaccagc aaggggtcct gtctgccacc 780
   atcctctatg agatcttgct agggaaggcc accttgtatg ccgtgctggt cagtgccctc 840
   gtgctgatgg ccatggtcaa gagaaaggat tccagaggct ag 882
<210> 531
   <211> 885
   <212> DNA
   <213> Homo sapiens
<220>
   <223> V-C entry TRBV2_TRBC1
<400> 531
   gccaccatgg atacctggct cgtatgctgg gcaattttta gtctcttgaa agcaggactc 60
   acagaacctg aagtcaccca gactcccagc catcaggtca cacagatggg acaggaagtg 120
   atcttgcgct gtgtccccat ctctaatcac ttatacttct attggtacag acaaatcttg 180
   gggcagaaag tcgagtttct ggtttccttt tataataatg aaatctcaga gaagtctgaa 240
   atattcgatg atcaattctc agttgaaagg cctgatggat caaatttcac tctgaagatc 300
   cggtccacaa agctggagga ctcagccatg tacttttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 532
   <211> 882
   <212> DNA
   <213> Homo sapiens
<220>
   <223> V-C entry TRBV3-1_TRBC1
<400> 532
   gccaccatgg gctgcaggct cctctgctgt gtggtctttt gcctcctcca agcaggtccc      60
   ttggacacag ctgtttccca gactccaaaa tacctggtca cacagatggg aaacgacaag     120
   tccattaaat gtgaacaaaa tctgggccat gatactatgt attggtataa acaggactct     180
   aagaaatttc tgaagataat gtttagctac aataataagg agctcattat aaatgaaaca     240
   gttccaaatc gcttctcacc taaatctcca gacaaagctc acttaaatct tcacatcaat     300
   tccctggagc ttggtgactc tgctgtgtat ttttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 533
   <211> 882
   <212> DNA
   <213> Homo sapiens
<220>
   <223> V-C entry TRBV4-1_TRBC1
<400> 533
   gccaccatgg gctgcaggct gctctgctgt gcggttctct gtctcctggg agcagttccc 60
   atagacactg aagttaccca gacaccaaaa cacctggtca tgggaatgac aaataagaag 120
   tctttgaaat gtgaacaaca tatggggcac agggctatgt attggtacaa gcagaaagct 180
   aagaagccac cggagctcat gtttgtctac agctatgaga aactctctat aaatgaaagt 240
   gtgccaagtc gcttctcacc tgaatgcccc aacagctctc tcttaaacct tcacctacac 300
   gccctgcagc cagaagattc agccctgtat ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 534
   <211> 882
   <212> DNA
   <213> Homo sapiens
<220>
   <223> V-C entry TRBV4-2_TRBC1
<400> 534
   gccaccatgg gctgcaggct gctctgctgt gcggttctct gtctcctggg agcggtcccc 60
   atggaaacgg gagttacgca gacaccaaga cacctggtca tgggaatgac aaataagaag 120
   tctttgaaat gtgaacaaca tctggggcat aacgctatgt attggtacaa gcaaagtgct 180
   aagaagccac tggagctcat gtttgtctac aactttaaag aacagactga aaacaacagt 240
   gtgccaagtc gcttctcacc tgaatgcccc aacagctctc acttattcct tcacctacac 300
   accctgcagc cagaagattc ggccctgtat ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 535
   <211> 882
   <212> DNA
   <213> Homo sapiens
<220>
   <223> V-C entry TRBV4-3_TRBC1
<400> 535
   gccaccatgg gctgcaggct gctctgctgt gcggttctct gtctcctggg agcggtcccc 60
   atggaaacgg gagttacgca gacaccaaga cacctggtca tgggaatgac aaataagaag 120
   tctttgaaat gtgaacaaca tctgggtcat aacgctatgt attggtacaa gcaaagtgct      180
   aagaagccac tggagctcat gtttgtctac agtcttgaag aacgggttga aaacaacagt      240
   gtgccaagtc gcttctcacc tgaatgcccc aacagctctc acttattcct tcacctacac      300
   accctgcagc cagaagattc ggccctgtat ctttgcagag accttgcggc cgcataggtc      360
   tcagtgttcc cacccgaggt cgctgtgttt gagccatcag aagcagagat ctcccacacc      420
   caaaaggcca cactggtgtg cctggccaca ggcttcttcc ccgaccacgt ggagctgagc      480
   tggtgggtga atgggaagga ggtgcacagt ggggtcagca cagacccgca gcccctcaag      540
   gagcagcccg ccctcaatga ctccagatac tgcctgagca gccgcctgag ggtgtcggcc      600
   accttctggc agaacccccg caaccacttc cgctgtcaag tccagttcta cgggctctcg      660
   gagaatgacg agtggaccca ggatagggcc aaacccgtca cccagatcgt cagcgccgag      720
   gcctggggta gagcagactg tggctttacc tcggtgtcct accagcaagg ggtcctgtct      780
   gccaccatcc tctatgagat cctgctaggg aaggccaccc tgtatgctgt gctggtcagc      840
   gcccttgtgt tgatggccat ggtcaagaga aaggatttct ga                         882
<210> 536
   <211> 882
   <212> DNA
   <213> Homo sapiens
<220>
   <223> V-C entry TRBV5-1_TRBC1
<400> 536
   gccaccatgg gctccaggct gctctgttgg gtgctgcttt gtctcctggg agcaggccca 60
   gtaaaggctg gagtcactca aactccaaga tatctgatca aaacgagagg acagcaagtg 120
   acactgagct gctcccctat ctctgggcat aggagtgtat cctggtacca acagacccca 180
   ggacagggcc ttcagttcct ctttgaatac ttcagtgaga cacagagaaa caaaggaaac 240
   ttccctggtc gattctcagg gcgccagttc tctaactctc gctctgagat gaatgtgagc 300
   accttggagc tgggggactc ggccctttat ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 537
   <211> 882
   <212> DNA
   <213> Homo sapiens
<220>
   <223> V-C entry TRBV5-4_TRBC1
<400> 537
   gccaccatgg gccctgggct cctctgctgg gtgctgcttt gtctcctggg agcaggctca      60
   gtggagactg gagtcaccca aagtcccaca cacctgatca aaacgagagg acagcaagtg     120
   actctgagat gctcttctca gtctgggcac aacactgtgt cctggtacca acaggccctg     180
   ggtcaggggc cccagtttat ctttcagtat tatagggagg aagagaatgg cagaggaaac     240
   ttccctccta gattctcagg actccagttc cctaattata gctctgagct gaatgtgaac     300
   gccttggagc tggacgactc ggccctgtat ctttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 538
   <211> 882
   <212> DNA
   <213> Homo sapiens
<220>
   <223> V-C entry TRBV5-5_TRBC1
<400> 538
   gccaccatgg gccctgggct cctctgctgg gtgctgcttt gtctcctggg agcaggccca      60
   gtggacgctg gagtcaccca aagtcccaca cacctgatca aaacgagagg acagcaagtg     120
   actctgagat gctctcctat ctctgggcac aagagtgtgt cctggtacca acaggtcctg     180
   ggtcaggggc cccagtttat ctttcagtat tatgagaaag aagagagagg aagaggaaac     240
   ttccctgatc gattctcagc tcgccagttc cctaactata gctctgagct gaatgtgaac     300
   gccttgttgc tgggggactc ggccctgtat ctttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 539
   <211> 882
   <212> DNA
   <213> Homo sapiens
<220>
   <223> V-C entry TRBV5-6_TRBC1
<400> 539
   gccaccatgg gccccgggct cctctgctgg gcactgcttt gtctcctggg agcaggctta      60
   gtggacgctg gagtcaccca aagtcccaca cacctgatca aaacgagagg acagcaagtg     120
   actctgagat gctctcctaa gtctgggcat gacactgtgt cctggtacca acaggccctg     180
   ggtcaggggc cccagtttat ctttcagtat tatgaggagg aagagagaca gagaggcaac     240
   ttccctgatc gattctcagg tcaccagttc cctaactata gctctgagct gaatgtgaac     300
   gccttgttgc tgggggactc ggccctctat ctttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 540
   <211> 882
   <212> DNA
   <213> Homo sapiens
<220>
   <223> V-C entry TRBV5-7_TRBC1
<400> 540
   gccaccatgg gccccgggct cctctgctgg gtgctgcttt gtcccctagg agaaggccca      60
   gtggacgctg gagtcaccca aagtcccaca cacctgatca aaacgagagg acagcacgtg     120
   actctgagat gctctcctat ctctgggcac accagtgtgt cctcgtacca acaggccctg     180
   ggtcaggggc cccagtttat ctttcagtat tatgagaaag aagagagagg aagaggaaac     240
   ttccctgatc aattctcagg tcaccagttc cctaactata gctctgagct gaatgtgaac     300
   gccttgttgc taggggactc ggccctctat ctttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 541
   <211> 882
   <212> DNA
   <213> Homo sapiens
<220>
   <223> V-C entry TRBV5-8_TRBC1
<400> 541
   gccaccatgg gacccaggct cctcttctgg gcactgcttt gtctcctcgg aacaggccca 60
   gtggaggctg gagtcacaca aagtcccaca cacctgatca aaacgagagg acagcaagcg 120
   actctgagat gctctcctat ctctgggcac accagtgtgt actggtacca acaggccctg 180
   ggtctgggcc tccagttcct cctttggtat gacgagggtg aagagagaaa cagaggaaac 240
   ttccctccta gattttcagg tcgccagttc cctaattata gctctgagct gaatgtgaac 300
   gccttggagc tggaggactc ggccctgtat ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 542
   <211> 882
   <212> DNA
   <213> Homo sapiens
<220>
   <223> V-C entry TRBV6-1_TRBC1
<400> 542
   gccaccatga gcatcgggct cctgtgctgt gtggcctttt ctctcctgtg ggcaagtcca      60
   gtgaatgctg gtgtcactca gaccccaaaa ttccaggtcc tgaaaacagg acagagcatg     120
   acactgcagt gtgcccagga tatgaaccat aactccatgt actggtatcg acaagaccca     180
   ggcatgggac tgaggctgat ttattactca gcttctgagg gtaccactga caaaggagaa     240
   gtccccaatg gctacaatgt ctccagatta aacaaacggg agttctcgct caggctggag     300
   tcggctgctc cctcccagac atctgtgtac ttttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 543
   <211> 882
   <212> DNA
   <213> Homo sapiens
<220>
   <223> V-C entry TRBV6-3_TRBC1
<400> 543
   gccaccatga gcctcgggct cctgtgctgt ggggtctttt ctctcctgtg ggcaggtcca      60
   gtgaatgctg gtgtcactca gaccccaaaa ttccgggtcc tgaaaacagg acagagcatg     120
   acactgctgt gtgcccagga tatgaaccat gaatacatgt actggtatcg acaagaccca     180
   ggcatggggc tgaggctgat tcattactca gttggtgagg gtacaactgc caaaggagag     240
   gtccctgatg gctacaatgt ctccagatta aaaaaacaga atttcctgct ggggttggag     300
   tcggctgctc cctcccaaac atctgtgtac ttttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 544
   <211> 882
   <212> DNA
   <213> Homo sapiens
<220>
   <223> V-C entry TRBV6-4_TRBC1
<400> 544
   gccaccatga gaatcaggct cctgtgctgt gtggcctttt ctctcctgtg ggcaggtcca      60
   gtgattgctg ggatcaccca ggcaccaaca tctcagatcc tggcagcagg acggcgcatg     120
   acactgagat gtacccagga tatgagacat aatgccatgt actggtatag acaagatcta     180
   ggactggggc taaggctcat ccattattca aatactgcag gtaccactgg caaaggagaa     240
   gtccctgatg gttatagtgt ctccagagca aacacagatg atttccccct cacgttggcg     300
   tctgctgtac cctctcagac atctgtgtac ttttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 545
   <211> 882
   <212> DNA
   <213> Homo sapiens
<220>
   <223> V-C entry TRBV6-5_TRBC1
<400> 545
   gccaccatga gcatcggcct cctgtgctgt gcagccttgt ctctcctgtg ggcaggtcca      60
   gtgaatgctg gtgtcactca gaccccaaaa ttccaggtcc tgaaaacagg acagagcatg     120
   acactgcagt gtgcccagga tatgaaccat gaatacatgt cctggtatcg acaagaccca     180
   ggcatggggc tgaggctgat tcattactca gttggtgctg gtatcactga ccaaggagaa     240
   gtccccaatg gctacaatgt ctccagatca accacagagg atttcccgct caggctgctg     300
   tcggctgctc cctcccagac atctgtgtac ttttgcagag accttgcggc cgcataggtc     360
   tcagtgttcc cacccgaggt cgctgtgttt gagccatcag aagcagagat ctcccacacc     420
   caaaaggcca cactggtgtg cctggccaca ggcttcttcc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggctttacc tcggtgtcct accagcaagg ggtcctgtct     780
   gccaccatcc tctatgagat cctgctaggg aaggccaccc tgtatgctgt gctggtcagc     840
   gcccttgtgt tgatggccat ggtcaagaga aaggatttct ga                        882
<210> 546
   <211> 882
   <212> DNA
   <213> Homo sapiens
<220>
   <223> V-C entry TRBV6-6_TRBC1
<400> 546
   gccaccatga gcatcagcct cctgtgctgt gcagcctttc ctctcctgtg ggcaggtcca      60
   gtgaatgctg gtgtcactca gaccccaaaa ttccgcatcc tgaagatagg acagagcatg     120
   acactgcagt gtacccagga tatgaaccat aactacatgt actggtatcg acaagaccca 180
   ggcatggggc tgaagctgat ttattattca gttggtgctg gtatcactga taaaggagaa 240
   gtcccgaatg gctacaacgt ctccagatca accacagagg atttcccgct caggctggag 300
   ttggctgctc cctcccagac atctgtgtac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 547
   <211> 879
   <212> DNA
   <213> Homo sapiens
<220>
   <223> V-C entry TRBV6-8_TRBC1
<400> 547
   gccaccatga gcctcgggct cctgtgctgt gcggcctttt ctctcctgtg ggcaggtccc 60
   gtgaatgctg gtgtcactca gaccccaaaa ttccacatcc tgaaaacagg acagagcatg 120
   acactgcagt gtgcccagga tatgaaccat ggatacatgt cctggtatcg acaagaccca 180
   ggcatggggc tgagactgat ttactactca gctgctgctg gtactactga caaagaagtc 240
   cccaatggct acaatgtctc tagattaaac acagaggatt tcccactcag gctggtgtcg 300
   gctgctccct cccagacatc tgtgtacctt tgcagagacc ttgcggccgc ataggtctca 360
   gtgttcccac ccgaggtcgc tgtgtttgag ccatcagaag cagagatctc ccacacccaa 420
   aaggccacac tggtgtgcct ggccacaggc ttcttccccg accacgtgga gctgagctgg 480
   tgggtgaatg ggaaggaggt gcacagtggg gtcagcacag acccgcagcc cctcaaggag 540
   cagcccgccc tcaatgactc cagatactgc ctgagcagcc gcctgagggt gtcggccacc 600
   ttctggcaga acccccgcaa ccacttccgc tgtcaagtcc agttctacgg gctctcggag 660
   aatgacgagt ggacccagga tagggccaaa cccgtcaccc agatcgtcag cgccgaggcc 720
   tggggtagag cagactgtgg ctttacctcg gtgtcctacc agcaaggggt cctgtctgcc 780
   accatcctct atgagatcct gctagggaag gccaccctgt atgctgtgct ggtcagcgcc 840
   cttgtgttga tggccatggt caagagaaag gatttctga 879
<210> 548
   <211> 882
   <212> DNA
   <213> Homo sapiens
<220>
   <223> V-C entry TRBV6-9_TRBC1
<400> 548
   gccaccatga gcatcgggct cctgtgctgt gtggcctttt ctctcctgtg ggcaggtcca 60
   gtgaatgctg gtgtcactca gaccccaaaa ttccacatcc tgaaaacagg acagagcatg 120
   acactgcagt gtgcccagga tatgaaccat ggatacttgt cctggtatcg acaagaccca 180
   ggcatggggc tgaggcgcat tcattactca gttgctgctg gtatcactga caaaggagaa 240
   gtccccgatg gctacaatgt atccagatca aacacagagg atttcccgct caggctggag 300
   tcagctgctc cctcccagac atctgtatac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 549
   <211> 885
   <212> DNA
   <213> Homo sapiens
<220>
   <223> V-C entry TRBV7-2_TRBC1
<400> 549
   gccaccatgg gcaccaggct cctcttctgg gtggccttct gtctcctggg ggcagatcac 60
   acaggagctg gagtctccca gtcccccagt aacaaggtca cagagaaggg aaaggatgta 120
   gagctcaggt gtgatccaat ttcaggtcat actgcccttt actggtaccg acagagcctg 180
   gggcagggcc tggagttttt aatttacttc caaggcaaca gtgcaccaga caaatcaggg 240
   ctgcccagtg atcgcttctc tgcagagagg actgggggat ccgtctccac tctgacgatc 300
   cagcgcacac agcaggagga ctcggccgtg tatctttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 550
   <211> 885
   <212> DNA
   <213> Homo sapiens
<220>
   <223> V-C entry TRBV7-3_TRBC1
<400> 550
   gccaccatgg gcaccaggct cctctgctgg gcagccctgt gcctcctggg ggcagatcac 60
   acaggtgctg gagtctccca gacccccagt aacaaggtca cagagaaggg aaaatatgta 120
   gagctcaggt gtgatccaat ttcaggtcat actgcccttt actggtaccg acaaagcctg 180
   gggcagggcc cagagtttct aatttacttc caaggcacgg gtgcggcaga tgactcaggg 240
   ctgcccaacg atcggttctt tgcagtcagg cctgagggat ccgtctctac tctgaagatc 300
   cagcgcacag agcgggggga ctcagccgtg tatctttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 551
   <211> 885
   <212> DNA
   <213> Homo sapiens
<220>
   <223> V-C entry TRBV7-6_TRBC1
<400> 551
   gccaccatgg gcaccagtct cctatgctgg gtggtcctgg gtttcctagg gacagatcac      60
   acaggtgctg gagtctccca gtctcccagg tacaaagtca caaagagggg acaggatgta     120
   gctctcaggt gtgatccaat ttcgggtcat gtatcccttt attggtaccg acaggccctg     180
   gggcagggcc cagagtttct gacttacttc aattatgaag cccaacaaga caaatcaggg     240
   ctgcccaatg atcggttctc tgcagagagg cctgagggat ccatctccac tctgacgatc     300
   cagcgcacag agcagcggga ctcggccatg tatcgttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                     885
<210> 552
   <211> 885
   <212> DNA
   <213> Homo sapiens
<220>
   <223> V-C entry TRBV7-7_TRBC1
<400> 552
   gccaccatgg gtaccagtct cctatgctgg gtggtcctgg gtttcctagg gacagatcac 60
   acaggtgctg gagtctccca gtctcccagg tacaaagtca caaagagggg acaggatgta 120
   actctcaggt gtgatccaat ttcgagtcat gcaacccttt attggtatca acaggccctg 180
   gggcagggcc cagagtttct gacttacttc aattatgaag ctcaaccaga caaatcaggg 240
   ctgcccagtg atcggttctc tgcagagagg cctgagggat ccatctccac tctgacgatt 300
   cagcgcacag agcagcggga ctcagccatg tatcgttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 553
   <211> 885
   <212> DNA
   <213> Homo sapiens
<220>
   <223> V-C entry TRBV7-8_TRBC1
<400> 553
   gccaccatgg gcaccaggct cctctgctgg gtggtcctgg gtttcctagg gacagatcac      60
   acaggtgctg gagtctccca gtcccctagg tacaaagtcg caaagagagg acaggatgta     120
   gctctcaggt gtgatccaat ttcgggtcat gtatcccttt tttggtacca acaggccctg     180
   gggcaggggc cagagtttct gacttatttc cagaatgaag ctcaactaga caaatcgggg     240
   ctgcccagtg atcgcttctt tgcagaaagg cctgagggat ccgtctccac tctgaagatc     300
   cagcgcacac agcaggagga ctccgccgtg tatctttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                     885
<210> 554
   <211> 885
   <212> DNA
   <213> Homo sapiens
<220>
   <223> V-C entry TRBV7-9_TRBC1
<400> 554
   gccaccatgg gcaccagcct cctctgctgg atggccctgt gtctcctggg ggcagatcac 60
   gcagatactg gagtctccca gaaccccaga cacaagatca caaagagggg acagaatgta 120
   actttcaggt gtgatccaat ttctgaacac aaccgccttt attggtaccg acagaccctg 180
   gggcagggcc cagagtttct gacttacttc cagaatgaag ctcaactaga aaaatcaagg 240
   ctgctcagtg atcggttctc tgcagagagg cctaagggat ctttctccac cttggagatc 300
   cagcgcacag agcaggggga ctcggccatg tatctttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 555
   <211> 882
   <212> DNA
   <213> Homo sapiens
<220>
   <223> V-C entry TRBV9_TRBC1
<400> 555
   gccaccatgg gcttcaggct cctctgctgt gtggcctttt gtctcctggg agcaggccca 60
   gtggattctg gagtcacaca aaccccaaag cacctgatca cagcaactgg acagcgagtg 120
   acgctgagat gctcccctag gtctggtgac ctctctgtgt actggtacca acagagcctg 180
   gaccagggcc tccagttcct cattcagtat tataatggag aagagagagc aaaaggaaac 240
   attcttgaac gattctccgc acaacagttc cctgacttgc actctgaact aaacctgagc 300
   tctctggagc tgggggactc agctttgtac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 556
   <211> 882
   <212> DNA
   <213> Homo sapiens
<220>
   <223> V-C entry TRBV10-1_TRBC1
<400> 556
   gccaccatgg gcacgaggct cttcttctat gtggcccttt gtctgctgtg ggcaggacac 60
   agggatgctg aaatcaccca gagcccaaga cacaagatca cagagacagg aaggcaggtg 120
   accttggcgt gtcaccagac ttggaaccac aacaatatgt tctggtatcg acaagacctg 180
   ggacatgggc tgaggctgat ccattactca tatggtgttc aagacactaa caaaggagaa 240
   gtctcagatg gctacagtgt ctctagatca aacacagagg acctccccct cactctggag 300
   tctgctgcct cctcccagac atctgtatac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 557
   <211> 882
   <212> DNA
   <213> Homo sapiens
<220>
   <223> V-C entry TRBV10-2_TRBC1
<400> 557
   gccaccatgg gcaccaggct cttcttctat gtggcccttt gtctgctgtg ggcaggacac 60
   agggatgctg gaatcaccca gagcccaaga tacaagatca cagagacagg aaggcaggtg 120
   accttgatgt gtcaccagac ttggagccac agctatatgt tctggtatcg acaagacctg 180
   ggacatgggc tgaggctgat ctattactca gcagctgctg atattacaga taaaggagaa      240
   gtccccgatg gctatgttgt ctccagatcc aagacagaga atttccccct cactctggag      300
   tcagctaccc gctcccagac atctgtgtac ttttgcagag accttgcggc cgcataggtc      360
   tcagtgttcc cacccgaggt cgctgtgttt gagccatcag aagcagagat ctcccacacc      420
   caaaaggcca cactggtgtg cctggccaca ggcttcttcc ccgaccacgt ggagctgagc      480
   tggtgggtga atgggaagga ggtgcacagt ggggtcagca cagacccgca gcccctcaag      540
   gagcagcccg ccctcaatga ctccagatac tgcctgagca gccgcctgag ggtgtcggcc      600
   accttctggc agaacccccg caaccacttc cgctgtcaag tccagttcta cgggctctcg      660
   gagaatgacg agtggaccca ggatagggcc aaacccgtca cccagatcgt cagcgccgag      720
   gcctggggta gagcagactg tggctttacc tcggtgtcct accagcaagg ggtcctgtct      780
   gccaccatcc tctatgagat cctgctaggg aaggccaccc tgtatgctgt gctggtcagc      840
   gcccttgtgt tgatggccat ggtcaagaga aaggatttct ga                         882
<210> 558
   <211> 882
   <212> DNA
   <213> Homo sapiens
<220>
   <223> V-C entry TRBV10-3_TRBC1
<400> 558
   gccaccatgg gcacaaggtt gttcttctat gtggcccttt gtctcctgtg gacaggacac 60
   atggatgctg gaatcaccca gagcccaaga cacaaggtca cagagacagg aacaccagtg 120
   actctgagat gtcaccagac tgagaaccac cgctatatgt actggtatcg acaagacccg 180
   gggcatgggc tgaggctgat ccattactca tatggtgtta aagatactga caaaggagaa 240
   gtctcagatg gctatagtgt ctctagatca aagacagagg atttcctcct cactctggag 300
   tccgctacca gctcccagac atctgtgtac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 559
   <211> 885
   <212> DNA
   <213> Homo sapiens
<220>
   <223> V-C entry TRBV11-1_TRBC1
<400> 559
   gccaccatga gcaccaggct tctctgctgg atggccctct gtctcctggg ggcagaactc      60
   tcagaagctg aagttgccca gtcccccaga tataagatta cagagaaaag ccaggctgtg     120
   gctttttggt gtgatcctat ttctggccat gctacccttt actggtaccg gcagatcctg     180
   ggacagggcc cggagcttct ggttcaattt caggatgaga gtgtagtaga tgattcacag     240
   ttgcctaagg atcgattttc tgcagagagg ctcaaaggag tagactccac tctcaagatc     300
   cagcctgcag agcttgggga ctcggccatg tatctttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                     885
<210> 560
   <211> 885
   <212> DNA
   <213> Homo sapiens
<220>
   <223> V-C entry TRBV11-2_TRBC1
<400> 560
   gccaccatgg gcaccaggct cctctgctgg gcggccctct gtctcctggg agcagaactc      60
   acagaagctg gagttgccca gtctcccaga tataagatta tagagaaaag gcagagtgtg     120
   gctttttggt gcaatcctat atctggccat gctacccttt actggtacca gcagatcctg     180
   ggacagggcc caaagcttct gattcagttt cagaataacg gtgtagtgga tgattcacag     240
   ttgcctaagg atcgattttc tgcagagagg ctcaaaggag tagactccac tctcaagatc     300
   cagcctgcaa agcttgagga ctcggccgtg tatctttgca gagaccttgc ggccgcatag     360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 561
   <211> 885
   <212> DNA
   <213> Homo sapiens
<220>
   <223> V-C entry TRBV11-3_TRBC1
<400> 561
   gccaccatgg gtaccaggct cctctgctgg gtggccttct gtctcctggt ggaagaactc 60
   atagaagctg gagtggttca gtctcccaga tataagatta tagagaaaaa acagcctgtg      120
   gctttttggt gcaatcctat ttctggccac aatacccttt actggtacct gcagaacttg      180
   ggacagggcc cggagcttct gattcgatat gagaatgagg aagcagtaga cgattcacag 240
   ttgcctaagg atcgattttc tgcagagagg ctcaaaggag tagactccac tctcaagatc 300
   cagcctgcag agcttgggga ctcggccgtg tatctttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 562
   <211> 885
   <212> DNA
   <213> Homo sapiens
<220>
   <223> V-C entry TRBV12-3_TRBC1
<400> 562
   gccaccatgg actcctggac cttctgctgt gtgtcccttt gcatcctggt agcgaagcat      60
   acagatgctg gagttatcca gtcaccccgc catgaggtga cagagatggg acaagaagtg     120
   actctgagat gtaaaccaat ttcaggccac aactcccttt tctggtacag acagaccatg     180
   atgcggggac tggagttgct catttacttt aacaacaacg ttccgataga tgattcaggg     240
   atgcccgagg atcgattctc agctaagatg cctaatgcat cattctccac tctgaagatc     300
   cagccctcag aacccaggga ctcagctgtg tacttttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                     885
<210> 563
   <211> 885
   <212> DNA
   <213> Homo sapiens
<220>
   <223> V-C entry TRBV12-4_TRBC1
<400> 563
   gccaccatgg actcctggac cctctgctgt gtgtcccttt gcatcctggt agcaaagcac 60
   acagatgctg gagttatcca gtcaccccgg cacgaggtga cagagatggg acaagaagtg 120
   actctgagat gtaaaccaat ttcaggacac gactaccttt tctggtacag acagaccatg 180
   atgcggggac tggagttgct catttacttt aacaacaacg ttccgataga tgattcaggg 240
   atgcccgagg atcgattctc agctaagatg cctaatgcat cattctccac tctgaagatc 300
   cagccctcag aacccaggga ctcagctgtg tacttttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 564
   <211> 885
   <212> DNA
   <213> Homo sapiens
<220>
   <223> V-C entry TRBV12-5_TRBC1
<400> 564
   gccaccatgg ccaccaggct cctctgctgt gtggttcttt gtctcctggg agaagagctt      60
   atagatgcta gagtcaccca gacaccaagg cacaaggtga cagagatggg acaagaagta     120
   acaatgagat gtcagccaat tttaggccac aatactgttt tctggtacag acagaccatg     180
   atgcaaggac tggagttgct ggcttacttc cgcaaccggg ctcctctaga tgattcgggg     240
   atgccgaagg atcgattctc agcagagatg cctgatgcaa ctttagccac tctgaagatc     300
   cagccctcag aacccaggga ctcagctgtg tacttttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                     885
<210> 565
   <211> 912
   <212> DNA
   <213> Homo sapiens
<220>
   <223> V-C entry TRBV13_TRBC1
<400> 565
   gccaccatgc ttagtcctga cctgcctgac tctgcctgga acaccaggct cctctgccat 60
   gtcatgcttt gtctcctggg agcagtttca gtggctgctg gagtcatcca gtccccaaga 120
   catctgatca aagaaaagag ggaaacagcc actctgaaat gctatcctat ccctagacac 180
   gacactgtct actggtacca gcagggtcca ggtcaggacc cccagttcct catttcgttt 240
   tatgaaaaga tgcagagcga taaaggaagc atccctgatc gattctcagc tcaacagttc 300
   agtgactatc attctgaact gaacatgagc tccttggagc tgggggactc agccctgtac 360
   ttttgcagag accttgcggc cgcataggtc tcagtgttcc cacccgaggt cgctgtgttt 420
   gagccatcag aagcagagat ctcccacacc caaaaggcca cactggtgtg cctggccaca 480
   ggcttcttcc ccgaccacgt ggagctgagc tggtgggtga atgggaagga ggtgcacagt 540
   ggggtcagca cagacccgca gcccctcaag gagcagcccg ccctcaatga ctccagatac 600
   tgcctgagca gccgcctgag ggtgtcggcc accttctggc agaacccccg caaccacttc 660
   cgctgtcaag tccagttcta cgggctctcg gagaatgacg agtggaccca ggatagggcc 720
   aaacccgtca cccagatcgt cagcgccgag gcctggggta gagcagactg tggctttacc 780
   tcggtgtcct accagcaagg ggtcctgtct gccaccatcc tctatgagat cctgctaggg 840
   aaggccaccc tgtatgctgt gctggtcagc gcccttgtgt tgatggccat ggtcaagaga 900
   aaggatttct ga 912
<210> 566
   <211> 885
   <212> DNA
   <213> Homo sapiens
<220>
   <223> V-C entry TRBV14_TRBC1
<400> 566
   gccaccatgg tttccaggct tctcagttta gtgtcccttt gtctcctggg agcaaagcac      60
   atagaagctg gagttactca gttccccagc cacagcgtaa tagagaaggg ccagactgtg     120
   actctgagat gtgacccaat ttctggacat gataatcttt attggtatcg acgtgttatg     180
   ggaaaagaaa taaaatttct gttacatttt gtgaaagagt ctaaacagga tgagtccggt     240
   atgcccaaca atcgattctt agctgaaagg actggaggga cgtattctac tctgaaggtg     300
   cagcctgcag aactggagga ttctggagtt tacttttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                    885
<210> 567
   <211> 882
   <212> DNA
   <213> Homo sapiens
<220>
   <223> V-C entry TRBV15_TRBC1
<400> 567
   gccaccatgg gtcctgggct tctccactgg atggcccttt gtctccttgg aacaggtcat 60
   ggggatgcca tggtcatcca gaacccaaga taccaggtta cccagtttgg aaagccagtg 120
   accctgagtt gttctcagac tttgaaccat aacgtcatgt actggtacca gcagaagtca 180
   agtcaggccc caaagctgct gttccactac tatgacaaag attttaacaa tgaagcagac 240
   acccctgata acttccaatc caggaggccg aacacttctt tctgctttct tgacatccgc 300
   tcaccaggcc tgggggacac agccatgtac ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 568
   <211> 885
   <212> DNA
   <213> Homo sapiens
<220>
   <223> V-C entry TRBV16_TRBC1
<400> 568
   gccaccatga gcccaatatt cacctgcatc acaatccttt gtctgctggc tgcaggttct 60
   cctggtgaag aagtcgccca gactccaaaa catcttgtca gaggggaagg acagaaagca 120
   aaattatatt gtgccccaat aaaaggacac agttatgttt tttggtacca acaggtcctg      180
   aaaaacgagt tcaagttctt gatttccttc cagaatgaaa atgtctttga tgaaacaggt      240
   atgcccaagg aaagattttc agctaagtgc ctcccaaatt caccctgtag ccttgagatc      300
   caggctacga agcttgagga ttcagcagtg tacttttgca gagaccttgc ggccgcatag      360
   gtctcagtgt tcccacccga ggtcgctgtg tttgagccat cagaagcaga gatctcccac      420
   acccaaaagg ccacactggt gtgcctggcc acaggcttct tccccgacca cgtggagctg      480
   agctggtggg tgaatgggaa ggaggtgcac agtggggtca gcacagaccc gcagcccctc      540
   aaggagcagc ccgccctcaa tgactccaga tactgcctga gcagccgcct gagggtgtcg      600
   gccaccttct ggcagaaccc ccgcaaccac ttccgctgtc aagtccagtt ctacgggctc      660
   tcggagaatg acgagtggac ccaggatagg gccaaacccg tcacccagat cgtcagcgcc      720
   gaggcctggg gtagagcaga ctgtggcttt acctcggtgt cctaccagca aggggtcctg      780
   tctgccacca tcctctatga gatcctgcta gggaaggcca ccctgtatgc tgtgctggtc      840
   agcgcccttg tgttgatggc catggtcaag agaaaggatt tctga                      885
<210> 569
   <211> 885
   <212> DNA
   <213> Homo sapiens
<220>
   <223> V-C entry TRBV18_TRBC1
<400> 569
   gccaccatgg acaccagagt actctgctgt gcggtcatct gccttctggg ggcaggactc      60
   tcaaatgccg gcgtcatgca gaacccaaga cacctggtca ggaggagggg acaggaggca     120
   agactgagat gcagcccaat gaaaggacac agtcatgttt actggtatcg gcagctccca     180
   gaggaaggtc tgaaattcat ggtttatctc cagaaagaaa atatcataga tgagtcagga     240
   atgccaaagg aacgattttc tgctgaattt cccaaagagg gccccagcat cctgaggatc     300
   cagcaggtag tgcgaggaga ttcggcagct tacttttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                     885
<210> 570
   <211> 882
   <212> DNA
   <213> Homo sapiens
<220>
   <223> V-C entry TRBV19_TRBC1
<400> 570
   gccaccatga gcaaccaggt gctctgctgt gtggtccttt gtttcctggg agcaaacacc 60
   gtggatggtg gaatcactca gtccccaaag tacctgttca gaaaggaagg acagaatgtg 120
   accctgagtt gtgaacagaa tttgaaccac gatgccatgt actggtaccg acaggaccca 180
   gggcaagggc tgagattgat ctactactca cagatagtaa atgactttca gaaaggagat 240
   atagctgaag ggtacagcgt ctctcgggag aagaaggaat cctttcctct cactgtgaca 300
   tcggcccaaa agaacccgac agctttctat ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 571
   <211> 876
   <212> DNA
   <213> Homo sapiens
<220>
   <223> V-C entry TRBV20-1_TRBC1
<400> 571
   gccaccatgc tgctgcttct gctgcttctg gggccaggct ccgggcttgg tgctgtcgtc 60
   tctcaacatc cgagctgggt tatctgtaag agtggaacct ctgtgaagat cgagtgccgt 120
   tccctggact ttcaggccac aactatgttt tggtatcgtc agttcccgaa acagagtctc 180
   atgctgatgg caacttccaa tgagggctcc aaggccacat acgagcaagg cgtcgagaag 240
   gacaagtttc tcatcaacca tgcaagcctg accttgtcca ctctgacagt gaccagtgcc 300
   catcctgaag atagcagctt ctacatttgc agagaccttg cggccgcata ggtctcagtg 360
   ttcccacccg aggtcgctgt gtttgagcca tcagaagcag agatctccca cacccaaaag 420
   gccacactgg tgtgcctggc cacaggcttc ttccccgacc acgtggagct gagctggtgg 480
   gtgaatggga aggaggtgca cagtggggtc agcacagacc cgcagcccct caaggagcag 540
   cccgccctca atgactccag atactgcctg agcagccgcc tgagggtgtc ggccaccttc 600
   tggcagaacc cccgcaacca cttccgctgt caagtccagt tctacgggct ctcggagaat 660
   gacgagtgga cccaggatag ggccaaaccc gtcacccaga tcgtcagcgc cgaggcctgg 720
   ggtagagcag actgtggctt tacctcggtg tcctaccagc aaggggtcct gtctgccacc 780
   atcctctatg agatcctgct agggaaggcc accctgtatg ctgtgctggt cagcgccctt 840
   gtgttgatgg ccatggtcaa gagaaaggat ttctga 876
<210> 572
   <211> 882
   <212> DNA
   <213> Homo sapiens
<220>
   <223> V-C entry TRBV24-1_TRBC1
<400> 572
   gccaccatgg cctccctgct cttcttctgt ggggcctttt atctcctggg aacagggtcc 60
   atggatgctg atgttaccca gaccccaagg aataggatca caaagacagg aaagaggatt 120
   atgctggaat gttctcagac taagggtcat gatagaatgt actggtatcg acaagaccca 180
   ggactgggcc tacggttgat ctattactcc tttgatgtca aagatataaa caaaggagag 240
   atctctgatg gatacagtgt ctctcgacag gcacaggcta aattctccct gtccctagag 300
   tctgccatcc ccaaccagac agctctttac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 573
   <211> 882
   <212> DNA
   <213> Homo sapiens
<220>
   <223> V-C entry TRBV25-1_TRBC1
<400> 573
   gccaccatga ctatcaggct cctctgctac atgggctttt attttctggg ggcaggcctc 60
   atggaagctg acatctacca gaccccaaga taccttgtta tagggacagg aaagaagatc 120
   actctggaat gttctcaaac catgggccat gacaaaatgt actggtatca acaagatcca 180
   ggaatggaac tacacctcat ccactattcc tatggagtta attccacaga gaagggagat 240
   ctttcctctg agtcaacagt ctccagaata aggacggagc attttcccct gaccctggag 300
   tctgccaggc cctcacatac ctctcagtac ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 574
   <211> 882
   <212> DNA
   <213> Homo sapiens
<220>
   <223> V-C entry TRBV27_TRBC1
<400> 574
   gccaccatgg gcccccagct ccttggctat gtggtccttt gccttctagg agcaggcccc 60
   ctggaagccc aagtgaccca gaacccaaga tacctcatca cagtgactgg aaagaagtta 120
   acagtgactt gttctcagaa tatgaaccat gagtatatgt cctggtatcg acaagaccca 180
   gggctgggct taaggcagat ctactattca atgaatgttg aggtgactga taagggagat 240
   gttcctgaag ggtacaaagt ctctcgaaaa gagaagagga atttccccct gatcctggag 300
   tcgcccagcc ccaaccagac ctctctgtac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 575
   <211> 882
   <212> DNA
   <213> Homo sapiens
<220>
   <223> V-C entry TRBV28_TRBC1
<400> 575
   gccaccatgg gaatcaggct cctctgtcgt gtggcctttt gtttcctggc tgtaggcctc 60
   gtagatgtga aagtaaccca gagctcgaga tatctagtca aaaggacggg agagaaagtt 120
   tttctggaat gtgtccagga tatggaccat gaaaatatgt tctggtatcg acaagaccca 180
   ggtctggggc tacggctgat ctatttctca tatgatgtta aaatgaaaga aaaaggagat 240
   attcctgagg ggtacagtgt ctctagagag aagaaggagc gcttctccct gattctggag 300
   tccgccagca ccaaccagac atctatgtac ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 576
   <211> 876
   <212> DNA
   <213> Homo sapiens
<220>
   <223> V-C entry TRBV29-1_TRBC1
<400> 576
   gccaccatgc tgagtctact gctccttctc ctgggactag gctctgtgtt cagtgctgtc      60
   atctctcaaa agccaagcag ggatatctgt caacgtggaa cctccctgac gatccagtgt     120
   caagtcgata gccaagtcac catgatgttc tggtaccgtc agcaacctgg acagagcctg     180
   acactgatcg caactgcaaa tcagggctct gaggccacat atgagagtgg atttgtcatt     240
   gacaagtttc ccatcagccg cccaaaccta acattctcaa ctctgactgt gagcaacatg     300
   agccctgaag atagcagcat atatctttgc agagaccttg cggccgcata ggtctcagtg     360
   ttcccacccg aggtcgctgt gtttgagcca tcagaagcag agatctccca cacccaaaag     420
   gccacactgg tgtgcctggc cacaggcttc ttccccgacc acgtggagct gagctggtgg     480
   gtgaatggga aggaggtgca cagtggggtc agcacagacc cgcagcccct caaggagcag     540
   cccgccctca atgactccag atactgcctg agcagccgcc tgagggtgtc ggccaccttc     600
   tggcagaacc cccgcaacca cttccgctgt caagtccagt tctacgggct ctcggagaat     660
   gacgagtgga cccaggatag ggccaaaccc gtcacccaga tcgtcagcgc cgaggcctgg     720
   ggtagagcag actgtggctt tacctcggtg tcctaccagc aaggggtcct gtctgccacc     780
   atcctctatg agatcctgct agggaaggcc accctgtatg ctgtgctggt cagcgccctt     840
   gtgttgatgg ccatggtcaa gagaaaggat ttctga                               876
<210> 577
   <211> 876
   <212> DNA
   <213> Homo sapiens
<220>
   <223> V-C entry TRBV30_TRBC1
<400> 577
   gccaccatgc tctgctctct ccttgccctt ctcctgggca ctttctttgg ggtcagatct 60
   cagactattc atcaatggcc agcgaccctg gtgcagcctg tgggcagccc gctctctctg 120
   gagtgcactg tggagggaac atcaaacccc aacctatact ggtaccgaca ggctgcaggc 180
   aggggcctcc agctgctctt ctactccgtt ggtattggcc agatcagctc tgaggtgccc 240
   cagaatctct cagcctccag accccaggac cggcagttca tcctgagttc taagaagctc 300
   cttctcagtg actctggctt ctatctttgc agagaccttg cggccgcata ggtctcagtg 360
   ttcccacccg aggtcgctgt gtttgagcca tcagaagcag agatctccca cacccaaaag 420
   gccacactgg tgtgcctggc cacaggcttc ttccccgacc acgtggagct gagctggtgg 480
   gtgaatggga aggaggtgca cagtggggtc agcacagacc cgcagcccct caaggagcag 540
   cccgccctca atgactccag atactgcctg agcagccgcc tgagggtgtc ggccaccttc 600
   tggcagaacc cccgcaacca cttccgctgt caagtccagt tctacgggct ctcggagaat 660
   gacgagtgga cccaggatag ggccaaaccc gtcacccaga tcgtcagcgc cgaggcctgg 720
   ggtagagcag actgtggctt tacctcggtg tcctaccagc aaggggtcct gtctgccacc 780
   atcctctatg agatcctgct agggaaggcc accctgtatg ctgtgctggt cagcgccctt 840
   gtgttgatgg ccatggtcaa gagaaaggat ttctga 876
<210> 578
   <211> 64
   <212> DNA
   <213> Artificial Sequence
<220>
   <223> TRBJ-C1_receiving_F1
<400> 578
   aattcggtct cgaagtcttc tgcggccgct gaagacacag gacctgaaca aggtgttgag 60
   accc 64
<210> 579
   <211> 64
   <212> DNA
   <213> Artificial Sequence
<220>
   <223> TKBJ-C2_receiving_F1
<400> 579
   aattcggtct cgaagtcttc tgcggccgct gaagacacag gacctgaaaa acgtgttgag 60
   accc 64
<210> 580
   <211> 64
   <212> DNA
   <213> Artificial Sequence
<220>
   <223> TRBJ-C1_receiving_R1
<400> 580
   tcgagggtct caacaccttg ttcaggtcct gtgtcttcag cggccgcaga agacttcgag 60
   accg 64
<210> 581
   <211> 64
   <212> DNA
   <213> Artificial Sequence
<220>
   <223> TRBJ-C2_receiving_R1
<400> 581
   tcgagggtct caacacgttt ttcaggtcct gtgtcttcag cggccgcaga agacttcgag 60
   accg 64
<210> 582
   <211> 2366
   <212> DNA
   <213> Artificial Sequence
<220>
   <223> TRB J_C1 receiving cassette vector
<400> 582
   gccagggttt tcccagtcac gacgttgtaa aacgacggcc agtgagcgcg acgtaatacg      60
   actcactata gggcgaattg gcggaaggcc gtcaaggccg catgaattcg gtctcgaagt     120
   cttctgcggc cgctgaagac acaggacctg aacaaggtgt tgagaccctc gagctgggcc     180
   tcatgggcct tccgctcact gcccgctttc cagtcgggaa acctgtcgtg ccagctgcat     240
   taacatggtc atagctgttt ccttgcgtat tgggcgctct ccgcttcctc gctcactgac     300
   tcgctgcgct cggtcgttcg ggtaaagcct ggggtgccta atgagcaaaa ggccagcaaa     360
   aggccaggaa ccgtaaaaag gccgcgttgc tggcgttttt ccataggctc cgcccccctg     420
   acgagcatca caaaaatcga cgctcaagtc agaggtggcg aaacccgaca ggactataaa     480
   gataccaggc gtttccccct ggaagctccc tcgtgcgctc tcctgttccg accctgccgc     540
   ttaccggata cctgtccgcc tttctccctt cgggaagcgt ggcgctttct catagctcac     600
   gctgtaggta tctcagttcg gtgtaggtcg ttcgctccaa gctgggctgt gtgcacgaac     660
   cccccgttca gcccgaccgc tgcgccttat ccggtaacta tcgtcttgag tccaacccgg     720
   taagacacga cttatcgcca ctggcagcag ccactggtaa caggattagc agagcgaggt     780
   atgtaggcgg tgctacagag ttcttgaagt ggtggcctaa ctacggctac actagaagaa     840
   cagtatttgg tatctgcgct ctgctgaagc cagttacctt cggaaaaaga gttggtagct     900
   cttgatccgg caaacaaacc accgctggta gcggtggttt ttttgtttgc aagcagcaga     960
   ttacgcgcag aaaaaaagga tctcaagaag atcctttgat cttttctacg gggtctgacg    1020
   ctcagtggaa cgaaaactca cgttaaggga ttttggtcat gagattatca aaaaggatct    1080
   tcacctagat ccttttaaat taaaaatgaa gttttaaatc aatctaaagt atatatgagt    1140
   aaacttggtc tgacagttag aaaaattcgt ccagcatcag atgaaattgc agtttgttca    1200
   tgtccgggtt atcaatacca tatttctgga acagacgttt ctgcaggctc gggctaaatt    1260
   cacccagaca attccacaga attgccagat cctgataacg atctgcaata ccaacacgac    1320
   caacatcaat gcagccaatc agtttaccct catcaaaaat caggttatcc aggctaaaat    1380
   caccatgggt aacaacgcta tccggactaa acggcagcag tttatgcatt tctttccaaa    1440
   cctgttcaac aggccaacca ttacgttcat catcaaaatc gcttgcatca accagaccat    1500
   tattcatacg gctctgtgcc tgtgccagac gaaaaacacg atcgctatta aacggacaat    1560
   tacaaaccgg aatgctatgc agacgacgca gaaaaactgc cagtgcatca acaatatttt 1620
   cgcctgaatc cggatattct tccagaacct gaaatgcggt tttacccgga attgcggtgg 1680
   tcagcagcca tgcatcatcc ggtgtacgaa taaaatgttt aatggtcggc agcggcataa 1740
   attcggtcag ccaattcaga cgaaccattt catcggtcac atcatttgca acgctacctt 1800
   taccatgttt cagaaacagt tccggtgcat ccggtttacc atacagacga taaatggttg 1860
   caccgctctg accaacatta tcacgtgccc atttatagcc atacagatct gcatccatat 1920
   tgctattcag acgcggacgg ctacagctgg tttcacgctg aatatggctc atactcttcc 1980
   tttttcaata ttattgaagc atttatcagg gttattgtct catgagcgga tacatatttg 2040
   aatgtattta gaaaaataaa caaatagggg ttccgcgcac atttccccga aaagtgccac 2100
   ctaaattgta agcgttaata ttttgttaaa attcgcgtta aatttttgtt aaatcagctc 2160
   attttttaac caataggccg aaatcggcaa aatcccttat aaatcaaaag aatagaccga 2220
   gatagggttg agtggccgct acagggcgct cccattcgcc attcaggctg cgcaactgtt 2280
   gggaagggcg tttcggtgcg ggcctcttcg ctattacgcc agctggcgaa agggggatgt 2340
   gctgcaaggc gattaagttg ggtaac 2366
<210> 583
   <211> 2366
   <212> DNA
   <213> Artificial Sequence
<220>
   <223> TRB J_C2 receiving cassette vector
<400> 583
   gccagggttt tcccagtcac gacgttgtaa aacgacggcc agtgagcgcg acgtaatacg      60
   actcactata gggcgaattg gcggaaggcc gtcaaggccg catgaattcg gtctcgaagt     120
   cttctgcggc cgctgaagac acaggacctg aaaaacgtgt tgagaccctc gagctgggcc     180
   tcatgggcct tccgctcact gcccgctttc cagtcgggaa acctgtcgtg ccagctgcat     240
   taacatggtc atagctgttt ccttgcgtat tgggcgctct ccgcttcctc gctcactgac     300
   tcgctgcgct cggtcgttcg ggtaaagcct ggggtgccta atgagcaaaa ggccagcaaa     360
   aggccaggaa ccgtaaaaag gccgcgttgc tggcgttttt ccataggctc cgcccccctg     420
   acgagcatca caaaaatcga cgctcaagtc agaggtggcg aaacccgaca ggactataaa     480
   gataccaggc gtttccccct ggaagctccc tcgtgcgctc tcctgttccg accctgccgc     540
   ttaccggata cctgtccgcc tttctccctt cgggaagcgt ggcgctttct catagctcac     600
   gctgtaggta tctcagttcg gtgtaggtcg ttcgctccaa gctgggctgt gtgcacgaac     660
   cccccgttca gcccgaccgc tgcgccttat ccggtaacta tcgtcttgag tccaacccgg     720
   taagacacga cttatcgcca ctggcagcag ccactggtaa caggattagc agagcgaggt     780
   atgtaggcgg tgctacagag ttcttgaagt ggtggcctaa ctacggctac actagaagaa 840
   cagtatttgg tatctgcgct ctgctgaagc cagttacctt cggaaaaaga gttggtagct 900
   cttgatccgg caaacaaacc accgctggta gcggtggttt ttttgtttgc aagcagcaga 960
   ttacgcgcag aaaaaaagga tctcaagaag atcctttgat cttttctacg gggtctgacg 1020
   ctcagtggaa cgaaaactca cgttaaggga ttttggtcat gagattatca aaaaggatct 1080
   tcacctagat ccttttaaat taaaaatgaa gttttaaatc aatctaaagt atatatgagt 1140
   aaacttggtc tgacagttag aaaaattcgt ccagcatcag atgaaattgc agtttgttca 1200
   tgtccgggtt atcaatacca tatttctgga acagacgttt ctgcaggctc gggctaaatt 1260
   cacccagaca attccacaga attgccagat cctgataacg atctgcaata ccaacacgac 1320
   caacatcaat gcagccaatc agtttaccct catcaaaaat caggttatcc aggctaaaat 1380
   caccatgggt aacaacgcta tccggactaa acggcagcag tttatgcatt tctttccaaa 1440
   cctgttcaac aggccaacca ttacgttcat catcaaaatc gcttgcatca accagaccat 1500
   tattcatacg gctctgtgcc tgtgccagac gaaaaacacg atcgctatta aacggacaat 1560
   tacaaaccgg aatgctatgc agacgacgca gaaaaactgc cagtgcatca acaatatttt 1620
   cgcctgaatc cggatattct tccagaacct gaaatgcggt tttacccgga attgcggtgg 1680
   tcagcagcca tgcatcatcc ggtgtacgaa taaaatgttt aatggtcggc agcggcataa 1740
   attcggtcag ccaattcaga cgaaccattt catcggtcac atcatttgca acgctacctt 1800
   taccatgttt cagaaacagt tccggtgcat ccggtttacc atacagacga taaatggttg 1860
   caccgctctg accaacatta tcacgtgccc atttatagcc atacagatct gcatccatat 1920
   tgctattcag acgcggacgg ctacagctgg tttcacgctg aatatggctc atactcttcc 1980
   tttttcaata ttattgaagc atttatcagg gttattgtct catgagcgga tacatatttg 2040
   aatgtattta gaaaaataaa caaatagggg ttccgcgcac atttccccga aaagtgccac 2100
   ctaaattgta agcgttaata ttttgttaaa attcgcgtta aatttttgtt aaatcagctc 2160
   attttttaac caataggccg aaatcggcaa aatcccttat aaatcaaaag aatagaccga 2220
   gatagggttg agtggccgct acagggcgct cccattcgcc attcaggctg cgcaactgtt 2280
   gggaagggcg tttcggtgcg ggcctcttcg ctattacgcc agctggcgaa agggggatgt 2340
   gctgcaaggc gattaagttg ggtaac 2366
<210> 584
   <211> 35
   <212> DNA
   <213> Homo sapiens
<220>
   <223> TRBJ1-1*01_BB-S_F1
<400> 584
   ctcgtttgga caaggcacca gactcacagt tgtag 35
<210> 585
   <211> 35
   <212> DNA
   <213> Homo sapiens
<220>
   <223> TRBJ1-2*01_BB-S_F1
<400> 585
   ctcgtttggt tcggggacca ggttaaccgt tgtag 35
<210> 586
   <211> 35
   <212> DNA
   <213> Homo sapiens
<220>
   <223> TRBJ1-3*01_BB-S_F1
<400> 586
   ctcgtttgga gagggaagtt ggctcactgt tgtag 35
<210> 587
   <211> 35
   <212> DNA
   <213> Homo sapiens
<220>
   <223> TRBJ1-4*01_BB-S_F1
<400> 587
   ctcgtttggc agtggaaccc agctctctgt cttgg 35
<210> 588
   <211> 35
   <212> DNA
   <213> Homo sapiens
<220>
   <223> TRBJ1-5*01_BB-S_F1
<400> 588
   ctcgtttggt gatgggactc gactctccat cctag 35
<210> 589
   <211> 35
   <212> DNA
   <213> Homo sapiens
<220>
   <223> TRBJ1-6*01_BB-S_F1
<400> 589
   ctcgtttggg aacgggacca ggctcactgt gacag 35
<210> 590
   <211> 35
   <212> DNA
   <213> Homo sapiens
<220>
   <223> TRBJ2-1*01_BB-S_F1
<400> 590
   ctcgtttggg ccagggacac ggctcaccgt gctag 35
<210> 591
   <211> 35
   <212> DNA
   <213> Homo sapiens
<220>
   <223> TRBJ2-2*01_BB-S_F1
<400> 591
   ctcgtttgga gaaggctcta ggctgaccgt actgg 35
<210> 592
   <211> 35
   <212> DNA
   <213> Homo sapiens
<220>
   <223> TRBJ2-3*01_BB-S_F1
<400> 592
   ctcgtttggc ccaggcaccc ggctgacagt gctcg 35
<210> 593
   <211> 35
   <212> DNA
   <213> Homo sapiens
<220>
   <223> TRBJ2-4*01_BB-S_F1
<400> 593
   ctcgtttggc gccgggaccc ggctctcagt gctgg 35
<210> 594
   <211> 35
   <212> DNA
   <213> Homo sapiens
<220>
   <223> TRBJ2-5*01_BB-S_F1
<400> 594
   ctcgtttggg ccaggcacgc ggctcctggt gctcg 35
<210> 595
   <211> 35
   <212> DNA
   <213> Homo sapiens
<220>
   <223> TRBJ2-6*01_BB-S_F1
<400> 595
   ctcgtttggg gccggcagca ggctgaccgt gctgg 35
<210> 596
   <211> 35
   <212> DNA
   <213> Homo sapiens
<220>
   <223> TRBJ2-7*01_BB-S_F1
<400> 596
   ctcgtttggg ccgggcacca ggctcacggt cacag 35
<210> 597
   <211> 35
   <212> DNA
   <213> Homo sapiens
<220>
   <223> TRBJ1-1*01_BB-S_R1
<400> 597
   tcctctacaa ctgtgagtct ggtgccttgt ccaaa 35
<210> 598
   <211> 35
   <212> DNA
   <213> Homo sapiens
<220>
   <223> TRBJ1-2*01_BB-S_R1
<400> 598
   tcctctacaa cggttaacct ggtccccgaa ccaaa 35
<210> 599
   <211> 35
   <212> DNA
   <213> Homo sapiens
<220>
   <223> TRBJ1-3*01_BB-S_P1
<400> 599
   tcctctacaa cagtgagcca acttccctct ccaaa 35
<210> 600
   <211> 35
   <212> DNA
   <213> Homo sapiens
<220>
   <223> TRBJ1-4*01_BB-S_P1
<400> 600
   tcctccaaga cagagagctg ggttccactg ccaaa 35
<210> 601
   <211> 35
   <212> DNA
   <213> Homo sapiens
<220>
   <223> TRBJ1-5*01_BB-S_R1
<400> 601
   tcctctagga tggagagtcg agtcccatca ccaaa 35
<210> 602
   <211> 35
   <212> DNA
   <213> Homo sapiens
<220>
   <223> TRBJ1-6*01_BB-S_R1
<400> 602
   tcctctgtca cagtgagcct ggtcccgttc ccaaa 35
<210> 603
   <211> 35
   <212> DNA
   <213> Homo sapiens
<220>
   <223> TRBJ2-1*01_BB-S_R1
<400> 603
   tcctctagca cggtgagccg tgtccctggc ccaaa 35
<210> 604
   <211> 35
   <212> DNA
   <213> Homo sapiens
<220>
   <223> TRBJ2-2*01_BB-S_R1
<400> 604
   tcctccagta cggtcagcct agagccttct ccaaa 35
<210> 605
   <211> 35
   <212> DNA
   <213> Homo sapiens
<220>
   <223> TRBJ2-3*01_BB-S_R1
<400> 605
   tcctcgagca ctgtcagccg ggtgcctggg ccaaa 35
<210> 606
   <211> 35
   <212> DNA
   <213> Homo sapiens
<220>
   <223> TRBJ2-4*01_BB-S_R1
<400> 606
   tcctccagca ctgagagccg ggtcccggcg ccaaa 35
<210> 607
   <211> 35
   <212> DNA
   <213> Homo sapiens
<220>
   <223> TRBJ2-5*01_BB-S_R1
<400> 607
   tcctcgagca ccaggagccg cgtgcctggc ccaaa 35
<210> 608
   <211> 35
   <212> DNA
   <213> Homo sapiens
<220>
   <223> TRBJ2-6*01_BB-S_R1
<400> 608
   tcctccagca cggtcagcct gctgccggcc ccaaa 35
<210> 609
   <211> 35
   <212> DNA
   <213> Homo sapiens
<220>
   <223> TRBJ2-7*01_BB-S_R1
<400> 609
   tcctctgtga ccgtgagcct ggtgcccggc ccaaa 35
<210> 610
   <211> 44
   <212> DNA
   <213> Homo sapiens
<220>
   <223> TRBJ1-1*01_BB-L_F1
<400> 610
   ctcggaagct ttctttggac aaggcaccag actcacagtt gtag 44
<210> 611
   <211> 44
   <212> DNA
   <213> Homo sapiens
<220>
   <223> TRBJ1-2*01_BB-L_F1
<400> 611
   ctcgggctac acctttggtt cggggaccag gttaaccgtt gtag 44
<210> 612
   <211> **44**
   <212> DNA
   <213> Homo sapiens
<220>
   <223> TRBJ1-3*01_BB-L_F1
<400> 612
   ctcgaccata tattttggag agggaagttg gctcactgtt gtag 44
<210> 613
   <211> 47
   <212> DNA
   <213> Homo sapiens
<220>
   <223> TRBJ1-4*01_BB-L_F1
<400> 613
   ctcggaaaag ctgttctttg gcagtggaac ccagctctct gtcttgg 47
<210> 614
   <211> 44
   <212> DNA
   <213> Homo sapiens
<220>
   <223> TRBJ1-5*01_BB-L_F1
<400> 614
   ctcgccccag cattttggtg atgggactcg actctccatc ctag 44
<210> 615
   <211> 47
   <212> DNA
   <213> Homo sapiens
<220>
   <223> TRBJ1-6*01_BB-L_F1
<400> 615
   ctcgtcaccc ctccactttg ggaacgggac caggctcact gtgacag 47
<210> 616
   <211> 44
   <212> DNA
   <213> Homo sapiens
<220>
   <223> TRB J Long segment part
<400> 616
   ctcggagcag ttctttgggc cagggacacg gctcaccgtg ctag 44
<210> 617
   <211> 47
   <212> DNA
   <213> Homo sapiens
<220>
   <223> TRB J Long segment part
<400> 617
   ctcgggggag ctgttctttg gagaaggctc taggctgacc gtactgg 47
<210> 618
   <211> 44
   <212> DNA
   <213> Homo sapiens
<220>
   <223> TRB J Long segment part
<400> 618
   ctcgacgcag tattttggcc caggcacccg gctgacagtg ctcg 44
<210> 619
   <211> 44
   <212> DNA
   <213> Homo sapiens
<220>
   <223> TRB J Long segment part
<400> 619
   ctcgattcag tactttggcg ccgggacccg gctctcagtg ctgg 44
<210> 620
   <211> 44
   <212> DNA
   <213> Homo sapiens
<220>
   <223> TRB J Long segment part
<400> 620
   ctcgacccag tactttgggc caggcacgcg gctcctggtg ctcg 44
<210> 621
   <211> 47
   <212> DNA
   <213> Homo sapiens
<220>
   <223> TRB J Long segment part
<400> 621
   ctcgaacgtc ctgacttttg gggccggcag caggctgacc gtgctgg 47
<210> 622
   <211> 41
   <212> DNA
   <213> Homo sapiens
<220>
   <223> TRB J Long segment part
<400> 622
   ctcgcagtac tttgggccgg gcaccaggct cacggtcaca g 41
<210> 623
   <211> 44
   <212> DNA
   <213> Homo sapiens
<220>
   <223> TRBJ1-1*01_BB-L_R1
<400> 623
   tcctctacaa ctgtgagtct ggtgccttgt ccaaagaaag cttc 44
<210> 624
   <211> 44
   <212> DNA
   <213> Homo sapiens
<220>
   <223> TRBJ1-2*01_BB-L_R1
<400> 624
   tcctctacaa cggttaacct ggtccccgaa ccaaaggtgt agcc 44
<210> 625
   <211> 44
   <212> DNA
   <213> Homo sapiens
<220>
   <223> TRBJ1-3*01_BB-L_R1
<400> 625
   tcctctacaa cagtgagcca acttccctct ccaaaatata tggt 44
<210> 626
   <211> 47
   <212> DNA
   <213> Homo sapiens
<220>
   <223> TRBJ1-4*01_BB-L_R1
<400> 626
   tcctccaaga cagagagctg ggttccactg ccaaagaaca gcttttc 47
<210> 627
   <211> 44
   <212> DNA
   <213> Homo sapiens
<220>
   <223> TRBJ1-5*01_BB-L_R1
<400> 627
   tcctctagga tggagagtcg agtcccatca ccaaaatgct gggg **44**
<210> 628
   <211> 47
   <212> DNA
   <213> Homo sapiens
<220>
   <223> TRBJ1-6*01_BB-L_R1
<400> 628
   tcctctgtca cagtgagcct ggtcccgttc ccaaagtgga ggggtga 47
<210> 629
   <211> 44
   <212> DNA
   <213> Homo sapiens
<220>
   <223> TRBJ2-1*01_BB-L_R1
<400> 629
   tcctctagca cggtgagccg tgtccctggc ccaaagaact gctc **44**
<210> 630
   <211> 47
   <212> DNA
   <213> Homo sapiens
<220>
   <223> TRBJ2-2*01_BB-L_R1
<400> 630
   tcctccagta cggtcagcct agagccttct ccaaagaaca gctcccc 47
<210> 631
   <211> 44
   <212> DNA
   <213> Homo sapiens
<220>
   <223> TRBJ2-3*01_BB-L_R1
<400> 631
   tcctcgagca ctgtcagccg ggtgcctggg ccaaaatact gcgt 44
<210> 632
   <211> 44
   <212> DNA
   <213> Homo sapiens
<220>
   <223> TRBJ2-4*01_BB-L_R1
<400> 632
   tcctccagca ctgagagccg ggtcccggcg ccaaagtact gaat 44
<210> 633
   <211> 44
   <212> DNA
   <213> Homo sapiens
<220>
   <223> TRBJ2-5*01_BB-L_R1
<400> 633
   tcctcgagca ccaggagccg cgtgcctggc ccaaagtact gggt 44
<210> 634
   <211> 47
   <212> DNA
   <213> Homo sapiens
<220>
   <223> TRBJ2-6*01_BB-L_R1
<400> 634
   tcctccagca cggtcagcct gctgccggcc ccaaaagtca ggacgtt 47
<210> 635
   <211> 41
   <212> DNA
   <213> Homo sapiens
<220>
   <223> TRBJ2-7*01_BB-L_R1
<400> 635
   tcctctgtga ccgtgagcct ggtgcccggc ccaaagtact g 41
<210> 636
   <211> 63
   <212> DNA
   <213> Homo sapiens
<220>
   <223> J Donor_Short_C1_TRBJ1-1
<400> 636
   ggtctcgttt ggacaaggca ccagactcac agttgtagag gacctgaaca aggtgttgag 60
   acc 63
<210> 637
   <211> 63
   <212> DNA
   <213> Homo sapiens
<220>
   <223> J Donor_Short_C1_TRBJ1-2
<400> 637
   ggtctcgttt ggttcgggga ccaggttaac cgttgtagag gacctgaaca aggtgttgag 60
   acc 63
<210> 638
   <211> 63
   <212> DNA
   <213> Homo sapiens
<220>
   <223> J Donor_Short_C1_TRBJ1-3
<400> 638
   ggtctcgttt ggagagggaa gttggctcac tgttgtagag gacctgaaca aggtgttgag 60
   acc 63
<210> 639
   <211> 63
   <212> DNA
   <213> Homo sapiens
<220>
   <223> J Donor_Short_C1_TRBJ1-4
<400> 639
   ggtctcgttt ggcagtggaa cccagctctc tgtcttggag gacctgaaca aggtgttgag 60
   acc 63
<210> 640
   <211> 63
   <212> DNA
   <213> Homo sapiens
<220>
   <223> J Donor_Short_C1_TRBJ1-5
<400> 640
   ggtctcgttt ggtgatggga ctcgactctc catcctagag gacctgaaca aggtgttgag 60
   acc 63
<210> 641
   <211> 63
   <212> DNA
   <213> Homo sapiens
<220>
   <223> J Donor_Short_C1_TRBJ1-6
<400> 641
   ggtctcgttt gggaacggga ccaggctcac tgtgacagag gacctgaaca aggtgttgag 60
   acc 63
<210> 642
   <211> 63
   <212> DNA
   <213> Homo sapiens
<220>
   <223> J Donor_Short_C1_TRBJ2-1
<400> 642
   ggtctcgttt gggccaggga cacggctcac cgtgctagag gacctgaaca aggtgttgag 60
   acc 63
<210> 643
   <211> 63
   <212> DNA
   <213> Homo sapiens
<220>
   <223> J Donor_Short_C1_TRBJ2-2
<400> 643
   ggtctcgttt ggagaaggct ctaggctgac cgtactggag gacctgaaca aggtgttgag 60
   acc 63
<210> 644
   <211> 63
   <212> DNA
   <213> Homo sapiens
<220>
   <223> J Donor_Short_C1_TRBJ2-3
<400> 644
   ggtctcgttt ggcccaggca cccggctgac agtgctcgag gacctgaaca aggtgttgag 60
   acc 63
<210> 645
   <211> 63
   <212> DNA
   <213> Homo sapiens
<220>
   <223> J Donor_Short_C1_TRBJ2-4
<400> 645
   ggtctcgttt ggcgccggga cccggctctc agtgctggag gacctgaaca aggtgttgag 60
   acc 63
<210> 646
   <211> 63
   <212> DNA
   <213> Homo sapiens
<220>
   <223> J Donor_Short_C1_TRBJ2-5
<400> 646
   ggtctcgttt gggccaggca cgcggctcct ggtgctcgag gacctgaaca aggtgttgag 60
   acc 63
<210> 647
   <211> 63
   <212> DNA
   <213> Homo sapiens
<220>
   <223> J Donor_Short_C1_TRBJ2-6
<400> 647
   ggtctcgttt ggggccggca gcaggctgac cgtgctggag gacctgaaca aggtgttgag 60
   acc 63
<210> 648
   <211> 63
   <212> DNA
   <213> Homo sapiens
<220>
   <223> J Donor_Short_C1_TRBJ2-7
<400> 648
   ggtctcgttt gggccgggca ccaggctcac ggtcacagag gacctgaaca aggtgttgag 60
   acc 63
<210> 649
   <211> 63
   <212> DNA
   <213> Homo sapiens
<220>
   <223> J Donor_Short_C2_TRBJ1-1
<400> 649
   ggtctcgttt ggacaaggca ccagactcac agttgtagag gacctgaaaa acgtgttgag 60
   acc 63
<210> 650
   <211> 63
   <212> DNA
   <213> Homo sapiens
<220>
   <223> J Donor_Short_C2_TRBJl-2
<400> 650
   ggtctcgttt ggttcgggga ccaggttaac cgttgtagag gacctgaaaa acgtgttgag 60
   acc 63
<210> 651
   <211> 63
   <212> DNA
   <213> Homo sapiens
<220>
   <223> J Donor_Short_C2_TRBJ1-3
<400> 651
   ggtctcgttt ggagagggaa gttggctcac tgttgtagag gacctgaaaa acgtgttgag 60
   acc 63
<210> 652
   <211> 63
   <212> DNA
   <213> Homo sapiens
<220>
   <223> J Donor_Short_C2_TRBJ1-4
<400> 652
   ggtctcgttt ggcagtggaa cccagctctc tgtcttggag gacctgaaaa acgtgttgag 60
   acc 63
<210> 653
   <211> 63
   <212> DNA
   <213> Homo sapiens
<220>
   <223> J Donor_Short_C2_TRBJl-5
<400> 653
   ggtctcgttt ggtgatggga ctcgactctc catcctagag gacctgaaaa acgtgttgag 60
   acc 63
<210> 654
   <211> 63
   <212> DNA
   <213> Homo sapiens
<220>
   <223> J Donor_Short_C2_TRBJl-6
<400> 654
   ggtctcgttt gggaacggga ccaggctcac tgtgacagag gacctgaaaa acgtgttgag 60
   acc 63
<210> 655
   <211> 63
   <212> DNA
   <213> Homo sapiens
<220>
   <223> J Donor_Short_C2_TRBJ2-1
<400> 655
   ggtctcgttt gggccaggga cacggctcac cgtgctagag gacctgaaaa acgtgttgag 60
   acc 63
<210> 656
   <211> 63
   <212> DNA
   <213> Homo sapiens
<220>
   <223> J Donor_Short_C2_TRBJ2-2
<400> 656
   ggtctcgttt ggagaaggct ctaggctgac cgtactggag gacctgaaaa acgtgttgag 60
   acc 63
<210> 657
   <211> 63
   <212> DNA
   <213> Homo sapiens
<220>
   <223> J Donor_Short_C2_TRBJ2-3
<400> 657
   ggtctcgttt ggcccaggca cccggctgac agtgctcgag gacctgaaaa acgtgttgag 60
   acc 63
<210> 658
   <211> 63
   <212> DNA
   <213> Homo sapiens
<220>
   <223> J Donor_Short_C2_TRBJ2-4
<400> 658
   ggtctcgttt ggcgccggga cccggctctc agtgctggag gacctgaaaa acgtgttgag 60
   acc 63
<210> 659
   <211> 63
   <212> DNA
   <213> Homo sapiens
<220>
   <223> J Donor_Short_C2_TRBJ2-5
<400> 659
   ggtctcgttt gggccaggca cgcggctcct ggtgctcgag gacctgaaaa acgtgttgag 60
   acc 63
<210> 660
   <211> 63
   <212> DNA
   <213> Homo sapiens
<220>
   <223> TRB C2 J Short donor vector
<400> 660
   ggtctcgttt ggggccggca gcaggctgac cgtgctggag gacctgaaaa acgtgttgag 60
   acc 63
<210> 661
   <211> 63
   <212> DNA
   <213> Homo sapiens
<220>
   <223> J Donor_Short_C2_TRBJ2-7
<400> 661
   ggtctcgttt gggccgggca ccaggctcac ggtcacagag gacctgaaaa acgtgttgag 60
   acc 63
<210> 662
   <211> 72
   <212> DNA
   <213> Homo sapiens
<220>
   <223> J Donor_Long_C1_TRBJ1-1
<400> 662
   ggtctcggaa gctttctttg gacaaggcac cagactcaca gttgtagagg acctgaacaa 60
   ggtgttgaga cc 72
<210> 663
   <211> 72
   <212> DNA
   <213> Homo sapiens
<220>
   <223> J Donor_Long_C1_TRBJ1-2
<400> 663
   ggtctcgggc tacacctttg gttcggggac caggttaacc gttgtagagg acctgaacaa 60
   ggtgttgaga cc 72
<210> 664
   <211> 72
   <212> DNA
   <213> Homo sapiens
<220>
   <223> J Donor_Long_C1_TRBJ1-3
<400> 664
   ggtctcgacc atatattttg gagagggaag ttggctcact gttgtagagg acctgaacaa 60
   ggtgttgaga cc 72
<210> 665
   <211> 75
   <212> DNA
   <213> Homo sapiens
<220>
   <223> J Donor_Long_C1_TRBJ1-4
<400> 665
   ggtctcggaa aagctgttct ttggcagtgg aacccagctc tctgtcttgg aggacctgaa 60
   caaggtgttg agacc 75
<210> 666
   <211> 72
   <212> DNA
   <213> Homo sapiens
<220>
   <223> J Donor_Long_C1_TRBJ1-5
<400> 666
   ggtctcgccc cagcattttg gtgatgggac tcgactctcc atcctagagg acctgaacaa 60
   ggtgttgaga cc 72
<210> 667
   <211> 75
   <212> DNA
   <213> Homo sapiens
<220>
   <223> J Donor_Long_C1_TRBJ1-6
<400> 667
   ggtctcgtca cccctccact ttgggaacgg gaccaggctc actgtgacag aggacctgaa 60
   caaggtgttg agacc 75
<210> 668
   <211> 72
   <212> DNA
   <213> Homo sapiens
<220>
   <223> J Donor_Long_C1_TRBJ2-1
<400> 668
   ggtctcggag cagttctttg ggccagggac acggctcacc gtgctagagg acctgaacaa 60
   ggtgttgaga cc 72
<210> 669
   <211> 75
   <212> DNA
   <213> Homo sapiens
<220>
   <223> J Donor_Long_C1_TRBJ2-2
<400> 669
   ggtctcgggg gagctgttct ttggagaagg ctctaggctg accgtactgg aggacctgaa 60
   caaggtgttg agacc 75
<210> 670
   <211> 72
   <212> DNA
   <213> Homo sapiens
<220>
   <223> J Donor_Long_C1_TRBJ2-3
<400> 670
   ggtctcgacg cagtattttg gcccaggcac ccggctgaca gtgctcgagg acctgaacaa 60
   ggtgttgaga cc 72
<210> 671
   <211> 72
   <212> DNA
   <213> Homo sapiens
<220>
   <223> J Donor_Long_C1_TRBJ2-4
<400> 671
   ggtctcgatt cagtactttg gcgccgggac ccggctctca gtgctggagg acctgaacaa 60
   ggtgttgaga cc 72
<210> 672
   <211> 72
   <212> DNA
   <213> Homo sapiens
<220>
   <223> J Donor_Long_C1_TRBJ2-5
<400> 672
   ggtctcgacc cagtactttg ggccaggcac gcggctcctg gtgctcgagg acctgaacaa 60
   ggtgttgaga cc 72
<210> 673
   <211> 75
   <212> DNA
   <213> Homo sapiens
<220>
   <223> J Donor_Long_C1_TRBJ2-6
<400> 673
   ggtctcgaac gtcctgactt ttggggccgg cagcaggctg accgtgctgg aggacctgaa 60
   caaggtgttg agacc 75
<210> 674
   <211> 69
   <212> DNA
   <213> Homo sapiens
<220>
   <223> J Donor_Long_C1_TRBJ2-7
<400> 674
   ggtctcgcag tactttgggc cgggcaccag gctcacggtc acagaggacc tgaacaaggt 60
   gttgagacc 69
<210> 675
   <211> 72
   <212> DNA
   <213> Homo sapiens
<220>
   <223> J Donor_Long_C2_TRBJ1-1
<400> 675
   ggtctcggaa gctttctttg gacaaggcac cagactcaca gttgtagagg acctgaaaaa 60
   cgtgttgaga cc 72
<210> 676
   <211> 72
   <212> DNA
   <213> Homo sapiens
<220>
   <223> J Donor_Long_C2_TRBJ1-2
<400> 676
   ggtctcgggc tacacctttg gttcggggac caggttaacc gttgtagagg acctgaaaaa 60
   cgtgttgaga cc 72
<210> 677
   <211> 72
   <212> DNA
   <213> Homo sapiens
<220>
   <223> J Donor_Long_C2_TRBJ1-3
<400> 677
   ggtctcgacc atatattttg gagagggaag ttggctcact gttgtagagg acctgaaaaa 60
   cgtgttgaga cc 72
<210> 678
   <211> 75
   <212> DNA
   <213> Homo sapiens
<220>
   <223> J Donor_Long_C2_TRBJ1-4
<400> 678
   ggtctcggaa aagctgttct ttggcagtgg aacccagctc tctgtcttgg aggacctgaa 60
   aaacgtgttg agacc 75
<210> 679
   <211> 72
   <212> DNA
   <213> Homo sapiens
<220>
   <223> J Donor_Long_C2_TRBJ1-5
<400> 679
   ggtctcgccc cagcattttg gtgatgggac tcgactctcc atcctagagg acctgaaaaa 60
   cgtgttgaga cc 72
<210> 680
   <211> 75
   <212> DNA
   <213> Homo sapiens
<220>
   <223> J Donor_Long_C2_TRBJ1-6
<400> 680
   ggtctcgtca cccctccact ttgggaacgg gaccaggctc actgtgacag aggacctgaa 60
   aaacgtgttg agacc 75
<210> 681
   <211> 72
   <212> DNA
   <213> Homo sapiens
<220>
   <223> J Donor_Long_C2_TRBJ2-1
<400> 681
   ggtctcggag cagttctttg ggccagggac acggctcacc gtgctagagg acctgaaaaa 60
   cgtgttgaga cc 72
<210> 682
   <211> 75
   <212> DNA
   <213> Homo sapiens
<220>
   <223> J Donor_Long_C2_TRBJ2-2
<400> 682
   ggtctcgggg gagctgttct ttggagaagg ctctaggctg accgtactgg aggacctgaa 60
   aaacgtgttg agacc 75
<210> 683
   <211> 72
   <212> DNA
   <213> Homo sapiens
<220>
   <223> J Donor_Long_C2_TRBJ2-3
<400> 683
   ggtctcgacg cagtattttg gcccaggcac ccggctgaca gtgctcgagg acctgaaaaa 60
   cgtgttgaga cc 72
<210> 684
   <211> 72
   <212> DNA
   <213> Homo sapiens
<220>
   <223> J Donor_Long_C2_TRBJ2-4
<400> 684
   ggtctcgatt cagtactttg gcgccgggac ccggctctca gtgctggagg acctgaaaaa 60
   cgtgttgaga cc 72
<210> 685
   <211> 72
   <212> DNA
   <213> Homo sapiens
<220>
   <223> J Donor_Long_C2_TRBJ2-5
<400> 685
   ggtctcgacc cagtactttg ggccaggcac gcggctcctg gtgctcgagg acctgaaaaa 60
   cgtgttgaga cc 72
<210> 686
   <211> 75
   <212> DNA
   <213> Homo sapiens
<220>
   <223> J Donor_Long_C2_TRBJ2-6
<400> 686
   ggtctcgaac gtcctgactt ttggggccgg cagcaggctg accgtgctgg aggacctgaa 60
   aaacgtgttg agacc 75
<210> 687
   <211> 69
   <212> DNA
   <213> Homo sapiens
<220>
   <223> J Donor_Long_C2_TRBJ2-7
<400> 687
   ggtctcgcag tactttgggc cgggcaccag gctcacggtc acagaggacc tgaaaaacgt 60
   gttgagacc 69
<210> 688
   <211> 2471
   <212> DNA
   <213> Artificial Sequence
<220>
   <223> V-C entry backbone F14/F15
<400> 688
   tctagacgaa gttcctattc cgaagttcct attcttatag gagtatagga acttcctcga      60
   gctgggcctc atgggccttc cgctcactgc ccgctttcca gtcgggaaac ctgtcgtgcc     120
   agctgcatta acatggtcat agctgtttcc ttgcgtattg ggcgctctcc gcttcctcgc     180
   tcactgactc gctgcgctcg gtcgttcggg taaagcctgg ggtgcctaat gagcaaaagg     240
   ccagcaaaag gccaggaacc gtaaaaaggc cgcgttgctg gcgtttttcc ataggctccg     300
   cccccctgac gagcatcaca aaaatcgacg ctcaagtcag aggtggcgaa acccgacagg     360
   actataaaga taccaggcgt ttccccctgg aagctccctc gtgcgctctc ctgttccgac     420
   cctgccgctt accggatacc tgtccgcctt tctcccttcg ggaagcgtgg cgctttctca     480
   tagctcacgc tgtaggtatc tcagttcggt gtaggtcgtt cgctccaagc tgggctgtgt     540
   gcacgaaccc cccgttcagc ccgaccgctg cgccttatcc ggtaactatc gtcttgagtc     600
   caacccggta agacacgact tatcgccact ggcagcagcc actggtaaca ggattagcag     660
   agcgaggtat gtaggcggtg ctacagagtt cttgaagtgg tggcctaact acggctacac     720
   tagaagaaca gtatttggta tctgcgctct gctgaagcca gttaccttcg gaaaaagagt     780
   tggtagctct tgatccggca aacaaaccac cgctggtagc ggtggttttt ttgtttgcaa     840
   gcagcagatt acgcgcagaa aaaaaggatc tcaagaagat cctttgatct tttctacggg     900
   gtctgacgct cagtggaacg aaaactcacg ttaagggatt ttggtcatga gattatcaaa     960
   aaggatcttc acctagatcc ttttaaatta aaaatgaagt tttaaatcaa tctaaagtat 1020
   atatgagtaa acttggtctg acagttacca atgcttaatc agtgaggcac ctatctcagc 1080
   gatctgtcta tttcgttcat ccatagttgc ctgactcccc gtcgtgtaga taactacgat 1140
   acgggagggc ttaccatctg gccccagtgc tgcaatgata ccgcgagaac cacgctcacc 1200
   ggctccagat ttatcagcaa taaaccagcc agccggaagg gccgagcgca gaagtggtcc 1260
   tgcaacttta tccgcctcca tccagtctat taattgttgc cgggaagcta gagtaagtag 1320
   ttcgccagtt aatagtttgc gcaacgttgt tgccattgct acaggcatcg tggtgtcacg 1380
   ctcgtcgttt ggtatggctt cattcagctc cggttcccaa cgatcaaggc gagttacatg 1440
   atcccccatg ttgtgcaaaa aagcggttag ctccttcggt cctccgatcg ttgtcagaag 1500
   taagttggcc gcagtgttat cactcatggt tatggcagca ctgcataatt ctcttactgt 1560
   catgccatcc gtaagatgct tttctgtgac tggtgagtac tcaaccaagt cattctgaga 1620
   atagtgtatg cggcgaccga gttgctcttg cccggcgtca atacgggata ataccgcgcc 1680
   acatagcaga actttaaaag tgctcatcat tggaaaacgt tcttcggggc gaaaactctc 1740
   aaggatctta ccgctgttga gatccagttc gatgtaaccc actcgtgcac ccaactgatc 1800
   ttcagcatct tttactttca ccagcgtttc tgggtgagca aaaacaggaa ggcaaaatgc 1860
   cgcaaaaaag ggaataaggg cgacacggaa atgttgaata ctcatactct tcctttttca 1920
   atattattga agcatttatc agggttattg tctcatgagc ggatacatat ttgaatgtat 1980
   ttagaaaaat aaacaaatag gggttccgcg cacatttccc cgaaaagtgc cacctaaatt 2040
   gtaagcgtta atattttgtt aaaattcgcg ttaaattttt gttaaatcag ctcatttttt 2100
   aaccaatagg ccgaaatcgg caaaatccct tataaatcaa aagaatagac cgagataggg 2160
   ttgagtggcc gctacagggc gctcccattc gccattcagg ctgcgcaact gttgggaagg 2220
   gcgtttcggt gcgggcctct tcgctattac gccagctggc gaaaggggga tgtgctgcaa 2280
   ggcgattaag ttgggtaacg ccagggtttt cccagtcacg acgttgtaaa acgacggcca 2340
   gtgagcgcga cgtaatacga ctcactatag ggcgaattgg cggaaggccg tcaaggccgc 2400
   atgaattcgc taccgggaag ttcctattcc gaagttccta ttctatcaga agtataggaa 2460
   cttcaggtac c 2471
<210> 689
   <211> 2471
   <212> DNA
   <213> Artificial Sequence
<220>
   <223> V-C entry backbone FRT/F3
<400> 689
   tctagacgaa gttcctattc cgaagttcct attcttcaaa tagtatagga acttcctcga 60
   gctgggcctc atgggccttc cgctcactgc ccgctttcca gtcgggaaac ctgtcgtgcc     120
   agctgcatta acatggtcat agctgtttcc ttgcgtattg ggcgctctcc gcttcctcgc     180
   tcactgactc gctgcgctcg gtcgttcggg taaagcctgg ggtgcctaat gagcaaaagg     240
   ccagcaaaag gccaggaacc gtaaaaaggc cgcgttgctg gcgtttttcc ataggctccg     300
   cccccctgac gagcatcaca aaaatcgacg ctcaagtcag aggtggcgaa acccgacagg     360
   actataaaga taccaggcgt ttccccctgg aagctccctc gtgcgctctc ctgttccgac     420
   cctgccgctt accggatacc tgtccgcctt tctcccttcg ggaagcgtgg cgctttctca     480
   tagctcacgc tgtaggtatc tcagttcggt gtaggtcgtt cgctccaagc tgggctgtgt     540
   gcacgaaccc cccgttcagc ccgaccgctg cgccttatcc ggtaactatc gtcttgagtc     600
   caacccggta agacacgact tatcgccact ggcagcagcc actggtaaca ggattagcag     660
   agcgaggtat gtaggcggtg ctacagagtt cttgaagtgg tggcctaact acggctacac     720
   tagaagaaca gtatttggta tctgcgctct gctgaagcca gttaccttcg gaaaaagagt     780
   tggtagctct tgatccggca aacaaaccac cgctggtagc ggtggttttt ttgtttgcaa     840
   gcagcagatt acgcgcagaa aaaaaggatc tcaagaagat cctttgatct tttctacggg     900
   gtctgacgct cagtggaacg aaaactcacg ttaagggatt ttggtcatga gattatcaaa     960
   aaggatcttc acctagatcc ttttaaatta aaaatgaagt tttaaatcaa tctaaagtat    1020
   atatgagtaa acttggtctg acagttacca atgcttaatc agtgaggcac ctatctcagc    1080
   gatctgtcta tttcgttcat ccatagttgc ctgactcccc gtcgtgtaga taactacgat    1140
   acgggagggc ttaccatctg gccccagtgc tgcaatgata ccgcgagaac cacgctcacc    1200
   ggctccagat ttatcagcaa taaaccagcc agccggaagg gccgagcgca gaagtggtcc    1260
   tgcaacttta tccgcctcca tccagtctat taattgttgc cgggaagcta gagtaagtag    1320
   ttcgccagtt aatagtttgc gcaacgttgt tgccattgct acaggcatcg tggtgtcacg    1380
   ctcgtcgttt ggtatggctt cattcagctc cggttcccaa cgatcaaggc gagttacatg    1440
   atcccccatg ttgtgcaaaa aagcggttag ctccttcggt cctccgatcg ttgtcagaag    1500
   taagttggcc gcagtgttat cactcatggt tatggcagca ctgcataatt ctcttactgt    1560
   catgccatcc gtaagatgct tttctgtgac tggtgagtac tcaaccaagt cattctgaga    1620
   atagtgtatg cggcgaccga gttgctcttg cccggcgtca atacgggata ataccgcgcc    1680
   acatagcaga actttaaaag tgctcatcat tggaaaacgt tcttcggggc gaaaactctc    1740
   aaggatctta ccgctgttga gatccagttc gatgtaaccc actcgtgcac ccaactgatc    1800
   ttcagcatct tttactttca ccagcgtttc tgggtgagca aaaacaggaa ggcaaaatgc    1860
   cgcaaaaaag ggaataaggg cgacacggaa atgttgaata ctcatactct tcctttttca    1920
   atattattga agcatttatc agggttattg tctcatgagc ggatacatat ttgaatgtat 1980
   ttagaaaaat aaacaaatag gggttccgcg cacatttccc cgaaaagtgc cacctaaatt 2040
   gtaagcgtta atattttgtt aaaattcgcg ttaaattttt gttaaatcag ctcatttttt 2100
   aaccaatagg ccgaaatcgg caaaatccct tataaatcaa aagaatagac cgagataggg 2160
   ttgagtggcc gctacagggc gctcccattc gccattcagg ctgcgcaact gttgggaagg 2220
   gcgtttcggt gcgggcctct tcgctattac gccagctggc gaaaggggga tgtgctgcaa 2280
   ggcgattaag ttgggtaacg ccagggtttt cccagtcacg acgttgtaaa acgacggcca 2340
   gtgagcgcga cgtaatacga ctcactatag ggcgaattgg cggaaggccg tcaaggccgc 2400
   atgaattcgc taccgggaag ttcctattcc gaagttccta ttctctagaa agtataggaa 2460
   cttcaggtac c 2471
<210> 690
   <211> 38
   <212> DNA
   <213> Artificial Sequence
<220>
   <223> Generic V cloning fragment (left part)
<220>
   <221> misc_feature
   <222> 38
   <223> /note="The last nucleotide of SEQ ID 690 is linked to the first nucleotide of XNn (described in specification), and the last nucleotide of XNn is then linked to the first nucleotide of SEQ ID 745."
<400> 690
   gtcagatact ccatgagcac gaagacttgt acgccacc 38
<210> 691
   <211> 44
   <212> DNA
   <213> Artificial Sequence
<220>
   <223> Generic C cloning fragment (left part)
<220>
   <221> misc_feature
   <222> 44
   <223> /note="The last nucleotide of SEQ ID 691 is linked to the first nucleotide of YNn (described in specification), and the last nucleotide of YNn is then linked to the first nucleotide of SEQ ID 746."
<400> 691
   gtgcactcct atgactaacg gaagactagg ccgcataggt ctca 44
<210> 692
   <211> 592
   <212> DNA
   <213> Artificial Sequence
<220>
   <223> Generic transient V-C entry vector (left part)
<220>
   <221> misc_feature
   <222> 592
   <223> /note="The last nucleotide of SEQ ID 692 is linked to the first nucleotide of XNn (described in specification), and the last nucleotide of XNn is then linked to the first nucleotide of SEQ ID 747."
<400> 692
   atagtaatca attacggggt cattagttca tagcccatat atggagttcc gcgttacata 60
   acttacggta aatggcccgc ctggctgacc gcccaacgac ccccgcccat tgacgtcaat 120
   aatgacgtat gttcccatag taacgccaat agggactttc cattgacgtc aatgggtgga 180
   gtatttacgg taaactgccc acttggcagt acatcaagtg tatcatatgc caagtacgcc 240
   ccctattgac gtcaatgacg gtaaatggcc cgcctggcat tatgcccagt acatgacctt 300
   atgggacttt cctacttggc agtacatcta cgtattagtc atcgctatta ccatggtgat 360
   gcggttttgg cagtacatca atgggcgtgg atagcggttt gactcacggg gatttccaag 420
   tctccacccc attgacgtca atgggagttt gttttggcac caaaatcaac gggactttcc 480
   aaaatgtcgt aacaactccg ccccattgac gcaaatgggc ggtaggcgtg tacggtggga 540
   ggtctatata agcagagctg gtttagtgaa ccgtcagatc aggtacgcca cc 592
<210> 693
   <211> 60
   <212> DNA
   <213> Artificial Sequence
<220>
   <223> Generic F14-F15 V-C entry vector (left part)
<220>
   <221> misc_feature
   <222> 60
   <223> /note="The last nucleotide of SEQ ID 693 is linked to the first nucleotide of XNn (described in specification), and the last nucleotide of XNn is then linked to the first nucleotide of SEQ ID 749."
<400> 693
   gaagttccta ttccgaagtt cctattctat cagaagtata ggaacttcag gtacgccacc 60
<210> 694
   <211> 60
   <212> DNA
   <213> Artificial Sequence
<220>
   <223> Generic FRT-F3 V-C entry vector (left part)
<220>
   <221> misc_feature
   <222> 60
   <223> /note="The last nucleotide of SEQ ID 694 is linked to the first nucleotide of XNn (described in specification), and the last nucleotide of XNn is then linked to the first nucleotide of SEQ ID 751."
<400> 694
   gaagttccta ttccgaagtt cctattctct agaaagtata ggaacttcag gtacgccacc 60
<210> 695
   <211> 38
   <212> DNA
   <213> Artificial Sequence
<220>
   <223> Generic J receiving cassette F (left part)
<220>
   <221> misc_feature
   <222> 38
   <223> /note="The last nucleotide of SEQ ID 695 is linked to the first nucleotide of Yâ\200\231Nn (described in specification), and the last nucleotide of Yâ\200\231Nn is then linked to the first nucleotide of TGAGACCC."
<400> 695
   aattcggtct cgaagtcttc tgcggccgct gaagacac 38
<210> 696
   <211> 12
   <212> DNA
   <213> Artificial Sequence
<220>
   <223> Generic J receiving cassette R (left part)
<220>
   <221> misc_feature
   <222> 12
   <223> /note="The last nucleotide of SEQ ID 696 is linked to the first nucleotide of Yâ\200\231Nn (described in specification), and the last nucleotide of Yâ\200\231Nn is then linked to the first nucleotide of
   SEQ ID 753."
<400> 696
   tcgagggtct ca 12
<210> 697
   <211> 38
   <212> DNA
   <213> Artificial Sequence
<220>
   <223> Generic J receiving cassette vector (left part)
<220>
   <221> misc_feature
   <222> 38
   <223> /note="The last nucleotide of SEQ ID 697 is linked to the first nucleotide of Yâ\200\231Nn (described in specification), and the last nucleotide of Yâ\200\231Nn is then linked to the first nucleotide of
   SEQ ID 754."
<400> 697
   aattcggtct cgaagtcttc tgcggccgct gaagacac 38
<210> 698
   <211> 10
   <212> DNA
   <213> Artificial Sequence
<220>
   <223> Generic J segment F (dummy, see specification for description)
<400> 698
   aaaaaaaaaa 10
<210> 699
   <211> 10
   <212> DNA
   <213> Artificial Sequence
<220>
   <223> Generic J segment R (dummy, see specification for description))
<400> 699
   aaaaaaaaaa 10
<210> 700
   <211> 13
   <212> DNA
   <213> Artificial Sequence
<220>
   <223> Generic J donor vector (left part)
<220>
   <221> misc_feature
   <222> 13
   <223> /note="The last nucleotide of SEQ ID 700 is linked to the first nucleotide of ZNnYâ\200\231Nn (described in specification), and the
   last nucleotide of ZNnYâ\200\231Nn is then linked to the first nucleotide of SEQ ID 755."
<400> 700
   gaattcggtc tcg 13
<210> 701
   <211> 816
   <212> DNA
   <213> Homo sapiens
<220>
   <223> JG9 TRA FL
<400> 701
   atgctccttg aacatttatt aataatcttg tggatgcagc tgacatgggt cagtggtcaa 60
   cagctgaatc agagtcctca atctatgttt atccaggaag gagaagatgt ctccatgaac 120
   tgcacttctt caagcatatt taacacctgg ctatggtaca agcaggaccc tggggaaggt 180
   cctgtcctct tgatagcctt atataaggct ggtgaattga cctcaaatgg aagactgact 240
   gctcagtttg gtataaccag aaaggacagc ttcctgaata tctcagcatc catacccagt 300
   gatgtaggca tctacttctg tgctggaccc atgaaaacct cctacgacaa ggtgatattt 360
   gggccaggga caagcttatc agtcattcca aatatccaga accctgaccc tgccgtgtac 420
   cagctgagag actctaaatc cagtgacaag tctgtctgcc tattcaccga ttttgattct 480
   caaacaaatg tgtcacaaag taaggattct gatgtgtata tcacagacaa aactgtgcta 540
   gacatgaggt ctatggactt caagagcaac agtgctgtgg cctggagcaa caaatctgac 600
   tttgcatgtg caaacgcctt caacaacagc attattccag aagacacctt cttccccagc 660
   ccagaaagtt cctgtgatgt caagctggtc gagaaaagct ttgaaacaga tacgaaccta 720
   aactttcaaa acctgtcagt gattgggttc cgaatcctcc tcctgaaagt ggccgggttt 780
   aatctgctca tgacgctgcg gctgtggtcc agctga 816
<210> 702
   <211> 927
   <212> DNA
   <213> Homo sapiens
<220>
   <223> JG9 TRB FL
<400> 702
   atggactcct ggaccttctg ctgtgtgtcc ctttgcatcc tggtagcaaa gcacacagat 60
   gctggagtta tccagtcacc ccggcacgag gtgacagaga tgggacaaga agtgactctg 120
   agatgtaaac caatttcagg acacgactac cttttctggt acagacagac catgatgcgg 180
   ggactggagt tgctcattta ctttaacaac aacgttccga tagatgattc agggatgccc 240
   gaggatcgat tctcagctaa gatgcctaat gcatcattct ccactctgaa gatccagccc 300
   tcagaaccca gggactcagc tgtgtacttc tgtgccagca gttcggcaaa ctatggctac 360
   accttcggtt cggggaccag gttaaccgtt gtagaggacc tgaacaaggt gttcccaccc 420
   gaggtcgctg tgtttgagcc atcagaagca gagatctccc acacccaaaa ggccacactg 480
   gtgtgcctgg ccacaggctt cttccctgac cacgtggagc tgagctggtg ggtgaatggg 540
   aaggaggtgc acagtggggt cagcacggac ccgcagcccc tcaaggagca gcccgccctc 600
   aatgactcca gatactgcct gagcagccgc ctgaggtctc cggccacctt ctggcagaac 660
   ccccgcaacc acttccgctg tcaagtccag ttctacgggc tctcggagaa tgacgagtgg 720
   acccaggata gggccaaacc cgtcacccag atcgtcagcg ccgaggcctg gggtagagca 780
   gactgtggct ttacctcggt gtcctaccag caaggggtcc tgtctgccac catcctctat 840
   gagatcctgc tagggaaggc caccctgtat gctgtgctgg tcagcgccct tgtgttgatg 900
   gccatggtca agagaaagga tttctga 927
<210> 703
   <211> 40
   <212> DNA
   <213> Homo sapiens
<220>
   <223> JG9 TRA odeCDR3 F
<400> 703
   ctgcgctgga cccatgaaaa cctcctacga caaggtgata 40
<210> 704
   <211> 40
   <212> DNA
   <213> Homo sapiens
<220>
   <223> JG9 TRA odeCDR3 R
<400> 704
   caaatatcac cttgtcgtag gaggttttca tgggtccagc 40
<210> 705
   <211> 34
   <212> DNA
   <213> Homo sapiens
<220>
   <223> JG9 TRB odeCDR3 F
<400> 705
   ttgcgccagc agttccgcaa actatggcta cacc 34
<210> 706
   <211> 34
   <212> DNA
   <213> Homo sapiens
<220>
   <223> JG9 TRB odeCDR3 R
<400> 706
   caaaggtgta gccatagttt gcggaactgc tggc 34
<210> 707
   <211> 257
   <212> DNA
   <213> Homo sapiens
<220>
   <223> TRA HBD4.1.S9-1.D5.a
<400> 707
   gaagacagaa agtccagcac cttgatcctg ccccacgcta cgctgagaga cactgctgtg     60
   tactattgca tcgtcaggga tcgttataac accggtaacc agttctattt tgggacaggg    120
   acaagtttga cggtcattcc aaatatccag aaccctgacc ctgccgtgta ccagctgaga    180
   gactctaaat ccagtgacaa gtctgtctgc ctattcaccg attttgattc tcaaacaaat    240
   gtgtcacaaa gtaagga                                                   257
<210> 708
   <211> 257
   <212> DNA
   <213> Homo sapiens
<220>
   <223> TRB HBD4.1.S9-1.D5.b
<400> 708
   cagggttttc ccagtcacga ctagaaaaga gaagaggaat ttccccctga tcctggagtc      60
   gcccagcccc aaccagacct ctctgtactt ctgtgccagc agtttgggcc caagctccta    120
   cgagcagtac ttcgggccgg gcaccaggct cacggtcaca gaggacctga aaaacgtgtt    180
   cccacccgag gtcgctgtgt ttgagccatc agaagcagag atcttccaca cccaaaaggc    240
   cacactggtg tgcctgg                                                   257
<210> 709
   <211> 298
   <212> DNA
   <213> Homo sapiens
<220>
   <223> TRA HBD4.1.S9-2.A6.a
<400> 709
   gttttcccag tcacgactcc agaaggcaag aaaatccgcc aaccttgtca tctccgcttc     60
   acaactgggg gactcagcaa tgtatttctg tgcaatgaga gagggcatgg atagcagcta    120
   taaattgatc ttcgggagtg ggaccagact gctggtcagg cctgatatcc agaaccctga    180
   ccctgccgtg taccagctga gagactctaa atccagtgac aagtctgtct gcctattcac    240
   cgattttgat tctcaaacaa atgtgtcaca aagtaaggat tctgtactga gtgagtat      298
<210> 710
   <211> 235
   <212> DNA
   <213> Homo sapiens
<220>
   <223> TRA HBD4.1.S9-2.A6.a
<400> 710
   ctttctccac tttggagatc cagcgcacag agcaggggga ctcggccatg tatctctgtg     60
   ccagcagttt gcacgaccgg ggggcgcgga ctgaagcttt ctttggacaa ggcaccagac    120
   tcacagttgt agaggacctg aacaaggtgt tcccacccga ggtcgctgtg tttgagccat    180
   cagaagcaga gatctcccac acccaaaagg ccacactggt gtgcctggcc acagg         235
<210> 711
   <211> 273
   <212> DNA
   <213> Homo sapiens
<220>
   <223> TRA HBD4.1.S9-3.A1.a
<400> 711
   cgacactaac ctttcagttt ggtgatgcaa gaaaggacag ttctctccac atcactgcag     60
   cccagcctgg tgatacaggc ctctacctct gtgcaggagc tcgaggaggc ttcaaaacta    120
   tctttggagc aggaacaaga ctatttgtta aagcaaatat ccagaaccct gaccctgccg    180
   tgtaccagct gagagactct aaatccagtg acaagtctgt ctgcctattc accgattttg    240
   attctcaaac aaatgtgtca caaagtaagg att                                 273
<210> 712
   <211> 221
   <212> DNA
   <213> Homo sapiens
<220>
   <223> TRB HBD4.1.S9-3.A1.b
<400> 712
   ccccctgatc ctggagtcgc ccaaccccaa ccaagcctct ctgtacttct gtgccagcar     60
   wttatcacca tctactggga actatggcta caccttcggt tcggggacca ggttaaccgt    120
   tgtagaggac ctgaacaagg tgttcccacc cgaggtcgct gtgtttgagc catcagaagc    180
   agagatctcc cacacccaaa aggcccacca ctggtgkgcc t                        221
<210> 713
   <211> 286
   <212> DNA
   <213> Homo sapiens
<220>
   <223> TRA HBD4.1.S9-7.E5.a
<400> 713
   aaggcaagaa aatccgccaa ccttgtcatm tccgcttcac aactggggga ctcagcaatg      60
   tatttctgtg caatgaggcc ttatactgga ggcttcaaaa ctatctttgg agcaggaaca     120
   agactatttg ttaaagcaaa tatccagaac cctgaccctg ccgtgtacca gctgagagac     180
   tctaaatcca gtgacaagtc tgtctgccta ttcaccgatt ttgattctca aacaaatgtg     240
   tcacaaagta aggattctga tgtgtatatc acagacaaaa ctktgc                    286
<210> 714
   <211> 238
   <212> DNA
   <213> Homo sapiens
<220>
   <223> TRA HBD4.1.S9-7.E5.a
<400> 714
   cgaaaagaga agaggaattt ccccmtgatc ctggagtcgc ccagccccaw ccagacctct      60
   ctgtacttct gtgccagcts cactagcccc tcgmcagctc gctatggcta cwccttcggt     120
   tcggggacca ggttaaccgt tgtagaggac ctgaacaagg tgttcccacc cgaggtcgct     180
   gtgtttgagc catcagaagc agmswtctcc cacacccaaa aggccacact gagkgkgc       238
<210> 715
   <211> 306
   <212> DNA
   <213> Homo sapiens
<220>
   <223> TRA HBD4.1.S9-7.C5.a
<400> 715
   tttgaacccg aatttaacaa gacacaaacc tccttccccc tgaagaaacc atctgccctt      60
   gtgagcgatt ccgctttgta cttctgtgct gtgagatccc ggatggatag cagctataaa     120
   ttgatcttcg ggagtgggac cagactactg gtcaggcctg atatccagaa ccctgaccct     180
   gccgtgtacc agctgagaga ctctaaatcc agtgacaagt ctgtctgcct attcaccgat     240
   tttgattctc aaacaaatgt gtcacaaagt aaggattctg atgtgtatat cacagacaaa     300
   actgtg                                                                306
<210> 716
   <211> 251
   <212> DNA
   <213> Homo sapiens
<220>
   <223> TRB HBD4.1.S9-7.C5.b
<400> 716
   acgacctcca gggttttcta agggatgttt ctccaccttg aagatccagc gcacagagca      60
   gggggactcg gccatgtatc tctgtgccag cagccccccc gacgcggcct acaatgagca     120
   gttcttcggg ccagggacac ggctcaccgt gctagaggac ctgaaaaacg tgttcccacc     180
   cgaggtcgct gtgtttgagc catcagaagc agagatctcc cacacccaaa aggccacact     240
   ggtgtgcctg g                                                          251
<210> 717
   <211> 319
   <212> DNA
   <213> Homo sapiens
<220>
   <223> TRA HBD4.1.S9-5.E3.a
<400> 717
   agggttttcc cagtcacgac ttaagagtca cgcttgacac ttccaagaaa agcagttcct      60
   tgttgatcac ggcttcccgg gcagcagaca ctgcttctta cttctgtgct acggagctcc     120
   ggatggatag cagctataaa ttgatcttcg ggagtgggac cagactgctg gtcaggcctg     180
   atatccagaa ccctgaccct gccgtgtacc agctgagaga ctctaaatcc agtgacaagt     240
   ctgtctgcct attcaccgat tttgattctc aaacaaatgt gtcacaaagt aaggattctg     300
   atgtgtatat cacagacaa                                                  319
<210> 718
   <211> 270
   <212> DNA
   <213> Homo sapiens
<220>
   <223> TRA HBD4.1.S9-5.E3.b
<400> 718
   agggttttcc cagtcacgac ctccagagag gcctaaggga tgtttctcca ctttggagat 60
   ccagcgcaca gagcaggggg actcggccat gtatctctgt gccagcagct taagggaagg 120
   acgactccca gagacccagt acttcgggcc aggcacgcgg ctcctggtgc tcgaggacct 180
   gaaaaacgtg ttcccacccg aggtcgctgt gtttgagcca tcagaagcag agatctccca 240
   cacccaaaag gccacactgg tgtgcctggc 270
<210> 719
   <211> 43
   <212> DNA
   <213> Homo sapiens
<220>
   <223> Forward TRA HBD4.1.S9-1.D5
<400> 719
   ctgcatcgtc agggatcgtt ataacaccgg taaccagttc tat 43
<210> 720
   <211> 43
   <212> DNA
   <213> Homo sapiens
<220>
   <223> Reverse TRA HBD4.1.S9-1.D5
<400> 720
   caaaatagaa ctggttaccg gtgttataac gatccctgac gat 43
<210> 721
   <211> 40
   <212> DNA
   <213> Homo sapiens
<220>
   <223> Forward TRB HBD4.1.S9-1.D5
<400> 721
   ttgcgccagc agtttgggcc caagctccta cgagcagtac 40
<210> 722
   <211> 40
   <212> DNA
   <213> Homo sapiens
<220>
   <223> Reverse TRB HBD4.1.S9-1.D5
<400> 722
   caaagtactg ctcgtaggag cttgggccca aactgctggc 40
<210> 723
   <211> 43
   <212> DNA
   <213> Homo sapiens
<220>
   <223> Forward TRA HBD4.1.S9-2.A6
<400> 723
   ctgcgcaatg agagagggca tggatagcag ctataaattg atc 43
<210> 724
   <211> 43
   <212> DNA
   <213> Homo sapiens
<220>
   <223> Reverse TRA HBD4.1.S9-2.A6
<400> 724
   caaagatcaa tttatagctg ctatccatgc cctctctcat tgc 43
<210> 725
   <211> 46
   <212> DNA
   <213> Homo sapiens
<220>
   <223> Forward TRB HBD4.1.S9-2.A6
<400> 725
   ttgcgccagc agtttgcacg accgtggtgc gcggactgaa gctttc 46
<210> 726
   <211> 46
   <212> DNA
   <213> Homo sapiens
<220>
   <223> Reverse TRB HBD4.1.S9-2.A6
<400> 726
   caaagaaagc ttcagtccgc gcaccacggt cgtgcaaact gctggc 46
<210> 727
   <211> 34
   <212> DNA
   <213> Homo sapiens
<220>
   <223> Forward TRA HBD4.1.S9-3.A1
<400> 727
   ctgcgcagga gctcgaggag gcttcaaaac tatc 34
<210> 728
   <211> 34
   <212> DNA
   <213> Homo sapiens
<220>
   <223> Reverse TRA HBD4.1.S9-3.A1
<400> 728
   caaagatagt tttgaagcct cctcgagctc ctgc 34
<210> 729
   <211> 46
   <212> DNA
   <213> Homo sapiens
<220>
   <223> Forward TRB HBD4.1.S9-3.A1
<400> 729
   ttgcgccagc agtttatcac catctactgg gaactatggc tacacc 46
<210> 730
   <211> 46
   <212> DNA
   <213> Homo sapiens
<220>
   <223> Reverse TRB HBD4.1.S9-3.A1
<400> 730
   caaaggtgta gccatagttc ccagtagatg gtgataaact gctggc 46
<210> 731
   <211> 40
   <212> DNA
   <213> Homo sapiens
<220>
   <223> Forward TRA HBD4.1.S9-7.E5
<400> 731
   ctgcgcaatg aggccttata ctggaggctt caaaactatc 40
<210> 732
   <211> 40
   <212> DNA
   <213> Homo sapiens
<220>
   <223> Reverse TRA HBD4.1.S9-7.E5
<400> 732
   caaagatagt tttgaagcct ccagtataag gcctcattgc 40
<210> 733
   <211> 46
   <212> DNA
   <213> Homo sapiens
<220>
   <223> Forward TRB HBD4.1.S9-7.E5
<400> 733
   ttgcgccagc tccactagcc cctcgacagc tcgctatggc tacacc 46
<210> 734
   <211> 46
   <212> DNA
   <213> Homo sapiens
<220>
   <223> Reverse TRB HBD4.1.S9-7.E5
<400> 734
   caaaggtgta gccatagcga gctgtcgagg ggctagtgga gctggc 46
<210> 735
   <211> 43
   <212> DNA
   <213> Homo sapiens
<220>
   <223> Forward TRA HBD4.1.S9-7.C5
<400> 735
   ctgcgctgtg agatcccgga tggatagcag ctataaattg atc 43
<210> 736
   <211> 43
   <212> DNA
   <213> Homo sapiens
<220>
   <223> Reverse TRA HBD4.1.S9-7.C5
<400> 736
   caaagatcaa tttatagctg ctatccatcc gggatctcac agc 43
<210> 737
   <211> 43
   <212> DNA
   <213> Homo sapiens
<220>
   <223> Forward TRB HBD4.1.S9-7.C5
<400> 737
   ttgcgccagc agccctcctg acgcggccta caatgagcag ttc 43
<210> 738
   <211> 43
   <212> DNA
   <213> Homo sapiens
<220>
   <223> Reverse TRB HBD4.1.S9-7.C5
<400> 738
   caaagaactg ctcattgtag gccgcgtcag gagggctgct ggc 43
<210> 739
   <211> 43
   <212> DNA
   <213> Homo sapiens
<220>
   <223> Forward TRA HBD4.1.S9-5.E3
<400> 739
   ctgcgctacg gagctccgga tggatagcag ctataaattg atc 43
<210> 740
   <211> 43
   <212> DNA
   <213> Homo sapiens
<220>
   <223> Reverse TRA HBD4.1.S9-5.E3
<400> 740
   caaagatcaa tttatagctg ctatccatcc ggagctccgt agc 43
<210> 741
   <211> 46
   <212> DNA
   <213> Homo sapiens
<220>
   <223> Forward TRB HBD4.1.S9-5.E3
<400> 741
   ttgcgccagc agcttaaggg aaggacgact cccagagaca cagtac 46
<210> 742
   <211> 46
   <212> DNA
   <213> Homo sapiens
<220>
   <223> Reverse TRB HBD4.1.S9-5.E3
<400> 742
   caaagtactg tgtctctggg agtcgtcctt cccttaagct gctggc 46
<210> 743
   <211> 40
   <212> DNA
   <213> Artificial Sequence
<220>
   <223> Forward TRA odeCDR3 - 3 positions degenerate
<220>
   <221> misc_feature
   <222> 15
   <223> /note="n = a, t, c or g"
<220>
   <221> misc_feature
   <222> 20
   <223> /note="n = a, t, c or g"
<220>
   <221> misc_feature
   <222> 26
   <223> /note="n = a, t, c or g"
<400> 743
   ctgcgctgga cccangaaan cctccnacga caaggtgata 40
<210> 744
   <211> 40
   <212> DNA
   <213> Artificial Sequence
<220>
   <223> Reverse TRA odeCDR3 - 3 positions degenerate
<220>
   <221> misc_feature
   <222> 19
   <223> /note="n = a, t, c or g"
<220>
   <221> misc_feature
   <222> 25
   <223> /note="n = a, t, c or g"
<220>
   <221> misc_feature
   <222> 30
   <223> /note="n = a, t, c or g"
<400> 744
   caaatatcac cttgtcgtng gaggntttcn tgggtccagc 40
<210> 745
   <211> 43
   <212> DNA
   <213> Artificial Sequence
<220>
   <223> Generic V cloning fragment (right part)
<220>
   <221> misc_feature
   <222> 1
   <223> /note="The first nucleotide of SEQ ID 745 is linked to the last nucleotide of XNn (described in specification), and the first nucleotide of XNn is then linked to the last nucleotide of SEQ ID 690. "
<400> 745
   agagaccttg cggccgtgtc ttcgactagt agctcaccta cga 43
<210> 746
   <211> 32
   <212> DNA
   <213> Artificial Sequence
<220>
   <223> Generic C cloning fragment (right part)
<220>
   <221> misc_feature
   <222> 1
   <223> /note="The first nucleotide of SEQ ID 746 is linked to the last nucleotide of YNn (described in specification), and the first nucleotide of YNn is then linked to the last nucleotide of SEQ ID 691."
<400> 746
   ctaggtgtct tccctatgct gaatcgatgg tc 32
<210> 747
   <211> 27
   <212> DNA
   <213> Artificial Sequence
<220>
   <223> Generic transient V-C entry vector (middle part)
<220>
   <221> misc_feature
   <222> 1
   <223> /note="The first nucleotide of SEQ ID 747 is linked to the last nucleotide of XNn (described in specification), and the first nucleotide of XNn is then linked to the last nucleotide of SEQ ID 692."
<220>
   <221> misc_feature
   <222> 27
   <223> /note="The last nucleotide of SEQ ID 747 is linked to the first nucleotide of YNn (described in specification), and the last nucleotide of YNn is then linked to the first nucleotide of SEQ ID 748."
<400> 747
   agagaccttg cggccgcata ggtctca 27
<210> 748
   <211> 2622
   <212> DNA
   <213> Artificial Sequence
<220>
   <223> Generic transient V-C entry vector (right part)
<220>
   <221> misc_feature
   <222> 1
   <223> /note="The first nucleotide of SEQ ID 748 is linked to the last nucleotide of YNn (described in specification), and the first nucleotide of YNn is then linked to the last nucleotide of SEQ ID 747. "
<400> 748
   ctagacctga tcataatcaa gccatatcac atctgtagag gtttacttgc tttaaaaaac      60
   ctccacacct ccccctgaac ctgaaacata aaatgaatgc aattgttgtt gttaacttgt    120
   ttattgcagc ttataatggt tacaaataaa gcaatagcat cacaaatttc acaaataaag    180
   catttttttc actgcattct agttgtggtt tgtccaaact catcaatgta tcttatcatg     240
   tctggatctg cggatccaat ctcgagctgg gcctcatggg ccttccgctc actgcccgct    300
   ttccagtcgg gaaacctgtc gtgccagctg cattaacatg gtcatagctg tttccttgcg    360
   tattgggcgc tctccgcttc ctcgctcact gactcgctgc gctcggtcgt tcgggtaaag    420
   cctggggtgc ctaatgagca aaaggccagc aaaaggccag gaaccgtaaa aaggccgcgt    480
   tgctggcgtt tttccatagg ctccgccccc ctgacgagca tcacaaaaat cgacgctcaa    540
   gtcagaggtg gcgaaacccg acaggactat aaagatacca ggcgtttccc cctggaagct    600
   ccctcgtgcg ctctcctgtt ccgaccctgc cgcttaccgg atacctgtcc gcctttctcc    660
   cttcgggaag cgtggcgctt tctcatagct cacgctgtag gtatctcagt tcggtgtagg    720
   tcgttcgctc caagctgggc tgtgtgcacg aaccccccgt tcagcccgac cgctgcgcct    780
   tatccggtaa ctatcgtctt gagtccaacc cggtaagaca cgacttatcg ccactggcag    840
   cagccactgg taacaggatt agcagagcga ggtatgtagg cggtgctaca gagttcttga    900
   agtggtggcc taactacggc tacactagaa gaacagtatt tggtatctgc gctctgctga    960
   agccagttac cttcggaaaa agagttggta gctcttgatc cggcaaacaa accaccgctg 1020
   gtagcggtgg tttttttgtt tgcaagcagc agattacgcg cagaaaaaaa ggatctcaag 1080
   aagatccttt gatcttttct acggggtctg acgctcagtg gaacgaaaac tcacgttaag 1140
   ggattttggt catgagatta tcaaaaagga tcttcaccta gatcctttta aattaaaaat 1200
   gaagttttaa atcaatctaa agtatatatg agtaaacttg gtctgacagt taccaatgct 1260
   taatcagtga ggcacctatc tcagcgatct gtctatttcg ttcatccata gttgcctgac 1320
   tccccgtcgt gtagataact acgatacggg agggcttacc atctggcccc agtgctgcaa 1380
   tgataccgcg agaaccacgc tcaccggctc cagatttatc agcaataaac cagccagccg 1440
   gaagggccga gcgcagaagt ggtcctgcaa ctttatccgc ctccatccag tctattaatt 1500
   gttgccggga agctagagta agtagttcgc cagttaatag tttgcgcaac gttgttgcca 1560
   ttgctacagg catcgtggtg tcacgctcgt cgtttggtat ggcttcattc agctccggtt 1620
   cccaacgatc aaggcgagtt acatgatccc ccatgttgtg caaaaaagcg gttagctcct 1680
   tcggtcctcc gatcgttgtc agaagtaagt tggccgcagt gttatcactc atggttatgg 1740
   cagcactgca taattctctt actgtcatgc catccgtaag atgcttttct gtgactggtg 1800
   agtactcaac caagtcattc tgagaatagt gtatgcggcg accgagttgc tcttgcccgg 1860
   cgtcaatacg ggataatacc gcgccacata gcagaacttt aaaagtgctc atcattggaa 1920
   aacgttcttc ggggcgaaaa ctctcaagga tcttaccgct gttgagatcc agttcgatgt 1980
   aacccactcg tgcacccaac tgatcttcag catcttttac tttcaccagc gtttctgggt 2040
   gagcaaaaac aggaaggcaa aatgccgcaa aaaagggaat aagggcgaca cggaaatgtt 2100
   gaatactcat actcttcctt tttcaatatt attgaagcat ttatcagggt tattgtctca 2160
   tgagcggata catatttgaa tgtatttaga aaaataaaca aataggggtt ccgcgcacat 2220
   ttccccgaaa agtgccacct aaattgtaag cgttaatatt ttgttaaaat tcgcgttaaa 2280
   tttttgttaa atcagctcat tttttaacca ataggccgaa atcggcaaaa tcccttataa 2340
   atcaaaagaa tagaccgaga tagggttgag tggccgctac agggcgctcc cattcgccat 2400
   tcaggctgcg caactgttgg gaagggcgtt tcggtgcggg cctcttcgct attacgccag 2460
   ctggcgaaag ggggatgtgc tgcaaggcga ttaagttggg taacgccagg gttttcccag 2520
   tcacgacgtt gtaaaacgac ggccagtgag cgcgacgtaa tacgactcac tatagggcga 2580
   attggcggaa ggccgtcaag gccgcatgaa ttcgctaccg gt 2622
<210> 749
   <211> 27
   <212> DNA
   <213> Artificial Sequence
<220>
   <223> Generic F14-F15 V-C entry vector (middle part)
<220>
   <221> misc_feature
   <222> 1
   <223> /note="The first nucleotide of SEQ ID 749 is linked to the last nucleotide of XNn (described in specification), and the first nucleotide of XNn is then linked to the last nucleotide of SEQ ID 693."
<220>
   <221> misc_feature
   <222> 27
   <223> /note="The last nucleotide of SEQ ID 749 is linked to the first nucleotide of YNn (described in specification), and the last nucleotide of YNn is then linked to the first nucleotide of SEQ ID 750."
<400> 749
   agagaccttg cggccgcata ggtctca 27
<210> 750
   <211> 2415
   <212> DNA
   <213> Artificial Sequence
<220>
   <223> Generic F14-F15 V-C entry vector (right part)
<220>
   <221> misc_feature
   <222> 1
   <223> /note="The first nucleotide of SEQ ID 750 is linked to the last nucleotide of YNn (described in specification), and the first nucleotide of YNn is then linked to the last nucleotide of SEQ ID 749."
<400> 750
   ctagacgaag ttcctattcc gaagttccta ttcttatagg agtataggaa cttcctcgag      60
   ctgggcctca tgggccttcc gctcactgcc cgctttccag tcgggaaacc tgtcgtgcca     120
   gctgcattaa catggtcata gctgtttcct tgcgtattgg gcgctctccg cttcctcgct     180
   cactgactcg ctgcgctcgg tcgttcgggt aaagcctggg gtgcctaatg agcaaaaggc     240
   cagcaaaagg ccaggaaccg taaaaaggcc gcgttgctgg cgtttttcca taggctccgc     300
   ccccctgacg agcatcacaa aaatcgacgc tcaagtcaga ggtggcgaaa cccgacagga     360
   ctataaagat accaggcgtt tccccctgga agctccctcg tgcgctctcc tgttccgacc     420
   ctgccgctta ccggatacct gtccgccttt ctcccttcgg gaagcgtggc gctttctcat     480
   agctcacgct gtaggtatct cagttcggtg taggtcgttc gctccaagct gggctgtgtg     540
   cacgaacccc ccgttcagcc cgaccgctgc gccttatccg gtaactatcg tcttgagtcc     600
   aacccggtaa gacacgactt atcgccactg gcagcagcca ctggtaacag gattagcaga     660
   gcgaggtatg taggcggtgc tacagagttc ttgaagtggt ggcctaacta cggctacact     720
   agaagaacag tatttggtat ctgcgctctg ctgaagccag ttaccttcgg aaaaagagtt 780
   ggtagctctt gatccggcaa acaaaccacc gctggtagcg gtggtttttt tgtttgcaag 840
   cagcagatta cgcgcagaaa aaaaggatct caagaagatc ctttgatctt ttctacgggg 900
   tctgacgctc agtggaacga aaactcacgt taagggattt tggtcatgag attatcaaaa 960
   aggatcttca cctagatcct tttaaattaa aaatgaagtt ttaaatcaat ctaaagtata 1020
   tatgagtaaa cttggtctga cagttaccaa tgcttaatca gtgaggcacc tatctcagcg 1080
   atctgtctat ttcgttcatc catagttgcc tgactccccg tcgtgtagat aactacgata 1140
   cgggagggct taccatctgg ccccagtgct gcaatgatac cgcgagaacc acgctcaccg 1200
   gctccagatt tatcagcaat aaaccagcca gccggaaggg ccgagcgcag aagtggtcct 1260
   gcaactttat ccgcctccat ccagtctatt aattgttgcc gggaagctag agtaagtagt 1320
   tcgccagtta atagtttgcg caacgttgtt gccattgcta caggcatcgt ggtgtcacgc 1380
   tcgtcgtttg gtatggcttc attcagctcc ggttcccaac gatcaaggcg agttacatga 1440
   tcccccatgt tgtgcaaaaa agcggttagc tccttcggtc ctccgatcgt tgtcagaagt 1500
   aagttggccg cagtgttatc actcatggtt atggcagcac tgcataattc tcttactgtc 1560
   atgccatccg taagatgctt ttctgtgact ggtgagtact caaccaagtc attctgagaa 1620
   tagtgtatgc ggcgaccgag ttgctcttgc ccggcgtcaa tacgggataa taccgcgcca 1680
   catagcagaa ctttaaaagt gctcatcatt ggaaaacgtt cttcggggcg aaaactctca 1740
   aggatcttac cgctgttgag atccagttcg atgtaaccca ctcgtgcacc caactgatct 1800
   tcagcatctt ttactttcac cagcgtttct gggtgagcaa aaacaggaag gcaaaatgcc 1860
   gcaaaaaagg gaataagggc gacacggaaa tgttgaatac tcatactctt cctttttcaa 1920
   tattattgaa gcatttatca gggttattgt ctcatgagcg gatacatatt tgaatgtatt 1980
   tagaaaaata aacaaatagg ggttccgcgc acatttcccc gaaaagtgcc acctaaattg 2040
   taagcgttaa tattttgtta aaattcgcgt taaatttttg ttaaatcagc tcatttttta 2100
   accaataggc cgaaatcggc aaaatccctt ataaatcaaa agaatagacc gagatagggt 2160
   tgagtggccg ctacagggcg ctcccattcg ccattcaggc tgcgcaactg ttgggaaggg 2220
   cgtttcggtg cgggcctctt cgctattacg ccagctggcg aaagggggat gtgctgcaag 2280
   gcgattaagt tgggtaacgc cagggttttc ccagtcacga cgttgtaaaa cgacggccag 2340
   tgagcgcgac gtaatacgac tcactatagg gcgaattggc ggaaggccgt caaggccgca 2400
   tgaattcgct accgg 2415
<210> 751
   <211> 27
   <212> DNA
   <213> Artificial Sequence
<220>
   <223> Generic FRT-F3 V-C entry vector (middle part)
<220>
   <221> misc_feature
   <222> 1
   <223> /note="The first nucleotide of SEQ ID 751 is linked to the last nucleotide of XNn (described in specification), and the first nucleotide of XNn is then linked to the last nucleotide of SEQ ID 694."
<220>
   <221> misc_feature
   <222> 27
   <223> /note="The last nucleotide of SEQ ID 751 is linked to the first nucleotide of YNn (described in specification), and the last nucleotide of YNn is then linked to the first nucleotide of SEQ ID 752."
<400> 751
   agagaccttg cggccgcata ggtctca 27
<210> 752
   <211> 2415
   <212> DNA
   <213> Artificial Sequence
<220>
   <223> Generic FRT-F3 V-C entry vector (middle part)
<220>
   <221> misc_feature
   <222> 1
   <223> /note="The first nucleotide of SEQ ID 752 is linked to the last nucleotide of YNn (described in specification), and the first nucleotide of YNn is then linked to the last nucleotide of SEQ ID 751."
<400> 752
   ctagacgaag ttcctattcc gaagttccta ttcttcaaat agtataggaa cttcctcgag 60
   ctgggcctca tgggccttcc gctcactgcc cgctttccag tcgggaaacc tgtcgtgcca 120
   gctgcattaa catggtcata gctgtttcct tgcgtattgg gcgctctccg cttcctcgct 180
   cactgactcg ctgcgctcgg tcgttcgggt aaagcctggg gtgcctaatg agcaaaaggc 240
   cagcaaaagg ccaggaaccg taaaaaggcc gcgttgctgg cgtttttcca taggctccgc 300
   ccccctgacg agcatcacaa aaatcgacgc tcaagtcaga ggtggcgaaa cccgacagga 360
   ctataaagat accaggcgtt tccccctgga agctccctcg tgcgctctcc tgttccgacc 420
   ctgccgctta ccggatacct gtccgccttt ctcccttcgg gaagcgtggc gctttctcat 480
   agctcacgct gtaggtatct cagttcggtg taggtcgttc gctccaagct gggctgtgtg 540
   cacgaacccc ccgttcagcc cgaccgctgc gccttatccg gtaactatcg tcttgagtcc 600
   aacccggtaa gacacgactt atcgccactg gcagcagcca ctggtaacag gattagcaga 660
   gcgaggtatg taggcggtgc tacagagttc ttgaagtggt ggcctaacta cggctacact 720
   agaagaacag tatttggtat ctgcgctctg ctgaagccag ttaccttcgg aaaaagagtt 780
   ggtagctctt gatccggcaa acaaaccacc gctggtagcg gtggtttttt tgtttgcaag 840
   cagcagatta cgcgcagaaa aaaaggatct caagaagatc ctttgatctt ttctacgggg 900
   tctgacgctc agtggaacga aaactcacgt taagggattt tggtcatgag attatcaaaa 960
   aggatcttca cctagatcct tttaaattaa aaatgaagtt ttaaatcaat ctaaagtata 1020
   tatgagtaaa cttggtctga cagttaccaa tgcttaatca gtgaggcacc tatctcagcg 1080
   atctgtctat ttcgttcatc catagttgcc tgactccccg tcgtgtagat aactacgata 1140
   cgggagggct taccatctgg ccccagtgct gcaatgatac cgcgagaacc acgctcaccg 1200
   gctccagatt tatcagcaat aaaccagcca gccggaaggg ccgagcgcag aagtggtcct 1260
   gcaactttat ccgcctccat ccagtctatt aattgttgcc gggaagctag agtaagtagt 1320
   tcgccagtta atagtttgcg caacgttgtt gccattgcta caggcatcgt ggtgtcacgc 1380
   tcgtcgtttg gtatggcttc attcagctcc ggttcccaac gatcaaggcg agttacatga 1440
   tcccccatgt tgtgcaaaaa agcggttagc tccttcggtc ctccgatcgt tgtcagaagt 1500
   aagttggccg cagtgttatc actcatggtt atggcagcac tgcataattc tcttactgtc 1560
   atgccatccg taagatgctt ttctgtgact ggtgagtact caaccaagtc attctgagaa 1620
   tagtgtatgc ggcgaccgag ttgctcttgc ccggcgtcaa tacgggataa taccgcgcca 1680
   catagcagaa ctttaaaagt gctcatcatt ggaaaacgtt cttcggggcg aaaactctca 1740
   aggatcttac cgctgttgag atccagttcg atgtaaccca ctcgtgcacc caactgatct 1800
   tcagcatctt ttactttcac cagcgtttct gggtgagcaa aaacaggaag gcaaaatgcc 1860
   gcaaaaaagg gaataagggc gacacggaaa tgttgaatac tcatactctt cctttttcaa 1920
   tattattgaa gcatttatca gggttattgt ctcatgagcg gatacatatt tgaatgtatt 1980
   tagaaaaata aacaaatagg ggttccgcgc acatttcccc gaaaagtgcc acctaaattg 2040
   taagcgttaa tattttgtta aaattcgcgt taaatttttg ttaaatcagc tcatttttta 2100
   accaataggc cgaaatcggc aaaatccctt ataaatcaaa agaatagacc gagatagggt 2160
   tgagtggccg ctacagggcg ctcccattcg ccattcaggc tgcgcaactg ttgggaaggg 2220
   cgtttcggtg cgggcctctt cgctattacg ccagctggcg aaagggggat gtgctgcaag 2280
   gcgattaagt tgggtaacgc cagggttttc ccagtcacga cgttgtaaaa cgacggccag 2340
   tgagcgcgac gtaatacgac tcactatagg gcgaattggc ggaaggccgt caaggccgca 2400
   tgaattcgct accgg 2415
<210> 753
   <211> 34
   <212> DNA
   <213> Artificial Sequence
<220>
   <223> Generic J receiving cassette R (right part)
<220>
   <221> misc_feature
   <222> 1
   <223> /note="The first nucleotide of SEQ ID 753 is linked to the last nucleotide of Yâ\200\231Nn (described in specification), and the first nucleotide of Yâ\200\231Nn is then linked to the last nucleotide of SEQ ID 696."
<400> 753
   gtgtcttcag cggccgcaga agacttcgag accg 34
<210> 754
   <211> 2310
   <212> DNA
   <213> Artificial Sequence
<220>
   <223> Generic J receiving cassette vector (right part)
<220>
   <221> misc_feature
   <222> 1
   <223> /note="The first nucleotide of SEQ ID 754 is linked to the last nucleotide of Yâ\200\231Nn (described in specification), and the first nucleotide of Yâ\200\231Nn is then linked to the last nucleotide of SEQ ID 697."
<400> 754
   tgagaccctc gagctgggcc tcatgggcct tccgctcact gcccgctttc cagtcgggaa 60
   acctgtcgtg ccagctgcat taacatggtc atagctgttt ccttgcgtat tgggcgctct 120
   ccgcttcctc gctcactgac tcgctgcgct cggtcgttcg ggtaaagcct ggggtgccta 180
   atgagcaaaa ggccagcaaa aggccaggaa ccgtaaaaag gccgcgttgc tggcgttttt 240
   ccataggctc cgcccccctg acgagcatca caaaaatcga cgctcaagtc agaggtggcg 300
   aaacccgaca ggactataaa gataccaggc gtttccccct ggaagctccc tcgtgcgctc 360
   tcctgttccg accctgccgc ttaccggata cctgtccgcc tttctccctt cgggaagcgt 420
   ggcgctttct catagctcac gctgtaggta tctcagttcg gtgtaggtcg ttcgctccaa 480
   gctgggctgt gtgcacgaac cccccgttca gcccgaccgc tgcgccttat ccggtaacta 540
   tcgtcttgag tccaacccgg taagacacga cttatcgcca ctggcagcag ccactggtaa 600
   caggattagc agagcgaggt atgtaggcgg tgctacagag ttcttgaagt ggtggcctaa 660
   ctacggctac actagaagaa cagtatttgg tatctgcgct ctgctgaagc cagttacctt 720
   cggaaaaaga gttggtagct cttgatccgg caaacaaacc accgctggta gcggtggttt 780
   ttttgtttgc aagcagcaga ttacgcgcag aaaaaaagga tctcaagaag atcctttgat 840
   cttttctacg gggtctgacg ctcagtggaa cgaaaactca cgttaaggga ttttggtcat     900
   gagattatca aaaaggatct tcacctagat ccttttaaat taaaaatgaa gttttaaatc     960
   aatctaaagt atatatgagt aaacttggtc tgacagttag aaaaattcgt ccagcatcag    1020
   atgaaattgc agtttgttca tgtccgggtt atcaatacca tatttctgga acagacgttt    1080
   ctgcaggctc gggctaaatt cacccagaca attccacaga attgccagat cctgataacg    1140
   atctgcaata ccaacacgac caacatcaat gcagccaatc agtttaccct catcaaaaat    1200
   caggttatcc aggctaaaat caccatgggt aacaacgcta tccggactaa acggcagcag    1260
   tttatgcatt tctttccaaa cctgttcaac aggccaacca ttacgttcat catcaaaatc    1320
   gcttgcatca accagaccat tattcatacg gctctgtgcc tgtgccagac gaaaaacacg    1380
   atcgctatta aacggacaat tacaaaccgg aatgctatgc agacgacgca gaaaaactgc    1440
   cagtgcatca acaatatttt cgcctgaatc cggatattct tccagaacct gaaatgcggt    1500
   tttacccgga attgcggtgg tcagcagcca tgcatcatcc ggtgtacgaa taaaatgttt    1560
   aatggtcggc agcggcataa attcggtcag ccaattcaga cgaaccattt catcggtcac    1620
   atcatttgca acgctacctt taccatgttt cagaaacagt tccggtgcat ccggtttacc    1680
   atacagacga taaatggttg caccgctctg accaacatta tcacgtgccc atttatagcc    1740
   atacagatct gcatccatat tgctattcag acgcggacgg ctacagctgg tttcacgctg    1800
   aatatggctc atactcttcc tttttcaata ttattgaagc atttatcagg gttattgtct    1860
   catgagcgga tacatatttg aatgtattta gaaaaataaa caaatagggg ttccgcgcac    1920
   atttccccga aaagtgccac ctaaattgta agcgttaata ttttgttaaa attcgcgtta    1980
   aatttttgtt aaatcagctc attttttaac caataggccg aaatcggcaa aatcccttat    2040
   aaatcaaaag aatagaccga gatagggttg agtggccgct acagggcgct cccattcgcc    2100
   attcaggctg cgcaactgtt gggaagggcg tttcggtgcg ggcctcttcg ctattacgcc    2160
   agctggcgaa agggggatgt gctgcaaggc gattaagttg ggtaacgcca gggttttccc   2220
   agtcacgacg ttgtaaaacg acggccagtg agcgcgacgt aatacgactc actatagggc    2280
   gaattggcgg aaggccgtca aggccgcatg                                     2310
<210> 755
   <211> 2309
   <212> DNA
   <213> Artificial Sequence
<220>
   <223> Generic J donor vector (right part)
<220>
   <221> misc_feature
   <222> 1
   <223> /note="The first nucleotide of SEQ ID 755 is linked to the last nucleotide of ZNnYâ\200\231Nn (described in specification), and the
   first nucleotide of ZNnYâ\200\231Nn is then linked to the last nucleotide of SEQ ID 700."
<400> 755
   tgagaccctc gagctgggcc tcatgggcct tccgctcact gcccgctttc cagtcgggaa      60
   acctgtcgtg ccagctgcat taacatggtc atagctgttt ccttgcgtat tgggcgctct     120
   ccgcttcctc gctcactgac tcgctgcgct cggtcgttcg ggtaaagcct ggggtgccta     180
   atgagcaaaa ggccagcaaa aggccaggaa ccgtaaaaag gccgcgttgc tggcgttttt     240
   ccataggctc cgcccccctg acgagcatca caaaaatcga cgctcaagtc agaggtggcg     300
   aaacccgaca ggactataaa gataccaggc gtttccccct ggaagctccc tcgtgcgctc     360
   tcctgttccg accctgccgc ttaccggata cctgtccgcc tttctccctt cgggaagcgt     420
   ggcgctttct catagctcac gctgtaggta tctcagttcg gtgtaggtcg ttcgctccaa     480
   gctgggctgt gtgcacgaac cccccgttca gcccgaccgc tgcgccttat ccggtaacta     540
   tcgtcttgag tccaacccgg taagacacga cttatcgcca ctggcagcag ccactggtaa     600
   caggattagc agagcgaggt atgtaggcgg tgctacagag ttcttgaagt ggtggcctaa     660
   ctacggctac actagaagaa cagtatttgg tatctgcgct ctgctgaagc cagttacctt     720
   cggaaaaaga gttggtagct cttgatccgg caaacaaacc accgctggta gcggtggttt     780
   ttttgtttgc aagcagcaga ttacgcgcag aaaaaaagga tctcaagaag atcctttgat     840
   cttttctacg gggtctgacg ctcagtggaa cgaaaactca cgttaaggga ttttggtcat     900
   gagattatca aaaaggatct tcacctagat ccttttaaat taaaaatgaa gttttaaatc     960
   aatctaaagt atatatgagt aaacttggtc tgacagttag aaaaattcgt ccagcatcag    1020
   atgaaattgc agtttgttca tgtccgggtt atcaatacca tatttctgga acagacgttt    1080
   ctgcaggctc gggctaaatt cacccagaca attccacaga attgccagat cctgataacg    1140
   atctgcaata ccaacacgac caacatcaat gcagccaatc agtttaccct catcaaaaat    1200
   caggttatcc aggctaaaat caccatgggt aacaacgcta tccggactaa acggcagcag    1260
   tttatgcatt tctttccaaa cctgttcaac aggccaacca ttacgttcat catcaaaatc    1320
   gcttgcatca accagaccat tattcatacg gctctgtgcc tgtgccagac gaaaaacacg    1380
   atcgctatta aacggacaat tacaaaccgg aatgctatgc agacgacgca gaaaaactgc    1440
   cagtgcatca acaatatttt cgcctgaatc cggatattct tccagaacct gaaatgcggt    1500
   tttacccgga attgcggtgg tcagcagcca tgcatcatcc ggtgtacgaa taaaatgttt    1560
   aatggtcggc agcggcataa attcggtcag ccaattcaga cgaaccattt catcggtcac    1620
   atcatttgca acgctacctt taccatgttt cagaaacagt tccggtgcat ccggtttacc    1680
   atacagacga taaatggttg caccgctctg accaacatta tcacgtgccc atttatagcc    1740
   atacagatct gcatccatat tgctattcag acgcggacgg ctacagctgg tttcacgctg 1800
   aatatggctc atactcttcc tttttcaata ttattgaagc atttatcagg gttattgtct 1860
   catgagcgga tacatatttg aatgtattta gaaaaataaa caaatagggg ttccgcgcac 1920
   atttccccga aaagtgccac ctaaattgta agcgttaata ttttgttaaa attcgcgtta 1980
   aatttttgtt aaatcagctc attttttaac caataggccg aaatcggcaa aatcccttat 2040
   aaatcaaaag aatagaccga gatagggttg agtggccgct acagggcgct cccattcgcc 2100
   attcaggctg cgcaactgtt gggaagggcg tttcggtgcg ggcctcttcg ctattacgcc 2160
   agctggcgaa agggggatgt gctgcaaggc gattaagttg ggtaacgcca gggttttccc 2220
   agtcacgacg ttgtaaaacg acggccagtg agcgcgacgt aatacgactc actatagggc 2280
   gaattggcgg aaggccgtca aggccgcat 2309
<210> 756
   <211> 2510
   <212> DNA
   <213> Artificial Sequence
<220>
   <223> V-Ca entry backbone FRT/F3
<400> 756
   ctagctagag acgttgtcga catcgtctct tacctagacg aagttcctat tccgaagttc      60
   ctattcttca aatagtatag gaacttcctc gagctgggcc tcatgggcct tccgctcact     120
   gcccgctttc cagtcgggaa acctgtcgtg ccagctgcat taacatggtc atagctgttt     180
   ccttgcgtat tgggcgctct ccgcttcctc gctcactgac tcgctgcgct cggtcgttcg     240
   ggtaaagcct ggggtgccta atgagcaaaa ggccagcaaa aggccaggaa ccgtaaaaag     300
   gccgcgttgc tggcgttttt ccataggctc cgcccccctg acgagcatca caaaaatcga     360
   cgctcaagtc agaggtggcg aaacccgaca ggactataaa gataccaggc gtttccccct     420
   ggaagctccc tcgtgcgctc tcctgttccg accctgccgc ttaccggata cctgtccgcc     480
   tttctccctt cgggaagcgt ggcgctttct catagctcac gctgtaggta tctcagttcg     540
   gtgtaggtcg ttcgctccaa gctgggctgt gtgcacgaac cccccgttca gcccgaccgc     600
   tgcgccttat ccggtaacta tcgtcttgag tccaacccgg taagacacga cttatcgcca     660
   ctggcagcag ccactggtaa caggattagc agagcgaggt atgtaggcgg tgctacagag     720
   ttcttgaagt ggtggcctaa ctacggctac actagaagaa cagtatttgg tatctgcgct     780
   ctgctgaagc cagttacctt cggaaaaaga gttggtagct cttgatccgg caaacaaacc     840
   accgctggta gcggtggttt ttttgtttgc aagcagcaga ttacgcgcag aaaaaaagga     900
   tctcaagaag atcctttgat cttttctacg gggtctgacg ctcagtggaa cgaaaactca     960
   cgttaaggga ttttggtcat gagattatca aaaaggatct tcacctagat ccttttaaat 1020
   taaaaatgaa gttttaaatc aatctaaagt atatatgagt aaacttggtc tgacagttac 1080
   caatgcttaa tcagtgaggc acctatctca gcgatctgtc tatttcgttc atccatagtt 1140
   gcctgactcc ccgtcgtgta gataactacg atacgggagg gcttaccatc tggccccagt 1200
   gctgcaatga taccgcgaga cccacgctca ccggctccag atttatcagc aataaaccag 1260
   ccagccggaa gggccgagcg cagaagtggt cctgcaactt tatccgcctc catccagtct 1320
   attaattgtt gccgggaagc tagagtaagt agttcgccag ttaatagttt gcgcaacgtt 1380
   gttgccattg ctacaggcat cgtggtgtca cgctcgtcgt ttggtatggc ttcattcagc 1440
   tccggttccc aacgatcaag gcgagttaca tgatccccca tgttgtgcaa aaaagcggtt 1500
   agctccttcg gtcctccgat cgttgtcaga agtaagttgg ccgcagtgtt atcactcatg 1560
   gttatggcag cactgcataa ttctcttact gtcatgccat ccgtaagatg cttttctgtg 1620
   actggtgagt actcaaccaa gtcattctga gaatagtgta tgcggcgacc gagttgctct 1680
   tgcccggcgt caatacggga taataccgcg ccacatagca gaactttaaa agtgctcatc 1740
   attggaaaac gttcttcggg gcgaaaactc tcaaggatct taccgctgtt gagatccagt 1800
   tcgatgtaac ccactcgtgc acccaactga tcttcagcat cttttacttt caccagcgtt 1860
   tctgggtgag caaaaacagg aaggcaaaat gccgcaaaaa agggaataag ggcgacacgg 1920
   aaatgttgaa tactcatact cttccttttt caatattatt gaagcattta tcagggttat 1980
   tgtctcatga gcggatacat atttgaatgt atttagaaaa ataaacaaat aggggttccg 2040
   cgcacatttc cccgaaaagt gccacctaaa ttgtaagcgt taatattttg ttaaaattcg 2100
   cgttaaattt ttgttaaatc agctcatttt ttaaccaata ggccgaaatc ggcaaaatcc 2160
   cttataaatc aaaagaatag accgagatag ggttgagtgg ccgctacagg gcgctcccat 2220
   tcgccattca ggctgcgcaa ctgttgggaa gggcgtttcg gtgcgggcct cttcgctatt 2280
   acgccagctg gcgaaagggg gatgtgctgc aaggcgatta agttgggtaa cgccagggtt 2340
   ttcccagtca cgacgttgta aaacgacggc cagtgagcgc gacgtaatac gactcactat 2400
   agggcgaatt ggcggaaggc cgtcaaggcc gcatgaattc gctaccggga agttcctatt 2460
   ccgaagttcc tattctctag aaagtatagg aacttcaggt actcaggtac 2510
<210> 757
   <211> 785
   <212> DNA
   <213> Artificial Sequence
<220>
   <223> V-C entry TRAV7_TRAC_FRT-F3
<400> 757
   gccaccatgg agaagatgcg tagacctgtc ctaattatat tttgtctatg tcttggctgg 60
<210> 758
   <211> 788
   <212> DNA
   <213> Artificial Sequence
<220>
   <223> V-C entry TRAV8-2_TRAC_FRT-F3
<400> 758 ctgactag 788
<210> 759
   <211> 784
   <212> DNA
   <213> Artificial Sequence
<220>
   <223> V-C entry TRAV8-4_TRAC_FRT-F3
<400> 759
   gccaccatgc tcctgctgct cgtcccagtg ctcgaggtga tttttaccct gggaggaacc 60
   agagcccagt cggtgaccca gcttggcagc cacgtttctg tctctgaagg agccctggtt 120
   ctgctgaggt gcaactactc atcgtctgtt ccaccatatc tcttctggta tgtgcaatac 180
   cccaaccaag gactccagct tctcctgaag tacacatcag cggccaccct ggttaaaggc 240
   atcaacggtt ttgaggctga atttaagaag agtgaaacct ccttccacct gacgaaaccc 300
   tcagcccata tgagcgacgc ggctgagtac ttctgcagag accttgcggc cgcataggtc 360
   tcaccagaac cctgaccctg ccgtgtacca gctgagagac tctaaatcca gtgacaagtc 420
   tgtctgccta ttcaccgatt ttgattctca aacaaatgtg tcacaaagta aggattctga 480
   tgtgtatatc acagacaaaa ctgtgctaga catgaggtct atggacttca agagcaacag 540
   tgctgtggcc tggagcaaca aatctgactt tgcatgtgca aacgccttca acaacagcat 600
   tattccagag gacaccttct tccccagccc agaaagttcc tgtgatgtca agctggtcga 660
   gaaaagcttt gaaacagata cgaacctaaa ctttcaaaac ctgtcagtga ttgggttccg 720
   aatcctcctc ctgaaagtgg ccgggtttaa tctgctcatg acgctgcggc tgtggtccag 780
   ctga 784
<210> 760
   <211> 778
   <212> DNA
   <213> Artificial Sequence
<220>
   <223> V-C entry TRAV18_TRAC_FRT-F3
<400> 760
   gccaccatgc tgtctgcttc ctgctcagga cttgtgatct tgttgatatt cagaaggacc 60
   agtggagact cggttaccca gacagaaggc ccagttaccc tccctgagag ggcagctctg 120
   acattaaact gcacttatca gtccagctat tcaacttttc tattctggta tgtccagtat 180
   ctaaacaaag agcctgagct cctcctgaaa agttcagaaa accaggagac tgacagcaga 240
   ggttttcagg ccagtcctat caagagtgac agttccttcc acctggagaa gccctcggtg 300
   cagctgtcgg actctgccgt gtactactgc agagaccttg cggccgcata ggtctcacca 360
   gaaccctgac cctgccgtgt accagctgag agactctaaa tccagtgaca agtctgtctg 420
   cctattcacc gattttgatt ctcaaacaaa tgtgtcacaa agtaaggatt ctgatgtgta 480
   tatcacagac aaaactgtgc tagacatgag gtctatggac ttcaagagca acagtgctgt 540
   ggcctggagc aacaaatctg actttgcatg tgcaaacgcc ttcaacaaca gcattattcc 600
   agaggacacc ttcttcccca gcccagaaag ttcctgtgat gtcaagctgg tcgagaaaag 660
   ctttgaaaca gatacgaacc taaactttca aaacctgtca gtgattgggt tccgaatcct 720
   cctcctgaaa gtggccgggt ttaatctgct catgacgctg cggctgtggt ccagctga 778
<210> 761
   <211> 790
   <212> DNA
   <213> Artificial Sequence
<220>
   <223> V-C entry TRAV38-2DV8_TRAC_FRT-F3
<400> 761
   gccaccatgg catgccctgg cttcctgtgg gcacttgtga tctccacctg tcttgaattt 60
   agcatggctc agacagtcac tcagtctcaa ccagagatgt ctgtgcagga ggcagaaacg 120
   gtgaccctga gctgcacata tgacaccagt gagagtgatt attatttatt ctggtacaag 180
   cagcctccca gcaggcagat gattctcgtt attcgccaag aagcttataa gcaacagaat 240
   gcaacagaga atcgtttctc tgtgaacttc cagaaagcag ccaaatcctt cagtctcaag 300
   atctcagact cacagctggg ggatgccgcg atgtatttct gcagagacct tgcggccgca 360
   taggtctcac cagaaccctg accctgccgt gtaccagctg agagactcta aatccagtga 420
   caagtctgtc tgcctattca ccgattttga ttctcaaaca aatgtgtcac aaagtaagga 480
   ttctgatgtg tatatcacag acaaaactgt gctagacatg aggtctatgg acttcaagag 540
   caacagtgct gtggcctgga gcaacaaatc tgactttgca tgtgcaaacg ccttcaacaa 600
   cagcattatt ccagaggaca ccttcttccc cagcccagaa agttcctgtg atgtcaagct 660
   ggtcgagaaa agctttgaaa cagatacgaa cctaaacttt caaaacctgt cagtgattgg 720
   gttccgaatc ctcctcctga aagtggccgg gtttaatctg ctcatgacgc tgcggctgtg 780
   gtccagctga 790
<210> 762
   <211> 775
   <212> DNA
   <213> Artificial Sequence
<220>
   <223> V-C entry TRAV39_TRAC_FRT-F3
<400> 762
   gccaccatga agaagctact agcaatgatt ctgtggcttc aactagaccg gttaagtgga      60
   gagctgaaag tggaacaaaa ccctctgttc ctgagcatgc aggagggaaa aaactatacc     120
   atctactgca attattcaac cacttcagac agactgtatt ggtacaggca ggatcctggg     180
   aaaagtctgg aatctctgtt tgtgttgcta tcaaatggag cagtgaagca ggagggacga     240
   ttaatggcct cacttgatac caaagcccga ctcagcaccc tccacatcac agctgccgtg     300
   catgacctct ctgccaccta cttctgcaga gaccttgcgg ccgcataggt ctcaccagaa     360
   ccctgaccct gccgtgtacc agctgagaga ctctaaatcc agtgacaagt ctgtctgcct     420
   attcaccgat tttgattctc aaacaaatgt gtcacaaagt aaggattctg atgtgtatat     480
   cacagacaaa actgtgctag acatgaggtc tatggacttc aagagcaaca gtgctgtggc     540
   ctggagcaac aaatctgact ttgcatgtgc aaacgccttc aacaacagca ttattccaga     600
   ggacaccttc ttccccagcc cagaaagttc ctgtgatgtc aagctggtcg agaaaagctt     660
   tgaaacagat acgaacctaa actttcaaaa cctgtcagtg attgggttcc gaatcctcct     720
   cctgaaagtg gccgggttta atctgctcat gacgctgcgg ctgtggtcca gctga          775
<210> 763
   <211> 760
   <212> DNA
   <213> Artificial Sequence
<220>
   <223> V-C entry TRAV40_TRAC_FRT-F3
<400> 763
   gccaccatga actcctctct ggactttcta attctgatct taatgtttgg aggaaccagc      60
   agcaattcag tcaagcagac gggccaaata accgtatcgg agggagcatc tgtgactatg     120
   aactgcacat acacatccac ggggtaccct acccttttct ggtatgtgga ataccccagc     180
   aaacctctgc agcttcttca gagagagaca atggaaaaca gcaaaaactt cggaggcgga     240
   aatattaaag acaaaaactc ccccattgtg aaatattcag tccaggtatc agactcagcc     300
   gtgtactact gcagagacct tgcggccgca taggtctcac cagaaccctg accctgccgt     360
   gtaccagctg agagactcta aatccagtga caagtctgtc tgcctattca ccgattttga     420
   ttctcaaaca aatgtgtcac aaagtaagga ttctgatgtg tatatcacag acaaaactgt     480
   gctagacatg aggtctatgg acttcaagag caacagtgct gtggcctgga gcaacaaatc     540
   tgactttgca tgtgcaaacg ccttcaacaa cagcattatt ccagaggaca ccttcttccc     600
   cagcccagaa agttcctgtg atgtcaagct ggtcgagaaa agctttgaaa cagatacgaa     660
   cctaaacttt caaaacctgt cagtgattgg gttccgaatc ctcctcctga aagtggccgg     720
   gtttaatctg ctcatgacgc tgcggctgtg gtccagctga                           760
<210> 764
   <211> 2501
   <212> DNA
   <213> Artificial Sequence
<220>
   <223> V-Cb entry backbone F14/F15
<400> 764
   ctagacctgg agacgacgcg tctagacgaa gttcctattc cgaagttcct attcttatag 60
   gagtatagga acttcctcga gctgggcctc atgggccttc cgctcactgc ccgctttcca 120
   gtcgggaaac ctgtcgtgcc agctgcatta acatggtcat agctgtttcc ttgcgtattg 180
   ggcgctctcc gcttcctcgc tcactgactc gctgcgctcg gtcgttcggg taaagcctgg 240
   ggtgcctaat gagcaaaagg ccagcaaaag gccaggaacc gtaaaaaggc cgcgttgctg 300
   gcgtttttcc ataggctccg cccccctgac gagcatcaca aaaatcgacg ctcaagtcag 360
   aggtggcgaa acccgacagg actataaaga taccaggcgt ttccccctgg aagctccctc 420
   gtgcgctctc ctgttccgac cctgccgctt accggatacc tgtccgcctt tctcccttcg 480
   ggaagcgtgg cgctttctca tagctcacgc tgtaggtatc tcagttcggt gtaggtcgtt 540
   cgctccaagc tgggctgtgt gcacgaaccc cccgttcagc ccgaccgctg cgccttatcc 600
   ggtaactatc gtcttgagtc caacccggta agacacgact tatcgccact ggcagcagcc 660
   actggtaaca ggattagcag agcgaggtat gtaggcggtg ctacagagtt cttgaagtgg 720
   tggcctaact acggctacac tagaagaaca gtatttggta tctgcgctct gctgaagcca 780
   gttaccttcg gaaaaagagt tggtagctct tgatccggca aacaaaccac cgctggtagc 840
   ggtggttttt ttgtttgcaa gcagcagatt acgcgcagaa aaaaaggatc tcaagaagat 900
   cctttgatct tttctacggg gtctgacgct cagtggaacg aaaactcacg ttaagggatt 960
   ttggtcatga gattatcaaa aaggatcttc acctagatcc ttttaaatta aaaatgaagt 1020
   tttaaatcaa tctaaagtat atatgagtaa acttggtctg acagttacca atgcttaatc 1080
   agtgaggcac ctatctcagc gatctgtcta tttcgttcat ccatagttgc ctgactcccc 1140
   gtcgtgtaga taactacgat acgggagggc ttaccatctg gccccagtgc tgcaatgata 1200
   ccgcgagaac cacgctcacc ggctccagat ttatcagcaa taaaccagcc agccggaagg 1260
   gccgagcgca gaagtggtcc tgcaacttta tccgcctcca tccagtctat taattgttgc 1320
   cgggaagcta gagtaagtag ttcgccagtt aatagtttgc gcaacgttgt tgccattgct 1380
   acaggcatcg tggtgtcacg ctcgtcgttt ggtatggctt cattcagctc cggttcccaa 1440
   cgatcaaggc gagttacatg atcccccatg ttgtgcaaaa aagcggttag ctccttcggt 1500
   cctccgatcg ttgtcagaag taagttggcc gcagtgttat cactcatggt tatggcagca 1560
   ctgcataatt ctcttactgt catgccatcc gtaagatgct tttctgtgac tggtgagtac 1620
   tcaaccaagt cattctgaga atagtgtatg cggcgaccga gttgctcttg cccggcgtca 1680
   atacgggata ataccgcgcc acatagcaga actttaaaag tgctcatcat tggaaaacgt 1740
   tcttcggggc gaaaactctc aaggatctta ccgctgttga gatccagttc gatgtaaccc 1800
   actcgtgcac ccaactgatc ttcagcatct tttactttca ccagcgtttc tgggtgagca 1860
   aaaacaggaa ggcaaaatgc cgcaaaaaag ggaataaggg cgacacggaa atgttgaata 1920
   ctcatactct tcctttttca atattattga agcatttatc agggttattg tctcatgagc 1980
   ggatacatat ttgaatgtat ttagaaaaat aaacaaatag gggttccgcg cacatttccc 2040
   cgaaaagtgc cacctaaatt gtaagcgtta atattttgtt aaaattcgcg ttaaattttt 2100
   gttaaatcag ctcatttttt aaccaatagg ccgaaatcgg caaaatccct tataaatcaa 2160
   aagaatagac cgagataggg ttgagtggcc gctacagggc gctcccattc gccattcagg 2220
   ctgcgcaact gttgggaagg gcgtttcggt gcgggcctct tcgctattac gccagctggc 2280
   gaaaggggga tgtgctgcaa ggcgattaag ttgggtaacg ccagggtttt cccagtcacg 2340
   acgttgtaaa acgacggcca gtgagcgcga cgtaatacga ctcactatag ggcgaattgg 2400
   cggaaggccg tcaaggccgc atgaattcgc taccgggaag ttcctattcc gaagttccta 2460
   ttctatcaga agtataggaa cttcaggtac gtctcaggta c 2501
<210> 765
   <211> 888
   <212> DNA
   <213> Artificial Sequence
<220>
   <223> V-Cb entry TRBV6-6_TRBC2_F14-F15
<400> 765
   gccaccatga gcatcagcct cctgtgctgt gcagcctttc ctctcctgtg ggcaggtcca      60
   gtgaatgctg gtgtcactca gaccccaaaa ttccgcatcc tgaagatagg acagagcatg     120
   acactgcagt gtacccagga tatgaaccat aactacatgt actggtatcg acaagaccca     180
   ggcatggggc tgaagctgat ttattattca gttggtgctg gtatcactga taaaggagaa     240
   gtcccgaatg gctacaacgt gtccagatca accacagagg atttcccgct caggctggag     300
   ttggctgctc cctcccagac atctgtgtac ttttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 766
   <211> 891
   <212> DNA
   <213> Artificial Sequence
<220>
   <223> V-Cb entry TRBV7-2_TRBC2_F14-F15
<400> 766
   gccaccatgg gcaccaggct cctcttctgg gtggccttct gtctcctggg ggcagatcac      60
   acaggagctg gagtctccca gtcccccagt aacaaggtca cagagaaggg aaaggatgta     120
   gagctcaggt gtgatccaat ttcaggtcat actgcccttt actggtaccg acagagcctg     180
   gggcagggcc tggagttttt aatttacttc caaggcaaca gtgcaccaga caaatcaggg     240
   ctgcccagtg atcgcttctc tgcagagagg actgggggat ccgtgtccac tctgacgatc     300
   cagcgcacac agcaggagga ctcggccgtg tatctttgca gagaccttgc ggccgcatag     360
   gtctcagtgt tcccacccga ggtcgctgtg tttgagccat cagaagcaga gatctcccac     420
   acccaaaagg ccacactggt atgcctggcc acaggcttct accccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttc acctccgagt cttaccagca aggggtcctg     780
   tctgccacca tcctctatga gatcttgcta gggaaggcca ccttgtatgc cgtgctggtc     840
   agtgccctcg tgctgatggc catggtcaag agaaaggatt ccagaggcta g              891
<210> 767
   <211> 891
   <212> DNA
   <213> Artificial Sequence
<220>
   <223> V-Cb entry TRBV7-3_TRBC2_F14-F15
<400> 767
   gccaccatgg gcaccaggct cctctgctgg gcagccctgt gcctcctggg ggcagatcac      60
   acaggtgctg gagtctccca gacccccagt aacaaggtca cagagaaggg aaaatatgta     120
   gagctcaggt gtgatccaat ttcaggtcat actgcccttt actggtaccg acaaagcctg     180
   gggcagggcc cagagtttct aatttacttc caaggcacgg gtgcggcaga tgactcaggg     240
   ctgcccaacg atcggttctt tgcagtcagg cctgagggat ccgtgtctac tctgaagatc 300
   cagcgcacag agcgggggga ctcagccgtg tatctttgca gagaccttgc ggccgcatag 360
   gtctcagtgt tcccacccga ggtcgctgtg tttgagccat cagaagcaga gatctcccac 420
   acccaaaagg ccacactggt atgcctggcc acaggcttct accccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttc acctccgagt cttaccagca aggggtcctg 780
   tctgccacca tcctctatga gatcttgcta gggaaggcca ccttgtatgc cgtgctggtc 840
   agtgccctcg tgctgatggc catggtcaag agaaaggatt ccagaggcta g 891
<210> 768
   <211> 891
   <212> DNA
   <213> Artificial Sequence
<220>
   <223> V-Cb entry TRBV7-8_TRBC2_F14-F15
<400> 768
   gccaccatgg gcaccaggct cctctgctgg gtggtcctgg gtttcctagg gacagatcac 60
   acaggtgctg gagtctccca gtcccctagg tacaaagtcg caaagagagg acaggatgta 120
   gctctcaggt gtgatccaat ttcgggtcat gtatcccttt tttggtacca acaggccctg 180
   gggcaggggc cagagtttct gacttatttc cagaatgaag ctcaactaga caaatcgggg 240
   ctgcccagtg atcgcttctt tgcagaaagg cctgagggat ctgtctccac tctgaagatc 300
   cagcgcacac agcaggagga ctccgccgtg tatctttgca gagaccttgc ggccgcatag 360
   gtctcagtgt tcccacccga ggtcgctgtg tttgagccat cagaagcaga gatctcccac 420
   acccaaaagg ccacactggt atgcctggcc acaggcttct accccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttc acctccgagt cttaccagca aggggtcctg 780
   tctgccacca tcctctatga gatcttgcta gggaaggcca ccttgtatgc cgtgctggtc 840
   agtgccctcg tgctgatggc catggtcaag agaaaggatt ccagaggcta g 891
<210> 769
   <211> 888
   <212> DNA
   <213> Artificial Sequence
<220>
   <223> V-Cb entry TRBV19_TRBC2_F14-F15
<400> 769
   gccaccatga gcaaccaggt gctctgctgt gtggtccttt gtttcctggg agcaaacacc      60
   gtggatggtg gaatcactca gtccccaaag tacctgttca gaaaggaagg acagaatgtg     120
   accctgagtt gtgaacagaa tttgaaccac gatgccatgt actggtaccg acaggaccca     180
   gggcaagggc tgagattgat ctactactca cagatagtaa atgactttca gaaaggagat     240
   atagctgaag ggtacagcgt gtctcgggag aagaaggaat cctttcctct cactgtgaca     300
   tcggcccaaa agaacccgac agctttctat ctttgcagag accttgcggc cgcataggtc     360
   tcagtgttcc cacccgaggt cgctgtgttt gagccatcag aagcagagat ctcccacacc     420
   caaaaggcca cactggtatg cctggccaca ggcttctacc ccgaccacgt ggagctgagc     480
   tggtgggtga atgggaagga ggtgcacagt ggggtcagca cagacccgca gcccctcaag     540
   gagcagcccg ccctcaatga ctccagatac tgcctgagca gccgcctgag ggtgtcggcc     600
   accttctggc agaacccccg caaccacttc cgctgtcaag tccagttcta cgggctctcg     660
   gagaatgacg agtggaccca ggatagggcc aaacccgtca cccagatcgt cagcgccgag     720
   gcctggggta gagcagactg tggcttcacc tccgagtctt accagcaagg ggtcctgtct     780
   gccaccatcc tctatgagat cttgctaggg aaggccacct tgtatgccgt gctggtcagt     840
   gccctcgtgc tgatggccat ggtcaagaga aaggattcca gaggctag                  888
<210> 770
   <211> 882
   <212> DNA
   <213> Artificial Sequence
<220>
   <223> V-Cb entry TRBV20-1_TRBC2_F14-F15
<400> 770
   gccaccatgc tgctgcttct gctgcttctg gggccaggct ccgggcttgg tgctgtcgtg      60
   tctcaacatc cgagctgggt tatctgtaag agtggaacct ctgtgaagat cgagtgccgt     120
   tccctggact ttcaggccac aactatgttt tggtatcgtc agttcccgaa acagagtctc     180
   atgctgatgg caacttccaa tgagggctcc aaggccacat acgagcaagg cgtcgagaag     240
   gacaagtttc tcatcaacca tgcaagcctg accttgtcca ctctgacagt gaccagtgcc     300
   catcctgaag atagcagctt ctacatttgc agagaccttg cggccgcata ggtctcagtg     360
   ttcccacccg aggtcgctgt gtttgagcca tcagaagcag agatctccca cacccaaaag     420
   gccacactgg tatgcctggc cacaggcttc taccccgacc acgtggagct gagctggtgg 480
   gtgaatggga aggaggtgca cagtggggtc agcacagacc cgcagcccct caaggagcag 540
   cccgccctca atgactccag atactgcctg agcagccgcc tgagggtgtc ggccaccttc 600
   tggcagaacc cccgcaacca cttccgctgt caagtccagt tctacgggct ctcggagaat 660
   gacgagtgga cccaggatag ggccaaaccc gtcacccaga tcgtcagcgc cgaggcctgg 720
   ggtagagcag actgtggctt cacctccgag tcttaccagc aaggggtcct gtctgccacc 780
   atcctctatg agatcttgct agggaaggcc accttgtatg ccgtgctggt cagtgccctc 840
   gtgctgatgg ccatggtcaa gagaaaggat tccagaggct ag 882
<210> 771
   <211> 882
   <212> DNA
   <213> Artificial Sequence
<220>
   <223> V-Cb entry TRBV6-6_TRBC1_F14-F15
<400> 771
   gccaccatga gcatcagcct cctgtgctgt gcagcctttc ctctcctgtg ggcaggtcca 60
   gtgaatgctg gtgtcactca gaccccaaaa ttccgcatcc tgaagatagg acagagcatg 120
   acactgcagt gtacccagga tatgaaccat aactacatgt actggtatcg acaagaccca 180
   ggcatggggc tgaagctgat ttattattca gttggtgctg gtatcactga taaaggagaa 240
   gtcccgaatg gctacaacgt gtccagatca accacagagg atttcccgct caggctggag 300
   ttggctgctc cctcccagac atctgtgtac ttttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 772
   <211> 885
   <212> DNA
   <213> Artificial Sequence
<220>
   <223> V-Cb entry TRBV7-2_TRBC1_F14-F15
<400> 772
   gccaccatgg gcaccaggct cctcttctgg gtggccttct gtctcctggg ggcagatcac 60
   acaggagctg gagtctccca gtcccccagt aacaaggtca cagagaaggg aaaggatgta 120
   gagctcaggt gtgatccaat ttcaggtcat actgcccttt actggtaccg acagagcctg 180
   gggcagggcc tggagttttt aatttacttc caaggcaaca gtgcaccaga caaatcaggg 240
   ctgcccagtg atcgcttctc tgcagagagg actgggggat ccgtgtccac tctgacgatc 300
   cagcgcacac agcaggagga ctcggccgtg tatctttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 773
   <211> 885
   <212> DNA
   <213> Artificial Sequence
<220>
   <223> V-Cb entry TRBV7-3_TRBC1_F14-F15
<400> 773
   gccaccatgg gcaccaggct cctctgctgg gcagccctgt gcctcctggg ggcagatcac 60
   acaggtgctg gagtctccca gacccccagt aacaaggtca cagagaaggg aaaatatgta 120
   gagctcaggt gtgatccaat ttcaggtcat actgcccttt actggtaccg acaaagcctg 180
   gggcagggcc cagagtttct aatttacttc caaggcacgg gtgcggcaga tgactcaggg 240
   ctgcccaacg atcggttctt tgcagtcagg cctgagggat ccgtgtctac tctgaagatc 300
   cagcgcacag agcgggggga ctcagccgtg tatctttgca gagaccttgc ggccgcatag 360
   gtctcagtgt tcccacccga ggtcgctgtg tttgagccat cagaagcaga gatctcccac 420
   acccaaaagg ccacactggt gtgcctggcc acaggcttct tccccgacca cgtggagctg 480
   agctggtggg tgaatgggaa ggaggtgcac agtggggtca gcacagaccc gcagcccctc 540
   aaggagcagc ccgccctcaa tgactccaga tactgcctga gcagccgcct gagggtgtcg 600
   gccaccttct ggcagaaccc ccgcaaccac ttccgctgtc aagtccagtt ctacgggctc 660
   tcggagaatg acgagtggac ccaggatagg gccaaacccg tcacccagat cgtcagcgcc 720
   gaggcctggg gtagagcaga ctgtggcttt acctcggtgt cctaccagca aggggtcctg 780
   tctgccacca tcctctatga gatcctgcta gggaaggcca ccctgtatgc tgtgctggtc 840
   agcgcccttg tgttgatggc catggtcaag agaaaggatt tctga 885
<210> 774
   <211> 885
   <212> DNA
   <213> Artificial Sequence
<220>
   <223> V-Cb entry TRBV7-8_TRBC1_F14-F15
<400> 774
   gccaccatgg gcaccaggct cctctgctgg gtggtcctgg gtttcctagg gacagatcac      60
   acaggtgctg gagtctccca gtcccctagg tacaaagtcg caaagagagg acaggatgta     120
   gctctcaggt gtgatccaat ttcgggtcat gtatcccttt tttggtacca acaggccctg     180
   gggcaggggc cagagtttct gacttatttc cagaatgaag ctcaactaga caaatcgggg     240
   ctgcccagtg atcgcttctt tgcagaaagg cctgagggat ctgtctccac tctgaagatc     300
   cagcgcacac agcaggagga ctccgccgtg tatctttgca gagaccttgc ggccgcatag     360
   gtctcagtgt tcccacccga ggtcgctgtg tttgagccat cagaagcaga gatctcccac     420
   acccaaaagg ccacactggt gtgcctggcc acaggcttct tccccgacca cgtggagctg     480
   agctggtggg tgaatgggaa ggaggtgcac agtggggtca gcacagaccc gcagcccctc     540
   aaggagcagc ccgccctcaa tgactccaga tactgcctga gcagccgcct gagggtgtcg     600
   gccaccttct ggcagaaccc ccgcaaccac ttccgctgtc aagtccagtt ctacgggctc     660
   tcggagaatg acgagtggac ccaggatagg gccaaacccg tcacccagat cgtcagcgcc     720
   gaggcctggg gtagagcaga ctgtggcttt acctcggtgt cctaccagca aggggtcctg     780
   tctgccacca tcctctatga gatcctgcta gggaaggcca ccctgtatgc tgtgctggtc     840
   agcgcccttg tgttgatggc catggtcaag agaaaggatt tctga                     885
<210> 775
   <211> 882
   <212> DNA
   <213> Artificial Sequence
<220>
   <223> V-Cb entry TRBV19_TRBC1_F14-F15
<400> 775
   gccaccatga gcaaccaggt gctctgctgt gtggtccttt gtttcctggg agcaaacacc      60
   gtggatggtg gaatcactca gtccccaaag tacctgttca gaaaggaagg acagaatgtg     120
   accctgagtt gtgaacagaa tttgaaccac gatgccatgt actggtaccg acaggaccca 180
   gggcaagggc tgagattgat ctactactca cagatagtaa atgactttca gaaaggagat 240
   atagctgaag ggtacagcgt gtctcgggag aagaaggaat cctttcctct cactgtgaca 300
   tcggcccaaa agaacccgac agctttctat ctttgcagag accttgcggc cgcataggtc 360
   tcagtgttcc cacccgaggt cgctgtgttt gagccatcag aagcagagat ctcccacacc 420
   caaaaggcca cactggtgtg cctggccaca ggcttcttcc ccgaccacgt ggagctgagc 480
   tggtgggtga atgggaagga ggtgcacagt ggggtcagca cagacccgca gcccctcaag 540
   gagcagcccg ccctcaatga ctccagatac tgcctgagca gccgcctgag ggtgtcggcc 600
   accttctggc agaacccccg caaccacttc cgctgtcaag tccagttcta cgggctctcg 660
   gagaatgacg agtggaccca ggatagggcc aaacccgtca cccagatcgt cagcgccgag 720
   gcctggggta gagcagactg tggctttacc tcggtgtcct accagcaagg ggtcctgtct 780
   gccaccatcc tctatgagat cctgctaggg aaggccaccc tgtatgctgt gctggtcagc 840
   gcccttgtgt tgatggccat ggtcaagaga aaggatttct ga 882
<210> 776
   <211> 876
   <212> DNA
   <213> Artificial Sequence
<220>
   <223> V-Cb entry TRBV20-1_TRBC1_F14-F15
<400> 776
   gccaccatgc tgctgcttct gctgcttctg gggccaggct ccgggcttgg tgctgtcgtg 60
   tctcaacatc cgagctgggt tatctgtaag agtggaacct ctgtgaagat cgagtgccgt 120
   tccctggact ttcaggccac aactatgttt tggtatcgtc agttcccgaa acagagtctc 180
   atgctgatgg caacttccaa tgagggctcc aaggccacat acgagcaagg cgtcgagaag 240
   gacaagtttc tcatcaacca tgcaagcctg accttgtcca ctctgacagt gaccagtgcc 300
   catcctgaag atagcagctt ctacatttgc agagaccttg cggccgcata ggtctcagtg 360
   ttcccacccg aggtcgctgt gtttgagcca tcagaagcag agatctccca cacccaaaag 420
   gccacactgg tgtgcctggc cacaggcttc ttccccgacc acgtggagct gagctggtgg 480
   gtgaatggga aggaggtgca cagtggggtc agcacagacc cgcagcccct caaggagcag 540
   cccgccctca atgactccag atactgcctg agcagccgcc tgagggtgtc ggccaccttc 600
   tggcagaacc cccgcaacca cttccgctgt caagtccagt tctacgggct ctcggagaat 660
   gacgagtgga cccaggatag ggccaaaccc gtcacccaga tcgtcagcgc cgaggcctgg 720
   ggtagagcag actgtggctt tacctcggtg tcctaccagc aaggggtcct gtctgccacc 780
   atcctctatg agatcctgct agggaaggcc accctgtatg ctgtgctggt cagcgccctt 840
   gtgttgatgg ccatggtcaa gagaaaggat ttctga 876
<210> 777
   <211> 519
   <212> DNA
   <213> Artificial Sequence
<220>
   <223> Bidirectional terminator element (Esp3I to Esp3I site)
<400> 777
   cgtctcggac tccgctgatc ataatcaagc catatcacat ctgtagaggt ttacttgctt 60
   taaaaaacct ccacacctcc ccctgaacct gaaacataaa atgaatgcaa ttgttgttgt 120
   taacttgttt attgcagctt ataatggtta caaataaagc aatagcatca caaatttcac 180
   aaataaagca tttttttcac tgcattctag ttgtggtttg tccaaactca tcaatgtatc 240
   ttatcatgtc tggatctgct gaagagaagc cttcttcacc atagagccca ccgcatcccc 300
   agcatgcctg ctattgtcta cccaatcctc ccccttgctg tcctgcccca ccccaccccc 360
   cagaatagaa tgacacctac tcagacaatg cgttgcaatt tcctcatttt attaggaaag 420
   gacagtggga gtggcacctt ccagggtcaa ggaaggcacg ggggaggggc aaacaacaga 480
   tggctggcaa ctagaaggca cagtcgcaag gtcgagacg 519
<210> 778
   <211> 825
   <212> DNA
   <213> Artificial Sequence
<220>
   <223> TRA ORF
<400> 778
   atggagaccc tcttgggcct gcttatcctt tggctgcagc tgcaatgggt tagcagcaaa      60
   caggaggtga cgcagattcc tgcagctctg agtgtcccag aaggagaaaa cttggttctc     120
   aactgcagtt tcactgatag cgctatttac aacctccagt ggtttaggca ggaccctggg     180
   aaaggactca catctctgtt gcttattcag tcaagtcaga gagagcaaac aagtggacgc     240
   cttaatgcct cgctggataa atcatcagga cgtagtactt tatacattgc agcttctcag     300
   cctggtgact cagccaccta cctctgcgcc gtgaggccaa ccagcggcgg cagctacatc     360
   ccaacctttg gaagaggaac cagccttatt gttcatccgt atatccagaa ccctgaccct     420
   gccgtgtacc agctgagaga ctctaaatcc agtgacaagt ctgtctgcct attcaccgat     480
   tttgattctc aaacaaatgt gtcacaaagt aaggattctg atgtgtatat cacagacaaa     540
   actgtgctag acatgaggtc tatggacttc aagagcaaca gtgctgtggc ctggagcaac     600
   aaatctgact ttgcatgtgc aaacgccttc aacaacagca ttattccaga ggacaccttc     660
   ttccccagcc cagaaagttc ctgtgatgtc aagctggtcg agaaaagctt tgaaacagat     720
   acgaacctaa actttcaaaa cctgtcagtg attgggttcc gaatcctcct cctgaaagtg     780
   gccgggttta atctgctcat gacgctgcgg ctgtggtcca gctga                     825
<210> 779
   <211> 936
   <212> DNA
   <213> Artificial Sequence
<220>
   <223> TRB ORF
<400> 779
   atgagcatcg gcctcctgtg ctgtgcagcc ttgtctctcc tgtgggcagg tccagtgaat      60
   gctggtgtca ctcagacccc aaaattccag gtcctgaaaa caggacagag catgacactg     120
   cagtgtgccc aggatatgaa ccatgaatac atgtcctggt atcgacaaga cccaggcatg     180
   gggctgaggc tgattcatta ctcagttggt gctggtatca ctgaccaagg agaagtcccc     240
   aatggctaca atgtctccag atcaaccaca gaggatttcc cgctcaggct gctgtcggct     300
   gctccctccc agacatctgt gtacttttgc gccagcagct acgtgggcaa caccggcgaa     360
   ctgttctttg gagaaggctc taggctgacc gtactggagg acctgaaaaa cgtgttccca     420
   cccgaggtcg ctgtgtttga gccatcagaa gcagagatct cccacaccca aaaggccaca     480
   ctggtatgcc tggccacagg cttctacccc gaccacgtgg agctgagctg gtgggtgaat     540
   gggaaggagg tgcacagtgg ggtcagcaca gacccgcagc ccctcaagga gcagcccgcc     600
   ctcaatgact ccagatactg cctgagcagc cgcctgaggg tgtcggccac cttctggcag     660
   aacccccgca accacttccg ctgtcaagtc cagttctacg ggctctcgga gaatgacgag     720
   tggacccagg atagggccaa acccgtcacc cagatcgtca gcgccgaggc ctggggtaga     780
   gcagactgtg gcttcacctc cgagtcttac cagcaagggg tcctgtctgc caccatcctc     840
   tatgagatct tgctagggaa ggccaccttg tatgccgtgc tggtcagtgc cctcgtgctg     900
   atggccatgg tcaagagaaa ggattccaga ggctag                               936